# EUROPEAN PATENT SPECIFICATION

(11) **EP 3 841 086 B1**
(45) Date of publication and mention of the grant of the patent: **23.04.2025**
(21) Application number: 19852649.3
(22) Date of filing: 20.08.2019
(51) Int. Cl.: C07D 401/14, A61K 31/4439, A61P 13/00, C07D 403/14, C07D 405/14, C07D 471/04, C07D 409/14, C07D 491/052, C07D 487/04, A61P 27/00, A61K 31/506, A61K 31/501, A61K 31/5025, A61K 31/437, A61K 31/519

(54) **PHARMACEUTICAL COMPOUNDS FOR THE TREATMENT OF COMPLEMENT FACTOR D MEDICAL DISORDERS**
PHARMAZEUTISCHE ZUSAMMENSETZUNGEN ZUR BEHANDLUNG VON MEDIZINISCHEN STÖRUNGEN IM ZUSAMMENHANG MIT KOMPLEMENTFAKTOR D
COMPOSÉS PHARMACEUTIQUES POUR LE TRAITEMENT DE TROUBLES MÉDICAUX LIÉS AU FACTEUR D DU COMPLÉMENT

(30) Priority: 20.08.2018 US 201862765318 P; 06.09.2018 US 201862727847 P
(43) Date of publication of application: 30.06.2021
(73) Proprietor: Achillion Pharmaceuticals, Inc., New Haven, CT 06511 (US)
(72) Inventor: WILES, Jason, Allan, New Haven, CT 06511 (US); GADHACHANDA, Venkat, Rao, New Haven, CT 06511 (US); EASTMAN, Kyle, J., New Haven, CT 06511 (US); PAIS, Godwin, New Haven, CT 06511 (US)
(74) Representative: J A Kemp LLP
(86) International application number: PCT/US2019/047252
(87) International publication number: WO 2020/041301

(56) References cited:
- WO-A1-2017/035353
- WO-A1-2017/035353
- WO-A1-2017/035409
- WO-A2-2017/098328
- US-A1- 2019 038 623
- DATABASE Pubmed compound U.S. National Library of Medicine; 23 February 2016 (2016-02-23), "2-[3-Acetyl-1-[2-[(2S,4R)-2-[(3-chloro-2- fluorophenyl)methylcarbamoyl]-4-fluoropyrrolidin-1-yl]-2-oxoethyl]indol-5-yl]-4-nitrophenyl] hydrogen carbonate", XP055689216, retrieved from NCBI Database accession no. 118324207
- DATABASE PubChem ANONYMOUS: "(2S)-1-[2-(3-Acetyl-5-pyridazin-4-ylindol-1-yl)acetyl]-N-[(3-chloro-5-fluorophenyl)methyl]pyrrolidine-2-carboxamide | C28H25ClFN5O3 - PubChem", XP055798035, retrieved from NCBI
- DATABASE Pubmed U.S. National Library of Medicine; 25 January 2019 (2019-01-25), "1-[2-(5-Acetylpyrrolo[2,3-c]pyridazin-7- yl)acetyl]-N-(3,3-dimethylcyclohexyl)pyrrolidine-2-carboxamide", XP055689233, retrieved from NCBI Database accession no. 123543544

## Description

### Field of the Invention

This invention provides aryl, heteroaryl, and heterocyclic drugs and their use in treating medical disorders, such as complement-mediated disorders, including Complement Factor D mediated disorders.

### Background of the Invention

The complement system is a part of the innate immune system which does not adapt to changes over the course of the host's life, but is recruited and used by the adaptive immune system. For example, it assists, or complements, the ability of antibodies and phagocytic cells to clear pathogens. This sophisticated regulatory pathway allows rapid reaction to pathogenic organisms while protecting host cells from destruction. Over thirty proteins and protein fragments make up the complement system. These proteins act through opsonization (enhancing phagocytosis of antigens), chemotaxis (attracting macrophages and neutrophils), cell lysis (rupturing membranes of foreign cells), and agglutination (clustering and binding of pathogens together).

The complement system has three pathways: classical, alternative, and lectin. Complement Factor D plays an early and central role in activation of the alternative pathway of the complement cascade. Activation of the alternative complement pathway is initiated by spontaneous hydrolysis of a thioester bond within the C3 protein to produce C3(H₂O), which associates with Factor B to form the C3(H₂O)B complex. Complement Factor D acts to cleave Factor B within the C3(H₂O)B complex to form Ba and Bb. The Bb fragment remains associated with C3(H₂O) to form the alternative pathway C3 convertase C3(H₂O)Bb. Additionally, C3b generated by any of the C3 convertases also associates with Factor B to form C3bB, which Factor D cleaves to generate the later stage alternative pathway C3 convertase C3bBb. This latter form of the alternative pathway C3 convertase may provide important downstream amplification within all three of the defined complement pathways, leading ultimately to the recruitment and assembly of additional factors in the complement cascade pathway, including the cleavage of C5 to C5a and C5b. C5b acts in the assembly of factors C6, C7, C8, and C9 into the membrane attack complex, which can destroy pathogenic cells by lysing the cell.

The dysfunction of or excessive activation of complement has been linked to certain autoimmune, inflammatory, and neurodegenerative diseases, as well as ischemia-reperfusion injury and cancer. For example, activation of the alternative pathway of the complement cascade contributes to the production of C3a and C5a, both potent anaphylatoxins, which also have roles in a number of inflammatory disorders. Therefore, in some instances, it is desirable to decrease the response of the complement pathway, including the alternative complement pathway. Some examples of disorders mediated by the complement pathway include age-related macular degeneration (AMD), paroxysmal nocturnal hemoglobinuria (PNH), multiple sclerosis, and rheumatoid arthritis.

Additional complement-mediated disorders include those classified under component 3 glomerulopathy (C3G). C3G is a recently defined entity comprised of dense deposit disease (DDD) and C3 glomerulonephritis (C3GN) which encompasses a population of chronic kidney diseases wherein elevated activity of the alternative complement pathway and terminal complement pathway results in glomerular deposits made solely of complement C3 and no immunoglobulin (Ig).

Immune-complex membranoproliferative glomerulonephritis (IC-MPGN) is a renal disease which shares many clinical, pathologic, genetic and laboratory features with C3G, and therefore can be considered a sister disease of C3G. In the majority of patients with IC-MPGN, an underlying disease or disorder-most commonly infections, autoimmune diseases, or monoclonal gammopathies-are identified to which the renal disease is secondary. Patients with idiopathic IC-MPGN can have low C3 and normal C4 levels, similar to those observed in C3G, as well as many of the same genetic or acquired factors that are associated with abnormal alternative pathway activity. Although there are current hypotheses suggesting that the majority of IC-MPGN is attributable to over activity of the classical pathway, those patients with a low C3 and a normal C4 are likely to have significant overactivity of the alternative pathway. IC-MPGN patients with a low C3 and a normal C4 may benefit from alternative pathway inhibition.

Other disorders that have been linked to the complement cascade include atypical hemolytic uremic syndrome (aHUS), hemolytic uremic syndrome (HUS), abdominal aortic aneurysm, hemodialysis complications, hemolytic anemia, or hemodialysis, neuromyelitis optica (NMO), myasthenia gravis (MG), fatty liver, nonalcoholic steatohepatitis (NASH), liver inflammation, cirrhosis, liver failure, dermatomyositis, and amyotrophic lateral sclerosis.

Factor D is an attractive target for inhibition or regulation of the complement cascade due to its early and essential role in the alternative complement pathway, and for its potential role in signal amplification within the classical and lectin complement pathways. Inhibition of Factor D effectively interrupts the pathway and attenuates the formation of the membrane attack complex.

While initial attempts have been made to develop inhibitors of Factor D, there are currently no clinically approved small molecule Factor D inhibitors. Examples of Factor D inhibitor compounds are described in the following disclosures.

BioCryst Pharmaceuticals Inc. US Pat. No. 6,653,340 titled "Compounds useful in the complement, coagulate and kallikrein pathways and method for their preparation" describes fused bicyclic ring compounds that are inhibitors of Factor D. US Patent Application 2019-0142802 assigned to BioCryst Pharmaceuticals describes benzopyrazole compounds for the treatment of aberrant complement disorders. Additional patents assigned to BioCryst for the treatment and prevention of complement disorders include granted US patent 10,125,102 and US Applications US2018-0362458. Development of BioCryst's Factor D inhibitor BCX1470 was discontinued due to lack of specificity and short half-life of the compound.

Novartis PCT patent publication WO2012/093101 titled "Indole compounds or analogues thereof useful for the treatment of age-related macular degeneration" describes certain Factor D inhibitors. Additional Factor D inhibitors are described in Novartis PCT patent publications WO2012093101, WO2013/164802, WO2013/192345, WO2014/002051, WO2014/002052, WO2014/002053, WO2014/002054, WO2014/002057, WO2014/002058, WO2014/002059, WO2014/005150, WO2014/009833, WO2014/143638, WO2015/009616, WO2015/009977, WO2015/066241, and WO2016088082.

Lifesci Pharmaceuticals PCT patent publication WO2017/098328 titled "Therapeutic Inhibitory Compounds" describes various Factor D inhibitors with variations in the central core heterocyclic ring; such compounds are said to be useful in treating complement related disorders including autoimmune, inflammatory, and neurodegenerative disorders.. PCT patent publication WO2018/015818 is also titled "Therapeutic Inhibitory Compounds" and describes Factor D inhibitors without a cyclic central core.

Bristol-Myers Squibb PCT patent publication WO2004/045518 titled "Open chain prolyl urea-related modulators of androgen receptor function" describes open chain prolyl urea and thiourea related compounds for the treatment of androgen receptor-associated conditions, such as age-related diseases, for example, sarcopenia.

Alexion Pharmaceuticals PCT patent publication WO1995/029697 titled "Methods and compositions for the treatment of glomerulonephritis and other inflammatory diseases" discloses antibodies directed to C5 of the complement pathway for the treatment of glomerulonephritis and inflammatory conditions involving pathologic activation of the complement system. Alexion Pharmaceutical's anti-C5 antibody eculizumab (Soliris^{®}) is currently the only complement-specific antibody on the market, and is the first and only approved treatment for paroxysmal nocturnal hemoglobinuria (PNH).

Additional complement factor D inhibitors are described in U.S. Patent Nos. 9,598,446; 9,643,986; 9,663,543; 9,695,205; 9,732,103; 9,732,104; 9,758,537; 9,796,741; 9,828,396; 10,000,516; 10,005,802; 10,011,612; 10,081,645; 10,087,203; 10,092,584; 10,100,072; 10,138,225; 10,189,869; 10,106,563; 10,301,336; and 10,287,301; International Publication Nos. WO2019/028284; WO2018/160889; WO2018/160891; WO2018/160892; WO2017/035348; WO2017/035349; WO 2017/035351; WO 2017/035352; WO 2017/035353; WO 2017/035355; WO2017/035357; WO2017/035360; WO2017/035361; WO2017//035362; WO2017/035415; WO2017/035401; WO2017/035405; WO2017/035413; WO2017/035409; WO2017/035411; WO2017/035417; WO2017/035408 WO2015/130784; WO2015/130795; WO2015/130806; WO2015/130830; WO2015/130838; WO2015/130842; WO2015/130845; and WO2015/130854; and U.S. Patent Publication Nos. US 2016-0361329; US 2016-0362432; US 2016-0362433; US 2016-0362399; US 2017-0056428; US 2017-0057950; US 2017-0057993; US 2017-0189410; US 2017-0226142; US 2017-0260219; US 2017-0298084; US 2017-0298085; US 2018-0022766; US 2018-0022767; US 2018-0072762; US 2018-0030075; US 2018-0169109; US 2018-0177761; US 2018-0179185; US 2018-0179186; US 2018-0179236; US 2018-0186782; US 2018-0201580; US 2019-0031692; US 2019-0048033; US 2019-0144473; and US 2019-0211033 all owned by Achillion Pharmaceuticals, Inc. WO 2017/035353 titled "Aryl, heteroaryl, and heterocyclic compounds for treatment of medical disorders" describes compounds, methods of use, and processes for making inhibitors of complement Factor D, as well as pharmaceutically acceptable salts of such compounds and compositions comprising such; the described inhibitors of Factor D are said to reduce the excessive activitatoin of complement.

Given the wide variety of medical disorders that are caused by detrimental immune or inflammatory responses, new compounds are needed for medical treatment.

### SUMMARY

Provided herein is a compound selected from: 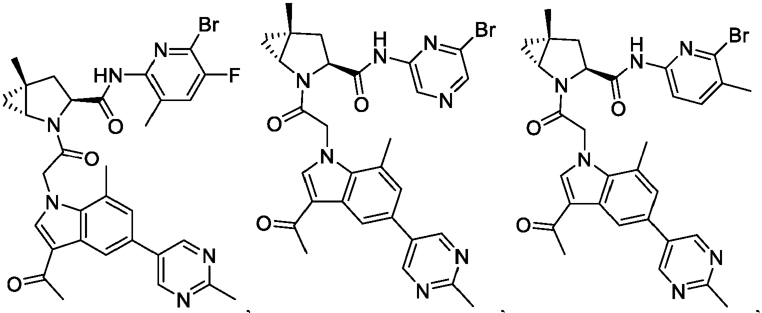 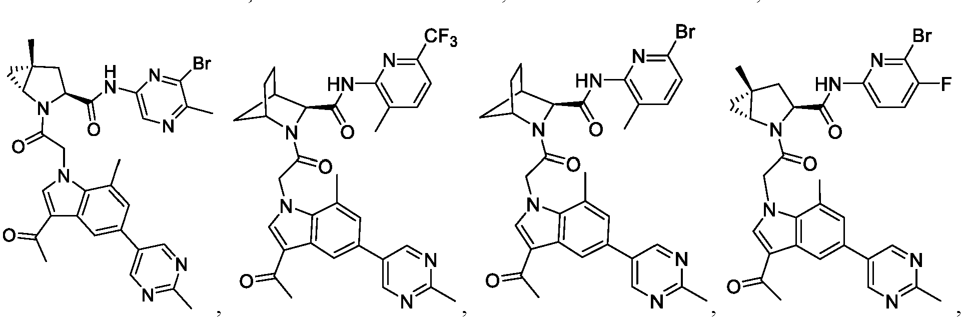 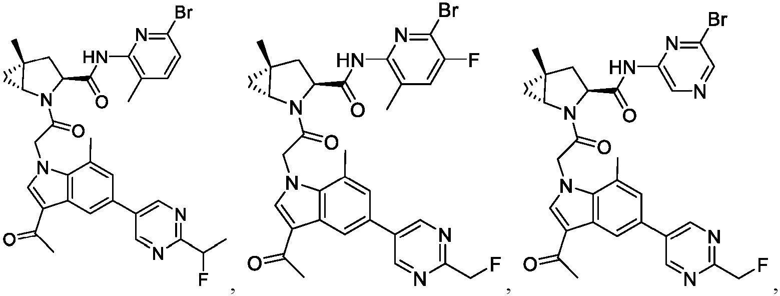 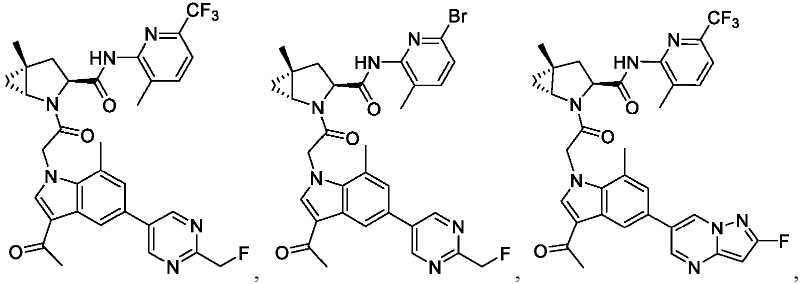 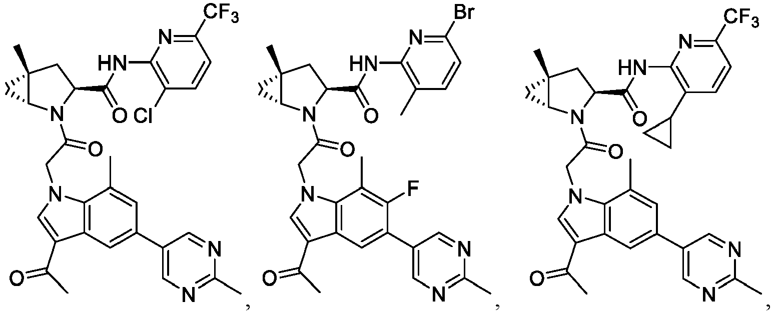 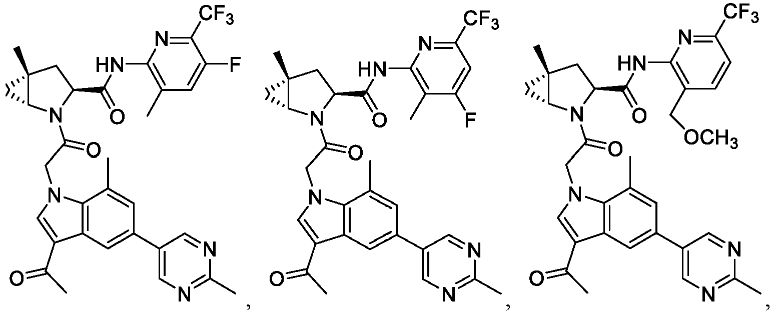 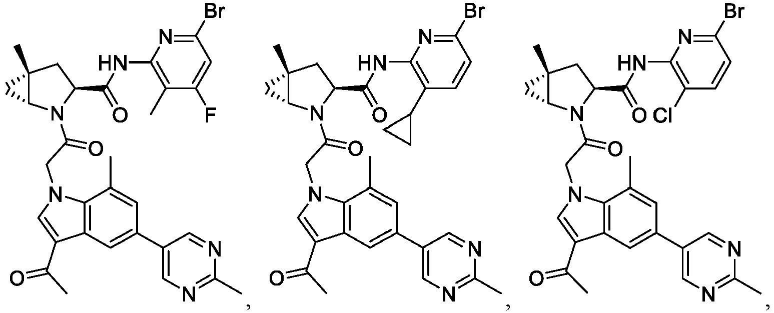 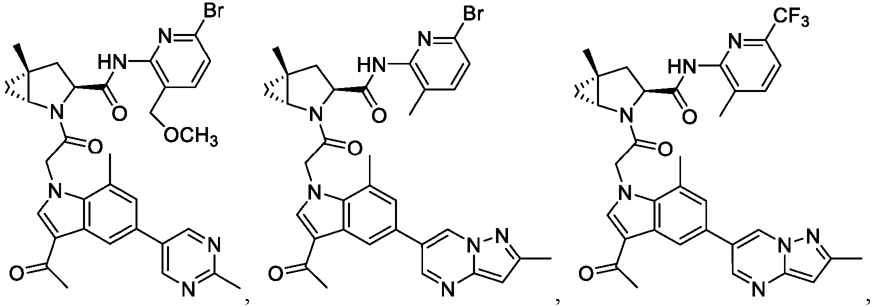 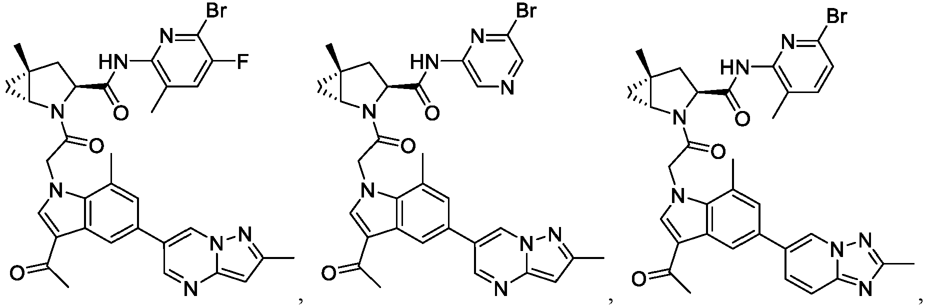 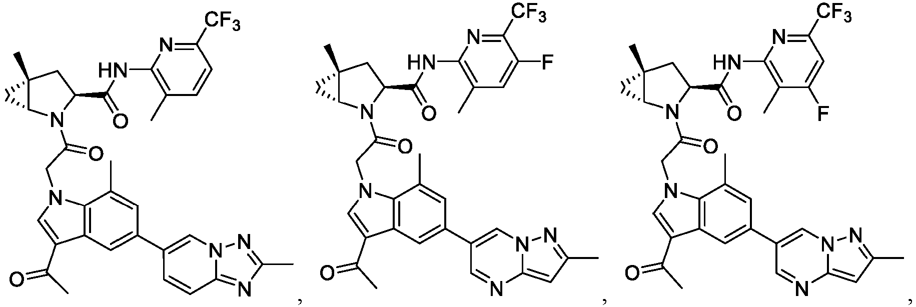 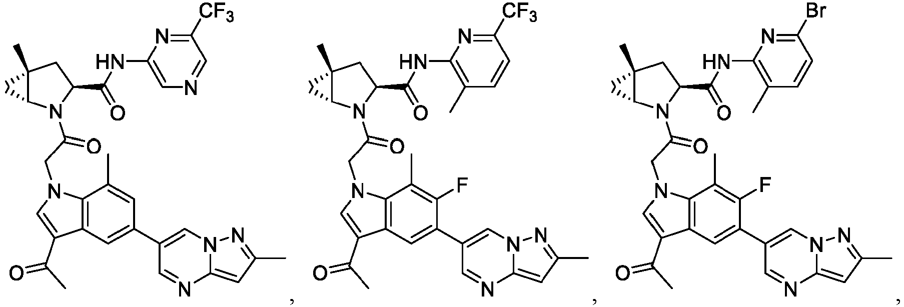 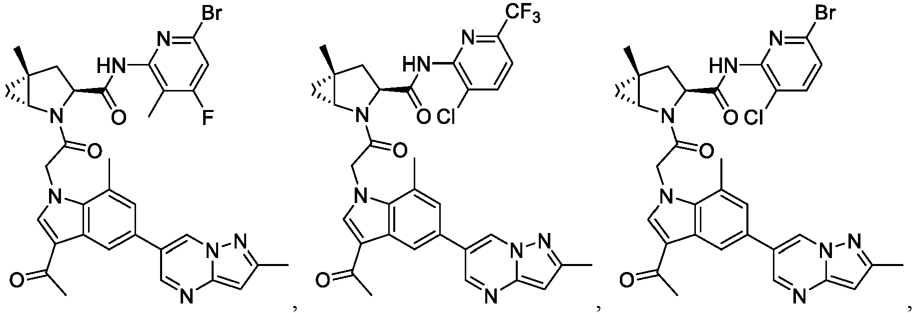 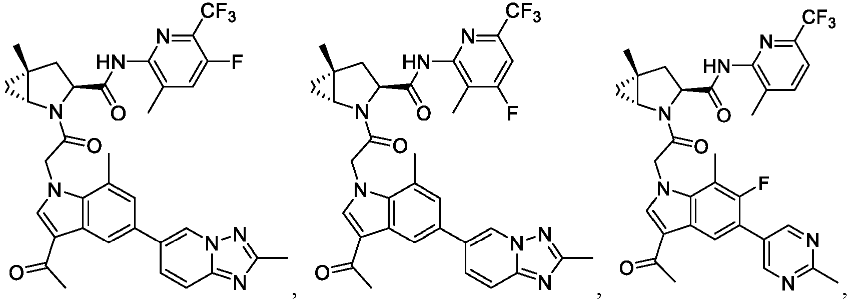 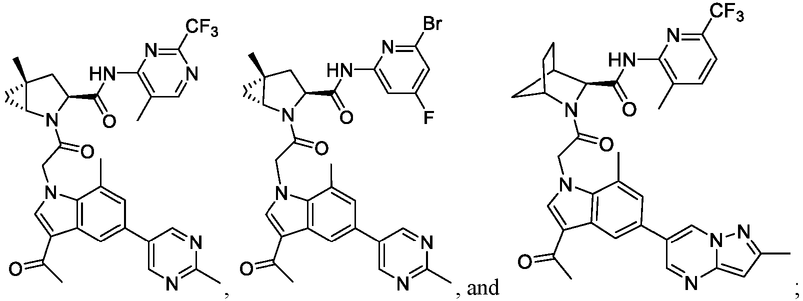 or a pharmaceutically acceptable salt thereof.

Also provided herein is a pharmaceutical composition comprising a compound provided herein or a pharmaceutically acceptable salt thereof, and a pharmaceutically acceptable carrier.

Also provided herein is a compound provided herein or a pharmaceutically acceptable salt thereof, or a composition comprising the compound or pharmaceutically acceptable salt and a pharmaceutically acceptable carrier, for use in treating a complement factor D mediated disorder in a subject in need thereof, wherein said use comprises administering a therapeutically effective amount of said compound or pharmaceutical composition to said subject;
wherein the disorder is selected from age-related macular degeneration (AMD), Alzheimer's disease, amyotrophic lateral sclerosis (ALS), antibody-mediated transplant rejection, arthritis, atypical hemolytic uremic syndrome (aHUS), an autoimmune disease, a cardiovascular disease, C3 glomerulopathy, C3 glomerulonephritis, dense deposit disease, dermatomyositis, geographic atrophy, IgA nephropathy, lupus, lupus nephritis, multiple sclerosis, myasthenia gravis, neuromyelitis optica, an ophthalmic disorder, paroxysmal nocturnal hemoglobinuria (PNH), a respiratory disease, rheumatoid arthritis, and sickle cell disease.

Also provided herein is a compound provided herein or a pharmaceutically acceptable salt thereof, or a composition comprising the compound or pharmaceutically acceptable salt and a pharmaceutically acceptable carrier, for use in treating a complement factor D mediated disorder in a subject in need thereof, wherein said use comprises administering a therapeutically effective amount of said compound or pharmaceutical composition to said subject,
wherein the disorder is selected from hereditary angioedema, capillary leak syndrome, hemolytic uremic syndrome (HUS), a neurological disorder, Guillain Barre Syndrome, a disease of the central nervous system or other neurodegenerative condition, glomerulonephritis, SLE nephritis, proliferative nephritis, liver fibrosis, tissue regeneration and neural regeneration, Barraquer-Simons Syndrome, inflammatory effects of sepsis, systemic inflammatory response syndrome (SIRS), a disorder of inappropriate or undesirable complement activation, interleukin-2 induced toxicity during IL-2 therapy, an inflammatory disorder, inflammation of autoimmune diseases, system lupus erythematosus (SLE), lupus nephritis, arthritis, an immune complex disorder, an autoimmune disease, systemic lupus or lupus erythematosus, ischemia/ reperfusion injury (I/R injury), myocardial infarction, myocarditis, post-ischemic reperfusion conditions, balloon angioplasty, atherosclerosis, post-pump syndrome in cardiopulmonary bypass or renal bypass, renal ischemia, mesenteric artery reperfusion after aortic reconstruction, antiphospholipid syndrome, autoimmune heart disease, obesity, diabetes, Alzheimer's dementia, stroke, schizophrenia, traumatic brain injury, trauma, Parkinson's disease, epilepsy, transplant rejection, prevention of fetal loss, a biomaterial reaction, hyperacute allograft rejection, xenograft rejection, transplantation, psoriasis, burn injury, thermal injury, crush injury, asthma, allergy, acute respiratory distress syndrome (ARDS), cystic fibrosis, adult respiratory distress syndrome, dyspnea, hemoptysis, chronic obstructive pulmonary disease (COPD), emphysema, pulmonary embolisms and infarcts, pneumonia, a fibrogenic dust disease, pulmonary fibrosis, an organic dust disease, chemical injury, smoke injury, bronchoconstriction, hypersensitivity pneumonitis, a parasitic disease, Goodpasture's Syndrome, pulmonary vasculitis, Pauci-immune vasculitis, and immune complex-associated inflammation.

Also provided herein is a compound provided herein or a pharmaceutically acceptable salt thereof, or a composition comprising the compound or pharmaceutically acceptable salt and a pharmaceutically acceptable carrier, for use in treating a complement factor D mediated disorder in a subject in need thereof, wherein said use comprises administering a therapeutically effective amount of said compound or pharmaceutical composition to said subject, wherein the disorder is selected from fatty liver, nonalcoholic steatohepatitis (NASH), liver inflammation, cirrhosis, and liver failure.

Also provided herein is a compound provided herein or a pharmaceutically acceptable salt thereof, or a composition comprising the compound or pharmaceutically acceptable salt and a pharmaceutically acceptable carrier, for use in treating a complement factor D mediated disorder in a subject in need thereof, wherein said use comprises administering a therapeutically effective amount of said compound or pharmaceutical composition to said subject, wherein the disorder is selected from glaucoma, diabetic retinopathy, a blistering cutaneous disease, ocular cicatricial pemphigoid, uveitis, adult macular degeneration, diabetic retinopathy retinitis pigmentosa, macular edema, diabetic macular edema, Behcet's uveitis, multifocal choroiditis, Vogt-Koyanagi-Harada syndrome, intermediate uveitis, birdshot retinochoroiditis, sympathetic ophthalmia, ocular pemphigus, non-arteritic ischemic optic neuropathy, postoperative inflammation, retinal vein occlusion, and central retinal vein occlusion (CVRO).

### FURTHER ASPECTS

In one embodiment, the compound or its salt or composition, as described herein is used to treat a medical disorder which is an inflammatory or immune condition, a disorder mediated by the complement cascade (including a dysfunctional cascade), a disorder or abnormality of a cell that adversely affects the ability of the cell to engage in or respond to normal complement activity including the alternative complement pathway, or an undesired complement-mediated response to a medical treatment, such as surgery or other medical procedure or a pharmaceutical or biopharmaceutical drug administration, a blood transfusion, or other allogenic tissue or fluid administration.

These compounds can be used to treat medical conditions in a host in need thereof, typically a human. In one embodiment, the active compound acts as an inhibitor of the complement factor D cascade. In one embodiment, the compounds are for use in treating such a disorder, wherein said use includes the administration of an effective amount of the compound, optionally in a pharmaceutically acceptable composition, as described in more detail below.

In certain embodiments, compounds are provided that have minimal effect on BSEP (bile salt export pump protein) (e.g., with an IC₅₀ of greater than about 20, 30, 40, 50, 60, 75 or 100 µM or greater), or with a therapeutic index of BSEP relative to complement D inhibition (e.g., IC₅₀ inhibition of BSEP/IC₅₀ inhibition of complement D inhibitor), of about at least 50, 100, 200, 300, 400, 500, 750 or 1000 or greater). BSEP inhibition correlates with cholestatic drug-induced liver injury.

The compounds provided herein may exhibit reduced hydrolysis of the amide bond *in vivo.* A substituent in the position ortho to the amide (for example 2-(attachment point)-3-methyl-6-substituted-pyridine or 2-(attachment point)-3-cyclopropyl-6-substituted-pyridine) may decrease the potential for formation of reactive metabolites. Acylation of an active compound provided herein may exhibit extended half-life or other advantageous pharmacokinetic properties, which may be achieved by albumin stabilization *in vivo.* The acylated analog can include several linking moieties in linear, branched or cyclic manner. Either one or a series of amino acids may be used as a linker to a terminal fatty acid. In one non-limiting example a non-natural amino acid such as one described below, for example 8-amino-3,6-dioxaoctanoic acid (one or several in sequence) may be covalently bound to a selected complement D inhibitor provided herein through a functional group such as a carboxylic acid, sulfonyl, hydroxyl or amino group. See generally Lau, et al., "Discovery of the Once-Weekly Glucagon-Like Peptide-1 (GLP-1) Analog Semiglutide", J. Med. Chem., 2015, 58, 7370-7380. The 8-amino-3,6-dioxaoctanoic acid or similar molecule may be covalently linked to an aliphatic acid, including but not limited to a C₁₆, C₁₈, C₂₀ aliphatic acid, or a dicarboxylic acid, including but not limited to a C₈, C₁₀, C₁₂, C₁₄, C₁₆, C₁₈ or C₂₀ diacid. One or more amino acids can also be used in the selected configuration to add length or functionality. A divalent linker moiety may be used, such as a dicarboxylic acid, amino acid, diamine, hydroxycarboxylic acid, hydroxyamine, di-hydroxy compound, or other compound that has at least two functional groups that can link the parent molecule with another linking moiety, and which may be albumin stabilized *in vivo.* 2, 3, 4 or 5 linking moieties may be covalently bound in sequence, branched or cyclic fashion to the parent compound. An acyl group may be located in a position of the active compound that does not significantly adversely affect the complement D inhibition of the molecule. The acyl group may have an aliphatic or heteroaliphatic carbon range of C₁₂, C₁₄, C₁₆, C₁₈, C₂₀, C₂₂ or C₂₄.

In one embodiment, the disorder is associated with the alternative complement cascade pathway. In yet another embodiment, the disorder is associated with the complement classical pathway. In a further embodiment, the disorder is associated with the complement lectin pathway. Alternatively, the active compound or its salt may act through a different mechanism of action than the complement cascade when being used in treating the disorder described herein.

In another embodiment, treating a host, typically a human, with a disorder mediated by the complement system, may include administration of a prophylactic antibiotic or vaccine to reduce the possibility of a bacterial infection during the treatment using one of the compounds described herein. In certain embodiments, the host, typically a human, is given a prophylactic vaccine prior to, during or after treatment with one of the compounds described herein. In certain embodiments, the host, typically a human, is given a prophylactic antibiotic prior to, during or after treatment with one of the compounds described herein. In some embodiments, the infection is a meningococcal infection (e.g., septicemia and/or meningitis), an Aspergillus infection, or an infection due to an encapsulated organism, for example, Streptococcus pneumoniae or Haemophilus influenza type b (Hib), especially in children. In other embodiments, the vaccine or antibiotic is administered to the patient after contracting an infection due to, or concommitent with inhibition of the complement system.

The present invention thus includes at least the following features:
a. a compound of the present invention or a pharmaceutically acceptable salt, optionally in a pharmaceutically acceptable composition
b. a compound of the present invention or a pharmaceutically acceptable salt, optionally in a pharmaceutically acceptable composition, for use in treating or preventing a disorder including but not limited to the development of fatty liver and conditions stemming from fatty liver, such as nonalcoholic steatohepatitis (NASH), liver inflammation, cirrhosis, or liver failure; dermatomyositis; amyotrophic lateral sclerosis; cytokine or inflammatory reactions in response to biotherapeutics (e.g. CAR T-cell therapy); paroxysmal nocturnal hemoglobinuria (PNH), rheumatoid arthritis, multiple sclerosis, age-related macular degeneration (AMD), retinal degeneration, other ophthalmic diseases (e.g., geographic atrophy), a respiratory disease, a cardiovascular disease, a complement alternative pathway (AP)-associated nephropathy, for example a component 3 glomerulopathy (C3G) disorder, for example C3 glomerulonephritis (C3GN) or dense deposit disease (DDD), or a membranoproliferative glomerulonephritis (MPGN) disorder, for example immune-complex membranoproliferative glomerulonephritis (IC-MPGN). a disorder of the central nervous system or peripheral nervous system such as acquired brain or spinal cord injury, including, but not limited to ischaemic-reperfusion injury or stroke, traumatic brain injury (TBI) and spinal cord injury (SCI), Alzheimers diseases (AD), multiple sclerosis, neuromyelitis optica (NMO), amyotrophic lateral sclerosis (ALS), Parkinson's disease (PD), Huntington's disease (HD), demyelinating myelinoclastic diseases, demyelinating leukostrophic diseases, and neurological inflammatory disorders.
c. a pharmaceutically acceptable composition of a compound of the present invention or its pharmaceutically acceptable salt, in a pharmaceutically acceptable carrier
d. a compound of the present invention or a pharmaceutically acceptable salt, optionally in a pharmaceutically acceptable composition, for use in treating or preventing a disorder mediated by the complement pathway, and for example, cascade Factor D
e. a compound of the present invention as described herein, or a pharmaceutically acceptable salt, optionally in a pharmaceutically acceptable composition, for use in treating or preventing a disorder, including but not limited to the development of fatty liver and conditions stemming from fatty liver, such as nonalcoholic steatohepatitis (NASH), liver inflammation, cirrhosis, liver failure; dermatomyositis; amyotrophic lateral sclerosis; cytokine or inflammatory reactions in response to biotherapeutics (e.g. CAR T-cell therapy); paroxysmal nocturnal hemoglobinuria (PNH), C3 glomerulonephritis, dense deposit disease, C3 glomerulopathy, rheumatoid arthritis, multiple sclerosis, age-related macular degeneration (AMD), retinal degeneration, other ophthalmic diseases (e.g., geographic atrophy), a respiratory disease or a cardiovascular disease
f. a compound provided herein for use in treating or preventing a disorder, or generally for treating or preventing disorders mediated by complement cascade Factor D
g. a compound of the present invention or a salt thereof as described herein in substantially pure form (e.g., at least 90, 95, or 98%)
h. a compound of the present invention as described herein, or a pharmaceutically acceptable salt, optionally in a carrier to form a pharmaceutically acceptable composition, for use in treating a medical disorder which is an inflammatory or immune condition, a disorder mediated by the complement cascade (including a dysfunctional cascade), a disorder or abnormality of a cell that adversely affects the ability of the cell to engage in or respond to normal complement activity, or an undesired complement-mediated response to a medical treatment, such as surgery or other medical procedure or a pharmaceutical or biopharmaceutical drug administration, a blood transfusion, or other allogenic tissue or fluid administration
i. For each of (a) through (h) above, and otherwise herein, each assembly of moieties and each active compound made therefrom or its use is considered and deemed specifically and individually disclosed, as such depiction is for convenience of space only and not intended to describe a only a genus or even a subgenus for such indication.

### DETAILED DESCRIPTION

### TERMINOLOGY

Compounds are described using standard nomenclature. Unless defined otherwise, all technical and scientific terms used herein have the same meaning as is commonly understood by one of skill in the art to which this invention belongs.

The compounds described herein include enantiomers, mixtures of enantiomers, diastereomers, tautomers, racemates and other isomers, such as rotamers, as if each is specifically described, unless otherwise indicated or otherwise excluded by context.

The terms "a" and "an" do not denote a limitation of quantity, but rather denote the presence of at least one of the referenced item. The term "or" means "and/or". Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable. All methods described herein can be performed in a suitable order unless otherwise indicated herein or otherwise clearly contradicted by context. The use of examples, or exemplary language (e.g., "such as"), is intended merely to better illustrate the invention and does not pose a limitation on the scope of the invention unless otherwise claimed. Unless defined otherwise, technical and scientific terms used herein have the same meaning as is commonly understood by one of skill in the art to which this invention belongs.

The present invention includes compounds with at least one desired isotopic substitution of an atom, at an amount above the natural abundance of the isotope, i.e., enriched.

Examples of isotopes that can be incorporated into compounds of the invention include isotopes of hydrogen, carbon, nitrogen, oxygen, phosphorous, fluorine, and chlorine, such as ²H, ³H, ¹¹C, ¹³C, ¹⁴C, ¹⁵N, ¹⁷O, ¹⁸O, ¹⁸F ³¹P, ³²P, ³⁵S, ³⁶CI, and ¹²⁵I respectively. In one embodiment, isotopically labelled compounds can be used in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F labeled compound may be particularly desirable for PET or SPECT studies. Isotopically labeled compounds of this invention and prodrugs thereof can generally be prepared by carrying out the procedures disclosed in the schemes or in the examples and preparations described below by substituting a readily available isotopically labeled reagent for a non-isotopically labeled reagent.

By way of general example and without limitation, isotopes of hydrogen, for example, deuterium (²H) and tritium (³H) may optionally be used anywhere in described structures that achieves the desired result. Alternatively or in addition, isotopes of carbon, e.g., ¹³C and ¹⁴C, may be used. In one embodiment, the isotopic substitution is replacing hydrogen with a deuterium at one or more locations on the molecule to improve the performance of the drug, for example, the pharmacodynamics, pharmacokinetics, biodistribution, half-life, stability, AUC, Tmax, Cmax, etc. For example, the deuterium can be bound to carbon in a location of bond breakage during metabolism (an α-deuterium kinetic isotope effect) or next to or near the site of bond breakage (a β-deuterium kinetic isotope effect).

Isotopic substitutions, for example deuterium substitutions, can be partial or complete. Partial deuterium substitution means that at least one hydrogen is substituted with deuterium. In certain embodiments, the isotope is 80, 85, 90, 95 or 99% or more enriched in an isotope at any location of interest. In certain embodiments deuterium is 80, 85, 90, 95 or 99% enriched at a desired location. Unless otherwise stated, the enrichment at any point is above natural abundance, and in an embodiment is enough to alter a detectable property of the drug in a human.

An alkyl residue may be deuterated (in nonlimiting embodiments, CDH₂, CD₂H, CD₃, CD₂CD₃, CHDCH₂D, CH₂CD₃, CHDCHD₂, OCDH₂, OCD₂H, or OCD₃ etc.)In certain other embodiments, when two substituents of the central core ring are combined to form a cyclopropyl ring, the unsubstituted methylene carbon may be deuterated.

The compounds of the present invention may form a solvate with solvents (including water). Therefore, in one embodiment, the invention includes a solvated form of the active compound. The term "solvate" refers to a molecular complex of a compound of the present invention (including a salt thereof) with one or more solvent molecules. Nonlimiting examples of solvents are water, ethanol, dimethyl sulfoxide, acetone and other common organic solvents. The term "hydrate" refers to a molecular complex comprising a compound of the invention and water. Pharmaceutically acceptable solvates in accordance with the invention include those wherein the solvent of crystallization may be isotopically substituted, *e.g.* D₂O, d₆-acetone, d₆-DMSO. A solvate can be in a liquid or solid form.

A dash ("-") that is not between two letters or symbols is used to indicate a point of attachment for a substituent. For example, -(C=O)NH₂ is attached through carbon of the keto (C=O) group.

The term "substituted", as used herein, means that any one or more hydrogens on the designated atom or group is replaced with a moiety selected from the indicated group, provided that the designated atom's normal valence is not exceeded and the resulting compound is stable. For example, when the substituent is oxo (i.e., =O) then two hydrogens on the atom are replaced. For example a pyridyl group substituted by oxo is a pyridone. Combinations of substituents and/or variables are permissible only if such combinations result in stable compounds or useful synthetic intermediates.

A stable active compound refers to a compound that can be isolated and can be formulated into a dosage form with a shelf life of at least one month. A stable manufacturing intermediate or precursor to an active compound is stable if it does not degrade within the period needed for reaction or other use. A stable moiety or substituent group is one that does not degrade, react or fall apart within the period necessary for use. Nonlimiting examples of unstable moieties are those that combine heteroatoms in an unstable arrangement, as typically known and identifiable to those of skill in the art.

Any suitable group may be present on a "substituted" or "optionally substituted" position that forms a stable molecule and meets the desired purpose of the invention and includes, but is not limited to, e.g., halogen (which can independently be F, Cl, Br or I); cyano; hydroxyl; nitro; azido; alkanoyl (such as a C₂-C₆ alkanoyl group); carboxamide; alkyl, cycloalkyl, alkenyl, alkynyl, alkoxy, aryloxy such as phenoxy; thioalkyl including those having one or more thioether linkages; alkylsulfinyl; alkylsulfonyl groups including those having one or more sulfonyl linkages; aryl (e.g., phenyl, biphenyl, naphthyl, or the like, each ring either substituted or unsubstituted); arylalkyl having for example, 1 to 3 separate or fused rings and from 6 to about 14 or 18 ring carbon atoms, with benzyl being an exemplary arylalkyl group; arylalkoxy, for example, having 1 to 3 separate or fused rings with benzyloxy being an exemplary arylalkoxy group; or a saturated or partially unsaturated heterocycle having 1 to 3 separate or fused rings with one or more N, O or S atoms, or a heteroaryl having 1 to 3 separate or fused rings with one or more N, O or S atoms, e.g. coumarinyl, quinolinyl, isoquinolinyl, quinazolinyl, pyridyl, pyrazinyl, pyrimidinyl, furanyl, pyrrolyl, thienyl, thiazolyl, triazinyl, oxazolyl, isoxazolyl, imidazolyl, indolyl, benzofuranyl, benzothiazolyl, tetrahydrofuranyl, tetrahydropyranyl, piperidinyl, morpholinyl, piperazinyl, and pyrrolidinyl. Such groups may be further substituted, e.g. with hydroxy, alkyl, alkoxy, halogen and amino. In certain embodiments "optionally substituted" includes one or more substituents independently selected from halogen, hydroxyl, amino, cyano, -CHO, -COOH, -CONH₂, alkyl including C₁-C₆alkyl, alkenyl including C₂-C₆alkenyl, alkynyl including C₂-C₆alkynyl, -C₁-C₆alkoxy, alkanoyl including C₂-C₆alkanoyl, (mono- and di-C₁-C₆alkylamino)C₀-C₂alkyl, haloalkyl including C₁-C₆haloalkyl, hydoxyC₁-C₆alkyl, ester, carbamate, urea, sulfonamide,-C₁-C₆alkyl(heterocyclo), C₁-C₆alkyl(heteroaryl), -C₁-C₆alkyl(C₃-C₇cycloalkyl), O-C₁-C₆alkyl(C₃-C₇cycloalkyl), B(OH)₂, phosphate, phosphonate and haloalkoxy including C₁-C₆haloalkoxy.

"Alkyl" is a branched, straight chain, or cyclic saturated aliphatic hydrocarbon group. In one embodiment, the alkyl contains from 1 to about 12 carbon atoms, more generally from 1 to about 6 carbon atoms or from 1 to about 4 carbon atoms. In one embodiment, the alkyl contains from 1 to about 8 carbon atoms. In certain embodiments, the alkyl is C₁-C₂, C₁-C₃, C₁-C₄, C₁-C₅ or C₁-C₆. The specified ranges as used herein indicate an alkyl group having each member of the range described as an independent species. For example, the term C₁-C₆ alkyl as used herein indicates a straight or branched alkyl group having from 1, 2, 3, 4, 5, or 6 carbon atoms and is intended to mean that each of these is described as an independent species. For example, the term C₁-C₄alkyl as used herein indicates a straight or branched alkyl group having from 1, 2, 3, or 4 carbon atoms and is intended to mean that each of these is described as an independent species. When C₀-Cₙ alkyl is used herein in conjunction with another group, for example, (C₃-C₇cycloalkyl)C₀-C₄ alkyl, or -C₀-C₄alkyl(C₃-C₇cycloalkyl), the indicated group, in this case cycloalkyl, is either directly bound by a single covalent bond (C₀alkyl), or attached by an alkyl chain in this case 1, 2, 3, or 4 carbon atoms. Alkyls can also be attached via other groups such as heteroatoms as in -O-C₀-C₄alkyl(C₃-C₇cycloalkyl). Examples of alkyl include, but are not limited to, methyl, ethyl, n-propyl, isopropyl, n-butyl, isobutyl, sec-butyl, t-butyl, n-pentyl, isopentyl, *tert-*pentyl, neopentyl, n-hexyl, 2-methylpentane, 3-methylpentane, 2,2-dimethylbutane, 2,3-dimethylbutane, and hexyl.

"Aliphatic" refers to a saturated or unsaturated, straight, branched, or cyclic hydrocarbon. "Aliphatic" is intended herein to include, but is not limited to, alkyl, alkenyl, alkynyl, cycloalkyl, cycloalkenyl, and cycloalkynyl moieties, and thus incorporates each of these definitions. In one embodiment, "aliphatic" is used to indicate those aliphatic groups having 1-20 carbon atoms. The aliphatic chain can be, for example, mono-unsaturated, di-unsaturated, tri-unsaturated, polyunsaturated, or alkynyl. Unsaturated aliphatic groups can be in a cis or trans configuration. In one embodiment, the aliphatic group contains from 1 to about 12 carbon atoms, more generally from 1 to about 6 carbon atoms or from 1 to about 4 carbon atoms. In one embodiment, the aliphatic group contains from 1 to about 8 carbon atoms. In certain embodiments, the aliphatic group is C₁-C₂, C₁-C₃, C₁-C₄, C₁-C₅ or C₁-C₆. The specified ranges as used herein indicate an aliphatic group having each member of the range described as an independent species. For example, the term C₁-C₆ aliphatic as used herein indicates a straight or branched alkyl, alkenyl, or alkynyl group having from 1, 2, 3, 4, 5, or 6 carbon atoms and is intended to mean that each of these is described as an independent species. For example, the term C₁-C₄ aliphatic as used herein indicates a straight or branched alkyl, alkenyl, or alkynyl group having from 1, 2, 3, or 4 carbon atoms and is intended to mean that each of these is described as an independent species. In one embodiment, the aliphatic group is substituted with one or more functional groups that results in the formation of a stable moiety.

The term "heteroaliphatic" refers to an aliphatic moiety that contains at least one heteroatom in the chain, for example, an amine, carbonyl, carboxy, oxo, thio, phosphate, phosphonate, nitrogen, phosphorus, silicon, or boron atoms in place of a carbon atom. In one embodiment, the only heteroatom is nitrogen. In one embodiment, the only heteroatom is oxygen. In one embodiment, the only heteroatom is sulfur. "Heteroaliphatic" is intended herein to include, but is not limited to, heteroalkyl, heteroalkenyl, heteroalkynyl, heterocycle, and heterocycloalkynyl moieties. In one embodiment, "heteroaliphatic" is used to indicate a heteroaliphatic group (cyclic, acyclic, substituted, unsubstituted, branched or unbranched) having 1-20 carbon atoms. In one embodiment, the heteroaliphatic group is optionally substituted in a manner that results in the formation of a stable moiety. Nonlimiting examples of heteroaliphatic moieties are polyethylene glycol, polyalkylene glycol, amide, polyamide, polylactide, polyglycolide, thioether, ether, alkyl-heterocycle-alkyl, -O-alkyl-O-alkyl, alkyl-O-haloalkyl, etc.

When a term is used that includes "alk" it should be understood that "cycloalkyl" or "carbocyclic" can be considered part of the definition, unless unambiguously excluded by the context. For example and without limitation, the terms alkyl, alkenyl, alkynyl, alkoxy, alkanoyl, alkenloxy, haloalkyl, etc. can all be considered to include the cyclic forms of alkyl, unless unambiguously excluded by context.

"Alkenyl" is a branched or straight chain aliphatic hydrocarbon group having one or more carbon-carbon double bonds that may occur at a stable point along the chain. Nonlimiting examples are C₂-C₈alkenyl, C₂-C₇alkenyl, C₂-C₆alkenyl, C₂-C₅alkenyl and C₂-C₄alkenyl. The specified ranges as used herein indicate an alkenyl group having each member of the range described as an independent species, as described above for the alkyl moiety. Examples of alkenyl include, but are not limited to, ethenyl and propenyl.

"Alkynyl" is a branched or straight chain aliphatic hydrocarbon group having one or more carbon-carbon triple bonds that may occur at any stable point along the chain, for example, C₂-C₈alkynyl or C₂-C₆alkynyl. The specified ranges as used herein indicate an alkynyl group having each member of the range described as an independent species, as described above for the alkyl moiety. Examples of alkynyl include, but are not limited to, ethynyl, propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

"Alkoxy" is an alkyl group as defined above covalently bound through an oxygen bridge (-O-). Examples of alkoxy include, but are not limited to, methoxy, ethoxy, n-propoxy, i-propoxy, n-butoxy, 2-butoxy, t-butoxy, n-pentoxy, 2-pentoxy, 3-pentoxy, isopentoxy, neopentoxy, n-hexoxy, 2-hexoxy, 3-hexoxy, and 3-methylpentoxy. Similarly an "alkylthio" or a "thioalkyl" group is an alkyl group as defined above with the indicated number of carbon atoms covalently bound through a sulfur bridge (-S-). In one embodiment, the alkoxy group is optionally substituted as described above.

"Alkenyloxy" is an alkenyl group as defined covalently bound to the group it substitutes by an oxygen bridge (-O-).

"Alkanoyl" is an alkyl group as defined above covalently bound through a carbonyl (C=O) bridge. The carbonyl carbon is included in the number of carbons, that is C₂alkanoyl is a CH₃(C=O)- group. In one embodiment, the alkanoyl group is optionally substituted as described

"Haloalkyl" indicates both branched and straight-chain alkyl groups substituted with 1 or more halogen atoms, up to the maximum allowable number of halogen atoms. Examples of haloalkyl include, but are not limited to, trifluoromethyl, monofluoromethyl, difluoromethyl, 2-fluoroethyl, and penta-fluoroethyl.

"Haloalkoxy" indicates a haloalkyl group as defined herein attached through an oxygen bridge (oxygen of an alcohol radical).

"Halo" or "halogen" indicates independently, any of fluoro, chloro, bromo or iodo.

"Aryl" indicates an aromatic group containing only carbon in the aromatic ring or rings. In one embodiment, the aryl group contains 1 to 3 separate or fused rings and is 6 to 14 or 18 ring atoms, without heteroatoms as ring members. When indicated, such aryl groups may be further substituted with carbon or non-carbon atoms or groups. Such substitution may include fusion to a 4 to 7 or a 5 to 7-membered saturated or partially unsaturated cyclic group that optionally contains 1, 2 or 3 heteroatoms independently selected from N, O, B, P, Si and S, to form, for example, a 3,4-methylenedioxyphenyl group. Aryl groups include, for example, phenyl and naphthyl, including 1-naphthyl and 2-naphthyl. In one embodiment, aryl groups are pendant. An example of a pendant ring is a phenyl group substituted with a phenyl group.

The term "heterocycle" refers to saturated and partially saturated heteroatom-containing ring radicals, where the heteroatoms may be selected from N, S, and O. The term "heterocycle" includes monocyclic 3-12 membered rings, as well as bicyclic 5-16 membered ring systems (which can include fused, bridged, or spiro, bicyclic ring systems). It does not include rings containing - O-O-. -O-S-, or -S-S- portions. Examples of saturated heterocycle groups include saturated 4- to 7-membered monocyclic groups containing 1 to 4 nitrogen atoms [e.g. pyrrolidinyl, imidazolidinyl, piperidinyl, pyrrolinyl, azetidinyl, piperazinyl, and pyrazolidinyl]; saturated 4 to 6-membered monocyclic groups containing 1 to 2 oxygen atoms and 1 to 3 nitrogen atoms [e.g. morpholinyl]; saturated 3 to 6-membered heteromonocyclic group containing 1 to 2 sulfur atoms and 1 to 3 nitrogen atoms [e.g., thiazolidinyl]. Examples of partially saturated heterocycle radicals include but are not limited to, dihydrothienyl, dihydropyranyl, dihydrofuryl, and dihydrothiazolyl. Examples of partially saturated and saturated heterocycle groups include but are not limited to, pyrrolidinyl, imidazolidinyl, piperidinyl, pyrrolinyl, pyrazolidinyl, piperazinyl, morpholinyl, tetrahydropyranyl, thiazolidinyl, dihydrothienyl, 2,3-dihydro-benzo[1,4]dioxanyl, indolinyl, isoindolinyl, dihydrobenzothienyl, dihydrobenzofuryl, isochromanyl, chromanyl, 1,2-dihydroquinolyl, 1,2,3,4- tetrahydro-isoquinolyl, 1 ,2,3,4-tetrahydro-quinolyl, 2,3,4,4a,9,9a-hexahydro-1H-3-aza-fluorenyl, 5,6,7- trihydro-1,2,4-triazolo[3,4-a]isoquinolyl, 3,4-dihydro-2H-benzo[l,4]oxazinyl, benzo[l,4]dioxanyl, 2,3- dihydro-1H-1λ'-benzo[d]isothiazol-6-yl, dihydropyranyl, dihydrofuryl and dihydrothiazolyl. "Bicyclic heterocycle" includes groups wherein the heterocyclic radical is fused with an aryl radical wherein the point of attachment is the heterocycle ring. "Bicyclic heterocycle" also includes heterocyclic radicals that are fused with a carbocycle radical. For example partially unsaturated condensed heterocyclic group containing 1 to 5 nitrogen atoms, for example, indoline, isoindoline, partially unsaturated condensed heterocyclic group containing 1 to 2 oxygen atoms and 1 to 3 nitrogen atoms, partially unsaturated condensed heterocyclic group containing 1 to 2 sulfur atoms and 1 to 3 nitrogen atoms, and saturated condensed heterocyclic group containing 1 to 2 oxygen or sulfur atoms.

Non-limiting examples of bicyclic heterocycles include:

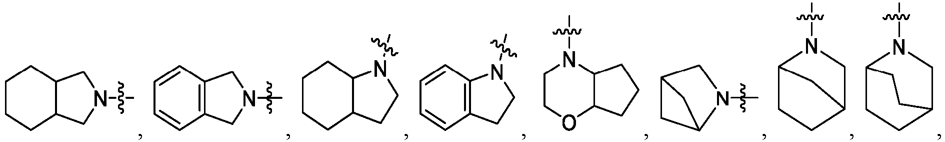

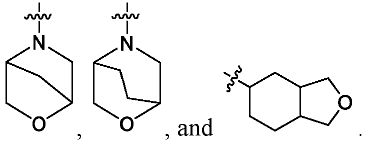

Unless otherwise drawn or clear from the context, the term "bicyclic heterocycle" includes cis and trans diastereomers. Non-limiting examples of chiral bicyclic heterocycles include:

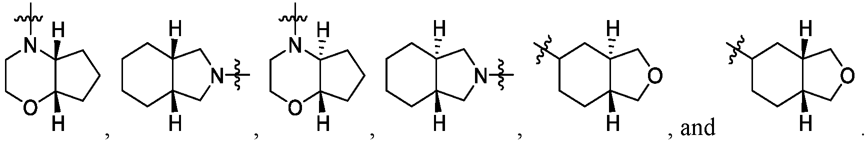

"Heteroaryl" refers to a stable monocyclic, bicyclic, or multicyclic aromatic ring which contains from 1 to 3, or in some embodiments from 1, 2, or 3 heteroatoms selected from N, O, S, B, and P (and typically selected from N, O, and S) with remaining ring atoms being carbon, or a stable bicyclic or tricyclic system containing at least one 5, 6, or 7 membered aromatic ring which contains from 1 to 3, or in some embodiments from 1 to 2, heteroatoms selected from N, O, S, B or P with remaining ring atoms being carbon. In one embodiment, the only heteroatom is nitrogen. In one embodiment, the only heteroatom is oxygen. In one embodiment, the only heteroatom is sulfur. Monocyclic heteroaryl groups typically have from 5 or 6 ring atoms. In some embodiments bicyclic heteroaryl groups are 8- to 10-membered heteroaryl groups, that is, groups containing 8 or 10 ring atoms in which one 5, 6, or 7 member aromatic ring is fused to a second aromatic or non-aromatic ring wherein the point of attachment is the aromatic ring. When the total number of S and O atoms in the heteroaryl group exceeds 1, these heteroatoms are not adjacent to one another. In one embodiment, the total number of S and O atoms in the heteroaryl group is not more than 2. In another embodiment, the total number of S and O atoms in the aromatic heterocycle is not more than 1. Examples of heteroaryl groups include, but are not limited to, pyridinyl (including, for example, 2-hydroxypyridinyl), imidazolyl, imidazopyridinyl, pyrimidinyl (including, for example, 4-hydroxypyrimidinyl), pyrazolyl, triazolyl, pyrazinyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, tetrahydrofuranyl, and furopyridinyl. Heteroaryl groups are optionally substituted independently with one or more substituents described herein. "Heteroaryloxy" is a heteroaryl group as described bound to the group it substituted via an oxygen, -O-, linker.

"Heterocycloalkyl" is a fully saturated heterocycle as defined herein. It may have, for example, include 1, 2, 3, or 4 heteroatoms independently selected from N, S, O, Si and B with the remaining ring atoms being carbon. In a typical embodiment, nitrogen is the heteroatom. Monocyclic heterocycloalkyl groups typically have from 3 to about 8 ring atoms or from 4 to 6 ring atoms.

A "dosage form" means a unit of administration of an active agent. Examples of dosage forms include tablets, capsules, injections, suspensions, liquids, emulsions, implants, particles, spheres, creams, ointments, suppositories, inhalable forms, transdermal forms, buccal, sublingual, topical, gel, mucosal, and the like. A "dosage form" can also include an implant, for example an optical implant.

"Pharmaceutical compositions" are compositions comprising at least one active agent, and at least one other substance, such as a carrier. "Pharmaceutical combinations" are combinations of at least two active agents which may be combined in a single dosage form or provided together in separate dosage forms with instructions that the active agents are to be used together to treat any disorder described herein.

A "pharmaceutically acceptable salt" is a derivative of the disclosed compound in which the parent compound is modified by making inorganic and organic, pharmaceutically acceptable, acid or base addition salts thereof. The salts of the present compounds can be synthesized from a parent compound that contains a basic or acidic moiety by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are typical, where practicable. Salts of the present compounds further include solvates of the compounds and of the compound salts.

Examples of pharmaceutically acceptable salts include, but are not limited to, mineral or organic acid salts of basic residues such as amines; alkali or organic salts of acidic residues such as carboxylic acids; and the like. The pharmaceutically acceptable salts include salts which are acceptable for human consumptionand the quaternary ammonium salts of the parent compound formed, for example, from inorganic or organic acids. Examples, of such salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH₂)₁₋₄-COOH, and the like, or using a different acid that produces the same counterion. Lists of additional suitable salts may be found, e.g., in Remington's Pharmaceutical Sciences, 17th ed., Mack Publishing Company, Easton, Pa., p. 1418 (1985).

The term "carrier" applied to pharmaceutical compositions/combinations of the invention refers to a diluent, excipient, or vehicle with which an active compound is provided.

A "pharmaceutically acceptable excipient" means an excipient that is useful in preparing a pharmaceutical composition/combination that is generally safe, acceptable for human consumption, and neither biologically nor otherwise inappropriate for administration to a host, typically a human. In one embodiment, an excipient is used that is acceptable for veterinary use.

A "patient" or "host" or "subject" is a human or non-human animal in need of treatment or prevention of any of the disorders as specifically described herein, including but not limited to by modulation of the complement Factor D pathway or with a condition that is treatable with one of the compounds described herein. Typically the host is a human. A "patient" or "host" or "subject" also refers to for example, a mammal, primate (e.g., human), cows, sheep, goat, horse, dog, cat, rabbit, rat, mice, bird and the like.

A "prodrug" as used herein, means a compound which when administered to a host *in vivo* is converted into a parent drug. As used herein, the term "parent drug" means any of the presently described chemical compounds herein. Prodrugs can be used to achieve any desired effect, including to enhance properties of the parent drug or to improve the pharmaceutic or pharmacokinetic properties of the parent, including to increase the half-life of the drug *in vivo.* Prodrug strategies provide choices in modulating the conditions for *in vivo* generation of the parent drug. Nonlimiting examples of prodrug strategies include covalent attachment of removable groups, or removable portions of groups, for example, but not limited to acylation, phosphorylation, phosphonylation, phosphoramidate derivatives, amidation, reduction, oxidation, esterification, alkylation, other carboxy derivatives, sulfoxy or sulfone derivatives, carbonylation or anhydride, among othersThe prodrug may render the parent compound more lipophilic. A prodrug can have several prodrug moieties in linear, branched or cyclic manner. For example, a divalent linker moiety such as a dicarboxylic acid, amino acid, diamine, hydroxycarboxylic acid, hydroxyamine, di-hydroxy compound, or other compound that has at least two functional groups can link the parent molecule with another prodrug moiety, and is typically biodegradable *in vivo.* 2, 3, 4, or 5 prodrug biodegradable moieties may be covalently bound in sequence, branched or cyclic fashion to the parent compound. Nonlimiting examples of prodrugs may be formed with:
i. a carboxylic acid on the parent drug and a hydroxylated prodrug moiety to form an ester;
ii. a carboxylic acid on the parent drug and an amine prodrug to form an amide;
iii. an amino on the parent drug and a carboxylic acid prodrug moiety to form an amide,
iv. an amino on the parent drug and a sulfonic acid to form a sulfonamide;
v. a sulfonic acid on the parent drug and an amino on the prodrug moiety to form a sulfonamide;
vi. a hydroxyl group on the parent drug and a carboxylic acid on the prodrug moiety to form an ester;
vii. a hydroxyl on the parent drug and a hydroxylated prodrug moiety to form an ether;
viii. a phosphonate on the parent drug and a hydroxylated prodrug moiety to form a phosphonate ester;
ix. a phosphoric acid on the parent drug and a hydroxylated prodrug moiety to form a phosphate ester;
x. a hydroxyl on the parent drug and a phosphonate on the prodrug to form a phosphonate ester;
xi. a hydroxyl on the parent drug and a phosphoric acid prodrug moiety to form a phosphate ester;
xii. a carboxylic acid on the parent drug and a prodrug of the structure HO-(CH₂)₂-O-(C₂₋₂₄ aliphatic group), for example, HO-(CH₂)₂-O-(C₂₋₂₄ alkyl group) to form an ester;
xiii. a carboxylic acid on the parent drug and a prodrug of the structure HO-(CH₂)₂-S-(C₂₋₂₄ aliphatic group), for example, HO-(CH₂)₂-S-(C₂₋₂₄ alkyl group) to form a thioester;
xiv. a hydroxyl on the parent drug and a prodrug of the structure HO-(CH₂)₂-O-(C₂₋₂₄ aliphatic group), for example, HO-(CH₂)₂-O-(C₂₋₂₄ alkyl group) to form an ether;
xv. a carboxylic acid on the parent drug and a prodrug of the structure HO-(CH₂)₂-S-(C₂₋₂₄ aliphatic group), for example, HO-(CH₂)₂-S-(C₂₋₂₄ alkyl group), to form a thioether; and
xvi. a carboxylic acid, oxime, hydrazide, hydrazone, amine or hydroxyl on the parent compound and a prodrug moiety that is a biodegradable polymer or oligomer including but not limited to polylactic acid, polylactide-co-glycolide, polyglycolide, polyethylene glycol, polyanhydride, polyester, polyamide or a peptide. An exemplary synthesis of Oxime linkages is provided in the paper published by Jin et. al. titled "Oxime Linkage: A Robust Tool for the Design of PH-Sensitive Polymeric Drug Carriers" in BioMacromolecules, 2011, 12(10), 3460-3468.

A prodrug may be provided by attaching a natural or non-natural amino acid to an appropriate functional moiety on the parent compound, for example, oxygen, nitrogen, or sulfur, and typically oxygen or nitrogen, usually in a manner such that the amino acid can be cleaved *in vivo* to provide the parent drug. The amino acid can be used alone or covalently linked (straight, branched or cyclic) to one or more other prodrug moieties to modify the parent drug to achieve the desired performance, such as increased half-life, lipophilicity, or other drug delivery or pharmacokinetic properties. The amino acid can be any compound with an amino group and a carboxylic acid, which includes an aliphatic amino acid, alkyl amino acid, aromatic amino acid, heteroaliphatic amino acid, heteroalkyl amino acid, or heterocyclic amino acid or heteroaryl amino acid.

"Providing a compound with at least one additional active agent," for example, in one embodiment can mean that the compound and the additional active agent(s) are provided simultaneously in a single dosage form, provided concomitantly in separate dosage forms, or provided in separate dosage forms for administration. In one embodiment, the compound administrations are separated by some amount of time that is within the time in which both the compound and the at least one additional active agent are within the blood stream of a patient. In certain embodiments the compound and the additional active agent need not be prescribed for a patient by the same medical care worker. In certain embodiments the additional active agent or agents need not require a prescription. Administration of the compound or the at least one additional active agent can occur via any appropriate route, for example, oral tablets, oral capsules, oral liquids, inhalation, injection, suppositories, parenteral, sublingual, buccal, intravenous, intraaortal, transdermal, polymeric controlled delivery, non-polymeric controlled delivery, nano or microparticles, liposomes, and/or topical contact. In one embodiment, the instructions for administration in a form of combination therapy is provided in the drug labeling.

A "therapeutically effective amount" of a pharmaceutical composition/combination of this invention means an amount effective, when administered to a host, provides a therapeutic benefit such as an amelioration of symptoms or reduction or dimunition of the disease itself. In one embodiment, a therapeutically effective amount is an amount sufficient to prevent a significant increase or will significantly reduce the detectable level of complement Factor D in the patient's blood, serum, or tissues.

### N-Oxides

Any of the active compounds can be provided in its N-oxide form to a patient in need thereof. An N-oxide of an active compound or a precursor of the active compound may be used in a manufacturing scheme. The N-oxide may be a metabolite of administration of one of the active compounds herein, and may have independent activity. The N-oxide can be formed by treating the compound of interest with an oxidizing agent, for example a suitable peroxyacid or peroxide, to generate an N-oxide compound. For example, a heteroaryl group, for example a pyridyl group, can be treated with an oxidizing agent such as sodium percarbonate in the presence of a rhenium-based catalyst under mild reaction conditions to generate an N-oxide compound. A person skilled in the art will understand that appropriate protecting groups may be necessary to carry out the chemistry. See, Jain, S.L. et al., "Rhenium-Catalyzed Highly Efficient Oxidations of Tertiary Nitrogen Compounds to N-Oxides Using Sodium Percarbonate as Oxygen Source, Synlett, 2261-2663, 2006.

Any of the active compounds with a sulfur can be provided in its sulfoxide or sulfone form to a patient in need thereof. A sulfoxide or sulfone of one of the active compounds or a precursor of the active compound may be used in a manufacturing scheme. A sulfur atom in a selected compound can be oxidized to form a sulfoxide 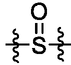 or a sulfone 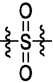 using known methods. For example, the compound 1,3,5-triazo-2,4,6-triphosphorine-2,2,4,4,6,6-tetrachloride (TAPC) is an efficient promoter for the oxidation of sulfides to sulfoxides. *See,* Bahrami, M. et al., "TAPC-Promoted Oxidation of sulfides and Deoxygenation of Sulfoxides", J. Org. Chem., 75, 6208-6213 (2010). Oxidation of sulfides with 30% hydrogen peroxide catalyzed by tantalum carbide provides sulfoxides in high yields, see, Kirihara, A., et al., "Tantalum Carbide or Niobium Carbide Catalyzed Oxidation of Sulfides with Hydrogen Peroxide: Highly Efficient and Chemoselective Syntheses of Sulfoxides and Sulfones", Synlett, 1557-1561 (2010). Sulfides can be oxidized to sulfones using, for example, niobium carbide as the catalyst, see, Kirihara, A., et al., "Tantalum Cardide or Niobium Carbide Catalyzed Oxidation of Sulfides with Hydrogen Peroxide: Highly Efficient and Chemoselective Syntheses of Sulfoxides and Sulfones", Synlett, 1557-1561 (2010). Urea-hydrogen peroxide adduct is a stable inexpensive and easily handled reagent for the oxidation of sulfides to sulfones, see Varma, R.S. and Naicker, K.P., "The Urea-Hydrogen Peroxide Complex: Solid-State Oxidative Protocols for Hydroxylated Aldehydes and Ketones (Dakin Reaction), Nitriles, Sulfides, and Nitrogen Heterocycles", Org. Lett., 1, 189-191 (1999). One skilled in the art will appreciate that other heteroatoms, such as nitrogen, may need to be protected and then deprotected while carrying out the oxidation of a sulfur atom to produce the desired compound.

### COMPOUNDS

Provided herein is a compound selected from 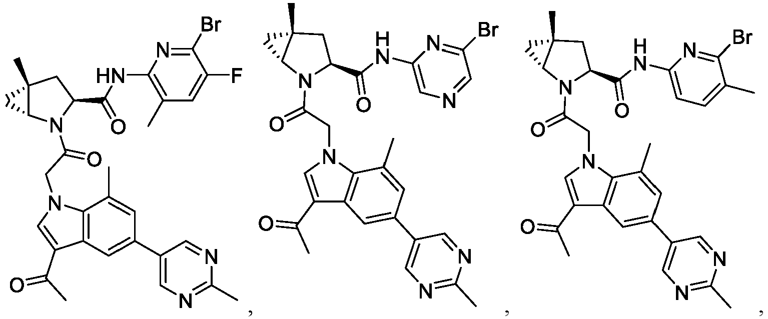 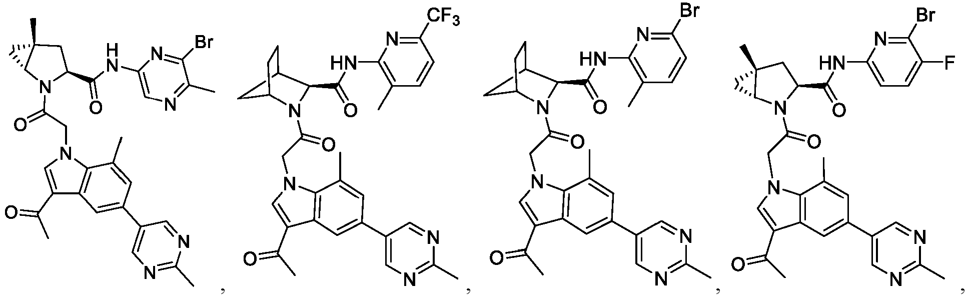 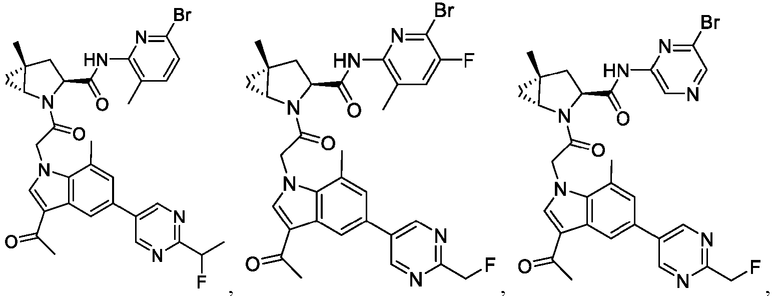 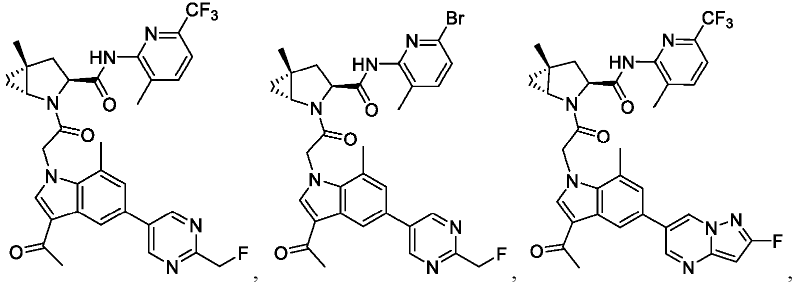 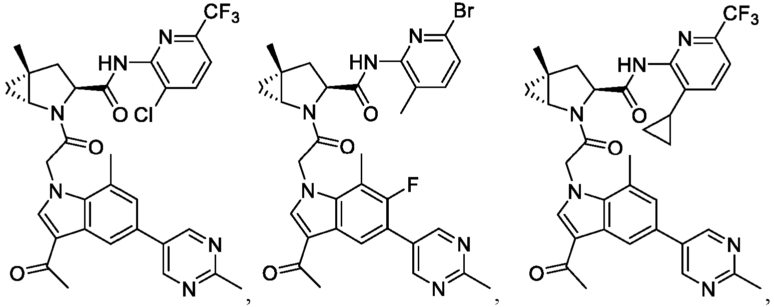 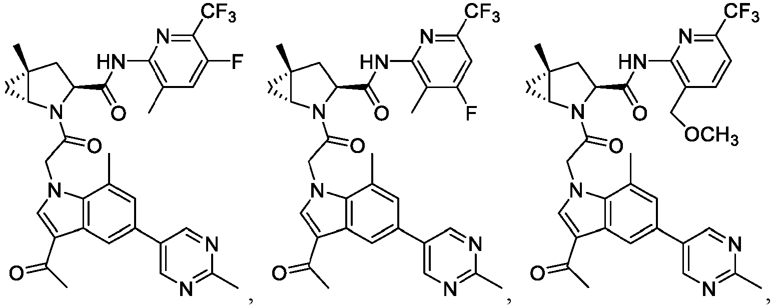 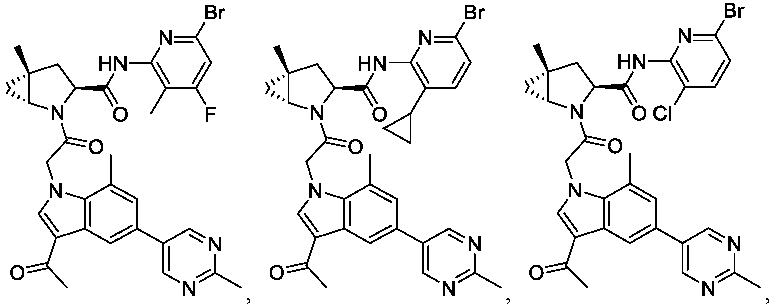 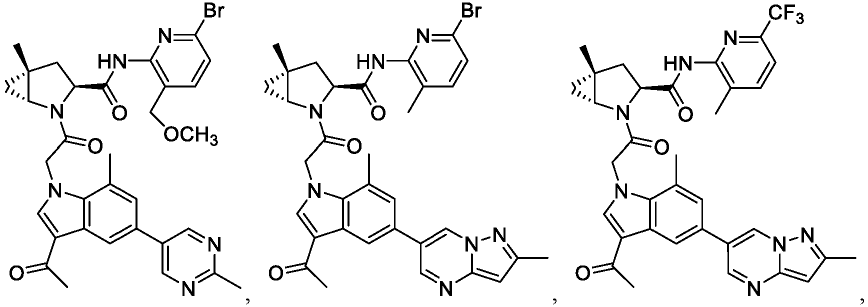 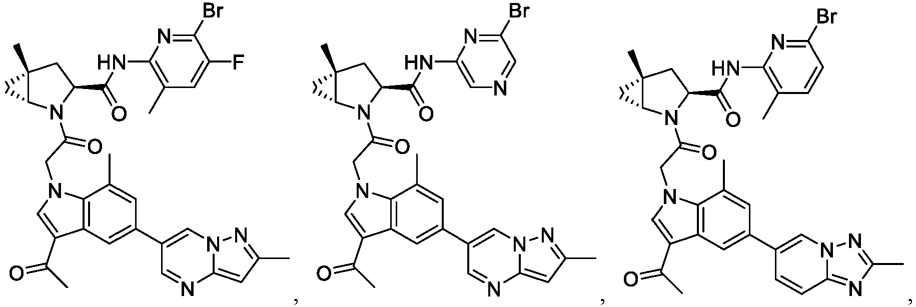 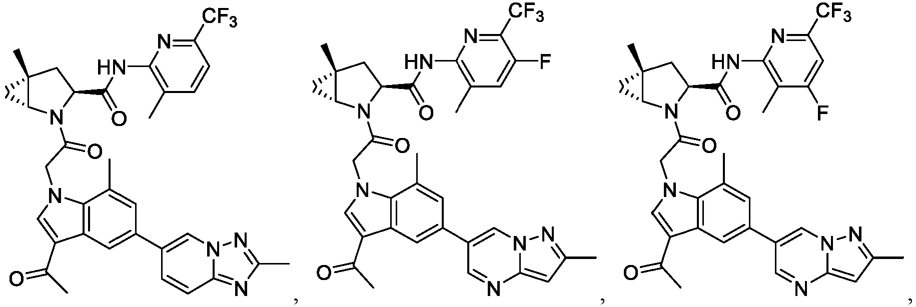 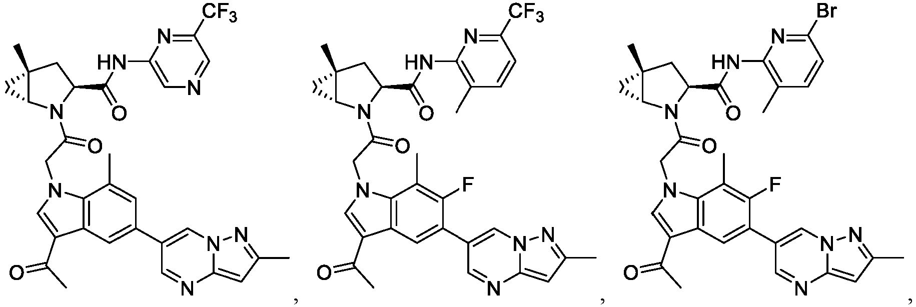 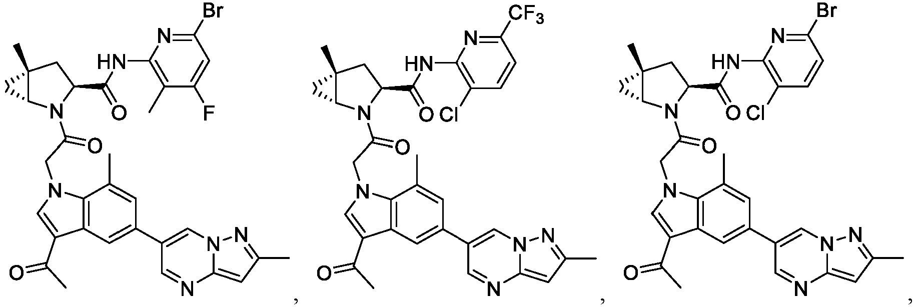 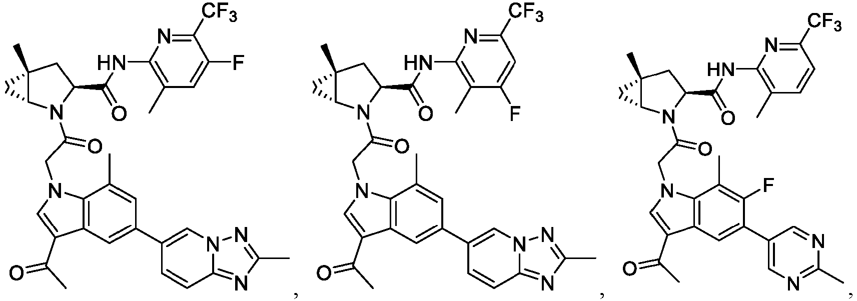 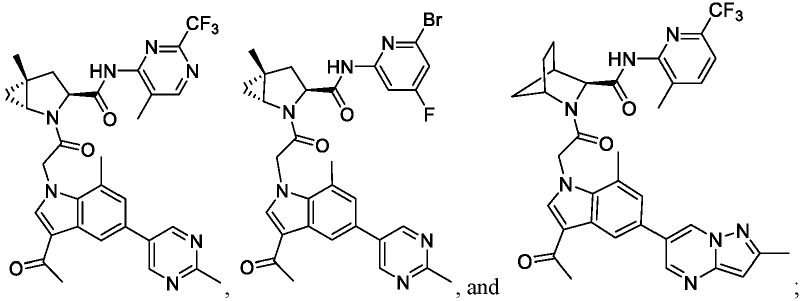 or a pharmaceutically acceptable salt thereof.

In one embodiment the compound is selected from: 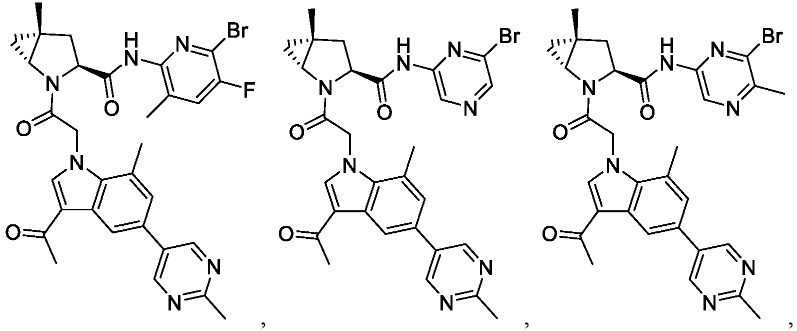 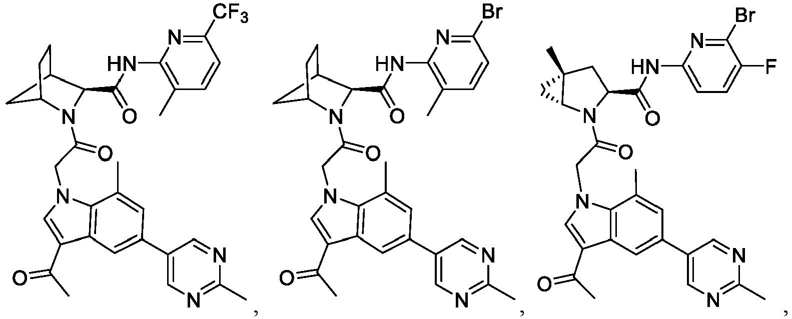 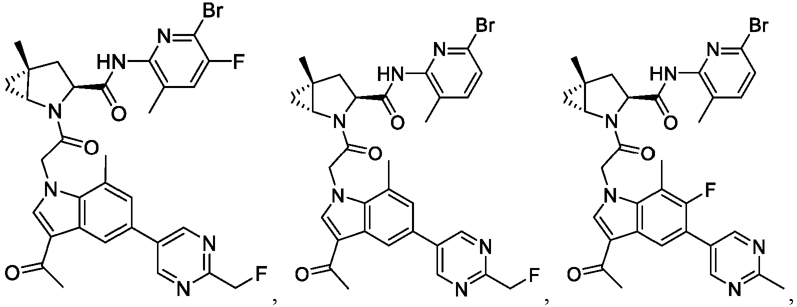 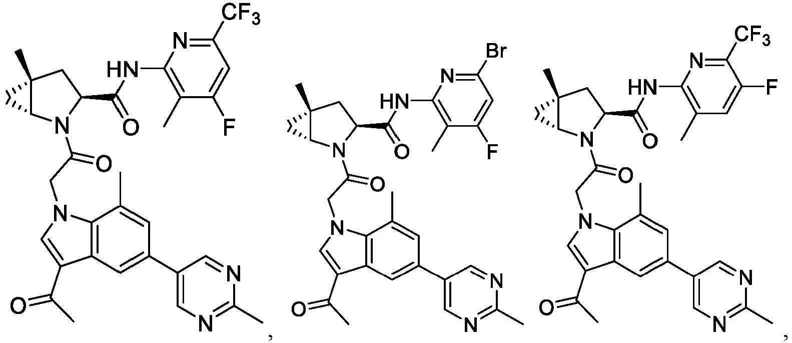 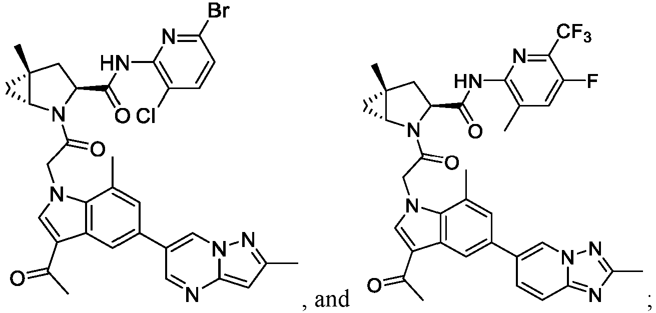 or a pharmaceutically acceptable salt thereof.

In certain embodiments the compound is selected from: 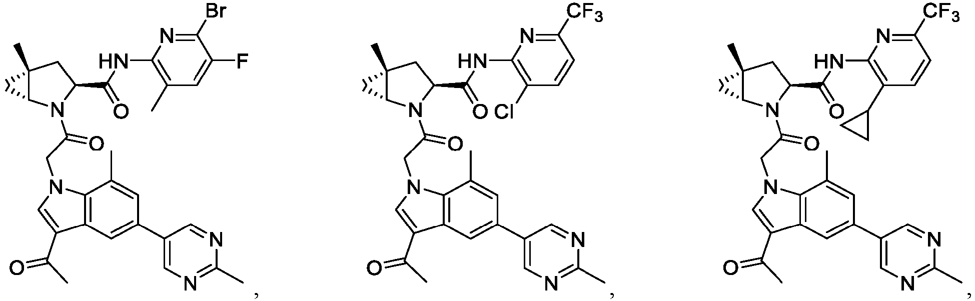 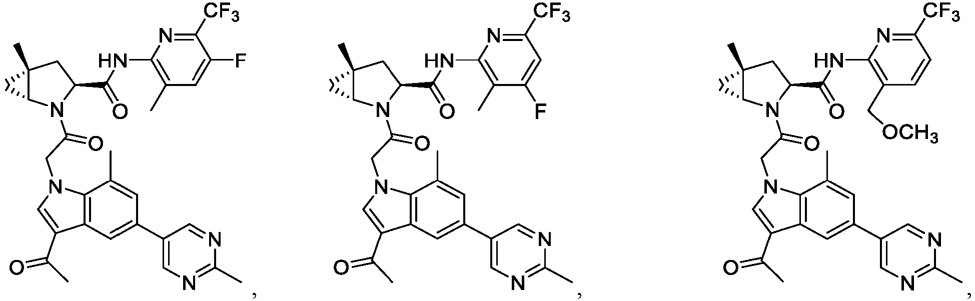 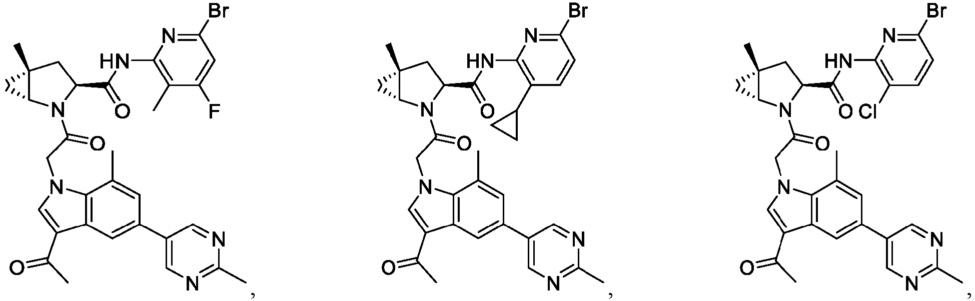 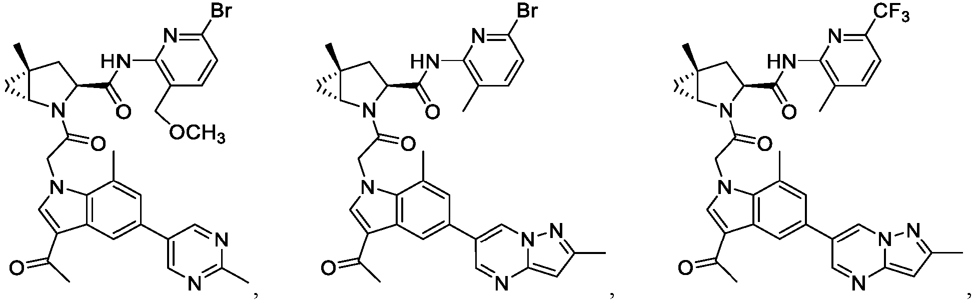 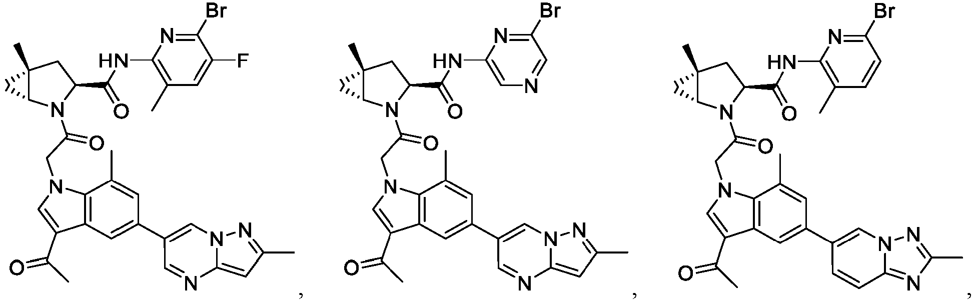 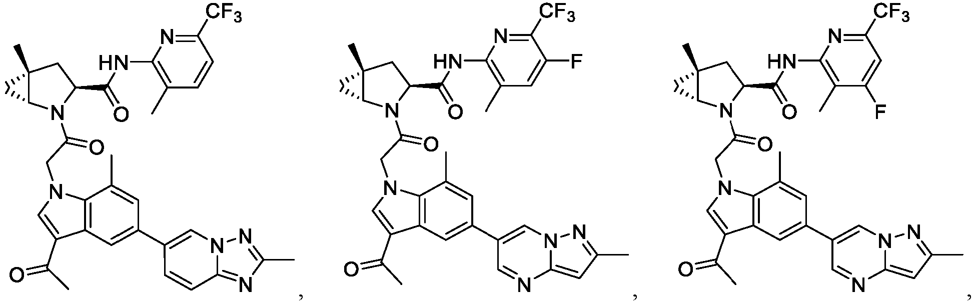 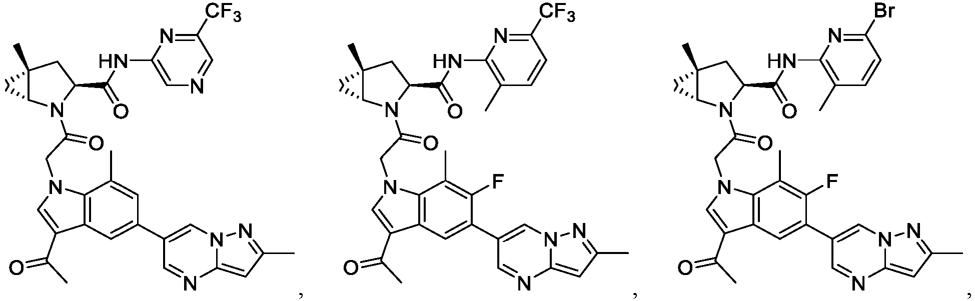 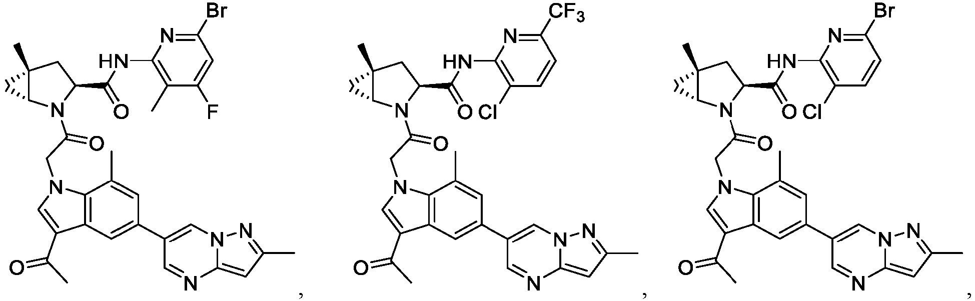 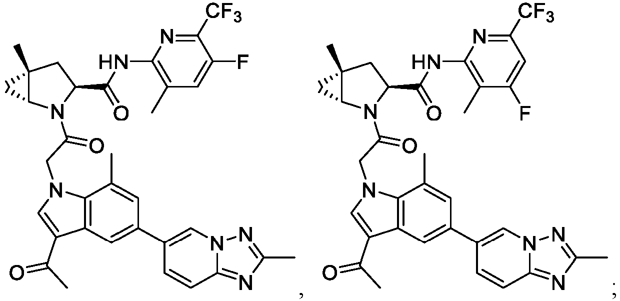 or a pharmaceutically acceptable salt thereof.

### PHARMACEUTICAL PREPARATIONS

Active compounds described herein can be administered to a host in need thereof as the neat chemical, but are more typically administered as a pharmaceutical composition that includes an effective amount for a host, typically a human, in need of such treatment of an active compound as described herein or its pharmaceutically acceptable salt thereof. Thus, in one embodiment, the disclosure provides pharmaceutical compositions comprising an effective amount of compound or pharmaceutically acceptable salt thereof together with at least one pharmaceutically acceptable carrier for any of the uses described herein. The pharmaceutical composition may contain a compound or salt as the only active agent, or, in an alternative embodiment, the compound and at least one additional active agent.

An effective amount of an active compound as described herein, or the active compound described herein in combination or alternation with, or preceded by, concomitant with or followed by another active agent, can be used in an amount sufficient to (a) inhibit the progression of a disorder mediated by the complement pathway, including an inflammatory, immune, including an autoimmune, disorder or complement Factor D related disorder; (b) cause a regression of an inflammatory, immune, including an autoimmune, disorder or complement Factor D related disorder; (c) cause a cure of an inflammatory, immune, including an autoimmune, disorder or complement Factor D related disorder; or inhibit or prevent the development of an inflammatory, immune, including an autoimmune, disorder or complement Factor D related disorder.

Accordingly, an effective amount of an active compound or its salt or composition described herein will provide a sufficient amount of the active agent when administered to a patient to provide a clinical benefit.

The exact amount of the active compound or pharmaceutical composition described herein to be delivered to the host, typically a human, in need thereof, will be determined by the health care provider to achieve the desired clinical benefit.

In certain embodiments the pharmaceutical composition is in a dosage form that contains from about 0.1 mg to about 2000 mg, from about 10 mg to about 1000 mg, from about 100 mg to about 800 mg, or from about 200 mg to about 600 mg of the active compound and optionally from about 0.1 mg to about 2000 mg, from about 10 mg to about 1000 mg, from about 100 mg to about 800 mg, or from about 200 mg to about 600 mg of an additional active agent in a unit dosage form. Examples are dosage forms with at least about 0.5, 1, 1.5, 2, 2.5, 3, 3.5, 4, 4.5, 5, 10, 15, 20, 25, 50, 75, 100, 150, 200, 250, 300, 350, 400, 450, 500, 550, 600, 650, 700, 750, 800, 900, 1000, 1100, 1200, 1250, 1300, 1400, 1500, or 1600 mg of active compound, or its salt. In one embodiment, the dosage form has at least about 1mg, 5 mg, 10 mg, 25 mg, 50 mg, 75 mg, 100 mg, 200 mg, 400 mg, 500 mg, 600 mg, 1000mg, 1200 mg, or 1600 mg of active compound or its salt. The amount of active compound in the dosage form is calculated without reference to the salt. The dosage form can be administered, for example, once a day (q.d.), twice a day (b.i.d.), three times a day (t.i.d.), four times a day (q.i.d.), once every other day (Q2d), once every third day (Q3d), as needed, or any dosage schedule that provides treatment of a disorder described herein.

Compounds disclosed herein or used as described herein may be administered orally, topically, parenterally, by inhalation or spray, sublingually, via implant, including ocular implant, transdermally, via buccal administration, rectally, as an ophthalmic solution, injection, including ocular injection, intravenous, intra-aortal, intracranial, subdermal, intraperitoneal, subcutaneous, transnasal, sublingual, intrathecal, or rectal or by other means, in dosage unit formulations containing conventional pharmaceutically acceptable carriers. For ocular delivery, the compound can be administered, as desired, for example, as a solution, suspension, or other formulation via intravitreal, intrastromal, intracameral, sub-tenon, sub-retinal, retro-bulbar, peribulbar, suprachorodial, subchorodial, chorodial, conjunctival, subconjunctival, episcleral, periocular, transscleral, retrobulbar, posterior juxtascleral, circumcorneal, or tear duct injections, or through a mucus, mucin, or a mucosal barrier, in an immediate or controlled release fashion or via an ocular device, injection, or topically administered formulation, for example a solution or suspension provided as an eye drop.

The pharmaceutical composition may be formulated as any pharmaceutically useful form, e.g., as an aerosol, a cream, a gel, a gel cap, a pill, a microparticle, a nanoparticle, an injection or infusion solution, a capsule, a tablet, a syrup, a transdermal patch, a subcutaneous patch, a dry powder, an inhalation formulation, in a medical device, suppository, buccal, or sublingual formulation, parenteral formulation, or an ophthalmic solution or suspension. Some dosage forms, such as tablets and capsules, are subdivided into suitably sized unit doses containing appropriate quantities of the active components, e.g., an effective amount to achieve the desired purpose.

Pharmaceutical compositions, and methods of manufacturing such compositions, suitable for administration as contemplated herein are known in the art. Examples of known techniques include, for example, US Patent Nos. 4,983,593, 5,013,557, 5,456,923, 5,576,025, 5,723,269, 5,858,411, 6,254,889, 6,303,148, 6,395,302, 6,497,903, 7,060,296, 7,078,057, 7,404,828, 8,202,912, 8,257,741, 8,263,128, 8,337,899, 8,431,159, 9,028,870, 9,060,938, 9,211,261, 9,265,731, 9,358,478, and 9,387,252.

The pharmaceutical compositions contemplated here can optionally include a carrier. Carriers must be of sufficiently high purity and sufficiently low toxicity to render them suitable for administration to the patient being treated. The carrier can be inert or it can possess pharmaceutical benefits of its own. The amount of carrier employed in conjunction with the compound is sufficient to provide a practical quantity of material for administration per unit dose of the compound. Classes of carriers include, but are not limited to binders, buffering agents, coloring agents, diluents, disintegrants, emulsifiers, fillers, flavorants, glidents, lubricants, pH modifiers, preservatives, stabilizers, surfactants, solubilizers, tableting agents, and wetting agents. Some carriers may be listed in more than one class, for example vegetable oil may be used as a lubricant in some formulations and a diluent in others. Exemplary pharmaceutically acceptable carriers include sugars, starches, celluloses, powdered tragacanth, malt, gelatin; talc, and vegetable oils. Examples of other matrix materials, fillers, or diluents include lactose, mannitol, xylitol, microcrystalline cellulose, calcium diphosphate, and starch. Examples of surface active agents include sodium lauryl sulfate and polysorbate 80. Examples of drug complexing agents or solubilizers include the polyethylene glycols, caffeine, xanthene, gentisic acid and cylodextrins. Examples of disintegrants include sodium starch gycolate, sodium alginate, carboxymethyl cellulose sodium, methyl cellulose, colloidal silicon dioxide, and croscarmellose sodium. Examples of binders include methyl cellulose, microcrystalline cellulose, starch, and gums such as guar gum, and tragacanth. Examples of lubricants include magnesium stearate and calcium stearate. Examples of pH modifiers include acids such as citric acid, acetic acid, ascorbic acid, lactic acid, aspartic acid, succinic acid, phosphoric acid, and the like; bases such as sodium acetate, potassium acetate, calcium oxide, magnesium oxide, trisodium phosphate, sodium hydroxide, calcium hydroxide, aluminum hydroxide, and the like, and buffers generally comprising mixtures of acids and the salts of said acids. Optional other active agents may be included in a pharmaceutical composition, which do not substantially interfere with the activity of the compound of the present invention.

In certain embodiments, the pharmaceutical composition for administration further includes a compound or salt as described herein and optionally comprises one or more of a phosphoglyceride; phosphatidylcholine; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohol such as polyethylene glycol (PEG); polyoxyethylene-9-lauryl ether; a surface active fatty acid, such as palmitic acid or oleic acid; fatty acid; fatty acid monoglyceride; fatty acid diglyceride; fatty acid amide; sorbitan trioleate (Span^{®}85) glycocholate; sorbitan monolaurate (Span^{®}20); polysorbate 20 (Tween^{®}20); polysorbate 60 (Tween^{®}60); polysorbate 65 (Tween^{®}65); polysorbate 80 (Tween^{®}80); polysorbate 85 (Tween^{®}85); polyoxyethylene monostearate; surfactin; a poloxomer; a sorbitan fatty acid ester such as sorbitan trioleate; lecithin; lysolecithin; phosphatidylserine; phosphatidylinositol; sphingomyelin; phosphatidylethanolamine (cephalin); cardiolipin; phosphatidic acid; cerebroside; dicetylphosphate; dipalmitoylphosphatidylglycerol; stearylamine; dodecylamine; hexadecyl-amine; acetyl palmitate; glycerol ricinoleate; hexadecyl sterate; isopropyl myristate; tyloxapol; poly(ethylene glycol)5000-phosphatidylethanolamine; poly(ethylene glycol)400-monostearate; phospholipid; synthetic and/or natural detergent having high surfactant properties; deoxycholate; cyclodextrin; chaotropic salt; ion pairing agent; glucose, fructose, galactose, ribose, lactose, sucrose, maltose, trehalose, cellbiose, mannose, xylose, arabinose, glucoronic acid, galactoronic acid, mannuronic acid, glucosamine, galatosamine, and neuramic acid; pullulan, cellulose, microcrystalline cellulose, hydroxypropyl methylcellulose (HPMC), hydroxycellulose (HC), methylcellulose (MC), dextran, cyclodextran, glycogen, hydroxyethylstarch, carageenan, glycon, amylose, chitosan, N,O-carboxylmethylchitosan, algin and alginic acid, starch, chitin, inulin, konjac, glucommannan, pustulan, heparin, hyaluronic acid, curdlan, and xanthan, mannitol, sorbitol, xylitol, erythritol, maltitol, and lactitol, a pluronic polymer, polyethylene, polycarbonate (e.g. poly(1,3-dioxan-2one)), polyanhydride (e.g. poly(sebacic anhydride)), polypropylfumerate, polyamide (e.g. polycaprolactam), polyacetal, polyether, polyester (e.g., polylactide, polyglycolide, polylactide-co-glycolide, polycaprolactone, polyhydroxyacid (e.g. poly((β-hydroxyalkanoate))), poly(orthoester), polycyanoacrylate, polyvinyl alcohol, polyurethane, polyphosphazene, polyacrylate, polymethacrylate, polyurea, polystyrene, and polyamine, polylysine, polylysine-PEG copolymer, and poly(ethyleneimine), poly(ethylene imine)-PEG copolymer, glycerol monocaprylocaprate, propylene glycol, Vitamin E TPGS (also known as d-α-Tocopheryl polyethylene glycol 1000 succinate), gelatin, titanium dioxide, polyvinylpyrrolidone (PVP), hydroxypropyl methyl cellulose (HPMC), hydroxypropyl cellulose (HPC), methyl cellulose (MC), block copolymers of ethylene oxide and propylene oxide (PEO/PPO), polyethyleneglycol (PEG), sodium carboxymethylcellulose (NaCMC), hydroxypropylmethyl cellulose acetate succinate (HPMCAS).

In some embodiments, the pharmaceutical preparation may include polymers for controlled delivery of the described compounds, including, but not limited to pluronic polymers, polyesters (e.g., polylactic acid, poly(lactic-co-glycolic acid), polycaprolactone, polyvalerolactone, poly(1,3-dioxan-2one)); polyanhydrides (e.g., poly(sebacic anhydride)); polyethers (e.g., polyethylene glycol); polyurethanes; polymethacrylates; polyacrylates; and polycyanoacrylates. In some embodiments, polymers may be modified with polyethylene glycol (PEG), with a carbohydrate, and/or with acyclic polyacetals derived from polysaccharides. See, e.g., Papisov, 2001, ACS Symposium Series, 786:301.

The compounds of the present invention can be formulated as particles. In one embodiment the particles are or include microparticles. In an alternative embodiment the particles are or include nanoparticles.

In an additional alternative embodiment, common techniques for preparing particles include, but are not limited to, solvent evaporation, solvent removal, spray drying, phase inversion, coacervation, and low temperature casting. Suitable methods of particle formulation are briefly described below. Pharmaceutically acceptable excipients, including pH modifying agents, disintegrants, preservatives, and antioxidants, can optionally be incorporated into the particles during particle formation.

In one embodiment, the particles are derived through a solvent evaporation method. In this method, a compound described herein (or polymer matrix and one or more compounds described herein) is dissolved in a volatile organic solvent, such as methylene chloride. The organic solution containing a compound described herein is then suspended in an aqueous solution that contains a surface active agent such as poly(vinyl alcohol). The resulting emulsion is stirred until most of the organic solvent evaporated, leaving solid nanoparticles or microparticles. The resulting nanoparticles or microparticles are washed with water and dried overnight in a lyophilizer. Nanoparticles with different sizes and morphologies can be obtained by this method.

Pharmaceutical compositions which contain labile polymers, such as certain polyanhydrides, may degrade during the fabrication process due to the presence of water. For these polymers, methods which are performed in completely or substantially anhydrous organic solvents can be used to make the particles.

Solvent removal can also be used to prepare particles from a compound that is hydrolytically unstable. In this method, the compound (or polymer matrix and one or more compounds) is dispersed or dissolved in a volatile organic solvent such as methylene chloride. This mixture is then suspended by stirring in an organic oil (such as silicon oil) to form an emulsion. Solid particles form from the emulsion, which can subsequently be isolated from the supernatant. The external morphology of spheres produced with this technique is highly dependent on the identity of the drug.

In one embodiment an active compound as described herein is administered to a patient in need thereof as particles formed by solvent removal. In another embodiment the present invention provides particles formed by solvent removal comprising a compound of the present invention and one or more pharmaceutically acceptable excipients as defined herein. In another embodiment the particles formed by solvent removal comprise a compound of the present invention and an additional therapeutic agent. In a further embodiment the particles formed by solvent removal comprise a compound of the present invention, an additional therapeutic agent, and one or more pharmaceutically acceptable excipients. In another embodiment any of the described particles formed by solvent removal can be formulated into a tablet and then coated to form a coated tablet. In an alternative embodiment the particles formed by solvent removal are formulated into a tablet but the tablet is uncoated.

In one embodiment, the particles are derived by spray drying. In this method, a compound (or polymer matrix and one or more compounds) is dissolved in an organic solvent such as methylene chloride. The solution is pumped through a micronizing nozzle driven by a flow of compressed gas, and the resulting aerosol is suspended in a heated cyclone of air, allowing the solvent to evaporate from the micro droplets, forming particles. Microparticles and nanoparticles can be obtained using this method.

In one embodiment an active compound as described herein is administered to a patient in need thereof as a spray dried dispersion (SDD). In another embodiment the present invention provides a spray dried dispersion (SDD) comprising a compound of the present invention and one or more pharmaceutically acceptable excipients as defined herein. In another embodiment the SDD comprises a compound of the present invention and an additional therapeutic agent. In a further embodiment the SDD comprises a compound of the present invention, an additional therapeutic agent, and one or more pharmaceutically acceptable excipients. In another embodiment any of the described spray dried dispersions can be coated to form a coated tablet. In an alternative embodiment the spray dried dispersion is formulated into a tablet but is uncoated. Particles can be formed from the active compound as described herein using a phase inversion method. In this method, the compound (or polymer matrix and one or more active compounds) is dissolved in a suitable solvent, and the solution is poured into a strong non-solvent for the compound to spontaneously produce, under favorable conditions, microparticles or nanoparticles. The method can be used to produce nanoparticles in a wide range of sizes, including, for example, from nanoparticles to microparticles, typically possessing a narrow particle size distribution.

In one embodiment, an active compound as described herein is administered to a patient in need thereof as particles formed by phase inversion. In another embodiment the present invention provides particles formed by phase inversion comprising a compound of the present invention and one or more pharmaceutically acceptable excipients as defined herein. In another embodiment the particles formed by phase inversion comprise a compound of the present invention and an additional therapeutic agent. In a further embodiment the particles formed by phase inversion comprise a compound of the present invention, an additional therapeutic agent, and one or more pharmaceutically acceptable excipients. In another embodiment any of the described particles formed by phase inversion can be formulated into a tablet and then coated to form a coated tablet. In an alternative embodiment the particles formed by phase inversion are formulated into a tablet but the tablet is uncoated.

Techniques for particle formation using coacervation are known in the art, for example, as described in GB-B-929 406; GB-B-929 40 1; and U.S. Patent Nos. 3,266,987, 4,794,000, and 4,460,563. Coacervation involves the separation of a compound (or polymer matrix and one or more compounds) solution into two immiscible liquid phases. One phase is a dense coacervate phase, which contains a high concentration of the compound, while the second phase contains a low concentration of the compound. Within the dense coacervate phase, the compound forms nanoscale or microscale droplets, which harden into particles. Coacervation may be induced by a temperature change, addition of a non-solvent or addition of a micro-salt (simple coacervation), or by the addition of another polymer thereby forming an interpolymer complex (complex coacervation).

In one embodiment an active compound as described herein is administered to a patient in need thereof as particles formed by coacervation. In another embodiment the present invention provides particles formed by coacervation comprising a compound of the present invention and one or more pharmaceutically acceptable excipients as defined herein. In another embodiment the particles formed by coacervation comprise a compound of the present invention and an additional therapeutic agent. In a further embodiment the particles formed by coacervation comprise a compound of the present invention, an additional therapeutic agent, and one or more pharmaceutically acceptable excipients. In another embodiment any of the described particles formed by coacervation can be formulated into a tablet and then coated to form a coated tablet. In an alternative embodiment the particles formed by coacervation are formulated into a tablet but the tablet is uncoated.

Methods for very low temperature casting of controlled release microspheres are described in U.S. Patent No. 5,019,400 to Gombotz et al. In this method, the compound is dissolved in a solvent. The mixture is then atomized into a vessel containing a liquid non-solvent at a temperature below the freezing point of the drug solution which freezes the compound droplets. As the droplets and non-solvent for the compound are warmed, the solvent in the droplets thaws and is extracted into the non-solvent, hardening the microspheres.

In one embodiment, a compound of the present invention is administered to a patient in need thereof as particles formed by low temperature casting. In another embodiment the present invention provides particles formed by low temperature casting comprising a compound of the present invention and one or more pharmaceutically acceptable excipients as defined herein. In another embodiment the particles formed by low temperature casting comprise a compound of the present invention and an additional therapeutic agent. In a further embodiment the particles formed by low temperature casting comprise a compound of the present invention, an additional therapeutic agent, and one or more pharmaceutically acceptable excipients. In another embodiment any of the described particles formed by low temperature casting can be formulated into a tablet and then coated to form a coated tablet. In an alternative embodiment the particles formed by low temperature casting are formulated into a tablet but the tablet is uncoated.

In one aspect of the present invention, an effective amount of an active compound as described herein is incorporated into a nanoparticle, *e.g*. for convenience of delivery and/or extended release delivery. The use of materials in nanoscale provides one the ability to modify fundamental physical properties such as solubility, diffusivity, blood circulation half-life, drug release characteristics, and/or immunogenicity. A number of nanoparticle-based therapeutic and diagnostic agents have been developed for the treatment of cancer, diabetes, pain, asthma, allergy, and infections. These nanoscale agents may provide more effective and/or more convenient routes of administration, lower therapeutic toxicity, extend the product life cycle, and ultimately reduce health-care costs. As therapeutic delivery systems, nanoparticles can allow targeted delivery and controlled release.

In addition, nanoparticle-based compound delivery can be used to release compounds at a sustained rate and thus lower the frequency of administration, deliver drugs in a targeted manner to minimize systemic side effects, or deliver two or more drugs simultaneously for combination therapy to generate a synergistic effect and suppress drug resistance. A number of nanotechnology-based therapeutic products have been approved for clinical use. Among these products, liposomal drugs and polymer-based conjugates account for a large proportion of the products. *See,* Zhang, L., et al., Nanoparticles in Medicine: Therapeutic Applications and Developments, Clin. Pharm. and Ther., 83(5):761-769, 2008.

Methods for producing nanoparticles are known in the art. For example, see Muller, R.H., et al., Solid lipid nanoparticles (SLN) for controlled drug delivery - a review of the state of the art, Eur. H. Pharm. Biopharm., 50:161-177, 2000; US 8,691,750 to Consien et al.; WO 2012/145801 to Kanwar. US 8,580,311 to Armes, S. et al.; Petros, R.A. and DeSimone, J.M., Strategies in the design of nanoparticles for therapeutic applications, Nature Reviews/Drug Discovery, vol. 9:615-627, 2010; US 8,465,775; US 8,444,899; US 8,420,124; US 8,263,129; US 8,158,728; 8,268,446; Pellegrino et al., 2005, Small, 1:48; Murray et al., 2000, Ann. Rev. Mat. Sci., 30:545; and Trindade et al., 2001, Chem. Mat., 13:3843. Additional methods have been described in the literature (see, e.g., Doubrow, Ed., "Microcapsules and Nanoparticles in Medicine and Pharmacy," CRC Press, Boca Raton, 1992; Mathiowitz et al., 1987, J. Control. Release, 5:13; Mathiowitz et al., 1987, Reactive Polymers, 6:275; and Mathiowitz et al., 1988, J. Appl. Polymer Sci., 35:755; U.S. Pat. Nos. 5,578,325 and 6,007,845; P. Paolicelli et al., "Surface-modified PLGA-based Nanoparticles that can Efficiently Associate and Deliver Virus-like Particles" Nanomedicine. 5(6):843-853 (2010)), U.S. Pat. No. 5,543,158 to Gref et al., or WO publication WO2009/051837 by Von Andrian et al. Zauner et al., 1998, Adv. Drug Del. Rev., 30:97; and Kabanov et al., 1995, Bioconjugate Chem., 6:7;(PEI; Boussif et al., 1995, Proc. Natl. Acad. Sci., USA, 1995, 92:7297), and poly(amidoamine) dendrimers (Kukowska-Latallo et al., 1996, Proc. Natl. Acad. Sci., USA, 93:4897; Tang et al., 1996, Bioconjugate Chem., 7:703; and Haensler et al., 1993, Bioconjugate Chem., 4:372; Putnam et al., 1999, Macromolecules, 32:3658; Barrera et al., 1993, J. Am. Chem. Soc., 115:11010; Kwon et al., 1989, Macromolecules, 22:3250; Lim et al., 1999, J. Am. Chem. Soc., 121:5633; and Zhou et al., 1990, Macromolecules, 23:3399). Examples of these polyesters include poly(L-lactide-co-L-lysine) (Barrera et al., 1993, J. Am. Chem. Soc., 115:11010), poly(serine ester) (Zhou et al., 1990, Macromolecules, 23:3399), poly(4-hydroxy-L-proline ester) (Putnam et al., 1999, Macromolecules, 32:3658; and Lim et al., 1999, J. Am. Chem. Soc., 121:5633), and poly(4-hydroxy-L-proline ester) (Putnam et al., 1999, Macromolecules, 32:3658; and Lim et al., 1999, J. Am. Chem. Soc., 121:5633; U.S. Pat. No. 6,123,727; U.S. Pat. No. 5,804,178; U.S. Pat. No. 5,770,417; U.S. Pat. No. 5,736,372; U.S. Pat. No. 5,716,404; U.S. Pat. No. 6,095,148; U.S. Pat. No. 5,837,752; U.S. Pat. No. 5,902,599; U.S. Pat. No. 5,696,175; U.S. Pat. No. 5,514,378; U.S. Pat. No. 5,512,600; U.S. Pat. No. 5,399,665; U.S. Pat. No. 5,019,379; U.S. Pat. No. 5,010,167; U.S. Pat. No. 4,806,621; U.S. Pat. No. 4,638,045; and U.S. Pat. No. 4,946,929; Wang et al., 2001, J. Am. Chem. Soc., 123:9480; Lim et al., 2001, J. Am. Chem. Soc., 123:2460; Langer, 2000, Acc. Chem. Res., 33:94; Langer, 1999, J. Control. Release, 62:7; and Uhrich et al., 1999, Chem. Rev., 99:3181; Concise Encyclopedia of Polymer Science and Polymeric Amines and Ammonium Salts, Ed. by Goethals, Pergamon Press, 1980; Principles of Polymerization by Odian, John Wiley & Sons, Fourth Edition, 2004; Contemporary Polymer Chemistry by Allcock et al., Prentice-Hall, 1981; Deming et al., 1997, Nature, 390:386; and in U.S. Pat. Nos. 6,506,577, 6,632,922, 6,686,446, and 6,818,732; C. Astete et al., "Synthesis and characterization of PLGA nanoparticles" J. Biomater. Sci. Polymer Edn, Vol. 17, No. 3, pp. 247-289 (2006); K. Avgoustakis "Pegylated Poly(Lactide) and Poly(Lactide-Co-Glycolide) Nanoparticles: Preparation, Properties and Possible Applications in Drug Delivery" Current Drug Delivery 1:321-333 (2004); C. Reis et al., "Nanoencapsulation I. Methods for preparation of drug-loaded polymeric nanoparticles" Nanomedicine 2:8-21 (2006); P. Paolicelli et al., "Surface-modified PLGA-based Nanoparticles that can Efficiently Associate and Deliver Virus-like Particles" Nanomedicine. 5(6):843-853 (2010); U.S. Pat. No. 6,632,671 to Unger Oct. 14, 2003.

In one embodiment, the polymeric particle is between about 0.1 nm to about 10000 nm, between about 1 nm to about 1000 nm, between about 10 nm and 1000 nm, between about 1 and 100 nm, between about 1 and 10 nm, between about 1 and 50 nm, between about 100 nm and 800 nm, between about 400 nm and 600 nm, or about 500 nm. In one embodiment, the micro-particles are no more than about 0.1 nm, 0.5 nm, 1.0 nm, 5.0 nm, 10 nm, 25 nm, 50 nm, 75 nm, 100 nm, 150 nm, 200 nm, 250 nm, 300 nm, 400 nm, 450 nm, 500 nm, 550 nm, 600 nm, 650 nm, 700 nm, 750 nm, 800 nm, 850 nm, 900 nm, 950 nm, 1000 nm, 1250 nm, 1500 nm, 1750 nm, or 2000 nm. In some embodiments, a compound described herein may be covalently coupled to a polymer used in the nanoparticle, for example a polystyrene particle, PLGA particle, PLA particle, or other nanoparticle.

The pharmaceutical compositions can be formulated for oral administration. These compositions can contain any amount of active compound that achieves the desired result, for example between 0.1 and 99 weight % (wt.%) of the compound and usually at least about 5 wt.% of the compound. Some embodiments contain at least about 10%, 15%, 20%, 25 wt.% to about 50 wt. % or from about 5 wt.% to about 75 wt.% of the compound.

Pharmaceutical compositions suitable for rectal administration are typically presented as unit dose suppositories. These may be prepared by admixing the active compound with one or more conventional solid carriers, for example, cocoa butter, and then shaping the resulting mixture.

Pharmaceutical compositions suitable for topical application to the skin preferably take the form of an ointment, cream, lotion, paste, gel, spray, aerosol, or oil. Carriers which may be used include petroleum jelly, lanoline, polyethylene glycols, alcohols, transdermal enhancers, and combinations of two or more thereof.

Pharmaceutical compositions suitable for transdermal administration may be presented as discrete patches adapted to remain in intimate contact with the epidermis of the recipient for a prolonged period of time. Pharmaceutical compositions suitable for transdermal administration may also be delivered by iontophoresis (*see,* for example, Pharmaceutical Research 3 (6):318 (1986)) and typically take the form of an optionally buffered aqueous solution of the active compound. In one embodiment, microneedle patches or devices are provided for delivery of drugs across or into biological tissue, particularly the skin. The microneedle patches or devices permit drug delivery at clinically relevant rates across or into skin or other tissue barriers, with minimal or no damage, pain, or irritation to the tissue.

Pharmaceutical compositions suitable for administration to the lungs can be delivered by a wide range of passive breath driven and active power driven single/-multiple dose dry powder inhalers (**DPI**). The devices most commonly used for respiratory delivery include nebulizers, metered-dose inhalers, and dry powder inhalers. Several types of nebulizers are available, including jet nebulizers, ultrasonic nebulizers, and vibrating mesh nebulizers. Selection of a suitable lung delivery device depends on parameters, such as nature of the drug and its formulation, the site of action, and pathophysiology of the lung.

Additional non-limiting examples of inhalation drug delivery devices and methods include, for example, US 7,383,837 titled "Inhalation device" (SmithKline Beecham Corporation); WO/2006/033584 titled "Powder inhaler" (Glaxo SmithKline Pharmaceuticals SA); WO/2005/044186 titled "Inhalable pharmaceutical formulations employing desiccating agents and methods of administering the same" (Glaxo Group Ltd and SmithKline Beecham Corporation); US9,095,670 titled "Inhalation device and method of dispensing medicament", US 8,205,611 titled "Dry powder inhaler" (Astrazeneca AB); WO/2013/038170 titled "Inhaler" (Astrazeneca AB and Astrazeneca UK Ltd.); US/2014/0352690 titled "Inhalation Device with Feedback System", US 8,910,625 and US/2015/0165137 titled "Inhalation Device for Use in Aerosol Therapy" (Vectura GmbH); US 6,948,496 titled "Inhalers", US/2005/0152849 titled "Powders comprising anti-adherent materials for use in dry powder inhalers", US 6,582,678, US 8,137,657, US/2003/0202944, and US/2010/0330188 titled "Carrier particles for use in dry powder inhalers", US 6,221,338 titled "Method of producing particles for use in dry powder inhalers", US 6,989,155 titled "Powders", US/2007/0043030 titled "Pharmaceutical compositions for treating premature ejaculation by pulmonary inhalation", US 7,845,349 titled "Inhaler", US/2012/0114709 and US 8,101,160 titled "Formulations for Use in Inhaler Devices", US/2013/0287854 titled "Compositions and Uses", US/2014/0037737 and US 8,580,306 titled "Particles for Use in a Pharmaceutical Composition", US/2015/0174343 titled "Mixing Channel for an Inhalation Device", US 7,744,855 and US/2010/0285142 titled "Method of making particles for use in a pharmaceutical composition", US 7,541,022, US/2009/0269412, and US/2015/0050350 titled "Pharmaceutical formulations for dry powder inhalers" (Vectura Limited).

Many methods and devices for drug delivery to the eye are known in the art. Non-limiting examples are described in the following patents and patent applications. Examples are US 8,192,408 titled "Ocular trocar assembly" (Psivida Us, Inc.); US 7,585,517 titled "Transcleral delivery" (Macusight, Inc.); US 5,710,182 and US 5,795,913 titled "Ophthalmic composition" (Santen OY); US 8,663,639 titled "Formulations for treating ocular diseases and conditions", US 8,486,960 titled "Formulations and methods for vascular permeability-related diseases or conditions", US 8,367,097 and US 8,927,005 titled "Liquid formulations for treatment of diseases or conditions", US 7,455,855 titled "Delivering substance and drug delivery system using the same" (Santen Pharmaceutical Co., Ltd.); WO/2011/050365 titled "Conformable Therapeutic Shield For Vision and Pain" and WO/2009/145842 titled "Therapeutic Device for Pain Management and Vision" (Forsight Labs, LLC); US 9,066,779 and US 8,623,395 titled "Implantable therapeutic device", WO/2014/160884 titled "Ophthalmic Implant for Delivering Therapeutic Substances", US 8,399,006, US 8,277,830, US 8,795,712, US 8,808,727, US 8,298,578, and WO/2010/088548 titled "Posterior segment drug delivery", WO/2014/152959 and US20140276482 titled "Systems for Sustained Intraocular Delivery of Low Solubility Compounds from a Port Delivery System Implant", US 8,905,963 and US 9,033,911 titled "Injector apparatus and method for drug delivery", WO/2015/057554 titled "Formulations and Methods for Increasing or Reducing Mucus", US 8,715,712 and US 8,939,948 titled "Ocular insert apparatus and methods", WO/2013/116061 titled "Insertion and Removal Methods and Apparatus for Therapeutic Devices", WO/2014/066775 titled "Ophthalmic System for Sustained Release of Drug to the Eye", WO/2015/085234 and WO/2012/019176 titled "Implantable Therapeutic Device", WO/2012/065006 titled "Methods and Apparatus to determine Porous Structures for Drug Delivery", WO/2010/141729 titled "Anterior Segment Drug Delivery", WO/2011/050327 titled "Corneal Denervation for Treatment of Ocular Pain", WO/2013/022801 titled "Small Molecule Delivery with Implantable Therapeutic Device", WO/2012/019047 titled "Subconjunctival Implant for Posterior Segment Drug Delivery", WO/2012/068549 titled "Therapeutic Agent Formulations for Implanted Devices", WO/2012/019139 titled " Combined Delivery Methods and Apparatus", WO/2013/040426 titled "Ocular Insert Apparatus and Methods", WO/2012/019136 titled "Injector Apparatus and Method for Drug Delivery", WO/2013/040247 titled "Fluid Exchange Apparatus and Methods" (ForSight Vision4, Inc.).

Additional non-limiting examples of how to deliver the active compounds are provided in WO/2015/085251 titled "Intracameral Implant for Treatment of an Ocular Condition" (Envisia Therapeutics, Inc.); WO/2011/008737 titled "Engineered Aerosol Particles, and Associated Methods", WO/2013/082111 titled "Geometrically Engineered Particles and Methods for Modulating Macrophage or Immune Responses", WO/2009/132265 titled "Degradable compounds and methods of use thereof, particularly with particle replication in non-wetting templates", WO/2010/099321 titled "Interventional drug delivery system and associated methods", WO/2008/100304 titled "Polymer particle composite having high fidelity order, size, and shape particles", WO/2007/024323 titled "Nanoparticle fabrication methods, systems, and materials" (Liquidia Technologies, Inc. and the University of North Carolina at Chapel Hill); WO/2010/009087 titled "Iontophoretic Delivery of a Controlled-Release Formulation in the Eye", (Liquidia Technologies, Inc. and Eyegate Pharmaceuticals, Inc.) and WO/2009/132206 titled "Compositions and Methods for Intracellular Delivery and Release of Cargo", WO/2007/133808 titled "Nano-particles for cosmetic applications", WO/2007/056561 titled "Medical device, materials, and methods", WO/2010/065748 titled "Method for producing patterned materials", WO/2007/081876 titled "Nanostructured surfaces for biomedical/biomaterial applications and processes thereof' (Liquidia Technologies, Inc.).

Additional non-limiting examples of methods and devices for drug delivery to the eye include, for example, WO2011/106702 and US 8,889,193 titled "Sustained delivery of therapeutic agents to an eye compartment", WO2013/138343 and US 8,962,577 titled "Controlled release formulations for the delivery of HIF-1 inhibitors", WO/2013/138346 and US2013/0272994 titled "Non-Linear Multiblock Copolymer-Drug Conjugates for the Delivery of Active Agents", WO2005/072710 and US 8,957,034 titled "Drug and Gene Carrier Particles that Rapidly Move Through Mucus Barriers", WO2008/030557, US2010/0215580, US2013/0164343 titled "Compositions and Methods for Enhancing Transport Through Mucous", WO2012/061703, US2012/0121718, and US2013/0236556 titled "Compositions and Methods Relating to Reduced Mucoadhesion", WO2012/039979 and US2013/0183244 titled "Rapid Diffusion of Large Polymeric Nanoparticles in the Mammalian Brain", WO2012/109363 and US2013/0323313 titled "Mucus Penetrating Gene Carriers", WO 2013/090804 and US2014/0329913 titled "Nanoparticles with enhanced mucosal penetration or decreased inflammation", WO2013/110028 titled "Nanoparticle formulations with enhanced mucosal penetration", WO2013/166498 and US2015/0086484 titled "Lipid-based drug carriers for rapid penetration through mucus linings" (The Johns Hopkins University); WO2013/166385 titled "Pharmaceutical Nanoparticles Showing Improved Mucosal Transport", US2013/0323179 titled "Nanocrystals, Compositions, And Methods that Aid Particle Transport in Mucus" (The Johns Hopkins University and Kala Pharmaceuticals, Inc.); WO/2015/066444 titled "Compositions and methods for ophthalmic and/or other applications", WO/2014/020210 and WO/2013/166408 titled "Pharmaceutical nanoparticles showing improved mucosal transport" (Kala Pharmaceuticals, Inc.); US 9,022,970 titled "Ophthalmic injection device including dosage control device", WO/2011/153349 titled "Ophthalmic compositions comprising pbo-peo-pbo block copolymers", WO/2011/140203 titled "Stabilized ophthalmic galactomannan formulations", WO/2011/068955 titled "Ophthalmic emulsion" , WO/2011/037908 titled "Injectable aqueous ophthalmic composition and method of use therefor", US2007/0149593 titled "Pharmaceutical Formulation for Delivery of Receptor Tyrosine Kinase Inhibiting (RTKi) Compounds to the Eye", US 8,632,809 titled "Water insoluble polymer matrix for drug delivery" (Alcon, Inc.).

Additional non-limiting examples of drug delivery devices and methods include, for example, US20090203709 titled "Pharmaceutical Dosage Form For Oral Administration Of Tyrosine Kinase Inhibitor" (Abbott Laboratories); US20050009910 titled "Delivery of an active drug to the posterior part of the eye via subconjunctival or periocular delivery of a prodrug", US 20130071349 titled "Biodegradable polymers for lowering intraocular pressure", US 8,481,069 titled "Tyrosine kinase microspheres", US 8,465,778 titled "Method of making tyrosine kinase microspheres", US 8,409,607 titled "Sustained release intraocular implants containing tyrosine kinase inhibitors and related methods", US 8,512,738 and US 2014/0031408 titled "Biodegradable intravitreal tyrosine kinase implants", US 2014/0294986 titled "Microsphere Drug Delivery System for Sustained Intraocular Release", US 8,911,768 titled "Methods For Treating Retinopathy With Extended Therapeutic Effect" (Allergan, Inc.); US 6,495,164 titled "Preparation of injectable suspensions having improved injectability" (Alkermes Controlled Therapeutics, Inc.); WO 2014/047439 titled "Biodegradable Microcapsules Containing Filling Material" (Akina, Inc.); WO 2010/132664 titled "Compositions And Methods For Drug Delivery" (Baxter International Inc. Baxter Healthcare SA); US20120052041 titled "Polymeric nanoparticles with enhanced drugloading and methods of use thereof" (The Brigham and Women's Hospital, Inc.); US20140178475, US20140248358, and US20140249158 titled "Therapeutic Nanoparticles Comprising a Therapeutic Agent and Methods of Making and Using Same" (BIND Therapeutics, Inc.); US 5,869,103 titled "Polymer microparticles for drug delivery" (Danbiosyst UK Ltd.); US 8628801 titled "Pegylated Nanoparticles" (Universidad de Navarra); US2014/0107025 titled "Ocular drug delivery system" (Jade Therapeutics, LLC); US 6,287,588 titled "Agent delivering system comprised of microparticle and biodegradable gel with an improved releasing profile and methods of use thereof", US 6,589,549 titled "Bioactive agent delivering system comprised of microparticles within a biodegradable to improve release profiles" (Macromed, Inc.); US 6,007,845 and US 5,578,325 titled "Nanoparticles and microparticles of non-linear hydrophilichydrophobic multiblock copolymers" (Massachusetts Institute of Technology); US20040234611, US20080305172, US20120269894, and US20130122064 titled "Ophthalmic depot formulations for periocular or subconjunctival administration (Novartis Ag); US 6,413,539 titled "Block polymer" (Poly-Med, Inc.); US 20070071756 titled "Delivery of an agent to ameliorate inflammation" (Peyman); US 20080166411 titled "Injectable Depot Formulations And Methods For Providing Sustained Release Of Poorly Soluble Drugs Comprising Nanoparticles" (Pfizer, Inc.); US 6,706,289 titled "Methods and compositions for enhanced delivery of bioactive molecules" (PR Pharmaceuticals, Inc.); and US 8,663,674 titled "Microparticle containing matrices for drug delivery" (Surmodics).

### USES OF ACTIVE COMPOUNDS FOR TREATMENT OF SELECTED DISORDERS

In one aspect, an effective amount of an active compound or its salt or composition as described herein is used to treat a medical disorder which is an inflammatory or immune condition, a disorder mediated by the complement cascade (including a dysfunctional cascade) including a complement-mediated disease or disorder including a complement factor D-related disorder or alternative complement pathway-related disorder, a disorder or abnormality of a cell that adversely affects the ability of the cell to engage in or respond to normal complement activity, or an undesired complement-mediated response to a medical treatment, such as surgery or other medical procedure or a pharmaceutical or biopharmaceutical drug administration, a blood transfusion, or other allogenic tissue or fluid administration.

A complement-mediated disease or disorder is a disease or disorder in which the amount or activity of complement is such as to cause disease or disorder in an individual. In some embodiments, the complement-mediated disease or disorder is selected from the group consisting of autoimmune disease, cancer, hematological disease, infectious disease, inflammatory disease, ischemia-reperfusion injury, neurodegenerative disease, neurodegenerative disorder, ocular disease, renal disease, transplant rejection, vascular disease, and vasculitis disease. In some embodiments, the complement-mediated disease or disorder is an autoimmune disease. In some embodiments, the complement-mediated disease or disorder is cancer. In some embodiments, the complement-mediated disease or disorder is an infectious disease. In some embodiments, the complement-mediated disease or disorder is an inflammatory disease. In some embodiments, the complement-mediated disease or disorder is a hematological disease. In some embodiments, the complement-mediated disease or disorder is an ischemia-reperfusion injury. In some embodiments, the complement-mediated disease or disorder is ocular disease. In some embodiments, the complement-mediated disease or disorder is a renal disease. In some embodiments, the complement-mediated disease or disorder is transplant rejection. In some embodiments, the complement-mediated disease or disorder is antibody-mediated transplant rejection. In some embodiments, the complement-mediated disease or disorder is a vascular disease. In some embodiments, the complement-mediated disease or disorder is a vasculitis disorder. In some embodiments, the complement-mediated disease or disorder is a neurodegenerative disease or disorder. In some embodiments, the complement-mediated disease is a neurodegenerative disease. In some embodiments, the complement-mediated disorder is a neurodegenerative disorder. In some embodiments, the complement-mediated disease or disorder is a tauopathy.

In some embodiments, a compound as provided herein, or a pharmaceutically acceptable salt, optionally in a pharmaceutically acceptable composition, is provided for use in the treatment of C3 glomerulonephritis (C3G), wherein the use includes the administration of an effective amount of the compound to a host, or the pharmaceutically acceptable salt thereof, optionally in the pharmaceutically acceptable composition. In some embodiments, a compound for use in the treatment of paroxysmal nocturnal hemoglobinuria (PNH) is provided, wherein the use includes the administration of an effective amount of a compound as provided herein to a host, or a pharmaceutically acceptable salt, optionally in a pharmaceutically acceptable composition. In another embodiment, a compound for use in the treatment of wet or dry age-related macular degeneration (AMD) in a host is provided, wherein the use includes the administration of an effective amount of a compound as provided herein, or a pharmaceutically acceptable salt, optionally in a pharmaceutically acceptable composition. In another embodiment, a compound for use in the treatment of rheumatoid arthritis in a host is provided, wherein the use includes the administration of an effective amount of a compound as provided herein, or a pharmaceutically acceptable salt thereof, optionally in a pharmaceutically acceptable composition. In another embodiment, a compound for use in the treatment of multiple sclerosis in a host is provided, wherein the use includes the administration of an effective amount of a compound as provided herein, or a pharmaceutically acceptable salt thereof, optionally in a pharmaceutically acceptable composition.

The active compound or its pharmaceutically acceptable salt thereof, optionally in a pharmaceutically acceptable composition, as disclosed herein is also useful for administration in combination (in the same or a different dosage form) or alternation with a second pharmaceutical agent for use in ameliorating or reducing a side effect of the second pharmaceutical agent. For example, in some embodiments, the active compound may be used in combination with an adoptive cell transfer therapy to reduce an inflammatory response associated with such therapy, for example, a cytokine mediated response such as cytokine response syndrome. In some embodiments, the adoptive cell transfer therapy is a chimeric antigen receptor T-Cell (CAR T) or a dendritic cell used to treat a hematologic or solid tumor, for example, a B-cell related hematologic cancer. In some embodiments, the hematologic or solid tumor is acute lymphoblastic leukemia (ALL), acute myeloid leukemia (AML), non-Hodgkin's lymphoma, chronic lymphocytic leukemia (CLL), pancreatic cancer, glioblastoma, or a cancer that expresses CD19. In some embodiments, the associated inflammatory response is a cytokine mediated response.

Another embodiment is provided that includes the administration of an effective amount of an active compound or a pharmaceutically acceptable salt thereof, optionally in a pharmaceutically acceptable composition to a host to treat an ocular, pulmonary, gastrointestinal, or other disorder that can benefit from topical or local delivery.

Any of the compounds described herein can be administered to the eye in any desired form of administration, including via intravitreal, intrastromal, intracameral, sub-tenon, sub-retinal, retro-bulbar, peribulbar, suprachorodial, choroidal, subchoroidal, conjunctival, subconjunctival, episcleral, posterior juxtascleral, scleral, circumcorneal, and tear duct injections, or through a mucus, mucin, or a mucosal barrier, in an immediate or controlled release fashion. In certain embodiments, the active compound includes a lipophilic group, such as a lipophilic acyl group, which is delivered to the eye in a polymeric drug delivery system such as polylactic acid, polylactide-co-glycolide, polyglycolide or other erodible polymer, or a combination thereof, or in another type of lipophilic material for ocular delivery. In some embodiments, the lipophilic active molecule is more soluble in the polymeric or other form of delivery system than in ocular fluid.

In other embodiments of the invention, an active compound provided herein can be used to treat or prevent a disorder in a host mediated by complement factor D, or by an excessive or detrimental amount of the complement-C3 amplification loop of the complement pathway. As examples, the invention includes an active compound or its salt or composition as provided herein for use in treating or preventing complement associated disorders that are induced by antibody-antigen interactions, a component of an immune or autoimmune disorder or by ischemic injury. The invention also provides an active compound or its salt or composition as provided herein for use in decreasing inflammation or an immune response, including an autoimmune response, where mediated or affected by factor D.

In some embodiments, the disorder is selected from fatty liver and conditions stemming from fatty liver, such as nonalcoholic steatohepatitis (NASH), liver inflammation, cirrhosis and liver failure. In some embodiments of the present invention, an active compound or its salt or composition as described herein is provided for use in treating fatty liver disease in a host, by administering an effective amount of the active compound or its salt or composition.

In another embodiment, an active compound or its salt or composition as described herein is used to modulate an immune response prior to or during surgery or other medical procedure. One non-limiting example is use in connection with acute or chronic graft versus host disease, which is a common complication as a result of allogeneic tissue transplant, and can also occur as a result of a blood transfusion.

In some embodiments, the present invention provides an active compound or its salt or composition as described herein for use in treating or preventing dermatomyositis, by administering to a subject in need thereof an effective amount of the active compound or its salt or composition.

In some embodiments, the present invention provides an active compound or its salt or composition as described herein for use in treating or preventing amyotrophic lateral sclerosis, by administering to a subject in need thereof an effective amount of the active compound or its salt or composition.

In some embodiments, the present invention provides an active compound or its salt or composition as described herein for use in treating or preventing abdominal aortic aneurysm, hemodialysis complications, hemolytic anemia, or hemodialysis, by administering to a subject in need thereof an effective amount of the active compound or its salt or composition.

In another embodiment, an active compound or its salt or composition as described herein is provided for use in the treatment or prevention of cytokine or inflammatory reactions in response to the administration of pharmaceutical or biotherapeutic (e.g. CAR T-cell therapy or monoclonal antibody therapy) in a host, by administering an effective amount of the active compound or its salt or composition. Various types of cytokine or inflammatory reactions may occur in response to a number of factors, such as the administrations of biotherapeutics. In some embodiments, the cytokine or inflammatory reaction is cytokine release syndrome. In some embodiments, the cytokine or inflammatory reaction is tumor lysis syndrome (which also leads to cytokine release). Symptoms of cytokine release syndrome range from fever, headache, and skin rashes to bronchospasm, hypotension and even cardiac arrest. Severe cytokine release syndrome is described as cytokine storm, and can be fatal.

Fatal cytokine storms have been observed in response to infusion with several monoclonal antibody therapeutics. See, Abramowicz D, et al. "Release of tumor necrosis factor, interleukin-2, and gamma-interferon in serum after injection of OKT3 monoclonal antibody in kidney transplant recipients" Transplantation (1989) 47(4):606-8; Chatenoud L, et al. "In vivo cell activation following OKT3 administration. Systemic cytokine release and modulation by corticosteroids" Transplantation (1990) 49(4):697-702; and Lim LC, Koh LP, and Tan P. "Fatal cytokine release syndrome with chimeric anti-CD20 monoclonal antibody rituximab in a 71-year-old patient with chronic lymphocytic leukemia" J. Clin Oncol. (1999) 17(6):1962-3.

Also contemplated herein, is the use of an active compound or its salt or composition as described herein to mediate an adverse immune response in patients receiving bi-specific T-cell engagers (BiTE). A bi-specific T-cell engager directs T-cells to target and bind with a specific antigen on the surface of a cancer cell. For example, Blinatumomab (Amgen), a BiTE has recently been approved as a second line therapy in Philadelphia chromosome-negative relapsed or refractory acute lymphoblastic leukemia. Blinatumomab is given by continuous intravenous infusion in 4-week cycles. The use of BiTE agents has been associated with adverse immune responses, including cytokine release syndrome. The most significantly elevated cytokines in the CRS associated with ACT include IL-10, IL-6, and IFN-γ (Klinger et al., Immunopharmacologic response of patients with B-lineage acute lymphoblastic leukemia to continuous infusion of T cell-engaging CD19/CD3-bispecific BiTE antibody blinatumomab. Blood (2012) 119:6226-6233).

In another embodiment, the disorder is episcleritis, idiopathic episcleritis, anterior episcleritis, or posterior episcleritis. In some embodiments, the disorder is idiopathic anterior uveitis, HLA-B27 related uveitis, herpetic keratouveitis, Posner Schlossman syndrome, Fuch's heterochromic iridocyclitis, or cytomegalovirus anterior uveitis.

In some embodiments, the present invention provides an active compound or its salt or composition as described herein for use in treating or preventing a C3 glomurenopathy, by administering to a subject in need thereof an effective amount of the active compound or its salt or composition. In some embodiments, the disorder is selected from dense deposit disease (DDD) and C3 glomerulonephritis (C3GN).

In some embodiments, the present invention provides an active compound or its salt or composition as described herein for use in treating or preventing a IC-MPGN, by administering to a subject in need thereof an effective amount of the active compound or its salt or composition.

In some embodiments, the present invention provides an active compound or its salt or composition as described herein for use in treating or preventing a paroxysmal nocturnal hemoglobinuria (PNH), by administering to a subject in need thereof an effective amount of the active compound or its salt or composition.

In some embodiments, the present invention provides an active compound or its salt or composition as described herein for use in treating or preventing age-related macular degeneration (AMD), by administering to a subject in need thereof an effective amount of the active compound or its salt or composition.

In some embodiments, the present invention provides an active compound or its salt or composition as described herein for use in treating or preventing rheumatoid arthritis, by administering to a subject in need thereof an effective amount of the active compound or its salt or composition.

In some embodiments, the present invention provides an active compound or its salt or composition as described herein for use in treating or preventing multiple sclerosis, by administering to a subject in need thereof an effective amount of the active compound or its salt or composition.

In some embodiments, the present invention provides an active compound or its salt or composition as described herein for use in treating or preventing myasthenia gravis, by administering to a subject in need thereof an effective amount of the active compound or its salt or composition.

In some embodiments, the present invention provides an active compound or its salt or composition as described herein for use in treating or preventing atypical hemolytic uremic syndrome (aHUS), by administering to a subject in need thereof an effective amount of the active compound or its salt or composition.

In some embodiments, the present invention provides an active compound or its salt or composition as described herein for use in treating or preventing neuromyelitis optica (NMO), by administering to a subject in need thereof an effective amount of the active compound or its salt or composition.

In yet another embodiment, the present invention an active compound or its salt or composition as described herein for use in treating or preventing a disorder as described below, by administering to a subject in need thereof an effective amount of the active compound or its salt or composition, including:
vitritis, sarcoidosis, syphilis, tuberculosis, or Lyme disease; retinal vasculitis, Eales disease, tuberculosis, syphilis, or toxoplasmosis; neuroretinitis, viral retinitis, or acute retinal necrosis;
varicella zoster virus, herpes simplex virus, cytomegalovirus, Epstein-Barr virus, lichen planus, or Dengue-associated disease (e.g., hemorraghic Dengue Fever); Masquerade syndrome, contact dermatitis, trauma induced inflammation, UVB induced inflammation, eczema, granuloma annulare, or acne.

In an additional embodiment, the disorder is selected from: acute myocardial infarction, aneurysm, cardiopulmonary bypass, dilated cardiomyopathy, complement activation during cardiopulmonary bypass operations, coronary artery disease, restenosis following stent placement, or percutaneous transluminal coronary angioplasty (PTCA); antibody-mediated transplant rejection, anaphylactic shock, anaphylaxis, allogenic transplant, humoral and vascular transplant rejection, graft dysfunction, graft-versus-host disease, Graves' disease, adverse drug reactions, or chronic graft vasculopathy; allergic bronchopulmonary aspergillosis, allergic neuritis, drug allergy, radiation- induced lung injury, eosinophilic pneumonia, radiographic contrast media allergy, bronchiolitis obliterans, or interstitial pneumonia; parkinsonism-dementia complex, sporadic frontotemporal dementia, frontotemporal dementia with Parkinsonism linked to chromosome 17, frontotemporal lobar degeneration, tangle only dementia, cerebral amyloid angiopathy, cerebrovascular disorder, certain forms of frontotemporal dementia, chronic traumatic encephalopathy (CTE), PD with dementia (PDD), argyrophilic grain dementia, dementia pugilistica, dementia with Lewy Bodies (DLB), or multi-infarct dementia; Creutzfeldt-Jakob disease, Huntington's disease, multifocal motor neuropathy (MMN), prion protein cerebral amyloid angiopathy, polymyositis, postencephalitic parkinsonism, subacute sclerosing panencephalitis, non-Guamanian motor neuron disease with neurofibrillary tangles, neural regeneration, or diffuse neurofibrillary tangles with calcification.

In some embodiments, the disorder is selected from: atopic dermatitis, dermatitis, dermatomyositis bullous pemphigoid, scleroderma, sclerodermatomyositis, psoriatic arthritis, pemphigus vulgaris, Discoid lupus erythematosus, cutaneous lupus, chilblain lupus erythematosus, or lupus erythematosus-lichen planus overlap syndrome; cryoglobulinemic vasculitis, mesenteric/enteric vascular disorder, peripheral vascular disorder, antineutrophil cytoplasm antibody (ANCA)-associated vasculitis (AAV), IL-2 induced vascular leakage syndrome, or immune complex vasculitis;angioedema, low platelets (HELLP) syndrome, sickle cell disease, platelet refractoriness, red cell casts, or typical or infectious hemolytic uremic syndrome (tHUS); hematuria, hemorrhagic shock, drug-induced thrombocytopenia, autoimmune hemolytic anemia (AIHA), azotemia, blood vessel and/or lymph vessel inflammation, rotational atherectomy, or delayed hemolytic transfusion reaction; British type amyloid angiopathy, Buerger's disease, bullous pemphigoid, C1q nephropathy, cancer, or catastrophic antiphospholipid syndrome.

In another embodiment, the disorder is selected from:wet (exudative) AMD, dry (non-exudative) AMD, chorioretinal degeneration, choroidal neovascularization (CNV), choroiditis, loss of RPE function, loss of vision (including loss of visual acuity or visual field), loss of vision from AMD, retinal damage in response to light exposure, retinal degeneration, retinal detachment, retinal dysfunction, retinal neovascularization (RNV), retinopathy of prematurity, pathological myopia, or RPE degeneration; pseudophakic bullous keratopathy, symptomatic macular degeneration related disorder, optic nerve degeneration, photoreceptor degeneration, cone degeneration, loss of photoreceptor cells, pars planitis, scleritis, proliferative vitreoretinopathy, or formation of ocular drusen; chronic urticaria, Churg-Strauss syndrome, cold agglutinin disease (CAD), corticobasal degeneration (CBD), cryoglobulinemia, cyclitis, damage of the Bruch's membrane, Degos disease, diabetic angiopathy, elevated liver enzymes, endotoxemia, epidermolysis bullosa, or epidermolysis bullosa acquisita; essential mixed cryoglobulinemia, excessive blood urea nitrogen-BUN, focal segmental glomerulosclerosis, Gerstmann-Straussler-Scheinker disease, giant cell arteritis, gout, Hallervorden-Spatz disease, Hashimoto's thyroiditis, Henoch-Schonlein purpura nephritis, or abnormal urinary sediments; hepatitis, hepatitis A, hepatitis B, hepatitis C or human immunodeficiency virus (HIV), a viral infection more generally, for example selected from Flaviviridae, Retroviruses, Coronaviridae, Poxviridae, Adenoviridae, Herpesviridae, Caliciviridae, Reoviridae, Picornaviridae, Togaviridae, Orthomyxoviridae, Rhabdoviridae, or Hepadnaviridae; Neisseria meningitidis, shiga toxin E. coli-related hemolytic uremic syndrome (STEC-HUS), hemolytic uremic syndrome (HUS); Streptococcus, or poststreptococcal glomerulonephritis.

In a further embodiment, the disorder is selected from: hyperlipidemia, hypertension, hypoalbuminemia, hypobolemic shock, hypocomplementemic urticarial vasculitis syndrome, hypophosphastasis, hypovolemic shock, idiopathic pneumonia syndrome, or idiopathic pulmonary fibrosis; inclusion body myositis, intestinal ischemia, iridocyclitis, iritis, juvenile chronic arthritis, Kawasaki's disease (arteritis), or lipiduria; membranoproliferative glomerulonephritis (MPGN) I, microscopic polyangiitis, mixed cryoglobulinemia, molybdenum cofactor deficiency (MoCD) type A, pancreatitis, panniculitis, Pick's disease, polyarteritis nodosa (PAN), progressive subcortical gliosis, proteinuria, reduced glomerular filtration rate (GFR), or renovascular disorder; multiple organ failure, multiple system atrophy (MSA), myotonic dystrophy, Niemann-Pick disease type C, chronic demyelinating diseases, or progressive supranuclear palsy; spinal cord injury, spinal muscular atrophy, spondyloarthropathies, Reiter's syndrome, spontaneous fetal loss, recurrent fetal loss, pre-eclampsia, synucleinopathy, Takayasu's arteritis, post-partum thryoiditis, thyroiditis, Type I cryoglobulinemia, Type II mixed cryoglobulinemia, Type III mixed cryoglobulinemia, ulcerative colitis, uremia, urticaria, venous gas embolus (VGE), or Wegener's granulomatosis; von Hippel-Lindau disease, histoplasmosis of the eye, hard drusen, soft drusen, pigment clumping, or photoreceptor and/or retinal pigmented epithelia (RPE) loss,.

In some embodiments, an active compound or its salt or composition as described herein is useful for use in treating or preventing a disorder selected from autoimmune oophoritis, endometriosis, autoimmune orchitis, Ord's thyroiditis, autoimmune enteropathy, coeliac disease, Hashimoto's encephalopathy, antiphospholipid syndrome (APLS) (Hughes syndrome), aplastic anemia, autoimmune lymphoproliferative syndrome (Canale-Smith syndrome), autoimmune neutropenia, Evans syndrome, pernicious anemia, pure red cell aplasia, thrombocytopenia, adipose dolorosa (Dercum's disease), adult onset Still's disease, ankylosing spondylitis, CREST syndrome, drug-induced lupus, eosinophilic fasciitis (Shulman's syndrome), Felty syndrome, IgG4-related disease, mixed connective tissue disease (MCTD), palindromic rheumatism (Hench-Rosenberg syndrome), Parry-Romberg syndrome, Parsonage-Turner syndrome, relapsing polychondritis (Meyenburg-Altherr-Uehlinger syndrome), retroperitonial fibrosis, rheumatic fever, Schnitzler syndrome, fibromyalgia, neuromyotonia (Isaac's disease), paraneoplastic degeneration, autoimmune inner ear disease, Meniere's disease, interstitial cystitis, autoimmune pancreatitis, zika virus-related disorders, chikungunya virus-related disorders, subacute bacterial endocarditis (SBE), IgA nephropathy, IgA vasculitis, polymyalgia rheumatic, rheumatoid vasculitis, alopecia areata, autoimmune progesterone dermatitis, dermatitis herpetiformis, erythema nodosum, gestational pemphigoid, hidradenitis suppurativa, lichen sclerosus, linear IgA disease (LAD), morphea, myositis, pityriasis lichenoides et varioliformis acuta, vitiligo post-myocardial infarction syndrome (Dressler's syndrome), post-pericardiotomy syndrome, autoimmune retinopathy, Cogan syndrome, Graves opthalmopathy, ligneous conjunctivitis, Mooren's ulcer, opsoclonus myoclonus syndrome, optic neuritis, retinocochleocerebral vasculopathy (Susac's syndrome), sympathetic opthalmia, Tolosa-Hunt syndrome, interstitial lung disease, anti synthetase syndrome, Addison's disease, autoimmune polyendocrine syndrome (APS) type I, autoimmune polyendocrine syndrome (APS) type II, autoimmune polyendocrine syndrome (APS) type III, disseminated sclerosis (multiple sclerosis, pattern II), rapidly progressing glomerulonephritis (RPGN), juvenile rheumatoid arthritis, enthesitis-related arthritis, reactive arthritis (Reiter's syndrome), autoimmune hepatitis or lupoid hepatitis, primary biliary cirrhosis (PBS), primary sclerosing cholangitis, microscopic colitis, latent lupus (undifferentiated connective tissue disease (UCTD)), acute disseminated encephalomyelitis (ADEM), acute motor axonal neuropathy, anti-n-methyl-D-aspartate receptor encephalitis, Balo concentric sclerosis (Schilders disease), Bickerstaff's encephalitis, chronic inflammatory demyelinating polyneuropathy, idiopathic inflammatory demyelinating disease, Lambert-Eaton mysathenic syndrome, Oshtoran syndrome, pediatric autoimmune neuropsychiatric disorder associated with streptococcus (PANDAS), progressive inflammatory neuropathy, restless leg syndrome, stiff person syndrome, Sydenhem syndrome, transverse myelitis, lupus vasculitis, leukocytoclastic vasculitis, Microscopic Polyangiitis, polymyositis or ischemic-reperfusion injury of the eye.

Examples of eye disorders that may be treated according to the uses disclosed herein include amoebic keratitis, fungal keratitis, bacterial keratitis, viral keratitis, onchorcercal keratitis, bacterial keratoconjunctivitis, viral keratoconjunctivitis, corneal dystrophic diseases, Fuchs' endothelial dystrophy, Sjogren's syndrome, Stevens-Johnson syndrome, autoimmune dry eye diseases, environmental dry eye diseases, corneal neovascularization diseases, post-corneal transplant rejection prophylaxis and treatment, autoimmune uveitis, infectious uveitis, posterior uveitis (including toxoplasmosis), pan-uveitis, an inflammatory disease of the vitreous or retina, endophthalmitis prophylaxis and treatment, macular edema, macular degeneration, age related macular degeneration, proliferative and non-proliferative diabetic retinopathy, hypertensive retinopathy, an autoimmune disease of the retina, primary and metastatic intraocular melanoma, other intraocular metastatic tumors, open angle glaucoma, closed angle glaucoma, pigmentary glaucoma and combinations thereof.

In a further embodiment, the disorder is selected from glaucoma, diabetic retinopathy, blistering cutaneous diseases (including bullous pemphigoid, pemphigus, and epidermolysis bullosa), ocular cicatrical pemphigoid, uveitis, adult macular degeneration, diabetic retinopa retinitis pigmentosa, macular edema, diabetic macular edema, Behcet's uveitis, multifocal choroiditis, Vogt-Koyangi-Harada syndrome, imtermediate uveitis, birdshot retino-chorioditis, sympathetic ophthalmia, ocular dicatricial pemphigoid, ocular pemphigus, nonartertic ischemic optic neuropathy, postoperative inflammation, and retinal vein occlusion, or central retinal vein occulusion (CVRO).

In some embodiments, complement mediated diseases include ophthalmic diseases (including early or neovascular age-related macular degeneration and geographic atrophy), autoimmune diseases (including arthritis, rheumatoid arthritis), respiratory diseases, cardiovascular diseases. In other embodiments, the compounds of the invention are suitable for use in the treatment of diseases and disorders associated with fatty acid metabolism, including obesity and other metabolic disorders.

Disorders that may be treated or prevented by use of an active compound or its salt or composition as described herein also include, but are not limited to: hereditary angioedema, capillary leak syndrome, hemolytic uremic syndrome (HUS), neurological disorders, Guillain Barre Syndrome, diseases of the central nervous system and other neurodegenerative conditions, glomerulonephritis (including membrane proliferative glomerulonephritis), SLE nephritis, proliferative nephritis, liver fibrosis, tissue regeneration and neural regeneration, or Barraquer-Simons Syndrome;
inflammatory effects of sepsis, systemic inflammatory response syndrome (SIRS), disorders of inappropriate or undesirable complement activation, interleukin-2 induced toxicity during IL-2 therapy, inflammatory disorders, inflammation of autoimmune diseases, system lupus erythematosus (SLE), lupus nephritides, arthritis, immune complex disorders and autoimmune diseases, systemic lupus, or lupus erythematosus; ischemia/ reperfusion injury (I/R injury), myocardial infarction, myocarditis, post-ischemic reperfusion conditions, balloon angioplasty, atherosclerosis, post-pump syndrome in cardiopulmonary bypass or renal bypass, renal ischemia, mesenteric artery reperfusion after aortic reconstruction, antiphospholipid syndrome, autoimmune heart disease, ischemia-reperfusion injuries, obesity, or diabetes; Alzheimer's dementia, stroke, schizophrenia, traumatic brain injury, trauma, Parkinson's disease, epilepsy, transplant rejection, prevention of fetal loss, biomaterial reactions (e.g. in hemodialysis, inplants), hyperacute allograft rejection, xenograft rejection, transplantation, psoriasis, burn injury, thermal injury including burns or frostbite, or crush injury; asthma, allergy, acute respiratory distress syndrome (ARDS), cystic fibrosis, adult respiratory distress syndrome, dyspnea, hemoptysis, chronic obstructive pulmonary disease (COPD), emphysema, pulmonary embolisms and infarcts, pneumonia, fibrogenic dust diseases, inert dusts and minerals (e.g., silicon, coal dust, beryllium, and asbestos), pulmonary fibrosis, organic dust diseases, chemical injury (due to irritant gases and chemicals, e.g., chlorine, phosgene, sulfur dioxide, hydrogen sulfide, nitrogen dioxide, ammonia, and hydrochloric acid), smoke injury, thermal injury (e.g., burn, freeze), bronchoconstriction, hypersensitivity pneumonitis, parasitic diseases, Goodpasture's Syndrome (anti-glomerular basement membrane nephritis), pulmonary vasculitis, Pauci-immune vasculitis, or immune complex- associated inflammation.

In some embodiments, an active compound or its salt or composition as described herein for use in the treatment of sickle cell in a host is provided, wherein said use includes the administration of an effective amount of the active compound or its salt or composition. In some embodiments, an active compound or its salt or composition as described herein for use in the treatment of immunothrombocytopenic purpura (ITP), thrombotic thrombocytopenic purpura (TTP), or idiopathic thrombocytopenic purpura (ITP) in a host is provided, wherein said use includes the administration of an effective amount of the active compound or its salt or composition. In some embodiments, an active compound or its salt or composition as described herein for use in the treatment of ANCA-vasculitis in a host is provided, wherein said use includes the administration of an effective amount of the active compound or its salt or composition. In some embodiments, an active compound or its salt or composition as described herein for use in the treatment of IgA nephropathy in a host is provided, wherein said use includes the administration of an effective amount of the active compound or its salt or composition. In some embodiments, an active compound or its salt or composition as described herein for use in the treatment of rapidly progressing glomerulonephritis (RPGN) in a host is provided, wherein said use includes the administration of an effective amount of the active compound or its salt or composition. In some embodiments, an active compound or its salt or composition as described herein for use in the treatment of lupus nephritis in a host is provided, wherein said use includes the administration of an effective amount of the active compound or its salt or composition. In some embodiments, an active compound or its salt or composition as described herein for use in the treatment of hemorraghic dengue fever in a host is provided, wherein said use includes the administration of an effective amount of the active compound or its salt or composition.

In an additional alternative embodiment, an active compound or its salt or composition as described herein is used in the treatment of an autoimmune disorder.
The complement pathway enhances the ability of antibodies and phagocytic cells to clear microbes and damaged cells from the body. It is part of the innate immune system and in healthy individuals is an essential process. Inhibiting the complement pathway will decrease the body's immune system response. Therefore, it is an object of the present invention to provide an active compound or its salt or composition as described herein for use in treating autoimmune disorders, by administering an effective dose of the active compound or its salt or composition to a subject in need thereof.

In some embodiments the autoimmune disorder is caused by activity of the complement system. In some embodiments the autoimmune disorder is caused by activity of the alternative complement pathway. In some embodiments the autoimmune disorder is caused by activity of the classical complement pathway. In another embodiment the autoimmune disorder is caused by a mechanism of action that is not directly related to the complement system, such as the over-proliferation of T-lymphocytes or the over-production of cytokines.

Non-limiting examples of autoimmune disorders include: lupus, allograft rejection, autoimmune thyroid diseases (such as Graves' disease and Hashimoto's thyroiditis), autoimmune uveoretinitis, giant cell arteritis, inflammatory bowel diseases (including Crohn's disease, ulcerative colitis, regional enteritis, granulomatous enteritis, distal ileitis, regional ileitis, and terminal ileitis), diabetes, multiple sclerosis, pernicious anemia, psoriasis, rheumatoid arthritis, sarcoidosis, and scleroderma.

In some embodiments, an active compound or its salt or composition as described herein is used in the treatment of lupus. Non-limiting examples of lupus include lupus erythematosus, cutaneous lupus, discoid lupus erythematosus, chilblain lupus erythematosus, or lupus erythematosus-lichen planus overlap syndrome.

Lupus erythematosus is a general category of disease that includes both systemic and cutaneous disorders. The systemic form of the disease can have cutaneous as well as systemic manifestations. However, there are also forms of the disease that are only cutaneous without systemic involvement. For example, SLE is an inflammatory disorder of unknown etiology that occurs predominantly in women, and is characterized by articular symptoms, butterfly erythema, recurrent pleurisy, pericarditis, generalized adenopathy, splenomegaly, as well as CNS involvement and progressive renal failure. The sera of most patients (over 98%) contain antinuclear antibodies, including anti-DNA antibodies. High titers of anti-DNA antibodies are essentially specific for SLE. Conventional treatment for this disease has been the administration of corticosteroids or immunosuppressants.

There are three forms of cutaneous lupus: chronic cutaneous lupus (also known as discoid lupus erythematosus or DLE), subacute cutaneous lupus, and acute cutaneous lupus. DLE is a disfiguring chronic disorder primarily affecting the skin with sharply circumscribed macules and plaques that display erythema, follicular plugging, scales, telangiectasia and atrophy. The condition is often precipitated by sun exposure, and the early lesions are erythematous, round scaling papules that are 5 to 10 mm in diameter and display follicular plugging. DLE lesions appear most commonly on the cheeks, nose, scalp, and ears, but they may also be generalized over the upper portion of the trunk, extensor surfaces of the extremities, and on the mucous membranes of the mouth. If left untreated, the central lesion atrophies and leaves a scar. Unlike SLE, antibodies against double-stranded DNA (e.g., DNA-binding test) are almost invariably absent in DLE.

Multiple Sclerosis is an autoimmune demyelinating disorder that is believed to be T lymphocyte dependent. MS generally exhibits a relapsing-remitting course or a chronic progressive course. The etiology of MS is unknown, however, viral infections, genetic predisposition, environment, and autoimmunity all appear to contribute to the disorder. Lesions in MS patients contain infiltrates of predominantly T lymphocyte mediated microglial cells and infiltrating macrophages. CD4+ T lymphocytes are the predominant cell type present at these lesions. The hallmark of the MS lesion is plaque, an area of demyelination sharply demarcated from the usual white matter seen in MRI scans. Histological appearance of MS plaques varies with different stages of the disease. In active lesions, the blood-brain barrier is damaged, thereby permitting extravasation of serum proteins into extracellular spaces. Inflammatory cells can be seen in perivascular cuffs and throughout white matter. CD4+ T-cells, especially Th1, accumulate around postcapillary venules at the edge of the plaque and are also scattered in the white matter. In active lesions, up-regulation of adhesion molecules and markers of lymphocyte and monocyte activation, such as IL2-R and CD26 have also been observed. Demyelination in active lesions is not accompanied by destruction of oligodendrocytes. In contrast, during chronic phases of the disease, lesions are characterized by a loss of oligodendrocytes and hence, the presence of myelin oligodendrocyte glycoprotein (MOG) antibodies in the blood.

Diabetes can refer to either type 1 or type 2 diabetes. In some embodiments an active compound or its salt or composition as described herein is provided at an effective dose to treat a patient with type 1 diabetes. In some embodiments an active compound or its salt or composition as described herein is provided at an effective dose to treat a patient with type 2 diabetes.
Type 1 diabetes is an autoimmune disease. An autoimmune disease results when the body's system for fighting infection (the immune system) attacks a part of the body. In the case of diabetes type 1, the pancreas then produces little or no insulin.

In certain aspects, an effective amount of an active compound described herein, or its pharmaceutically acceptable salt, is used to treat a medical disorder of the central nervous system (CNS) or peripheral nervous system disorders involving complement activation. In embodiments, the CNS disorder is an acquired brain or spinal cord injury, including, but not limited to ischaemic-reperfusion injury or stroke, traumatic brain injury (TBI) and spinal cord injury (SCI). In embodiments, the disorder is a neurodegeneration disorder. In embodiments, the disorder is a neuroinflammation disorder.

In certain aspects, active compound described herein, or it pharmaceutically acceptable salt is used to treat Alzheimer's disease (AD). AD is characterized by two hallmark pathologies; amyloid-β (Aβ) plaques and neurofibrillary tangles comprising hyperphosphorylated tau. Recent studies have implicated complement in AD pathogenesis, including genome wide association studies identifying single nucleotide polymorphisms (SNPs) associated with risk of late-onset AD in genes encoding complement proteins Clusterin (CLU) and CR1 (CR1). See Carpanini et al., Therapeutic Inhibition of the Complement System in Diseases of the Central Nervous System, Front. Immunol., 04 March 2019. Biomarker studies have also identified complement proteins and activation products in plasma and/or CSF that distinguish AD from controls and predict risk of progression to AD. (Id.)

In certain aspects, an effective amount of active compound described herein, or it pharmaceutically acceptable salt is used to treat certain forms of frontotemporal dementia including, but not limited to, Pick's disease, sporadic Frontotemporal dementia and Frontotemporal dementia with Parkinsonism linked to chromosome 17, Progressive supranuclear palsy (PSP), Corticobasal degeneration (CBD), and Subacute sclerosing panencephalitis.

In certain aspects, an effective amount of active compound described herein, or it pharmaceutically acceptable salt is used to treat multiple sclerosis (MS). Multiple sclerosis (MS) is the most common cause of neurological disability in young adults in northern European-Caucasian populations, with an approximate lifetime risk of one in 400. C3 has been shown to be deposited in the brains of MS patients. TCC has been shown to be in association with capillary endothelial cells, predominantly within plaques and adjacent white matter. Localization of C activation to areas of active myelin destruction has also been shown, with TCC deposited exclusively in such areas. C3d has been shown to be deposited in association with short segments of disrupted myelin in plaques with low-grade active demyelination and provides evidence for a C contribution to disease progression as well as acute inflammation. See Ingram et al., Complement in multiple sclerosis: its role in disease and potential as a biomarker. Clin Exp Immunol. 2009 Feb;155(2):128-39.

In certain aspects, active compound described herein, or it pharmaceutically acceptable salt is used to treat neuromyelitis optica (NMO). Neuromyelitis optica (NMO) is an inflammatory demyelinating disease affecting predominantly the optic nerves and spinal cord. Traditionally seen as a variant of MS, it has been redefined recently according to new criteria using a combination of phenotypic subtyping along with a newly developed biomarker of disease, NMO-immunoglobulin G (IgG) (reported sensitivity of 58-76% and specificity of 85-99% for NMO). NMO patients have higher levels of C3a and anti-C1q antibodies than healthy controls. C3a levels correlated with disease activity, neurological disability and aquaporin-4 IgG. Nytrova et al., Complement activation in patients with neuromyelitis optica. J Neuroimmunol. 2014 Sep 15;274(1-2):185-91.

In certain aspects, active compound described herein, or it pharmaceutically acceptable salt is used to treat amyotrophic lateral sclerosis (ALS). ALS is caused by progressive loss of upper and lower (α) motor neurons resulting in denervation of neuromuscular junctions in the peripheral nervous system, progressive muscle weakness, atrophy, spasticity, respiratory failure, and ultimately paralysis and death. Recent studies have shown increased C1q protein in motor cortex and spinal cord of ALS post-mortem tissue; C3 activation fragments and TCC in areas of pathology; C4d and TCC staining of degenerating neurons and glia in ALS motor cortex and spinal cord, and C5aR1 upregulation in areas of pathology. C3d and C4d have been found on oligodendroglia and degenerating neurites, surrounded by CR4-positive microglia, in spinal cord and motor cortex, and C1q, C3, and TCC have been shown to be present on motor end-plates in intercostal muscles in ALS donors even early in the disease process. See Carpanini et al., Therapeutic Inhibition of the Complement System in Diseases of the Central Nervous System, Front. Immunol., 04 March 2019.

In certain aspects, active compound described herein, or it pharmaceutically acceptable salt is used to treat Parkinson's disease (PD). PD is characterized by loss of dopaminergic neurons in the substantia nigra and deposits of the protein α-synuclein that form the pathological hallmarks of the disease, Lewy bodies. Patients present with resting tremor, bradykinesia, and rigidity. Complement activation has been associated with α-synuclein and Lewy bodies in Parkinson's disease; in vitro studies have demonstrated that the disease-associated splice variant α-synuclein 112, but not the full-length protein, cause activation of complement. In vivo, C3d, C4d, C7 and C9 localization in Lewy bodies has been reported. More recently, deposition of iC3b and C9 in Lewy bodies and melanized neurons has been reported, and iC3b immunoreactivity has been shown to be increased with normal ageing and was further elevated in PD vs. age-matched controls. Furthermore, correlation between the ratios of C3/Aβ42 or FH/Aβ42 in CSF and severity of Parkinson's disease motor and cognitive symptoms has been shown. See Carpanini et al., Therapeutic Inhibition of the Complement System in Diseases of the Central Nervous System, Front. Immunol., 04 March 2019. In some embodiments, the subject to be treated suffers from Parkinson's Disease with dementia (PDD).

In certain aspects, active compound described herein, or it pharmaceutically acceptable salt is used to treat Huntington's disease (HD). HD is an autosomal dominant, inherited neurodegenerative disease characterized by progressive motor symptoms, psychiatric disturbances, and dementia. It is caused by expansion of a three-base-pair (CAG) repeat (39-121 repeats vs. normal range 8-39 repeats) in exon 1 of the HTT gene that translates into a polyglutamine tract at the N-terminus of the protein. This results in a polyglutamine length-dependent misfolding and accumulation of huntingtin protein in the striatum and cortex (layers 3, 5, and 6) followed by neuronal loss in these areas which spreads to the hippocampus. It has been shown that neurons, astrocytes, and myelin sheaths in the HD caudate and striatum were immunoreactive for C1q, C4, C3 and neo-epitopes in iC3b and TCC. Expression of mRNA encoding early complement components C1q (c-chain), C1r, C3, and C4, complement regulators C1INH, Clusterin, MCP, DAF and CD59, a See Carpanini et al., Therapeutic Inhibition of the Complement System in Diseases of the Central Nervous System, Front. Immunol., 04 March 2019.nd complement receptors C3a and C5a has been shown to be upregulated in the HD striatum.

In certain aspects, active compound described herein, or it pharmaceutically acceptable salt is used to treat argyrophilic grain dementia, British type amyloid angiopathy, cerebral amyloid angiopathy, Creutzfeldt-Jakob disease, dementia pugilistica, diffuse neurofibrillary tangles with calcification, Down's syndrome, frontotemporal lobar degeneration, Gerstmann-Straussler-Scheinker disease, Hallervorden-Spatz disease, inclusion body myositis, multiple system atrophy (MSA), myotonic dystrophy, Niemann-Pick disease type C, non-Guamanian motor neuron disease with neurofibrillary tangles, postencephalitic parkinsonism, prion protein cerebral amyloid angiopathy, progressive subcortical gliosis, progressive supranuclear palsy, subacute sclerosing panencephalitis, Tangle only dementia, multi-infarct dementia, ischemic stroke, chronic traumatic encephalopathy (CTE), traumatic brain injury (TBI), and stroke.

In certain aspects active compound described herein, or it pharmaceutically acceptable salt is used to treat a hereditary motor and sensory neuropathy (HMSN). In some embodiments, the hereditary and sensory neuropathy is Charcot-Marie-Tooth (CMT) disease. In some embodiments, the HSMN is Charcot-Marie-Tooth disease type 1A or type 1B. In some embodiments, the HSMN is Charcot-Marie-Tooth disease type 2. In some embodiments, the HSMN is Dejerine-Sottas disease (Charcot-Marie-Tooth type 3). In some embodiments, the HSMN is Refsum disease. In some embodiments, the HSMN is Charcot-Marie-Tooth with pyramidal features. In some embodiments, the HSMN is Charcot-Marie-Tooth type 6. In some embodiments, the HSMN is HMSN+retinitis pigmentosa.

In some embodiments, an active compound as described herein is used to treat Churg-Strauss syndrome.

In some embodiments, an active compound as described herein is used to treat a peripheral artery disease (PAD).

In certain aspects, an effective amount of active compound described herein, or its pharmaceutically acceptable salt can be used to treat myasthenia gravis with CNS involvement.

In certain aspects, an effective amount of active compound described herein, or its pharmaceutically acceptable salt is used to treat dementia with Lewy bodies.

In certain aspects, an active compound described herein, or its pharmaceutically acceptable salt is used to treat an individual suffering from prion disease.

In certain aspects, an active compound described herein, or its pharmaceutically acceptable salt is used to treat Behcet's Disease.

In certain aspects, an active compound described herein, or its pharmaceutically acceptable salt is used to treat congenital myasthenia.

In certain aspects, an active compound described herein, or its pharmaceutically acceptable salt is used to treat subacute sclerosing panencephalitis (SSPE).

In certain aspects, an active compound described herein, or its pharmaceutically acceptable salt is used to treat Guillain-Barré syndrome.

In certain aspects, an active compound or its salt or composition as described herein is used in treating a CNS disorder, wherein the CNS disorder to be treated is a demyelinating disease, including, but not limited to demyelinating myelinoclastic diseases and demyelinating leukostrophic disease.

In certain aspects, the disorder to be treated is a demyelinating myelonoclastic disease including, but not limited to, multiple sclerosis, neuromyelitis optica, neuromyelitis optica spectrum of disorders (NMOSD), idiopathic inflammatory demyelinating diseases (IIDD), anti-NMDA receptor encephalitis, acute disseminated encephalomyelitis, anti-MOG autoimmune encephalomyelitis, chronic relapsing inflammatory optic neuritis (CRION), acute disseminated encephalomyelitis (ADEM), immune-mediated encephalomyelitis, progressive multifocal leukoencephalopathy (PML); McDonalds-positive multiple sclerosis, acute hemorrhagic leukoencephalitis, Rasmussen's Encephalitis, Marburg multiple sclerosis, pseudotumefactive and tumefactive multiple sclerosis, Balo concentric sclerosis, diffuse myelinoclastic sclerosis, solitary sclerosis, multiple sclerosis with cavitary lesions, myelocortical multiple sclerosis (MCMS), atypical optic-spinal multiple sclerosis, pure spinal multiple sclerosis, HLA DRB3 *02:02 multiple sclerosis, autoimmune GFAP astrocytopathy, Chronic inflammatory demyelinating polyneuropathy (CIDP), Guillain-Barré syndrome, progressive inflammatory neuropathy, Lewis-Sumner Syndrome, combined central and peripheral demyelination (CCPD), Bickerstaff brainstem encephalitis, Fisher syndrome, trigeminal neuralgia, NMDAR anti-NMDA receptor encephalitis, primary progressive MS (PPMS), OPA1 variant multiple sclerosis, KIR4.1 multiple sclerosis, aquaporine-related multiple sclerosis, chronic cerebrospinal venous insufficiency (CCSVI or CCVI), diffuse sclerosis or Schilder's disease,

In certain aspects, the disorder to be treated is a demyelinating leukostrophic disease including, but not limited to, myelitis, central pontine myelinolysis (CPM), extrapontine myelinolysis, tabes dorsalis, progressive multifocal leukoencephalopathy, leukoencephalopathy with vanishing white matter, leukoencephalopathy with neuroaxonal spheroids, reversible posterior leukoencephalopathy syndrome, megalencephalic leukoencephalopathy with subcortical cysts, megalencephalic leukoencephalopathy with subcortical cysts 1, hypertensive leukoencephalopathy, Metachromatic leukodystrophy, Krabbe disease, Canavan disease, X-linked adrenoleukodystrophy, Alexander disease, cerebrotendineous xanthomatosis, Pelizaeus-Merzbacher disease, Refsum disease.

In some embodiments, an active compound as described herein is used to treat Buerger's disease, also known as thromboangiitis obliterans.

In some embodiments, an active compound as described herein is used to treat giant cell arteritis.

In some embodiments, an active compound as described herein is used to treat Raynaud's disease.

In certain aspects, the disorder to be treated is a demyelinating disease of the peripheral nervous system, including, but not limited to, Guillain-Barré syndrome and its chronic counterpart, chronic inflammatory demyelinating polyneuropathy, anti-MAG peripheral neuropathy, Charcot-Marie-Tooth disease and its counterpart Hereditary neuropathy with liability to pressure palsy, Copper deficiency-associated conditions (peripheral neuropathy, myelopathy, and rarely optic neuropathy), and progressive inflammatory neuropathy.

In certain aspects, an active compound or its salt or composition as described herein is used in treating a neurological inflammatory disorder. In embodiments, the disorder to be treated is cranial arteritis; giant cell arteritis; Holmes-Adie syndrome; inclusion body myositis (IBM); meningitis; neurologic paraneoplastic syndrome including, but not limited to, Lambert-Eaton myasthenic syndrome, stiff-person syndrome, encephalomyelitis (inflammation of the brain and spinal cord), myasthenia gravis, cerebellar degeneration, limbic and/or brainstem encephalitis, neuromyotonia, and opsoclonus (involving eye movement) and sensory neuropathy; polymyositis; transverse myelitis; vasculitis including temporal arteritis; arachnoiditis; Kinsbourne syndrome or opsoclonus myoclonus syndrome (OMS); or Saint Vitus Dance or sydenham chorea (SD) disease.

In some embodiments, an active compound or its salt or composition as described herein is used to treat transverse myelitis.

In certain aspects, the disorder to be treated is a peripheral neuropathy. In some embodiments, the peripheral neuropathy is a mononeuropathy. In some embodiments, the neuropathy is a polyneuropathy. In some embodiments, the polyneuropathy is distal axonopathy, diabetic neuropathy, a demyelinating polyneuropathy, small fiber peripheral neuropathy, mononeuritis multiplex, polyneuritis multiplex, autonomic neuropathy, or neuritis.

In some embodiments, an active compound or its salt or composition as described herein is used to treat an autoimmune vascular disease. In some embodiments, the autoimmune vascular disease is vasculitis. In some embodiments, the vasculitis includes, but is not limited to, autoimmune inflammatory vasculitis, Cutaneous small-vessel vasculitis, Granulomatosis with polyangiitis , Eosinophilic granulomatosis with polyangiitis, Behget's disease, Kawasaki disease, Buerger's disease, and "Limited" granulomatosis with polyangiitis vasculitis.

In some embodiments, an active compound or its salt or composition as described herein is used to treat an arteritis. In some embodiments, the arteritis includes, but is not limited to, giant cell arteritis, Takayasu arteritis, temporal arteritis, and polyarteritis nodosa.
In some embodiments, the complement-mediated disease or disorder comprises transplant rejection. In some embodiments, the complement-mediated disease or disorder is antibody-mediated transplant rejection.

In certain aspects, an active compound or its salt or composition as described herein is used to treat a proliferative disorder, including, but not limited to, cancer. Targeted cancers suitable for administration of an active compound or its salt described herein include, but are not limited to, estrogen-receptor positive cancer, HER2-negative advanced breast cancer, late-line metastatic breast cancer, liposarcoma, non-small cell lung cancer, liver cancer, ovarian cancer, glioblastoma, refractory solid tumors, retinoblastoma positive breast cancer as well as retinoblastoma positive endometrial, vaginal and ovarian cancers and lung and bronchial cancers, adenocarcinoma of the colon, adenocarcinoma of the rectum, central nervous system germ cell tumors, teratomas, estrogen receptor-negative breast cancer, estrogen receptor-positive breast cancer, familial testicular germ cell tumors, HER2-negative breast cancer, HER2-positive breast cancer, male breast cancer, ovarian immature teratomas, ovarian mature teratoma, ovarian monodermal and highly specialized teratomas, progesterone receptor-negative breast cancer, progesterone receptor-positive breast cancer, recurrent breast cancer, recurrent colon cancer, recurrent extragonadal germ cell tumors, recurrent extragonadal non-seminomatous germ cell tumor, recurrent extragonadal seminomas, recurrent malignant testicular germ cell tumors, recurrent melanomas, recurrent ovarian germ cell tumors, recurrent rectal cancer, stage III extragonadal non-seminomatous germ cell tumors, stage III extragonadal seminomas, stage III malignant testicular germ cell tumors, stage III ovarian germ cell tumors, stage IV breast cancers, stage IV colon cancers, stage IV extragonadal non-seminomatous germ cell tumors, stage IV extragonadal seminoma, stage IV melanomas, stage IV ovarian germ cell tumors, stage IV rectal cancers, testicular immature teratomas, testicular mature teratomas. In particular embodiments, the targeted cancers included estrogen-receptor positive, HER2-negative advanced breast cancer, late-line metastatic breast cancer, liposarcoma, non-small cell lung cancer, liver cancer, ovarian cancer, glioblastoma, refractory solid tumors, retinoblastoma positive breast cancer as well as retinoblastoma positive endometrial, vaginal and ovarian cancers and lung and bronchial cancers, metastatic colorectal cancer, metastatic melanoma with CDK4 mutation or amplification, or cisplatin-refractory, unresectable germ cell tumors, lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, fibrosarcoma, myxosarcoma, chondrosarcoma, osteosarcoma, chordoma, malignant fibrous histiocytoma, hemangiosarcoma, angiosarcoma, lymphangiosarcoma, Mesothelioma, leiomyosarcoma, rhabdomyosarcoma, squamous cell carcinoma; epidermoid carcinoma, malignant skin adnexal tumors, adenocarcinoma, hepatoma, hepatocellular carcinoma, renal cell carcinoma, hypernephroma, cholangiocarcinoma, transitional cell carcinoma, choriocarcinoma, seminoma, embryonal cell carcinoma, glioma anaplastic; glioblastoma multiforme, neuroblastoma, medulloblastoma, malignant meningioma, malignant schwannoma, neurofibrosarcoma, parathyroid carcinoma, medullary carcinoma of thyroid, bronchial carcinoid, pheochromocytoma, Islet cell carcinoma, malignant carcinoid, malignant paraganglioma, melanoma, Merkel cell neoplasm, cystosarcoma phylloide, salivary cancers, thymic carcinomas, bladder cancer, and Wilms tumor, a blood disorder or a hematologic malignancy, including, but not limited to, myeloid disorder, lymphoid disorder, leukemia, lymphoma, myelodysplastic syndrome (MDS), myeloproliferative disease (MPD), mast cell disorder, and myeloma (e.g., multiple myeloma), among others, T-cell or NK-cell lymphoma, for example, but not limited to: peripheral T-cell lymphoma; anaplastic large cell lymphoma, for example anaplastic lymphoma kinase (ALK) positive, ALK negative anaplastic large cell lymphoma, or primary cutaneous anaplastic large cell lymphoma; angioimmunoblastic lymphoma; cutaneous T-cell lymphoma, for example mycosis fungoides, Sézary syndrome, primary cutaneous anaplastic large cell lymphoma, primary cutaneous CD30+ T-cell lymphoproliferative disorder; primary cutaneous aggressive epidermotropic CD8+ cytotoxic T-cell lymphoma; primary cutaneous gamma-delta T-cell lymphoma; primary cutaneous small/medium CD4+ T-cell lymphoma, and lymphomatoid papulosis; Adult T-cell Leukemia/Lymphoma (ATLL); Blastic NK-cell Lymphoma; Enteropathy-type T-cell lymphoma; Hematosplenic gamma-delta T-cell Lymphoma; Lymphoblastic Lymphoma; Nasal NK/T-cell Lymphomas; Treatment-related T-cell lymphomas; for example lymphomas that appear after solid organ or bone marrow transplantation; T-cell prolymphocytic leukemia; T-cell large granular lymphocytic leukemia; Chronic lymphoproliferative disorder of NK-cells; Aggressive NK cell leukemia; Systemic EBV+ T-cell lymphoproliferative disease of childhood (associated with chronic active EBV infection); Hydroa vacciniforme-like lymphoma; Adult T-cell leukemia/ lymphoma; Enteropathy-associated T-cell lymphoma; Hepatosplenic T-cell lymphoma; or Subcutaneous panniculitis-like T-cell lymphoma. In some embodiments, the methods described herein can be used to treat a host, for example a human, with a lymphoma or lymphocytic or myelocytic proliferation disorder or abnormality. For example, the methods as described herein can be administered to a host with a Hodgkin Lymphoma or a Non-Hodgkin Lymphoma. For example, the host can have a Non-Hodgkin Lymphoma such as, but not limited to: an AIDS-Related Lymphoma; Anaplastic Large-Cell Lymphoma; Angioimmunoblastic Lymphoma; Blastic NK-Cell Lymphoma; Burkitt's Lymphoma; Burkitt-like Lymphoma (Small Non-Cleaved Cell Lymphoma); Chronic Lymphocytic Leukemia/Small Lymphocytic Lymphoma; Cutaneous T-Cell Lymphoma; Diffuse Large B-Cell Lymphoma; Enteropathy-Type T-Cell Lymphoma; Follicular Lymphoma; Hepatosplenic Gamma-Delta T-Cell Lymphoma; Lymphoblastic Lymphoma; Mantle Cell Lymphoma; Marginal Zone Lymphoma; Nasal T-Cell Lymphoma; Pediatric Lymphoma; Peripheral T-Cell Lymphomas; Primary Central Nervous System Lymphoma; T-Cell Leukemias; Transformed Lymphomas; Treatment-Related T-Cell Lymphomas; or Waldenstrom's Macroglobulinemia, a Hodgkin Lymphoma, such as, but not limited to: Nodular Sclerosis Classical Hodgkin's Lymphoma (CHL); Mixed Cellularity CHL; Lymphocyte-depletion CHL; Lymphocyte-rich CHL; Lymphocyte Predominant Hodgkin Lymphoma; or Nodular Lymphocyte Predominant HL, a specific B-cell lymphoma or proliferative disorder such as, but not limited to: multiple myeloma; Diffuse large B cell lymphoma; Follicular lymphoma; Mucosa-Associated Lymphatic Tissue lymphoma (MALT); Small cell lymphocytic lymphoma; Mediastinal large B cell lymphoma; Nodal marginal zone B cell lymphoma (NMZL); Splenic marginal zone lymphoma (SMZL); Intravascular large B-cell lymphoma; Primary effusion lymphoma; or Lymphomatoid granulomatosis; B-cell prolymphocytic leukemia; Hairy cell leukemia; Splenic lymphoma/leukemia, unclassifiable; Splenic diffuse red pulp small B-cell lymphoma; Hairy cell leukemia-variant; Lymphoplasmacytic lymphoma; Heavy chain diseases, for example, Alpha heavy chain disease, Gamma heavy chain disease, Mu heavy chain disease; Plasma cell myeloma; Solitary plasmacytoma of bone; Extraosseous plasmacytoma; Primary cutaneous follicle center lymphoma; T cell/histiocyte rich large B-cell lymphoma; DLBCL associated with chronic inflammation; Epstein-Barr virus (EBV)+ DLBCL of the elderly; Primary mediastinal (thymic) large B-cell lymphoma; Primary cutaneous DLBCL, leg type; ALK+ large B-cell lymphoma; Plasmablastic lymphoma; Large B-cell lymphoma arising in HHV8-associated multicentric; Castleman disease; B-cell lymphoma, unclassifiable, with features intermediate between diffuse large B-cell lymphoma; or B-cell lymphoma, unclassifiable, with features intermediate between diffuse large B-cell lymphoma and classical Hodgkin lymphoma, a leukemia, for example, an acute or chronic leukemia of a lymphocytic or myelogenous origin, such as, but not limited to: Acute lymphoblastic leukemia (ALL); Acute myelogenous leukemia (AML); Chronic lymphocytic leukemia (CLL); Chronic myelogenous leukemia (CML); juvenile myelomonocytic leukemia (JMML); hairy cell leukemia (HCL); acute promyelocytic leukemia (a subtype of AML); large granular lymphocytic leukemia; or Adult T-cell chronic leukemia. In some embodiments, the patient has an acute myelogenous leukemia, for example an undifferentiated AML (M0); myeloblastic leukemia (M1; with/without minimal cell maturation); myeloblastic leukemia (M2; with cell maturation); promyelocytic leukemia (M3 or M3 variant [M3V]); myelomonocytic leukemia (M4 or M4 variant with eosinophilia [M4E]); monocytic leukemia (M5); erythroleukemia (M6); or megakaryoblastic leukemia (M7), small cell lung cancer, retinoblastoma, HPV positive malignancies like cervical cancer and certain head and neck cancers, MYC amplified tumors such as Burkitts' Lymphoma, and triple negative breast cancer; certain classes of sarcoma, certain classes of non-small cell lung carcinoma, certain classes of melanoma, certain classes of pancreatic cancer, certain classes of leukemia, certain classes of lymphoma, certain classes of brain cancer, certain classes of colon cancer, certain classes of prostate cancer, certain classes of ovarian cancer, certain classes of uterine cancer, certain classes of thyroid and other endocrine tissue cancers, certain classes of salivary cancers, certain classes of thymic carcinomas, certain classes of kidney cancers, certain classes of bladder cancers, and certain classes of testicular cancers.

In certain aspects, an active compound or its salt as described herein can be used to preserve or prevent damage to an organ or blood product. For example, an active compound or its salt described herein can be used to prevent damage to an organ, tissue, cell product, or blood product, that has been harvested for transplantation. In some embodiments, the organ is the heart, kidney, pancreas, lung, liver, or intestine. In some embodiments, the tissue is derived from the cornea, bone, tendon, muscle, heart valve, nerve, artery or vein, or the skin. In some embodiments, the blood product is whole blood, plasma, red blood cells or reticulocytes.

In some embodiments, an active compound or its salt or composition as described herein prevents or delays the onset of at least one symptom of a complement-mediated disease or disorder in an individual. In some embodiment, an active compound or its salt or composition as described herein reduces or eliminates at least one symptom of a complement-mediated disease or disorder in an individual. Examples of symptoms include, but are not limited to, symptoms associated with autoimmune disease, cancer, hematological disease, infectious disease, inflammatory disease, ischemia-reperfusion injury, neurodegenerative disease, neurodegenerative disorder, renal disease, transplant rejection, ocular disease, vascular disease, or a vasculitis disorder. The symptom can be a neurological symptom, for example, impaired cognitive function, memory impairment, loss of motor function, etc. The symptom can also be the activity of factor D protein in a cell, tissue, or fluid of an individual. The symptom can also be the extent of complement activation in a cell, tissue, or fluid of an individual.

In some embodiments, administering an active compound or its salt or composition as described herein to an individual modulates complement activation in a cell, tissue, or fluid of an individual. In some embodiments, administration of an active compound or its salt or composition as described herein to an individual inhibits complement activation in a cell, tissue, or fluid of an individual. For example, in some embodiments, an active compound or its salt or composition as described herein, when administered in one or more doses as monotherapy or in combination therapy to an individual having a complement-mediated disease or disorder, inhibits complement activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to complement activation in the individual before treatment with the compounds described herein.

In some embodiments, an active compound or its salt or composition as described herein reduces C3 deposition onto red blood cells; for example, in some embodiments, an an active compound or its salt or composition as described herein reduces deposition of C3b, iC3b, etc., onto RBCs. In some embodiments, an active compound or its salt or composition as described herein inhibits complement-mediated red blood cell lysis.

In some embodiments, an active compound or its salt or composition as described herein reduces C3 deposition onto platelets; for example, in some embodiments, an active compound or its salt or composition as described herein reduces deposition of C3b, iC3b, etc., onto platelets.

In some embodiments, administering an active compound or its salt or composition as described herein results in an outcome selected from the group consisting of: (a) a reduction in complement activation; (b) an improvement in cognitive function; (c) a reduction in neuron loss; (d) a reduction in phospho-Tau levels in neurons; (e) a reduction in glial cell activation; (f) a reduction in lymphocyte infiltration; (g) a reduction in macrophage infiltration; (h) a reduction in antibody deposition, (i) a reduction in glial cell loss; (j) a reduction in oligodendrocyte loss; (k) a reduction in dendritic cell infiltration; (l) a reduction in neutrophil infiltration; (m) a reduction in red blood cell lysis; (n) a reduction in red blood cell phagocytosis; (o) a reduction in platelet phagocytosis; (p) a reduction in platelet lysis; (q) an improvement in transplant graft survival; (r) a reduction in macrophage mediated phagocytosis; (s) an improvement in vision; (t) an improvement in motor control; (u) an improvement in thrombus formation; (v) an improvement in clotting; (w) an improvement in kidney function; (x) a reduction in antibody mediated complement activation; (y) a reduction in autoantibody mediated complement activation; (z) an improvement in anemia; (aa) reduction of demyelination; (ab) reduction of eosinophilia; (ac) a reduction of C3 deposition on red blood cells (e.g., a reduction of deposition of C3b, iC3b, etc., onto RBCs); and (ad) a reduction in C3 deposition on platelets (e.g., a reduction of deposition of C3b, iC3b, etc., onto platelets); and (ae) a reduction of anaphylatoxin toxin production; (af) a reduction in autoantibody mediated blister formation; (ag) a reduction in autoantibody induced pruritis; (ah) a reduction in autoantibody induced erythematosus; (ai) a reduction in autoantibody mediated skin erosion; (aj) a reduction in red blood cell destruction due to transfusion reactions; (ak) a reduction in red blood cell lysis due to alloantibodies; (al) a reduction in hemolysis due to transfusion reactions; (am) a reduction in allo-antibody mediated platelet lysis; (an) a reduction in platelet lysis due to transfusion reactions; (ao) a reduction in mast cell activation; (ap) a reduction in mast cell histamine release; (aq) a reduction in vascular permeability; (ar) a reduction in edema; (as) a reduction in complement deposition on transplant graft endothelium; (at) a reduction of anaphylatoxin generation in transplant graft endothelium; (au) a reduction in the separation of the dermal-epidermal junction; (av) a reduction in the generation of anaphylatoxins in the dermal-epidermal junction; (aw) a reduction in alloantibody mediated complement activation in transplant graft endothelium; (ax) a reduction in antibody mediated loss of the neuromuscular junction; (ay) a reduction in complement activation at the neuromuscular junction; (az) a reduction in anaphylatoxin generation at the neuromuscular junction; (ba) a reduction in complement deposition at the neuromuscular junction; (bb) a reduction in paralysis; (bc) a reduction in numbness; (bd) increased bladder control; (be) increased bowel control; (bf) a reduction in mortality associated with autoantibodies; and (bg) a reduction in morbidity associated with autoantibodies.

In some embodiments, an active compound or its salt or composition as described herein, when administered in one or more doses as monotherapy or in combination therapy to an individual having a complement-mediated disease or disorder, is effect to achieve a reduction of at least about 10%, at least about 15%, at least about 20%, at least about 25%, at least about 30%, at least about 40%, at least about 50%, at least about 60%, at least about 70%, at least about 80%, at least about 90%, or more than 90%, of one or more of the following outcomes: (a) complement activation; (b) decline in cognitive function; (c) neuron loss; (d) phospho-Tau levels in neurons; (e) glial cell activation; (f) lymphocyte infiltration; (g) macrophage infiltration; (h) antibody deposition, (i) glial cell loss; (j) oligodendrocyte loss; (k) dendritic cell infiltration; (l) neutrophil infiltration; (m) red blood cell lysis; (n) red blood cell phagocytosis; (o) platelet phagocytosis; (p) platelet lysis; (q) transplant graft rejection; (r) macrophage mediated phagocytosis; (s) vision loss; (t) antibody mediated complement activation; (u) autoantibody mediated complement activation; (v) demyelination; (w) eosinophilia; compared to the level or degree of the outcome in the individual before treatment with the active compound.

In some embodiments, an active compound or its salt or composition as described herein, when administered in one or more doses as monotherapy or in combination therapy to an individual having a complement-mediated disease or disorder, is effect to achieve an improvement of at least about 10%, at least about 15%, at least about 20%, at least about 25%, at least about 30%, at least about 40%, at least about 50%, at least about 60%, at least about 70%, at least about 80%, at least about 90%, or more than 90%, of one or more of the following outcomes: a) cognitive function; b) transplant graft survival; c) vision; d) motor control; e) thrombus formation; f) clotting; g) kidney function; and h) hematocrit (red blood cell count), compared to the level or degree of the outcome in the individual before treatment with the active compound.

In some embodiments, administering an active compound or its salt or composition as described herein to an individual reduces complement activation in the individual. For example, in some embodiments, an active compound or its salt or composition as described herein, when administered in one or more doses as monotherapy or in combination therapy to an individual having a complement-mediated disease or disorder, reduces complement activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to complement activation in the individual before treatment with the active compound or its salt.

In some embodiments, administering an active compound or its salt or composition as described herein improves cognitive function in the individual. For example, in some embodiments, an active compound described herein, when administered in one or more doses as monotherapy or in combination therapy to an individual having a complement-mediated disease or disorder, improves cognitive function in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the cognitive function in the individual before treatment with the active compound.

In some embodiments, administering an active compound or its salt or composition as described herein reduces the rate of decline in cognitive function in the individual. For example, in some embodiments, an active compound or its salt, when administered in one or more doses as monotherapy or in combination therapy to an individual having a complement-mediated disease or disorder, reduces the rate of decline of cognitive function in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the rate of decline in cognitive function in the individual before treatment with the active compound or its salt.

In some embodiments, administering an active compound or its salt or composition as described herein to an individual reduces neuron loss in the individual. For example, in some embodiments, an active compound or its salt, when administered in one or more doses as monotherapy or in combination therapy to an individual having a complement-mediated disease or disorder, reduces neuron loss in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to neuron loss in the individual before treatment with the active compound.

In some embodiments, administering an active compound or its salt or composition as described herein to an individual reduces phospho-Tau levels in the individual. For example, in some embodiments, an active compound or its salt, when administered in one or more doses as monotherapy or in combination therapy to an individual having a complement-mediated disease or disorder, reduces phospho-Tau in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the phospho-Tau level in the individual before treatment with the active compound or its salt.

In some embodiments, administering an active compound or its salt or composition as described herein to an individual reduces glial cell activation in the individual. For example, in some embodiments, an active compound or its salt, when administered in one or more doses as monotherapy or in combination therapy to an individual having a complement-mediated disease or disorder, reduces glial activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to glial cell activation in the individual before treatment with the active compound or its salt. In some embodiments, the glial cells are astrocytes or microglia.

In some embodiments, administering an active compound or its salt or composition as described herein to an individual reduces lymphocyte infiltration in the individual. For example, in some embodiments, an active compound or its salt, when administered in one or more doses as monotherapy or in combination therapy to an individual having a complement-mediated disease or disorder, reduces lymphocyte infiltration in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to lymphocyte infiltration in the individual before treatment with the active compound or its salt.

In some embodiments, administering an active compound or its salt or composition as described herein to an individual reduces macrophage infiltration in the individual. For example, in some embodiments, an active compound or its salt, when administered in one or more doses as monotherapy or in combination therapy to an individual having a complement-mediated disease or disorder, reduces macrophage infiltration in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to macrophage infiltration in the individual before treatment with the active compound or its salt.

In some embodiments, administering an active compound or its salt or composition as described herein to an individual reduces antibody deposition in the individual. For example, in some embodiments, an active compound or its salt, when administered in one or more doses as monotherapy or in combination therapy to an individual having a complement-mediated disease or disorder, reduces antibody deposition in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to antibody deposition in the individual before treatment with the active compound or its salt.

In some embodiments, administering an active compound or its salt or composition as described herein to an individual reduces anaphylatoxin (e.g., C3a, C4a, C5a) production in an individual. For example, in some embodiments, an active compound or its salt, when administered in one or more doses as monotherapy or in combination therapy to an individual having a complement-mediated disease or disorder, reduces anaphylatoxin production in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the level of anaphylatoxin production in the individual before treatment with the active compound or its salt.
The present disclosure provides for use of an active compound or its salt of the present disclosure or a pharmaceutical composition comprising an active compound or its salt of the present disclosure and a pharmaceutically acceptable excipient to treat an individual having a complement-mediated disease or disorder. In some embodiments, the present disclosure provides for use of an active compound or its salt of the present disclosure to treat an individual having a complement-mediated disease or disorder. In some embodiments, the present disclosure provides for use of a pharmaceutical composition comprising an active compound or its salt of the present disclosure and a pharmaceutically acceptable excipient to treat an individual having a complement-mediated disease or disorder.

### COMBINATION THERAPY

In some embodiments an active compound or its salt or composition as described herein may be provided in combination or alternation with or preceded by, concomitant with or followed by, an effective amount of at least one additional therapeutic agent, for example, for use in treatment of a disorder listed herein. Non-limiting examples of second active agents for such combination therapy are provided below.

In some embodiments, an active compound or its salt or composition as described herein may be provided in combination or alternation with at least one additional inhibitor of the complement system or a second active compound with a different biological mechanism of action. In the description below and herein generally, whenever any of the terms referring to an active compound or its salt or composition as described herein are used, it should be understood that pharmaceutically acceptable salts or compositions are considered included, unless otherwise stated or inconsistent with the text.

In non-limiting embodiments, an active compound or its salt or composition as described herein may be provided together with a protease inhibitor, a soluble complement regulator, a therapeutic antibody (monoclonal or polyclonal), complement component inhibitor, receptor agonist, or siRNA.

In other embodiments, an active compound described herein is administered in combination or alternation with an antibody against tumor necrosis factor (TNF), including but not limited to infliximab (Remicade), adalimumab, certolizumab, golimumab, or a receptor fusion protein such as etanercept (Embrel).

In another embodiment, an active compound as described herein can be administered in combination or alternation with an anti-CD20 antibody, including but not limited to rituximab (Rituxan), adalimumab (Humira), ofatumumab (Arzerra), tositumomab (Bexxar), obinutuzumab (Gazyva), or ibritumomab (Zevalin).

In an alternative embodiment, an active compound as described herein can be administered in combination or alternation with an anti-IL6 antibody, including but not limited to tocilizumab (Actemra) and siltuximab (Sylvant).

In an alternative embodiment, an active compound as described herein can be administered in combination or alternation with an IL17 inhibitor, including but not limited to secukibumab (Cosentyx).

In an alternative embodiment, an active compound as described herein can be administered in combination or alternation with a p40 (IL12/IL23) inhibitor, including but not limited to ustekinumab (Stelara).

In an alternative embodiment, an active compound as described herein can be administered in combination or alteration with an IL23 inhibitor, including but not limited to risankizumab.

In an alternative embodiment, an active compound as described herein can be administered in combination or alteration with an anti-interferon α antibody, for example but not limited to sifalimumab.

In an alternative embodiment, an active compound as described herein can be administered in combination or alteration with a kinase inhibitor, for example but not limited to a JAK1/JAK3 inhibitor, for example but not limited to tofacitinib (Xelianz). In an alternative embodiment, an active compound as described herein can be administered in combination or alteration with a JAK1/JAK2 inhibitor, for example but not limited to baracitibib.

In an alternative embodiment, an active compound as described herein can be administered in combination or alteration with an anti-VEGF agent, for example but not limited to: aflibercept (Eylea^{®}; Regeneron Pharmaceuticals); ranibizumab (Lucentis^{®}: Genentech and Novartis); pegaptanib (Macugen^{®}; OSI Pharmaceuticals and Pfizer); bevacizumab (Avastin; Genentech/Roche); lapatinib (Tykerb); sunitinib (Sutent); axitinib (Inlyta); pazopanib; sorafenib (Nexavar); ponatinib (Inclusig); regorafenib (Stivarga); cabozantinib (Abometyx; Cometriq); vendetanib (Caprelsa); ramucirumab (Cyramza); lenvatinib (Lenvima); ziv-aflibercept (Zaltrap); cediranib (Recentin); anecortane acetate, squalamine lactate, and corticosteroids.

In another embodiment, an active compound as described herein can be administered in combination or alternation with an immune checkpoint inhibitor. Non-limiting examples of checkpoint inhibitors include anti-PD-1 or anti-PDL1 antibodies, for example, nivolumab (Opdivo), pembrolizumab (Keytruda), pidilizumab, AMP-224 (AstraZeneca and MedImmune), PF-06801591 (Pfizer), MEDI0680 (AstraZeneca), PDR001 (Novartis), REGN2810 (Regeneron), SHR-12-1 (Jiangsu Hengrui Medicine Company and Incyte Corporation), TSR-042 (Tesaro), and the PD-L1/VISTA inhibitor CA-170 (Curis Inc.), atezolizumab, durvalumab, and KN035, or anti-CTLA4 antibodies , for example Ipilimumab, Tremelimumab, AGEN1884 and AGEN2041 (Agenus).

Non-limiting examples of active agents that can be used in combination with active compounds described herein are:
Protease inhibitors: plasma-derived C1-INH concentrates, for example **Cetor**^{®} (Sanquin), **Berinert-P**^{®} (CSL Behring, Lev Pharma), and Cinryze^{®}; recombinant human C1-inhibitors, for example Rhucin^{®}; ritonavir (Norvir^{®}, Abbvie, Inc.);
Soluble complement regulators: **Soluble complement receptor 1 (TP10)** (Avant Immunotherapeutics); sCR1-sLe^{X}/TP-20 (Avant Immunotherapeutics); **MLN-2222** /**CAB-2** (Millenium Pharmaceuticals); Mirococept (Inflazyme Pharmaceuticals); Therapeutic antibodies: Eculizumab/Soliris and Ravulizumab/Ultomiris (Alexion Pharmaceuticals); Pexelizumab (Alexion Pharmaceuticals); Ofatumumab (Genmab A/S); TNX-234 (Tanox); TNX-558 (Tanox); TA106 (Taligen Therapeutics); Neutrazumab (G2 Therapies); Anti-properdin (Novelmed Therapeutics); HuMax-CD38 (Genmab A/S);
Complement component inhibitors: Compstatin/POT-4 (Potentia Pharmaceuticals); ARC1905 (Archemix); 4(1MEW)APL-1,APL-2 (Appelis); CP40/AMY-101,PEG-Cp40 (Amyndas);
PDGF inhibitors: Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; Anti-factor H or anti-factor B agents: Anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas);
Complement C3 or CAP C3 Convertase targeting molecules: TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH; Anti-CR3, anti-MASP2, anti C1s, and anti-C1n molecules: Cynryze (ViroPharma/Baxter); TNT003 (True North); OMS721 (Omeros); OMS906 (Omeros); and Imprime PGG (Biothera);
Receptor agonists: PMX-53 (Peptech Ltd.); JPE-137 (Jerini); JSM-7717 (Jerini);
Others: Recombinant human MBL (rhMBL; Enzon Pharmaceuticals); Imides and glutarimide derivatives such as thalidomide, lenalidomide, pomalidomide; Additional non-limiting examples that can be used in combination or alternation with an active compound or its salt or composition as described herein include the following.

| **Non-limiting examples for combination therapy** | | | |
|---|---|---|---|
| **Name** | **Target** | **Company** | **Class of Molecule** |
| LFG316 | C5 | Novartis/Morphosys | Monoclonal antibody |
| SB12 | C5 | Samsung Bioepsis | |
| IONIS-FB-LRx | CFB | Ionis | Antisense Inhibitor |
| 4(1MEW)APL-1,APL-2 | C3/C3b | Apellis | Compstatin Family |
| 4(1MeW)POT-4 | C3/C3b | Potentia | Compstatin Family |
| Anti-C5 siRNA | C5 | Alnylam | Si-RNA |
| Anti-FB siRNA | CFB | Alnylam | SiRNA |
| ARC1005 | C5 | Novo Nordisk | Aptamers |
| ATA | C5 | N.A. | Chemical |
| Coversin | C5 | Volution Immuno-Pharmaceuticals | Small animal protein |
| CP40/AMY-101,PEG-Cp40 | C3/C3b | Amyndas | Compstatin Family |
| CRIg/CFH | CAP C3 convertase | NA | CFH-based protein |
| Cynryze | C1n/C1s | ViroPharma/Baxter | Human purified protein |
| FCFD4514S | CFD | Genentech/Roche | Monoclonal antibody |
| H17 | C3 (C3b/iC3b) | EluSys Therapeutics | Monoclonal antibody |
| Mini-CFH | CAP C3 convertase | Amyndas | CFH-based protein |
| Mirococept (APT070) | CAP and CCP C3 | NA | CR1-based protein |
| Mubodine | C5 | Adienne | Monoclonal antibody |
| RA101348 | C5 | Rapharma | Small molecule |
| sCR1 (CDX-1135) | CAP and CP C3 | Celldex | CR1-based protein |
| SOBI002 | C5 | Swedish Orphan Biovitrum | Affibody |
| SOMAmers | C5 | SomaLogic | Aptamers |
| SOMAmers | CFB and CFD | SomaLogic | Aptamers (SELEX) |
| TA106 | CFB | Alexion Pharmaceuticals | Monoclonal antibody |
| TNT003 | C1s | True North | Monoclonal antibody |
| TT30 (CR2/CFH) | CAP C3 convertase | Alexion | CFH-based protein |
| TT32 (CR2/CR1) | CAP and CCP C3 | Alexion Pharmaceuticals | CR1-based protein |
| Nafamostat (FUT-175, Futhan) | C1s, CFD, other proteases | Torri Pharmaceuticals | Small molecule |
| OMS721 | MASP-2 | Omeros | Monoclonal antibody |
| OMS906 | MASP-2 | Omeros | Monoclonal antibody |
| Bikaciomab, NM9308 | CFB | Novelmed | Monoclonal antibody |
| NM9401 | Properdin | Novelmed | Monoclonal antibody |
| CVF, HC-1496 | C3 | InCode | Recombinant peptide |
| ALXN1102/ALXN1103 (TT30) | C3-conv, C3b | Alexion Pharmaceuticals | Regulator |
| rFH | C3-conv, C3b | Optherion | Regulator |
| 5C6, AMY-301 | CFH | Amyndas | Regulator |
| Erdigna | C5 | Adienne Pharma | Antibody |
| ARC1905 | C5 | Opthotech | Monoclonal Antibody |
| MEDI7814 | C5/C5a | MedImmune | Monoclonal Antibody |
| NOX-D19 | C5a | Noxxon | Aptamer (Spiegelmer) |
| IFX-1, CaCP29 | C5a | InflaRx | Monoclonal Antibody |
| PMX53, PMX205 | C5aR | Cephalon, Teva | Peptidomimetic |
| CCX168 | C5aR | ChemoCentryx | Small molecule |
| ADC-1004 | C5aR | Alligator Bioscience | Small molecule |
| Anti-C5aR-151, NN8209; Anti-C5aR-215, NN8210 | C5aR | Novo Nordisk | Monoclonal Antibody |
| Imprime PGG | CR3 | Biothera | Soluble beta-glucan |
| ANX005; ANX007 | C1q | Annexon | Monoclonal Antibody |
| Lampalizumab | fD | Roche | Monoclonal Antibody |
| avacincaptad pegol | C5 | Opthotech | Aptamer |
| regenemab | C6 | Regenesance | Monoclonal Antibody |
| BIVV020 | C1s | Bioverativ | Monoclonal Antibody |
| PRO-02 | C2 | Broteio/Argen-x | Monoclonal Antibody |
| 5C6, compsorbin | fH | Amyndas | Peptide |
| SOBI005 | C5 | Sobi | Protein |
| ISU305 | C5 | ISU ABXIS | Monoclonal Antibody |
| Mubodina | C5 | Adienne | Monoclonal Antibody |
| IFX-2, IFX-3 | C5a | InflaRx | Monoclonal Antibody |
| ALS-205 | C5aR1 | Alsonex | Peptide |
| DF2593A | C5aR1 | Dompé | Small Molecule |
| IPH5401 | C5aR1 | Innate Pharma | Monoclonal Antibody |
| C6-LNA | C6 | Regenesance | Oligonucleotide |
| SKY59 | C5 | Roche | Monoclonal Antibody |
| REGN3918 | C5 | Regeneron | Monoclonal Antibody |
| Aptamers to Factor D | fD | Vitrisa Therapeutics | Aptamer |
| CLG561 | Properdin | Novartis | Monoclonal Antibody |
| Tesidolumab; LFG316 | C5 | Novartis and MorphoSys | Monoclonal Antibody |

In one embodiment the agent for combination therapy is a biosimilar of any agent named in the above table.

In some embodiments, an active compound or its salt or composition as described herein may be provided together with a compound that inhibits an enzyme that metabolizes an administered protease inhibitor. In some embodiments, a compound or salt may be provided together with ritonavir.

In some embodiments, an active compound or its salt or composition as described herein may be provided in combination with a complement C5 inhibitor or C5 convertase inhibitor. In another embodiment, an active compound or its salt or composition as described herein may be provided in combination with eculizumab, a monoclonal antibody directed to the complement factor C5 and manufactured and marketed by Alexion Pharmaceuticals under the tradename Soliris. Eculizumab has been approved by the U.S. FDA for the treatment of PNH and aHUS. In another embodiment, an active compound or its salt or composition as described herein may be provided in combination with ravulizumab, a monoclonal antibody directed to the complement factor C5 and manufactured and marketed by Alexion Pharmaceuticals under the tradename Ultomiris.

In some embodiments, an active compound or its salt or composition as described herein may be provided together with a compound that inhibits Complement Factor D. In some embodiments of the invention, an active compound or its salt or composition as described herein as described herein can be used in combination or alternation with a compound described in Biocryst Pharmaceuticals US Pat. No. 6,653,340 titled "Compounds useful in the complement, coagulate and kallikrein pathways and method for their preparation" describes fused bicyclic ring compounds that are potent inhibitors of Factor D; Novartis PCT patent publication WO2012/093101 titled "Indole compounds or analogs thereof useful for the treatment of age-related macular degeneration" describes certain Factor D inhibitors; Novartis PCT patent publications WO2013/164802, WO2013/192345, WO2014/002051, WO2014/002052, WO2014/002053, WO2014/002054, WO2014/002057, WO2014/002058, WO2014/002059, WO2014/005150, WO2014/009833, WO2014/143638, WO2015/009616, WO2015/009977, WO2015/066241, Bristol-Myers Squibb PCT patent publication WO2004/045518_titled "Open chain prolyl urea-related modulators of androgen receptor function"; Japan Tobacco Inc. PCT patent publication WO1999/048492 titled "Amide derivatives and nociceptin antagonists"; Ferring B.V. and Yamanouchi Pharmaceutical Co. LTD. PCT patent publication WO1993/020099 titled "CCK and/or gastrin receptor ligands"; Alexion Pharmaceuticals PCT patent publication WO1995/029697 titled "Methods and compositions for the treatment of glomerulonephritis and other inflammatory diseases"; or Achillion Pharmaceuticals filed PCT Patent Application No. PCT/US2015/017523 and U.S. Patent Application No. 14/631,090 titled "Alkyne Compounds for Treatment of Complement Mediated Disorders"; PCT Patent Application No. PCT/US2015/017538 and U.S. Patent Application No. 14/631,233 titled "Amide Compounds for Treatment of Complement Mediated Disorders"; PCT Patent Application No. PCT/US2015/017554 and U.S. Patent Application No. 14/631,312 titled "Amino Compounds for Treatment of Complement Mediated Disorders"; PCT Patent Application No. PCT/US2015/017583 and U.S. Patent Application No. 14/631,440 titled "Carbamate, Ester, and Ketone Compounds for Treatment of Complement Mediated Disorders"; PCT Patent Application No. PCT/US2015/017593 and U.S. Patent Application No. 14/631,625 titled "Aryl, Heteroaryl, and Heterocyclic Compounds for Treatment of Complement Mediated Disorders"; PCT Patent Application No. PCT/US2015/017597 and U.S. Patent Application No. 14/631,683 titled "Ether Compounds for Treatment of Complement Mediated Disorders"; PCT Patent Application No. PCT/US2015/017600 and U.S. Patent Application No. 14/631,785 titled "Phosphonate Compounds for Treatment of Complement Mediated Disorders"; and PCT Patent Application No. PCT/US2015/017609 and U.S. Patent Application No. 14/631,828 titled "Compounds for Treatment of Complement Mediated Disorders."

In some embodiments, an active compound or its salt or composition as described herein is administered in combination with an anti-inflammatory drug, antimicrobial agent, anti-angiogenesis agent, immunosuppressant, antibody, steroid, ocular antihypertensive drug or combinations thereof. Examples of such agents include amikacin, anecortane acetate, anthracenedione, anthracycline, an azole, amphotericin B, bevacizumab, camptothecin, cefuroxime, chloramphenicol, chlorhexidine, chlorhexidine digluconate, clortrimazole, a clotrimazole cephalosporin, corticosteroids, dexamethasone, desamethazone, econazole, eftazidime, epipodophyllotoxin, fluconazole, flucytosine, fluoropyrimidines, fluoroquinolines, gatifloxacin, glycopeptides, imidazoles, itraconazole, ivermectin, ketoconazole, levofloxacin, macrolides, miconazole, miconazole nitrate, moxifloxacin, natamycin, neomycin, nystatin, ofloxacin, polyhexamethylene biguanide, prednisolone, prednisolone acetate, pegaptanib, platinum analogs, polymicin B, propamidine isethionate, pyrimidine nucleoside, ranibizumab, squalamine lactate, sulfonamides, triamcinolone, triamcinolone acetonide, triazoles, vancomycin, anti-vascular endothelial growth factor (VEGF) agents, VEGF antibodies, VEGF antibody fragments, vinca alkaloid, timolol, betaxolol, travoprost, latanoprost, bimatoprost, brimonidine, dorzolamide, acetazolamide, pilocarpine, ciprofloxacin, azithromycin, gentamycin, tobramycin, cefazolin, voriconazole, gancyclovir, cidofovir, foscarnet, diclofenac, nepafenac, ketorolac, ibuprofen, indomethacin, fluoromethalone, rimexolone, anecortave, cyclosporine, methotrexate, tacrolimus, anti-PDGFR molecule, and combinations thereof.

In some embodiments of the present invention, an active compound or its salt or composition as described herein can be administered in combination or alternation with at least one immunosuppressive agent. The immunosuppressive agent as non-limiting examples, may be a calcineurin inhibitor, e.g. a cyclosporin or an ascomycin, e.g. Cyclosporin A (NEORAL^{®}), FK506 (tacrolimus), pimecrolimus, a mTOR inhibitor, e.g. rapamycin or a derivative thereof, e.g. Sirolimus (RAPAMUNE^{®}), Everolimus (Certican^{®}), temsirolimus, zotarolimus, biolimus-7, biolimus-9, a rapalog, e.g.ridaforolimus, azathioprine, campath 1H, a S1P receptor modulator, e.g. fingolimod or an analog thereof, an anti IL-8 antibody, mycophenolic acid or a salt thereof, e.g. sodium salt, or a prodrug thereof, e.g. Mycophenolate Mofetil (CELLCEPT^{®}), OKT3 (ORTHOCLONE OKT3^{®}), Prednisone, ATGAM^{®}, THYMOGLOBULIN^{®}, Brequinar Sodium, OKT4, T10B9.A-3A, 33B3.1, 15-deoxyspergualin, tresperimus, Leflunomide ARAVA^{®}, CTLAI-Ig, anti-CD25, anti-IL2R, Basiliximab (SIMULECT^{®}), Daclizumab (ZENAPAX^{®}), mizorbine, methotrexate, dexamethasone, ISAtx-247, SDZ ASM 981 (pimecrolimus, Elidel^{®}), CTLA4lg (Abatacept), belatacept, LFA3lg, etanercept (sold as Enbrel^{®} by Immunex), adalimumab (Humira^{®}), infliximab (Remicade^{®}), an anti-LFA-1 antibody, natalizumab (Antegren^{®}), Enlimomab, gavilimomab, antithymocyte immunoglobulin, siplizumab, Alefacept efalizumab, pentasa, mesalazine, asacol, codeine phosphate, benorylate, fenbufen, naprosyn, diclofenac, etodolac and indomethacin, tocilizumab (Actemra), siltuximab (Sylvant), secukibumab (Cosentyx), ustekinumab (Stelara), risankizumab, sifalimumab, aspirin and ibuprofen.

Examples of anti-inflammatory agents include methotrexate, dexamethasone, dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate, lodoxamide tromethamine, aspirin, ibuprofen, suprofen, piroxicam, meloxicam, flubiprofen, naproxan, ketoprofen, tenoxicam, diclofenac sodium, ketotifen fumarate, diclofenac sodium, nepafenac, bromfenac, flurbiprofen sodium, suprofen, celecoxib, naproxen, rofecoxib, glucocorticoids, diclofenac, and any combination thereof. In some embodiments, an active compound or its salt or composition as described herein is combined with one or more non-steroidal anti-inflammatory drugs (NSAIDs) selected from naproxen sodium (Anaprox), celecoxib (Celebrex), sulindac (Clinoril), oxaprozin (Daypro), salsalate (Disalcid), diflunisal (Dolobid), piroxicam (Feldene), indomethacin (Indocin), etodolac (Lodine), meloxicam (Mobic), naproxen (Naprosyn), nabumetone (Relafen), ketorolac tromethamine (Toradol), naproxen/esomeprazole (Vimovo), and diclofenac (Voltaren), and combinations thereof.

In some embodiments, an active compound or its salt or composition as described herein is administered in combination or alteration with an omega-3 fatty acid or a peroxisome proliferator-activated receptor (PPARs) agonist. Omega-3 fatty acids are known to reduce serum triglycerides by inhibiting DGAT and by stimulating peroxisomal and mitochondrial beta oxidation. Two omega-3 fatty acids, eicosapentaenoic acid (EPA) and docosahexaenoic acid (DHA), have been found to have high affinity for both PPAR-alpha and PPAR-gamma. Marine oils, e.g., fish oils, are a good source of EPA and DHA, which have been found to regulate lipid metabolism. Omega-3 fatty acids have been found to have beneficial effects on the risk factors for cardiovascular diseases, especially mild hypertension, hypertriglyceridemia and on the coagulation factor VII phospholipid complex activity. Omega-3 fatty acids lower serum triglycerides, increase serum HDL- cholesterol, lower systolic and diastolic blood pressure and the pulse rate, and lower the activity of the blood coagulation factor VII-phospholipid complex. Further, omega-3 fatty acids seem to be well tolerated, without giving rise to any severe side effects. One such form of omega-3 fatty acid is a concentrate of omega-3, long chain, polyunsaturated fatty acids from fish oil containing DHA and EPA and is sold under the trademark Omacor^{®}. Such a form of omega-3 fatty acid is described, for example, in U.S. Patent Nos. 5,502,077, 5,656,667 and 5,698,594.

Peroxisome proliferator-activated receptors (PPARs) are members of the nuclear hormone receptor superfamily ligand-activated transcription factors that are related to retinoid, steroid and thyroid hormone receptors. There are three distinct PPAR subtypes that are the products of different genes and are commonly designated PPAR-alpha, PPAR-beta/delta (or merely, delta) and PPAR-gamma. General classes of pharmacological agents that stimulate peroxisomal activity are known as PPAR agonists, e.g., PPAR-alpha agonists, PPAR-gamma agonists and PPAR-delta agonists. Some pharmacological agents are combinations of PPAR agonists, such as alpha/gamma agonists, etc., and some other pharmacological agents have dual agonist/antagonist activity. Fibrates such as fenofibrate, bezafibrate, clofibrate and gemfibrozil, are PPAR-alpha agonists and are used in patients to decrease lipoproteins rich in triglycerides, to increase HDL and to decrease atherogenic-dense LDL. Fibrates are typically orally administered to such patients. Fenofibrate or 2-[4-(4-chlorobenzoyl)phenoxy]-2-methyl-propanoic acid, 1-methylethyl ester, has been known for many years as a medicinally active principle because of its efficacy in lowering blood triglyceride and cholesterol levels.

In some embodiments, the present invention provides an active compound or its salt or composition as described herein in combination with an anti-VEGF agent for use in treating or preventing age-related macular degeneration (AMD) by administering to a subject in need thereof an effective amount of the active compound or its salt or composition in combination with the anti-VEGF agent. Non-limiting examples of anti-VEGF agents include, but are not limited to, aflibercept (Eylea^{®}; Regeneron Pharmaceuticals); ranibizumab (Lucentis^{®}: Genentech and Novartis); pegaptanib (Macugen^{®}; OSI Pharmaceuticals and Pfizer); bevacizumab (Avastin; Genentech/Roche); lapatinib (Tykerb); sunitinib (Sutent); axitinib (Inlyta); pazopanib; sorafenib (Nexavar); ponatinib (Inclusig); regorafenib (Stivarga); Cabozantinib (Abometyx; Cometriq); vendetanib (Caprelsa); ramucirumab (Cyramza); lenvatinib (Lenvima); ziv-aflibercept (Zaltrap); cediranib (Recentin); anecortane acetate, squalamine lactate, and corticosteroids, including, but not limited to, triamcinolone acetonide.

In some embodiments, the present invention provides an active compound or its salt or composition as described herein in combination with a complement C5 inhibitor for use in treating or preventing age-related macular degeneration (AMD) by administering to a subject in need thereof an effective amount of the active compound or its salt or composition in combination with the complement C5 inhibitor, for example, a complement C5 inhibitor described herein and in the table above titled Non-limiting examples of potential therapeutics for combination therapy, including, but not limited to, eculizumab; ravulizumab; LFG316 (Novartis/Morphosys); Anti-C5 siRNA (Alnylam); ARC1005 (Novo Nordisk); Coversin (Volution Immuno-Pharmaceuticals); Mubodine (Adienne Pharma); RA101348 (Ra Pharma); SOBI002 (Swedish Orphan Biovitrum); SOMAmers (SomaLogic); Erdigna (Adienne Pharma); ARC1905 (Opthotech); MEDI7814 (MedImmune); NOX-D19 (Noxxon); IFX-1, CaCP29 (InflaRx); PMX53, PMX205 (Cephalon, Teva); CCX168 (ChemoCentryx); ADC-1004 (Alligator Bioscience); and Anti-C5aR-151, NN8209; Anti-C5aR-215, NN8210 (Novo Nordisk).

In some embodiments, the present invention provides an active compound or its salt or composition as described herein in combination with an anti-properidin agent for use in treating or preventing age-related macular degeneration (AMD) by administering to a subject in need thereof an effective amount of the active compound or its salt or composition in combination with an anti-properidin agent, for example, an anti-properidin agent as described above, including but not limited to NM9401 (Novelmed).

In some embodiments, the present invention provides an active compound or its salt or composition as described herein in combination with a complement C3 inhibitor for use in treating or preventing age-related macular degeneration (AMD) by administering to a subject in need thereof an effective amount of the active compound or its salt or composition in combination with a complement C3 inhibitor for example, a complement C3 inhibitor described above, including, but not limited to, a compstatin or compstatin analog, for example Compstatin/POT-4 (Potentia Pharmaceuticals); ARC1905 (Archemix); 4(1MEW)APL-1,APL-2 (Appelis); CP40/AMY-101,PEG-Cp40 (Amyndas) Complement C3 or CAP C3 Convertase targeting molecules: TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); and CRIg/CFH.

In some embodiments, the present invention provides an active compound or its salt or composition as described herein for use in treating or preventing age-related macular degeneration (AMD) by administering to a subject in need thereof an effective amount of the active compound or its salt or composition in combination with an anti-factor H or anti-factor B agent selected from Anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas).

In some embodiments, the present invention provides an active compound or its salt or composition as described herein in combination with an anti-MASP2, anti-C1s or anti-CR3 molecules for use in treating or preventing age-related macular degeneration (AMD) by administering to a subject in need thereof an effective amount of the active compound or its salt or composition in combination with the anti-MASP2, anti-C1s or anti-CR3 molecules, for example, but not limited to: Cynryze (ViroPharma/Baxter); TNT003 (True North); OMS721 (Omeros); OMS906 (Omeros); and Imprime PGG (Biothera).

In some embodiments, the present invention provides an active compound or its salt or composition as described herein in combination with an PDGF inhibitor for use in treating or preventing age-related macular degeneration (AMD), by administering to a subject in need thereof an effective amount of the active compound or its salt or composition in combination with the PDGF inhibitor, for example as described herein including but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil.

In some embodiments, the present invention provides an active compound or its salt or composition as described herein for use in treating or preventing paroxysmal nocturnal hemoglobinuria (PNH), by administering to a subject in need thereof an effective amount of the active compound or its salt or composition with an additional inhibitor of the complement system or another active compound with a different biological mechanism of action. In another embodiment, the present invention provides an active compound or its salt or composition as described herein for use in treating or preventing paroxysmal nocturnal hemoglobinuria (PNH) by administering to a subject in need thereof an effective amount of the active compound or its salt or composition in combination or alternation with eculizumab or ravulizumab. In another embodiment, the present invention provides an active compound or its salt or composition as described herein for use in treating or preventing paroxysmal nocturnal hemoglobinuria (PNH), by administering to a subject in need thereof an effective amount of the active compound or its salt or composition in combination or alternation with CP40. In some embodiments, the additional agent is PEGylated-CP40. CP40 is a peptide inhibitor that shows a strong binding affinity for C3b and inhibits hemolysis of paroxysmal nocturnal hemoglobinuria (PNH) erythrocytes. In some embodiments, the additional agent is a complement component inhibitor, for example but not limited to Compstatin/POT-4 (Potentia Pharmaceuticals); ARC1905 (Archemix); 4(1MEW)APL-1,APL-2 (Appelis); CP40/AMY-101,PEG-Cp40 (Amyndas); a PDGF inhibitor, for example, but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; an anti-factor H or anti-factor B agent, for example anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas); a complement C3 or CAP C3 convertase targeting molecule, for example but not limited to TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH, an anti-CR3, anti-MASP2, anti C1s, or anti-C1n molecule, for example but not limited to Cynryze (ViroPharma/Baxter); TNT003 (True North); OMS721 (Omeros); OMS906 (Omeros); and Imprime PGG (Biothera)

In some embodiments, the present invention provides an active compound or its salt or composition as described herein for use in treating or preventing rheumatoid arthritis by administering to a subject in need thereof an effective amount of a composition comprising the active compound or its salt or composition in combination or alternation with an additional inhibitor of the complement system, or an active agent that functions through a different mechanism of action. In another embodiment, the present invention provides an active compound or its salt or composition as described herein for use in treating or preventing rheumatoid arthritis by administering to a subject in need thereof an effective amount of the active compound or its salt or composition in combination or alternation with methotrexate. In certain embodiments, an active compound or its salt or composition as described herein is administered in combination or alternation with at least one additional therapeutic agent selected from: salicylates including aspirin (Anacin, Ascriptin, Bayer Aspirin, Ecotrin) and salsalate (Mono-Gesic, Salgesic); nonsteroidal anti-inflammatory drugs (NSAIDs); nonselective inhibitors of the cyclo-oxygenase (COX-1 and COX-2) enzymes, including diclofenac (Cataflam, Voltaren), ibuprofen (Advil, Motrin), ketoprofen (Orudis), naproxen (Aleve, Naprosyn), piroxicam (Feldene), etodolac (Lodine), indomethacin, oxaprozin (Daypro), nabumetone (Relafen), and meloxicam (Mobic); selective cyclo-oxygenase-2 (COX-2) inhibitors including Celecoxib (Celebrex); disease-modifying antirheumatic drugs (DMARDs), including azathioprine (Imuran), cyclosporine (Sandimmune, Neoral), gold salts (Ridaura, Solganal, Aurolate, Myochrysine), hydroxychloroquine (Plaquenil), leflunomide (Arava), methotrexate (Rheumatrex), penicillamine (Cuprimine), and sulfasalazine (Azulfidine); biologic drugs including abatacept (Orencia), etanercept (Enbrel), infliximab (Remicade), adalimumab (Humira), and anakinra (Kineret); corticosteroids including betamethasone (Celestone Soluspan), cortisone (Cortone), dexamethasone (Decadron), methylprednisolone (SoluMedrol, DepoMedrol), prednisolone (Delta-Cortef), prednisone (Deltasone, Orasone), and triamcinolone (Aristocort); gold salts, including Auranofin (Ridaura); Aurothioglucose (Solganal); Aurolate; Myochrysine; or any combination thereof.

In some embodiments, the present invention provides an active compound or its salt or composition as described herein for use in treating or preventing multiple sclerosis by administering to a subject in need thereof an effective amount of the active compound or its salt or composition in combination or alternation with an additional inhibitor of the complement system, or an active agent that functions through a different mechanism of action. In another embodiment, the present invention provides an active compound or its salt or composition as described herein for use in treating or preventing multiple sclerosis by administering to a subject in need thereof an effective amount of the active compound or its salt or composition in combination or alternation with a corticosteroid. Examples of corticosteroids include, but are not limited to, prednisone, dexamethasone, solumedrol, and methylprednisolone. In some embodiments, an active compound or its salt or composition as described herein is combined with at least one anti-multiple sclerosis drug, for example, selected from: Aubagio (teriflunomide), Avonex (interferon beta-1a), Betaseron (interferon beta-1b), Copaxone (glatiramer acetate), Extavia (interferon beta-1b), Gilenya (fingolimod), Lemtrada (alemtuzumab), Novantrone (mitoxantrone), Plegridy (peginterferon beta-1a), Rebif (interferon beta-1a), Tecfidera (dimethyl fumarate), Tysabri (natalizumab), Solu-Medrol (methylprednisolone), High-dose oral Deltasone (prednisone), H.P. Acthar Gel (ACTH), or a combination thereof.

In some embodiments, an active compound or its salt or composition as described herein is useful in a combination with another pharmaceutical agent to ameliorate or reduce a side effect of the agent. For example, in some embodiments, an active compound or its salt or composition as described herein may be used in combination with adoptive cell transfer therapies to reduce an associated inflammatory response associated with such therapies, for example, a cytokine mediated response such as cytokine release syndrome. In some embodiments, the adoptive cell transfer therapy includes the use of a chimeric antigen receptor T-Cell (CAR T). In some embodiments, the adoptive cell transfer therapy includes the use of a chimeric antigen receptor T-Cell (CAR T) or a dendritic cell to treat a hematologic or solid tumor, for example, a B-cell related hematologic cancer. In some embodiments, the hematologic or solid tumor is acute lymphoblastic leukemia (ALL), acute myeloid leukemia (AML), non-Hodgkin's lymphoma, chronic lymphocytic leukemia (CLL), pancreatic cancer, glioblastoma, or a cancer that expresses CD19.

In an additional alternative embodiment, an active compound or its salt or composition as described herein may be provided in combination with eculizumab or ravulizumab for the treatment of PNH, aHUSs, STEC-HUS, ANCA-vasculitis, AMD, CAD, C3 glomerulopathy, for example DDD or C3GN, chronic hemolysis, neuromyelitis optica, or transplantation rejection. In some embodiments, an active compound or its salt or composition as described herein may be provided in combination with compstatin or a compstatin derivative for the treatment of PNH, aHUSs, STEC-HUS, ANCA-vasculitis, AMD, CAD, C3 glomerulopathy, for example DDD or C3GN, chronic hemolysis, neuromyelitis optica, or transplantation rejection. In some embodiments, the additional agent is a complement component inhibitor, for example but not limited to Compstatin/POT-4 (Potentia Pharmaceuticals); ARC1905 (Archemix); 4(1MEW)APL-1,APL-2 (Appelis); CP40/AMY-101,PEG-Cp40 (Amyndas); a PDGF inhibitor, for example, but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; an anti-factor H or anti-factor B agent, for example anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas); a complement C3 or CAP C3 convertase targeting molecule, for example but not limited to TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH, an anti-CR3, anti-MASP2, anti C1s, or anti-C1n molecule, for example but not limited to Cynryze (ViroPharma/Baxter); TNT003 (True North); OMS721 (Omeros); OMS906 (Omeros); and Imprime PGG (Biothera).

In some embodiments, an active compound or its salt or composition as described herein may be provided in combination with rituxan for the treatment of a complement mediated disorder. In some embodiments, the complement mediated disorder is, for example, rheumatoid arthritis, Granulomatosis with Polyangiitis (GPA) (Wegener's Granulomatosis), and Microscopic Polyangiitis (MPA). In some embodiments, the disorder is Lupus.

In some embodiments, an active compound or its salt or composition as described herein may be provided in combination with cyclophosphamide for the treatment of a complement mediated disorder. In some embodiments, the disorder is an autoimmune disease. In some embodiments, the complement mediated disorder is, for example, rheumatoid arthritis, Granulomatosis with Polyangiitis (GPA) (Wegener's Granulomatosis), and Microscopic Polyangiitis (MPA). In some embodiments, the disorder is Lupus.

In some embodiments, an active compound or its salt or composition as described herein is dosed in combination with a conventional DLE treatment for the treatment of lupus to a subject in need thereof.

Examples of conventional DLE treatments include topical corticosteroid ointments or creams, such as triamcinolone acetonide, fluocinolone, flurandrenolide, betamethasone valerate, or betamethasone dipropionate. Resistant plaques can be injected with an intradermal corticosteroid. Other potential DLE treatments include calcineurin inhibitors such as pimecrolimus cream or tacrolimus ointment. Particularly resistant cases can be treated with systemic antimalarial drugs, such as hydroxychloroquine (PLAQUENIL).

In some embodiments, an active compound or its salt or composition as described herein may be provided in combination with methotrexate for the treatment of Lupus.

In some embodiments, an active compound or its salt or composition as described herein may be provided in combination with azathioprine for the treatment of Lupus.

In some embodiments, an active compound or its salt or composition as described herein may be provided in combination with a non-steroidal anti-inflammatory drug for the treatment of Lupus.

In some embodiments, an active compound or its salt or composition as described herein may be provided in combination with a corticosteroid for the treatment of Lupus.

In some embodiments, an active compound or its salt or composition as described herein may be provided in combination with a belimumab (Benlysta) for the treatment of Lupus.

In some embodiments, an active compound or its salt or composition as described herein may be provided in combination with hydroxychloroquine (Plaquenil) for the treatment of Lupus.

In some embodiments, an active compound or its salt or composition as described herein may be provided in combination with sifalimumab for the treatment of Lupus.

In some embodiments, an active compound or its salt or composition as described herein may be provided in combination with OMS721 (Omeros) for the treatment of a complement mediated disorder. In some embodiments, an active compound or its salt or composition as described herein may be provided in combination with OMS906 (Omeros) for the treatment of a complement mediated disorder. In some embodiments, the complement mediated disorder is, for example, thrombotic thrombocytopenic purpura (TTP) or aHUS.

In some embodiments, an active compound or its salt or composition as described herein may be provided in combination with an anti-inflammatory agent, immunosuppressive agent, or anti-cytokine agent for the treatment or prevention of cytokine or inflammatory reactions in response to the administration of pharmaceuticals or biotherapeutics (e.g. adoptive T-cell therapy (ACT) such as CAR T-cell therapy, or monoclonal antibody therapy). In some embodiments, an active compound or its salt or composition as described herein may be provided in combination with a corticosteroid, for example prednisone, dexamethasone, solumedrol, and methylprednisolone, and/or anti-cytokine compounds targeting, e.g., IL-4, IL-10, IL-11, IL-13 and TGFβ. In some embodiments, an active compound or its salt or composition as described herein may be provided in combination with an anti-cytokine inhibitor including, but are not limited to, adalimumab, infliximab, etanercept, protopic, efalizumab, alefacept, anakinra, siltuximab, secukibumab, ustekinumab, golimumab, and tocilizumab, or a combination thereof. Additional anti-inflammatory agents that can be used in combination with an active compound or its salt or composition as described herein include, but are not limited to, non-steroidal anti-inflammatory drug(s) (NSAIDs); cytokine suppressive anti-inflammatory drug(s) (CSAIDs); CDP-571/BAY-10-3356 (humanized anti-TNFα antibody; Celltech/Bayer); cA2/infliximab (chimeric anti-TNFα antibody; Centocor); 75 kdTNFR-IgG/etanercept (75 kD TNF receptor-IgG fusion protein; Immunex); 55 kdTNF-IgG (55 kD TNF receptor-IgG fusion protein; Hoffmann-LaRoche); IDEC-CE9.1/SB 210396 (non-depleting primatized anti-CD4 antibody; IDEC/SmithKline); DAB 486-IL-2 and/or DAB 389-IL-2 (IL-2 fusion proteins; Seragen); Anti-Tac (humanized anti-IL-2Rα; Protein Design Labs/Roche); IL-4 (anti-inflammatory cytokine; DNAX/Schering); IL-10 (SCH 52000; recombinant IL-10, anti-inflammatory cytokine; DNAX/Schering); IL-4; IL-10 and/or IL-4 agonists (e.g., agonist antibodies); IL-1RA (IL-1 receptor antagonist; Synergen/Amgen); anakinra (Kineret^{®}/Amgen); TNF-bp/s-TNF (soluble TNF binding protein); R973401 (phosphodiesterase Type IV inhibitor); MK-966 (COX-2 Inhibitor); Iloprost, leflunomide (anti-inflammatory and cytokine inhibiton); tranexamic acid (inhibitor of plasminogen activation); T-614 (cytokine inhibitor); prostaglandin E1; Tenidap (non-steroidal anti-inflammatory drug); Naproxen (non-steroidal anti-inflammatory drug); Meloxicam (non-steroidal anti-inflammatory drug); Ibuprofen (non-steroidal anti-inflammatory drug); Piroxicam (non-steroidal anti-inflammatory drug); Diclofenac (non-steroidal anti-inflammatory drug); Indomethacin (non-steroidal anti-inflammatory drug); Sulfasalazine; Azathioprine; ICE inhibitor (inhibitor of the enzyme interleukin-1β converting enzyme); zap-70 and/or lck inhibitor (inhibitor of the tyrosine kinase zap-70 or lck); TNF-convertase inhibitors; anti-IL-12 antibodies; anti-IL-18 antibodies; interleukin-11; interleukin-13; interleukin-17 inhibitors; gold; penicillamine; chloroquine; chlorambucil; hydroxychloroquine; cyclosporine; cyclophosphamide; anti-thymocyte globulin; anti-CD4 antibodies; CD5-toxins; orally-administered peptides and collagen; lobenzarit disodium; Cytokine Regulating Agents (CRAB) HP228 and HP466 (Houghten Pharmaceuticals, Inc.); ICAM-1 antisense phosphorothioate oligo-deoxynucleotides (ISIS 2302; Isis Pharmaceuticals, Inc.); soluble complement receptor 1 (TP10; T Cell Sciences, Inc.); prednisone; orgotein; glycosaminoglycan polysulphate; minocycline; anti-IL2R antibodies; marine and botanical lipids (fish and plant seed fatty acids); auranofin; phenylbutazone; meclofenamic acid; flufenamic acid; intravenous immune globulin; zileuton; azaribine; mycophenolic acid (RS-61443); tacrolimus (FK-506); sirolimus (rapamycin); amiprilose (therafectin); cladribine (2-chlorodeoxyadenosine).

In a specific embodiment, an active compound or its salt or composition as described herein may be provided in combination with a corticosteroid for the treatment or prevention of cytokine or inflammatory reactions in response to the administration of pharmaceuticals or biotherapeutics. In another embodiment, an active compound or its salt or composition as described herein may be provided in combination with etarnercept for the treatment or prevention of cytokine or inflammatory reactions in response to the administration of pharmaceuticals or biotherapeutics. In another embodiment, an active compound or its salt or composition as described herein may be provided in combination with tocilizumab for the treatment or prevention of cytokine or inflammatory reactions in response to the administration of pharmaceuticals or biotherapeutics. In another embodiment, an active compound or its salt or composition as described herein may be provided in combination with etarnercept and tocilizumab for the treatment or prevention of cytokine or inflammatory reactions in response to the administration of pharmaceuticals or biotherapeutics. In another embodiment, an active compound or its salt or composition as described herein may be provided in combination with infliximab for the treatment or prevention of cytokine or inflammatory reactions in response to the administration of pharmaceuticals or biotherapeutics. In another embodiment, an active compound or its salt or composition as described herein may be provided in combination with golimumab for the treatment or prevention of cytokine or inflammatory reactions in response to the administration of pharmaceuticals or biotherapeutics.

### C5 Inhibitors

Provided herein is a compound as provided herein for use in treating PNH in a subject, wherein said use comprises administering to the subject an effective amount of a C5 inhibitor in combination or alternation with an effective amount of the compound.

C5 inhibitors are known in the art. In some embodiments, the C5 inhibitor is a monoclonal antibody targeting C5. In some embodiments, the C5 inhibitor is eculizumab (Soliris^{™} Alexion Pharmaceuticals, New Haven, CT, see, e.g., U.S. Patent No. 9,352,035). In some embodiments, the C5 inhibitor is ravulizumab (Ultomiris^{™} Alexion Pharmaceuticals, New Haven, CT, see, e.g., U.S. Patent No. 9,371,377; 9,079,949 and 9,663,574).

In some embodiments, the C5 inhibitor may be, but is not limited to: a recombinant human minibody, for example Mubodina^{®} (monoclonal antibody, Adienne Pharma and Biotech, Bergamo, Italy; see U.S. Patent No. 7,999,081); coversin (small animal protein, Volution Immuno-pharmaceuticals, Geneva, Switzerland; see e.g. Penabad et al. Lupus, 2012, 23(12):1324-6); LFG316 (monoclonal antibody, Novartis, Basel, Switzerland, and Morphosys, Planegg, Germany; see U.S. Patent Nos. 8,241,628 and 8,883,158); ARC-1905 (pegylated RNA aptamer, Ophthotech, Princeton, NJ and New York, NY; see Keefe et al., Nature Reviews Drug Discovery, 9, 537-550); RA101348 and RA101495 (macrocyclic peptides, Ra Pharmaceuticals, Cambridge, MA); SOBI002 (affibody, Swedish Orphan Biovitrum, Stockholm, Sweden); ALN-CC5 (Si-RNA, Alnylam Pharmaceuticals, Cambridge, MA); ARC1005 (aptamers, Novo Nordisk, Bagsvaerd, Denmark); SOMAmers (aptamers, SomaLogic, Boulder, Co); SSL7 (bacterial protein toxin, see, e.g. Laursen et al. Proc. Natl. Acad. Sci. U.S.A., 107(8):3681-6); MEDI7814 (monoclonal antibody, MedImmune, Gaithersburg, MD); aurin tricarboxylic acid; aurin tricarboxylic acid derivatives (Aurin Biotech, Vancouver, BC, see U.S. Patent Appl. Pub. 2013/003592); RG6107 (anti-C5 recycling antibody, Roche Pharmaceuticals, Basel, Switzerland); ALXN1210 and ALXN5500 (monoclonal antibodies, Alexion Pharmaceuticals, New Haven, CT); TT30 (fusion protein, Alexion Pharmaceuticals, New Haven, CT); REGN3918 (monoclonal antibody, Regeneron, Tarrytown, NY); ABP959 (eculizumab biosimilar, Amgen, Thousand Oaks, CA); or combinations thereof.

In some embodiments, the C5 inhibitor is a recombinant human minibody, for example Mubodina^{®}. Mubodina^{®} is a fully human recombinant antibody C5 developed by Adienne Pharma and Biotech. Mubodina^{®} is described in U.S. Patent No. 7,999,081.

In some embodiments, the C5 inhibitor is coversin. Coversin is a recombinant protein derived from a protein discovered in the saliva of the *Ornithodoros moubata* tick currently developed as a recombinant protein by Akari Therapeutics. Coversin is described in Penabad et al. Lupus 2012, 23(12):1324-6.

In some embodiments, the C5 inhibitor is Tesidolumab/LFG316. Tesidolumab is a monoclonal antibody developed by Novartis and Morphosys. Tesidolumab is described in U.S. Patent Nos. 8,241,628 and 8,883,158.

In some embodiments, the C5 inhibitor is ARC-1905. ARC-1905 is a pegylated RNA aptamer developed by Ophthotech. ARC-1905 is described in Keefe et al. Nature Reviews Drug Discovery, 9:537-550.

In some embodiments, the C5 inhibitor is RA101348. RA101348 is a macrocyclic peptide developed by Ra Pharmaceuticals.

In some embodiments, the C5 inhibitor is RA101495. RA101495 is a macrocyclic peptide developed by Ra Pharmaceuticals.

In some embodiments, the C5 inhibitor is SOBI002. SOBI002 is an affibody developed by the Swedish Orphan Biovitrum.

In some embodiments, the C5 inhibitor is ARC1005. ARC1005 is an aptamer developed by Novo Nordisk.

In some embodiments, the C5 inhibitor is SOMAmers for C5. SOMAmers are aptamers developed by SomaLogic.

In some embodiments, the C5 inhibitor is SSL7. SSL7 is a bacterial protein toxin described in Laursen et al. Proc. Natl. Acad. Sci. U.S.A., 107(8):3681-6.

In some embodiments, the C5 inhibitor is MEDI7814. MEDI7814 is a monoclonal antibody developed by MedImmune.

In some embodiments, the C5 inhibitor is aurin tricarboxylic acid. In another embodiment, the C5 inhibitor is an aurin tricarboxylic acid derivative. These aurin derivatives were developed by Aurin Biotech and are further described in U.S. Patent Appl. Pub. No. 2013/003592).

In some embodiments, the C5 inhibitor is RG6107/SKY59. RG6107/SKY59 is an anti-C5 recycling antibody developed by Roche Pharmaceuticals.

In some embodiments, the C5 inhibtior is ALXN1210. In another embodiment, the C5 inhibitor is ALXN5500. ALXN1210 and ALXN5500 are monoclonal antibodies developed by Alexion Pharmaceuticals.

In some embodiments, the C5 inhibitor is TT30. TT30 is a fusion protein developed by Alexion Pharmaceuticals.

In some embodiments, the C5 inhibitor is ABP959. ABP959 is an eculizamab biosimilar monoclonal antibody developed by Amgen.

In some embodiments, the C5 inhibtor is Anti-C5 siRNA. Anti-C5 siRNA was developed by Alnylam Pharmaceuticals.

In some embodiments, the C5 inhibitor is Erdigna^{®}. Erdigna^{®} is an antibody developed by Adienne Pharma.

In some embodiments, the C5 inhibitor is avacincaptad pegol/Zimura^{®}. Avacincaptad pegol is in aptamer developed by Opthotech.

In some embodiments, the C5 inhibitor is SOBI005. SOBI005 is a protein in developed by the Swedish Orphan Biovitrum.

In some embodiments, the C5 inhibitor is ISU305. ISU305 is a monoclonal antibody developed by ISU ABXIS.

In some embodiments, the C5 inhibitor is REGN3918. REGN3918 is a monoclonal antibody developed by Regeneron.

### C3 Inhibitors

Provided herein is a compound as provided herein for use in treating PNH in a subject comprising administering to the subject an effective amount of a C3 inhibitor in combination or alternation with an effective amount of the compound.

C3 inhibitors are known in the art. In some embodiments, a compound of the present invention is administered in combination or alternation with compstatin and/or a compstatin analog. Compstatin and compastin analogs are known and are found to be useful inhibitors of C3, see U.S. Patent Nos. 9,056,076; 8,168,584; 9,421,240; 9,291,622; 8,580,735; 9371365; 9,169,307; 8,946,145; 7,989,589; 7,888,323; 6,319,897; and US Patent Appl. Pub. Nos. 2016/0060297; 2016/0015810; 2016/0215022; 2016/0215020; 2016/0194359; 2014/0371133; 2014/0323407; 2014/0050739; 2013/0324482; and 2015/0158915. In some embodiments, the compstatin analog having the amino acid sequence ICVVQDWGHHCRT (SEQ. ID. NO. 1). In another embodiment, the C3 inhibitor is a compstatin analog. In some embodiments, the compstatin analog is 4(1MeW)/APL-1 of the sequence Ac-ICV(1-mW)QDWGAHRCT(SEQ. ID. NO. 2), wherein Ac is acetyl and 1-mW is 1-methyltryptophan. In another embodiment, the compstatin analog is Cp40/AMY-101, which has an amino acid sequence yICV(1mW)QDW-Sar-AHRC-mI (SEQ. ID. NO. 3), wherein y is D-tyrosine, 1mW is 1-methyltryptophan, Sar is sarcosine, and mI is *N-*methylisoleucine. In yet another embodiment, the compstatin analog is PEG-Cp40, having the amino acid sequence PEG-yICV(1mW)QDW-Sar-AHRC-mI (SEQ. ID. NO. 4), wherein PEG is polyethyleneglycol (40 kDa), y is D-tyrosine, 1mW is 1-methyltryptophan, Sar is sarcosine, and mI is *N*-methylisoleucine. In yet another embodiment, the compstatin analog is 4(1MeW)POT-4. 4(1MeW)POT-4 was developed by Potentia. In yet another embodiment, the compstatin analog is AMY-201. AMY-201 was developed by Amyndas Pharmaceuticals.

In some embodiments, a compound of the present invention can be combined with C3 inhibitors that include, but are not limited to: H17 (monoclonal antibody, EluSys Therapeutics, Pine Brook, NJ); mirococept (CR1-based protein); sCR1 (CR1-based protein, Celldex, Hampton, NJ); TT32 (CR-1 based protein, Alexion Pharmaceuticals, New Haven, CT); HC-1496 (recombinant peptide); CB 2782 (enzyme, Catalyst Biosciences, South San Francisco, CA); APL-2 (pegylated synthetic cyclic peptide, Apellis Pharmaceuticals, Crestwood, KY); or combinations thereof.

In some embodiments, the C3 inhibitor is H17. H17 is a humanized monoclonal antibody in development by EluSys Therapeutics. H17 is described in Paixao-Cavalcante et al. J. Immunol. 2014, 192(10):4844-4851.

In some embodiments, the C3 inhibitor is mirococept. Mirococept is a CR1-based protein developed by Inflazyme Pharmaceuticals.

In some embodiments, the C3 inhibitor is sCR1. sCR1 is a soluble form of the CR1 protein developed by Celldex.

In some embodiments, the C3 inhibitor is TT32. TT32 is a CR-1 based protein developed by Alexion Pharmaceuticals.

In some embodiments, the C3 inhibitor is HC-1496. HC-1496 is a recombinant peptide developed by InCode.

In some embodiments, the C3 inhibitor is CB 2782. CB 2782 is novel protease derived from human membrane type serine protease 1 (MTSP-1) that was developed by Catalyst Biosciences.

In some embodiments, the C3 inhibitor is APL-2. APL-2 is a pegylated version of APL-1 developed by Apellis Pharmaceuticals.

### Complement Factor B (CFB) Inhibitors

Provided herein is a compound of the present invention for use in treating PNH by administering a CFB inhibitor in combination or alternation with the compound of the present invention. CFB inhibitors are known in the art. In some embodiments, a compound of the present invention can be combined with CFB inhibitors that include, but are not limited to: anti-FB SiRNA (Alnylam Pharmaceuticals, Cambridge, MA); TA106 (monoclonal antibody, Alexion Pharmaceuticals, New Haven, CT); LNP023 (small molecule, Novartis, Basel, Switzerland); SOMAmers (aptamers, SomaLogic, Boulder, CO); bikaciomab (Novelmed Therapeutics, Cleveland, OH); complin (see, Kadam et al., J. Immunol. 2010, DOI:10.409/jimmunol.10000200); Ionis-FB-L_{Rx} (ligand conjugated antisense drug, Ionis Pharmaceuticals, Carlsbad, CA); or a combination thereof. In another embodiment, CFB inhibitors that can be combined with a compound of the present invention include those disclosed in PCT/US17/39587. In another embodiment, CFB inhibitors that can be combined with a compound of the present invention as described herein include those disclosed in PCT/US17/014458. In another embodiment, CFB inhibitors that can be combined with a compound of the present invention as described herein include those disclosed in U.S. Patent Appl. Pub. No. 2016/0024079; . PCT Int. Appl. WO 2013/192345; PCT Int. Appl. WO 2013/164802; PCT Int. Appl. WO 2015/066241; PCT Int. Appl. WO 2015/009616 (assigned to Novartis AG).

In some embodiments, the CFB inhibitor is

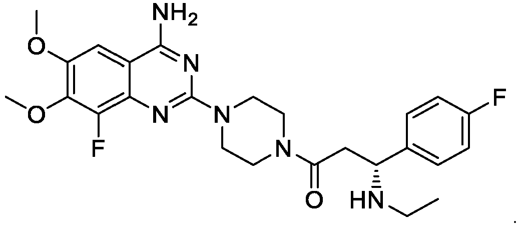

In another embodiment, the CFB inhibitor is

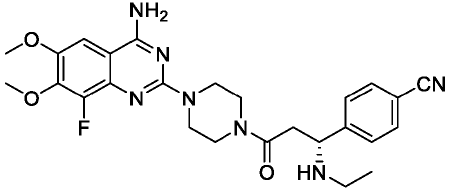

In another embodiment, the CFB inhibitor is

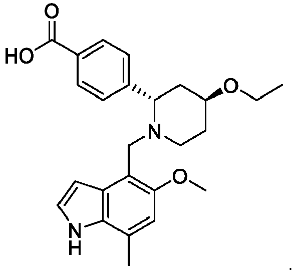

In some embodiments, the CFB inhibitor is anti-FB siRNA. Anti-FB siRNA was developed by Alnylam Pharmaceuticals.

In some embodiments, the CFB inhibitor is TA106. TA106 is a monoclonal antibody developed by Alexion Pharmaceuticals.

In some embodiments, the CFB inhibitor is LNP023. LNP023 is a small molecule inhibitor of CFB developed by Novartis.

In some embodiments, the CFB inhibitor is complin. Complin is a peptide inhibitor that is described in Kadam et al. J. Immunol. 2010 184(12):7116-24.

In some embodiments, the CFB inhibitor is Ionis-FB-L_{Rx}. Ionis-FB-L_{Rx} is a ligand conjugated antisense drug developed by Ionis Pharmaceuticals.

### Pan-inhibitors of Complement Components

Provided herein is a compounds of the present invention for use in treating PNH, comprising administering a pan-inhibitor of complement components in combination or alternation with the compound of the present invention. Pan-inhibitors of complement components are known in the art. In some embodiments, the inhibitor is FUT-175.

### COMBINATIONS FOR PROPHYLACTIC OR CONCOMMITANT ANTI-BACTERIAL THERAPY

In one aspect of the present invention, an active compound or its salt or composition as provided herein is provided for use in treating a host in need thereof, wherein said use comprises administering an effective amount of a prophylactic anti-bacterial vaccine prior to administration of the active compound or its salt or composition for any of the disorders described herein. In another aspect of the present invention, an active compound or its salt or composition as provided herein is provided for use in treating a host in need thereof, wherein said use comprises administering an effective amount of a prophylactic anti-bacterial drug, such as a pharmaceutical drug, prior to administration of the active compound or its salt or composition for any of the disorders described herein. In one aspect of the present invention, an active compound or its salt or composition as provided herein is provided for use in treating a host in need thereof, wherein said use comprises administering an effective amount of an anti-bacterial vaccine after administration of the active compound or its salt or composition for any of the disorders described herein. In another aspect of the present invention, an active compound or its salt or composition as provided herein is provided for use in treating a host in need thereof, wherein said use comprises administering an effective amount of an anti-bacterial drug, such as a pharmaceutical drug, after administration of the active compound or its salt or composition for any of the disorders described herein. In one embodiment, the disorder is PNH, C3G, or aHUS. In one embodiment, the host has received an organ or other tissue or biological fluid transplant. In one embodiment, the host is also administered eculizumab.

In one aspect of the present invention, an active compound or its salt or composition as described herein is administered to a host concomitantly to a subject following the prophylactic administration of a vaccine against a bacterial infection. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present invention, an active compound or its salt or composition as described herein is administered to a subject concomitantly with the prophylactic administration of a vaccine against a bacterial infection. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present invention, an active compound or its salt or composition as described herein is administered to a subject and, during the administration period of the compound or salt, a vaccine against a bacterial infection is administered to the subject. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present invention, the subject is administered an active compound or its salt or composition as described herein in combination with an antibiotic compound for the duration of Factor D inhibitor administration. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present invention, an active compound or its salt or composition as described herein is administered to a subject following the prophylactic administration of a vaccine against a bacterial infection, and in combination with an antibiotic compound for the duration of Factor D inhibitor administration. In one embodiment, the complement mediated disorder is PNH or aHUS. In one embodiment, the subject has received an organ or other tissue or biological fluid transplant. In one embodiment, the subject is also administered eculizumab. In one embodiment, the subject, prior to receiving an active compound or its salt or composition as described herein, is vaccinated against a bacterial infection caused by the bacterium Neisseria meningitidis. In one embodiment, the subject is vaccinated against a bacterial infection caused by the bacterium Haemophilus influenzae. In one embodiment, the Haemophilus influenzae is Haemophilus influenzae serotype B (Hib). In one embodiment, the subject is vaccinated against a bacterial infection caused by Streptococcus pneumoniae. In one embodiment, the subject is vaccinated against a bacterial infection caused by the bacterium Nisseria meningitidis, Haemophilus influenzae, or Streptococcus pneumoniae, or a combination of one or more of Nisseria meningitidis, Haemophilus influenzae, or Streptococcus pneumoniae. In one embodiment, the subject is vaccinated against a bacterial infection caused by the bacterium Nisseria meningitidis, Haemophilus influenzae, and Streptococcus pneumoniae.

In other embodiments, the subject is vaccinated against a bacterial infection caused by a bacterium selected from a Gram-negative bacterium. In one embodiment, the subject is vaccinated against a bacterial infection caused by a bacterium selected from a Gram-positive bacterium. In one embodiment, the subject is vaccinated against a bacterial infection caused by the bacterium Nisseria meningitidis, Haemophilus influenzae, or Streptococcus pneunemoniae, or a combination of one or more of Nisseria meningitidis, Haemophilus influenzae, or Streptococcus pneumoniae, and one or more of, but not limited to, Bacillus anthracis, Bordetella pertussis, Clostridium tetani, Corynebacterium diphtheria, Coxiella burnetii, Mycobacterium tuberculosis, Salmonella typhi, Vibrio cholerae, Anaplasma phagocytophilum, Ehrlichia ewingii, Ehrlichia chaffeensis, Ehrlichia canis, Neorickettsia sennetsu, Mycobacterium leprae, Borrelia burgdorferi, Borrelia mayonii, Borrelia afzelii, Borrelia garinii, Mycobacterium bovis, Staphylococcus aureus, Streptococcus pyogenes, Treponema pallidum, Francisella tularensis, Yersinia pestis,

In one embodiment, the subject is vaccinated with one or more vaccines selected from, but not limited to, typhoid vaccine, live (Vivotif Berna Vaccine, PaxVax), typhoid Vi polysaccharide vaccine (Typhim Vi, Sanofi), pneumococcal 23-polyvalent vaccine, PCV13 (Pneumovax 23, Merck), pneumococcal 7-valent vaccine, PCV7 (Prevnar, Pfizer), pneumococcal 13-valent vaccine, PCV13 (Prevnar 13, Pfizer), haemophilus b conjugate (prp-t) vaccine (ActHIB, Sanofi; Hibrix, GSK), haemophilus b conjugate (hboc) vaccine (HibTITER, Neuron Biotech), haemophilus b conjugate (prp-omp) vaccine (PedvaxHIB, Merck), haemophilus b conjugate (prp-t) vaccine/meningococcal conjugate vaccine (MenHibrix, GSK), haemophilus b conjugate (prp-t) vaccine/meningococcal conjugate vaccine/Hepatitis B vaccine (Comvax, Merck), meningococcal polysaccharide vaccine (Menomune A / C / Y / W-135, Sanofi), meningococcal conjugate vaccine/diphtheria CRM197 conjugate (Menveo, GSK; Menactra, Sanofi), meningococcal group B vaccine (Bexsero, GSK; Trumenba, Pfizer), anthrax vaccine adsorbed (Biothrax, Emergent Biosolutions), tetanus toxoid (Te Anatoxal Berna, Hendricks Regional Health), Bacillus Calmette and Guérin, live, intravesical (TheraCys, Sanofi; Tice BCG, Organon), cholera vaccine, live, oral (Vachora, Sanofi; Dukoral, SBL Vaccines; ShanChol, Shantha Biotec; Micromedex, Truven Health), tetanus toxoids and diphtheria absorbed (Tdap; Decavac, Sanofi; Tenivac, Sanofi; td, Massachusetts Biological Labs), diphtheria and tetanus toxois and pertussis (DTap; Daptacel, Sanofi; Infanrix, GSK; Tripedia, Sanofi), diphtheria and tetanus toxois and pertussis/polio (Kinrix, GSK; Quadracel, Sanofi), diphtheria and tetanus toxois and pertussis tetanus/hepatitis B/polio (Pediarix, GSK), diphtheria and tetanus toxois and pertussis/ polio, haemophilus influenza tybe b (Pentacel, Sanofi), and/or diphtheria, and pertussis (Tdap; Boostrix, GSK; Adacel, Sanofi), or a combination thereof.

As described above, a subject receiving a compound of the present invention to treat a disorder is prophylactically administered an antibiotic compound in addition to a Factor D inhibitor described herein. In one embodiment, the subject is administered an antibiotic compound for the duration of administration of the active compound to reduce the development of a bacterial infection. Antibiotic compounds for concomitant administration with a Factor D inhibitor described herein can be any antibiotic useful in preventing or reducing the effect of a bacterial infection. Antibiotics are well known in the art and include, but are not limited to, amikacin (Amikin), gentamicin (Garamycin), kanamycin (Kantrex), neomycin (Neo-Fradin), netilmicin (Netromycin), tobramycin (Nebcin), paromomycin (Humatin), streptomycin, spectinomycin (Trobicin), geldanamycin, herbimycin, rifaximin (Xifaxan), loracarbef (Lorabid), ertapenem (Invanz), doripenem (Doribax), imipenem/cilastatin (Primaxin), meropenem (Merrem), cefadroxil (Duricef), cefazolin (Ancef), cefalotin/cefalothin (Keflin), cephalexin (Keflex), cefaclor (Distaclor), cefamandole (Mandol), cefoxitin (Mefoxin), cefprozil (Cefzil), cefuroxime (Ceftin, Zinnat), cefixime (Cefspan), cefdinir (Omnicef, Cefdiel), cefditoren (Spectracef, Meiact), cefoperazone (Cefobid), cefotaxime (Claforan), cefpodoxime (Vantin) ceftazidime (Fortaz), ceftibuten (Cedax), ceftizoxime (Cefizox), ceftriaxone (Rocephin), cefepime (Maxipime), ceftaroline fosamil (Teflaro), ceftobiprole (Zeftera), teicoplanin (Targocid), vancomycin (Vancocin), telavancin (Vibativ), dalbavancin (Dalvance), oritavancin (Orbactiv), clindamycin (Cleocin), lincomycin (Lincocin), daptomycin (Cubicin), azithromycin (Zithromax, Sumamed, Xithrone), clarithromycin (Biaxin), dirithromycin (Dynabac), erythromycin (Erythocin, Erythroped), roxithromycin, troleandomycin (Tao), telithromycin (Ketek), spiramycin (Rovamycine), aztreonam (Azactam), furazolidone (Furoxone), nitrofurantoin (Macrodantin, Macrobid), linezolid (Zyvox), posizolid, radezolid, torezolid, amoxicillin (Novamox, Amoxil), ampicillin (Principen),azlocillin, carbenicillin (Geocillin), cloxacillin (Tegopen), dicloxacillin (Dynapen), flucloxacillin (Floxapen), mezlocillin (Mezlin), methicillin (Staphcillin), nafcillin (Unipen),oxacillin (Prostaphlin), penicillin G (Pentids),penicillin V (Veetids (Pen-Vee-K), piperacillin (Pipracil), penicillin G (Pfizerpen), temocillin (Negaban),ticarcillin (Ticar), amoxicillin/clavulanate (Augmentin), ampicillin/sulbactam (Unasyn), piperacillin/tazobactam (Zosyn), ticarcillin/clavulanate (Timentin),bacitracin, colistin (Coly-Mycin-S), polymyxin B, ciprofloxacin (Cipro, Ciproxin, Ciprobay), enoxacin (Penetrex), gatifloxacin (Tequin), gemifloxacin (Factive), levofloxacin (Levaquin), lomefloxacin (Maxaquin), moxifloxacin (Avelox), nalidixic acid (NegGram), norfloxacin (Noroxin), ofloxacin (Floxin, Ocuflox), trovafloxacin (Trovan), grepafloxacin (Raxar), sparfloxacin (Zagam), temafloxacin (Omniflox), mafenide (Sulfamylon), sulfacetamide (Sulamyd, Bleph-10), sulfadiazine (Micro-Sulfon), silver sulfadiazine (Silvadene), sulfadimethoxine (Di-Methox, Albon), sulfamethizole (Thiosulfil Forte), sulfamethoxazole (Gantanol), sulfanilamide, sulfasalazine (Azulfidine), sulfisoxazole (Gantrisin), trimethoprim-sulfamethoxazole (Co-trimoxazole) (TMP-SMX) (Bactrim, Septra), sulfonamidochrysoidine (Prontosil), demeclocycline (Declomycin), doxycycline (Vibramycin), minocycline (Minocin), oxytetracycline (Terramycin), tetracycline (Sumycin, Achromycin V, Steclin), clofazimine (Lamprene), dapsone (Avlosulfon), capreomycin (Capastat), cycloserine (Seromycin), ethambutol (Myambutol), ethionamide (Trecator), isoniazid (I.N.H.), pyrazinamide (Aldinamide), rifampicin (Rifadin, Rimactane), rifabutin (Mycobutin), rifapentine (Priftin), streptomycin, arsphenamine (Salvarsan), chloramphenicol (Chloromycetin), fosfomycin (Monurol, Monuril), fusidic acid (Fucidin), metronidazole (Flagyl), mupirocin (Bactroban), platensimycin, quinupristin/dalfopristin (Synercid), thiamphenicol, tigecycline (Tigacyl), tinidazole (Tindamax Fasigyn), trimethoprim (Proloprim, Trimpex), and/or teixobactin, or a combination thereof.

In one embodiment, the subject is administered a prophylactic antibiotic selected from cephalosporin, for example, ceftriaxone or cefotaxime, ampicillin-sulbactam, Penicillin G, ampicillin, chloramphenicol, fluoroquinolone, aztreonam, levofloxacin, moxifloxacin, gemifloxacin, vancomycin, clindamycin, cefazolin, azithromycin, meropenem, ceftaroline, tigecycline, clarithromycin, moxifloxacin, trimethoprim/sulfamethoxazole, cefuroxime, axetil, ciprofloxacin, rifampin, minocycline, spiramycin, and cefixime, or a combination of two or more thereof.

### PROCESS OF PREPARATION OF COMPOUNDS OF OF THE PRESENT INVENTION

### ABBREVIATIONS

- ACN: Acetonitrile
- Ac: Acetyl
- Ac₂O: Acetic anhydride
- AcOEt, EtOAc: ethyl acetate
- AcOH: Acetic acid
- Boc₂O: di-tert-butyl dicarbonate
- Bu: Butyl
- CAN: Ceric ammonium nitrate
- CBz: Carboxybenzyl
- CDI: Carbonyldiimidazole
- CH₃OH, MeOH: Methanol
- CsF: Cesium fluoride
- CuI: Cuprous iodide
- DCM, CH₂Cl₂: Dichloromethane
- DIEA, DIPEA: N,N-diisopropylethylamine
- DMA: N,N-dimethylacetamide
- DMAP: 4-Dimethylaminopyridine
- DMF: N,N-dimethylformamide
- DMS: Dimethyl sulfide
- DMSO: Dimethylsulfoxide
- DPPA: Diphenyl phosphoryl azide
- EDCI: 1-Ethyl-3-(3-dimethylaminopropyl)carbodiimide
- EEDQ: N-Ethoxycarbonyl-2-ethoxy-1,2-dihydroquinoline
- Et: Ethyl
- Et₃N, TEA: Triethylamine
- EtOAc: Ethylacetate
- EtOH: Ethanol
- HATU: 1-[Bis(dimethylamino)methylene]-1H-1,2,3-triazolo[4,5-b]pyridinium3-oxide hexafluorophosphate
- HCl: Hydrochloric acid
- HOBT: Hydroxybenzotriazole
- iBu, *i*-Bu, isoBu: Isobutyl
- iPr, *i*-Pr, isoPr: Isopropyl
- ⁱPr₂NEt: N,N-diisopropylethylamine
- K₂CO₃: Potassium carbonate
- K₂CO₃: Potassium carbonate
- LiOH: Lithium hydroxide
- Me: Methyl
- MeI: Methyl iodide
- Ms: Mesyl
- MsCl: Mesylchloride
- MTBE: Methyl *^{t}*butylether
- Na₂SO₄: Sodium sulfate
- NaCl: Sodium chloride
- NaH: Sodium hydride
- NaHCO₃: Sodium bicarbonate
- NBS: N-bromo succinimide
- NCS: N-chloro succinimide
- NEt₃: Trimethylamine
- NMP: N-Methyl-2-pyrrolidone
- PCC: Pyridinium chlorochromate
- Pd (OAc)₂: Palladium acetate
- Pd(dppf)Cl₂: [1,1'-Bis(diphenylphosphino) ferrocene]dichloropalladium(II)
- Pd(PPh₃)₂Cl₂: Bis(triphenylphosphine)palladium(II) dichloride
- Pd(PPh₃)₄: Tetrakis(triphenylphosphine)palladium(0)
- Pd/C: Palladium on carbon
- Pd₂ (dba)₃: Tris(dibenzylideneacetone)dipalladium(0)
- PMB: 4-Methoxybenzyl ether
- PPh₃: Triphenylphosphine
- Pr: Propyl
- Py, py: Pyridine
- RT: Room temperature
- T3P: Propane phosphonic acid anhydride
- TBAF: Tetra-n-butylammonium fluoride
- TBAT: Tetrabutylammonium difluorotriphenylsilicate
- tBu, *t*-Bu: *tert*butyl
- tBuOK: Potassium *tert*-butoxide
- TEA: Trimethylamine
- Tf₂O: Trifluoromethanesulfonic anhydride
- TFA: Trifluoroacetic acid
- THF: Tetrahydrofuran
- TMS: Trimethylsilane
- TMSBr: Bromotrimethylsilane
- *t_{R}*: Retention time
- Troc: 2,2,2-Trichlorethoxycarbonyl chloride
- Zn (CN)₂: Zinc cyanide

### GENERAL METHODS

All nonaqueous reactions were performed under an atmosphere of dry argon or nitrogen gas using anhydrous solvents. The progress of reactions and the purity of target compounds were determined using one of the two liquid chromatography (LC) methods listed below. The structure of starting materials, intermediates, and final products was confirmed by standard analytical techniques, including NMR spectroscopy and mass spectrometry.

### LC Method A

Instrument: Waters Acquity Ultra Performance LC
Column: ACQUITY UPLC BEH C18 2.1 × 50 mm, 1.7 µm
Column Temperature: 40 °C
Mobile Phase: Solvent A: H₂O + 0.05% FA; Solvent B: CH₃CN + 0.05% FA
Flow Rate: 0.8 mL/min
Gradient: 0.24 min @ 15% B, 3.26 min gradient (15-85% B), then 0.5 min @ 85% B.
Detection: UV (PDA), ELS, and MS (SQ in EI mode)

### LC Method B

Instrument: Shimadzu LC-2010A HT
Column: Athena, C18-WP, 50 × 4.6 mm, 5 µm
Column Temperature: 40 °C
Mobile Phase: Solvent A: H₂O/CH₃OH/FA = 90/10/0.1; Solvent B: H₂O/CH₃OH/FA = 10/90/0.1
Flow Rate: 3 mL/min
Gradient: 0.4 min @ 30% B, 3.4 min gradient (30-100% B), then 0.8 min @ 100% B
Detection: UV (220/254 nm)

### LC Method C

Instrument: Agilent 1100 / 1200 series LC system with DAD detector
Column: Atlantis dC18 (250 × 4.6) mm, 5 µm
Column Temperature: Ambient
Mobile Phase A: 0.1% TFA in water, Mobile Phase B: Acetonitrile
Flow Rate: 1.0 mL/min
Gradient:

| Time (min) | 0.0 | 15 | 20 | 23 | 30 |
|---|---|---|---|---|---|
| % B | 10 | 100 | 100 | 10 | 10 |

Detection: (210-400 nm)

### LC Method D

Instrument: Shimadzu LC 20AD system with PDA detector
Column: Phenomenex Gemini NX C18 (150 × 4.6) mm, 5 µm
Column Temperature: Ambient
Mobile Phase A: 10mM NH₄OAC in water, Mobile Phase B: Acetonitrile
Flow Rate: 1.0 mL/min
Gradient:

| Time (min) | 0.0 | 15 | 20 | 23 | 30 |
|---|---|---|---|---|---|
| %B | 10 | 100 | 100 | 10 | 10 |

Detection: (210-400 nm)

### REFERENCE SYNTHETIC METHODS

The below schemes provide synthetic methods for reference.

The skilled artisan will recognize that there are various modifications that can be performed to make analogs or prepare compounds in other ways. For example, wherever a Suzuki coupling is used there are other methods known in the art to conduct the coupling such as those described in the literature (Molander, G. A., Trice, S. L. J., & Kennedy, S. M. (2012). Scope of the two-step, one-pot palladium-catalyzed borylation/Suzuki cross-coupling reaction utilizing bis-boronic acid. Journal of Organic Chemistry, 77(19), 8678-8688.)

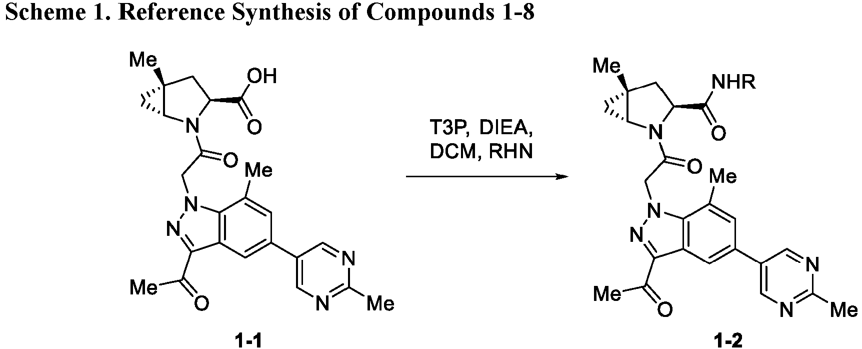

**General synthetic procedure for Compounds 1-8:** To a stirring solution of (1*R*,3*S*,5*R*)-2-(2-(3-acetyl-7-methyl-5-(2-methylpyrimidin-5-yl)-1*H*-indazol-1-yl)acetyl)-5-methyl-2-azabicyclo[3.1.0]hexane-3-carboxylic acid (**1-1**, 1 equiv) in DCM (10 vol) at 0 °C under nitrogen atmosphere was added N,N-diisopropylethylamine (DIEA) (3 equiv) and propane phosphonic acid anhydride (T3P) (1.5 equiv). The reaction mixture was stirred at 0 °C for 10 minutes. Amine (RHN) was added to the reaction mixture at 0 °C and the mixture was stirred at room temperature for 16 hours. After completion of the reaction, the reaction mixture was concentrated and purified directly by reverse phase HPLC to afford compound **1-2.**

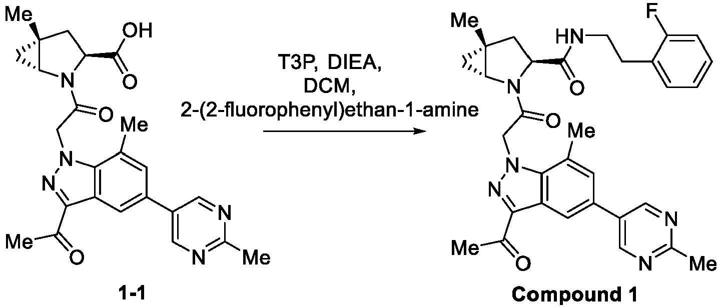

**(1*R*,3*S*,5*R*)-2-(2-(3-Acetyl-7-methyl-5-(2-methylpyrimidin-5-yl)-1*H*-indazol-1-yl)acetyl)-*N-*(2-fluorophenethyl)-5-methyl-2-azabicyclo[3.1.0]hexane-3-carboxamide (Compound 1):** ¹H NMR (400 MHz, Methanol-d4) δ 9.02 (s, 2H), 8.42 (d, J = 1.6 Hz, 1H), 8.07 (t, J = 5.9 Hz, 1H), 7.54 (s, 1H), 7.23 (ddd, J = 13.1, 7.9, 6.0 Hz, 2H), 7.05 (q, J = 9.8, 8.4 Hz, 2H), 5.90 (d, J = 17.7 Hz, 1H), 5.79 (d, J = 17.7 Hz, 1H), 4.27 (dd, J = 9.2, 5.3 Hz, 1H), 3.58 - 3.34 (m, 2H), 2.85 (qd, J = 13.9, 6.9 Hz, 2H), 2.77 (s, 3H), 2.74 (s, 3H), 2.70 (s, 3H), 2.45 (dd, J = 13.4, 9.2 Hz, 1H), 1.98 (dd, J = 13.9, 5.7 Hz, 1H), 1.32 (s, J = 13.0 Hz, 3H), 1.31 (s, 1H), 1.09 (t, J = 5.4 Hz, 1H), 0.96 - 0.86 (m, 1H).

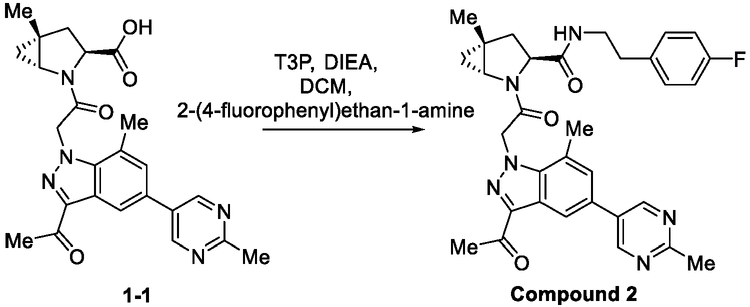

**(1*R*,3*S*,5*R*)-2-(2-(3-Acetyl-7-methyl-5-(2-methylpyrimidin-5-yl)-1*H*-indazol-1-yl)acetyl)-*N-*(4-fluorophenethyl)-5-methyl-2-azabicyclo[3.1.0]hexane-3-carboxamide (Compound 2):** ¹H NMR (400 MHz, Methanol-d4) δ 9.02 (s, 2H), 8.45 (d, J = 1.4 Hz, 1H), 8.02 (s, 1H), 7.56 (s, 1H), 7.21 (dd, J = 8.5, 5.5 Hz, 2H), 6.96 (t, J = 8.7 Hz, 2H), 5.92 (d, J = 17.8 Hz, 1H), 5.81 (d, J = 17.7 Hz, 1H), 4.28 (dd, J = 9.2, 5.3 Hz, 1H), 3.50 - 3.35 (m, 3H), 2.77 (s, 3H), 2.75 (s, 3H), 2.71 (s, 3H), 2.46 (dd, J = 13.3, 9.2 Hz, 1H), 1.96 (dd, J = 13.3, 5.4 Hz, 1H), 1.34 (s, 3H), 1.31 (s, 1H), 1.09 (t, J = 5.5 Hz, 1H), 0.94 - 0.84 (m, 1H).

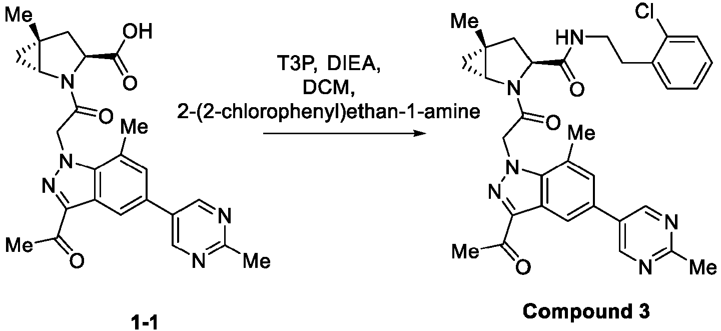

**(1*R*,3*S*,5*R*)-2-(2-(3-Acetyl-7-methyl-5-(2-methylpyrimidin-5-yl)-1*H*-indazol-1-yl)acetyl)-*N-*(2-chlorophenethyl)-5-methyl-2-azabicyclo[3.1.0]hexane-3-carboxamide (Compound 3):** ¹H NMR (400 MHz, Methanol-d4) δ 9.00 (s, 2H), 8.43 (d, J = 1.7 Hz, 1H), 8.08 (t, J = 6.0 Hz, 1H), 7.55 (s, 1H), 7.53 - 7.41 (m, 2H), 7.24 - 7.15 (m, 2H), 5.90 (d, J = 17.6 Hz, 1H), 5.80 (d, J = 17.7 Hz, 1H), 4.28 (dd, J = 9.2, 5.4 Hz, 1H), 3.56 - 3.37 (m, 2H), 3.03 - 2.93 (m, 1H), 2.96 - 2.86 (m, 1H), 2.76 (s, 3H), 2.75 (s, 3H), 2.70 (s, 3H), 2.47 (dd, J = 13.4, 9.2 Hz, 1H), 2.11 - 1.93 (m, 1H), 1.35 (s, 3H), 1.09 (t, J = 5.5 Hz, 1H), 0.90 (dd, J = 5.4, 2.5 Hz, 1H).

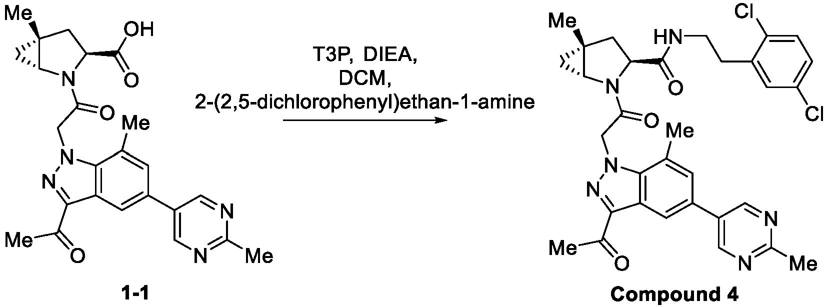

**(1*R*,3*S*,5*R*)-2-(2-(3-Acetyl-7-methyl-5-(2-methylpyrimidin-5-yl)-1*H*-indazol-1-yl)acetyl)-*N-*(2,5-dichlorophenethyl)-5-methyl-2-azabicyclo[3.1.0]hexane-3-carboxamide (Compound 4):** ¹H NMR (400 MHz, Methanol-d4) δ 9.03 (s, 2H), 8.43 (d, J = 1.7 Hz, 1H), 8.09 (q, J = 7.3, 6.5 Hz, 1H), 7.55 (s, 1H), 7.50 - 7.29 (m, 2H), 7.20 (dd, J = 8.5, 2.6 Hz, 1H), 5.91 (d, J = 17.7 Hz, 1H), 5.79 (d, J = 17.7 Hz, 1H), 4.25 (dd, J = 9.2, 5.6 Hz, 1H), 3.58 - 3.34 (m, 3H), 3.07 - 2.82 (m, 2H), 2.77 (s, 3H), 2.75 (s, 3H), 2.70 (s, 3H), 2.58 - 2.42 (m, 1H), 1.98 (dd, J = 13.3, 5.6 Hz, 1H), 1.35 (s, 3H), 1.08 (t, J = 5.6 Hz, 1H), 0.90 (dd, J = 5.5, 2.4 Hz, 1H).

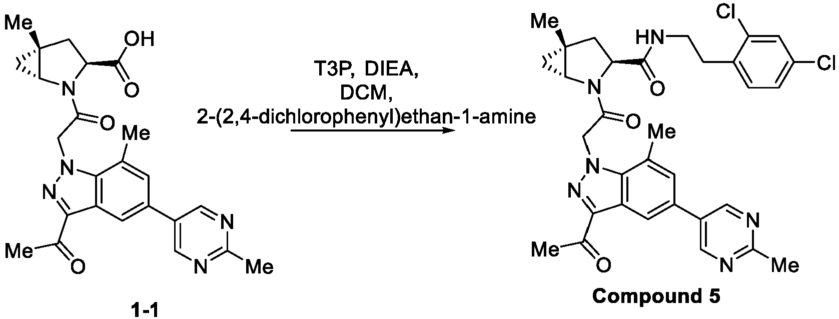

**(1*R*,3*S*,5*R*)-2-(2-(3-Acetyl-7-methyl-5-(2-methylpyrimidin-5-yl)-1*H*-indazol-1-yl)acetyl)-*N-*(2,4-dichlorophenethyl)-5-methyl-2-azabicyclo[3.1.0]hexane-3-carboxamide (Compound 5):** ¹H NMR (400 MHz, Methanol-d4) δ 9.03 (s, 2H), 8.45 (d, J = 1.6 Hz, 1H), 8.09 (d, J = 6.1 Hz, 1H), 7.56 (s, 1H), 7.41 (m, 1H), 7.25 (d, J = 8.2 Hz, 1H), 7.16 (dd, J = 8.2, 2.2 Hz, 1H), 5.91 (d, J = 17.7 Hz, 1H), 5.80 (d, J = 17.7 Hz, 1H), 4.26 (dd, J = 9.2, 5.4 Hz, 1H), 3.51 - 3.34 (m, 2H), 2.92 (td, J = 6.8, 4.1 Hz, 2H), 2.77 (s, 3H), 2.75 (s, 3H), 2.70 (s, 3H), 2.53 - 2.41 (m, 1H), 2.04 - 1.93 (m, 1H), 1.35 (s, 3H), 1.09 (t, J = 5.5 Hz, 1H), 0.90 (dd, J = 5.4, 2.4 Hz, 1H).

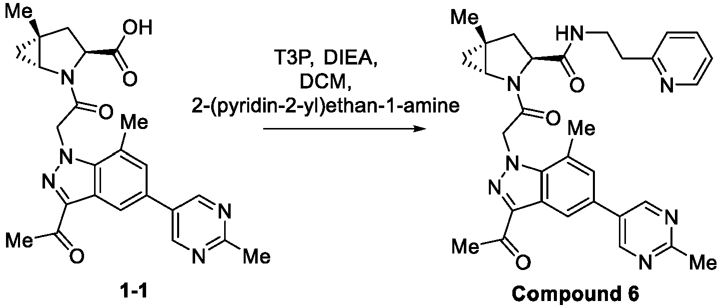

**(1*R*,3*S*,5*R*)-2-(2-(3-Acetyl-7-methyl-5-(2-methylpyrimidin-5-yl)-1*H*-indazol-1-yl)acetyl)-5-methyl-*N*-(2-(pyridin-2-yl)ethyl)-2-azabicyclo[3.1.0]hexane-3-carboxamide (Compound 6):** ¹H NMR (400 MHz, Methanol-d4) δ 9.05 (s, 2H), 8.49 (d, J = 1.6 Hz, 1H), 8.40 - 8.33 (m, 1H), 8.31 - 8.22 (m, 1H), 7.88 (d, J = 8.0 Hz, 1H), 7.70 - 7.59 (m, 2H), 5.91 (d, J = 17.7 Hz, 1H), 5.80 (d, J = 17.7 Hz, 1H), 4.15 (dd, J = 9.1, 6.2 Hz, 1H), 3.77 (ddd, J = 13.4, 7.6, 5.1 Hz, 1H), 3.56 - 3.44 (m, 2H), 3.28 - 3.08 (m, 2H), 2.78 (s, 3H), 2.73 (s, 6H), 2.49 (dd, J = 13.4, 9.2 Hz, 1H), 1.97 (dd, J = 13.4, 6.1 Hz, 1H), 1.35 (s, 3H), 1.07 (t, J = 5.5 Hz, 1H), 0.90 (dd, J = 5.6, 2.4 Hz, 1H).

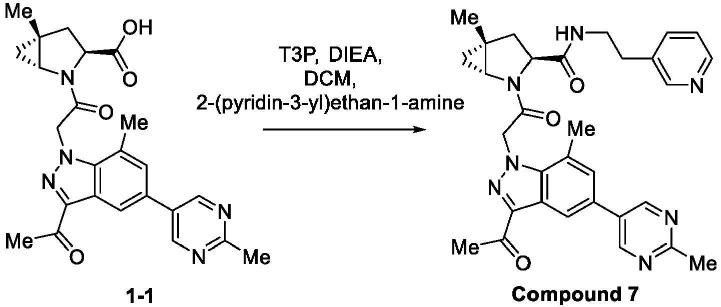

**(1*R*,3S,5*R*)-2-(2-(3-Acetyl-7-methyl-5-(2-methylpyrimidin-5-yl)-1*H*-indazol-1-yl)acetyl)-5-methyl-*N*-(2-(pyridin-3-yl)ethyl)-2-azabicyclo[3.1.0]hexane-3-carboxamide (Compound 7):** ¹H NMR (400 MHz, Methanol-d4) δ 9.06 (s, 2H), 8.66 - 8.61 (m, 1H), 8.51 - 8.42 (m, 2H), 8.38 (d, J = 5.7 Hz, 1H), 8.10 (t, J = 6.2 Hz, 1H), 7.78 (dd, J = 8.1, 5.8 Hz, 1H), 7.60 (t, J = 1.4 Hz, 1H), 5.90 (d, J = 17.8 Hz, 1H), 5.79 (d, J = 17.8 Hz, 1H), 4.17 (dd, J = 9.3, 5.8 Hz, 1H), 3.69 (dq, J = 12.6, 6.3 Hz, 1H), 3.51 - 3.34 (m, 2H), 3.03 (td, J = 6.4, 2.3 Hz, 2H), 2.78 (s, 3H), 2.73 (s, 3H), 3.73 (s, 3H), 2.56 - 2.45 (m, 1H), 2.03 - 1.92 (m, 1H), 1.35 (s, 3H), 1.08 (t, J = 5.6 Hz, 1H), 0.89 (dd, J = 5.6, 2.4 Hz, 1H).

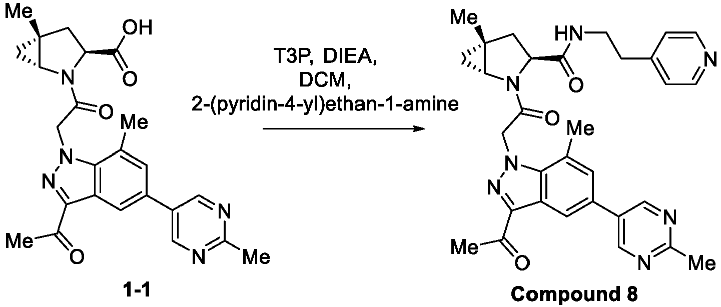

**(1*R*,3*S*,5*R*)-2-(2-(3-Acetyl-7-methyl-5-(2-methylpyrimidin-5-yl)-1*H*-indazol-1-yl)acetyl)-5-methyl-*N*-(2-(pyridin-4-yl)ethyl)-2-azabicyclo[3.1.0]hexane-3-carboxamide (Compound 8):** ¹H NMR (400 MHz, Methanol-d4) δ 9.06 (s, 2H), 8.55 (d, J = 6.2 Hz, 2H), 8.45 (d, J = 1.7 Hz, 1H), 7.91 (d, J = 6.0 Hz, 2H), 7.58 (s, 1H), 5.91 (d, J = 17.8 Hz, 1H), 5.82 (d, J = 17.8 Hz, 1H), 4.21 (dd, J = 9.3, 5.7 Hz, 1H), 3.66 (dt, J = 12.8, 6.2 Hz, 1H), 3.60 - 3.45 (m, 2H), 3.12 (t, J = 6.4 Hz, 2H), 2.79 (s, 3H), 2.75 (s, 3H), 2.72 (s, 3H), 2.56 - 2.45 (m, 1H), 1.99 (dd, J = 13.2, 5.9 Hz, 1H), 1.36 (s, 3H), 1.09 (t, J = 5.5 Hz, 1H), 0.90 (dd, J = 5.6, 2.5 Hz, 1H).

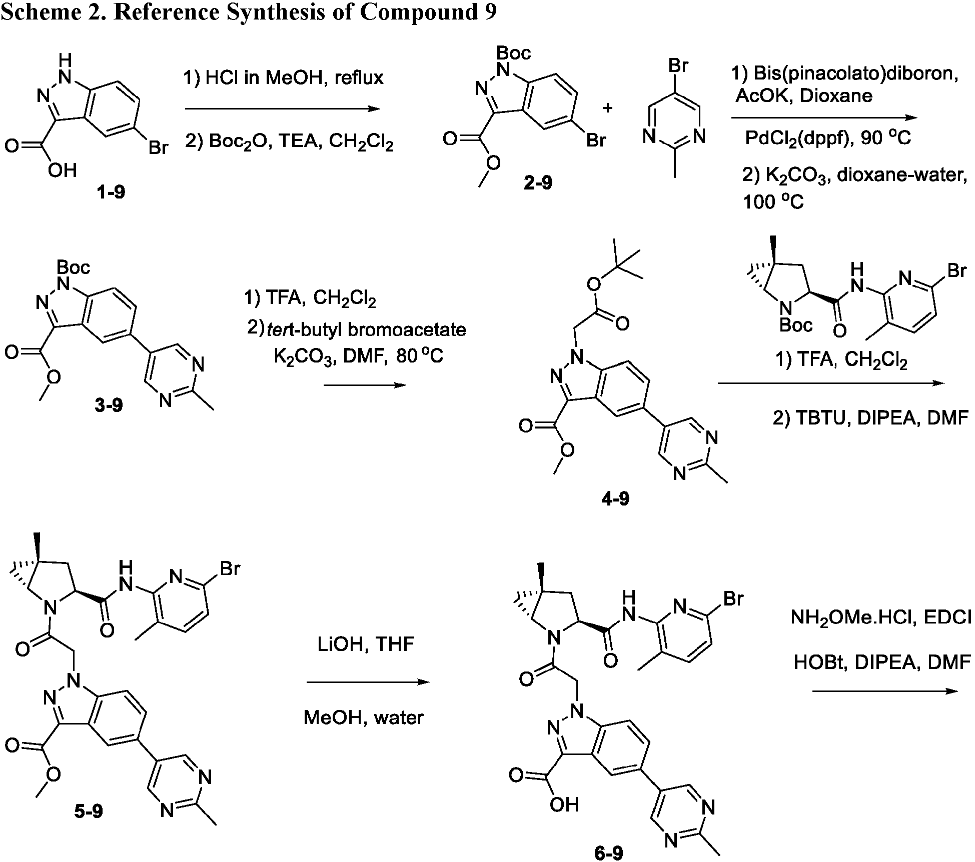

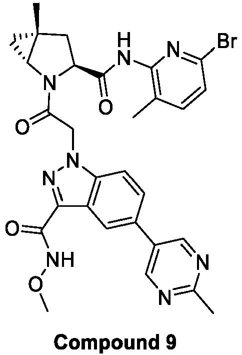

**Step 1: 1-(*tert*-Butyl) 3-methyl 5-bromo-1*H*-indazole-1,3-dicarboxylate (2-9):** A heterogeneous mixture of 5-bromo-1*H*-indazole-3-carboxylic acid (0.6 g, 2.489 mmol, 1 equiv.) in methanolic HCl was refluxed for 24 hours with the occasional addition of fresh methanolic HCl. The solvent was removed under reduced pressure. The residue was diluted with dichloromethane and Boc₂O (1.09 g, 4.994 mmol, 2.006 equiv.) and TEA (1.086 g, 1.5 mL, 10.732 mmol, 4.312 equiv.) were added. The heterogeneous mixture was stirred overnight at room temperature. The solvent was removed under reduced pressure. The residue was purified by silica gel column chromatography (eluent: 0-1.5 % MeOH in dichloromethane) to afford the desired compound as a light yellow solid.

**Step 2: 1-(*tert*-Butyl) 3-methyl 5-(2-methylpyrimidin-5-yl)-1*H*-indazole-1,3-dicarboxylate (3-9):** To a solution of 5-bromo-2-methylpyrimidine (0.231 g, 1.335 mmol, 1.103 equiv.) in dioxane (10 mL) were added bis(pinacolato)diboron (0.368 g, 1.454 mmol, 1.201 equiv.), potassium acetate (0.238 g, 2.425 mmol, 2.003 equiv.) and PdCl₂(dppf) (0.05 g, 0.061 mmol, 0.051 equiv.). The solution was degassed with argon and heated overnight at 90 ° C under argon. The reaction was cooled to room temperature and solid 1-tert-butyl 3-methyl 5-bromoindazole-1,3-dicarboxylate (0.43 g, 1.211 mmol, 1 equiv.) was added followed by potassium carbonate (0.502 g, 3.632 mmol, 3 equiv.). Dioxane (4 mL) and water (3.5 mL) were added and the reaction was heated at 100 °C for 2 hours. The reaction was cooled to room temperature and the reaction was stirred with 1 equiv. (0.246 g) of (Boc)₂O at room temperature for 1 hour. The solvent was removed under reduced pressure and the residue was purified by silica gel column chromatography (eluent: 0- 1.5 % MeOH in dichloromethane) to afford 0.38 g of a cream colored solid. ¹H-NMR: δ 1.76 (s, 9H), 2.82 (s, 3H), 4.08 (s, 3H), 7.77 (dd, J = 8.4, 1.2 Hz, 1H), 8.35 (d, J = 8.8 Hz, 1H), 8.45 (d, J = 0.8 Hz, 1H), 8.94 (s, 2H).

**Step 3: Methyl 1-(2-(*tert*-butoxy)-2-oxoethyl)-5-(2-methylpyrimidin-5-yl)-1*H-*indazole-3-carboxylate (4-9):** A solution of 1-*tert*-butyl 3-methyl 5-(2-methylpyrimidin-5-yl)indazole-1,3-dicarboxylate (0.1 g, 0.271 mmol, 1 equiv.) in dichloromethane (1 mL) and TFA (1 mL) was stirred at room temperature for 30 minutes. The volatiles were removed under reduced pressure and the residue was taken up in DMF (2 mL). Potassium carbonate (0.188 g, 1.36 mmol, 5.011 equiv.) and *tert*-butyl bromoacetate (0.106 g, 0.08 mL, 0.543 mmol, 2.002 equiv.) were added. The reaction was stirred at 80° C for 2 hours. The reaction was diluted with chloroform and filtered. The filtrate was concentrated and the residue was purified by silica gel column chromatography (eluent: 0-2 % MeOH in dichloromethane) to afford 40 mg of a white solid. ¹H-NMR : δ 1.46 (s, 9H), 2.82 (s, 3H), 7.07 (s, 3H), 7.52 (d, J = 8.8 Hz, 1H), 7.66 (dd, J = 8.4, 1.2 Hz, 1H), 8.44 (s, 1H), 8.92 (s, 2H).

**Step 4: Methyl 1-(2-((1*R*,3*S*,5*R*)-3-((6-bromo-3-methylpyridin-2-yl)carbamoyl)-5-methyl-2-azabicyclo[3.1.0]hexan-2-yl)-2-oxoethyl)-5-(2-methylpyrimidin-5-yl)-1*H*-indazole-3-carboxylate (5-9)**: In a vial, methyl 1-[2-(tert-butoxy)-2-oxoethyl]-5-(2-methylpyrimidin-5-yl)indazole-3-carboxylate (0.04 g, 0.105 mmol, 1 equiv.) was stirred with 1 mL of dichloromethane and 2 mL of TFA for 3 hours. In another vial, *tert*-butyl (1R,3S,5R)-3-[(6-bromo-3-methylpyridin-2-yl)carbamoyl]-5-methyl-2-azabicyclo[3.1.0]hexane-2-carboxylate (0.047 g, 0.115 mmol, 1.1 equiv.) was stirred with 1 mL of TFA and 1 mL of dichloromethane for 30 minutes. The contents from both the vials were combined and concentrated. The residue was taken up in DMF (1 mL) and cooled in an ice bath before DIPEA (0.091mL) and TBTU (0.037 g) were added. Then the reaction was stirred at room temperature for 30 minutes. The solvent was removed under reduced pressure. The residue was taken up in chloroform and washed with saturated aqueous sodium bicarbonate solution. The organic layer was dried with Na₂SO₄ and concentrated. The residue was purified by silica gel column chromatography (eluent: 0-3 % MeOH in dichloromethane) to afford 50 mg of product as a white solid.

**Step 5: 1-(2-((1*R*,3*S*,S*R)*-3-((6-Bromo-3-methylpyridin-2-yl)carbamoyl)-5-methyl-2-azabicyclo[3.1.0]hexan-2-yl)-2-oxoethyl)-5-(2-methylpyrimidin-5-yl)-1*H*-indazole-3-carboxylic acid (6-9):** To an ice-cold solution of methyl 1-{2-[(1*R*,3*S*,5*R*)-3-[(6-bromo-3-methylpyridin-2-yl)carbamoyl]-5-methyl-2-azabicyclo[3.1.0]hexan-2-yl]-2-oxoethyl}-5-(2-methylpyrimidin-5-yl)indazole-3-carboxylate (0.05 g, 0.081 mmol, 1 equiv.) in THF (3 mL)-MeOH (1 mL), lithium hydroxide monohydrate (0.006 g, 0.143 mmol, 1.769 equiv.) in water (1 mL) was added. The cooling bath was removed and the reaction was stirred at room temperature for 2 days. The organic solvent was removed under reduced pressure. The aqueous residue was cooled and acidified with cold 1N aqueous HCl. This acidified solution was lyophilized to afford 50 mg of a yellow solid that was used as such for the next step.

**Step 6: 1-(2-((1*R*,3*S*,5*R*)-3-((6-Bromo-3-methylpyridin-2-yl)carbamoyl)-5-methyl-2-azabicyclo[3.1.0]hexan-2-yl)-2-oxoethyl)-N-methoxy-5-(2-methylpyrimidin-5-yl)-1*H-*indazole-3-carboxamide (Compound 9):** A solution of 1-{2-[(1*R*,3*S*,5*R*)-3-[(6-bromo-3-methylpyridin-2-yl)carbamoyl]-5-methyl-2-azabicyclo[3.1.0]hexan-2-yl]-2-oxoethyl}-5-(2-methylpyrimidin-5-yl)indazole-3-carboxylic acid (0.045 g, 0.074 mmol, 1 equiv.) in DMF (1 mL) was cooled in an ice-bath. HOBt (0.011 g, 0.081 mmol, 1.094 equiv.), O-methylhydroxylamine hydrochloride (0.007 g, 0.084 mmol, 1.126 equiv.) and DIPEA (40 µL) were added, followed by EDCI (0.016 g, 0.083 mmol, 1.121 equiv.). The reaction was stirred at room temperature overnight. The solvent was removed under reduced pressure and the residue was purified by silica gel column chromatography (eluent: 0-4 % MeOH in dichloromethane) to afford a colorless solid. ¹H NMR (400 MHz, Chloroform-*d*) δ 0.84-0.87 (m, 1H), 1.10 (t, *J =* 5.4 Hz, 1H), 1.39 (s, 3H), 2.06 (s, 3H), 2.30 (dd, *J =* 8.9, 13.4 Hz, 1H), 2.65 (d, *J* = 13.4 Hz, 1H), 2.81 (s, 3H), 3.14-3.16 (m, 1H), 3.93 (s, 3H), 4.76 - 4.83 (m, 1H), 5.44 (d, *J* = 3.1 Hz, 2H), 7.22 (d, *J* = 7.9 Hz, 1H), 7.34 (d, *J* = 8.0 Hz, 1H), 7.59 (d, J = 8.8 Hz, 1H), 7.66 (dd, J = 8.8, 1.2 Hz, 1H), 8.56 (s, 1H), 8.74 (s, 1H), 8.91 (s, 2H), 9.55 (s, 1H).

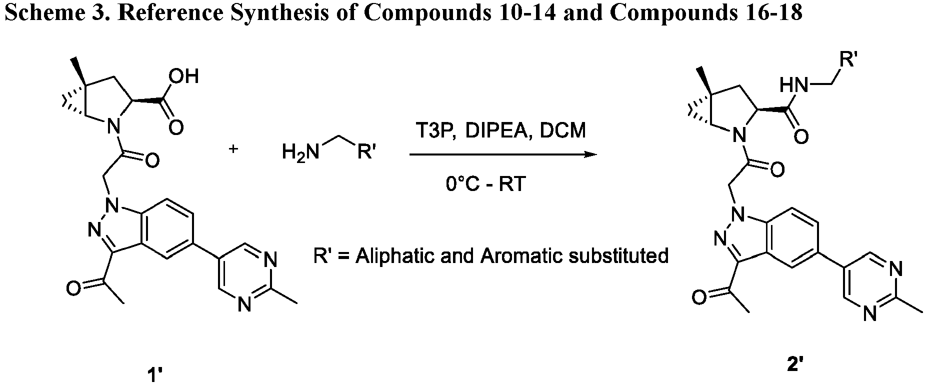

**General synthetic procedure for Compounds 10-14 and 16-18:** To a solution of (1*R*,3*S*,5*R*)-2-(2-(3-acetyl-5-(2-methylpyrimidin-5-yl)-1*H*-indazol-1-yl)acetyl)-5-methyl-2-azabicyclo[3.1.0]hexane-3-carboxylic acid (**3-1,** 1 equiv) in DCM (10 vol) at 0 °C under nitrogen atmosphere was added DIPEA (3 equiv) and T3P (1.5 equiv). The reaction mixture was stirred at 0 °C for 10 minutes. Amine (1.2 equiv) was added to the reaction mixture at 0 °C and stirred at room temperature for 16 hours. After completion of the reaction, the reaction mixture was concentrated and purified by preparative purification to afford compound **2'.**

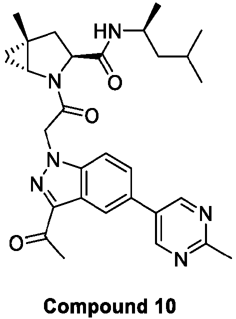

**(1*R*,3*S*,5*R*)-2-(2-(3-Acetyl-5-(2-methylpyrimidin-5-yl)-1*H*-indazol-1-yl)acetyl)-5-methyl-*N-*((*S*)-4-methylpentan-2-yl)-2-azabicyclo[3.1.0]hexane-3-carboxamide (Compound 10):** ¹H-NMR (400 MHz, DMSO-d6): δ 9.05 (s, 2H), 8.44 (s, 1H), 7.87 (s, 2H), 7.51 (d, J = 8.56 Hz, 1H), 5.90 (d, J = 17.28 Hz, 1H), 5.52 (d, J = 17.40 Hz, 1H), 4.15 - 4.13 (m, 1H), 3.84 - 3.83 (m, 1H), 3.51 (s, 1H), 2.69 (s, 3H), 2.66 (s, 3H), 2.38 - 2.35 (m, 2H), 1.87 - 1.82 (m, 1H), 1.50 (s, 1H), 1.34 - 1.32 (m, 1H), 1.16 - 1.10 (m, 2H), 0.99 - 0.91 (m, 6H), 0.81 - 0.78 (m, 6H).

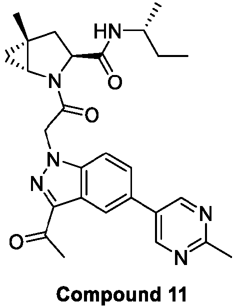

**(1*R*,3*S*,5*R*)-2-(2-(3-Acetyl-5-(2-methylpyrimidin-5-yl)-1*H*-indazol-1-yl)acetyl)-*N*-((*R*)-*sec-*butyl)-5-methyl-2-azabicyclo[3.1.0]hexane-3-carboxamide (Compound 11):** ¹H-NMR (400 MHz, DMSO-d6): δ 9.06 (s, 2H), 8.45 (s, 1H), 7.87 (s, 2H), 7.52 (d, J = 8.36 Hz, 1H), 5.90 (d, J = 17.20 Hz, 1H), 5.53 (d, J = 17.28 Hz, 1H), 4.18 - 4.15 (m, 1H), 3.65 - 3.60 (m, 1H), 3.51 (s, 1H), 2.70 (s, 3H), 2.66 (s, 3H), 2.40 - 2.34 (m, 2H), 1.90 (d, J = 4.40 Hz, 1H), 1.36 (t, J = 7.72 Hz, 2H), 1.28 (s, 3H), 1.00 - 0.83 (m, 4H), 0.78 (t, J = 7.36 Hz, 3H).

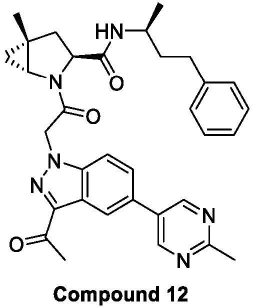

***(*1*R*,3*S*,5*R*)-2-(2-(3-Acetyl-5-(2-methylpyrimidin-5-yl)-1*H*-indazol-1-yl)acetyl)-5-methyl-*N-*((*S*)-4-phenylbutan-2-yl)-2-azabicyclo[3.1.0]hexane-3-carboxamide (Compound 12):** ¹H-NMR (400 MHz, DMSO-d6): δ 9.01 (s, 2H), 8.43 (s, 1H), 7.84 (q, J = 11.12 Hz, 2H), 7.66 (d, J = 8.04 Hz, 1H), 7.17 - 7.10 (m, 2H), 7.08 - 7.05 (m, 3H), 5.92 (d, J = 17.24 Hz, 1H), 5.54 (d, J = 17.04 Hz, 1H), 4.20 - 4.17 (m, 1H), 3.75 - 3.72 (m, 1H), 3.53 (s, 1H), 2.68 (s, 3H), 2.62 (s, 3H), 2.37 - 2.33 (m, 2H), 1.93 (d, J = 4.56 Hz, 1H), 1.62 - 1.61 (m, 2H), 1.29 - 1.16 (m, 4H), 1.03 - 0.95 (m, 5H).

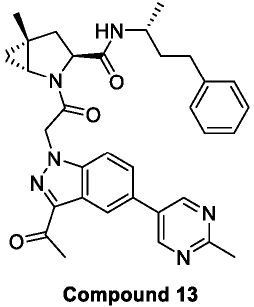

**(1R,3*S*,5*R*)-2-(2-(3-Acetyl-5-(2-methylpyrimidin-5-yl)-1*H*-indazol-1-yl)acetyl)-5-methyl-N-((R)-4-phenylbutan-2-yl)-2-azabicyclo[3.1.0]hexane-3-carboxamide (Compound 13): ¹H-**NMR (400 MHz, DMSO-d6): δ 4.98 (s, 2H), 8.42 (s, 1H), 7.89 - 7.87 (m, 1H), 7.72 (d, J = 1.44 Hz, 1H), 7.63 (d, J = 8.40 Hz, 1H), 7.17 - 7.13 (m, 2H), 7.09 - 7.05 (m, 3H), 5.91 (d, J = 17.24 Hz, 1H), 5.53 (d, J = 17.04 Hz, 1H), 4.15 - 4.13 (m, 1H), 3.75 - 3.73 (m, 1H), 3.53 (s, 1H), 2.68 (s, 3H), 2.63 (s, 3H), 2.44 - 2.41 (m, 2H), 1.91 - 1.88 (m, 1H), 1.64 - 1.61 (m, 2H), 1.28 - 1.15 (m, 4H), 1.05 (d, J = 6.44 Hz, 3H), 0.96 (s, 2H).

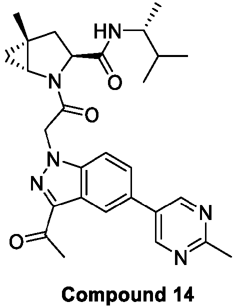

**(1*R*,3*S*,5*R*)-2-(2-(3-Acetyl-5-(2-methylpyrimidin-5-yl)-1*H*-indazol-1-yl)acetyl)-5-methyl-*N-*((*R*)-3-methylbutan-2-yl)-2-azabicyclo[3.1.0]hexane-3-carboxamide (Compound 14):** ¹H-NMR (400 MHz, DMSO-d6): δ 9.05 (s, 1H), 8.44 (s, 1H), 7.87 (s, 2H), 7.46 (d, J = 8.72 Hz, 1H), 5.90 (d, J = 17.16 Hz, 1H), 5.52 (d, J = 17.20 Hz, 1H), 4.19 (q, J = 4.64 Hz, 1H), 3.57 - 3.49 (m, 2H), 2.68 (s, 3H), 2.65 (s, 3H), 2.38 - 2.33 (m, 1H), 1.91 (d, J = 4.16 Hz, 1H), 1.58 (q, J = 6.76 Hz, 1H), 1.27 - 1.15 (m, 5H), 0.99 - 0.94 (m, 4H), 0.80 - 0.77 (m, 6H).

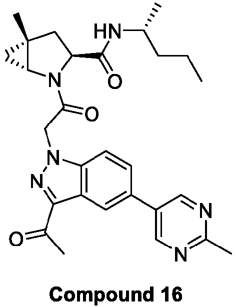

**(1*R*,3*S*,5*R*)-2-(2-(3-Acetyl-5-(2-methylpyrimidin-5-yl)-1*H*-indazol-1-yl)acetyl)-5-methyl-*N-*((*R*)-pentan-2-yl)-2-azabicyclo[3.1.0]hexane-3-carboxamide (Compound 16):** ¹H-NMR (400 MHz, DMSO-d6): δ 9.06 (s, 2H), 8.45 (s, 1H), 7.86 (s, 2H), 7.52 (d, J = 8.40 Hz, 1H), 5.90 (d, J = 17.36 Hz, 1H), 5.53 (d, J = 17.64 Hz, 1H), 4.16 - 4.15 (m, 1H), 3.75 - 3.68 (m, 1H), 3.50 (s, 1H), 2.69 (s, 3H), 2.66 (s, 3H), 2.40 - 2.34 (m, 2H), 1.90 (d, J = 7.72 Hz, 1H), 1.33 - 1.16 (m, 7H), 1.00 - 0.96 (m, 4H), 0.83 - 0.81 (m, 3H).

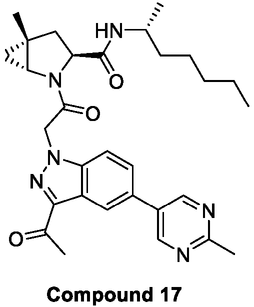

**(1*R*,3*S*,5*R*)-2-(2-(3-Acetyl-5-(2-methylpyrimidin-5-yl)-1*H*-indazol-1-yl)acetyl)-*N*-((*R*)-heptan-2-yl)-5-methyl-2-azabicyclo[3.1.0]hexane-3-carboxamide (Compound 17):** ¹H-NMR (400 MHz, DMSO-d6): δ 9.05 (s, 2H), 8.44 (s, 1H), 7.86 (s, 2H), 7.51 (d, J = 8.56 Hz, 1H), 5.89 (d, J = 17.44 Hz, 1H), 5.52 (d, J = 17.32 Hz, 1H), 4.17 - 4.14 (m, 1H), 3.73 - 3.70 (m, 1H), 3.50 (s, 1H), 2.69 (s, 3H), 2.66 (s, 3H), 2.40 - 2.34 (m, 1H), 1.91 - 1.87 (m, 1H), 1.33 - 1.27 (m, 5H), 1.24 - 1.18 (m, 7H), 1.00 - 0.95 (m, 4H), 0.77 - 0.76 (m, 3H).

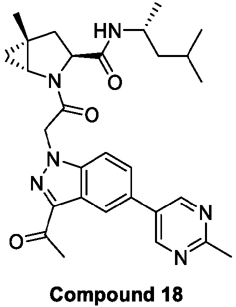

**(1*R*,3*S*,5*R*)-2-(2-(3-Acetyl-5-(2-methylpyrimidin-5-yl)-1*H*-indazol-1-yl)acetyl)-5-methyl-*N-*((*R*)-4-methylpentan-2-yl)-2-azabicyclo[3.1.0]hexane-3-carboxamide (Compound 18):** ¹H-NMR (400 MHz, DMSO-d6): δ 9.05 (s, 2H), 8.44 (s, 1H), 7.86 (s, 2H), 7.50 (d, J = 8.84 Hz, 1H), 5.89 (d, J = 17.28 Hz, 1H), 5.52 (d, J = 17.64 Hz, 1H), 4.17 (s, 1H), 3.84 - 3.81 (m, 1H), 3.48 (s, 1H), 2.69 (s, 3H), 2.66 (s, 3H), 2.41 - 2.33 (m, 1H), 1.89 (d, J = 12.84 Hz, 1H), 1.57 - 1.37 (m, 1H), 1.35 - 1.32 (m, 1H), 1.27 (s, 3H), 1.20 - 1.13 (m, 4H), 0.98 (d, J = 6.04 Hz, 4H), 0.88 (d, J = 5.80 Hz, 4H.

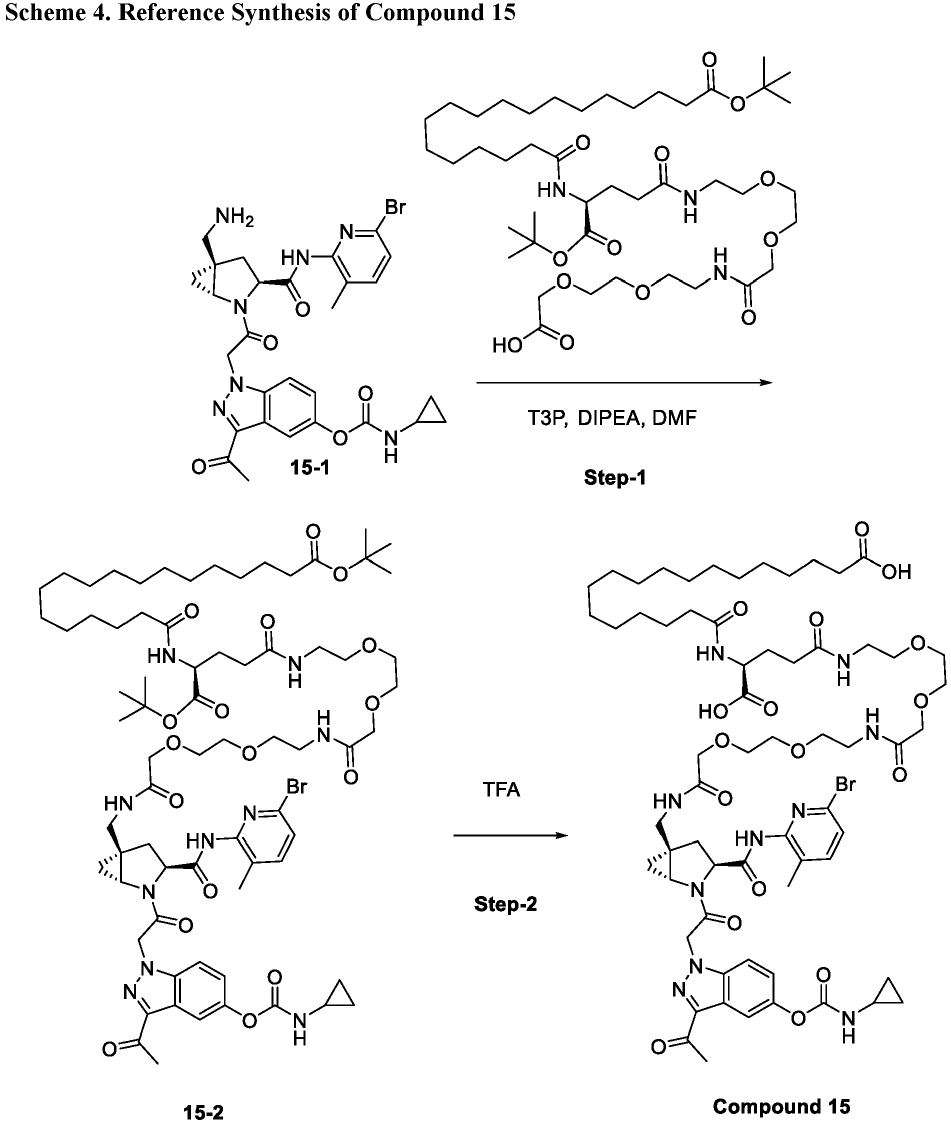

**Step 1: *tert*-Butyl (*S*)-1-((1*R*,3*S*,5*R*)-2-(2-(3-acetyl-5-((cyclopropylcarbamoyl)oxy)-1*H*-indazol-1-yl)acetyl)-3-((6-bromo-3-methylpyridin-2-yl)carbamoyl)-2-azabicyclo[3.1.0]hexan-5-yl)-24-(*tert*-butoxycarbonyl)-3,12,21,26-tetraoxo-5,8,14,17-tetraoxa-2,11,20,25-tetraazatritetracontan-43-oate (15-2):** To a solution of 3-acetyl-1-(2-((1*R*,3*S*,5*R*)-5-(aminomethyl)-3-((6-bromo-3-methylpyridin-2-yl)carbamoyl)-2-azabicyclo[3.1.0]hexan-2-yl)-2-oxoethyl)-1*H*-indazol-5-yl cyclopropylcarbamate in DMF (**15-1**, 10 vol) at 0 °C under nitrogen atmosphere were added (*S*)-22-(*tert*-butoxycarbonyl)-43,43-dimethyl-10,19,24,41-tetraoxo-3,6,12,15,42-pentaoxa-9,18,23-triazatetratetracontanoic acid (1.1 equiv), T3P (1.5 equiv) and DIPEA (5 equiv). The reaction mixture was stirred at room temperature for 16 hours. After completion of the reaction, the reaction mixture was quenched with water. The resulting mixture was extracted with DCM. The organic layer was washed with brine, dried over anhydrous Na₂SO₄, filtered and then concentrated. The residue was purified by column chromatography on silica gel using DCM/MeOH to afford compound 15-2.

**Step 2: (*S*)-1-((1*R*,3*S*,5*R*)-2-(2-(3-acetyl-5-((cyclopropylcarbamoyl)oxy)-1*H*-indazol-1-yl)acetyl)-3-((6-bromo-3-methylpyridin-2-yl)carbamoyl)-2-azabicyclo[3.1.0]hexan-5-yl)-24-carboxy-3,12,21,26-tetraoxo-5,8,14,17-tetraoxa-2,11,20,25-tetraazatritetracontan-43-oic acid (Compound 15):** To a solution of compound **15-2** (1 equiv) in DCM (10 vol) at 0 °C was added TFA (5 vol). The reaction mixture was stirred at room temperature for 30 minutes. The volatiles were removed under reduced pressure and the resulting residue was purified by preparative purification to afford **Compound 15.** ¹H NMR (400 MHz, CD3OD) δ 7.99 (s, 1H), 7.65 (d, J = 7.8 Hz, 1H), 7.55 (d, J = 7.8 Hz, 1H), 7.40 (d, J = 7.6 Hz, 1H), 7.25 (d, J = 7.6 Hz, 1H), 5.75 (d, J = 17.2 Hz, 1H), 5.63 (d, J = 17.2 Hz, 1H), 4.64 - 4.62 (m, 1H), 4.40 - 4.38 (m, 1H), 4.11 - 4.00 (m, 4H), 3.77 - 3.32 (m, 17H), 2.67 (s, 4H), 2.53 - 2.29 (m, 6H), 1.86 - 1.80 (m, 5H), 1.66 - 1.64 (m, 5H), 1.39 - 1.29 (m, 27H), 0.75 - 0.61 (m, 5H).

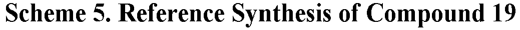

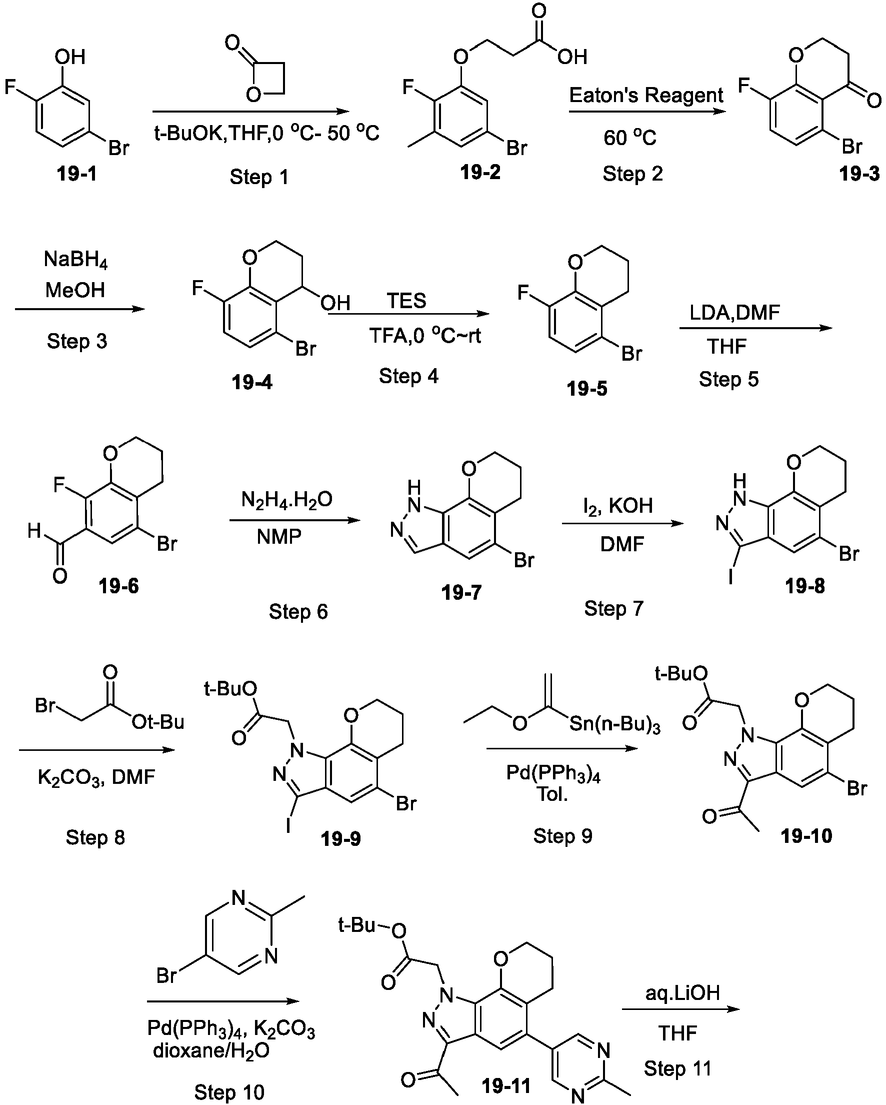

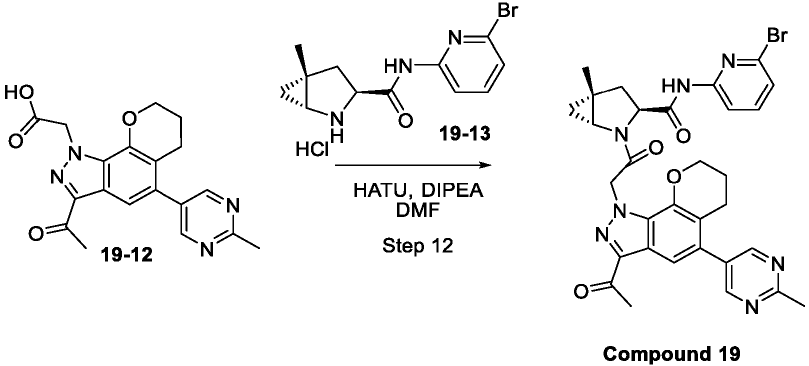

**Step 1: 3-(5-Bromo-2-fluorophenoxy)propanoic acid (19-2):** To a solution of compound **19-1** (13.8 g, 73 mmol) in THF (140 mL) was added t-BuOK (76.7 mL, 1 M in THF) drop-wise at 0 °C under N₂ atmosphere followed by oxetan-2-one (5.8 g, 80.3 mmol). After addition, the mixture was stirred at 50°C under N₂ atmosphere for 16 hours. The mixture was quenched with 1 M aqueous HCl and the pH was adjusted to 3 and extracted with EtOAc twice. The combined organic layers were washed with brine, dried with anhydrous Na₂SO₄, filtered and concentrated to dryness to afford compound **19-2** (16 g, 83.8% yield) as a white solid. LC/MS (ESI) m/z: 261/263(M-H)⁻.

**Step 2: 5-Bromo-8-fluorochroman-4-one (19-3):** A solution of compound **19-2** (16 g, 61 mmol) in Eaton's Reagent (100 mL) was stirred at 60 °C for 2 hours. The mixture was quenched with ice-cooled water and extracted with EtOAc twice. The combined organic layers were washed with brine, dried with anhydrous Na₂SO₄, filtered and concentrated to dryness. The residue was purified by chromatography on silica gel (eluted with PE: EtOAc = 40: 1) to afford compound **19-3** (11 g, 73.9% yield) as a yellow solid. LC/MS (ESI) m/z: 245/247(M+H)⁺.

**Step 3: 5-bromo-8-fluorochroman-4-ol (19-4):** To a solution of compound **19-3** (11 g, 44.7 mmol) in MeOH (100 mL) and was added NaBH₄ (2.55 g, 67.1 mmol) at 0 °C and the mixture was stirred at 0°C for 0.5 hour. The mixture was quenched with ice-cooled water and extracted with DCM. The combined organic layers were washed with brine, dried with anhydrous Na₂SO₄, filtered and concentrated to dryness to afford compound **19-4** (11.6 mg, yield 100%) as a light yellow oil that was directly used in the next reaction without purification. LC/MS (ESI) m/z: 247/249 (M+H)⁺.

**Step 4: 5-Bromo-8-fluorochroman (19-5):** To a solution of compound **19-4** (11.6 g, 44.7 mmol) in TFA (100 mL) and was added triethylsilane (26 g, 223.5 mmol) at 0 °C and the mixture was stirred at 25°C for 1 hour. The mixture was quenched with ice-cooled water and extracted with DCM twice. The combined organic layers were washed with brine, dried with anhydrous Na₂SO₄, filtered and concentrated to dryness. The residue was purified by chromatography on silica gel (eluted with PE: EtOAc = 60: 1) to afford compound **19-5** (8.8 g, 85.6% yield) as a white solid.

**Step 5: 5-Bromo-8-fluorochroman-7-carbaldehyde (19-6):** To a solution of compound **19-5** (8.8 g, 38.3 mmol) in THF (100 mL) was added LDA (24.9 mL, 2 M in THF) drop-wise at - 78°C under N₂ atmosphere and the mixture was stirred at -78°C for 0.5 hour. N,N-dimethylformamide (4.2 g, 57.5 mmol) was added and the resulting mixture was stirred at 25°C under N₂ atmosphere for 1 hour. The mixture was quenched with saturated aqueous NH₄Cl solution and extracted with EtOAc twice. The combined organic layers were washed with brine, dried over anhydrous Na₂SO₄, filtered and concentrated to dryness. The residue was purified by chromatography on silica gel (eluted with PE: EtOAc = 60: 1) to afford compound **19-6** (9 g, 91.8% yield) as a white solid.

**Step 6: 5-Bromo-1,6,7,8-tetrahydropyrano[3,2-g]indazole (19-7):** To a solution of compound **19-6** (1 g, 3.88 mmol) in NMP (3 mL) was added hydrazine hydrate (0.97 g, 19.4 mmol) and K₂CO₃ (1.6 g, 11.6 mmol). After addition, the mixture was stirred in CEM microwave reactore at 160 °C for 1 hour. The mixture was diluted with EtOAc and washed with saturated aqueous NH₄Cl solution and brine, dried with anhydrous Na₂SO₄, filtered and concentrated to dryness. The residue was purified by chromatography on silica gel (eluted with PE: EtOAc = 10: 1) to afford compound **19-7** (220 mg, 22.6% yield) as a white solid. LC/MS (ESI) m/z: 253/255 (M+H)⁺.

**Step 7: 5-Bromo-3-iodo-1,6,7,8-tetrahydropyrano[3,2-g]indazole (19-8):** To a solution of compound **19-7** (220 mg, 0.87 mmol) in DMF (5 mL) was added KOH (110 mg, 1.96 mmol) and I₂ (332 mg, 1.3 mmol) at 0 °C and the mixture was stirred at room temperature for 1 hour. The mixture was diluted with water and extracted with EtOAc twice. The combined organic layers were washed with 5% aqueous Na₂S₂O₃ solution and brine, dried over anhydrous Na₂SO₄, filtered and concentrated to dryness to afford compound **19-8** (220 mg, yield 67.1%) as a yellow solid that was used directly in the next step. LC/MS (ESI) m/z: 379/381 (M+H)⁺.

**Step 8: *tert*-Butyl 2-(5-bromo-3-iodo-7,8-dihydropyrano[3,2-g]indazol-1(6*H*)-yl)acetate (19-9):** To a solution of compound **19-8** (220 mg, 0.58 mmol) in DMF (5 mL) was added K₂CO₃ (201 mg, 1.45 mmol) followed by tert-butyl 2-bromoacetate (69 mg, 0.35 mmol) and the reaction mixture was stirred at room temperature overnight. The mixture was diluted with water and extracted with EtOAc twice. The combined organic layers were washed with brine, dried and concentrated under reduced pressure. The residue was purified by chromatography on silica gel (eluted with PE: EtOAc = 80: 1 to 50: 1) to afford compound **19-9** (150 mg, 52.6% yield) as a white solid. LC/MS (ESI) m/z: 493/495 (M+H)⁺.

**Step 9: *tert*-Butyl 2-(3-acetyl-5-bromo-7,8-dihydropyrano[3,2-g]indazol-1(6*H*)-yl)acetate (19-10):** To a mixture of compound 19-9 (150 mg, 0.30 mmol) and tributyl(1-ethoxyethenyl)stannane (110 mg, 0.30 mmol) in toluene (5 mL) was added Pd(PPh₃)₄ (35 mg, 0.03 mmol). The mixture was degassed under N₂ atmosphere three times and stirred at 100 °C under N₂ atmosphere overnight. The mixture was concentrated to dryness and the residue was dissolved in THF (3 mL) and MeOH (3 mL) and 0.5 N aqueous HCl solution (3 mL) was added to the mixture. The mixture was stirred at room temperature for 1 hour. The mixture was extracted with EtOAc, washed with brine, dried and concentrated to dryness. The residue was purified by silica gel chromatography (eluted with PE: EtOAc= 30: 1) to afford compound **19-10** (110 mg, yield 89.9%) as a yellow solid. LC/MS (ESI) m/z: 409/411 (M+H)⁺.

**Step 10: *tert*-butyl 2-(3-acetyl-5-(2-methylpyrimidin-5-yl)-7,8-dihydropyrano[3,2-g]indazol-1(6H)-yl)acetate (19-11):** To a mixture of compound **19-10** (110 mg, 0.27 mmol) and 2-methyl-5-(4,4,5,5-tetramethyl-1,3,2-dioxaborolan-2-yl)pyrimidine (71 mg, 0.32 mmol) in 1,4-dioxane (5 mL) and water (5 mL) was added K₂CO₃ (112 mg, 0.81 mmol) and Pd(PPh₃)₄ (31 mg, 0.03 mmol). The mixture was degassed under N₂ atmosphere three times and stirred at 90 °C under N₂ atmosphere for 2 hours. The mixture was diluted with EtOAc, washed with water and brine, dried over Na₂SO₄, filtered and concentrated to afford crude product that was purified by silica gel chromatography (eluted with PE: EtOAc= 2: 1) to afford compound **19-11** (80 mg, yield 70.2%) as a yellow solid. LC/MS (ESI) m/z: 423 (M+H)⁺.

**Step 11: 2-(3-acetyl-5-(2-methylpyrimidin-5-yl)-7,8-dihydropyrano[3,2-g]indazol-1(6H)-yl)acetic acid:** To a solution of compound **19-11** (80 mg, 0.19 mmol) in MeOH (3 mL) and THF (3 mL) was added a solution of LiOH (24 mg, 0.57 mmol) in water (3 mL) at 0 °C and the mixture was stirred at room temperature for 1 hour. The reaction mixture was concentrated to dryness and the residue was dissolved in water. The mixture was washed with tert-butyl methyl ether and acidified with 1 N aqueous HCl to a pH of approximately 3. The mixture was extracted with DCM twice and the combined organic layers were washed with brine, dried over Na₂SO₄, filtered and concentrated to dryness to afford compound **19-12** (68 mg, yield 97.8%) as a yellow solid. LC/MS (ESI) m/z: 367 (M+H)⁺.

**Step 12: 2-(3-Acetyl-5-(5-methylpyrimidin-2-yl)-7,8-dihydropyrano[3,2-g]indazol-1(6*H*)-yl)acetic acid. (Compound 19):** To a mixture of compound **19-12** (34 mg, 0.09 mmol) and (1R,3S,5R)-N-(6-bromopyridin-2-yl)-5-methyl-2-azabicyclo[3.1.0]hexane-3-carboxamide hydrochloride (30 mg, 0.09 mmol) in DMF (5 mL) was added DIPEA (46 mg, 0.36 mmol) at 0 °C followed by HATU (62 mg, 0.16 mmol). After addition, the mixture was stirred at 25 °C for 2 hours. The mixture was diluted with EtOAc, washed with saturated aqueous NH₄Cl solution and brine successively, dried over Na₂SO₄, filtered and concentrated to afford crude product that was purified by preparative HPLC to afford **Compound 19** (10 mg, yield 17.3%) as a white solid. ¹H-NMR (400 MHz, CD₃OD) δ 8.71 (s, 2H), 8.09 (d, J= 8.3 Hz, 1H), 7.55- 7.67 (m, 2H), 7.26 (d, J= 7.7 Hz, 1H), 5.85 (d, J= 16.9 Hz, 1H), 5.74 (d, J= 16.9 Hz, 1H), 4.49-4.53 (m, 1H), 4.34 (t, J= 5.1 Hz, 2H), 3.46-3.48 (m, 1H), 2.75 (s, 3H), 2.61-2.66 (m, 5H), 2.51-2.57 (m, 1H), 2.21-2.26 (m, 1H), 1.96-2.00 (m, 2H), 1.38 (s, 3H), 1.10 (t, J= 5.3 Hz, 1H), 0.92- 0.95 (m, 1H). LC/MS (ESI) m/z: 644/646 (M+H)⁺. 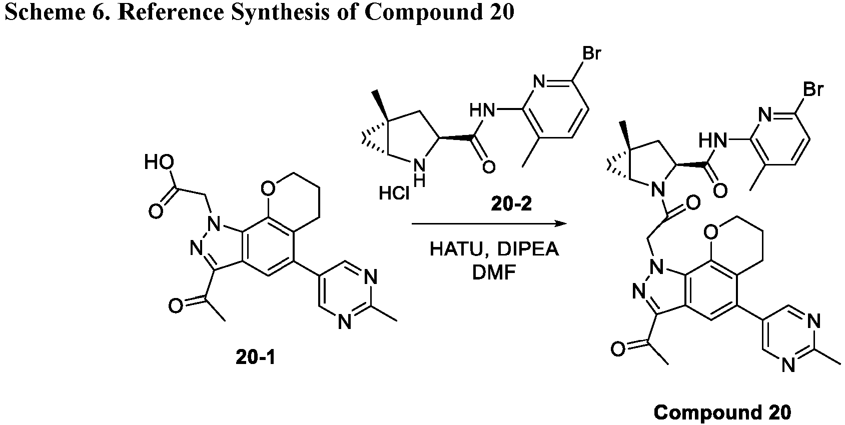 To a mixture of compound 20-1 (34 mg, 0.09 mmol) and (1R,3S,5R)-N-(6-bromo-3-methylpyridin-2-yl)-5-methyl-2-azabicyclo[3.1.0]hexane-3-carboxamide hydrochloride (20-2, 31 mg, 0.09 mmol) in DMF (5 mL) was added DIPEA (46 mg, 0.36 mmol) at 0 °C followed by HATU (62 mg, 0.16 mmol). After addition, the mixture was stirred at 25 °C for 2 hours. The mixture was diluted with EtOAc, washed with saturated aqueous NH₄Cl solution and brine successively, dried over Na₂SO₄, filtered and concentrated to afford crude product that was purified by preparative HPLC to afford **Compound 20** (12 mg, yield 20.3%) as a white solid. ¹H-NMR (400 MHz, CD₃OD) δ 8.71 (s, 2H), 7.66 (s, 1H), 7.55 (d, J = 8.0 Hz, 1H), 7.38 (d, J = 7.9 Hz, 1H), 5.80 (d, J= 1.1 Hz, 2H), 4.52-4.56 (m, 1H), 4.31 -4.34 (m, 2H), 3.45-3.46 (m, 1H), 2.76 (s, 3H), 2.57-2.66 (m, 6H), 2.30-2.35 (m, 1H), 2.13 (s, 3H), 1.95-2.05 (m, 2H), 1.40 (s, 3H), 1.11 (t, J= 5.4 Hz, 1H), 0.92-0.94 (m, 1H). LC/MS (ESI) m/z: 658/660 (M+H)⁺.

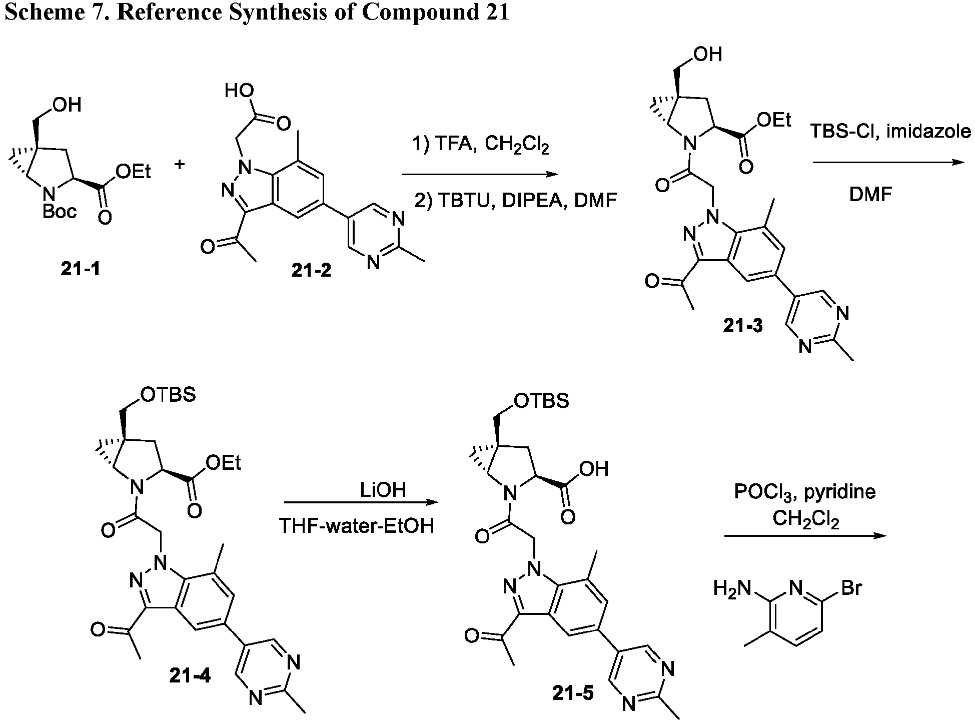

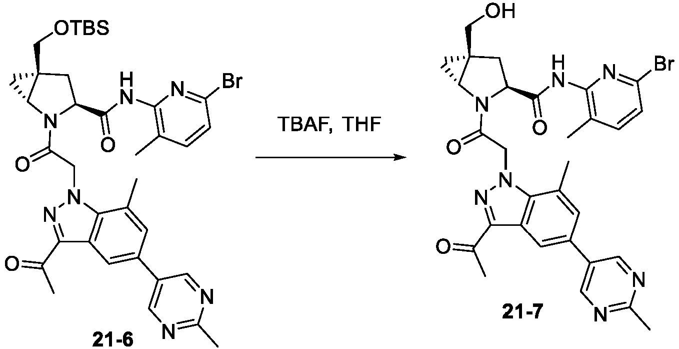

**Step 1. Ethyl (1*R*,3*S*,5*S*)-2-(2-(3-Acetyl-7-methyl-5-(2-methylpyrimidin-5-yl)-1*H-*indazol-1-yl)acetyl)-5-(hydroxymethyl)-2-azabicyclo[3.1.0]hexane-3-carboxylate (21-3):** 2-*tert*-Butyl 3-ethyl (1R,3S,5S)-5-(hydroxymethyl)-2-azabicyclo[3.1.0]hexane-2,3-dicarboxylate (0.11 g, 0.385 mmol, 1 equiv.) was stirred in a 1:1 mixture of dichloromethane and TFA at room temperature for 15 minutes. The volatiles were removed under reduced pressure. The residue was taken up in DMF (1 mL) and [3-acetyl-7-methyl-5-(2-methylpyrimidin-5-yl)indazol-1-yl]acetic acid (0.125 g) was added. The heterogeneous mixture was cooled in an ice bath. To this solution were added DIPEA (0.306 mL) and TBTU (0.135 g). The cooling bath was removed and the resulting homogeneous solution was stirred for 30 minutes at room temperature. Solvent was removed under reduced pressure and the residue was purified by silica gel column chromatography (4 g column, eluent: 0-4 % MeOH in dichloromethane) to afford 0.18 g of a cream colored solid.

**Step 2. Ethyl (1*R*,3*S*,5*S*)-2-(2-(3-acetyl-7-methyl-5-(2-methylpyrimidin-5-yl)-1*H-*indazol-1-yl)acetyl)-5-(((*tert*-butyldimethylsilyl)oxy)methyl)-2-azabicyclo[3.1.0]hexane-3-carboxylate (21-4):** To a solution of ethyl (1R,3S,5S)-2-{2-[3-acetyl-7-methyl-5-(2-methylpyrimidin-5-yl)indazol-1-yl]acetyl}-5-(hydroxymethyl)-2-azabicyclo[3.1.0]hexane-3-carboxylate (0.18 g, 0.366 mmol, 1 equiv.) in DMF (5 mL) were added imidazole (0.075 g, 1.099 mmol, 3 equiv.) and TBS-Cl (0.072 g, 0.478 mmol, 1.305 equiv.). The reaction was then stirred at room temperature overnight. The solvent was removed under reduced pressure. The residue was purified by silica gel column chromatography (eluent:0-2 % MeOH in dichloromethane) to afford 0.15 g of colorless resin.

**Step 3. (1*R*,3*S*,5*S*)-2-(2-(3-Acetyl-7-methyl-5-(2-methylpyrimidin-5-yl)-1*H*-indazol-1-yl)acetyl)-5-(((*tert*-butyldimethylsilyl)oxy)methyl)-2-azabicyclo[3.1.0]hexane-3-carboxylic acid (21-5):** To a solution of ethyl (1*R*,3*S*,5*S*)-2-{2-[3-acetyl-7-methyl-5-(2-methylpyrimidin-5-yl)indazol-1-yl] acetyl }-5-{ [(tert-butyldimethylsilyl)oxy]methyl }-2-azabicyclo[3 .1.0]hexane-3-carboxylate (0.155 g, 0.256 mmol, 1 equiv.) in THF (2 mL) -EtOH (1 mL) in an ice-bath was added LiOH (16 mg) in water (1 mL). The resulting solution was stirred for an hour at room temperature and then diluted with water (2 mL) and the organic solvents were removed under reduced pressure. The aqueous residue was then extracted once with ether and the organic layer was discarded. The aqueous layer was cooled in an ice-bath and acidified with cold 1*N* aqueous HCl. The organic layer was extracted with chloroform. The organic layer was then dried with Na₂SO₄ and concentrated to afford 90 mg of a white solid that was used directly in the next step.

**Step 4. (1*R*,3*S*,5*S*)-2-(2-(3-Acetyl-7-methyl-5-(2-methylpyrimidin-5-yl)-1*H*-indazol-1-yl)acetyl)-*N*-(6-bromo-3-methylpyridin-2-yl)-5-(((*tert*-butyldimethylsilyl)oxy)methyl)-2-azabicyclo[3.1.0]hexane-3-carboxamide (21-6):** To an ice-cold solution of (1R,3S,5S)-2-{2-[3-acetyl-7-methyl-5-(2-methylpyrimidin-5-yl)indazol-1-yl]acetyl}-5-{[(tertbutyldimethylsilyl)oxy]methyl}-2-azabicyclo[3.1.0]hexane-3-carboxylic acid (0.171 mmol, 1 equiv.) in dichloromethane were added 6-bromo-3-methylpyridin-2-amine (0.032 g, 0.171 mmol), pyridine (0.064 mL) and phosphorous oxychloride (0.014 mL). The reaction was stirred at 0 ° C for 1 hour and quenched by the careful addition of saturated aqueous sodium bicarbonate solution. The reaction was then stirred at room temperature for 15 minutes. The layers were separated and the aqueous layer was extracted with dichloromethane. The combined organic layer was dried with Na₂SO₄ and concentrated. The residue was purified by silica gel column chromatography (eluent: 0-2 % MeOH in dichloromethane) to afford 70 mg of a white solid.

**Step 5. (1*R*,3*S*,5*S*)-2-(2-(3-Acetyl-7-methyl-5-(2-methylpyrimidin-5-yl)-1*H*-indazol-1-yl)acetyl)-N-(6-bromo-3-methylpyridin-2-yl)-5-(hydroxymethyl)-2-azabicyclo[3.1.0]hexane-3-carboxamide (Compound 21):** To a solution of (1*R*,3*S*,5*S*)-2-{2-[3-acetyl-7-methyl-5-(2-methylpyrimidin-5-yl)indazol-1-yl]acetyl}-*N*-(6-bromo-3-methylpyridin-2-yl)-5-{[(*tert*butyldimethylsilyl)oxy]methyl}-2-azabicyclo[3.1.0]hexane-3-carboxamide (0.07 g, 0.094 mmol, 1 equiv.) in THF was added TBAF (0.094 mL, 1.0 M in THF) at room temperature and the reaction was stirred for 1 hour at room temperature. Then the volatiles were removed under reduced pressure. The residue was purified by silica gel flash column chromatography (eluent: 0-4 % MeOH in dichloromethane) to afford 35 mg of a white solid. ¹H NMR (400 MHz, DMSO-*d*₆) δ 0.87 - 1.00 (m, 1H), 1.19 (t, *J =* 5.2 Hz, 1H), 2.05 (s, 3H), 2.24 (dd, *J =* 4.9, 13.4 Hz, 1H), 2.22-2.51 (m, 1H), 2.64 (s, 3H), 2.68 (s, 6H), 3.54 (s, 2H), 3.66 (dd, *J =* 2.5, 5.7 Hz, 1H), 4.26 (brs, 1H), 4.48 (dd, *J =* 5.1, 9.3 Hz, 1H), 5.70 (d, *J =* 17.7 Hz, 1H), 6.03 (d, *J =* 17.9 Hz, 1H), 7.43 (d, *J =* 7.9 Hz, 1H), 7.61,(s, 1H), 7.62 (d, *J* = 7.7 Hz, 1H), 8.32 (d, *J =* 1.7 Hz, 1H), 9.03 (s, 2H), 10.27 (s, 1H).

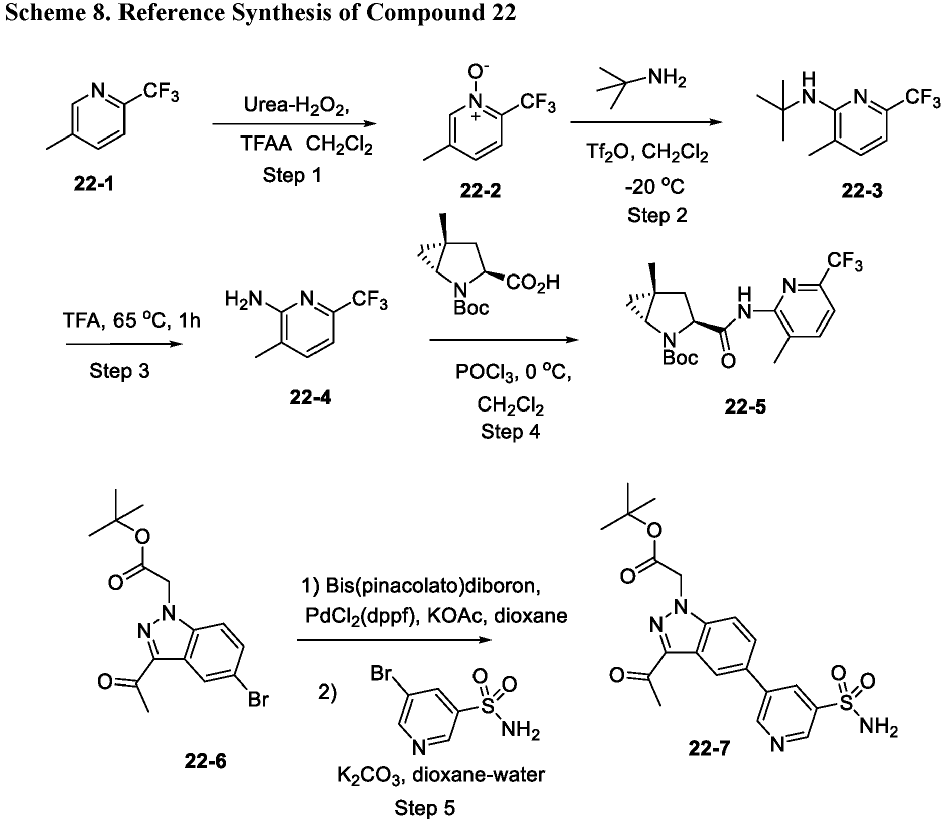

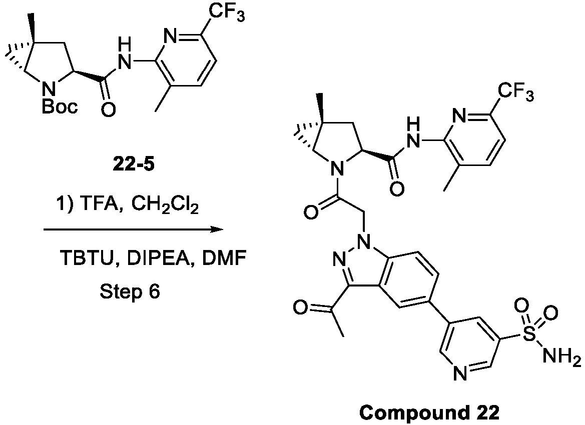

**Step 1: 5-Methyl-2-(trifluoromethyl)pyridine-*N*-oxide (22-2):** 5-Methyl-2-(trifluoromethyl)pyridine (**22-1,** 1 g, 6.206 mmol, 1 equiv.) was taken up in dichloromethane and the solution was cooled in an ice bath. Powdered urea hydrogen peroxide complex (0.899 g, 9.557 mmol, 1.54 equiv.) was added followed by the dropwise addition of trifluoroacetic anhydride (3.26 g, 2.158 mL, 15.522 mmol, 2.501 equiv.). The cooling bath was removed and the reaction was stirred overnight at room temperature. The reaction was quenched by the careful addition of saturated sodium metabisulfite solution and the reaction was stirred at room temperature for 15 minutes. Then the solution was diluted with dichloromethane and made basic by the careful addition of saturated aqueous sodium bicarbonate solution. The organic layer was separated and the aqueous layer was extracted with dichloromethane. The combined organic layers were dried and concentrated to afford orange red oil that was used directly in the next step.

**Step 2: N-(*tert*-Butyl)-3-methyl-6-(trifluoromethyl)pyridin-2-amine (22-3):** To a stirred solution of 5-methyl-2-(trifluoromethyl)pyridine-*N*-oxide (**22-2,** 1.05 g, 5.928 mmol, 1 equiv.) in dichloromethane (25 mL) at -20 ° C was added *tert*-butylamine (3.21 mL). Triflic anhydride (3 mL) was then added dropwise and the reaction was stirred at -20 ° C for 1 hour before being quenched by the addition of water. The reaction was stirred at room temperature for 15 minutes. The layers were separated and the aqueous layer was extracted once with dichloromethane. The combined organic layers were dried and concentrated. The residue was purified by silica gel column chromatography (eluent: hexane) to afford a colorless liquid.

**Step 3: 3-Methyl-6-(trifluoromethyl)pyridin-2-amine (22-4):** A solution of *N-tert-*butyl-3-methyl-6-(trifluoromethyl)pyridin-2-amine (**22-3**, 0.72 g, 3.1 mmol, 1 equiv.) in TFA was stirred at 65 ° C for 1 hour. The reaction was cooled to room temperature and the volatiles were removed under reduced pressure. The residue was taken up in dichloromethane and made basic with saturated aqueous NaHCO₃ solution. The organic layer was separated, dried and concentrated to afford 0.49 g of a white solid that was used directly in the next step.

**Step 4: *tert*-Butyl (1R,3S,SR)-5-methyl-3-((3-methyl-6-(trifluoromethyl)pyridin-2-yl)carbamoyl)-2-azabicyclo[3.1.0]hexane-2-carboxylate (22-5):** To an ice cold solution of 3-methyl-6-(trifluoromethyl)pyridin-2-amine (**22-5**, 0.39 g, 2.214 mmol, 1 equiv.) and (1*R*,3*S*,5*R*)-2-(tert-butoxycarbonyl)-5-methyl-2-azabicyclo[3.1.0]hexane-3-carboxylic acid (0.588 g, 2.436 mmol, 1.1 equiv.) in dichloromethane (8 mL), pyridine (0.9 mL) was added followed by the dropwise addition of POCl₃ (0.21 mL). The reaction was stirred at 0 ° C for 1 hour and was quenched by the addition of saturated aqueous NaHCO₃ solution. The cooling bath was removed and the reaction was stirred at room temperature for 15 minutes. Then the layers were separated and the organic layer was washed with cold 1*N* aqueous HCl and water. The organic layer was dried and concentrated to afford 0.85 g of a white solid that was used directly in the next step.

**Step 5: *tert*-Butyl 2-(3-acetyl-5-(5-sulfamoylpyridin-3-yl)-1*H*-indazol-1-yl)acetate (22-7):** A heterogeneous solution of tert-butyl 2-(3-acetyl-5-bromoindazol-1-yl)acetate (**22-6**, 0.41 g, 1.161 mmol), bis(pinacolato)diboron (0.402 g, 1.583 mmol), potassium acetate (0.207 g, 2.109 mmol) and PdCl₂(dppf) (0.043 g) in dioxane (10 mL) was degassed with argon and heated overnight at 90 ° C under argon. The reaction was cooled to room temperature and 5-bromopyridine-3-sulfonamide (0.25 g, 1.055 mmol) and potassium carbonate (0.437 g, 3.162 mmol) were added. Water (2.5 mL) was added and the reaction was degassed with argon. The reaction was then heated at 100 ° C for 3 hours. The volatiles were then removed under reduced pressure. The residue was purified by silica gel column chromatography (eluent: 0-3 % MeOH in dichloromethane) to afford 0.36 g of a cream colored solid.

**Step 6: (1*R*,3*S*,5*R*)-2-(2-(3-Acetyl-5-(5-sulfamoylpyridin-3-yl)-1*H*-indazol-1-yl)acetyl)-5-methyl-*N*-(3-methyl-6-(trifluoromethyl)pyridin-2-yl)-2-azabicyclo[3.1.0]hexane-3-carboxamide (Compound 22):** *tert*-Buty*l* 2-[3-acetyl-5-(5-sulfamoylpyridin-3-yl)indazol-1-yl]acetate (**22-7**, 0.05 g, 0.116 mmol) was stirred with TFA (2 mL) and dichloromethane (1 mL) for 3 hours at room temperature. In another vial, tert-butyl (1R, 3S,5R)-5-methyl-3-{[3-methyl-6-(trifluoromethyl)pyridin-2-yl]carbamoyl}-2-azabicyclo[3.1.0]hexane-2-carboxylate (**22-5**, 0.046 g, 0.116 mmol) was stirred with TFA (1 mL) and dichloromethane (1 mL) at room temperature for 15 minutes. The contents of the two vials were combined and the volatiles were removed under reduced pressure. The residue was dissolved in DMF (1 mL) and cooled in an ice bath. DIPEA (0.101 mL, 0.116 mmol) and TBTU (0.041 g, 0.128 mmol) were added to the solution. The cooling bath was then removed and the reaction was stirred at room temperature for 30 minutes. The solvent was removed under reduced pressure and the residue was dissolved in chloroform. The organic layers were washed with saturated aqueous sodium bicarbonate solution. The separated organic layers were dried (Na₂SO₄) and concentrated. The residue was purified by silica gel column chromatography (eluent: 0-3.5 % MeOH in dichloromethane) to afford a white solid. ¹H NMR (400 MHz, DMSO-*d*₆) δ 0.97 - 1.08 (m, 2H), 1.33 (s, 3H), 2.07 (dd, *J =* 5.2, 13.3 Hz, 1H), 2.18 (s, 3H), 2.51 - 2.66 (m, 1H), 2.66 (s, 3H), 3.61 (dd, *J =* 2.5, 5.5 Hz, 1H), 4.47 (dd, *J =* 5.1, 9.3 Hz, 1H), 5.59 (d, *J =* 17.2 Hz, 1H), 5.94 (d, *J =* 17.3 Hz, 1H), 7.66 - 7.74 (m, 3H), 7.85 - 7.98 (m, 3H), 8.45 - 8.53 (m, 2H), 8.97 (d, *J =* 2.1 Hz, 1H), 9.16 (d, *J =* 2.1 Hz, 1H), 10.42 (s, 1H). ¹⁹ F : δ -65.99.

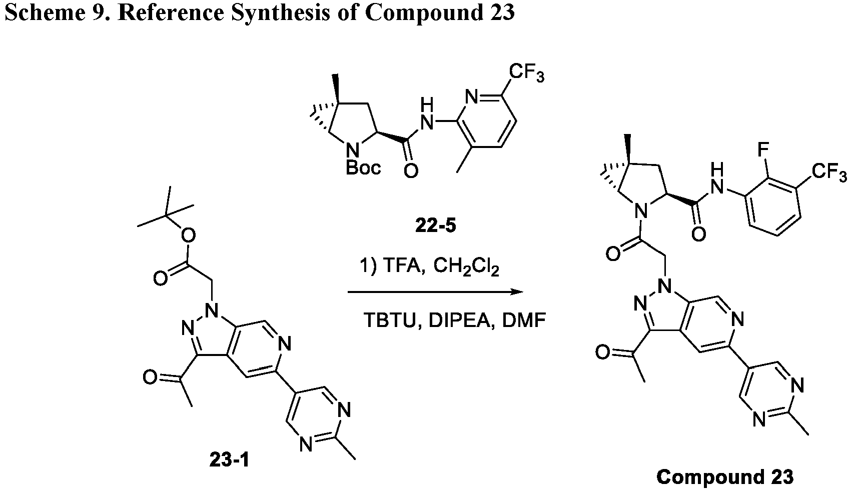

**(1*R*,3*S*,5*R*)-2-(2-(3-Acetyl-5-(2-methylpyrimidin-5-yl)-1*H*-pyrazolo[3,4-C]pyridine-1-yl)acetly)-*N*-(2-fluoro-3-(trifluoromethyl)phenyl)-5-methyl-2-azabicyclo[3.1.0]hexane-3-carboxamide (Compound 23):** *tert*-Butyl 2-[3-acetyl-5-(2-methylpyrimidin-5-yl)pyrazolo[3,4-c]pyridin-1-yl]acetate (**23-1**, 0.05 g, 0.136 mmol, 1 equiv.) was stirred with 1 mL of TFA and 0.5 mL of dichloromethane at room temperature for 3 hours. In another vial, tert-butyl (1*R*,3*S*,5*R*)-3-{[2-fluoro-3-(trifluoromethyl)phenyl]carbamoyl}-5-methyl-2-azabicyclo[3.1.0]hexane-2-carboxylate (**22-**5, 0.055 g, 0.136 mmol, 1 equiv.) was stirred with 0.5 mL of TFA and 0.5 mL of dichloromethane for 30 minutes at room temperature. The contents of both the vials were combined and the volatiles were removed under reduced pressure. The residue was taken up in DMF (1 mL) and cooled in an ice bath. DIPEA (0.088 g, 0.119 mL, 0.68 mmol, 5 equiv.) and TBTU (0.048 g, 0.15 mmol, 1.1 equiv.) were added to this reaction. The cooling bath was removed and the reaction was stirred at room temperature for 30 minutes. The solvent was removed under reduced pressure and the residue was dissolved in dichloromethane. The organic layer was washed with saturated aqueous sodium bicarbonate, dried and concentrated. The residue was purified by silica gel flash column chromatography (eluent: 0-2.5 % MeOH in dichloromethane) to afford a white solid. ¹H NMR (400 MHz, DMSO-*d*₆) δ 1.01 (t, *J =* 5.5 Hz, 1H), 1.08 (dd, *J* = 2.4, 5.4 Hz, 1H), 1.33 (s, 3H), 2.08 (dd, *J =* 5.5, 13.2 Hz, 1H), 2.48-2.54 (m, 1H), 2.68 (s, 3H), 2.69 (s, 3H), 3.62 (dd, *J =* 2.4, 5.6 Hz, 1H), 4.51 (dd, *J* = 5.4, 9.1 Hz, 1H), 5.74 (d, *J =* 17.2 Hz, 1H), 6.08 (d, *J* = 17.3 Hz, 1H), 7.36 (t, *J* = 8.0 Hz, 1H), 7.52 (t, *J =* 6.9 Hz, 1H), 8.07 (t, *J =* 7.6 Hz, 1H), 8.61 (d, *J= 1.3* Hz, 1H), 9.33 (d, J = 1.2 Hz, 1H), 9.35 (s, 2H), 9.97 (s, 1H). ¹⁹F NMR (376 MHz, DMSO-*d*₆) δ , -59.98 (d, *J* = 11.3 Hz), -126.97 (q, J = 11.3 Hz).

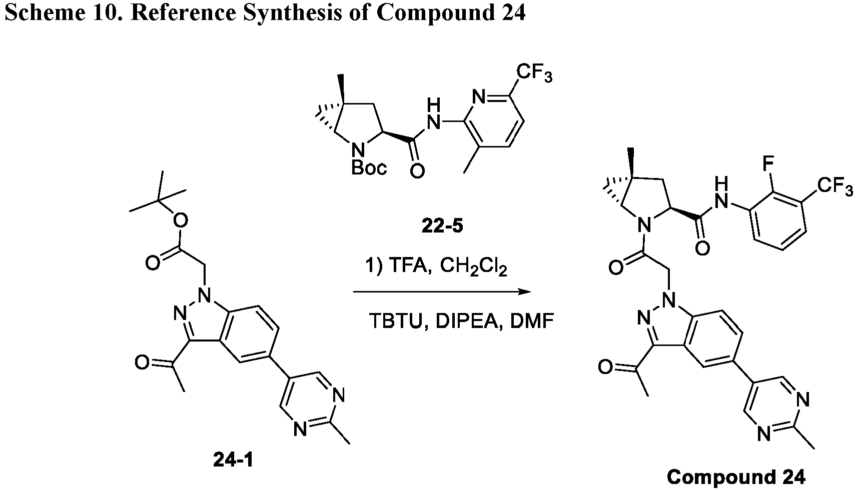

**(1*R*,3*S*,5*R*)-2-(2-(3-Acetyl-5-(2-methylpyrimidin-5-yl)-1*H*-indazol-1-yl)acetyl)-*N*-(2-fluoro-3-(trifluoromethyl)phenyl)-5-methyl-2-azabicyclo[3.1.0]hexane-3-carboxamide (Compound 24):** *tert*-butyl (1*R*,3*S*,5*R*)-3-{[2-fluoro-3-(trifluoromethyl)phenyl]carbamoyl}-5-methyl-2-azabicyclo[3.1.0]hexane-2-carboxylate (**22-5,** 0.051 g, 0.126 mmol, 1 equiv.) was stirred with 0.5 mL of TFA and 0.5 mL of dichloromethane at room temperature for 30 minutes. Then the volatiles were removed under reduced pressure. The residue was taken up in DMF (1 mL) and cooled in an ice bath. [3-Acetyl-5-(2-methylpyrimidin-5-yl)indazol-1-yl]acetic acid (**24-1**, 0.039 g, 0.126 mmol, 1 equiv.), DIPEA (0.081 g, 0.109 mL, 0.628 mmol, 5 equiv.) and TBTU (0.044 g, 0.138 mmol, 1.1 equiv.) were added. The reaction was stirred at room temperature for 30 minutes. The DMF was removed under reduced pressure. The residue was dissolved in dichloromethane and washed with saturated aqueous sodium bicarbonate solution. The organic layer was dried and concentrated. The residue was purified by silica gel flash chromatography (eluent: 0-2 % MeOH in dichloromethane) to afford a white solid. ¹H NMR (400 MHz, DMSO-*d*₆) δ 0.96 - 1.06 (m, 2H), 1.32 (s, 3H), 2.07 (dd, *J =* 5.4, 13.3 Hz, 1H), 2.46-2.52 (m, 1H), 2.65 (s, 3H), 2.69 (s, 3H), 3.61 (dd, *J =* 2.6*,* 5.3 Hz, 1H), 4.49 (dd, *J =* 5.4, 9.0 Hz, 1H), 5.59 (d, *J =* 17.2 Hz, 1H), 5.94 (d, *J =* 17.3 Hz, 1H), 7.37 (t, *J =* 8.0 Hz, 1H), 7.52 (t, *J =* 7.2 Hz, 1H), 7.86 (t, *J =* 9.6 Hz, 2H), 8.07 (t, *J* = 7.6 Hz, 1H), 8.44 (s, 1H), 9.04 (s, 2H), 9.96 (s, 1H). ¹⁹F NMR (376 MHz, DMSO-*d*₆) δ -126.92 (q, *J* = 11.3 Hz), -59.97 (d, *J* = 11.3 Hz).

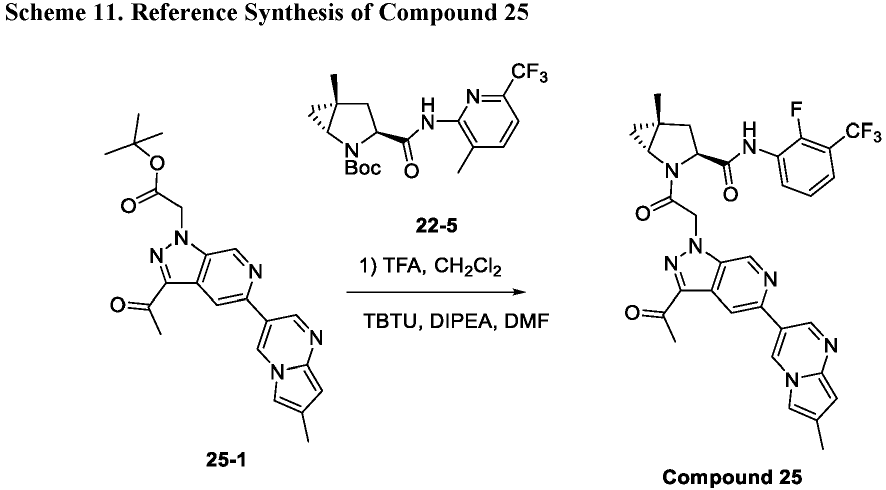

**(1*R*,3*S*,5*R*)-2-(2-(3-Acetyl-5-(2-methylpyrazolol[1,5-A]pyrimidin-6-yl)-1*H*-indazol-1-yl)acetyl)-*N*-(2-fluoro-3-(trifluoromethyl)phenyl)-5-methyl-2-azabicyclo[3.1.0]hexane-3-carboxamide (Compound 25):** *tert*-butyl 2-(3-acetyl-5-{2-methylpyrazolo[1,5-a]pyrimidin-6-yl}indazol-1-yl)acetate (**25-1**, 0.05 g, 0.123 mmol, 1 equiv.) was stirred with TFA (1 mL) and dichloromethane (0.5 mL) at room temperature for 3 hours. In another vial, tert-butyl (1R,3S,5R)-3-{[2-fluoro-3-(trifluoromethyl)phenyl]carbamoyl}-5-methyl-2-azabicyclo[3.1.0]hexane-2-carboxylate (**22-5**, 0.05 g, 0.123 mmol, 1 equiv.) was stirred with 0.5 mL of TFA and 0.5 mL of dichloromethane for 30 minutes at room temperature. The contents from both vials were combined and concentrated. The residue was taken up in DMF (1 mL) and the solution was cooled in an ice bath. DIPEA (0.08 g, 0.107 mL, 0.617 mmol, 5 equiv.) and TBTU (0.044 g, 0.136 mmol, 1.1 equiv.) were added. The resulting solution was stirred at room temperature for 30 minutes. DMF was removed under reduced pressure. The residue was dissolved in chloroform and washed with saturated aqueous sodium bicarbonate. The organic layer was dried and concentrated. The residue was purified by silica gel column chromatography (eluent: 0-2 % MeOH in dichloromethane) to afford a white solid ¹H NMR (400 MHz, DMSO-*d*₆) δ 0.97 - 1.06 (m, 2H), 1.32 (s, 3H), 2.07 (dd, *J =* 5.4, 13.3 Hz, 1H), 2.47 (s, 4H), 2.66 (s, 3H), 3.62 (dd, *J =* 2.6, 5.4 Hz, 1H), 4.49 (dd, *J =* 5.4, 9.1 Hz, 1H), 5.59 (d, *J =* 17.2 Hz, 1H), 5.94 (d, *J =* 17.3 Hz, 1H), 6.58 (s, 1H), 7.37 (t, *J =* 8.1 Hz, 1H), 7.52 (t, *J =* 7.3 Hz, 1H), 7.83 - 7.94 (m, 2H), 8.08 (t, *J =* 7.6 Hz, 1H), 8.47 (s, 1H), 8.85 (d, *J =* 2.3 Hz, 1H), 9.35 (d, *J =* 2.2 Hz, 1H), 9.96 (s, 1H). ¹⁹F NMR (376 MHz, DMSO-*d*₆) δ -126.91 (q, *J =* 11.3 Hz), -59.96 (d, *J =* 11.3 Hz).

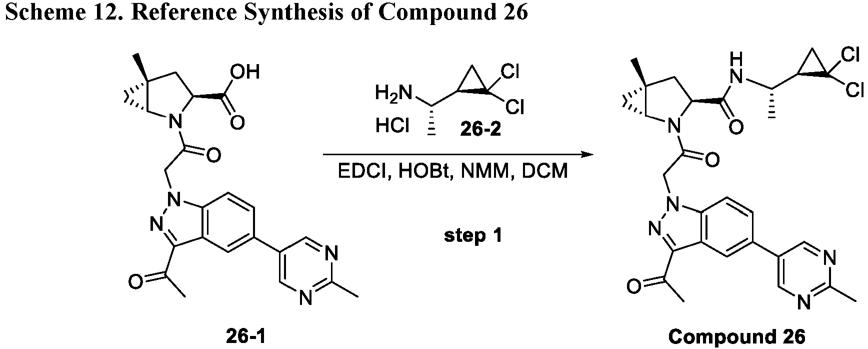

To a mixture of compound **26-1** (25 mg, 0.058 mmol) and compound **26-2** (13 mg, 0.087 mmol) in DCM (2 mL) was added NMM (29 mg, 0.29 mmol), EDCI (17 mg,0.087 mmol) and HOBt (12 mg, 0.087 mmol) at 0 °C and the mixture was stirred at room temperature overnight. The mixture was diluted with DCM and washed with water and brine, dried over anhydrous Na₂SO₄, filtered and concentrated to dryness. The residue was purified by preparative HPLC to afford **Compound 26** (5 mg, yield 15.3%) as a white solid. ¹H-NMR (400 MHz, CD₃OD) δ 9.00 (s, 2H), 8.54 (s, 1H), 7.84-7.73 (m, 2H), 5.75 (d, J= 17.2 Hz, 1H), 5.60 (d, J= 17.2 Hz, 1H), 4.24 (dd, J= 9.2, 5.2 Hz, 1H), 3.77-3.65 (m, 1H), 3.48 (dd, J= 5.6, 2.4 Hz, 1H), 2.75 (s, 3H), 2.69 (s, 3H), 2.54 -2.46 (m, 1H), 2.02 (dd, J= 13.2, 5.6 Hz, 1H), 1.74-1.64 (m, 1H), 1.54 (dd, J= 10.4, 7.2 Hz, 1H), 1.41-1.37 (m, 1H), 1.35 (s, 3H), 1.34-1.30 (m, 3H), 1.04 (t, J= 5.6 Hz, 1H), 0.93 (dd, J= 5.6, 2.4 Hz, 1H). LC/MS (ESI) m/z: 569 (M+H)⁺.

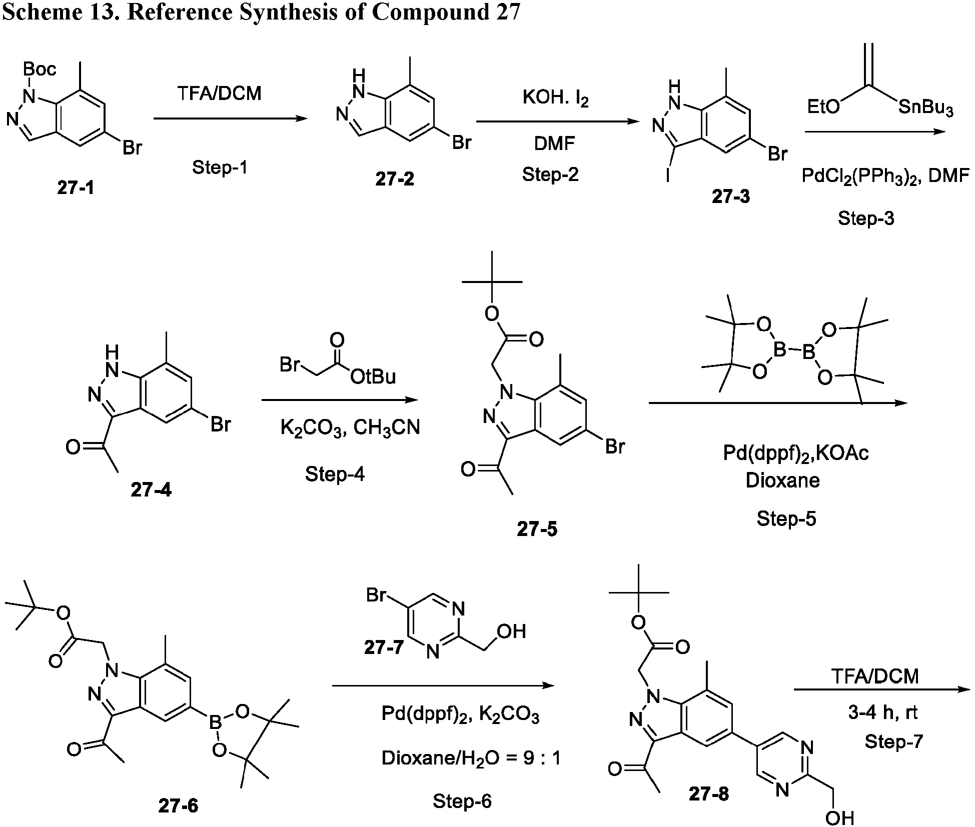

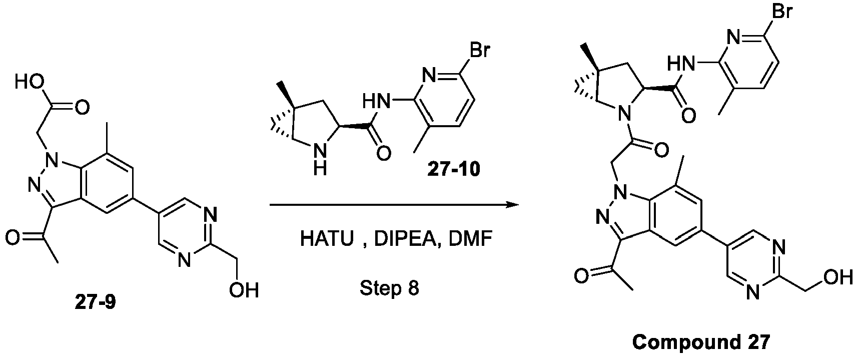

**Step 1: 5-Bromo-7-methyl-1H-indazole (27-2):** To a solution of compound **27-1** (1 equiv) in DCM (10 vol) at 0 °C under an atmosphere of argon was added TFA (5 vol). The reaction mixture was stirred at room temperature for 3 hours and then concentrated to dryness. The remaining material was used directly in the next synthetic step.

**Step 2: 5-Bromo-3-iodo-7-methyl-1*H*-indazole (27-3):** To a solution of 5-bromo-7-methyl-1H-indazole (0.678 g, 3.214 mmol, 1 equiv.) in dimethyl-formamide (5 mL, 0.643 M, 7.371 vols) were added potassium hydroxide (0.379 g, 6.749 mmol, 2.1 equiv.) and molecular iodine (1.06 g, 4.178 mmol, 1.3 equiv.) at 0 °C. The reaction mixture was stirred at room temperature for 48 hours. The mixture was then quenched with 5% aqueous Na₂S₂O₃ solution and extracted with EtOAc. The organic layer was separated, dried, and concentrated to afford the crude product, which was purified by column chromatography on silica gel (eluted with MeOH/DCM) to afford compound 27-3.

**Step 3: 1-(5-Bromo-7-methyl-1H-indazol-3-yl)ethan-1-one (27-4):** To a solution of 5-bromo-3-iodo-7-methyl-1H-indazole (**27-3**, 1 g, 2.968 mmol, 1 equiv.) in dry dimethyl-formamide (10 mL, 0.297 M, 10 vols) were added tributyl(1-ethoxyethenyl)stannane (1.179 g, 1.103 mL, 3.265 mmol, 1.1 equiv.) and dichlorobis(triphenylphosphine)palladium (0.104 g, 0.148 mmol, 0.05 equiv.). The resulting mixture was stirred at 80 °C under argon overnight and then concentrated. The remaining residue was diluted with DCM and washed with 2N aqueous HCl and organic layer was separated, dried over anhydrous Na₂SO₄, and concentrated to dryness. The obtained crude product was purified by column chromatography on silica gel (eluted with DCM/ MeOH) to afford compound 1-(5-bromo-7-methyl-1H-indazol-3-yl)ethanone (0.2 g, 0.79 mmol, yield 26.627%) .

**Step 4: *tert*-Butyl 2-(3-acetyl-5-bromo-7-methyl-1*H*-indazol-1-yl)acetate (27-5):** To a solution of 1-(5-bromo-7-methyl-1*H*-indazol-3-yl)ethanone (0.751 g, 2.968 mmol, 1 equiv.) in dimethyl-formamide (10 mL, 0.297 M, 13.312 vols) were added potassium carbonate (0.41 g, 2.968 mmol, 1 equiv.) and tert-butyl 2-bromoacetate (0.637 g, 0.482 mL, 3.265 mmol, 1.1 equiv.). The mixture was stirred at room temperature overnight under an atmosphere of argon and mixture was filtered through celite and washed with CH₃CN. The filtrate was concentrated under reduced pressure and the remaining residue was purified by column chromatography on silica gel (eluted with DCM/EtOAc) to afford *tert*-butyl 2-(3-acetyl-5-bromo-7-methylindazol-1-yl)acetate (0.4 g, 1.089 mmol, yield 36.698%)

**Step 5: *tert-Butyl* 2-(3-acetyl-7-methyl-5-(4,4,5,5-tetramethyl-1,3,2-dioxaborolan-2-yl)-1H-indazol-1-yl)acetate (27-6):** To a solution of *tert*-butyl 2-(3-acetyl-5-bromo-7-methylindazol-1-yl)acetate (0.063 g, 0.172 mmol, 1 equiv.) in dioxane (1 mL, 0.172 M, 15.873 vols) under an atmosphere of argon were added bis(pinacolato)diboron (0.044 g, 0.172 mmol, 1 equiv.), KOAc (0.034 g, 0.343 mmol, 2 equiv.) and [1,1'-bis(diphenylphosphino)ferrocene]dichloropalladium(II),complexwithdichloromethane (0.007 g, 0.009 mmol, 0.05 equiv.). The resultimg reaction mixture was stirred at 90 °C for 4 hours and the resulting reaction mixture was directly used in the next synthetic step without further purification.

**Step 6: *tert*-Butyl 2-(3-acetyl-5-(2-(hydroxymethyl)pyrimidin-5-yl)-7-methyl-1*H-*indazol-1-yl)acetate (27-8):** A To a solution of *tert*-butyl 2-[3-acetyl-7-methyl-5-(4,4,5,5-tetramethyl-1,3,2-dioxaborolan-2-yl)indazol-1-yl]acetate (0.071 g, 0.172 mmol, 1 equiv.) in dioxane (1 mL) and water (0.1 mL) were added (5-bromopyrimidin-2-yl)methanol (27-7, 0.033 g, 0.172 mmol, 1 equiv.), potassium carbonate (0.071 g, 0.516 mmol, 3 equiv.) and [1,1'-bis(diphenylphosphino)ferrocene]dichloropalladium(II),complexwithdichloromethane (0.14 g, 0.172 mmol, 1 equiv.). The reaction mixture was stirred at 90 °C for 5 hours and then concentrated to dryness. The remaining residue was purified by column chromatography on silica gel (eluted with DCM/ MeOH) to afford compound tert-butyl 2-{3-acetyl-5-[2-(hydroxymethyl)pyrimidin-5-yl]-7-methylindazol-1-yl}acetate (0.05 g, 0.126 mmol, yield 73.326%).

**Step 7: 2-(3-Acetyl-5-(2-(hydroxymethyl)pyrimidin-5-yl)-7-methyl-1*H*-indazol-1-yl)acetic acid (27-9):** To a solution of compound *tert*-butyl 2-{3-acetyl-5-[2-(hydroxymethyl)pyrimidin-5-yl]-7-methylindazol-1-yl}acetate (0.05 g, 0.126 mmol, 1 equiv.) in dichloromethane (2 mL) at 0 °C under an atmosphere of argon was added trifluoroacetic acid (1 mL). The reaction mixture was stirred at room temperature for 3 hours and concentrated to dryness. The remaining material was used directly in the next synthetic step.

**Step 8: (1*R*,3*S*,5*R*)-2-(2-(3-Acetyl-5-(2-(hydroxymethyl)pyrimidin-5-yl)-7-methyl-1*H-*indazol-1-yl)acetyl)-*N*-(6-bromo-3-methylpyridin-2-yl)-5-methyl-2-azabicyclo[3.1.0]hexane-3-carboxamide (Compound 27):** To a solution of {3-acetyl-5-[2-(hydroxymethyl)pyrimidin-5-yl]-7-methylindazol-1-yl}acetic acid (0.043 g, 0.126 mmol, 1 equiv.) in dimethyl-formamide (1 mL, 0.126 M, 23.319 vols) at 0 °C under an atmosphere of argon were added (1*R*,3*S*,5*R*)-*N-*(6-bromo-3-methylpyridin-2-yl)-5-methyl-2-azabicyclo[3.1.0]hexane-3-carboxamide (0.039 g, 0.126 mmol, 1 equiv.), HATU (0.101 g, 0.265 mmol, 2.1 equiv.) and DIPEA (0.081 g, 0.11 mL, 0.63 mmol, 5 equiv.). The reaction mixture was stirred at room temperature for 1 hour and then quenched with water (10 vol). The resulting mixture was extracted with DCM. The organic layer was washed with brine, dried over anhydrous Na₂SO₄, filtered, and concentrated to dryness. The remaining residue was purified by column chromatography on silica gel (eluted with DCM/MeOH) to afford **Compound 27.** 1H NMR (400 MHz, DMSO-d6) δ 0.95 (dd, J = 2.4, 5.4 Hz, 1H), 1.03 (t, J = 5.4 Hz, 1H), 1.33 (s, 3H), 2.06 (m, 4H), 2.54 - 2.61 (m, 1H), 2.65 (s, 3H), 2.70 (s, 3H), 3.60 (dd, J = 2.4, 5.5 Hz, 1H), 4.43 (dd, J = 5.4, 9.2 Hz, 1H), 4.68 (d, J = 6.3 Hz, 2H), 5.34 (t, J = 6.3 Hz, 1H), 5.70 (d, J = 17.8 Hz, 1H), 6.05 (d, J = 17.8 Hz, 1H), 7.44 (d, J = 7.9 Hz, 1H), 7.59 - 7.72 (m, 2H), 8.36 (d, J = 1.6 Hz, 1H), 9.12 (s, 2H), 10.30 (s, 1H).

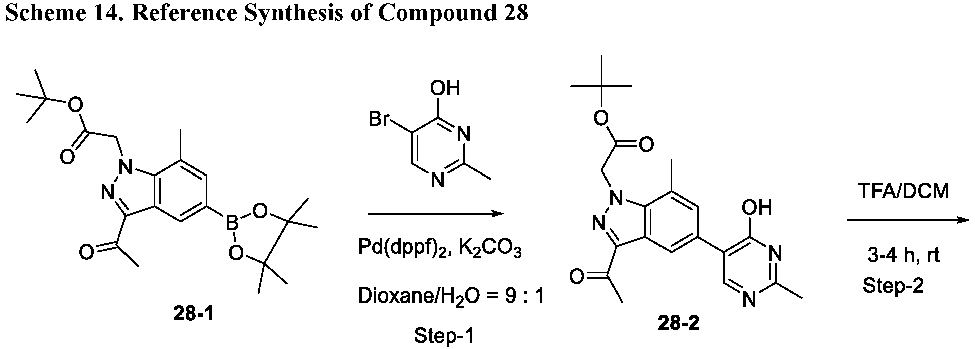

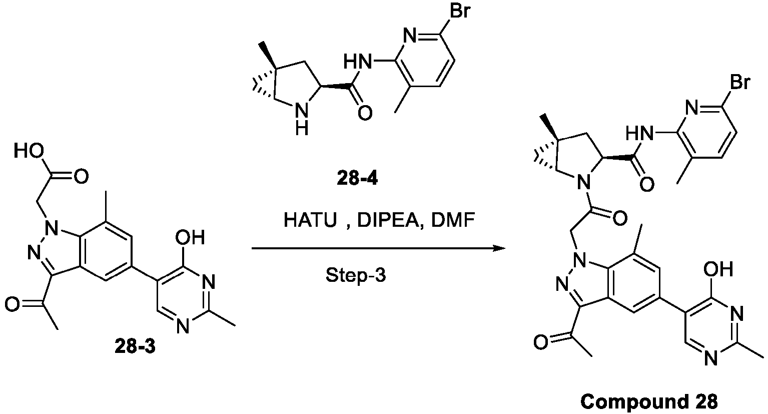

**Step 1: *tert*-Butyl 2-(3-acetyl-5-(4-hydroxy-2-methylpyrimidin-5-yl)-7-methyl-1*H-*indazol-1-yl)acetate (28-2):** To a solution of tert-butyl 2-[3-acetyl-7-methyl-5-(4,4,5,5-tetramethyl-1,3,2-dioxaborolan-2-yl)indazol-1-yl]acetate (0.071 g, 0.172 mmol, 1 equiv.) in dioxane (1 mL) and water (0.1 mL) were added compound 5-bromo-2-methylpyrimidin-4-ol (0.033 g, 0.172 mmol, 1 equiv.), potassium carbonate (0.071 g, 0.516 mmol, 3 equiv.) and [1,1'-Bis(diphenylphosphino)ferrocene]dichloropalladium(II),complexwithdichloromethane (0.14 g, 0.172 mmol, 1 equiv.). The reaction mixture was stirred at 90°C for 5 hours and then concentrated to dryness. The remaining residue was purified by column chromatography on silica gel (eluted with DCM/ MeOH) to afford tert-butyl 2-[3-acetyl-5-(4-hydroxy-2-methylpyrimidin-5-yl)-7-methylindazol-1-yl]acetate (0.014 g, 0.035 mmol, yield 20.531%).

**Step 2: 2-(3-Acetyl-5-(4-hydroxy-2-methylpyrimidin-5-yl)-7-methyl-1*H*-indazol-1-yl)acetic acid (28-3):** To a solution of compound *tert*-butyl 2-[3-acetyl-5-(4-hydroxy-2-methylpyrimidin-5-yl)-7-methylindazol-1-yl]acetate (**28-2,** 0.014 g, 0.035 mmol, 1 equiv.) in dichloromethane (1 mL) at 0 °C under an atmosphere of argon was added trifluoroacetic acid (0.5 mL). The reaction mixture was stirred at room temperature for 3 hours and then concentrated to dryness. The remaining material was used directly in the next synthetic step.

**Step 3: (1*R*,3*S*,5*R*)-2-(2-(3-acetyl-5-(4-hydroxy-2-methylpyrimidin-5-yl)-7-methyl-1*H*-indazol-1-yl)acetyl)-*N*-(6-bromo-3-methylpyridin-2-yl)-5-methyl-2-azabicyclo[3.1.0]hexane-3-carboxamide (Compound 28):** To a solution of compound [3-acetyl-5-(4-hydroxy-2-methylpyrimidin-5-yl)-7-methylindazol-1-yl]acetic acid (**28-3**, 0.012 g, 0.035 mmol, 1 equiv.) in dimethyl-formamide (1 mL, 0.035 M, 83.95 vols) at 0 °C under an atmosphere of argon were added (1*R*,3*S*,5*R*)-*N*-(6-bromo-3-methylpyridin-2-yl)-5-methyl-2-azabicyclo[3.1.0]hexane-3-carboxamide (**28-4**, 0.011 g, 0.035 mmol, 1 equiv.), HATU (0.028 g, 0.074 mmol, 2.1 equiv.) and DIPEA (0.023 g, 0.03 mL, 0.175 mmol, 5 equiv.). The reaction mixture was stirred at room temperature for 1 hour and quenched with water (10 vol). The resulting mixture was extracted with DCM. The organic layer was washed with brine, dried over anhydrous Na₂SO₄, filtered, and concentrated to dryness. The remaining residue was purified by column chromatography on silica gel (eluted with DCM/MeOH) to afford **Compound 28.** ¹H NMR (400 MHz, DMSO-d6) δ 0.93 (dd, J = 2.4, 5.2 Hz, 1H), 1.02 (t, J = 5.4 Hz, 1H), 1.32 (s, 3H), 2.05 (m, 4H), 2.34 (s, 3H), 2.53 - 2.59 (m, 1H), 2.62 (s, 6H), 3.58 (dd, J = 2.4, 5.5 Hz, 1H), 4.42 (dd, J = 5.4, 9.2 Hz, 1H), 5.66 (d, J = 17.7 Hz, 1H), 5.99 (d, J = 17.8 Hz, 1H), 7.36 - 7.50 (m, 2H), 7.62 (d, J = 8.0 Hz, 1H), 8.07 (s, 1H), 8.35 - 8.54 (m, 1H), 10.29 (s, 1H), 12.64 (s, 1H).

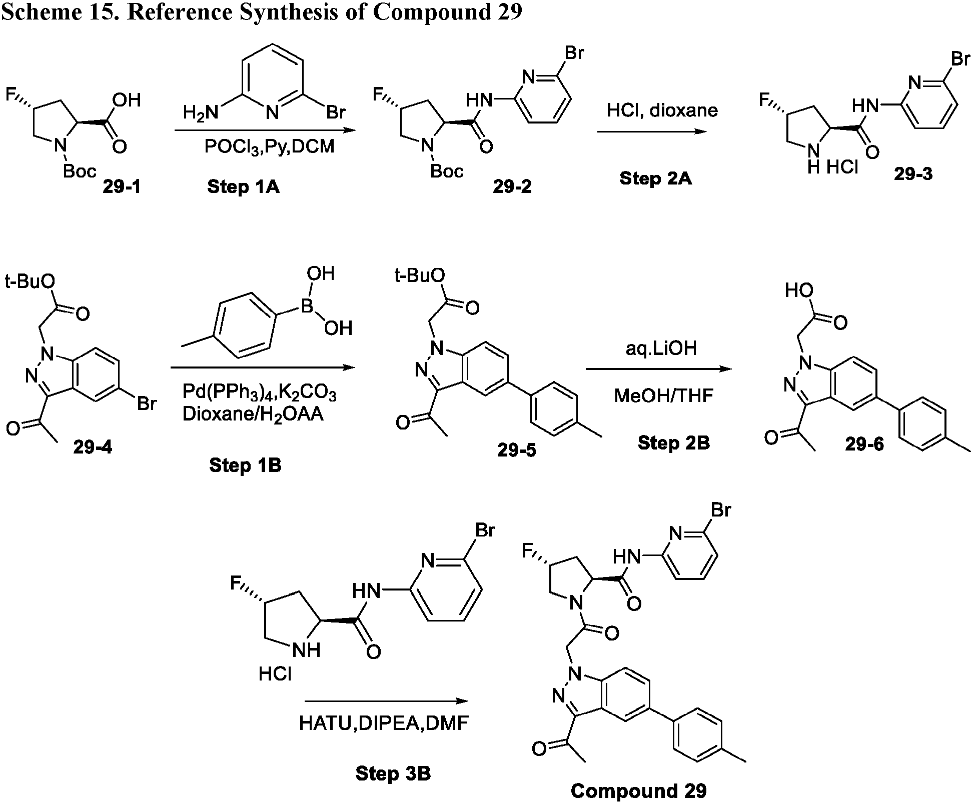

**Step 1A: (2*S*,4*R*)-*tert*-Butyl 2-((6-bromopyridin-2-yl)carbamoyl)-4-fluoropyrrolidine-1-carboxylate (29-2):** To a mixture of compound **29-1** (900 mg, 3.86 mmol) and 6-bromopyridin-2-amine (668 mg, 3.86 mmol) in DCM (10 mL) was added pyridine (1.526 g, 19.31 mmol) followed by the drop-wise addition of POCl₃ (649 mg, 4.25 mmol) at 0 °C and the mixture was stirred at room temperature under N₂ atmosphere for 1 hour. The mixture was poured into ice-water and extracted with DCM twice. The combined organic layers were washed with brine, dried over anhydrous Na₂SO₄, filtered and concentrated to dryness. The residue was purified by flash chromatography (eluted with PE: EtOAc= 100: 1 to 10: 1) to afford compound **29-2** (1.23 g, yield 82.05%) as a white solid. LC/MS (ESI) m/z: 388/390 (M+H)⁺.

**Step 2A: (2*S*,4*R*)-*N*-(6-Bromopyridin-2-yl)-4-fluoropyrrolidine-2-carboxamide hydrochloride (29-3):** A solution of compound **29-2** (600 mg, 1.55 mmol) in HCl/1,4-dioxane solution (6 mL, 4M) was stirred at 0 °C at room temperature for 1 hour. The reaction mixture was concentrated to dryness, washed with ether and dried under vacuum to afford compound **29-3** (444 mg, yield 99.8%) as a white solid. LC/MS (ESI) m/z: 288/290 (M+H)⁺.

**Step 1B: *tert*-Butyl 2-(3-acetyl-5-(*p*-tolyl)-1*H*-indazol-1-yl)acetate (29-5):** To a mixture of compound **29-4** (300 mg, 0.85 mmol) and p-tolylboronic acid (127 mg, 0.94 mmol) in dioxane (7 mL) and water (1 mL) was added potassium carbonate (294 mg, 2.13 mmol) and Pd(PPh₃)₄ (98 mg, 0.085 mmol) at 0 °C and the mixture was stirred at 95 °C under N₂ atmosphere for 2 hours. The mixture was poured into ice-water and extracted with EtOAC twice. The combined organic layers were washed with brine, dried with anhydrous Na₂SO₄, filtered and concentrated to dryness. The residue was purified by flash chromatography (eluted with PE: EtOAc= 50:1 to 1: 1) to afford compound **29-5** (255 mg, yield 82.5%) as a white solid. LC/MS (ESI) m/z: 365 (M+H)⁺.

**Step 2B: 2-(3-Acetyl-5-(*p*-tolyl)-1*H*-indazol-1-yl)acetic acid (29-6):** To a solution of compound **29-5** (255 mg, 0.70 mmol) in methanol (4 mL) and THF (2 mL) was added a solution of LiOH (88 mg, 2.10 mmol) in water (2 mL) at 0 °C and the mixture was stirred at room temperature for 2 hours. The mixture was diluted with water and washed with ether. The aqueous layer was acidified with 1N aqueous HCl solution to a pH of approximately 3 and extracted with DCM twice. The combined organic layers were washed with brine, dried with anhydrous Na₂SO₄, filtered and concentrated to dryness to afford compound **29-6** (192 mg, yield 89.3%) as a white solid. LC/MS (ESI) m/z: 309 (M+H)⁺.

**Step 3B: (2*S*,4*R*)-1-(2-(3-Acetyl-5-(*p*-tolyl)-1*H*-indazol-1-yl)acetyl)-*N*-(6-bromopyridin-2-yl)-4-fluoropyrrolidine-2-carboxamide (Compound 29):** To a mixture of compound **29-6** (40 mg, 0.13 mmol) and (2S,4R)-N-(6-bromopyridin-2-yl)-4-fluoropyrrolidine-2-carboxamide hydrochloride (42 mg, 0.13 mmol) in DMF (3 mL) was added DIPEA (84 mg, 0.65 mmol) followed by HATU (74 mg, 0.19 mmol) at 0 °C and the mixture was stirred at room temperature for 1 hour. The mixture was diluted with EtOAc and washed with saturated aqueous NH₄Cl solution and brine, dried over anhydrous Na₂SO₄, filtered and concentrated to dryness. The residue was purified by preparative HPLC to afford **Compound 29** (41 mg, yield 54.7%) as a white solid. ¹H-NMR (400 MHz, DMSO-d₆) δ 11.01 (s, 1H), 8.33 (t, J= 1.2 Hz, 1H), 8.03 (d, J= 8.0 Hz, 1H), 7.76 (d, J= 1.6 Hz, 2H), 7.71 (t, J= 8.0 Hz, 1H), 7.60-7.57 (m, 2H), 7.33-7.29 (m, 3H), 5.82 (d, J= 17.6 Hz, 1H), 5.63-5.59 (m, 1H), 4.66 (t, J= 8.4 Hz, 1H), 4.23 (dd, J= 22.0, 22.0 Hz, 1H), 4.28-3.95 (m,1H), 2.63 (s, 3H), 2.62-2.51 (m, 2H), 2.36 (s, 3H), 2.24-2.17 (m, 0.5H), 2.14-2.05 (m, 0.5H). LC/MS (ESI) m/z: 578/580 (M+H)⁺.

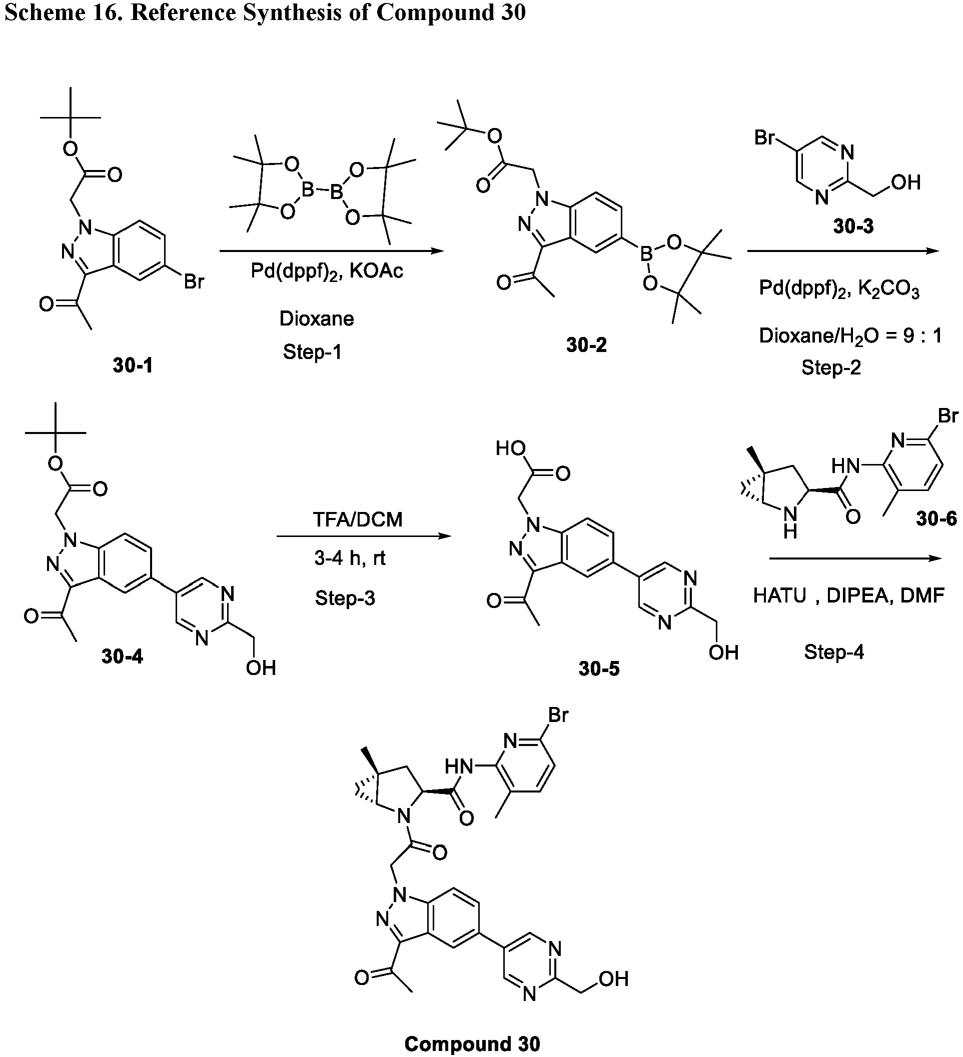

**Step 1: *tert*-Butyl 2-(3-acetyl-5-(4,4,5,5-tetramethyl-1,3,2-dioxaborolan-2-yl)-1*H-*indazol-1-yl)acetate (30-2):** To a solution of compound *tert*-butyl 2-(3-acetyl-5-bromoindazol-1-yl)acetate (2 g, 5.662 mmol, 1 equiv.) in dioxane (20 mL, 0.283 M, 10 vols) under an atmosphere of argon were added bis(pinacolato)diboron (1.438 g, 5.662 mmol, 1 equiv.), potassium acetate (1.111 g, 11.325 mmol, 2 equiv.) and [1,1'-bis(diphenylphosphino)ferrocene]dichloropalladium(II), complexwithdichloromethane (0.231 g, 0.283 mmol, 0.05 equiv.). The resultimg reaction mixture was stirred at 90 °C for 4 hours and the resulting reaction mixture was used in the next synthetic step without further purification.

**Step 2: *tert*-Butyl 2-(3-acetyl-5-(2-(hydroxymethyl)pyrimidin-5-yl)-1*H*-indazol-1-yl)acetate (30-4):** To a solution of *tert*-butyl 2-[3-acetyl-5-(4,4,5,5-tetramethyl-1,3,2-dioxaborolan-2-yl)indazol-1-yl]acetate (**30-2**, 2.266 g, 5.66 mmol, 1 equiv.) in dioxane (20 mL) and water (1 mL) were added (5-bromopyrimidin-2-yl)methanol (**30-3**, 1.07 g, 5.66 mmol, 1 equiv.), and [1,1'-bis(diphenylphosphino)ferrocene]dichloropalladium(II) complex with dichloromethane (0.231 g, 0.283 mmol, 0.05 equiv.). The reaction mixture was stirred at 90 °C for 5 hours and then concentrated to dryness. The remaining residue was purified by column chromatography on silica gel (eluted with DCM/ MeOH) to afford compound tert-butyl 2-{3-acetyl-5-[2-(hydroxymethyl)pyrimidin-5-yl]indazol-1-yl}acetate (1.5 g, 3.922 mmol, yield 69.3%).

**Step 3: 2-(3-Acetyl-5-(2-(hydroxymethyl)pyrimidin-5-yl)-1*H*-indazol-1-yl)acetic acid (30-5):** To a solution of compound *tert*-butyl 2-{3-acetyl-5-[2-(hydroxymethyl)pyrimidin-5-yl]indazol-1-yl}acetate (0.05 g, 0.131 mmol, 1 equiv.) in dichloromethane (2 mL) at 0 °C under an atmosphere of argon was added trifluoroacetic acid (1 mL). The reaction mixture was stirred at room temperature for 3 hours and concentrated to dryness. The remaining material was used directly in the next synthetic step.

**Step 4: (1*R*,3*S*,5*R*)-2-(2-(3-acetyl-5-(2-(hydroxymethyl)pyrimidin-5-yl)-1*H*-indazol-1-yl)acetyl)-*N*-(6-bromo-3-methylpyridin-2-yl)-5-methyl-2-azabicyclo[3.1.0]hexane-3-carboxamide (Compound 30):** To a solution of compound {3-acetyl-5-[2-(hydroxymethyl)pyrimidin-5-yl]indazol-1-yl}acetic acid (0.1 g, 0.306 mmol, 1 equiv.) in dimethyl-formamide (1 mL, 0.306 M, 10 vols) at 0 °C under an atmosphere of argon were added (1*R*,3*S*,5*R*)-*N*-(6-bromo-3-methylpyridin-2-yl)-5-methyl-2-azabicyclo[3.1.0]hexane-3-carboxamide (0.095 g, 0.306 mmol, 1 equiv.), HATU (0.245 g, 0.644 mmol, 2.1 equiv.) and DIPEA (0.198 g, 0.267 mL, 1.532 mmol, 5 equiv.). The reaction mixture was stirred at room temperature for 1 hour and quenched with water (10 vol). The resulting mixture was extracted with DCM. The organic layer was washed with brine, dried over anhydrous Na₂SO₄, filtered, and concentrated to dryness. The remaining residue was purified by column chromatography on silica gel (eluted with DCM/MeOH) to afford **Compound 30.** ¹H NMR (400 MHz, DMSO-d6) δ 1.03 (m, 2H), 1.13- 1.32 (m, 1H), 1.33 (s, 3H), 2.01 - 2.11 (m, 4H), 2.54 - 2.59 (m, 1H), 2.67 (s, 3H), 3.60 (dd, J = 2.5, 5.5 Hz, 1H), 4.42 (dd, J = 5.1, 9.2 Hz, 1H), 4.69 (s, 2H), 5.59 (d, J = 17.2 Hz, 1H), 5.93 (d, J = 17.3 Hz, 1H), 7.45 (d, J = 7.9 Hz, 1H), 7.63 (d, J = 7.9 Hz, 1H), 7.79 - 7.93 (m, 2H), 8.48 (s, 1H), 9.15 (s, 2H), 10.27 (s, 1H).

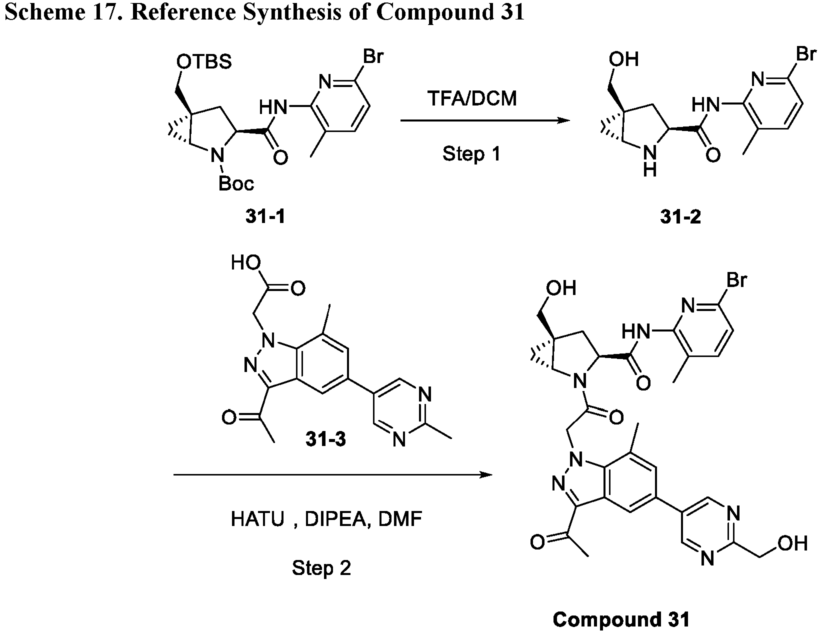

**Step 1: (1*R*,3*S*,5*S*)-*N*-(6-Bromo-3-methylpyridin-2-yl)-5-(hydroxymethyl)-2-azabicyclo[3.1.0]hexane-3-carboxamide (33-2):** To a solution of compound tert-butyl (1*R*,3*S*,5*S*)-3-[(6-bromo-3-methylpyridin-2-yl)carbamoyl]-5-{[*tert*butyldimethylsilyl)oxy]methyl}-2-azabicyclo[3.1.0]hexane-2-carboxylate (0.076 g, 0.141 mmol, 1 equiv.) in dichloromethane (1 mL) at 0 °C under an atmosphere of argon was added trifluoroacetic acid (1 mL). The reaction mixture was stirred at room temperature for 3 hours and concentrated to dryness. The remaining material was used directly in the next synthetic step.

**Step 2: (1*R*,3*S*,5*R*)-2-(2-(3-Acetyl-7-methyl-5-(2-methylpyrimidin-5-yl)-1*H-*pyrazolo[3,4-c]pyridin-1-yl)acetyl)-*N*-(6-bromo-5-fluoro-3-methylpyridin-2-yl)-5-methyl-2-azabicyclo[3.1.0]hexane-3-carboxamide (Compound 33):** To a solution of compound {3-acetyl-5-[2-(hydroxymethyl)pyrimidin-5-yl]indazol-1-yl}acetic acid (**33-3**, 0.046 g, 0.141 mmol, 1 equiv.) in dimethyl-formamide (1 mL, 0.141 M, 21.734 vols) at 0 °C under an atmosphere of argon were added (1*R*,3*S*,5*S*)-*N*-(6-bromo-3-methylpyridin-2-yl)-5-(hydroxymethyl)-2-azabicyclo[3.1.0]hexane-3-carboxamide (0.046 g, 0.141 mmol, 1 equiv.), HATU (0.113 g, 0.296 mmol, 2.1 equiv.), and DIPEA (0.091 g, 0.123 mL, 0.705 mmol, 5 equiv.). The reaction mixture was stirred at room temperature for 1 hour and quenched with water (10 vol). The resulting mixture was extracted with DCM. The organic layer was washed with brine, dried over anhydrous Na₂SO₄, filtered, and concentrated to dryness. The remaining residue was purified by column chromatography on silica gel (eluted with DCM/MeOH) to afford **Compound 33.** ¹H NMR (400 MHz, DMSO-d6) δ 0.98 (m, 1H), 1.11 - 1.27 (m, 1H), 2.06 (s, 3H), 2.25 (dd, J = 4.9, 13.4 Hz, 1H), 2.45 - 2.49 (m, 1H), 2.66 (s, 3H), 2.71 (s, 3H), 3.55 (d, J = 5.6 Hz, 2H), 3.63 - 3.70 (m, 1H), 4.49 (dd, J = 5.1, 9.3 Hz, 1H), 4.68 (d, J = 6.3 Hz, 2H), 4.89 (t, J = 5.5 Hz, 1H), 5.34 (t, J = 6.3 Hz, 1H), 5.72 (d, J = 17.8 Hz, 1H), 6.05 (d, J = 17.8 Hz, 1H), 7.44 (d, J = 7.9 Hz, 1H), 7.61 - 7.69 (m, 2H), 8.36 (d, J = 1.6 Hz, 1H), 9.12 (s, 2H), 10.28 (s, 1H).

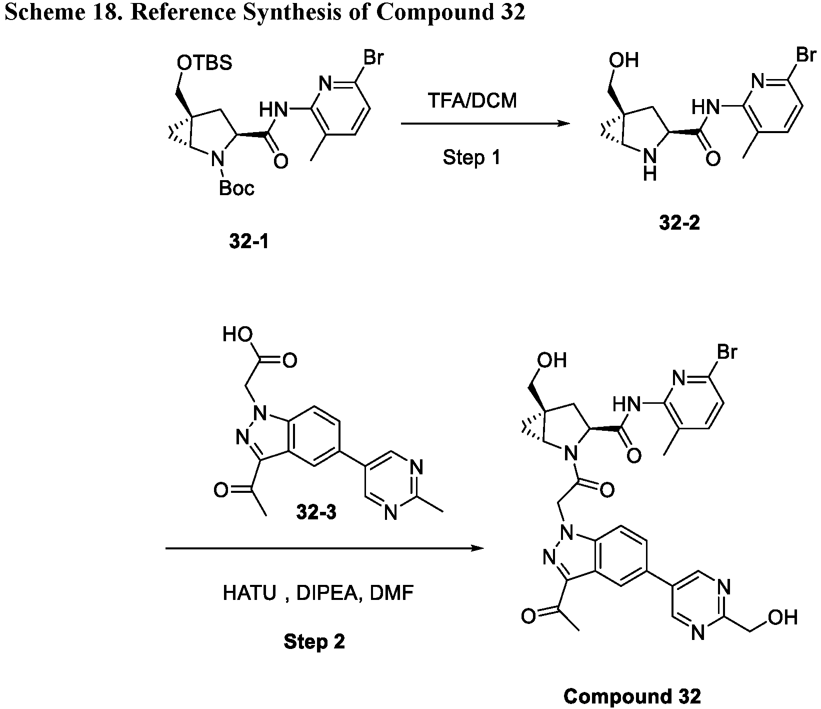

**Step 1: (1*R*,3*S*,5*S*)-*N*-(6-Bromo-3-methylpyridin-2-yl)-5-(hydroxymethyl)-2-azabicyclo[3.1.0]hexane-3-carboxamide (32-2):** To a solution of *tert*-butyl (1*R*,3*S*,5*S*)-3-[(6-bromo-3-methylpyridin-2-yl)carbamoyl]-5-{[(tert-butyldimethylsilyl)oxy]methyl}-2-azabicyclo[3.1.0]hexane-2-carboxylate (0.076 g, 0.141 mmol, 1 equiv.) in dichloromethane (1 mL) at 0 °C under an atmosphere of argon was added trifluoroacetic acid (1 mL). The reaction mixture was stirred at room temperature for 3 hours and concentrated to dryness. The remaining material was used directly in the next synthetic step.

**Step 2: (1*R*,3*S*,5*S*)-2-(2-(3-Acetyl-5-(2-(hydroxymethyl)pyrimidin-5-yl)-1*H*-indazol-1-yl)acetyl)-*N*-(6-bromo-3-methylpyridin-2-yl)-5-(hydroxymethyl)-2-azabicyclo[3.1.0]hexane-3-carboxamide (Compound 32):** To a solution of {3-acetyl-5-[2-(hydroxymethyl)pyrimidin-5-yl]indazol-1-yl}acetic acid (0.046 g, 0.141 mmol, 1 equiv.) in dimethyl-formamide (1 mL, 0.141 M, 21.734 vols) at 0 °C under an atmosphere of argon were added (1*R*,3*S*,5*S*)-*N*-(6-bromo-3-methylpyridin-2-yl)-5-(hydroxymethyl)-2-azabicyclo[3.1.0]hexane-3-carboxamide **(32-3,** 0.046 g, 0.141 mmol, 1 equiv.), HATU (0.113 g, 0.296 mmol, 2.1 equiv.) and DIPEA (0.091 g, 0.123 mL, 0.705 mmol, 5 equiv.). The reaction mixture was stirred at room temperature for 1 hour and quenched with water (10 vol). The resulting mixture was extracted with DCM. The organic layer was washed with brine, dried over anhydrous Na₂SO₄, filtered, and concentrated to dryness. The remaining residue was purified by column chromatography on silica gel (eluted with DCM/MeOH) to afford **Compound 32** (0.02 g, 0.032 mmol, yield 22.356%). ¹H NMR (400 MHz, DMSO-d6) δ 1.04 - 1.11 (m, 1H), 1.19 (t, J = 5.5 Hz, 1H), 2.06 (s, 3H), 2.23 - 2.30 (m, 1H), 2.44 - 2.49 (m, 1H), 2.67 (s, 3H), 3.54 (d, J = 5.5 Hz, 2H), 3.67 (d, J = 5.0 Hz, 1H), 4.48 (dd, J = 4.8, 9.4 Hz, 1H), 4.69 (d, J = 6.3 Hz, 2H), 4.89 (d, J = 5.6 Hz, 1H), 5.35 (t, J = 6.3 Hz, 1H), 5.61 (d, J = 17.3 Hz, 1H), 5.92 (d, J = 17.3 Hz, 1H), 7.45 (d, J = 8.3 Hz, 1H), 7.63 (d, J = 8.0 Hz, 1H), 7.84 - 7.94 (m, 2H), 8.48 (s, 1H), 9.14 (s, 2H), 10.23 (s, 1H).

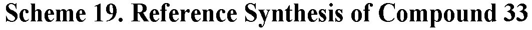

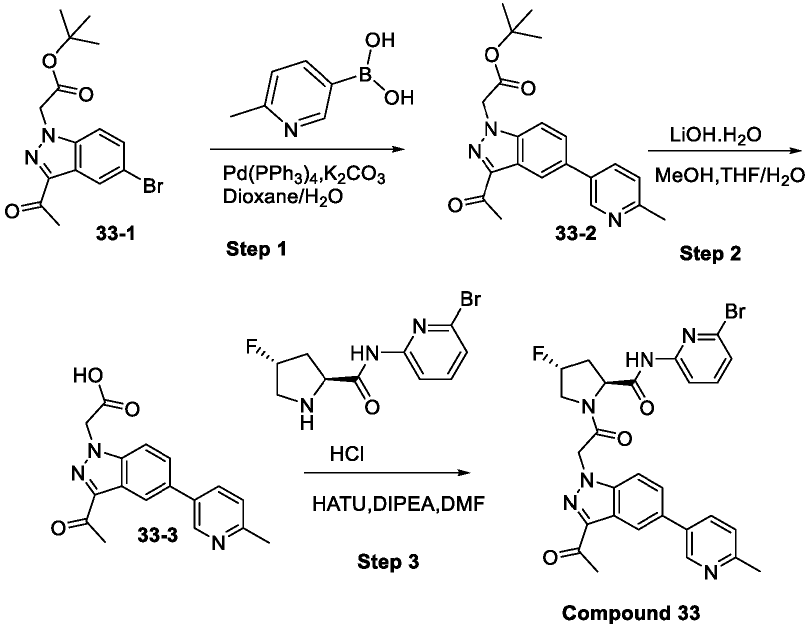

**Step 1: *tert*-Butyl 2-(3-acetyl-5-(6-methylpyridin-3-yl)-1*H*-indazol-1-yl)acetate (33-2):** To a mixture of compound **33-1** (300 mg, 0.85 mmol) and (6-methylpyridin-3-yl)boronic acid (129 mg, 0.94 mmol) in 1,4-dioxane (7 mL) and water (1 mL) was added potassium carbonate (294 mg, 2.13 mmol) and Pd(PPh₃)₄ (98 mg, 0.085 mmol) at 0 °C and the mixture was stirred at 90 °C under N₂ atmosphere for 2 hours. The mixture was diluted with EtOAC, washed with water and brine, dried over anhydrous Na₂SO₄, filtered and concentrated to dryness. The residue was purified by flash chromatography (eluted with PE: EtOAc= 50: 1 to 1: 1) to afford compound **33-2** (159 mg, yield 51.3%) as a yellow solid. LC/MS (ESI) m/z: 366 (M+H)⁺.

**Step 2: 2-(3-Acetyl-5-(6-methylpyridin-3-yl)-1*H*-indazol-1-yl)acetic acid (33-3):** To a solution of compound **33-2** (159 mg, 0.44 mmol) in methanol (4 mL) and THF (2 mL) was added a solution of LiOH (92 mg, 2.18 mmol) in water (2 mL) at 0 °C and the mixture was stirred at room temperature for 2 hours. The mixture was diluted with water and washed with ether. The aqueous layer was acidified with 1N aqueous HCl solution to a pH of approximately 3 and extracted with DCM twice. The combined organic layers were washed with brine, dried with anhydrous Na₂SO₄, filtered and concentrated to dryness to afford compound **33-3** (90 mg, yield 67.7%) as a white solid. LC/MS (ESI) m/z: 310 (M+H)⁺.

**Step 3: (2*S*,4*R*)-1-(2-(3-Acetyl-5-(*p*-tolyl)-1*H*-indazol-1-yl)acetyl)-N-(6-bromopyridin-2-yl)-4-fluoropyrrolidine-2-carboxamide (Compound 33):** To a mixture of compound **33-3** (30 mg, 0.097 mmol) and (2S,4R)-N-(6-bromopyridin-2-yl)-4-fluoropyrrolidine-2-carboxamide hydrochloride (31 mg, 0.097 mmol) in DMF (3 mL) was added DIPEA (63 mg, 0.49 mmol) followed by HATU (55 mg, 0.15 mmol) at 0 °C and the mixture was stirred at room temperature for 1 hour. The mixture was diluted with EtOAc, washed with saturated aqueous NH₄Cl solution and brine, dried over anhydrous Na₂SO₄, filtered and concentrated to dryness. The residue was purified by preparative HPLC to afford **Compound 33** (25 mg, yield 43.8%) as a white solid. ¹H-NMR (400 MHz, DMSO-d₆) δ 11.01 (s, 1H), 8.76 (d, J= 2.4 Hz, 1H), 8.36 (d, J= 1.4 Hz, 1H), 8.05-7.96 (m, 2H), 7.81 (d, J= 1.6 Hz, 2H), 7.71 (t, J= 7.8 Hz, 1H), 7.34 (dd, J= 18.4, 18.4 Hz, 2H), 5.83 (d, J= 17.2 Hz, 1H), 5.63 (d, J= 17.2 Hz, 1H), 5.53-5.07 (m, 1H), 4.68-4.64 (m, 1H), 4.09-4.23 (m, 1H), 4.10-3.96 (m, 1H), 2.64 (s, 3H), 2.61-2.54 (m, 1H), 2.53 (s, 3H), 2.24-2.17 (m, 0.5H), 2.15-2.06 (m, 0.5H). LC/MS (ESI) m/z: 579/581 (M+H)⁺.

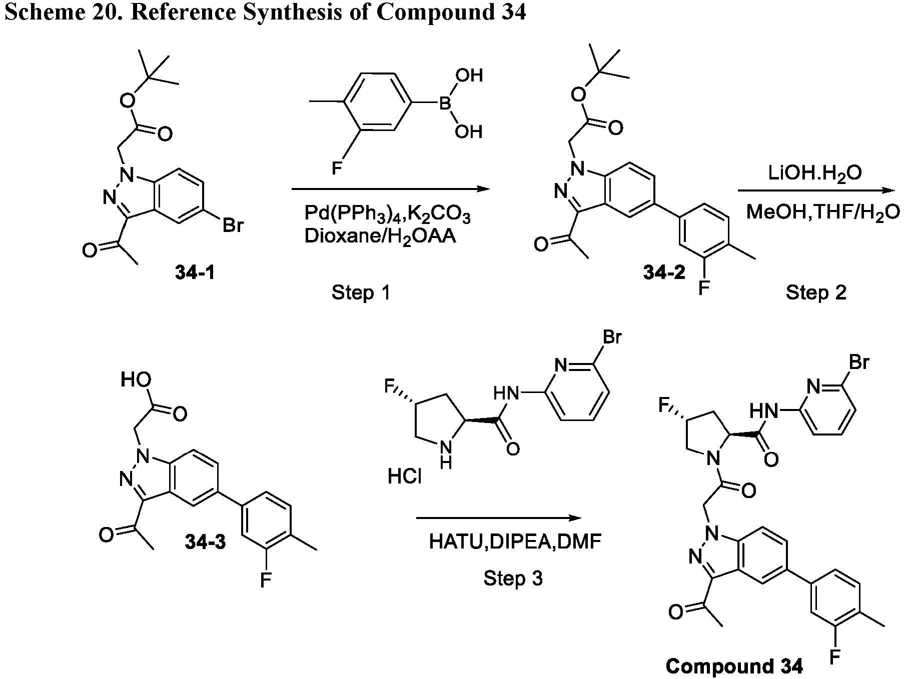

**Step 1: *tert*-Butyl 2-(3-acetyl-5-(3-fluoro-4-methylphenyl)-1*H*-indazol-1-yl)acetate (34-2):** To a mixture of compound **34-1** (300 mg, 0.85 mmol) and (3-fluoro-4-methylphenyl)boronic acid (119 mg, 0.94 mmol) in 1,4-dioxane (7 mL) and water (1 mL) was added potassium carbonate (293 mg, 2.13 mmol) and Pd(PPh₃)₄ (98 mg, 0.085 mmol) at 0 °C and the mixture was stirred at 90 °C under N₂ atmosphere for 2 hours. The mixture was poured into ice-water and extracted with EtOAC twice. The combined organic layers were washed with brine, dried over anhydrous Na₂SO₄, filtered and concentrated to dryness. The residue was purified by flash chromatography (PE: EtOAc= 50:1 to 1: 1) to afford compound **34-2** (266 mg, yield 82.1 %) as a white solid. LC/MS (ESI) m/z: 383 (M+H)⁺.

**Step 2: 2-(3-Acetyl-5-(3-fluoro-4-methylphenyl)-1*H*-indazol-1-yl)acetic acid (34-3):** To a solution of compound **34-2** (266 mg, 0.70 mmol) in methanol (4 mL) and THF (2 mL) was added a solution of LiOH (147 mg, 3.5 mmol) in water (2 mL) at 0 °C and the mixture was stirred at room temperature for 2 hours. The mixture was diluted with water and washed with ether. The aqueous layer was acidified with 1N aqueous HCl solution to a pH of approximately 3 and extracted with DCM twice. The combined organic layers were washed with brine, dried with anhydrous Na₂SO₄, filtered and concentrated to dryness to afford compound 34-3 (190 mg, yield 83.3%) as a white solid. LC/MS (ESI) m/z: 327 (M+H)⁺.

**Step 3: (2*S*,4*R*)-1-(2-(3-Acetyl-5-(3-fluoro-4-methylphenyl)-1*H*-indazol-1-yl)acetyl)-*N*-(6-bromopyridin-2-yl)-4-fluoropyrrolidine-2-carboxamide (Compound 34):** To a mixture of compound **34-3** (40 mg, 0.12 mmol) and (2S,4R)-N-(6-bromopyridin-2-yl)-4-fluoropyrrolidine-2-carboxamide hydrochloride (40 mg, 0.097 mmol) in DMF (3 mL) was added DIPEA (77 mg, 0.60 mmol) followed by HATU (68 mg, 0.18 mmol) at 0 °C and the mixture was stirred at room temperature for 1 hour. The mixture was diluted with EtOAc, washed with saturated aqueous NH₄Cl solution and brine, dried over anhydrous Na₂SO₄, filtered and concentrated to dryness. The residue was purified by preparative HPLC to afford **Compound 34** (34 mg, yield 47.9%) as a white solid. ¹H-NMR (400 MHz, DMSO-d₆) δ 11.01 (s, 1H), 8.35 (t, J= 1.4 Hz, 1H), 8.03 (d, J= 8.0 Hz, 1H), 7.82-7.77 (m, 2H), 7.71 (t, J= 8.0 Hz, 1H), 7.49-7.39 (m, 3H), 7.32 (d, J= 8.0 Hz, 1H), 5.82 (d, J= 17.2 Hz, 1H), 5.64 -5.60 (m, 1H), 5.52-5.04 (m, 1H), 4.68-4.64 (m, 1H), 4.23 (dd, J= 22.4, 22.4 Hz, 1H), 4.10-3.96 (m, 1H), 2.64 (s, 3H), 2.61-2.54 (m, 1H), 2.28 (d, J= 2.0 Hz, 3H), 2.24-2.17 (m, 0.5H), 2.14-2.07 (m, 0.5H). LC/MS (ESI) m/z: 596/598 (M+H)⁺.

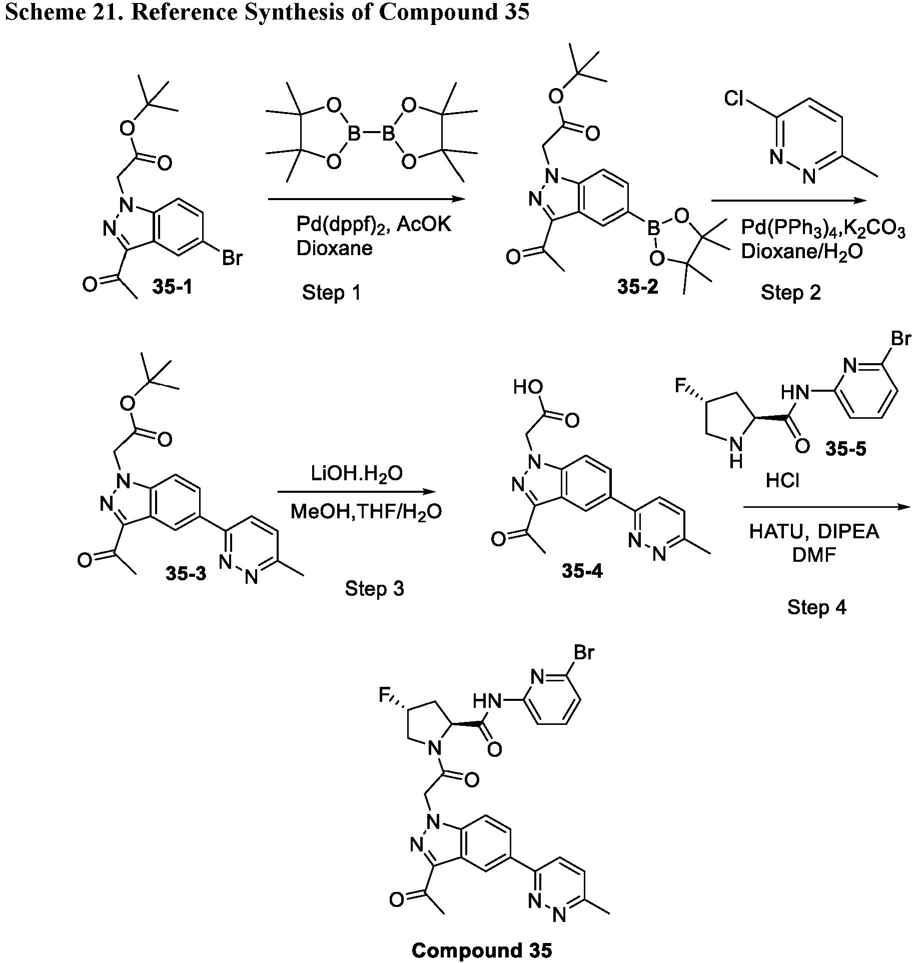

**Step 1: *tert*-Butyl 2-(3-acetyl-5-(4,4,5,5-tetramethyl-1,3,2-dioxaborolan-2-yl)-1*H-*indazol-1-yl)acetate (35-2):** To a mixture of compound **35-1** (1.0 g, 2.84 mmol) and 4,4,4',4',5,5,5',5'-octamethyl-2,2'-bi(1,3,2-dioxaborolane) (1.082 mg, 4.26 mmol) in 1,4-dioxane (10 mL) was added AcOK (835 mg, 8.52 mmol) and Pd(dppf)Cl₂ (104 mg, 0.14 mmol) at 0 °C and the mixture was stirred at 95 °C under N₂ atmosphere for 2 hours. The mixture was poured into ice-water and extracted with EtOAc twice. The combined organic layers were washed with brine, dried with anhydrous Na₂SO₄, filtered and concentrated to dryness. The residue was purified by flash chromatography (eluted with PE: EtOAc= 100: 1 to 1: 1) to afford compound **35-2** (980 mg, yield 86.2%) as a white solid. LC/MS (ESI) m/z: 401 (M+H)⁺.

**Step 2: *tert-Butyl* 2-(3-acetyl-5-(6-methylpyridazin-3-yl)-1*H*-indazol-1-yl)acetate (35-3):** To a mixture of compound **35-2** (200 mg, 0.50 mmol) and 3-chloro-6-methylpyridazine (70 mg, 0.55 mmol) in 1,4-dioxane (7 mL) and water (1 mL) was added potassium carbonate (172 mg, 1.25 mmol) and Pd(PPh₃)₄ (58 mg, 0.05 mmol) at 0 °C and the mixture was stirred at 90 °C under N₂ atmosphere for 2 hours. The mixture was diluted with EtOAc, washed with water and brine, dried over anhydrous Na₂SO₄, filtered and concentrated to dryness. The residue was purified by flash chromatography (eluted with PE: EtOAc= 50: 1 to 1: 1) to afford compound **35-3** (100 mg, yield 54.6%) as a yellow solid. LC/MS (ESI) m/z: 367 (M+H)⁺.

**Step 3: 2-(3-Acetyl-5-(6-methylpyridazin-3-yl)-1*H*-indazol-1-yl)acetic acid (35-4):** To a solution of compound **35-3** (100 mg, 0.27 mmol) in methanol (4 mL) and THF (2 mL) was added a solution of LiOH (58 mg, 1.37 mmol) in water (2 mL) at 0 °C and the mixture was stirred at room temperature for 2 hours. The mixture was diluted with water and washed with ether. The aqueous layer was acidified with 1N aqueous HCl solution to a pH of approximately 3 and extracted with DCM twice. The combined organic layers were washed with brine, dried with anhydrous Na₂SO₄, filtered and concentrated to dryness to afford compound **35-4** (50 mg, yield 59.7%) as a white solid. LC/MS (ESI) m/z: 311 (M+H)⁺.

**Step 4: (2*S*,4*R*)-1-(2-(3-Acetyl-5-(6-methylpyridazin-3-yl)-1*H*-indazol-1-yl)acetyl)-*N-*(6-bromopyridin-2-yl)-4-fluoropyrrolidine-2-carboxamide (Compound 35):** To a mixture of compound **35-4** (30 mg, 0.097 mmol) and (2S,4R)-N-(6-bromopyridin-2-yl)-4-fluoropyrrolidine-2-carboxamide hydrochloride (31 mg, 0.097 mmol) in DMF (3 mL) was added DIPEA (63 mg, 0.49 mmol) followed by HATU (57 mg, 0.15 mmol) at 0 °C and the mixture was stirred at room temperature for 1 hour. The mixture was diluted with EtOAc, washed with saturated aqueous NH₄Cl solution and brine, dried over anhydrous Na₂SO₄, filtered and concentrated to dryness. The residue was purified by preparative HPLC to afford **Compound 35** (13 mg, yield 23.2%) as a white solid. ¹H-NMR (400 MHz, DMSO-d₆) δ 11.01 (s, 1H), 8.90-8.87 (m, 1H), 8.25 (dd, J= 8.8, 8.8 Hz, 1H), 8.18 (d, J= 8.8 Hz, 1H), 8.03 (d, J= 8.4 Hz, 1H), 7.88-7.82 (m, 1H), 7.73-7.65 (m, 2H), 7.32 (d, J= 7.6 Hz, 1H), 5.85 (d, J= 17.6 Hz, 1H), 5.65 (d, J= 17.2 Hz, 1H), 5.49-5.00 (m, 1H), 4.70-4.65 (m, 1H), 4.24 (m, 1H), 4.10-3.96 (m, 1H), 2.68 (s, 3H), 2.66 (s, 3H), 2.63-2.54 (m, 1H), 2.25-2.17 (m, 0.5H), 2.14-2.07 (m, 0.5H). LC/MS (ESI) m/z: 580/582 (M+H)⁺.

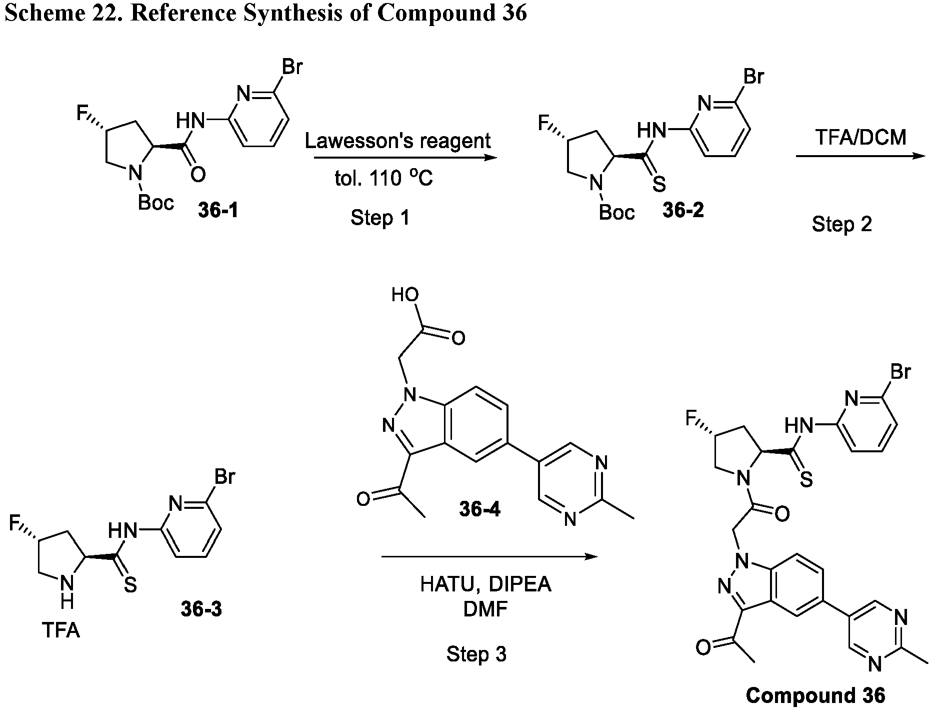

**Step 1: (2*S,*4*R*)*-tert*-Butyl 2-((6-bromopyridin-2-yl)carbamothioyl)-4-fluoropyrrolidine-1-carboxylate (36-2):** To a solution of compound **36-1** (200 mg, 0.52 mmol) in toluene (6 mL) was added Lawesson's reagent (210 mg, 0.52 mmol) under N₂ atmosphere and the mixture was stirred at 110 °C under N₂ atmosphere for 16 hours. The mixture was diluted with water and extracted with EtOAC twice. The combined organic layers were washed with brine, dried with anhydrous Na₂SO₄, filtered and concentrated to dryness. The residue was purified by chromatography on silica gel (eluted with PE: EtOAc= 30: 1) to afford compound **36-2** (68 mg, yield 32.5 %) as a yellow solid. LC/MS (ESI) m/z: 404/406 (M+H)⁺.

**Step 2: (2*S*,4*R*)-*N*-(6-Bromopyridin-2-yl)-4-fluoropyrrolidine-2-carbothioamide TFA salt (36-3):** To a solution of compound **36-2** (68 mg, 0.17 mmol) in DCM (2 mL) was added TFA (1 mL) at 0 °C and the mixture was stirred at room temperature for 1 hour. The reaction mixture was concentrated to dryness, washed with ether and dried under vacuum to afford compound **36-3** (50 mg, yield 74.1%) as yellow oil. LC/MS (ESI) m/z: 304/306 (M+H)⁺.

**Step 3: (2*S,*4*R*)-1-(2-(3-Acetyl-5-(2-methylpyrimidin-5-yl)-1*H*-indazol-1-yl)acetyl)-*N-*(6-bromopyridin-2-yl)-4-fluoropyrrolidine-2-carbothioamide (Compound 36):** To a mixture of compound **36-3** (50 mg, 0.124 mmol) and 2-(3-acetyl-5-(2-methylpyrimidin-5-yl)-1H-indazol-1-yl)acetic acid (53 mg, 0.17 mmol) in DMF (3 mL) was added DIPEA (110 mg, 0.85 mmol) followed by HATU (99 mg, 0.26 mmol) at 0 °C. The mixture was stirred at room temperature for 1 hour. The mixture was diluted with EtOAc and washed with saturated aqueous NH₄Cl solution and brine, dried over anhydrous Na₂SO₄, filtered and concentrated to dryness. The residue was purified by preparative HPLC to afford **Compound 36** (11.5 mg, yield 10.9%) as white solid. ¹H-NMR (400 MHz, DMSO-d₆) δ 12.43 (s, 1H), 9.03 (s, 2H), 8.81 (d, J= 8.4 Hz, 1H), 8.43-8.41 (m, 1H), 7.87-7.77 (m, 3H), 7.52 (d, J= 7.6 Hz, 1H), 5.86 (d, J= 17.2 Hz, 1H), 5.59 (d, J= 17.2 Hz, 1H), 5.20 (t, J= 8.2 Hz, 1H), 4.27-4.36 (m, 1H), 4.09-3.99 (m, 1H), 2.68 (s, 3H), 2.67 (m, 1H), 2.64 (s, 3H), 2.63-2.60 (m, 1H), 2.35-2.24 (m, 1H). LC/MS (ESI) m/z: 596/598 (M+H)⁺.

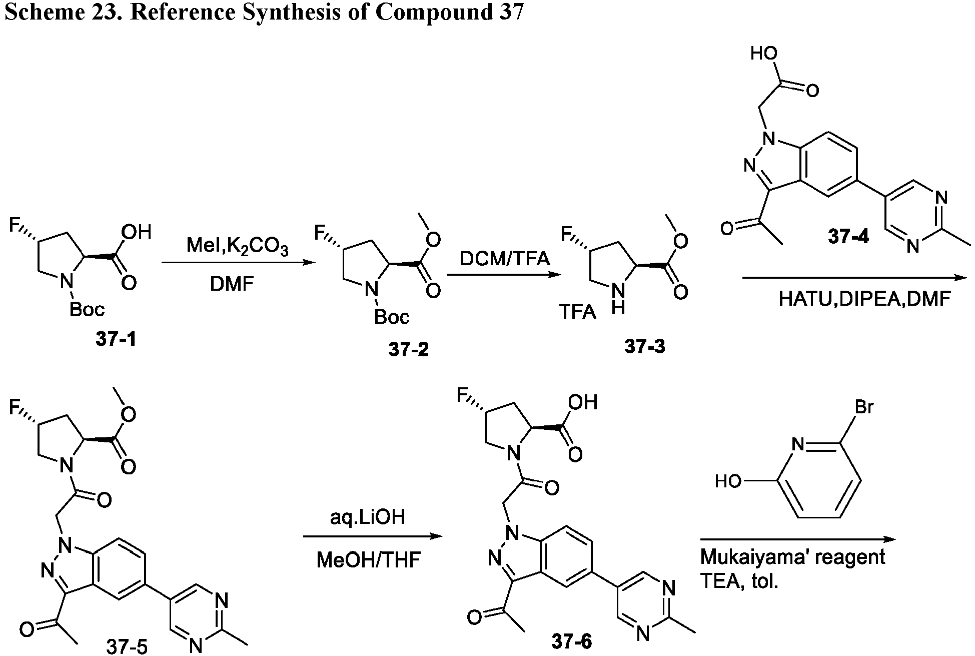

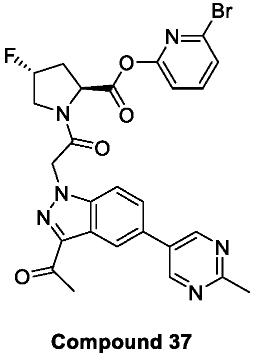

**Step 1: (2*S*,4*R*)-1-*tert*-Butyl 2-methyl 4-fluoropyrrolidine-1,2-dicarboxylate (37-2):** To a solution of compound 37-1 (1.0 g, 4.29 mmol) in DMF (10mL) was added K₂CO₃ (1.18 g, 8.58 mmol) followed by MeI (1.83 g, 12.9 mmol) at 0 °C and the mixture was stirred at room temperature under N₂ atmosphere for 2 hours. The mixture was poured into iced-water and extracted with EtOAc twice. The combined organic layers were washed with saturated aqueous NH₄Cl solution and brine, dried with anhydrous Na₂SO₄, filtered and concentrated to dryness. The residue was purified by chromatography on silica gel (eluted with PE: EtOAc= 20: 1) to afford compound **37-2** (1.01 g, yield 95.3%) as a light yellow oil. LC/MS (ESI)m/z: 248 (M+H)⁺.

**Step 2: (2*S*,4*R*)-Methyl 4-fluoropyrrolidine-2-carboxylate TFA salt (37-3):** To a solution of compound **37-2** (1.0 g, 4.05 mmol) in DCM (8 mL) was added TFA (4 mL) at 0 °C and the mixture was stirred at room temperature for 1 hour. The reaction mixture was concentrated to dryness, washed with ether and dried under vacuum to afford compound **37-3** (1.1 g, yield 100%) as a yellow oil that was directly used in the next reaction without purification. LC/MS (ESI) m/z: 148 (M+H)⁺.

**Step 3: (2S,4R)-Methyl 1-(2-(3-acetyl-5-(2-methylpyrimidin-5-yl)-1*H*-indazol-1-yl)acetyl)-4-fluoropyrrolidine-2-carboxylate (37-5):** To a mixture of compound **37-3** (159 mg, 0.65 mmol) and 2-(3-acetyl-5-(2-methylpyrimidin-5-yl)-1H-indazol-1-yl)acetic acid (200 mg, 0.65 mmol) in DMF (5 mL) was added DIPEA (419 mg, 3.25 mmol) followed by HATU (37-4, 372 mg, 0.98 mmol) at 0 °C and the mixture was stirred at room temperature for 1 hour. The mixture was diluted with EtOAc and washed with saturated aqueous NH₄Cl solution and brine, dried over anhydrous Na₂SO₄, filtered and concentrated to dryness. The residue was purified by flash chromatography (eluted with PE: EtOAc= 20: 1 to 1: 1) to afford compound **37-5** (210 mg, yield 73.5%) as yellow solid. LC/MS (ESI) m/z: 440 (M+H)⁺.

**Step** 4: **(2*S*,4*R*)-1-(2-(3-Acetyl-5-(2-methylpyrimidin-5-yl)-1*H*-indazol-1-yl)acetyl)-4-fluoropyrrolidine-2-carboxylic acid (37-6):** To a solution of compound **37-5** (200 mg, 0.46 mmol) in methanol (4 mL) and THF (2 mL) was added a solution of LiOH (97 mg, 2.30 mmol) in water (2 mL) at 0 °C and the mixture was stirred at room temperature for 1 hour. The mixture was diluted with water and washed with ether. The aqueous layer was acidified with 1N aqueous HCl solution to a pH of approximately 3 and extracted with DCM twice. The combined organic layers were washed with brine, dried with anhydrous Na₂SO₄, filtered and concentrated to dryness to afford compound **37-6** (190 mg, yield 99.5%) as a white solid. LC/MS (ESI) m/z: 426 (M+H)⁺.

**Step 5: (2*S*,4*R*)-6-Bromopyridin-2-yl 1-(2-(3-acetyl-5-(2-methylpyrimidin-5-yl)-1H-indazol-1-yl)acetyl)-4-fluoropyrrolidine-2-carboxylate (Compound 37):** To a mixture of compound **37-6** (190 mg, 0.45 mmol) and 6-bromopyridin-2-ol (78 mg, 0.45 mmol) in toluene (6 mL) was added TEA (136 mg, 1.35 mmol) followed by Mukaiyama's reagent (230 mg, 0.90 mmol) at 0 °C and the mixture was stirred at 60 °C under N₂ atmosphere for 16 hours. The mixture was diluted with water and extracted with EtOAC twice. The combined organic layers were washed with brine, dried with anhydrous Na₂SO₄, filtered and concentrated to dryness. The residue was purified by preparative HPLC to afford **Compound 37** (21.3 mg, yield 8.2%) as white solid. ¹H-NMR (400 MHz, DMSO-d₆) δ 9.04 (s, 2H), 8.44 (s, 1H), 7.90-7.83 (m, 3H), 7.65 (d, *J=* 8.0 Hz, 1H), 7.08 (d, *J=* 8.0 Hz, 1H), 5.89 (d, *J=* 17.2 Hz, 1H), 5.76 (d, *J=* 17.2 Hz, 1H), 4.68 (t, *J=* 8.6 Hz, 1H), 4.24-4.33 (m, 1H), 4.06-4.13 (m, 1H), 2.85-2.75 (m, 1H), 2.69 (s, 3H), 2.67-2.64 (m, 1H), 2.63 (s, 3H), 2.41-2.30 (m, 1H). LC/MS (ESI) m/z: 581/583 (M+H)⁺.

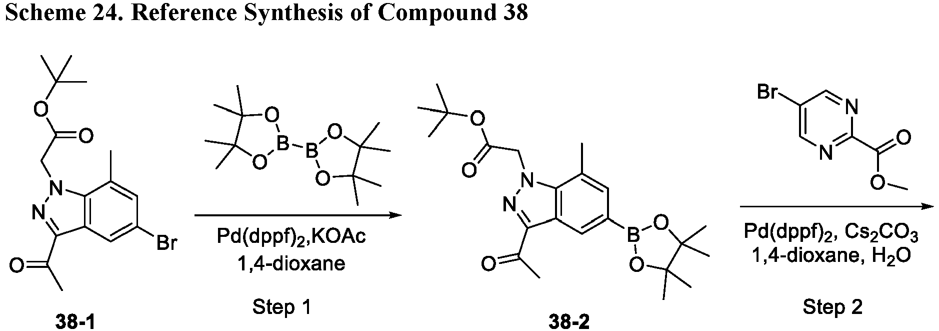

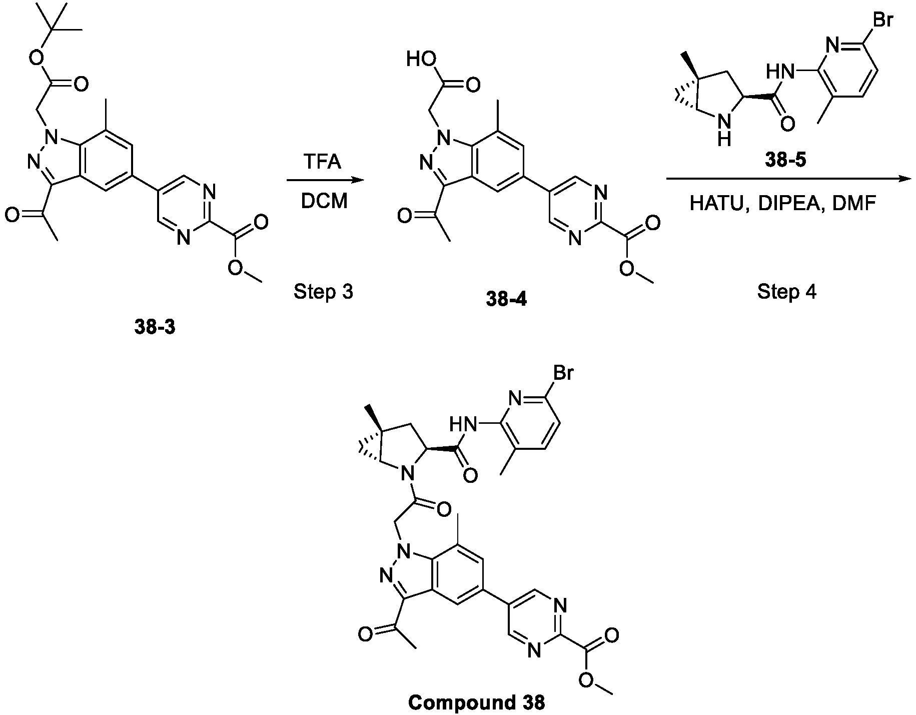

**Step 1: tert-butyl 2-(3-acetyl-7-methyl-5-(4,4,5,5-tetramethyl-1,3,2-dioxaborolan-2-yl)-1H-indazol-1-yl)acetate (38-2):** To a solution of compound **38-1** (0.225 g, 0.613 mmol, 1 equiv.) in 1,4-dioxane (1 mL) under an atmosphere of argon were added bis(pinacolato)diboron (0.156 g, 0.613 mmol, 1 equiv.) KOAc (0.120 g, 1.22 mmol, 2 equiv.) and[1,1'-bis(diphenylphosphino)ferrocene]dichloropalladium(II) (0.025g, 0.031 mmol, 0.05 equiv.). The reaction mixture was stirred at 90 °C for 4 h and was used in the next synthetic step without purification.

**Step 2: methyl 5-(3-acetyl-1-(2-(tert-butoxy)-2-oxoethyl)-7-methyl-1H-indazol-5-yl)pyrimidine-2-carboxylate (38-3):** To a solution of compound **38-2** (0.254 g, 0.613 mmol, 1 equiv.) in 1,4-dioxane (1 mL) and water (0.1 mL) were added methyl 5-bromopyrimidine-2-carboxylate (0.133 g, 0.613 mmol, 1 equiv.), cesium carbonate (0.399 g, 1.226 mmol, 2 equiv.), and [1,1'-bis(diphenylphosphino)ferrocene]dichloropalladium(II) (0.025 g, 0.031 mmol, 0.05 equiv.). The reaction mixture was stirred at 90 °C for 5 h and then concentrated to dryness. The residue was purified by column chromatography on silica gel (eluted with DCM/ MeOH) to give compound **38-3.**

**Step 3: 2-(3-acetyl-5-(2-(methoxycarbonyl)pyrimidin-5-yl)-7-methyl-1H-indazol-1-yl)acetic acid (38-4):** To a solution of compound **38-3** (0.169 g, 0.398 mmol, 1 equiv.) in dichloromethane (2 mL) at 0 °C under an atmosphere of argon was added trifluoroacetic acid (1 mL). The reaction mixture was stirred at rt for 3 h and concentrated to dryness. The material was used directly in the next synthetic step without purification.

**Step 4: methyl 5-(3-acetyl-1-(2-((1R,3S,5R)-3-((6-bromo-3-methylpyridin-2-yl)carbamoyl)-5-methyl-2-azabicyclo[3.1.0]hexan-2-yl)-2-oxoethyl)-7-methyl-1H-indazol-5-yl)pyrimidine-2-carboxylate (Compound 38):** To a solution of compound **38-5** (0.122 g, 0.394 mmol, 1 equiv.) in dimethylformamide (1 mL) at 0 °C under an atmosphere of argon were added compound **38-4** (0.145 g, 0.394mmol, 1 equiv.), HATU (0.315 g, 0.827 mmol, 2.1 equiv.), and DIPEA (0.255 g, 0.313mL, 1.97 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and then concentrated to dryness. The remaining residue was purified by column chromatography on silica gel (eluted with DCM/MeOH) to give **Compound 38.** ¹H NMR (400 MHz, DMSO-d6) δ 0.96 (s, 1H), 1.03 (d, J = 5.6 Hz, 1H), 1.34 (s, 3H), 2.06 (m, 4H), 2.57 (m, 1H), 2.66 (s, 3H), 2.71 (s, 3H), 3.54 - 3.67 (m, 1H), 3.95 (s, 3H), 4.43 (dd, J = 5.4, 9.3 Hz, 1H), 5.71 (d, J = 17.7 Hz, 1H), 6.06 (d, J = 17.9 Hz, 1H), 7.44 (d, J = 7.9 Hz, 1H), 7.63 (d, J = 8.0 Hz, 1H), 7.73 (s, 1H), 8.46 (s, 1H), 9.34 (s, 2H), 10.31 (s, 1H).

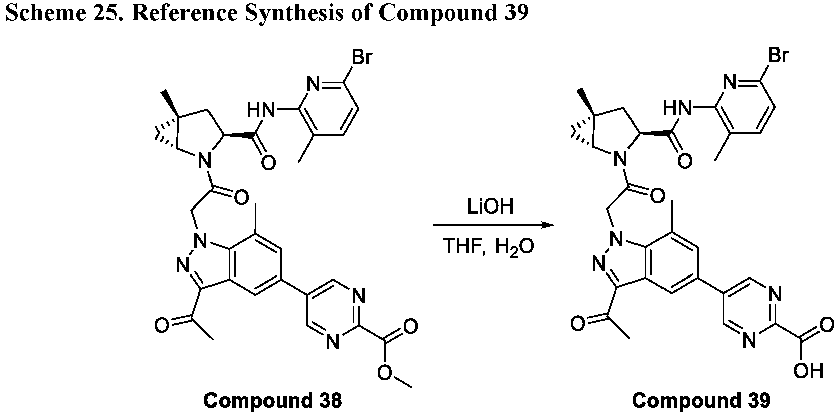

**5-(3-acetyl-1-(2-((1R,3S,5R)-3-((6-bromo-3-methylpyridin-2-yl)carbamoyl)-5-methyl-2-azabicyclo[3.1.0]hexan-2-yl)-2-oxoethyl)-7-methyl-1H-indazol-5-yl)pyrimidine-2-carboxylic acid (Compound 39):** To a solution of **Compound 38** (1 equiv.) in THF/H₂O (3:1, 10 vol) was added LiOH (2.1 equiv) and the reaction mixture was stirred at rt for 5 h, then concentrated under reduced pressure. The residue was neutralized using 2N HCl, and the resultant solid was filtered and purified by column chromatography on silica gel (eluted with DCM/ MeOH) to give **Compound 39.** ¹H NMR (400 MHz, DMSO-d6) δ 0.97 - 1.02 (m, 1H), 1.06 - 1.13 (m, 1H), 1.39 (s, 3H), 2.11 (s, 4H), 2.60 - 2.66 (m, 1H), 2.71 (s, 3H), 2.76 (s, 3H), 3.66 (dd, J = 2.4, 5.4 Hz, 1H), 4.48 (dd, J = 5.4, 9.2 Hz, 1H), 5.76 (d, J = 17.9 Hz, 1H), 6.11 (d, J = 17.9 Hz, 1H), 7.49 (d, J = 7.9 Hz, 1H), 7.68 (d, J = 7.9 Hz, 1H), 7.77 (s, 1H), 8.50 (d, J = 1.6 Hz, 1H), 9.34 (s, 2H), 10.36 (s, 1H), 13.62 (s, 1H).

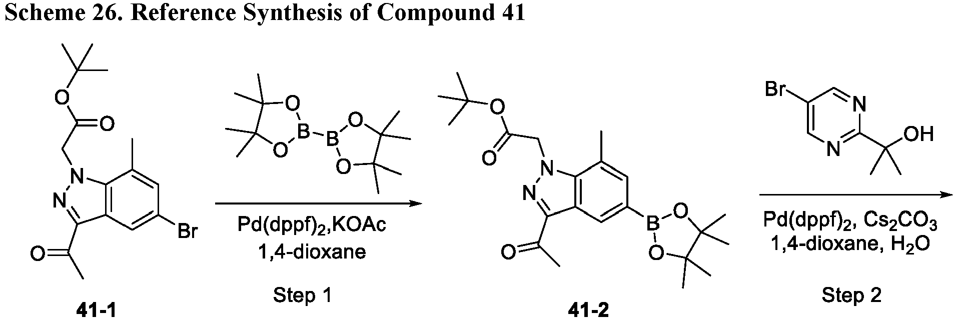

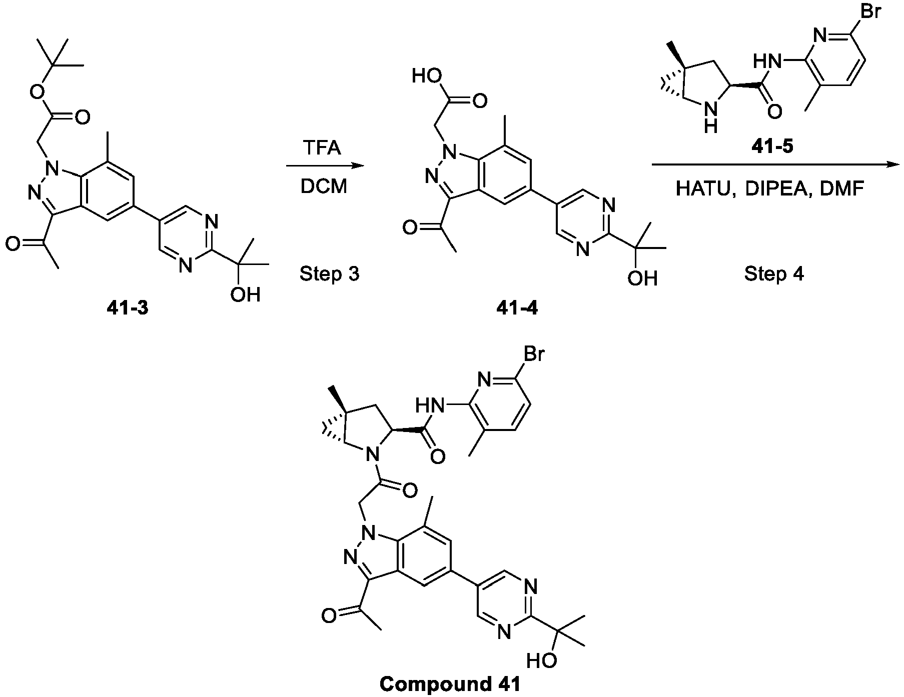

**Step 1: tert-butyl 2-(3-acetyl-7-methyl-5-(4,4,5,5-tetramethyl-1,3,2-dioxaborolan-2-yl)-1H-indazol-1-yl)acetate (41-2):** To a solution of compound **41-1** (0.200 g, 0.545 mmol, 1 equiv.) in 1,4-dioxane (1 mL) under an atmosphere of argon were added bis(pinacolato)diboron (0.138 g, 0.545 mmol, 1 equiv.), KOAc (0.107 g, 1.089 mmol, 2 equiv), and [1,1'-bis(diphenylphosphino)ferrocene]dichloropalladium(II), (0.022 g, 0.027 mmol, 0.05 equiv). The reaction mixture was stirred at 90 °C for 4 h and was used in the next synthetic step without purification.

**Step 2: tert-butyl 2-(3-acetyl-5-(2-(2-hydroxypropan-2-yl)pyrimidin-5-yl)-7-methyl-1H-indazol-1-yl)acetate (Compound 41-3):** To a solution of compound **41-3** (0.226 g, 0.545 mmol, 1 equiv.) in 1,4-dioxane (1 mL) and water (0.1 mL) were added 2-(5-bromopyrimidin-2-yl)propan-2-ol (0.118 g, 0.545 mmol, 1 equiv.), cesium carbonate (0.355 g, 1.09 mmol, 2 equiv.) and [1,1'-bis(diphenylphosphino)ferrocene]dichloropalladium(II), (0.022 g, 0.027 mmol, 0.05 equiv.). The reaction mixture was stirred at 90 °C for 5 h and then concentrated to dryness. The residue was purified by column chromatography on silica gel (eluted with DCM/ MeOH) to give compound **41-3.**

**Step 3: 2-(3-acetyl-5-(2-(2-hydroxypropan-2-yl)pyrimidin-5-yl)-7-methyl-1H-indazol-1-yl)acetic acid (41-4):** To a solution of compound **41-3** (0.07 g, 0.165 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The material was used directly in the next synthetic step without purification.

**Step 4: (1R,3S,5R)-2-(2-(3-acetyl-5-(2-(2-hydroxypropan-2-yl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 41):** To a solution of compound **41-4** (0.061 g, 0.165 mmol, 1 equiv.) in dimethylformamide (1 mL) at 0 °C under an atmosphere of argon were added compound **41-5** (0.051 g, 0.165 mmol, 1 equiv.), HATU (0.132 g, 0.347 mmol, 2.1 equiv.), and DIPEA (0.107 g, 0.825 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 41.** ¹H NMR (400 MHz, DMSO-d6) δ 0.93 - 0.98 (m, 1H), 1.00 - 1.06 (m, 1H), 1.34 (s, 3H), 1.56 (s, 6H), 2.01 - 2.09 (m, 4H), 2.54 - 2.60 (m, 1H), 2.65 (s, 3H), 2.70 (s, 3H), 3.55 - 3.66 (m, 1H), 4.36 - 4.47 (m, 1H), 5.10 (s, 1H), 5.69 (d, J = 17.8 Hz, 1H), 6.05 (d, J = 17.9 Hz, 1H), 7.44 (d, J = 7.9 Hz, 1H), 7.56 - 7.72 (m, 2H), 8.35 (d, J = 1.6 Hz, 1H), 9.12 (s, 2H), 10.30 (s, 1H).

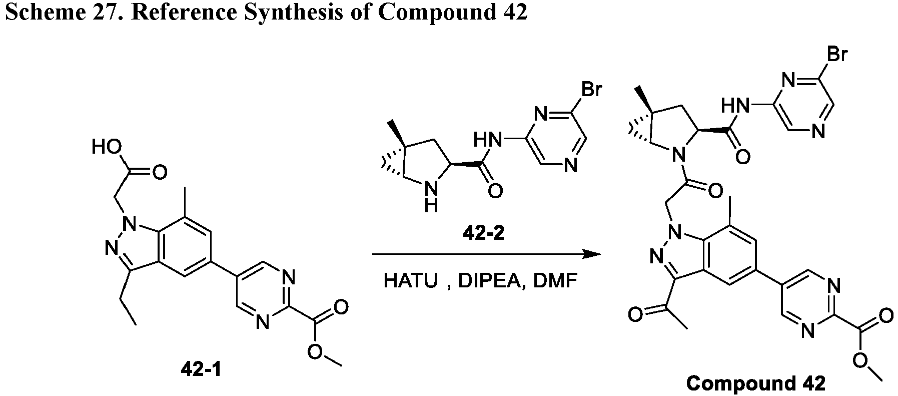

**Methyl 5-(3-acetyl-1-(2-((1R,3S,5R)-3-((6-bromopyrazin-2-yl)carbamoyl)-5-methyl-2-azabicyclo[3.1.0]hexan-2-yl)-2-oxoethyl)-7-methyl-1H-indazol-5-yl)pyrimidine-2-carboxylate (Compound 42):** To a solution of compound **42-2** (0.05 g, 0.136 mmol, 1 equiv.) in dimethylformamide (1 mL) at 0 °C under an atmosphere of argon were added compound **42-1** (0.04 g, 0.136 mmol, 1 equiv.), HATU (0.108 g, 0.288 mmol, 2.1 equiv.), and DIPEA (0.088 g, 0.679 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 42.** ¹H NMR (400 MHz, DMSO-d6) δ 0.94 (d, J = 2.6 Hz, 1H), 1.01 (t, J = 5.4 Hz, 1H), 1.32 (s, 3H), 2.05 (dd, J = 6.1, 13.3 Hz, 1H), 2.56 (m, 1H), 2.66 (s, 3H), 2.73 (s, 3H), 3.63 - 3.71 (m, 1H), 3.93 - 3.99 (m, 3H), 4.46 (dd, J = 6.1, 9.1 Hz, 1H), 5.70 (d, J = 17.8 Hz, 1H), 6.12 (d, 1H), 7.74 (s, 1H), 8.45 (d, J = 1.6 Hz, 1H), 8.54 (s, 1H), 9.27 (s, 1H), 9.33 (s, 2H), 11.17 (s, 1H).

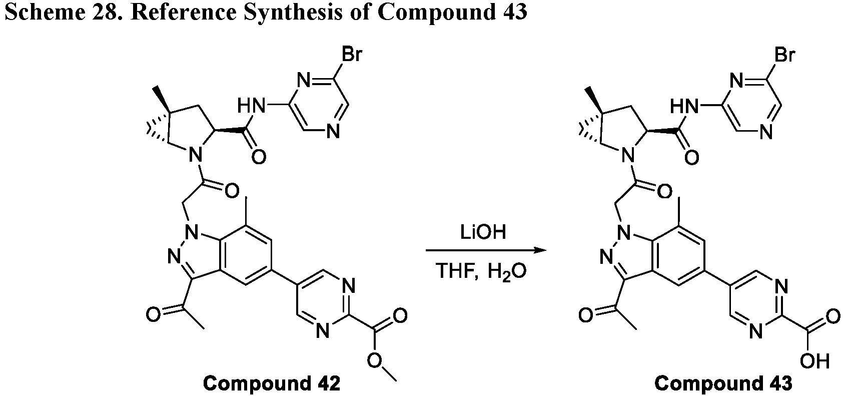

**5-(3-acetyl-1-(2-((1R,3S,5R)-3-((6-bromopyrazin-2-yl)carbamoyl)-5-methyl-2-azabicyclo[3.1.0]hexan-2-yl)-2-oxoethyl)-7-methyl-1H-indazol-5-yl)pyrimidine-2-carboxylic acid (Compound 43):** To a solution **Compound 42** (1 equiv) in THF/H₂O (3:1, 10 vol) was added LiOH (2.1 equiv) and the reaction mixture was stirred at rt for 5 h and then concentrated under reduced pressure. The remaining residue was neutralized using 2N HCl, and the resultant solid was filtered and purified by column chromatography on silica gel (eluted with DCM/ MeOH) to give **Compound 43.** ¹H NMR (400 MHz, DMSO-d6) δ 0.94 (s, 1H), 1.01 (t, J = 5.4 Hz, 1H), 1.32 (s, 3H), 2.05 (dd, J = 6.1, 13.3 Hz, 1H), 2.53 - 2.57 (m, 1H), 2.66 (s, 3H), 2.73 (s, 3H), 3.62 - 3.69 (m, 1H), 4.42 - 4.52 (m, 1H), 5.70 (d, J = 17.9 Hz, 1H), 6.10 (d, J = 18.0 Hz, 1H), 7.73 (s, 1H), 8.43 (s, 1H), 8.54 (s, 1H), 9.26 - 9.32 (m, 3H), 11.17 (s, 1H).

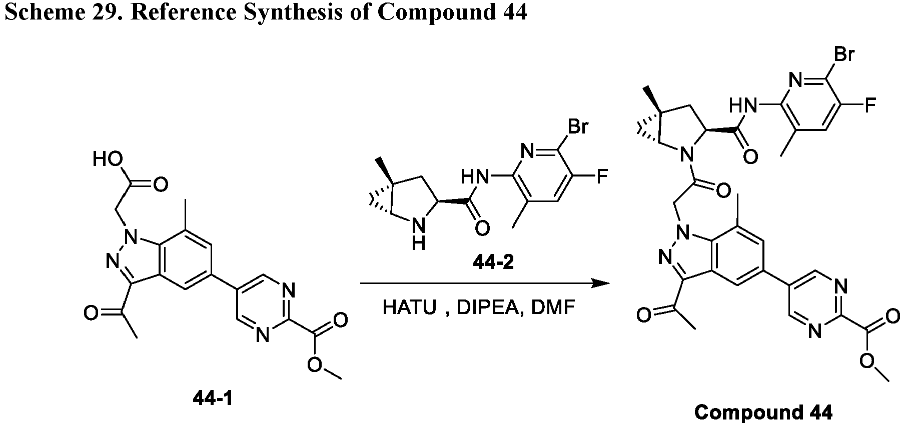

**Methyl 5-(3-acetyl-1-(2-((1R,3S,5R)-3-((6-bromo-5-fluoro-3-methylpyridin-2-yl)carbamoyl)-5-methyl-2-azabicyclo[3.1.0]hexan-2-yl)-2-oxoethyl)-7-methyl-1H-indazol-5-yl)pyrimidine-2-carboxylate (Compound 44):** To a solution of compound 44-2 (0.05 g, 0.136 mmol, 1 equiv.) in dimethylformamide (2 mL) at 0 °C under an atmosphere of argon were added compound **44-1** (0.045 g, 0.136 mmol, 1 equiv.), HATU (0.109 g, 0.286 mmol, 2.1 equiv.), and DIPEA (0.088 g, 0.680 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 44.** ¹H NMR (400 MHz, DMSO-d6) δ 0.92 - 0.98 (m, 1H), 1.01 - 1.06 (m, 1H), 1.33 (s, 3H), 2.01 - 2.11 (m, 4H), 2.53 - 2.62 (m, 1H), 2.66 (s, 3H), 2.71 (s, 3H), 3.53 - 3.65 (m, 1H), 3.95 (s, 3H), 4.29 - 4.44 (m, 1H), 5.71 (d, J = 17.8 Hz, 1H), 6.06 (d, J = 17.9 Hz, 1H), 7.74 (s, 1H), 7.84 (d, 1H), 8.46 (d, J = 1.6 Hz, 1H), 9.34 (s, 2H), 10.31 (s, 1H).

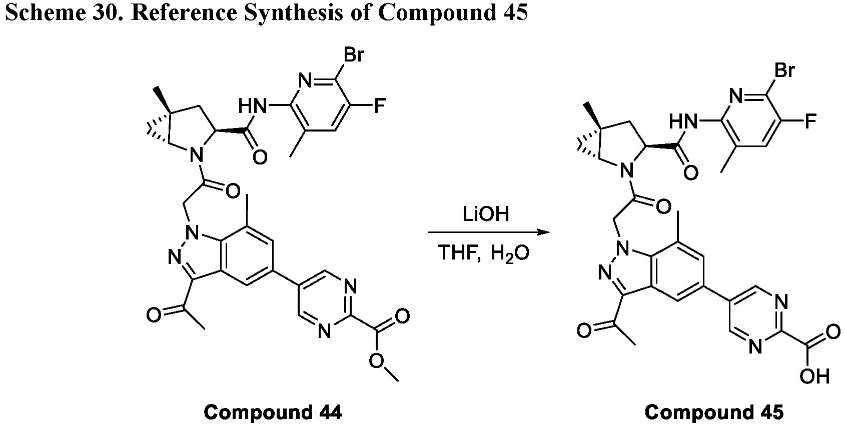

**5-(3-acetyl-1-(2-((1R,3S,5R)-3-((6-bromo-5-fluoro-3-methylpyridin-2-yl)carbamoyl)-5-methyl-2-azabicyclo[3.1.0]hexan-2-yl)-2-oxoethyl)-7-methyl-1H-indazol-5-yl)pyrimidine-2-carboxylic acid (Compound 45):** To a solution of **Compound 44** (1 equiv.) in THF/H₂O (3:1, 10 vol) was added LiOH (2.1 equiv) and the reaction mixture was stirred at rt for 5 h and then concentrated under reduced pressure. The residue was neutralized using 2N HCl, and the resultant solid was filtered and purified by column chromatography on silica gel (eluted with DCM/ MeOH) to give **Compound 45.** ¹H NMR (400 MHz, DMSO-d6) δ 0.99 - 1.02 (m, 1H), 1.06 - 1.10 (m, 1H), 1.38 (s, 3H), 2.05 - 2.16 (m, 4H), 2.58 - 2.66 (m, 1H), 2.71 (s, 3H), 2.76 (s, 3H), 3.64 - 3.71 (m, 1H), 4.37 - 4.54 (m, 1H), 5.76 (d, J = 17.8 Hz, 1H), 6.11 (d, J = 17.8 Hz, 1H), 7.77 (s, 1H), 7.88 (d, J = 8.4 Hz, 1H), 8.50 (d, J = 1.6 Hz, 1H), 9.35 (s, 2H), 10.36 (s, 1H), 13.62 (s, 1H).

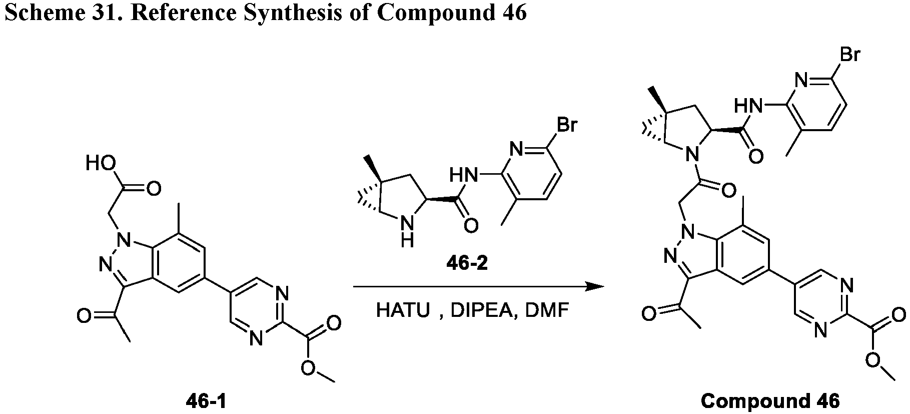

**Step 1: methyl 5-(3-acetyl-1-(2-((1R,3S,SR)-3-((6-bromo-3-methylpyridin-2-yl)carbamoyl)-5-methyl-2-azabicyclo[3.1.0]hexan-2-yl)-2-oxoethyl)-1H-indazol-5-yl)pyrimidine-2-carboxylate (Compound 46):** To a solution of compound **46-2** (0.131 g, 0.423 mmol, 1 equiv.) in dimethylformamide (1 mL) at 0 °C under an atmosphere of argon were added compound **46-1** (0.150 g, 0.423 mmol, 1 equiv.), HATU (0.388 g, 0.889 mmol, 2.1 equiv.), and DIPEA (0.274 g, 2.117 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 46.** ¹H NMR (400 MHz, DMSO-d6) δ 0.99 - 1.07 (m, 2H), 1.33 (s, 3H), 2.05 (s, 4H), 2.54 - 2.60 (m, 1H), 2.68 (s, 3H), 3.60 (dd, J = 2.4, 5.6 Hz, 1H), 3.96 (s, 3H), 4.42 (dd, J = 5.1, 9.2 Hz, 1H), 5.60 (d, J = 17.2 Hz, 1H), 5.94 (d, J = 17.3 Hz, 1H), 7.45 (d, J = 7.9 Hz, 1H), 7.63 (d, J = 7.9 Hz, 1H), 7.91 (d, J = 8.9 Hz, 1H), 7.98 (dd, J = 1.7, 8.8 Hz, 1H), 8.59 (d, J = 1.6 Hz, 1H), 9.36 (s, 2H), 10.27 (s, 1H).

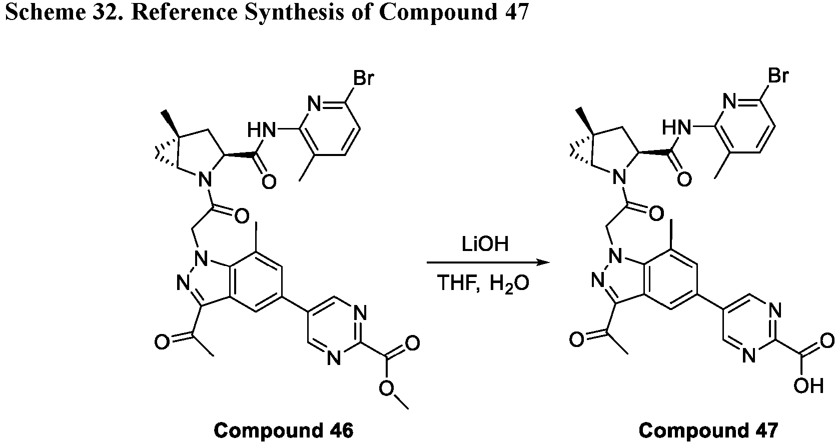

**5-(3-acetyl-1-(2-((1R,3S,SR)-3-((6-bromo-3-methylpyridin-2-yl)carbamoyl)-5-methyl-2-azabicyclo[3.1.0]hexan-2-yl)-2-oxoethyl)-1H-indazol-5-yl)pyrimidine-2-carboxylic acid (Compound 47):** To a solution of **Compound 46** (1 equiv) in THF/H₂O (3:1, 10 vol) was added LiOH (2.1 equiv) and the reaction mixture was stirred at rt for 5 h and then concentrated under reduced pressure. The residue was neutralized using 2N HCl, and the resultant solid was filtered and purified by column chromatography on silica gel (eluted with DCM/ MeOH) to give **Compound 47.** ¹H NMR (400 MHz, DMSO-d6) δ 0.98 - 1.09 (m, 2H), 1.34 (s, 3H), 2.01 - 2.10 (m, 4H), 2.54 - 2.59 (m, 1H), 2.68 (s, 3H), 3.59 - 3.66 (m, 1H), 4.40 - 4.49 (m, 1H), 5.60 (d, J = 17.2 Hz, 1H), 5.94 (d, J = 17.3 Hz, 1H), 7.45 (d, J = 7.9 Hz, 1H), 7.63 (d, J = 8.0 Hz, 1H), 7.90 (d, J = 8.8 Hz, 1H), 7.96 - 8.03 (m, 1H), 8.57 (s, 1H), 9.33 (s, 2H), 10.27 (s, 1H), 13.57 (s, 1H).

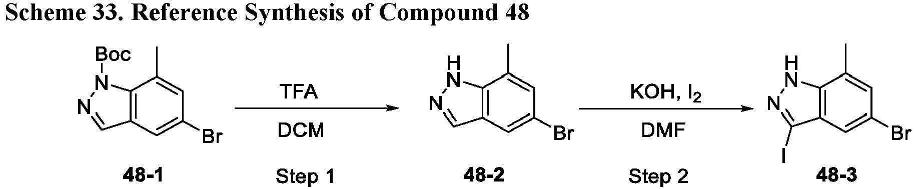

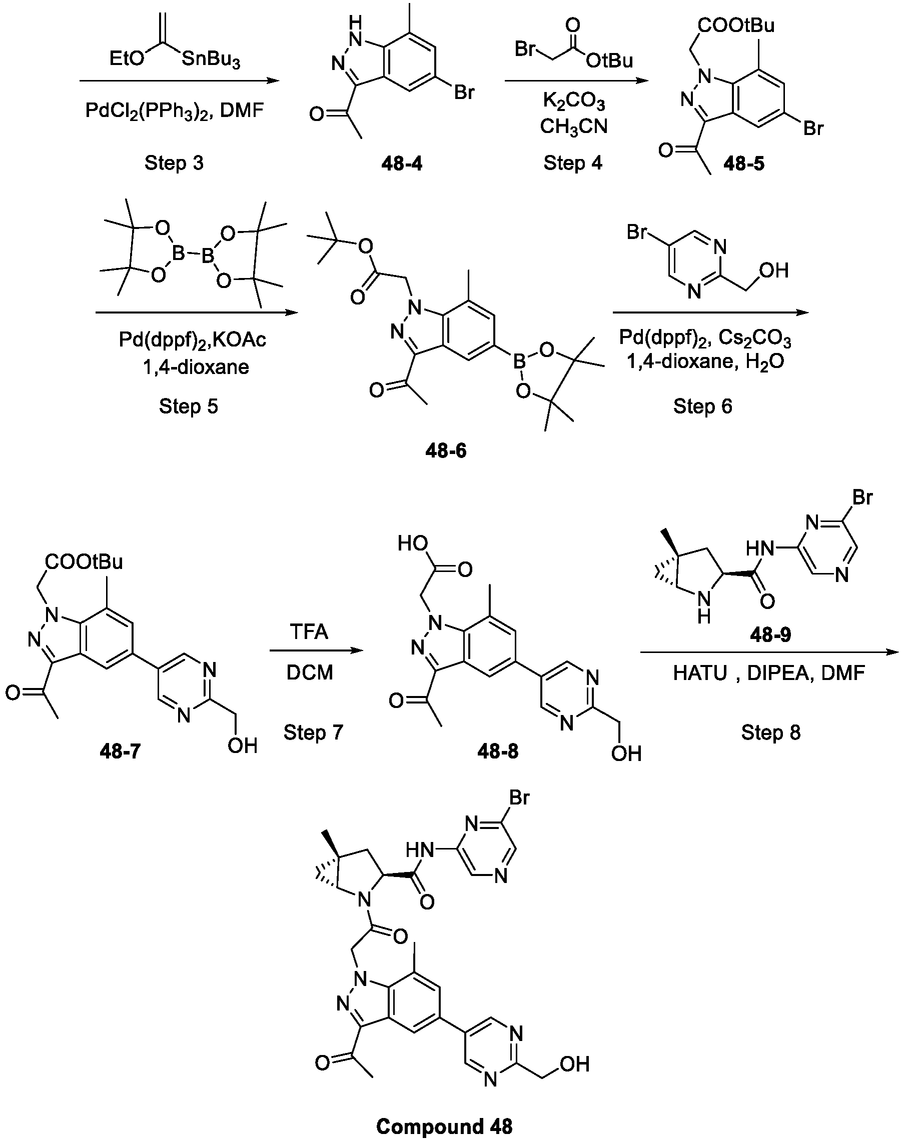

**Step 1: 5-bromo-7-methyl-1H-indazole (48-2):** To a solution of compound **48-1** (1 equiv) in DCM (10 vol) at 0 °C under an atmosphere of argon was added TFA (5 vol). The reaction mixture was stirred at room temperature for 3 h and then concentrated to dryness. The remaining material was used directly in the next synthetic step.

**Step 2: 5-bromo-3-iodo-7-methyl-1H-indazole (48-3):** To a solution of compound **48-2** (0.678 g, 3.21 mmol, 1 equiv.) in dimethylformamide (5 mL) were added potassium hydroxide (0.379 g, 6.75 mmol, 2.1 equiv.) and molecular iodine (1.06 g, 4.178 mmol, 1.3 equiv.) at 0 °C. The reaction mixture was stirred at rt for 48h, then quenched with 5% aqueous Na₂S₂O₃ solution and extracted with EtOAc. The organic layer was separated, dried, and concentrated to give the crude product, which was purified by column chromatography on silica gel (eluted with MeOH/DCM) to give compound **48-3.**

**Step 3: 1-(5-bromo-7-methyl-1H-indazol-3-yl)ethan-1-one (48-4):** To a solution of compound **48-3** (1 g, 2.97 mmol, 1 equiv.) in dry dimethyl-formamide (10 mL) were added tributyl(1-ethoxyethenyl)stannane (1.18 g, 1.10 mL, 3.27 mmol, 1.1 equiv.) and dichlorobis(triphenylphosphine)palladium (0.104 g, 0.148 mmol, 0.05 equiv.), then the resulting mixture was stirred at 80 °C under argon overnight and then concentrated. The remaining residue was diluted with DCM and washed with 2N aqueous HCl, and the organic layer was separated, dried over anhydrous Na₂SO₄, and concentrated to dryness. The obtained crude product was purified by column chromatography on silica gel (eluted with DCM/ MeOH) to give compound **48-4.**

**Step 4: tert-butyl 2-(3-acetyl-5-bromo-7-methyl-1H-indazol-1-yl)acetate (48-5):** To a solution of compound **48-4** (0.751 g, 2.97 mmol, 1 equiv.) in dimethyl-formamide (10 mL) were added potassium carbonate (0.41 g, 2.97 mmol, 1 equiv.) and tert-butyl 2-bromoacetate (0.637 g, 0.482 mL, 3.265 mmol, 1.1 equiv.). The mixture was stirred at rt overnight under an atmosphere of argon and mixture was filtered through Celite and washed with CH₃CN. The filtrate was concentrated under reduced pressure and the remaining residue was purified by column chromatography on silica gel (eluted with DCM/EtOAc) to give compound **48-5**

**Step 5: tert-butyl 2-(3-acetyl-7-methyl-5-(4,4,5,5-tetramethyl-1,3,2-dioxaborolan-2-yl)-1H-indazol-1-yl)acetate (48-6):** To a solution of compound **48-5** (0.622 g, 1.694 mmol, 1 equiv.) in 1,4-dioxane (10 mL) under an atmosphere of argon were added bis(pinacolato)diboron (0.430g, 1.694 mmol, 1 equiv.), KOAc (0.332 g, 0.387 mmol, 2 equiv.), and [1,1'-bis(diphenylphosphino)ferrocene]dichloropalladium(II) (0.069 g, 0.085 mmol, 0.05 equiv.). The reaction mixture was stirred at 90 °C for 4 h and was directly used in the next synthetic step without purification.

**Step 6: tert-butyl 2-(3-acetyl-5-(2-(hydroxymethyl)pyrimidin-5-yl)-7-methyl-1H-indazol-1-yl)acetate (48-7):** To a solution of compound **48-6** (0.526 g, 1.27 mmol, 1 equiv.) in 1,4-dioxane (10 mL) and water (1 mL) were added (5-bromopyrimidin-2-yl)methanol (0.240 g, 1.27 mmol, 1 equiv.), cesium carbonate (0.827 g, 2.54 mmol, 2 equiv.) and [1,1'-bis(diphenylphosphino)ferrocene]dichloropalladium(II) (0.052 g, 0.063 mmol, 1 equiv.). The reaction mixture was stirred at 90 °C for 5 h and then concentrated to dryness. The remaining residue was purified by column chromatography on silica gel (eluted with DCM/ MeOH) to give compound **48-7.**

**Step 7: 2-(3-acetyl-5-(2-(hydroxymethyl)pyrimidin-5-yl)-7-methyl-1H-indazol-1-yl)acetic acid (48-8):** To a solution of compound **48-7** (0.160g, 0.404 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The material was used directly in the next synthetic step.

**Step 8: (1R,3S,5R)-2-(2-(3-acetyl-5-(2-(hydroxymethyl)pyrimidin-5-yl)-7-methyl-1H-indazol-1-yl)acetyl)-N-(6-bromopyrazin-2-yl)-5-methyl-2-azabicyclo[3.1.0]hexane-3-carboxamide (Compound 48):** To a solution of compound **48-8** (0.043 g, 0.126 mmol, 1 equiv.) in dimethylformamide (1 mL) at 0 °C under an atmosphere of argon were added compound **48-9** (0.037 g, 0.126 mmol, 1 equiv.), HATU (0.101 g, 0.265 mmol, 2.1 equiv.), and DIPEA (0.081 g, 0.11 mL, 0.63 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 48.** ¹H NMR (400 MHz, DMSO-d6) δ 0.91 - 0.96 (m, 1H), 0.98 - 1.04 (m, 1H), 1.24 - 1.29 (m, 1H), 1.32 (s, 3H), 2.04 (dd, J = 6.1, 13.3 Hz, 1H), 2.53 - 2.57 (m, 1H), 2.65 (s, 3H), 2.72 (s, 3H), 3.61 - 3.69 (m, 1H), 4.45 (dd, J = 6.1, 9.1 Hz, 1H), 4.68 (s, 2H), 5.68 (d, J = 17.8 Hz, 1H), 6.09 (d, J = 17.9 Hz, 1H), 7.65 (s, 1H), 8.34 (d, J = 1.6 Hz, 1H), 8.54 (s, 1H), 9.11 (s, 2H), 9.27 (s, 1H), 11.16 (s, 1H).

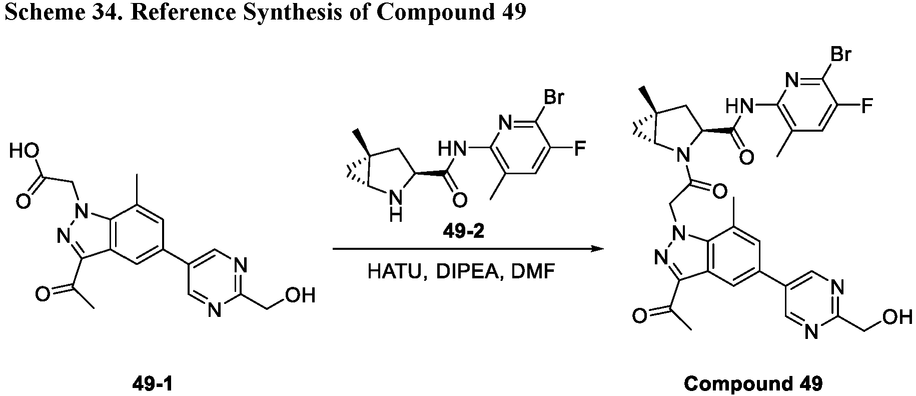

**(1R,3S,5R)-2-(2-(3-acetyl-5-(2-(hydroxymethyl)pyrimidin-5-yl)-7-methyl-1H-indazol-1-yl)acetyl)-N-(6-bromo-5-fluoro-3-methylpyridin-2-yl)-5-methyl-2-azabicyclo[3.1.0]hexane-3-carboxamide (Compound 49):** To a solution of compound **49-1** (0.04 g, 0.117 mmol, 1 equiv.) in dimethylformamide (1 mL) at 0 °C under an atmosphere of argon were added compound **49-2** (0.038 g, 0.117 mmol, 1 equiv.), HATU (0.093 g, 0.246 mmol, 2.1 equiv.), and DIPEA (0.076 g, 0.585 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound 49.** ¹H NMR (400 MHz, DMSO-d6) δ 0.93 - 0.97 (m, 1H), 1.03 (t, J = 5.4 Hz, 1H), 1.33 (s, 3H), 2.00 - 2.07 (m, 1H), 2.08 (s, 3H), 2.55 - 2.61 (m, 1H), 2.65 (s, 3H), 2.70 (s, 3H), 3.54 - 3.66 (m, 1H), 4.40 (dd, J = 5.5, 9.2 Hz, 1H), 4.68 (d, J = 6.3 Hz, 2H), 5.34 (t, J = 6.3 Hz, 1H), 5.70 (d, J = 17.8 Hz, 1H), 6.04 (d, J = 17.9 Hz, 1H), 7.65 (s, 1H), 7.83 (d, J = 8.3 Hz, 1H), 8.36 (s, 1H), 9.12 (s, 2H), 10.31 (s, 1H).

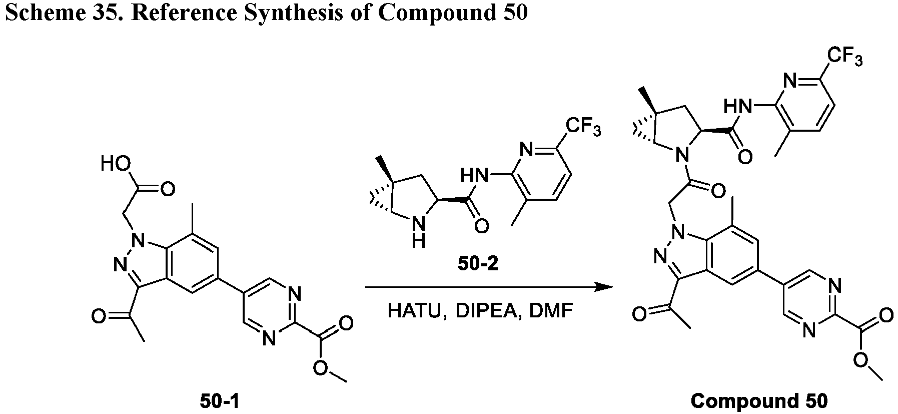

**Methyl 5-(3-acetyl-7-methyl-1-(2-((1R,3S,SR)-5-methyl-3-((3-methyl-6-(trifluoromethyl)pyridin-2-yl)carbamoyl)-2-azabicyclo[3.1.0]hexan-2-yl)-2-oxoethyl)-1H-indazol-5-yl)pyrimidine-2-carboxylate (Compound 50):** To a solution of compound **50-2** (0.037 g, 0.125mmol, 1 equiv.) in dimethylformamide (1 mL) at 0 °C under an atmosphere of argon were added 50-1 (0.046 g, 0.125 mmol, 1 equiv.), HATU (0.10 g, 0.263 mmol, 2.1 equiv.), and DIPEA (0.081 g, 0.625 mmol, 5 equiv.). The reaction mixture was stirred at room temperature for 1 h and then quenched with water (10 vol). The mixture was extracted with DCM, the organic layer was washed with brine, dried over anhydrous Na₂SO₄, filtered, and concentrated to dryness. The residue was purified by column chromatography on silica gel (eluted with DCM/MeOH) to give **Compound 50.** ¹H NMR (400 MHz, DMSO-d6) δ 0.93 - 0.98 (m, 1H), 1.01 - 1.07 (m, 1H), 1.34 (s, 3H), 2.07 (dd, J = 5.4, 13.3 Hz, 1H), 2.19 (s, 3H), 2.54 - 2.62 (m, 1H), 2.66 (s, 3H), 2.71 (s, 3H), 3.62 (dd, J = 2.4, 5.5 Hz, 1H), 3.95 (s, 3H), 4.48 (dd, J = 5.5, 9.2 Hz, 1H), 5.71 (d, J = 17.8 Hz, 1H), 6.07 (d, J = 17.8 Hz, 1H), 7.66 - 7.79 (m, 2H), 7.95 (d, J = 7.9 Hz, 1H), 8.46 (d, J = 1.6 Hz, 1H), 9.33 (s, 2H), 10.47 (s, 1H).

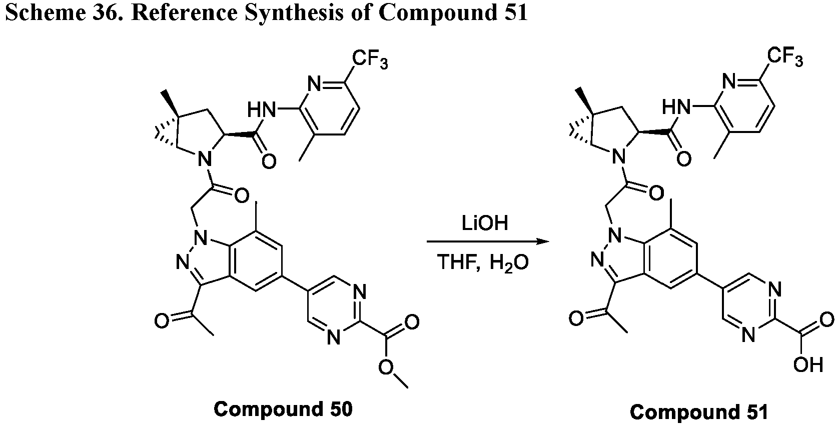

**5-(3-acetyl-7-methyl-1-(2-((1R,3S,SR)-5-methyl-3-((3-methyl-6-(trifluoromethyl)pyridin-2-yl)carbamoyl)-2-azabicyclo[3.1.0]hexan-2-yl)-2-oxoethyl)-1H-indazol-5-yl)pyrimidine-2-carboxylic acid (Compound 51):** To a solution of **Compound 50** (1 equiv.) in THF/H₂O (3:1, 10 vol) was added LiOH (2.1 equiv), and the reaction mixture was stirred at rt for 5 h and then concentrated under reduced pressure. The residue was neutralized using 2N HCl, and the resultant solid was filtered and purified by column chromatography on silica gel (eluted with DCM/ MeOH) to give **Compound 51.** ¹H NMR (400 MHz, DMSO-d6) δ 0.97 - 1.03 (m, 1H), 1.06 - 1.12 (m, 1H), 1.39 (s, 3H), 2.12 (dd, J = 5.4, 13.2 Hz, 1H), 2.24 (s, 3H), 2.59 - 2.68 (m, 1H), 2.71 (s, 3H), 2.77 (s, 3H), 3.67 (dd, J = 2.4, 5.4 Hz, 1H), 4.54 (dd, J = 5.5, 9.1 Hz, 1H), 5.76 (d, J = 17.8 Hz, 1H), 6.12 (d, J = 17.9 Hz, 1H), 7.70 - 7.80 (m, 2H), 8.00 (d, J = 7.8 Hz, 1H), 8.49 (s, 1H), 9.34 (s, 2H), 10.52 (s, 1H), 13.62 (s, 1H).

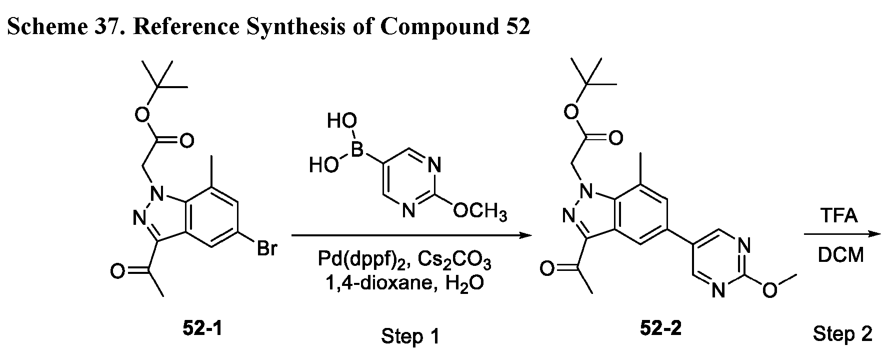

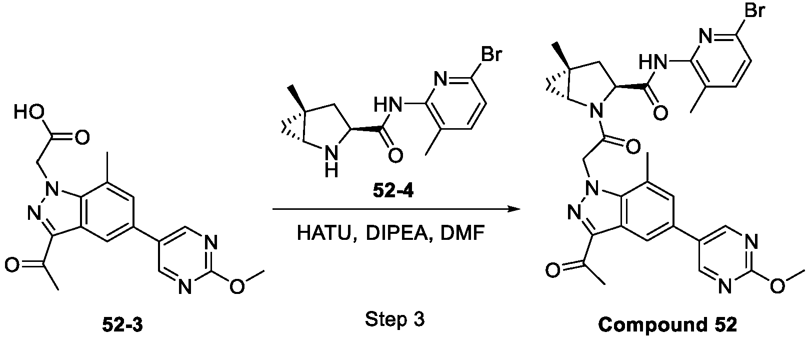

**Step 1: tert-butyl 2-(3-acetyl-5-(2-methoxypyrimidin-5-yl)-7-methyl-1H-indazol-1-yl)acetate (52-2):** To a solution of compound **52-1** (0.335 g, 0.912 mmol, 1 equiv.) in 1,4-dioxane (5 mL) and water (0.5 mL) were added (2-methoxypyrimidin-5-yl)boronic acid (0.140 g, 0.912 mmol, 1 equiv.), cesium carbonate (0.594 g, 1.82 mmol, 2 equiv.), and [1,1'-Bis(diphenylphosphino)ferrocene]dichloropalladium(II) (0.037 g, 0.046 mmol, 0.05 equiv.). The reaction mixture was stirred at 90 °C for 5 h and then concentrated to dryness. The residue was purified by column chromatography on silica gel (eluted with DCM/ MeOH) to give compound **52-2.**

**Step 2: 2-(3-acetyl-5-(2-methoxypyrimidin-5-yl)-7-methyl-1H-indazol-1-yl)acetic acid (52-3):** To a solution of compound **52-2** (0.160 g, 0.404 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The crude material was used directly in the next synthetic step without purification.

**Step 3: (1R,3S,5R)-2-(2-(3-acetyl-5-(2-methoxypyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 52):** To a solution of compound **52-4** (0.047 g, 0.138 mmol, 1 equiv.) in dimethyl-formamide (1 mL) at 0 °C under an atmosphere of argon were added compound **52-3** (0.043 g, 0.138 mmol, 1 equiv.), HATU (0.110 g, 0.290 mmol, 2.1 equiv.), and DIPEA (0.089 g, 0.690 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound 52.** ¹H NMR (400 MHz, DMSO-d6) δ 0.92 - 0.97 (m, 1H), 1.03 (t, J = 5.4 Hz, 1H), 1.33 (s, 3H), 2.00 - 2.08 (m, 4H), 2.53 - 2.60 (m, 1H), 2.65 (s, 3H), 2.68 (s, 3H), 3.58 - 3.64 (m, 1H), 3.99 (s, 3H), 4.42 (dd, J = 5.4, 9.2 Hz, 1H), 5.68 (d, J = 17.8 Hz, 1H), 6.03 (d, J = 17.9 Hz, 1H), 7.44 (d, J = 7.9 Hz, 1H), 7.56 (s, 1H), 7.63 (d, J = 7.9 Hz, 1H), 8.27 (d, J = 1.6 Hz, 1H), 8.93 (s, 2H), 10.30 (s, 1H).

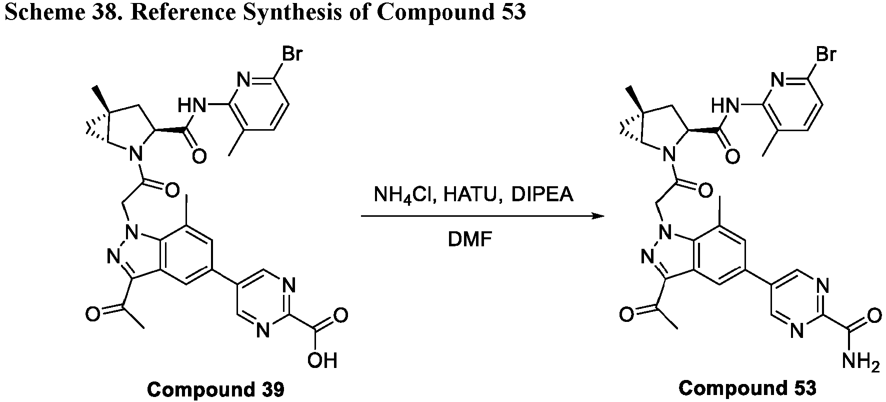

**(1R,3S,5R)-2-(2-(3-acetyl-5-(2-carbamoylpyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 53):** To a solution of **Compound 39** (0.05 g, 0.077 mmol, 1 equiv.) in dimethylformamide (2 mL) at 0 °C under an atmosphere of argon were added NH₄Cl (5 equiv.), HATU (2.1 equiv.), and DIPEA (5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 53.** ¹H NMR (400 MHz, DMSO-d6) δ 0.93 - 0.98 (m, 1H), 1.01 - 1.06 (m, 1H), 1.34 (s, 3H), 2.06 (s, 4H), 2.54 - 2.62 (m, 1H), 2.66 (s, 3H), 2.71 (s, 3H), 3.61 (dd, J = 2.4, 5.5 Hz, 1H), 4.43 (dd, J = 5.4, 9.2 Hz, 1H), 5.70 (d, J = 17.8 Hz, 1H), 6.06 (d, J = 17.9 Hz, 1H), 7.44 (d, J = 7.9 Hz, 1H), 7.63 (d, J = 8.0 Hz, 1H), 7.72 (s, 1H), 7.79 - 7.84 (m, 1H), 8.22 (s, 1H), 8.43 (d, J = 1.6 Hz, 1H), 9.27 (s, 2H), 10.31 (s, 1H).

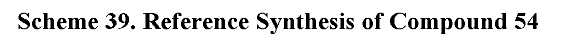

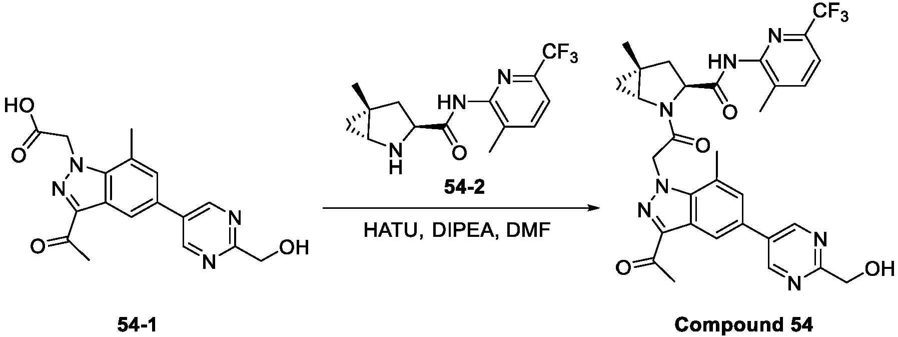

**(1R,3S,5R)-2-(2-(3-acetyl-5-(2-(hydroxymethyl)pyrimidin-5-yl)-7-methyl-1H-indazol-1-yl)acetyl)-5-methyl-N-(3-methyl-6-(trifluoromethyl)pyridin-2-yl)-2-azabicyclo[3.1.0]hexane-3-carboxamide (Compound 54):** To a solution of compound **54-1** (0.036 g, 0.105 mmol, 1 equiv.) in dimethylformamide (1 mL) at 0 °C under an atmosphere of argon were added compound **54-2** (0.031 g, 0.105 mmol, 1 equiv.), HATU (0.084 g, 0.221 mmol, 2.1 equiv.), and DIPEA (0.068 g, 0.525 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and then concentrated to dryness. The remaining residue was purified by column chromatography on silica gel (eluted with DCM/MeOH) to give **Compound 54.** ¹H NMR (400 MHz, DMSO-d6) δ 0.93 - 0.97 (m, 1H), 1.00 - 1.07 (m, 1H), 1.34 (s, 3H), 2.07 (dd, J = 5.4, 13.2 Hz, 1H), 2.19 (s, 3H), 2.54 - 2.60 (m, 1H), 2.65 (s, 3H), 2.70 (s, 3H), 3.59 - 3.67 (m, 1H), 4.48 (dd, J = 5.5, 9.2 Hz, 1H), 4.68 (d, J = 6.1 Hz, 2H), 5.34 (t, J = 6.3 Hz, 1H), 5.69 (d, J = 17.9 Hz, 1H), 6.06 (d, J = 17.8 Hz, 1H), 7.64 (s, 1H), 7.70 (d, J = 7.8 Hz, 1H), 7.95 (d, J = 7.9 Hz, 1H), 8.35 (d, J = 1.6 Hz, 1H), 9.12 (s, 2H), 10.47 (s, 1H).

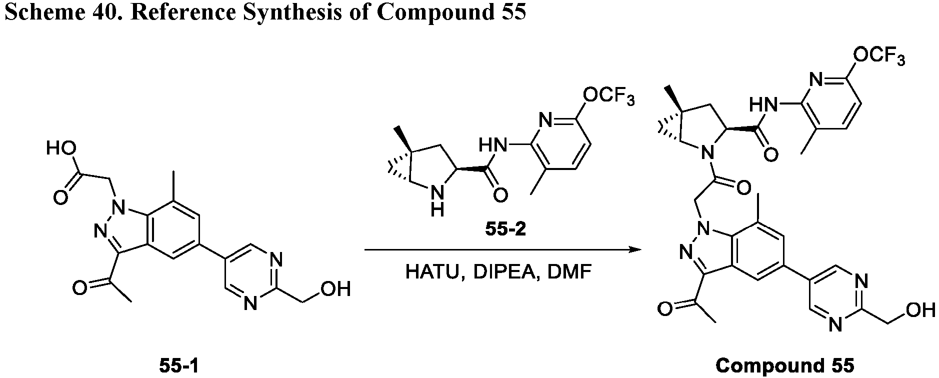

**(1R,3S,5R)-2-(2-(3-acetyl-5-(2-(hydroxymethyl)pyrimidin-5-yl)-7-methyl-1H-indazol-1-yl)acetyl)-5-methyl-N-(3-methyl-6-(trifluoromethoxy)pyridin-2-yl)-2-azabicyclo[3.1.0]hexane-3-carboxamide (Compound 55):** To a solution of compound **55-1** (0.033 g, 0.096 mmol, 1 equiv.) in dimethylformamide (1 mL) at 0 °C under an atmosphere of argon were added compound **55-2** (0.03 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 55.** ¹H NMR (400 MHz, DMSO-d6) δ 0.92 - 0.97 (m, 1H), 1.02 - 1.09 (m, 1H), 1.33 (s, 3H), 2.04 (dd, J = 5.4, 13.3 Hz, 1H), 2.11 (s, 3H), 2.53 - 2.60 (m, 1H), 2.65 (s, 3H), 2.70 (s, 3H), 3.60 - 3.66 (m, 1H), 4.47 - 4.51 (m, 1H), 4.68 (d, J = 6.3 Hz, 2H), 5.34 (t, J = 6.3 Hz, 1H), 5.68 (d, J = 17.8 Hz, 1H), 6.06 (d, J = 17.9 Hz, 1H), 7.12 (d, J = 8.1 Hz, 1H), 7.65 (s, 1H), 7.88 (d, J = 8.1 Hz, 1H), 8.35 (d, J = 1.7 Hz, 1H), 9.12 (s, 2H), 10.23 (s, 1H).

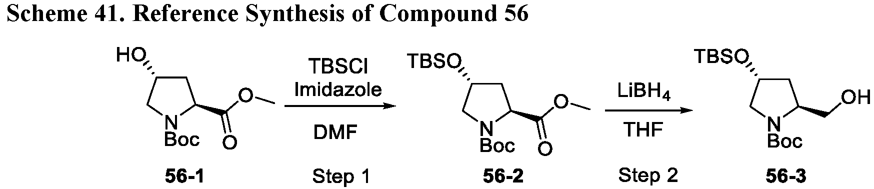

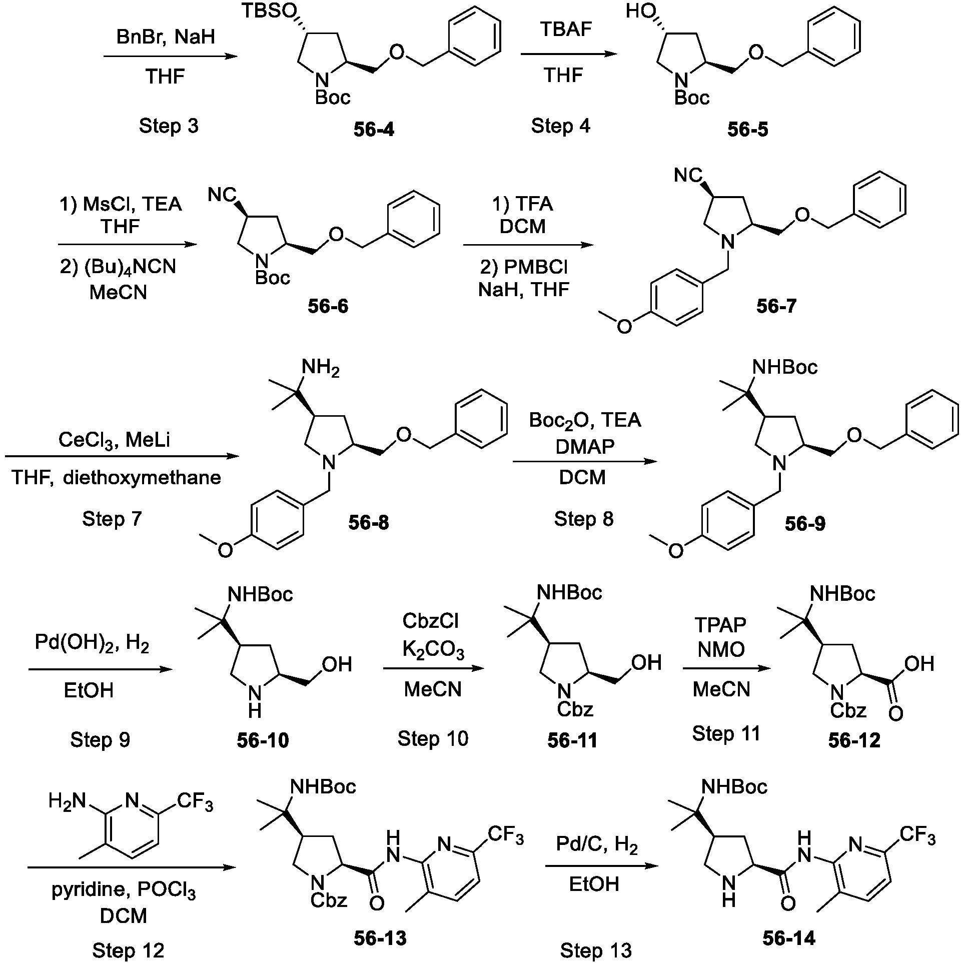

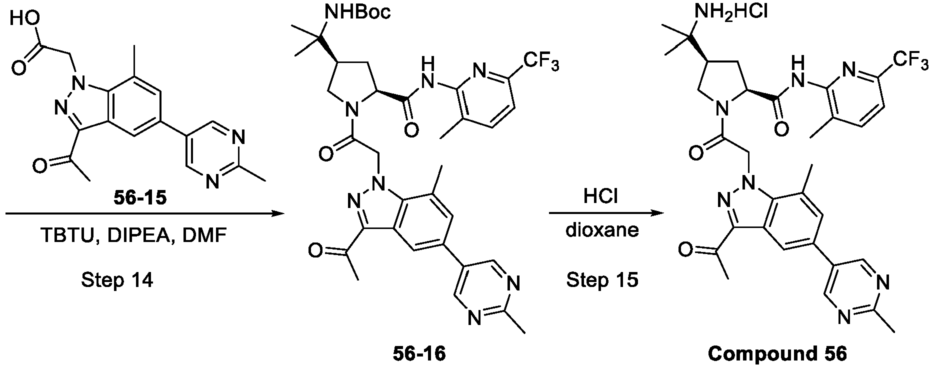

**Step 1. 1-(*tert*-Butyl) 2-methyl (2S,4R)-4-((*tert*-butyldimethylsilyl)oxy)pyrrolidine-1,2-dicarboxylate (56-2):** To a stirred solution containing compound **56-1** (2.5 g, 10.193 mmol, 1 equiv.) in DMF (25 mL) were added TBSCl (2.30 g, 15.3 mmol, 1.5 equiv.) and imidazole (1.39 g, 20.4 mmol, 2 equiv.) at rt. The reaction mixture was allowed to stir at rt for 6h, then the solvent was removed under reduced pressure, and the residue was purified by silica gel flash column chromatography (eluent: 0-15 % EtOAc in hexanes) to give compound **56-2** as colorless oil.

**Step 2. *tert-Butyl* (2S,4R)-4-((tert-butyldimethylsilyl)oxy)-2-(hydroxymethyl)pyrrolidine-1-carboxylate (56-3):** To an ice-cold solution of compound **56-2** (3.5 g, 9.74 mmol, 1 equiv.) in THF (70 mL) was added lithium borohydride (0.318 g) portion-wise. After the addition was complete, the cooling bath was removed, and the reaction mixture was stirred overnight at rt. Then the reaction mixture was cooled in an ice bath and quenched by the careful addition of cold 1N aqueous HCl. The solid was filtered off, and the filtrate was concentrated under reduced pressure. The residue was dissolved in dichloromethane, washed with saturated aqueous sodium bicarbonate, and dried (Na₂SO₄). The solvent was removed under reduced pressure to give a colorless oil, which was used in the next step without purification.

**Step 3. tert-butyl (2S,4R)-2-((benzyloxy)methyl)-4-((*tert*butyldimethylsilyl)oxy)pyrrolidine-1-carboxylate (56-4):** To an ice-cold solution of compound **56-3** (3.2 g, 9.65 mmol, 1 equiv.) in THF (30 mL) was added sodium hydride (0.464 g, 19.3 mmol, 2.00 equiv.) portion-wise. After the addition of sodium hydride was complete, the reaction mixture was stirred in an ice bath for 30 min, then benzyl bromide (2.48 g, 1.73 mL, 14.5 mmol, 1.50 equiv.) was added dropwise. The cooling bath was removed, and the reaction mixture was stirred overnight at rt. The reaction mixture was cooled and quenched by the careful addition of cold. 1N aqueous HCl, and the THF was removed under reduced pressure. The mixture was diluted with EtOAc and saturated aqueous NaHCO₃ solution. The organic layer was separated, and the aqueous layer was extracted with EtOAc. The combined organic layers were dried (Na₂SO₄) and concentrated. The residue was purified by silica gel flash chromatography (eluent: 0-0.5 % MeOH in dichloromethane) to give 2.6 g of colorless viscous liquid.

**Step 4. *tert-Butyl* (2S,4R)-2-((benzyloxy)methyl)-4-hydroxypyrrolidine-1-carboxylate (56-5):** To an ice-cold solution of compound **56-4** (2.6 g, 6.17 mmol, 1 equiv.) in THF (30 mL) was added TBAF (6.7 mL, 1.0 M in THF). The cooling bath was removed, and the reaction mixture was stirred at rt for 3 h. The solvent was removed under reduced pressure and the residue was purified by silica gel column chromatography (eluent:0-2 % MeOH in dichloromethane) to give compound **56-5** as colorless oil.

**Step 5. (3S,55)-5-((Benzyloxy)methyl)-1-(4-methoxybenzyl)pyrrolidine-3-carbonitrile (56-6):** To a solution of compound **56-5** (1.8 g, 5.86 mmol, 1 equiv.) and TEA (1.64 mL) in THF (20 mL) at -10 °C was added mesyl chloride (545 µL) dropwise and the reaction mixture was stirred at -10 °C for 2 h. Then the reaction mixture was quenched by the careful addition of saturated aqueous sodium bicarbonate, and the volatiles were removed under reduced pressure and the residue was extracted with dichloromethane. The organic layer was dried (Na₂SO₄) and concentrated to give a light-yellow oil. This crude mesylate was dissolved in acetonitrile (5 mL) and solid tetrabutylammonium cyanide (2.99 g, 11.1 mmol, 1.9 equiv.) was added. The resulting heterogeneous reaction mixture was stirred under argon at 65 ° C overnight. Then an additional 1 g of tetrabutylammonium cyanide (2.987 g, 11.126 mmol, 1.9 equiv.) was added and the reaction mixture was stirred for an additional 24 h. The reaction mixture was then cooled to rt, diluted with toluene, and washed with saturated aqueous sodium bicarbonate. The organic layer was dried (Na₂SO₄) and concentrated. The residue was purified by silica gel flash column chromatography (eluent: 0-3 % MeOH in dichloromethane) to give 1.45 g of colorless oil.

**Step 6. (3S,55)-5-((Benzyloxy)methyl)-1-(4-methoxybenzyl)pyrrolidine-3-carbonitrile (56-7):** A solution of compound **56-6** (3.4 g, 10.7 mmol, 1 equiv.) in TFA (15 mL) and dichloromethane (15 mL) was stirred at rt for 2 h. The volatiles were removed under reduced pressure, and the residue was dissolved in dichloromethane and washed with saturated aqueous sodium bicarbonate. The organic layer was washed with water, dried (Na₂SO₄), and concentrated to give 2.2 g of colorless liquid, which was used without further purification. To an ice-cold solution of crude (3S,5S)-5-[(benzyloxy)methyl]pyrrolidine-3-carbonitrile (2.2 g, 10.172 mmol, 1 equiv.) in THF (30 mL) was added NaH (0.458 g, 60 % in mineral oil) portion-wise. After the addition of NaH was complete, the reaction mixture was stirred in the ice-bath for 30 min. Then 4-methoxybenzyl chloride (2.45 g, 2.12 mL, 15.644 mmol, 1.538 equiv.) was added dropwise at 0° C, and the reaction mixture was stirred for 2 h at 0 ° C. The reaction mixture was allowed to warm to rt and stirred overnight at rt. Another portion of 4-methoxybenzyl chloride (1 mL) was added and the reaction mixture was allowed to stir at rt for 24h. The reaction mixture was quenched by the careful addition of water, and THF was removed under reduced pressure. The remaining aqueous layer was extracted with dichloromethane, and the combined extracts were dried and concentrated. The residue was purified by silica gel flash column chromatography (eluent: 0-35 % EtOAc in hexanes, then 0-5 % MeOH in dichloromethane) to give 1.95 g of the desired product as cream colored solid.

**Step 7. 2-((3S,55)-5-((Benzyloxy)methyl)-1-(4-methoxybenzyl)pyrrolidin-3-yl)propan-2-amine (56-8):** Anhydrous cerium chloride (5.5 g) was heated at 150 ° C for 1 h under high vacuum in a 3-necked flask, and the flask was kept under vacuum overnight. The vacuum was disconnected, and the flask was carefully filled with argon then charged with anhydrous THF (30 mL) and cooled to -78 °C. MeLi (4.46 mL, 3.1 M in diethoxymethane) was added dropwise at this temp. After the addition of MeLi was complete, the bright yellow mixture was stirred at -25 to -20 ° C for 30 min. Compound 56-7 (1 g, 2.97 mmol, 1 equiv.) in THF (15 mL) was added dropwise, and the heterogeneous mixture was stirred for 3 h at temperatures between -20 to -15 °C. The reaction mixture was quenched by the careful addition of 2 mL of strong ammonia solution at -15 ° C, and the orange color disappeared with attendant formation of a white solid. The solids were filtered off and the cake was washed thoroughly with THF. The filtrate was then concentrated under reduced pressure.

One more batch of 1g of compound **56-7** (1 g, 2.97 mmol, 1 equiv.) was treated in similar way. The combined crude products were purified by silica gel flash column chromatography (eluent: 0-15 % EtOAc in hexanes and then 0-20 % MeOH in CH₂Cl₂) to give 1.56 g of orange resin. ¹H NMR (400 MHz, Chloroform-**d**) δ 1.23 (d, **J** = 2 Hz, 6 H), 1.67-1.70 (m, 1H), 2.04-2.09 (m, 1H), 2.38 (t, **J** = 8.9 Hz, 1H), 2.79 (s, 2H), 2.90 - 2.98 (m, 2H), 3.35 (d, **J** = 13.2 Hz, 1H), 3.53 (dd, **J** = 9.6, 4 Hz, 2H), 3.61 (dd, **J** = 4.6, 9.8 Hz, 1H), 3.80 (s, 3H), 3.99 (d, **J** = 13.2 Hz, 1H), 4.54 (dd, **J** = 18.4, 12 Hz, 2H), 6.83 (d, **J** = 8.4 Hz, 2H), 7.18 (d, **J** = 8.4 Hz, 2H), 7.24 - 7.39 (m, 5H).

**Step 8. *tert-Butyl* (2-((3S,55)-5-((benzyloxy)methyl)-1-(4-methoxybenzyl)pyrrolidin-3-yl)propan-2-yl)carbamate (56-9):** To a solution of compound **56-8** (1.37 g, 3.71 mmol, 1 equiv.) in dichloromethane (20 mL), cooled with an ice-bath, were added TEA (1.56 mL, 3 equiv.), DMAP (0.005 g) and Boc₂O (1.62 g, 7.42 mmol, 2 equiv.) successively. The cooling bath was removed, and the reaction mixture was stirred at rt for 18 h. Then 0.8 g of Boc₂O was added and the reaction mixture was stirred for 15 h at rt. The solvent was removed under reduced pressure and the residue was purified by silica gel column chromatography (eluent: 0-2 % MeOH in dichloromethane) to give **56-9** as a light yellow oil.

**Step 9. *tert-Butyl* (2-((3S,5S)-5-(hydroxymethyl)pyrrolidin-3-yl)propan-2-yl)carbamate (56-10):** To a solution of compound **56-9** (0.2 g, 0.427 mmol, 1 equiv.) in EtOH (10 mL) was added Pd(OH)₂ (0.2 g) and the mixture was stirred at 45° C under hydrogen overnight. The reaction mixture was filtered through a pad of Celite, and the washing the celite thoroughly with MeOH. The filtrate was concentrated to get 0.1 g of colorless resin. Used as such for the next step.

**Step 10. Benzyl (2S,4S)-4-(2-((*tert*-butoxycarbonyl)amino)propan-2-yl)-2-(hydroxymethyl)pyrrolidine-1-carboxylate (56-11):** To a solution of compound **56-10** (0.1 g, 0.387 mmol, 1 equiv.) in acetonitrile (4 mL) at -20° C were added potassium carbonate (0.13 g) and Cbz-Cl (61 µL). The reaction mixture was stirred at that temperature for 1 h and then at 0 ° C for 1 h. The reaction mixture was quenched by the addition of saturated aqueous sodium bicarbonate solution. Most of the organic solvent was removed and the aqueous layer was extracted with dichloromethane. The organic layer was separated, dried (Na₂SO₄), concentrated, and purified by silica gel flash column chromatography (eluent: 0-30 % EtOAc in hexanes) to get 0.14 g of cream colored sticky solid, which was contaminated with di- protected compound.

**Step 11. (2S,4S)-1-((Benzyloxy)carbonyl)-4-(2-((tert-butoxycarbonyl)amino)propan-2-yl)pyrrolidine-2-carboxylic acid (56-12):** To a solution of compound **56-11** (0.14 g, 0.357 mmol, 1 equiv.) in acetonitrile (2 mL) were added N-methylmorpholine-N-oxide hydrate (0.483 g, 3.57 mmol, 10 equiv.) and TPAP (0.013 g, 0.037 mmol, 0.10 equiv.). The reaction mixture was stirred at rt for 1.5 h, and the solvent was removed under reduced pressure. The residue was made basic by addition of 5% aqueous LiOH and extracted with dichloromethane. The aqueous layer was separated and the combined dichloromethane extracts were again extracted once with water. The organic layer was discarded. The combined aqueous layers were cooled in an ice bath and acidified with cold 1N aqueous HCl, and this acidified aqueous layer was extracted repeatedly with dichloromethane. The combined organic layer was dried and concentrated to give **56-12** as a light brown solid, which was used in the next step without further purification.

**Step** 12. **Benzyl (2S,4S)-4-(2-((*tert*-butoxycarbonyl)amino)propan-2-yl)-2-((3-methyl-6-(trifluoromethyl)pyridin-2-yl)carbamoyl)pyrrolidine-1-carboxylate (56-13):** To an ice-cold solution of compound **56-12** (0.04 g, 0.098 mmol, 10 equiv.) and 3-methyl-6-(trifluoromethyl)pyridin-2-amine (0.018 g, 0.102 mmol, 10.4 equiv.) in dichloromethane (2 mL) were added pyridine (0.04 mL) and phosphorus oxychloride (0.01 mL) successively. The reaction mixture was stirred at 0 °C for 1.5 h and quenched by the careful addition of saturated aqueous sodium bicarbonate solution. The reaction mixture was stirred at rt for 30 min and the organic and aqueous layers were separated. The aqueous layer was extracted with dichloromethane, and the combined organic layers were dried (Na₂SO₄), concentrated, and purified by silica gel flash column chromatography (eluent:0-2 % MeOH in dichloromethane) to give compound 56-13 as a white solid.

**Step 13: *tert*-Butyl (2-((3S,5S)-5-((3-methyl-6-(trifluoromethyl)pyridin-2-yl)carbamoyl)pyrrolidin-3-yl)propan-2-yl)carbamate (56-14):** A heterogeneous mixture of compound **56-13** (0.02 g, 0.035 mmol, 1 equiv.) and Pd/C (10 % Pd, 2 mg) in EtOH (1.5 mL) was stirred at rt under hydrogen atmosphere for 2h. The reaction mixture was filtered through a pad of Celite, and the Celite was washed thoroughly with MeOH. The filtrate was concentrated to give 18 mg of brown solid, which was used without further purification in the following step.

**Step 14: *tert*-Butyl (2-((3S,5S)-1-(2-(3-acetyl-7-methyl-5-(2-methylpyrimidin-5-yl)-1*H*-indazol-1-yl)acetyl)-5-((3-methyl-6-(trifluoromethyl)pyridin-2-yl)carbamoyl)pyrrolidin-3-yl)propan-2-yl)carbamate (56-16):** To an ice-cold solution of compound **56-14** (0.018 g, 0.042 mmol, 1 equiv.) and compound **56-15** (0.014 g, 0.045 mmol, 1.05 equiv.) in DMF (1 mL) were added DIPEA (0.033 mL, 0.042 mmol, 1 equiv.) and TBTU (0.015 g, 0.047 mmol, 1.1 equiv.). The cooling bath was removed and the reaction mixture was stirred at rt for 30 min. The solvent was removed under reduced pressure and the residue was dissolved in chloroform and washed with saturated aqueous sodium bicarbonate solution. The phases were separated, and the organic layer was dried and concentrated. The resultant residue was purified by silica gel flash column chromatography (eluent: 0-3.5 % MeOH in dichloromethane) to get 12 mg of cream colored solid.

**Step 15: (2S,4S)-1-(2-(3-acetyl-7-methyl-5-(2-methylpyrimidin-5-yl)-1H-indazol-1-yl)acetyl)-4-(2-aminopropan-2-yl)-N-(3-methyl-6-(trifluoromethyl)pyridin-2-yl)pyrrolidine-2-carboxamide hydrochloride (Compound 56):** A solution of 4 M hydrogen chloride in 1,4-dioxane containing compound **56-16** (0.012 g, 0.016 mmol, 1 equiv.) was stirred at rt for 30 min. Precipitation of some solids was noted. The volatiles were removed under reduced pressure, and the residue was washed with a hexane-ether (1:1) mixture and dried to give **Compound 56** as a light yellow solid. ¹H NMR (400 MHz, Methanol-*d*₄) δ 1.51 (s, 3H), 1.53 (s, 3H), 1.77 (s, 0H), 1.98 - 2.12 (m, 1H), 2.16 - 2.27 (m, 1H), 2.26 (s, 3H), 2.62-2.69 (m, 1H), 2.70 (s, 3H), 2.76 (s, 3H), 2.87 (s, 3H), 3.57 - 3.80 (m, 1H), 4.18-4.23 (m, 1H), 4.70-4.77 (m,1H), 5.77 (d, J = 16 Hz, 1H), 5.84 (d, J = 16 Hz, 1H), 7.47 - 7.75 (m, 2H), 7.86 (d, *J* = 8.0 Hz, 1H), 8.52 (s, 1H), 9.27 (s, 2H). ¹⁹F (376 MHz): δ -69.1.

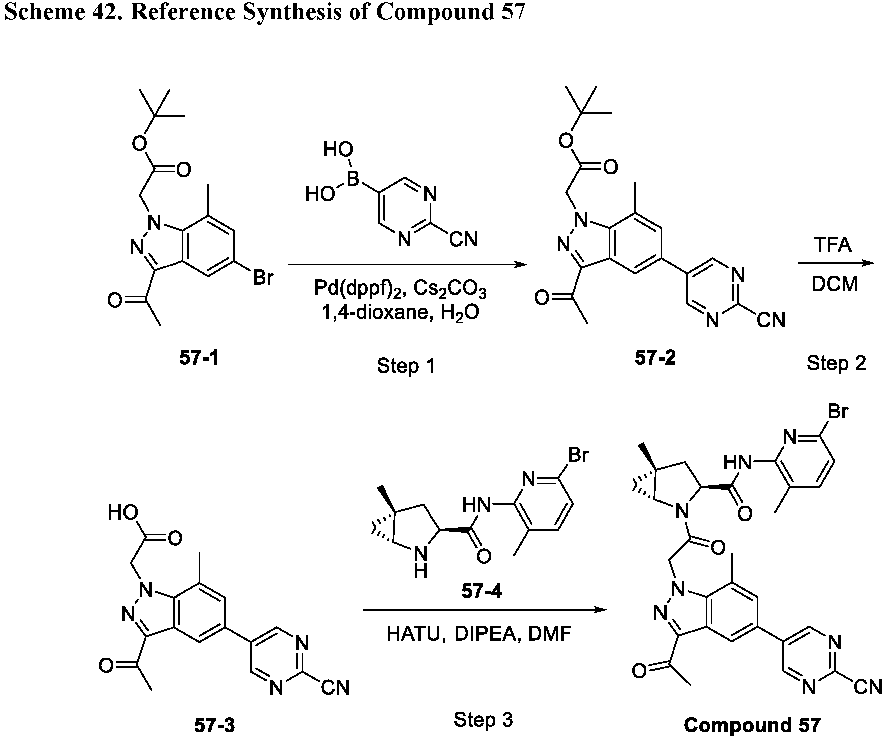

**Step 1: tert-butyl 2-(3-acetyl-5-(2-cyanopyrimidin-5-yl)-7-methyl-1H-indazol-1-yl)acetate (57-2):** To a solution of compound **57-1** (0.06 g, 0.163 mmol, 1 equiv.) in 1,4-dioxane (1 mL) and water (0.1 mL) were added compound 2-cyanopyrimidin-5-ylboronic acid (0.024 g, 0.163 mmol, 1 equiv.), cesium carbonate (0.106 g, 0.326 mmol, 2 equiv.) and [1,1'-bis(diphenylphosphino)ferrocene]dichloropalladium(II) (0.133 g, 0.163 mmol, 1 equiv.). The reaction mixture was stirred at 90 °C for 5 h and then concentrated to dryness. The residue was purified by column chromatography on silica gel (eluted with DCM/ MeOH) to give compound **57-2.**

**Step 2: 2-(3-acetyl-5-(2-cyanopyrimidin-5-yl)-7-methyl-1H-indazol-1-yl)acetic acid (57-3):** To a solution of compound **57-2** (0.064 g, 0.163 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The crude material was used directly in the next synthetic step without purification.

**Step 3: (1R,3S,5R)-2-(2-(3-acetyl-5-(2-cyanopyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 57):** To a solution of compound **57-4** (0.055 g, 0.163 mmol, 1 equiv.) in dimethylformamide (1 mL) at 0 °C under an atmosphere of argon were added compound 57-3 2-(3-acetyl-5-(2-cyanopyrimidin-5-yl)-7-methyl-1H-indazol-1-yl)acetic acid (0.051 g, 0.163 mmol, 1 equiv.), HATU (0.130 g, 0.342 mmol, 2.1 equiv.), and DIPEA (0.105 g, 0.11 mL, 0.815 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 57.** ¹H NMR (400 MHz, DMSO-d6) δ 0.92 - 0.97 (m, 1H), 1.03 (t, J = 5.4 Hz, 1H), 1.33 (s, 3H), 2.00 - 2.08 (m, 4H), 2.54 - 2.60 (m, 1H), 2.66 (s, 3H), 2.70 (s, 3H), 3.56 - 3.70 (m, 1H), 4.42 (dd, J = 5.4, 9.2 Hz, 1H), 5.71 (d, J = 17.8 Hz, 1H), 6.06 (d, J = 17.9 Hz, 1H), 7.44 (d, J = 7.9 Hz, 1H), 7.63 (d, J = 7.9 Hz, 1H), 7.75 (s, 1H), 8.49 (d, J = 1.7 Hz, 1H), 9.40 (s, 2H), 10.30 (s, 1H).

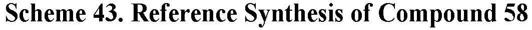

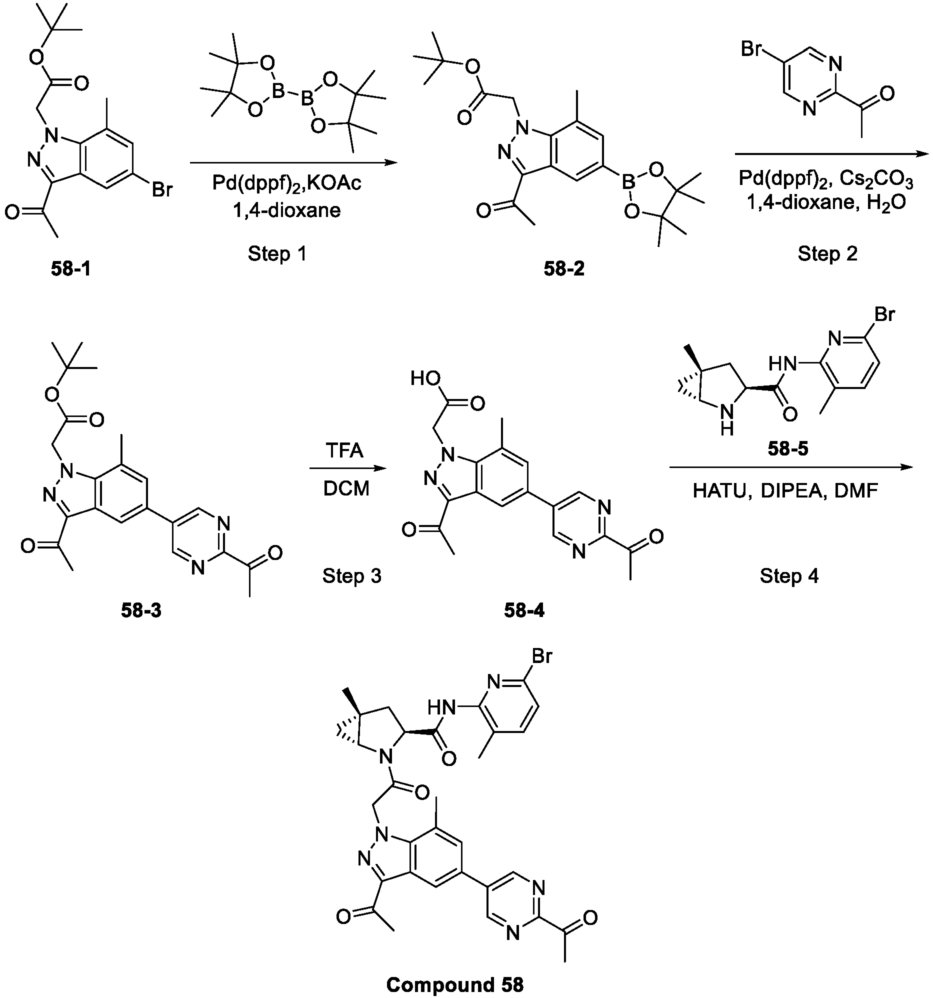

**Step 1: tert-butyl 2-(3-acetyl-7-methyl-5-(4,4,5,5-tetramethyl-1,3,2-dioxaborolan-2-yl)-1H-indazol-1-yl)acetate (58-2):** To a solution of compound **58-1** (0.085 g, 0.231 mmol, 1 equiv.) in dioxane (1 mL) under an atmosphere of argon were added bis(pinacolato)diboron (0.059 g, 0.231 mmol, 1 equiv.), KOAc (0.045 g, 0.463 mmol, 2 equiv.) and [1,1'-bis(diphenylphosphino)ferrocene]dichloropalladium(II) (0.009 g, 0.012 mmol, 0.05 equiv.). The reaction mixture was stirred at 90 °C for 4 h and was used in the next synthetic step without purification.

**Step 2: tert-butyl 2-(3-acetyl-5-(2-acetylpyrimidin-5-yl)-7-methyl-1H-indazol-1-yl)acetate (58-3):** To a solution of compound **58-2** (0.096 g, 0.231 mmol, 1 equiv.) in 1,4-dioxane (1 mL) and water (0.1 mL) were added compound 1-(5-bromopyrimidin-2-yl)ethanone (0.046 g, 0.231 mmol, 1 equiv.), cesium carbonate (0.151 g, 0.462 mmol, 2 equiv.), and [1,1'-Bis(diphenylphosphino)ferrocene]dichloropalladium(II) (0.189 g, 0.231 mmol, 1 equiv.). The reaction mixture was stirred at 90 °C for 5 h and then concentrated to dryness. The remaining residue was purified by column chromatography on silica gel (eluted with DCM/ MeOH) to give compound **58-3.**

**Step 3: 2-(3-acetyl-5-(2-acetylpyrimidin-5-yl)-7-methyl-1H-indazol-1-yl)acetic acid (58-4):** To a solution of compound **58-3** (0.094 g, 0.231 mmol, 1 equiv.) in dichloromethane (1 mL) at 0 °C under an atmosphere of argon was added trifluoroacetic acid (0.5 mL). The reaction mixture was stirred at rt for 3 h and then concentrated to dryness. The crude material was used directly in the next synthetic step.

**Step 4: ((1R,3S,5R)-2-(2-(3-acetyl-5-(2-acetylpyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 58):** To a solution of compound **58-4** (0.081 g, 0.231 mmol, 1 equiv.) in dimethylformamide (1 mL) at 0 °C under an atmosphere of argon were added compound **58-5** (0.072 g, 0.231 mmol, 1 equiv.), HATU (0.184 g, 0.485 mmol, 2.1 equiv.), and DIPEA (0.149 g, 0.201 mL, 1.155 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 58.** ¹H NMR (400 MHz, DMSO-d6) δ 0.92 - 0.99 (m, 1H), 1.03 (t, J = 5.4 Hz, 1H), 1.34 (s, 3H), 1.99 - 2.09 (m, 4H), 2.54 - 2.60 (m, 1H), 2.66 (s, 3H), 2.71 (s, 3H), 2.73 (s, 3H), 3.55 - 3.67 (m, 1H), 4.43 (dd, J = 5.4, 9.2 Hz, 1H), 5.70 (d, J = 17.8 Hz, 1H), 6.06 (d, J = 17.9 Hz, 1H), 7.44 (d, J = 7.9 Hz, 1H), 7.63 (d, J = 7.9 Hz, 1H), 7.73 (s, 1H), 8.41 - 8.57 (m, 1H), 9.36 (s, 2H), 10.31 (s, 1H).

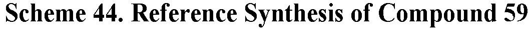

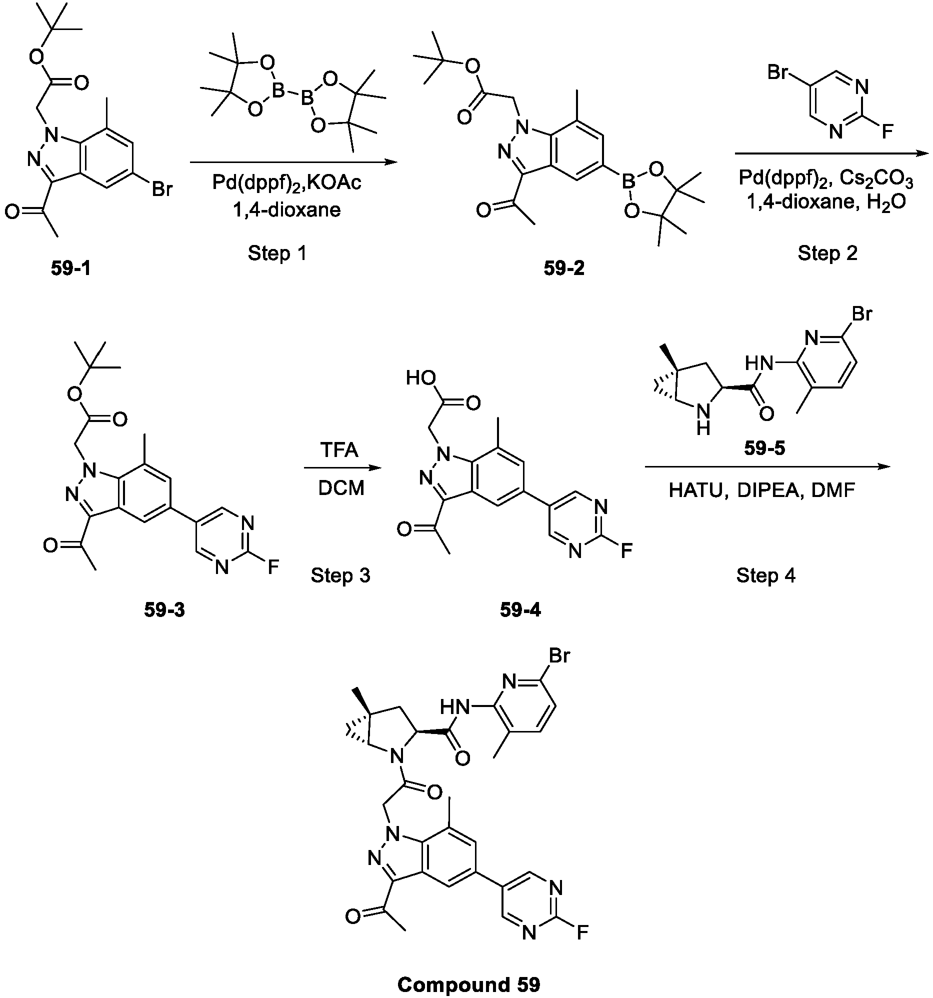

**Step 1: tert-butyl 2-(3-acetyl-7-methyl-5-(4,4,5,5-tetramethyl-1,3,2-dioxaborolan-2-yl)-1H-indazol-1-yl)acetate (59-2):** To a solution of compound **59-1** (0.085 g, 0.231 mmol, 1 equiv.) in 1,4-dioxane (1 mL) under an atmosphere of argon were added bis(pinacolato)diboron (0.059 g, 0.231 mmol, 1 equiv.), KOAc (0.045 g, 0.463 mmol, 2 equiv.) and [1,1'-bis(diphenylphosphino)ferrocene]dichloropalladium(II) (0.009 g, 0.012 mmol, 0.05 equiv.).The reaction mixture was stirred at 90 °C for 4 h and was used in the next synthetic step without purification.

**Step 2: tert-butyl 2-(3-acetyl-5-(2-fluoropyrimidin-5-yl)-7-methyl-1H-indazol-1-yl)acetate (59-3):** To a solution of compound **59-2** (0.096 g, 0.231 mmol, 1 equiv.) in 1,4-dioxane (1 mL) and water (0.1 mL) were added 5-bromo-2-fluoropyrimidine (0.041 g, 0.231 mmol, 1 equiv.), cesium carbonate (0.151 g, 0.462 mmol, 2 equiv.), and [1,1'-bis(diphenylphosphino)ferrocene]dichloropalladium(II) (0.189 g, 0.231 mmol, 1 equiv.). The reaction mixture was stirred at 90 °C for 5 h and then concentrated to dryness. The residue was purified by column chromatography on silica gel (eluted with DCM/ MeOH) to give compound **59-3.**

**Step 3: 2-(3-acetyl-5-(2-fluoropyrimidin-5-yl)-7-methyl-1H-indazol-1-yl)acetic acid (59-4):** To a solution of compound **59-3** (0.089 g, 0.231 mmol, 1 equiv.) in dichloromethane (1 mL) at 0 °C under an atmosphere of argon was added trifluoroacetic acid (0.5 mL). The reaction mixture was stirred at rt for 3 h and then concentrated to dryness. The material was used directly in the next synthetic step without purification.

**Step 4: (1R,3S,5R)-2-(2-(3-acetyl-5-(2-fluoropyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 59):** To a solution of compound **59-4** (0.076 g, 0.231 mmol, 1 equiv.) in dimethylformamide (1 mL) at 0 °C under an atmosphere of argon were added compound **59-5** (0.072 g, 0.231 mmol, 1 equiv.), HATU (0.184 g, 0.485 mmol, 2.1 equiv.), and DIPEA (0.149 g, 0.201 mL, 1.155 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 59.** ¹H NMR (400 MHz, DMSO-d6) δ 0.92 - 0.98 (m, 1H), 1.03 (t, J = 5.4 Hz, 1H), 1.33 (s, 3H), 2.01 - 2.09 (m, 4H), 2.54 - 2.61 (m, 1H), 2.65 (s, 3H), 2.69 (s, 3H), 3.60 (dd, J = 2.4, 5.6 Hz, 1H), 4.42 - 4.50 (m, 1H), 5.70 (d, J = 17.8 Hz, 1H), 6.05 (d, J = 17.9 Hz, 1H), 7.44 (d, J = 7.9 Hz, 1H), 7.57 - 7.67 (m, 2H), 8.36 (d, J = 1.6 Hz, 1H), 9.13 (d, J = 1.4 Hz, 2H), 10.30 (s, 1H).

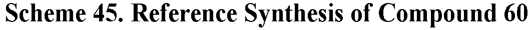

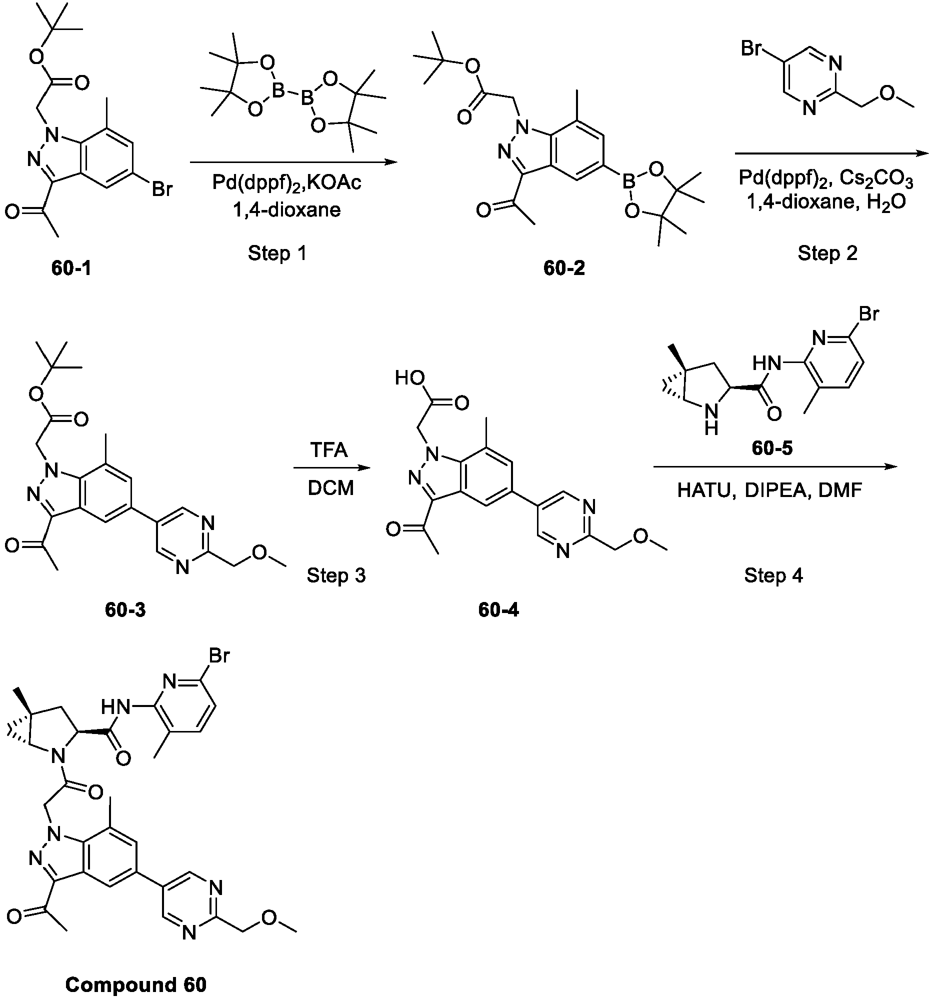

**Step 1: tert-butyl 2-(3-acetyl-7-methyl-5-(4,4,5,5-tetramethyl-1,3,2-dioxaborolan-2-yl)-1H-indazol-1-yl)acetate (60-2):** To a solution of compound **60-1** (0.085 g, 0.231 mmol, 1 equiv.) in 1,4-dioxane (1 mL) under an atmosphere of argon were added bis(pinacolato)diboron (0.059 g, 0.231 mmol, 1 equiv.), KOAc (0.045 g, 0.363 mmol, 2 equiv.), and [1,1'-bis(diphenylphosphino)ferrocene]dichloropalladium(II), (0.05 g, 0.012 mmol, 0.05 equiv.). The reaction mixture was stirred at 90°C for 4 h and was directly used in the next synthetic step without purification.

**Step 2: tert-butyl 2-(3-acetyl-5-(2-(methoxymethyl)pyrimidin-5-yl)-7-methyl-1H-indazol-1-yl)acetate (60-3):** To a solution of compound **60-2** (0.096 g, 0.231 mmol, 1 equiv.) in 1,4-dioxane (1 mL) and water (0.1 mL) were added 5-bromo-2-(methoxymethyl)pyrimidine (0.047 g, 0.231 mmol, 1 equiv.), cesium carbonate (0.151 g, 0.462 mmol, 2 equiv.) and [1,1'-bis(diphenylphosphino)ferrocene]dichloropalladium(II), (0.189 g, 0.231 mmol, 1 equiv.). The reaction mixture was stirred at 90 °C for 5 h and then concentrated to dryness. The residue was purified by column chromatography on silica gel (eluted with DCM/ MeOH) to give compound **60-3.**

**Step 3: 2-(3-acetyl-5-(2-(methoxymethyl)pyrimidin-5-yl)-7-methyl-1H-indazol-1-yl)acetic acid (60-4):** To a solution of compound **60-3** (0.095 g, 0.231 mmol, 1 equiv.) in dichloromethane (1 mL) at 0 °C under an atmosphere of argon was added trifluoroacetic acid (0.5 mL). The reaction mixture was stirred at rt for 3 h and then concentrated to dryness. The crude material was used directly in the next synthetic step without purification.

**Step 4: (1R,3S,5R)-2-(2-(3-acetyl-5-(2-(methoxymethyl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 60):** To a solution of compound **60-4** (0.082 g, 0.231 mmol, 1 equiv.) in dimethyl-formamide (1 mL) at 0 °C under an atmosphere of argon were added compound **60-**5 (0.072 g, 0.231 mmol, 1 equiv.), HATU (0.184 g, 0.485 mmol, 2.1 equiv.), and DIPEA (0.149 g, 0.201 mL, 1.155 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 60.** ¹H NMR (400 MHz, DMSO-d6) δ 0.96 (d, J = 2.9 Hz, 1H), 1.03 (t, J = 5.4 Hz, 1H), 1.33 (s, 3H), 2.05 (m, 4H), 2.50 (s, 3H), 2.57 (dd, J = 9.2, 13.3 Hz, 1H), 2.65 (s, 3H), 2.70 (s, 3H), 3.60 (d, J = 4.0 Hz, 1H), 4.43 (dd, J = 5.3, 9.1 Hz, 1H), 4.65 (s, 2H), 5.62 - 5.75 (m, 1H), 6.05 (d, J = 17.9 Hz, 1H), 7.44 (d, J = 8.0 Hz, 1H), 7.60 - 7.68 (m, 2H), 8.36 (d, J = 1.7 Hz, 1H), 9.14 (d, J = 2.9 Hz, 2H), 10.30 (s, 1H).

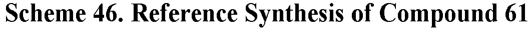

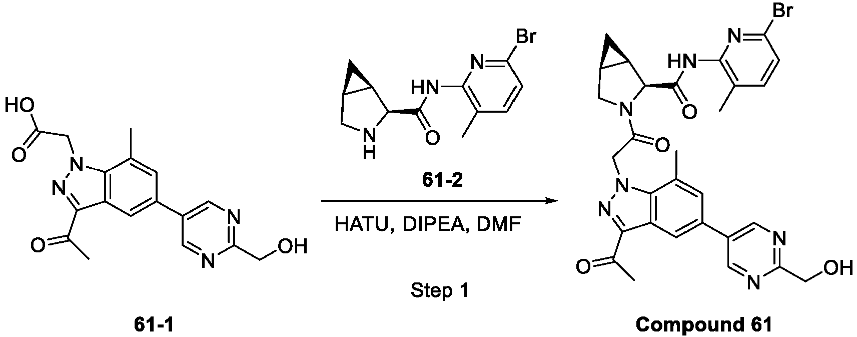

**Step 1: (1R,2S,5S)-3-(2-(3-acetyl-5-(2-(hydroxymethyl)pyrimidin-5-yl)-7-methyl-1H-indazol-1-yl)acetyl)-N-(6-bromo-3-methylpyridin-2-yl)-3-azabicyclo[3.1.0]hexane-2-carboxamide (Compound 61):** To a solution of compound 61-1 (0.03 g, 0.088 mmol, 1 equiv.) in dimethylformamide (1 mL) at 0 °C under an atmosphere of argon were added compound **61-2** (0.026 g, 0.088 mmol, 1 equiv.), HATU (0.07 g, 0.185 mmol, 2.1 equiv.), and DIPEA (0.057 g, 0.404 mmol, 5 equiv.). The reaction mixture was stirred at room temperature for 1 h then quenched with water (10 vol). The resulting mixture was extracted with DCM, and the organic layer was washed with brine, dried over anhydrous Na₂SO₄, filtered, and then concentrated to dryness. The remaining residue was purified by column chromatography on silica gel (eluted with DCM/MeOH) to give **Compound 61.** ¹H NMR(400 MHz, DMSO-d6) δ 0.74 - 0.86 (m, 2H), 1.23 - 1.29 (m, 1H), 2.04 (s, 3H), 2.64 (s, 3H), 2.68 (s, 3H), 3.88 (d, J = 9.8 Hz, 1H), 4.03 (dd, J = 5.4, 9.9 Hz, 1H), 4.66 (dd, J = 5.9, 16.0 Hz, 3H), 5.33 (t, J = 6.3 Hz, 1H), 5.59 - 5.84 (m, 2H), 7.41 (d, J = 7.9 Hz, 1H), 7.59 (d, J = 8.3 Hz, 2H), 8.33 (d, J = 1.6 Hz, 1H), 9.10 (s, 2H), 10.36 (s, 1H).

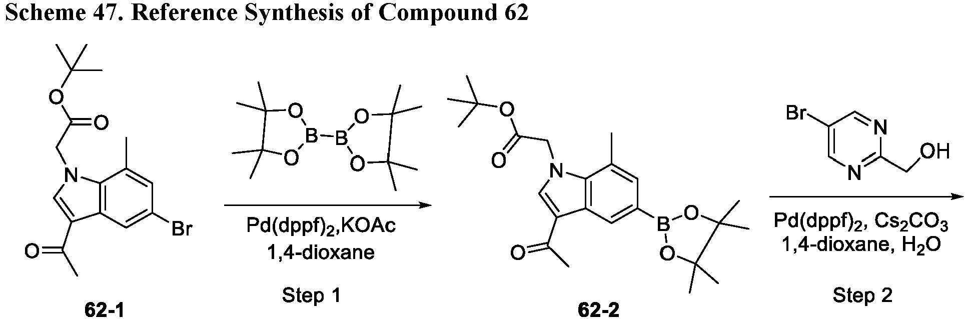

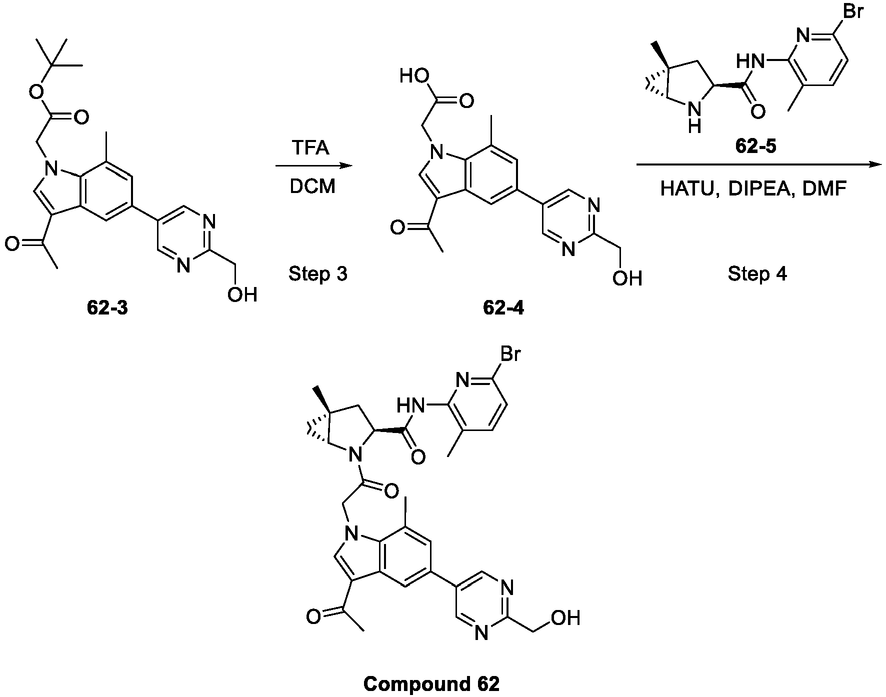

**Step 1: tert-butyl 2-(3-acetyl-7-methyl-5-(4,4,5,5-tetramethyl-1,3,2-dioxaborolan-2-yl)-1H-indol-1-yl)acetate (62-2):** To a solution of compound **62-1** (0.308g, 0.833 mmol, 1 equiv.) in 1,4-dioxane (5 mL) under an atmosphere of argon were added bis(pinacolato)diboron (0.211 g, 833 mmol, 1 equiv.) KOAc (0.163 g, 1.66 mmol, 2 equiv.) and [1,1'-bis(diphenylphosphino)ferrocene]dichloropalladium(II) (0.034 g, 0.042 mmol, 0.05 equiv.). The reaction mixture was stirred at 90 °C for 4 h and the reaction mixture was directly used in the next synthetic step without further purification.

**Step 2: tert-butyl 2-(3-acetyl-5-(2-(hydroxymethyl)pyrimidin-5-yl)-7-methyl-1H-indol-1-yl)acetate (62-3):** To a solution of compound **62-2** (0.344 g, 0.833 mmol, 1 equiv.) in 1,4-dioxane (5 mL) and water (0.5 mL) were added (5-bromopyrimidin-2-yl)methanol (0.157 g, 0.833 mmol, 1 equiv.), cesium carbonate (0.453 g, 1.66 mmol, 3 equiv.) and [1,1'-bis(diphenylphosphino)ferrocene]dichloropalladium(II) (0.034 g, 0.042 mmol, 0.05 equiv.). The reaction mixture was stirred at 90 °C for 5 h and then concentrated to dryness, and the residue was purified by column chromatography on silica gel (eluted with DCM/ MeOH) to give compound **62-3.**

**Step 3: 2-(3-acetyl-5-(2-(hydroxymethyl)pyrimidin-5-yl)-7-methyl-1H-indol-1-yl)acetic acid (62-4):** To a solution of compound **62-3** (0.091 g, 0.231 mmol, 1 equiv.) in dichloromethane (1 mL) at 0 °C under an atmosphere of argon was added trifluoroacetic acid (0.5 mL). The reaction mixture was stirred at rt for 3 h and then concentrated to dryness. The material was used directly in the next synthetic step.

**Step 4: (1R,3S,5R)-2-(2-(3-acetyl-5-(2-(hydroxymethyl)pyrimidin-5-yl)-7-methyl-1H-indol-1-yl)acetyl)-N-(6-bromo-3-methylpyridin-2-yl)-5-methyl-2-azabicyclo[3.1.0]hexane-3-carboxamide (Compound 62):** To a solution of compound **62-4** (0.055 g, 0.177 mmol, 1 equiv.) in dimethylformamide (1 mL) at 0 °C under an atmosphere of argon were added compound **62-5** (0.06 g, 0.177 mmol, 1 equiv.), HATU (0.141 g, 0.371 mmol, 2.1 equiv.), and DIPEA (0.144 g, 0.884 mmol, 5 equiv.). The reaction mixture was stirred at room temperature for 1 h and quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 62.** ¹H NMR (400 MHz, DMSO-d6) δ 0.87 - 0.94 (m, 1H), 0.96 - 1.05 (m, 1H), 1.33 (s, 3H), 2.05 (s, 4H), 2.46 (s, 3H), 2.53 - 2.58 (m, 1H), 2.65 (s, 3H), 3.49 - 3.62 (m, 1H), 4.36 - 4.46 (m, 1H), 4.67 (d, J = 6.3 Hz, 2H), 5.31 (t, J = 6.3 Hz, 1H), 5.42 (d, J = 17.9 Hz, 1H), 5.74 (d, J = 17.9 Hz, 1H), 7.37 (s, 1H), 7.43 (d, J = 7.9 Hz, 1H), 7.62 (d, J = 8.0 Hz, 1H), 8.32 (s, 1H), 8.39 (d, J = 1.8 Hz, 1H), 9.05 (s, 2H), 10.29 (s, 1H).

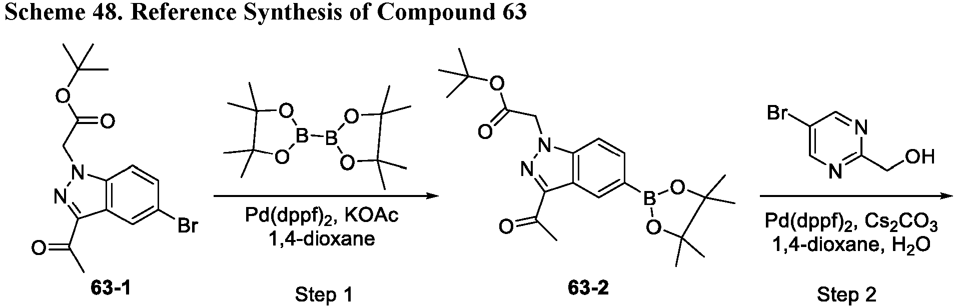

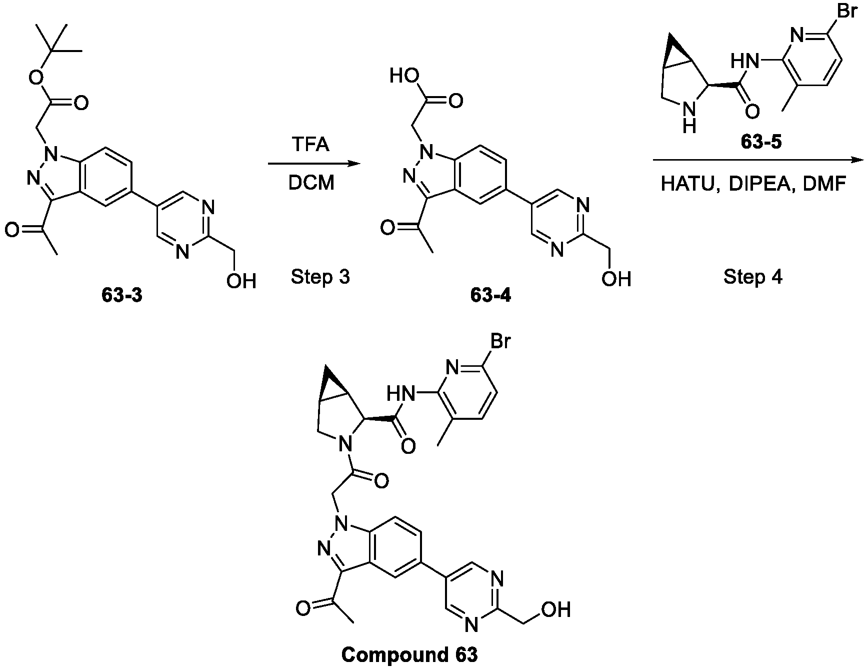

**Step 1: tert-butyl 2-(3-acetyl-5-(4,4,5,5-tetramethyl-1,3,2-dioxaborolan-2-yl)-1H-indazol-1-yl)acetate (63-2):** To a solution of compound **63-1** (2 g, 5.66 mmol, 1 equiv.) in 1,4-dioxane (20 mL) under an atmosphere of argon were added bis(pinacolato)diboron (1.44 g, 5.66 mmol, 1 equiv.), potassium acetate (1.11 g, 11.3 mmol, 2 equiv.) and [1,1'-bis(diphenylphosphino)ferrocene]dichloropalladium(II) (0.231 g, 0.283 mmol, 0.05 equiv.).The reaction mixture was stirred at 90 °C for 4 h and used in the next synthetic step without purification.

**Step 2: tert-butyl 2-(3-acetyl-5-(2-(hydroxymethyl)pyrimidin-5-yl)-1H-indazol-1-yl)acetate (63-3):** To a solution of **63-2** (2.266 g, 5.66 mmol, 1 equiv.) in 1,4-dioxane (20 mL) and water (1 mL) were added compound (5-bromopyrimidin-2-yl)methanol (1.07 g, 5.66 mmol, 1 equiv.), cesium carbonate (2 equiv.), and [1,1'-bis(diphenylphosphino)ferrocene]dichloropalladium(II) (0.231 g, 0.283 mmol, 0.05 equiv.). The reaction mixture was stirred at 90 °C for 5 h and concentrated to dryness, and the residue was purified by column chromatography on silica gel (eluted with DCM/ MeOH) to give compound **63-3.**

**Step 3: 2-(3-acetyl-5-(2-(hydroxymethyl)pyrimidin-5-yl)-1H-indazol-1-yl)acetic acid (63-4):** To a solution of compound **63-3** (0.05 g, 0.131 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The material was used directly in the next synthetic step without purification.

**Step 4: (1R,2S,5S)-3-(2-(3-acetyl-5-(2-(hydroxymethyl)pyrimidin-5-yl)-1H-indazol-1-yl)acetyl)-N-(6-bromo-3-methylpyridin-2-yl)-3-azabicyclo[3.1.0]hexane-2-carboxamide (Compound 63):** To a solution of compound **63-4** (0.023 g, 0.07 mmol, 1 equiv.) in dimethylformamide (1 mL) at 0 °C under an atmosphere of argon were added compound **63-5** (0.021 g, 0.07 mmol, 1 equiv.), HATU (0.056 g, 0.147 mmol, 2.1 equiv.), and DIPEA (0.045g, 0.350 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound 63.** ¹H NMR (400 MHz, DMSO-d6) δ 0.75 - 0.89 (m, 2H), 2.00 - 2.08 (m, 4H), 2.65 (s, 3H), 3.90 (d, J = 9.6 Hz, 1H), 4.05 (dd, J = 5.3, 9.6 Hz, 1H), 4.60 (d, J = 5.5 Hz, 1H), 4.69 (d, J = 6.3 Hz, 2H), 5.34 (t, J = 6.3 Hz, 1H), 5.54 - 5.69 (m, 2H), 7.42 (d, J = 7.9 Hz, 1H), 7.61 (m, 1H), 7.82 - 7.93 (m, 2H), 8.46 (s, 1H), 9.13 (s, 2H), 10.28 (s, 1H).

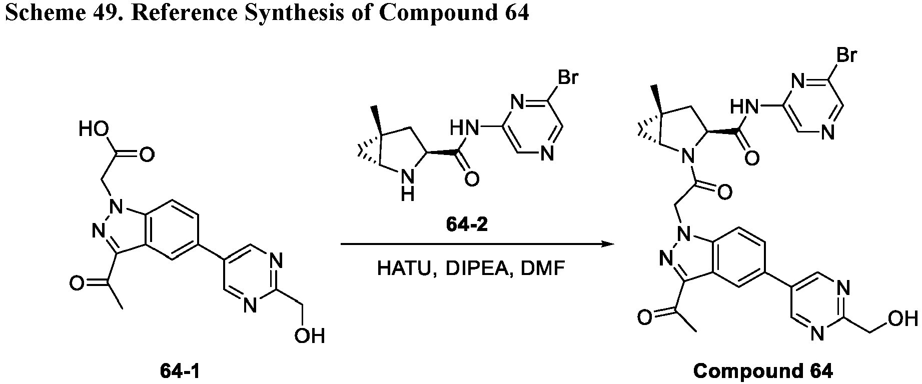

**(1R,3S,5R)-2-(2-(3-acetyl-5-(2-(hydroxymethyl)pyrimidin-5-yl)-1H-indazol-1-yl)acetyl)-N-(6-bromopyrazin-2-yl)-5-methyl-2-azabicyclo[3.1.0]hexane-3-carboxamide (Compound 64):** To a solution of compound **64-1** (0.031 g, 0.096 mmol, 1 equiv.) in dimethylformamide (1 mL) at 0 °C under an atmosphere of argon were added compound **64-2** (0.029 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 64.** ¹H NMR (400 MHz, DMSO-d6) δ 0.95 - 1.05 (m, 2H), 1.32 (s, 3H), 2.04 (dd, J = 5.9, 13.3 Hz, 1H), 2.46 - 2.55 (m, 1H), 2.66 (s, 3H), 3.66 (dd, J = 2.7, 5.5 Hz, 1H), 4.43 - 4.48 (m, 1H), 4.69 (d, J = 6.3 Hz, 2H), 5.34 (t, J = 6.3 Hz, 1H), 5.59 (d, J = 17.2 Hz, 1H), 5.97 (d, J = 17.3 Hz, 1H), 7.90 (d, J = 1.8 Hz, 2H), 8.47 (s, 1H), 8.54 (s, 1H), 9.13 (s, 2H), 9.27 (s, 1H), 11.13 (s, 1H).

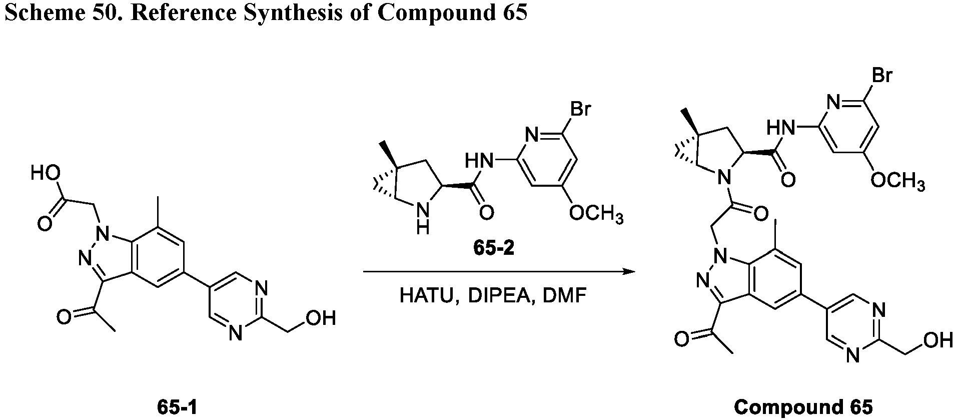

**Step** 1: **(1R,3S,5R)-2-(2-(3-acetyl-5-(2-(hydroxymethyl)pyrimidin-5-yl)-7-methyl-1H-indazol-1-yl)acetyl)-N-(6-bromo-4-methoxypyridin-2-yl)-5-methyl-2-azabicyclo[3.1.0]hexane-3-carboxamide (Compound 65):** To a solution of compound **65-1** (0.024 g, 0.07 mmol, 1 equiv.) in dimethylformamide (1 mL) at 0 °C under an atmosphere of argon were added compound **65-2** (0.023 g, 0.07 mmol, 1 equiv.), HATU (0.056 g, 0.147 mmol, 2.1 equiv.), and DIPEA (0.045 g, 0.350 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The remaining residue was purified by column chromatography on silica gel (eluted with DCM/MeOH) to give **Compound 65.** ¹H NMR (400 MHz, DMSO-d6) δ 0.87 - 0.94 (m, 1H), 0.97 - 1.05 (m, 1H), 1.31 (s, 3H), 1.99 (dd, J = 5.9, 13.2 Hz, 1H), 2.46 - 2.50 (m, 1H), 2.65 (s, 3H), 2.71 (s, 3H), 3.63 (dd, J = 2.4, 5.6 Hz, 1H), 3.84 (s, 3H), 4.43 (dd, J = 5.9, 9.1 Hz, 1H), 4.68 (d, J = 6.3 Hz, 2H), 5.33 (t, J = 6.3 Hz, 1H), 5.68 (d, J = 17.8 Hz, 1H), 6.07 (d, J = 17.9 Hz, 1H), 6.96 (d, J = 2.0 Hz, 1H), 7.60 - 7.74 (m, 2H), 8.34 (d, J = 1.6 Hz, 1H), 9.11 (s, 2H), 10.73 (s, 1H).

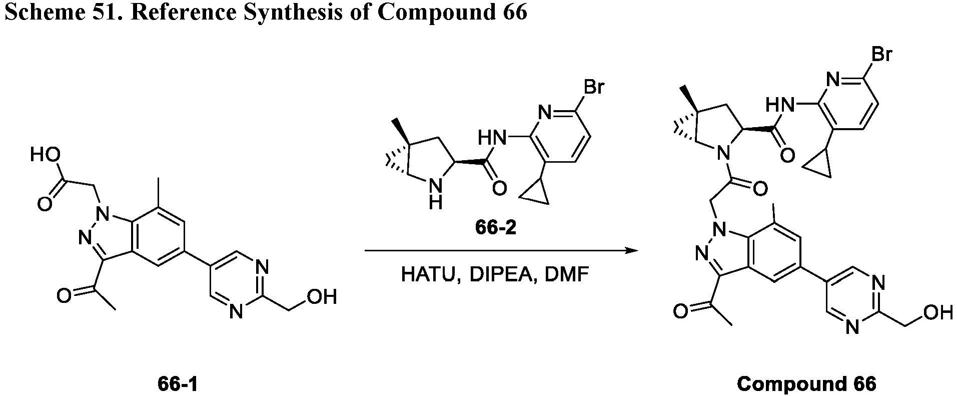

**Step 1: (1R,3S,5R)-2-(2-(3-acetyl-5-(2-(hydroxymethyl)pyrimidin-5-yl)-7-methyl-1H-indazol-1-yl)acetyl)-N-(6-bromo-3-cyclopropylpyridin-2-yl)-5-methyl-2-azabicyclo[3.1.0]hexane-3-carboxamide (Compound 66):** To a solution of compound **66-1** (0.033 g, 0.096 mmol, 1 equiv.) in dimethylformamide (1 mL) at 0 °C under an atmosphere of argon were added compound **66-2** (0.032 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 66.** ¹H NMR (400 MHz, DMSO-d6) δ 0.77 - 0.86 (m, 2H), 0.92 - 0.99 (m, 3H), 1.03 - 1.08 (m, 1H), 1.33 (s, 3H), 2.01 - 2.11 (m, 1H), 2.18 (dd, J = 4.7, 13.3 Hz, 1H), 2.46 - 2.48 (m, 1H), 2.65 (s, 3H), 2.71 (s, 3H), 3.53 - 3.68 (m, 1H), 4.52 (dd, J = 4.6, 9.2 Hz, 1H), 4.68 (s, 2H), 5.34 (s, 1H), 5.68 (d, J = 17.7 Hz, 1H), 6.06 (d, J = 17.8 Hz, 1H), 7.11 (d, J = 8.2 Hz, 1H), 7.65 (s, 1H), 7.93 (d, J = 8.2 Hz, 1H), 8.36 (s, 1H), 9.12 (s, 2H), 10.05 (s, 1H).

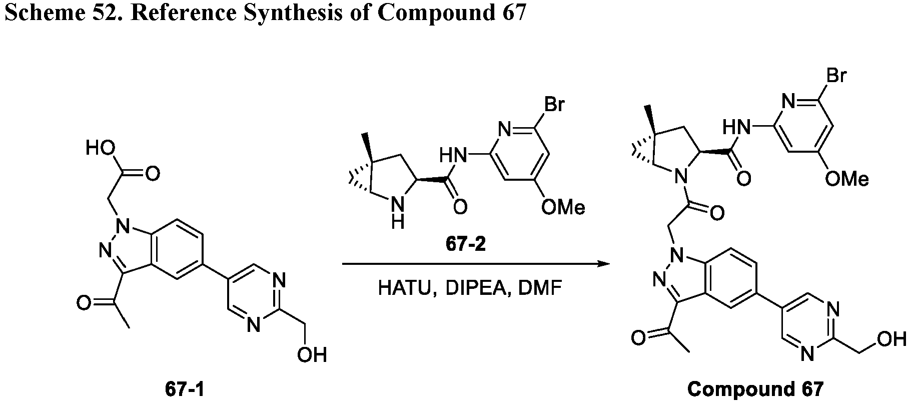

**(1R,3S,5R)-2-(2-(3-acetyl-5-(2-(hydroxymethyl)pyrimidin-5-yl)-1H-indazol-1-yl)acetyl)-N-(6-bromo-4-methoxypyridin-2-yl)-5-methyl-2-azabicyclo[3.1.0]hexane-3-carboxamide (Compound 67):** To a solution of compound 67-1 (0.046 g, 0.141 mmol, 1 equiv.) in dimethylformamide (1 mL) at 0 °C under an atmosphere of argon were added compound 67-2 (0.046 g, 0.141 mmol, 1 equiv.), HATU (0.113 g, 0.296 mmol, 2.1 equiv.), and DIPEA (0.091 g, 0.123 mL, 0.705 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 67.** ¹H NMR (400 MHz, DMSO-d6) δ 0.96 - 1.01 (m, 2H), 1.24 - 1.35 (m, 4H), 1.99 (dd, J = 5.8, 13.1 Hz, 1H), 2.45 - 2.48 (m, 1H), 2.66 (s, 3H), 3.63 (t, J = 4.1 Hz, 1H), 3.83 (s, 3H), 4.43 (dd, J = 5.7, 9.1 Hz, 1H), 4.69 (s, 2H), , 5.58 (d, J = 17.2 Hz, 1H), 5.96 (d, J = 17.3 Hz, 1H), 6.96 (d, J = 2.0 Hz, 1H), 7.65 (d, J = 2.0 Hz, 1H), 7.90 (d, J = 1.6 Hz, 2H), 8.47 (s, 1H), 9.14 (s, 2H), 10.70 (s, 1H).

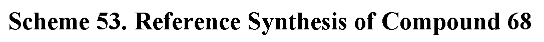

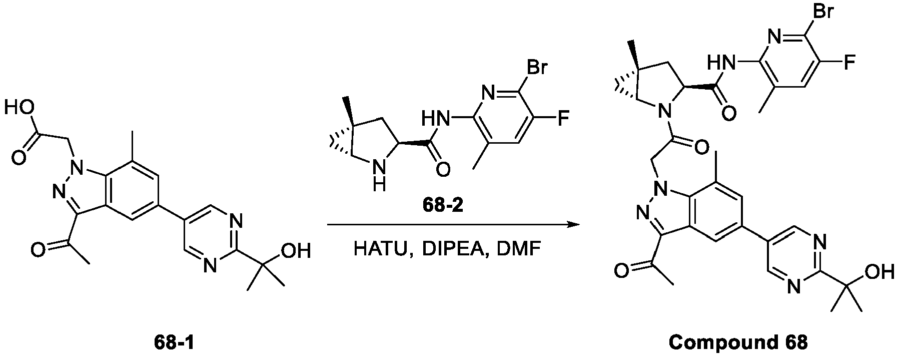

**(1R,3S,5R)-2-(2-(3-acetyl-5-(2-(2-hydroxypropan-2-yl)pyrimidin-5-yl)-7-methyl-1H-indazol-1-yl)acetyl)-N-(6-bromo-5-fluoro-3-methylpyridin-2-yl)-5-methyl-2-azabicyclo[3.1.0]hexane-3-carboxamide (Compound 68):** To a solution of compound **68-1** (0.05 g, 0.136 mmol, 1 equiv.) in dimethylformamide (1 mL) at 0 °C under an atmosphere of argon were added compound **68-2** (0.045 g, 0.141 mmol, 1 equiv.), HATU (0.109 g, 0.286 mmol, 2.1 equiv.), and DIPEA (0.088 g, 0.680 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound** 68. ¹H NMR (400 MHz, DMSO-d6) δ 0.91 - 0.98 (m, 1H), 1.03 (t, J = 5.4 Hz, 1H), 1.33 (s, 3H), 1.56 (s, 6H), 2.01 - 2.12 (m, 4H), 2.54 - 2.61 (m, 1H), 2.65 (s, 3H), 2.70 (s, 3H), 4.40 (dd, J = 5.6, 9.2 Hz, 1H), 5.10 (s, 1H), 5.70 (d, J = 17.8 Hz, 1H), 6.04 (d, J = 17.8 Hz, 1H), 7.64 (s, 1H), 7.83 (d, J = 8.4 Hz, 1H), 8.10 - 8.23 (m, 1H), 8.35 (d, J = 1.6 Hz, 1H), 9.12 (s, 2H), 10.31 (s, 1H).

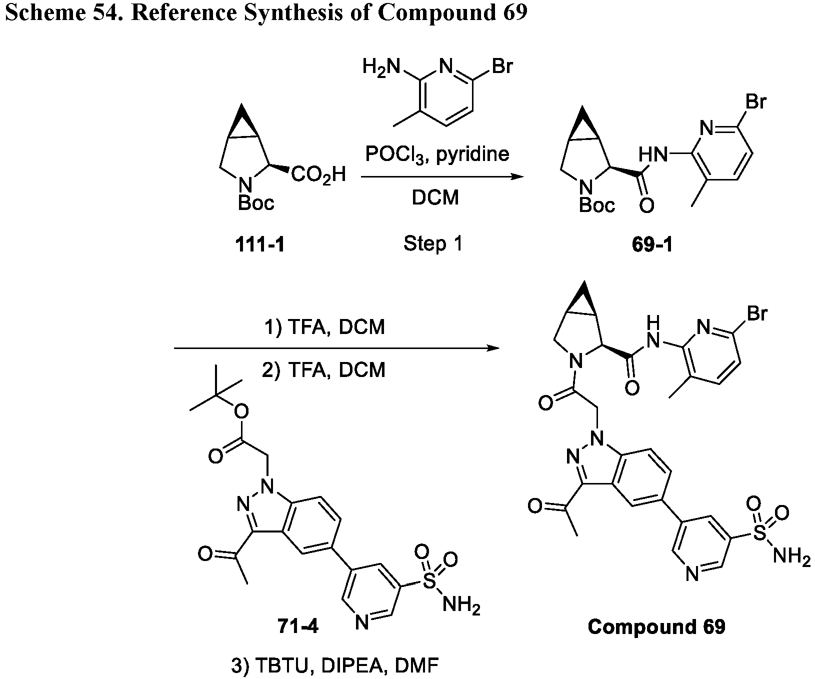

**Step 1. tert-Butyl (1R,2S,5S)-2-((6-bromo-3-methylpyridin-2-yl)carbamoyl)-3-azabicyclo[3.1.0]hexane-3-carboxylate (69-1):** To an ice cooled solution of compound **111-1** (5.0 g) and 6-bromo-3-methylpyridin-2-amine (4.12 g) in DCM 100 ml pyridine (8.9 mL) was added followed by the slow dropwise addition of POCl₃ (1 equiv.) at 5 °C. The reaction mixture was stirred for 4 hr at 5 °C, and saturated aqueous NaHCO₃ solution was added and stirred for 1h at room temperature. The organic layer was separated, and the aqueous layer was extracted with DCM (20 mL). The combined organic layer was dried (Na₂SO₄), filtered, concentrated, and the residue was purified by silica gel flash column chromatography to afford compound 69-1.

**Step 2. (1R,2S,5S)-3-(2-(3-Acetyl-5-(5-sulfamoylpyridin-3-yl)-1*H*-indazol-1-yl)acetyl)-N-(6-bromo-3-methylpyridin-2-yl)-3-azabicyclo[3.1.0]hexane-2-carboxamide (Compound 68):** Compound **71-4** (, 0.05 g, 0.116 mmol, 1 equiv.) was stirred with TFA (2 mL) and dichloromethane (1 mL) for 3 h. In another vial, compound **69-1** (0.046 g, 0.116 mmol, 1 equiv.) was stirred with 1 mL of TFA and 1 mL of dichloromethane for 30 min. The contents from both vials were combined and the volatiles were removed under reduced pressure. The residue was dissolved in DMF (2 mL) and cooled with an ice-bath. To this cold solution were added successively DIPEA (0.075 g, 0.101 mL, 0.581 mmol, 5 equiv.) and TBTU (0.041 g, 0.128 mmol, 1.1 equiv.). The cooling bath was removed, and the reaction mixture was stirred at rt for 30 min. The solvent was removed under reduced pressure, and the residue was dissolved in dichloromethane and washed with saturated aqueous sodium bicarbonate. The organic layer was dried (Na₂SO₄), filtered, concentrated, and the crude product was purified by silica gel flash column chromatography (eluent: 0-2.5 % MeOH in dichloromethane) to give **Compound 69** as a white solid. ¹H NMR (400 MHz, Methanol-*d*₄) δ 0.84 - 0.99 (m, 2H), 1.97 (s, 1H), 2.12 (s, 3H), 2.18 (s, 1H),2.68 (s, 3H), 3.95 (d, *J =* 9.8 Hz, 1H), 4.08 (dd, *J =* 5.3, 9.6 Hz, 1H), 5.52 (d, *J* = 5.2 Hz, 2H), 7.35 (d, *J =* 7.8 Hz, 1H), 7.49 (d, *J = 7.9* Hz, 1H), 7.79 (s, 2H), 8.51 - 8.60 (m, 2H), 9.00 (d, *J =* 2.1 Hz, 1H), 9.05 (d, *J =* 2.1 Hz, 1H).

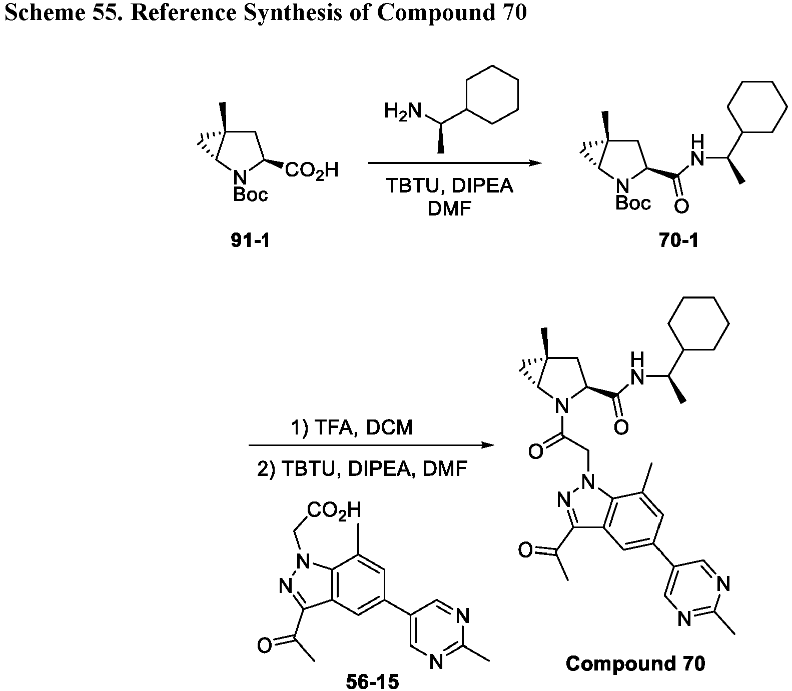

**Step 1. *tert*-Butyl** (1R,3S,5R)-3-(((R)-1-cyclohexylethyl)carbamoyl)-5-methyl-2-azabicyclo[3.1.0]hexane-2-carboxylate (70-1): To an ice cold solution of compound 91-1 (0.104 g, 0.432 mmol, 1 equiv.) and (1R)-1-cyclohexylethanamine (0.055 g, 0.432 mmol, 1 equiv.) in DMF (2 mL) were added DIPEA (0.361 mL, 0.432 mmol, 1 equiv.) and TBTU (0.153 g, 0.476 mmol, 1.1 equiv.) successively. The cooling bath was removed and the reaction mixture was stirred at rt for 30 min. The solvent was removed under reduced pressure and the residue was dissolved in dichloromethane. The dichloromethane solution was washed successively with saturated aqueous sodium bicarbonate and cold 1N aqueous HCl. The organic layer was separated, dried (Na₂SO₄), and concentrated to give compound 70-1 as a colorless resin, which was used in the next step without further purification.

**Step 2. (1R,3S,5R)-2-(2-(3-acetyl-7-methyl-5-(2-methylpyrimidin-5-yl)-1H-indazol-1-yl)acetyl)-N-((R)-1-cyclohexylethyl)-5-methyl-2-azabicyclo[3.1.0]hexane-3-carboxamide (Compound 70):** Compound **70-1** (0.13 g, 0.371 mmol, 1 equiv.) was stirred at rt with TFA (1 mL) and CH₂Cl₂ (1 mL) for 30 min, then the volatiles were removed under reduced pressure. The residue was dissolved in DMF (2 mL), and compound **56-15** (0.12 g) and DIPEA (0.24 g, 0.323 mL, 1.854 mmol, 5 equiv.) were added to this solution at 0 °C, followed by TBTU (0.131 g, 0.408 mmol, 1.1 equiv.). The cooling bath was removed, and the reaction mixture was stirred at rt for 30 min. The solvent was removed under reduced pressure, and the residue was dissolved in dichloromethane and washed with saturated aqueous sodium bicarbonate solution. The organic layer was dried (Na₂SO₄) and concentrated, and the crude product was purified by silica gel column chromatography (eluent: 0-3 % MeOH in dichloromethane) to give **Compound 70** as a white solid. ¹H NMR (400 MHz, Chloroform-d) δ 0.83-0.89 (m, 3H), 0.99 (d, J = 6.8 Hz, 3H), 1.06-1.26 (m, 4H), 1.44 (s, 3H), 1.58-1.65 (m, 6H), 2.12 (dd, *J=* 8.7, 13.3 Hz, 1H),2.68-2.72 (m, 1H), 2.70 (s, 3H), 2.71 (s, 3H), 2.80 (s, 3H), 3.10 (dd, *J =* 2.3, 5.4 Hz, 1H), 3.70 - 3.84 (m, 1H), 4.54 (dd, *J =* 2.8, 8.6 Hz, 1H), 5.63 (s, 2H), 6.50 (d, *J =* 8.9 Hz, 1H), 7.32 (s, 1H), 8.47 (s, 1H), 8.88 (s, 2H).

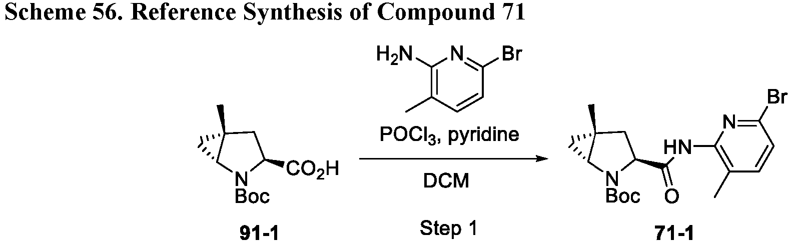

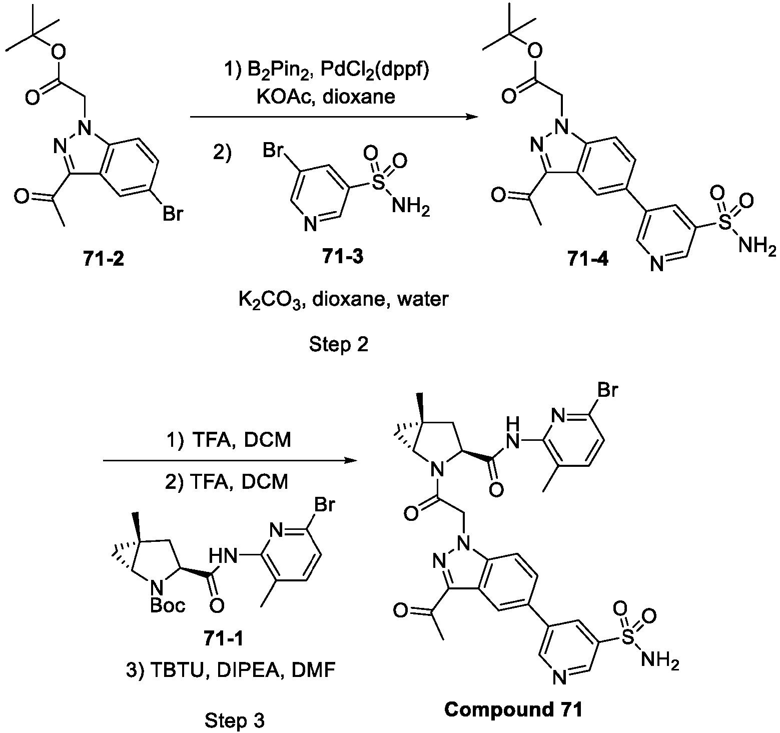

**Step 1. tert-Butyl (1R,35,5R)-3-((6-bromo-3-methylpyridin-2-yl)carbamoyl)-5-methyl-2-azabicyclo[3.1.0]hexane-2-carboxylate (71-1): To** an ice-cooled solution of compound **91-1** (0.15 g) and 6-bromo-3-methylpyridin-2-amine (0.12 g) in DCM (5 ml), pyridine (0.25 mL) was added followed by the slow dropwise addition of POCl₃ (58 µL) at 5 °C. The reaction mixture was stirred for 3 hr at 5 °C, then saturated aqueous NaHCO₃ was added and stirred for 1h at room temperature. The organic layer was separated and aqueous layer was extracted with CH₂Cl₂ (20 mL). The combined organic layers were dried (Na₂SO₄), filtered, and concentrated. The residue was purified by silica gel flash column chromatography to afford compound 71-1.

**Step 2. *tert-Butyl* 2-(3-acetyl-5-(5-sulfamoylpyridin-3-yl)-1H-indazol-1-yl)acetate (71-4):** A heterogeneous solution of compound 71-2 (0.41 g, 1.16 mmol), bis(pinacolato)diboron (0.402 g, 1.58 mmol), potassium acetate (0.207 g, 2.11 mmol) and PdCl₂(dppf) (0.043 g) in dioxane (10 mL) was degassed with argon and heated overnight at 90 °C under argon. The reaction mixture was cooled to rt and compound **71-3** (5-bromopyridine-3-sulfonamide, 0.25 g, 1.06 mmol) and potassium carbonate (0.437 g, 3.162 mmol) were added. 2.5 mL of water was added, and the reaction mixture was degassed with argon then heated at 100 °C for 3 h. The volatiles were then removed under reduced pressure, and the residue was purified by silica gel column chromatography (eluent: 0-3 % MeOH in dichloromethane) to give 0.36 g of cream colored solid.

**Step 3. (1R,3S,5R)-2-(2-(3-Acetyl-5-(5-sulfamoylpyridin-3-yl)-1*H*-indazol-1-yl)acetyl)-*N*-(6-bromo-3-methylpyridin-2-yl)-5-methyl-2-azabicyclo[3.1.0]hexane-3-carboxamide (Compound 71):** Compound 71-4 (0.05 g, 0.116 mmol, 1 equiv.) was stirred with TFA (2 mL) and dichloromethane (1 mL) for 3 h. In another vial, compound 71-1 (0.048 g, 0.116 mmol, 1 equiv.) was stirred with 1 mL of TFA and 1 mL of dichloromethane for 30 min. The contents from both the vials were combined and the volatiles were removed under reduced pressure. The residue was dissolved in DMF (2 mL) and cooled in an ice-bath. To this cold solution were added successively DIPEA (0.075 g, 0.101 mL, 0.581 mmol, 5 equiv.) and TBTU (0.041 g, 0.128 mmol, 1.1 equiv.). The cooling bath was removed, and the reaction mixture was stirred at rt for 30 min. The solvent was removed under reduced pressure, and the residue was dissolved in dichloromethane and washed with saturated aqueous sodium bicarbonate solution. The organic layer was dried (Na₂SO₄), filtered, concentrated, and the crude product was purified by silica gel flash column chromatography (eluent: 0-2.5 % MeOH in dichloromethane) to give Compound 71 as a white solid. ¹H NMR (400 MHz, Chloroform-d) δ 0.97 (dd, *J = 2.4,* 5.4 Hz, 1H), 1.16 (t, *J =* 5.5 Hz, 1H),1.43 (s, 3H), 2.01 (s, 3H), 2.39 (dd, *J =* 8.9, 13.5 Hz, 1H), 2.58 - 2.66 (m, 1H), 2.70 (s, 3H), 3.32 (dd, *J =* 2.3, 5.5 Hz, 1H), 4.80 (dd, *J =* 3.7, 8.7 Hz, 1H), 5.50 (d, *J =* 16.9 Hz, 1H), 5.59 (d, *J =* 16.9 Hz, 1H), 5.79 (s, 2H), 7.13 (d, *J =* 7.9 Hz, 1H), 7.26 (d, *J =* 8.0 Hz, 1H), 7.43 (d, *J =* 8.7 Hz, 1H), 7.54 (d, *J = 8.7* Hz, 1H), 8.02 (s, 1H), 8.22 (s, 1H), 8.45 (s, 1H), 8.86 (s, 1H), 8.93 (d, *J =* 2.0 Hz, 1H), 9.02 (s, 1H).

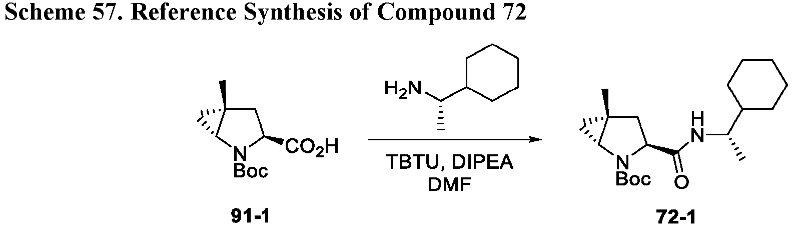

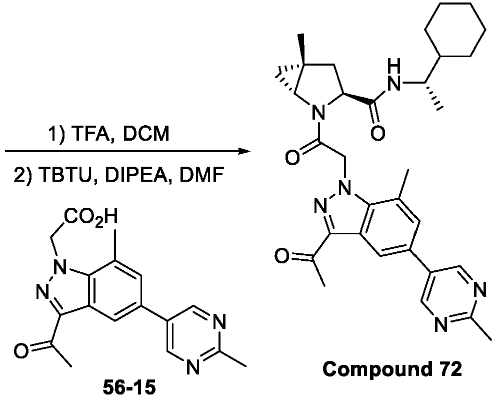

**Step 1. *tert*-Butyl (1R,3S,5R)-3-(((S)-1-cyclohexylethyl)carbamoyl)-5-methyl-2-azabicyclo[3.1.0]hexane-2-carboxylate (72-1):** To an ice-cold solution of compound 91-1 (0.1 g, 0.414 mmol, 1 equiv.) and (1S)-1-cyclohexylethanamine (0.053 g, 0.414 mmol, 1 equiv.) in DMF (2 mL) were added DIPEA (0.361 mL, 0.432 mmol, 1 equiv.), and TBTU (0.153 g, 0.476 mmol, 1.1 equiv.) successively. The cooling bath was removed and the reaction mixture was stirred at rt for 30 min. The solvent was removed under reduced pressure and the residue was dissolved in dichloromethane. The dichloromethane solution was washed successively with saturated aqueous sodium bicarbonate and cold 1N aqueous HCl. The organic layer was separated, dried (Na₂SO₄), and concentrated to give compound **72-1** as a colorless resin, which was used without further purification.

**Step 2. (1R,3S,5R)-2-(2-(3-Acetyl-7-methyl-5-(2-methylpyrimidin-5-yl)-1H-indazol-1-yl)acetyl)-*N-*((S)-1-cyclohexylethyl)-5-methyl-2-azabicyclo[3.1.0]hexane-3-carboxamide (Compound 72):** Compound **72-1** (0.13 g) was stirred at rt with TFA (1 mL) and DCM (1 mL) for 30 min, then the volatiles were removed under reduced pressure. The residue was dissolved in DMF (2 mL), and compound **56-15** (0.12 g) and DIPEA (0.24 g, 0.323 mL, 1.854 mmol, 5 equiv.) were added to this solution at 0 °C, followed by TBTU (0.131 g, 0.408 mmol, 1.1 equiv.). The cooling bath was removed and the reaction mixture was stirred at rt for 30 min. The solvent was removed under reduced pressure, and the residue was dissolved in dichloromethane, then washed with saturated aqueous sodium bicarbonate. The organic layer was dried (Na₂SO₄) and concentrated, and the residue was purified by silica gel flash column chromatography (eluent: 0-3 % MeOH in dichloromethane) to give **Compound 72** as a white solid. ¹H NMR (400 MHz, Chloroform-d) δ 0.76 - 0.94 (m, 3H), 0.99 (d, J = 6.8 Hz, 3H), 1.03-1.26 (m, 4H), 1.43 (s, 3H), 1.55-1.67 (m, 6H), 2.13 (dd, *J* = 8.7, 13.3 Hz, 1H), 2.63 - 2.72 (m, 1H), 2.70 (s, 6H), 2.80 (s, 3H), 3.09 (dd, *J =* 2.4, 5.4 Hz, 1H), 3.74-3.81(m, 1H), 4.56 (dd, *J =* 2.8, 8.7 Hz, 1H), 5.63 (d, *J =* 1.9 Hz, 2H), 6.45 (d, *J =* 8.9 Hz, 1H), 7.33 (s, 1H), 8.47 (s, 1H), 8.89 (s, 2H).

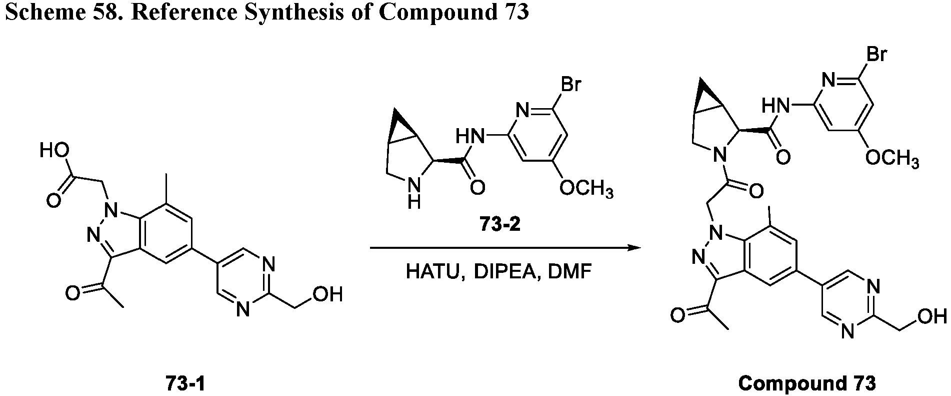

**(1R,2S,5S)-3-(2-(3-acetyl-5-(2-(hydroxymethyl)pyrimidin-5-yl)-7-methyl-1H-indazol-1-yl)acetyl)-N-(6-bromo-4-methoxypyridin-2-yl)-3-azabicyclo[3.1.0]hexane-2-carboxamide (Compound 73):** To a solution of compound 73-1 (0.033 g, 0.096 mmol, 1 equiv.) in dimethylformamide (1 mL, 0.141 M, 22 Vols) at 0 °C under an atmosphere of argon were added compound 73-2 (0.03 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, then concentrated to dryness. The residue was purified by column chromatography on silica gel (eluted with DCM/MeOH) to give **Compound 73.** ¹H NMR (400 MHz, DMSO-d6) δ 0.70 - 0.79 (m, 1H), 1.24 - 1.30 (m, 1H), 1.86 - 1.92 (m, 1H), 1.97 - 2.04 (m, 1H), 2.64 (s, 3H), 2.70 (s, 3H), 3.82 (s, 3H), 3.86 - 3.92 (m, 1H), 4.01 (dd, J = 5.1, 10.0 Hz, 1H), 4.66 (d, J = 8.8 Hz, 3H), 5.33 (s, 1H), 5.61 - 5.91 (m, 2H), 6.94 (d, J = 2.1 Hz, 1H), 7.60 (d, J = 2.0 Hz, 1H), 7.64 (s, 1H), 8.32 (d, J = 1.7 Hz, 1H), 9.11 (s, 2H), 10.84 (s, 1H).

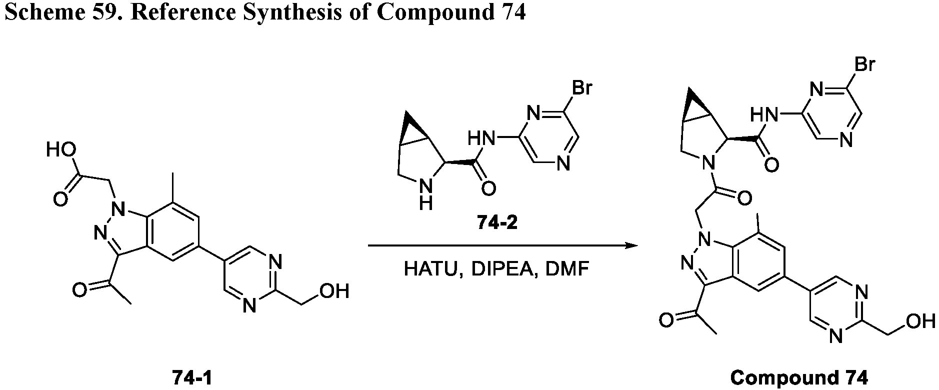

**(1R,2S,5S)-3-(2-(3-acetyl-5-(2-(hydroxymethyl)pyrimidin-5-yl)-7-methyl-1H-indazol-1-yl)acetyl)-N-(6-bromopyrazin-2-yl)-3-azabicyclo[3.1.0]hexane-2-carboxamide (Compound 74):** To a solution of compound 73-1 (0.026 g, 0.078 mmol, 1 equiv.) in dimethylformamide (1 mL) at 0 °C under an atmosphere of argon were added compound **74-2** (0.022 g, 0.078 mmol, 1 equiv.), HATU (0.062 g, 0.164 mmol, 2.1 equiv.), and DIPEA (0.05g, 0.390 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 74.** ¹H NMR (400 MHz, DMSO-d6) δ 0.72 - 0.85 (m, 1H), 1.23 - 1.31 (m, 1H), 1.91 (s, 1H), 2.01 (d, J = 11.1 Hz, 1H), 2.64 (s, 3H), 2.71 (s, 3H), 3.92 (d, J = 9.8 Hz, 1H), 4.03 (dd, J = 5.6, 10.1 Hz, 1H), 4.64 - 4.73 (m, 3H), 5.33 (t, J = 6.4 Hz, 1H), 5.67 (d, J = 17.8 Hz, 1H), 5.79 (d, J = 17.8 Hz, 1H), 7.64 (s, 1H), 8.32 (d, J = 1.5 Hz, 1H), 8.52 (s, 1H), 9.10 (s, 2H), 9.24 (s, 1H), 11.27 (s, 1H).

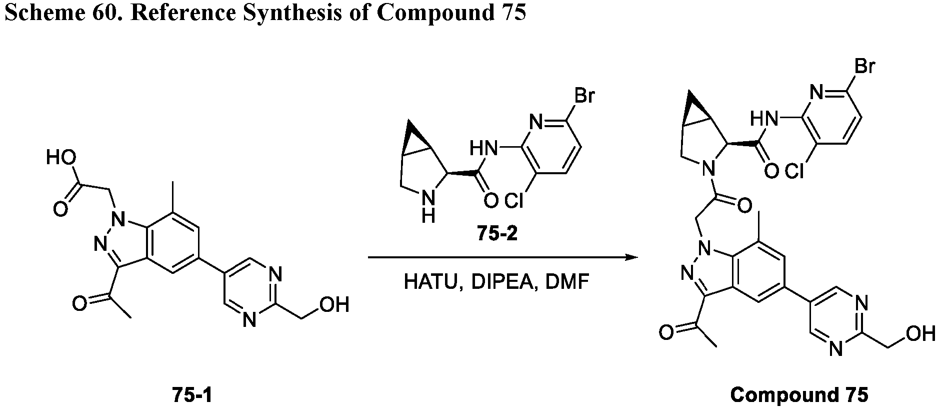

**Step** 1: **(1R,2S,5S)-3-(2-(3-acetyl-5-(2-(hydroxymethyl)pyrimidin-5-yl)-7-methyl-1H-indazol-1-yl)acetyl)-N-(6-bromo-3-chloropyridin-2-yl)-3-azabicyclo[3.1.0]hexane-2-carboxamide (Compound 75):** To a solution of compound **75-1** (0.03 g, 0.088 mmol, 1 equiv.) in dimethylformamide (1 mL) at 0 °C under an atmosphere of argon were added compound 75-2 (0.028 g, 0.088 mmol, 1 equiv.), HATU (0.07 g, 0.185 mmol, 2.1 equiv.), and DIPEA (0.057 g, 0.441 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound** 75. ¹H NMR (400 MHz, DMSO-d6) δ 0.74 - 0.88 (m, 2H), 1.23 - 1.30 (m, 1H), 2.05 (d, J = 12.9 Hz, 1H), 2.64 (s, 3H), 2.68 (s, 3H), 3.79 - 3.94 (m, 1H), 4.04 (q, J = 4.8, 6.1 Hz, 1H), 4.67 (t, J = 5.2 Hz, 3H), 5.33 (t, J = 6.2 Hz, 1H), 5.63 - 5.82 (m, 2H), 7.33 (d, J = 8.2 Hz, 1H), 7.60 (s, 1H), 8.15 (d, J = 8.3 Hz, 1H), 8.32 (s, 1H), 9.10 (s, 2H), 10.41 (s, 1H).

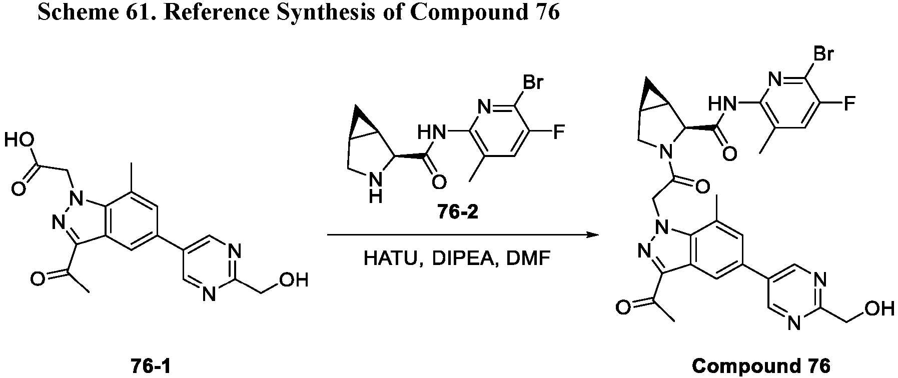

**(1R,2S,5S)-3-(2-(3-acetyl-5-(2-(hydroxymethyl)pyrimidin-5-yl)-7-methyl-1H-indazol-1-yl)acetyl)-N-(6-bromo-5-fluoro-3-methylpyridin-2-yl)-3-azabicyclo[3.1.0]hexane-2-carboxamide (Compound 76):** To a solution of compound **76-1** (0.033 g, 0.096 mmol, 1 equiv.) in dimethylformamide (1 mL) at 0 °C under an atmosphere of argon were added compound **76-2** (0.03 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound 76.** ¹H NMR (400 MHz, DMSO-d6) δ 0.72 - 0.89 (m, 2H), 1.99 - 2.11 (m, 4H), 2.64 (s, 3H), 2.68 (s, 3H), 3.88 (d, J = 9.8 Hz, 1H), 4.02 (dd, J = 5.2, 9.8 Hz, 1H), 4.62 (d, J = 5.4 Hz, 1H), 4.68 (d, J = 6.3 Hz, 2H), 5.33 (t, J = 6.3 Hz, 1H), 5.57 - 5.86 (m, 2H), 7.61 (s, 1H), 7.79 (d, J = 8.4 Hz, 1H), 8.27 - 8.38 (m, 1H), 9.10 (s, 2H), 10.37 (s, 1H).

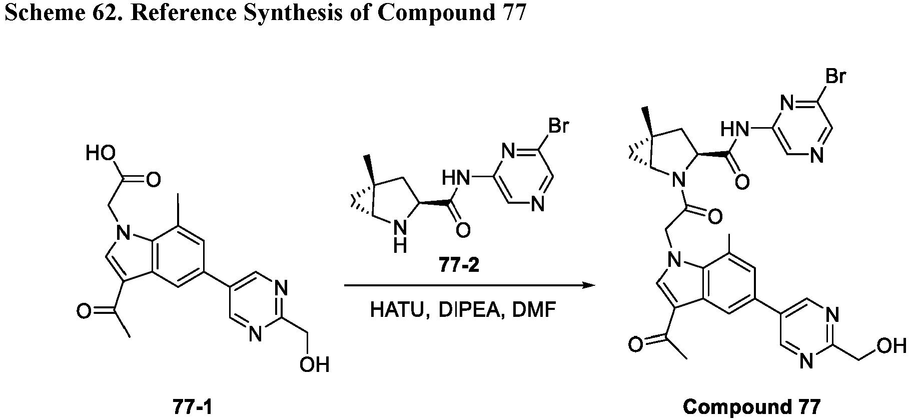

**(1R,3S,5R)-2-(2-(3-acetyl-5-(2-(hydroxymethyl)pyrimidin-5-yl)-7-methyl-1H-indol-1-yl)acetyl)-N-(6-bromopyrazin-2-yl)-5-methyl-2-azabicyclo[3.1.0]hexane-3-carboxamide (Compound 77):** To a solution of compound **77-1** (0.03 g, 0.088 mmol, 1 equiv.) in dimethylformamide (1 mL) at 0 °C under an atmosphere of argon were added compound 77-2 (0.026 g, 0.088 mmol, 1 equiv.), HATU (0.071 g, 0.186 mmol, 2.1 equiv.), and DIPEA (0.057 g, 0.442 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound 77.** ¹H NMR (400 MHz, DMSO-d6) δ 0.85 - 0.94 (m, 1H), 1.01 (t, J = 5.4 Hz, 1H), 1.32 (s, 3H), 2.04 (dd, J = 6.0, 13.3 Hz, 1H), 2.45 (s, 3H), 2.48 - 2.56 (m, 1H), 2.68 (s, 3H), 3.50 - 3.64 (m, 1H), 4.45 (dd, J = 6.1, 9.1 Hz, 1H), 4.66 (d, J = 6.3 Hz, 2H), 5.31 (t, J = 6.3 Hz, 1H), 5.43 (d, J = 17.9 Hz, 1H), 5.76 (d, J = 17.9 Hz, 1H), 7.38 (d, J = 1.8 Hz, 1H), 8.33 (s, 1H), 8.38 (d, J = 1.8 Hz, 1H), 8.54 (s, 1H), 9.05 (s, 2H), 9.27 (s, 1H), 11.17 (s, 1H).

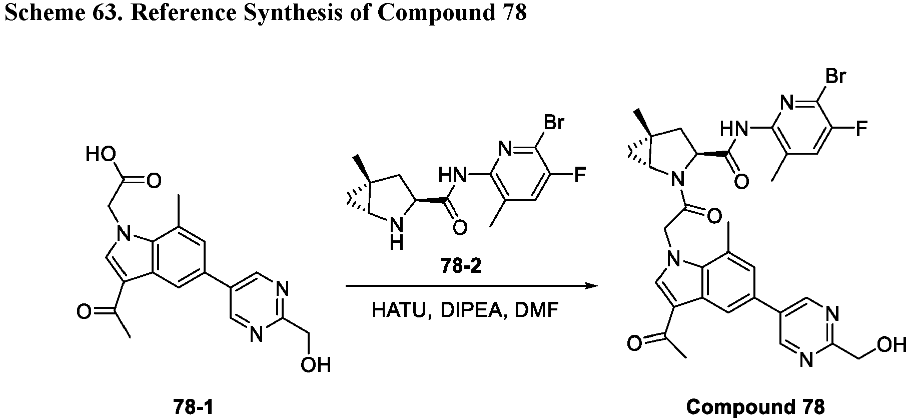

**(1R,3S,5R)-2-(2-(3-acetyl-5-(2-(hydroxymethyl)pyrimidin-5-yl)-7-methyl-1H-indol-1-yl)acetyl)-N-(6-bromo-5-fluoro-3-methylpyridin-2-yl)-5-methyl-2-azabicyclo[3.1.0]hexane-3-carboxamide (Compound 78):** To a solution of compound **78-1** (0.033 g, 0.096 mmol, 1 equiv.) in dimethylformamide (1 mL) at 0 °C under an atmosphere of argon were added compound **78-2** (0.032 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 78.** ¹H NMR (400 MHz, DMSO-d6) δ 0.89 - 0.92 (m, 1H), 1.02 (t, J = 5.5 Hz, 1H), 1.33 (s, 3H), 2.01 - 2.11 (m, 4H), 2.46 (s, 3H), 2.54 - 2.59 (m, 1H), 2.65 (s, 3H), 3.55 (dd, J = 2.3, 5.6 Hz, 1H), 4.39 (dd, J = 5.6, 9.2 Hz, 1H), 4.67 (s, 2H), 5.42 (d, J = 17.9 Hz, 1H), 5.74 (d, J = 18.0 Hz, 1H), 7.37 (d, J = 1.8 Hz, 1H), 7.82 (d, J = 8.4 Hz, 1H), 8.32 (s, 1H), 8.39 (d, J = 1.8 Hz, 1H), 9.05 (s, 2H), 10.30 (s, 1H).

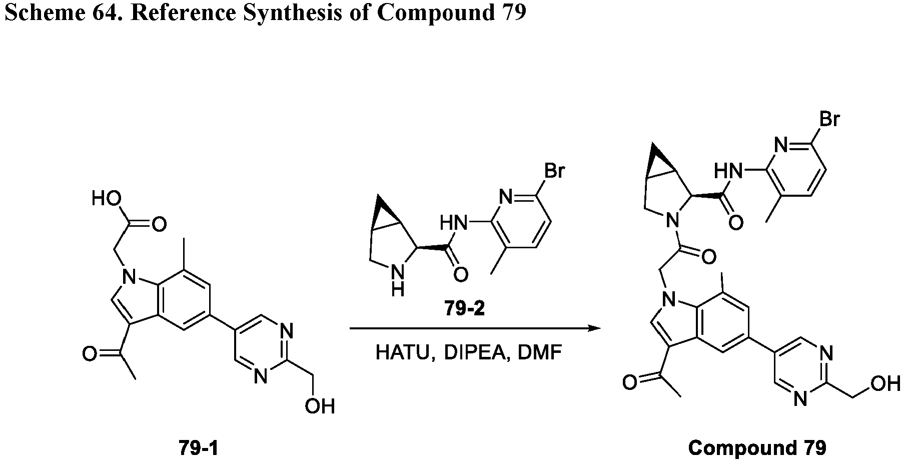

**(1R,2S,5S)-3-(2-(3-acetyl-5-(2-(hydroxymethyl)pyrimidin-5-yl)-7-methyl-1H-indol-1-yl)acetyl)-N-(6-bromo-3-methylpyridin-2-yl)-3-azabicyclo[3.1.0]hexane-2-carboxamide (Compound 79):** To a solution of compound **79-1** (0.034 g, 0.101 mmol, 1 equiv.) in dimethylformamide (1 mL) at 0 °C under an atmosphere of argon were added compound **79-2** (0.03 g, 0.101 mmol, 1 equiv.), HATU (0.081 g, 0.212 mmol, 2.1 equiv.), and DIPEA (0.065 g, 0.505 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 79.** ¹H NMR (400 MHz, DMSO-d6) δ 0.75 - 0.85 (m, 2H), 1.92 (d, J = 7.4 Hz, 1H), 2.03 - 2.07 (m, 4H), 2.45 (s, 3H), 2.62 (s, 3H), 3.83 (d, J = 9.8 Hz, 1H), 3.93 (dd, J = 5.1, 9.8 Hz, 1H), 4.65 (d, J = 12.9 Hz, 4H), 5.32 (d, J = 17.9 Hz, 1H), 5.44 (d, J = 17.8 Hz, 1H), 7.34 (d, J = 1.8 Hz, 1H), 7.41 (d, J = 7.9 Hz, 1H), 7.60 (d, J = 7.9 Hz, 1H), 8.22 (s, 1H), 8.37 (d, J = 1.8 Hz, 1H), 9.04 (s, 2H), 10.36 (s, 1H).

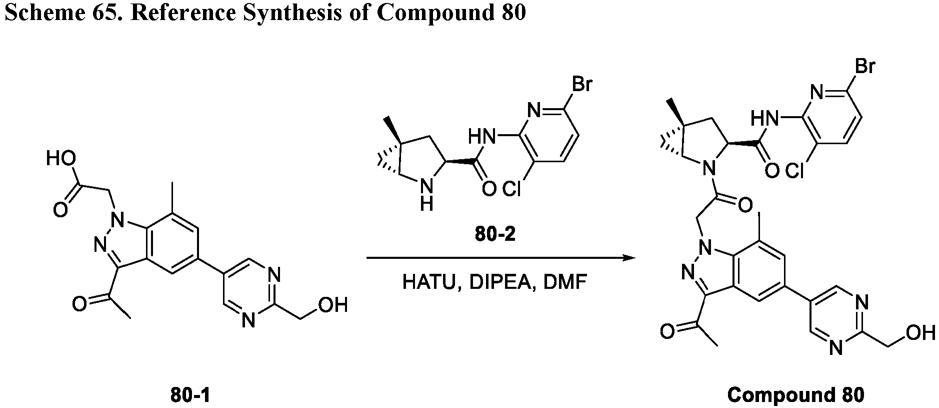

**(1R,3S,5R)-2-(2-(3-acetyl-5-(2-(hydroxymethyl)pyrimidin-5-yl)-7-methyl-1H-indazol-1-yl)acetyl)-N-(6-bromo-3-chloropyridin-2-yl)-5-methyl-2-azabicyclo[3.1.0]hexane-3-carboxamide (Compound 80):** To a solution of compound 80-1 (0.03 g, 0.088 mmol, 1 equiv.) in dimethylformamide (1 mL) at 0 °C under an atmosphere of argon were added compound 80-2 (0.029 g, 0.088 mmol, 1 equiv.), HATU (0.07 g, 0.185 mmol, 2.1 equiv.), and DIPEA (0.057 g, 0.441 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound** 80. ¹H NMR (400 MHz, DMSO-d6) δ 0.93 - 0.99 (m, 1H), 1.05 (t, J = 5.3 Hz, 1H), 1.32 - 1.36 (m, 3H), 2.12 - 2.20 (m, 1H), 2.53 - 2.59 (m, 1H), 2.66 (s, 3H), 2.71 (s, 3H), 3.54 - 3.68 (m, 1H), 4.40 - 4.53 (m, 1H), 4.68 (d, J = 6.3 Hz, 2H), 5.34 (t, J = 6.3 Hz, 1H), 5.70 (d, J = 17.8 Hz, 1H), 6.05 (d, J = 17.8 Hz, 1H), 7.38 (d, J = 8.3 Hz, 1H), 7.65 (s, 1H), 8.19 (d, J = 8.3 Hz, 1H), 8.36 (d, J = 1.6 Hz, 1H), 9.12 (s, 2H), 10.41 (s, 1H).

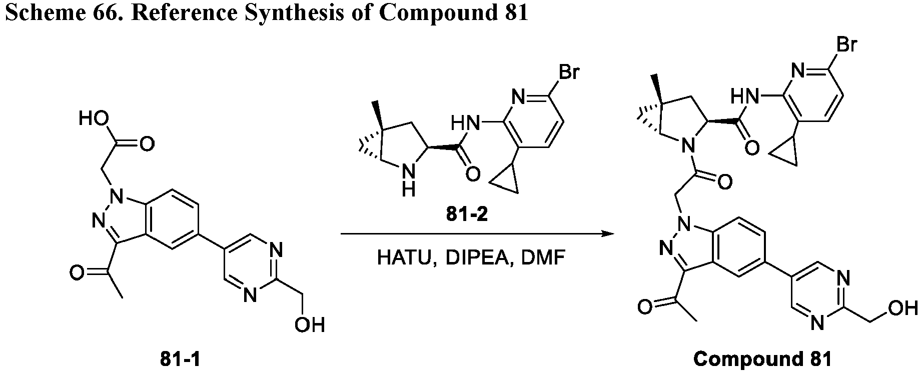

**(1R,3S,5R)-2-(2-(3-acetyl-5-(2-(hydroxymethyl)pyrimidin-5-yl)-1H-indazol-1-yl)acetyl)-N-(6-bromo-3-cyclopropylpyridin-2-yl)-5-methyl-2-azabicyclo[3.1.0]hexane-3-carboxamide (Compound 81):** To a solution of compound **81-1** (0.03 g, 0.092 mmol, 1 equiv.) in dimethylformamide (1 mL) at 0 °C under an atmosphere of argon were compound **81-2** (0.031 g, 0.092 mmol, 1 equiv.), HATU (0.073 g, 0.193 mmol, 2.1 equiv.), and DIPEA (0.059 g, 0.460 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound 81.** ¹H NMR (400 MHz, DMSO-d6) δ 0.78 - 0.89 (m, 2H), 0.95 - 1.00 (m, 1H), 1.01 - 1.07 (m, 2H), 1.24 - 1.30 (m, 2H), 1.34 (s, 3H), 2.08 (td, J = 4.2, 8.3 Hz, 1H), 2.19 (dd, J = 4.2, 13.4 Hz, 1H), 2.67 (s, 3H), 3.52 - 3.69 (m, 1H), 4.51 (dd, J = 4.2, 9.3 Hz, 1H), 4.69 (d, J = 6.3 Hz, 2H), 5.35 (t, J = 6.3 Hz, 1H), 5.58 (d, J = 17.2 Hz, 1H), 5.94 (d, J = 17.2 Hz, 1H), 7.12 (d, J = 8.2 Hz, 1H), 7.83 - 7.97 (m, 3H), 8.49 (d, J = 1.4 Hz, 1H), 9.15 (s, 2H), 10.02 (s, 1H).

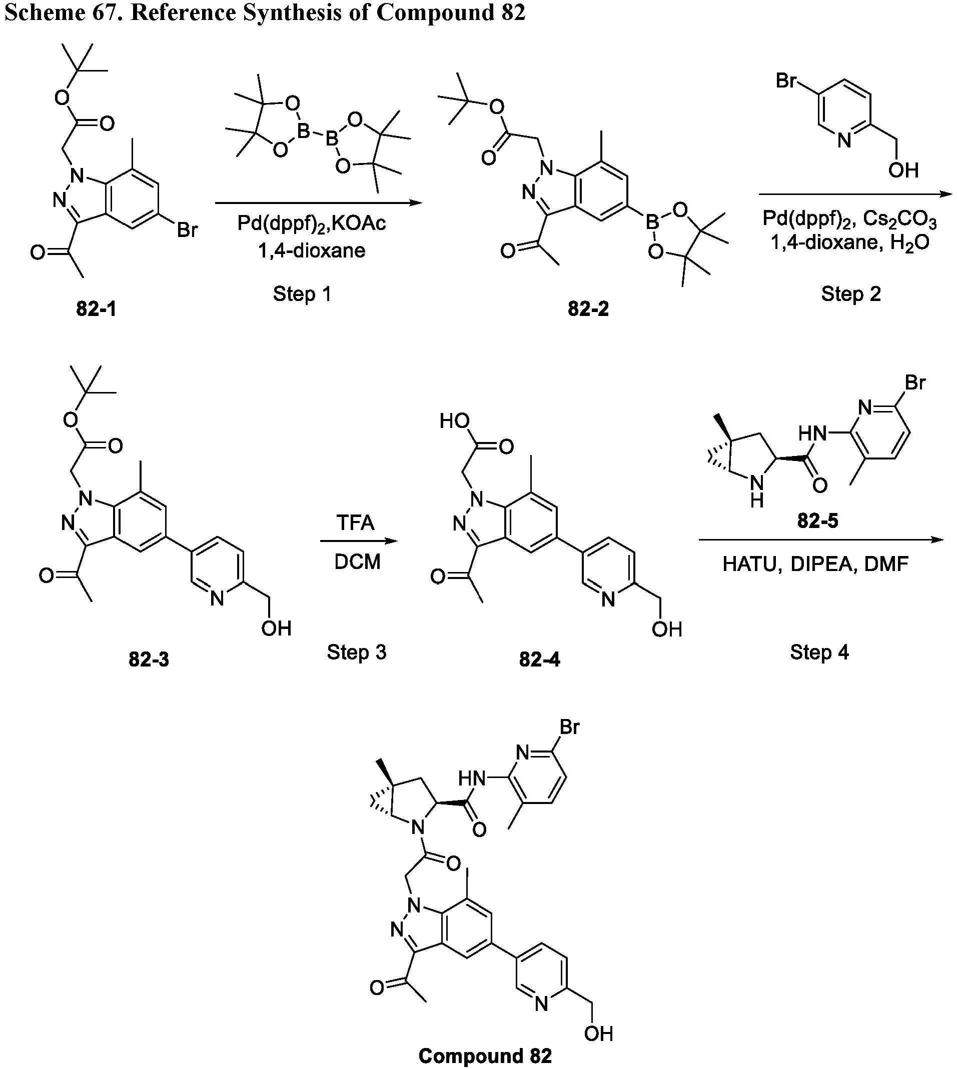

**Step 1: tert-butyl 2-(3-acetyl-7-methyl-5-(4,4,5,5-tetramethyl-1,3,2-dioxaborolan-2-yl)-1H-indazol-1-yl)acetate (82-2):** To a solution of compound **82-1** (0.2 g, 0.545 mmol, 1 equiv.) in 1,4-dioxane (5 mL) under an atmosphere of argon were added bis(pinacolato)diboron (0.138 g, 0.545 mmol, 1 equiv.), KOAc (0.107 g, 1.089 mmol, 2 equiv.) and [1,1'-bis(diphenylphosphino)ferrocene]dichloropalladium(II) (0.022 g, 0.027 mmol, 0.05 equiv.). The reaction mixture was stirred at 90 °C for 4 h and was used in the next synthetic step without purification.

**Step 2: tert-butyl 2-(3-acetyl-5-(6-(hydroxymethyl)pyridin-3-yl)-7-methyl-1H-indazol-1-yl)acetate (82-3):** To a solution of compound **82-2** (0.226 g, 0.545 mmol, 1 equiv.) in 1,4-dioxane (5 mL) and water (0.5 mL) were added compound (5-bromopyridin-2-yl)methanol (0.102 g, 0.545 mmol, 1 equiv.), cesium carbonate (0.355 g, 1.09 mmol, 2 equiv.), and [1,1'-bis(diphenylphosphino)ferrocene]dichloropalladium(II) (0.022 g, 0.027 mmol, 0.05 equiv.). The reaction mixture was stirred at 90 °C for 5 h and then concentrated to dryness. The residue was purified by column chromatography on silica gel (eluted with DCM/ MeOH) to give compound **82-3.**

**Step 3: 2-(3-acetyl-5-(6-(hydroxymethyl)pyridin-3-yl)-7-methyl-1H-indazol-1-yl)acetic acid (82-4):** To a solution of compound **82-3** (0.078 g, 0.231 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The material was used directly in the next synthetic step without purification.

**Step 4: (1R,3S,5R)-2-(2-(3-acetyl-5-(6-(hydroxymethyl)pyridin-3-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 82):** To a solution of compound **82-5** (0.06 g, 0.177 mmol, 1 equiv.) in dimethylformamide (1 mL) at 0 °C under an atmosphere of argon were added compound **82-4** (0.055 g, 0.177 mmol, 1 equiv.), HATU (0.141 g, 0.371 mmol, 2.1 equiv.), and DIPEA (0.114 g, 0.884 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The remaining residue was purified by column chromatography on silica gel (eluted with DCM/MeOH) to give **Compound 82.** ¹H NMR (400 MHz, DMSO-d6) δ 0.92 - 0.97 (m, 1H), 1.03 (t, J = 5.4 Hz, 1H), 1.33 (s, 3H), 1.99 - 2.09 (m, 4H), 2.54 - 2.62 (m, 1H), 2.65 (s, 3H), 2.69 (s, 3H), 3.60 (dd, J = 2.4, 5.5 Hz, 1H), 4.43 (dd, J = 5.4, 9.2 Hz, 1H), 4.63 (d, J = 5.8 Hz, 2H), 5.44 (t, J = 5.8 Hz, 1H), 5.69 (d, J = 17.7 Hz, 1H), 6.03 (d, J = 17.9 Hz, 1H), 7.44 (d, J = 7.9 Hz, 1H), 7.55 - 7.68 (m, 3H), 8.11 (dd, J = 2.4, 8.2 Hz, 1H), 8.30 (d, J = 1.6 Hz, 1H), 8.80 (d, J = 2.3 Hz, 1H), 10.30 (s, 1H).

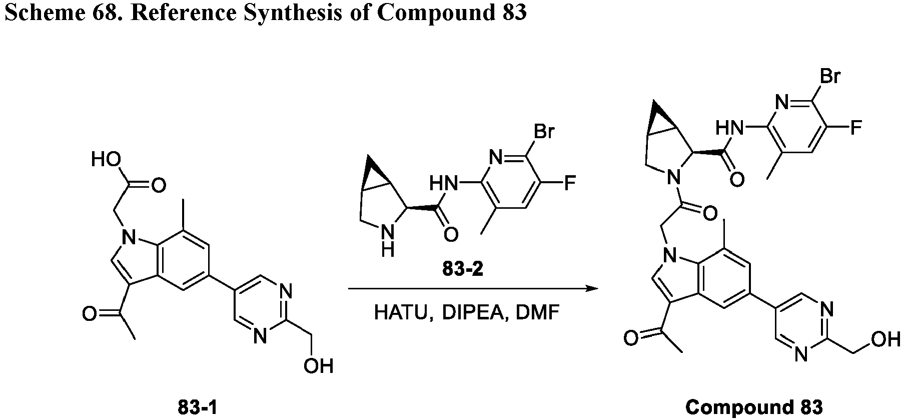

**(1R,2S,5S)-3-(2-(3-acetyl-5-(2-(hydroxymethyl)pyrimidin-5-yl)-7-methyl-1H-indol-1-yl)acetyl)-N-(6-bromo-5-fluoro-3-methylpyridin-2-yl)-3-azabicyclo[3.1.0]hexane-2-carboxamide (Compound 83):** To a solution of compound **83-1** (0.03 g, 0.088 mmol, 1 equiv.) in dimethylformamide (1 mL) at 0 °C under an atmosphere of argon were added compound **83-2** (0.028 g, 0.088 mmol, 1 equiv.), HATU (0.07 g, 0.185 mmol, 2.1 equiv.), and DIPEA (0.057 g, 0.441 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 83.** ¹H NMR (400 MHz, DMSO-d6) δ 0.72 - 0.85 (m, 1H), 1.87 - 1.96 (m, 1H), 2.08 (m, 4H), 2.44 - 2.46 (m, 3H), 2.62 (s, 3H), 3.28 (s, 1H), 3.83 (d, J = 9.8 Hz, 1H), 3.93 (dd, J = 5.2, 9.9 Hz, 1H), 4.64 (dd, J = 5.8, 19.4 Hz, 3H), 5.28 - 5.38 (m, 2H), 5.44 (d, J = 17.8 Hz, 1H), 7.34 (d, J = 1.8 Hz, 1H), 7.79 (d, J = 8.4 Hz, 1H), 8.21 (s, 1H), 8.37 (d, J = 1.8 Hz, 1H), 9.04 (s, 2H), 10.37 (s, 1H).

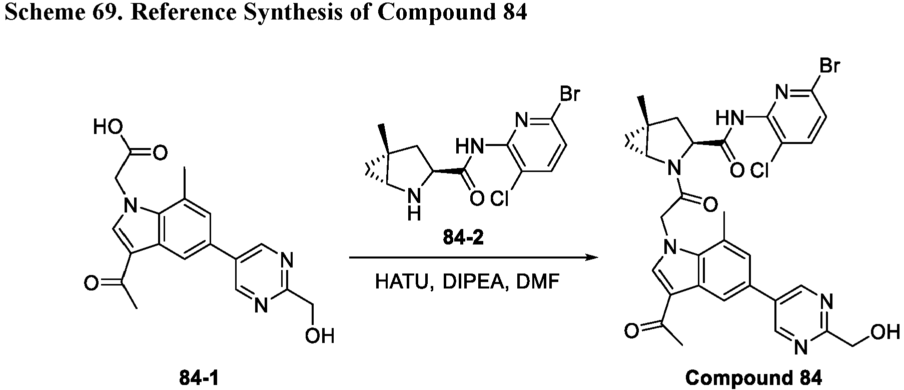

**(1R,3S,5R)-2-(2-(3-acetyl-5-(2-(hydroxymethyl)pyrimidin-5-yl)-7-methyl-1H-indol-1-yl)acetyl)-N-(6-bromo-3-chloropyridin-2-yl)-5-methyl-2-azabicyclo[3.1.0]hexane-3-carboxamide (Compound 84):** To a solution of compound **84-1** (0.033 g, 0.096 mmol, 1 equiv.) in dimethylformamide (1 mL) at 0 °C under an atmosphere of argon were added compound **84-2** (0.032 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 84.** ¹H NMR (400 MHz, DMSO-d6) δ 0.87 - 0.96 (m, 1H), 1.04 (t, J = 5.4 Hz, 1H), 1.34 (s, 3H), 2.13 - 2.19 (m, 1H), 2.46 (s, 3H), 2.53 - 2.56 (m, 1H), 2.66 (s, 3H), 3.54 (dd, J = 2.3, 5.4 Hz, 1H), 4.48 (dd, J = 5.1, 9.2 Hz, 1H), 4.67 (d, J = 6.3 Hz, 2H), 5.31 (d, J = 6.4 Hz, 1H), 5.42 (d, J = 17.9 Hz, 1H), 5.75 (d, J = 17.9 Hz, 1H), 7.34 - 7.41 (m, 2H), 8.18 (d, J = 8.3 Hz, 1H), 8.32 (s, 1H), 8.39 (d, J = 1.8 Hz, 1H), 9.05 (s, 2H), 10.39 (s, 1H).

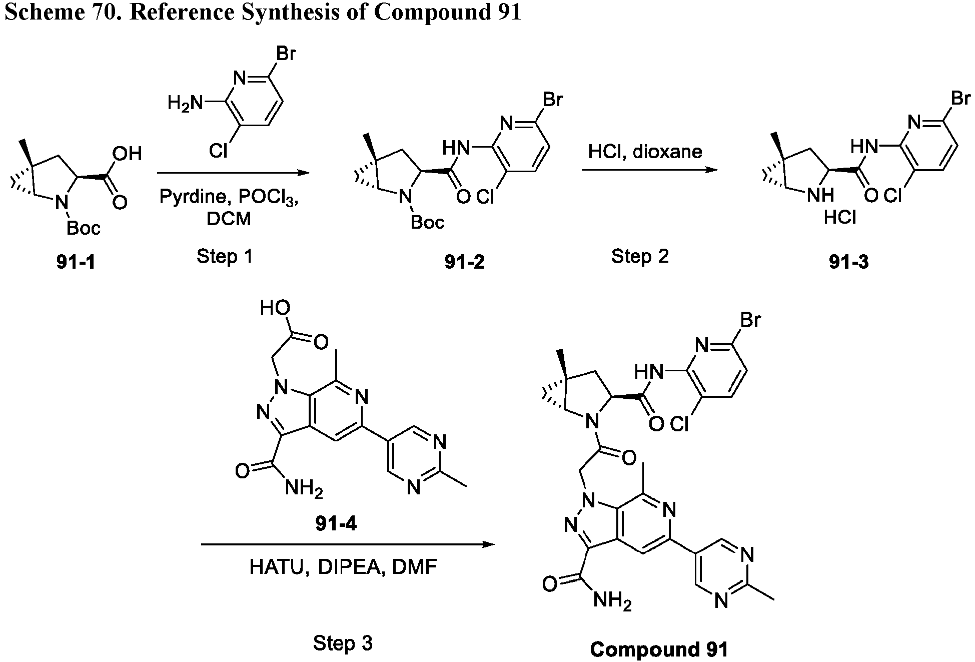

**Step** 1: **3-(6-Bromo-3-chloro-pyridin-2-ylcarbamoyl)-5-methyl-2-aza-bicyclo[3.1.0]hexane-2-carboxylic acid tert-butyl ester (91-2):** To a mixture of compound 91-1 (58 mg, 0.24 mmol) and 6-Bromo-3-chloro-pyridin-2-ylamine (50 mg, 0.24 mmol) in DCM (5 mL) was added pyridine (95 mg, 1.2 mmol) followed by phosphoryl chloride (40 mg, 0.26 mmol) at 0°C and the mixture was stirred at room temperature under N₂ atmosphere for 2 hrs. The mixture was poured into ice water and extracted with DCM twice. The combined organic layers were washed with brine, dried with anhydrous Na₂SO₄, filtered and concentrated to dryness. The residue was purified by chromatography on silica gel (PE: EtOAc= 30: 1 to 15: 1) to give compound 91-2 (80 mg, yield 77.7%) as a light oil. LC/MS (ESI) m/z: 430/432 (M+H)⁺.

Step 2: **5-Methyl-2-aza-bicyclo[3.1.0]hexane-3-carboxylic acid (6-bromo-3-chloro-pyridin-2-yl)-amide hydrochloride (91-3):** A solution of 4 M hydrogen chloride in 1,4-dioxane (3 mL) containing compound 91-2 (80 mg, 0.19 mmol) was stirred at room temperature for 1 hr. The reaction mixture was concentrated to dryness, washed with ether, and dried under vacuum to give compound 91-3 (60 mg, yield 86.0%) as a white solid. LC/MS (ESI) m/z: 330/332 (M+H)⁺.

**Step** 3: **1-{2-[3-(6-Bromo-3-chloro-pyridin-2-ylcarbamoyl)-5-methyl-2-aza-bicyclo[3.1.0]hex-2-yl]-2-oxo-ethyl}-7-methyl-5-(2-methyl-pyrimidin-5-yl)-1H-pyrazolo[3,4-c]pyridine-3-carboxylic acid amide (Compound 91):** To a mixture of compound 91-3 (22 mg, 0.061 mmol) and compound 91-4 (20 mg, 0.061 mmol) in DMF (3 mL) was added DIPEA (39 mg, 0.31 mmol) followed by HATU (35 mg, 0.092 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by prep-TLC (DCM: MeOH= 40: 1) to give **Compound 91** (8 mg, yield 20.5%) as white solid. ¹H-NMR (400 MHz, DMSO-*d*₆) δ 11.01 (s, 1H), 9.30 (s, 2H), 8.47 (s, 1H), 8.07 - 8.00 (m, 2H), 7.89 (s, 1H), 7.61 (s, 1H), 6.08 (d, *J=* 18.0 Hz, 1H), 5.67 (d, *J* = 18.0 Hz, 1H), 4.41 (dd, *J =* 9.2, 9.2 Hz, 1H), 3.66 (dd, *J =* 5.6, 5.6 Hz, 1H), 3.20 - 3.11 (m, 1H), 2.90 (s, 3H), 2.68 (s, 3H), 2.00 (dd, *J =* 13.2, 13.2 Hz, 1H), 1.31 (s, 3H), 1.00 (t, *J=* 5.4 Hz, 1H), 0.88 (dd, *J=* 5.6, 5.6 Hz, 1H). LC/MS (ESI) m/z: 638/640 (M+H)⁺.

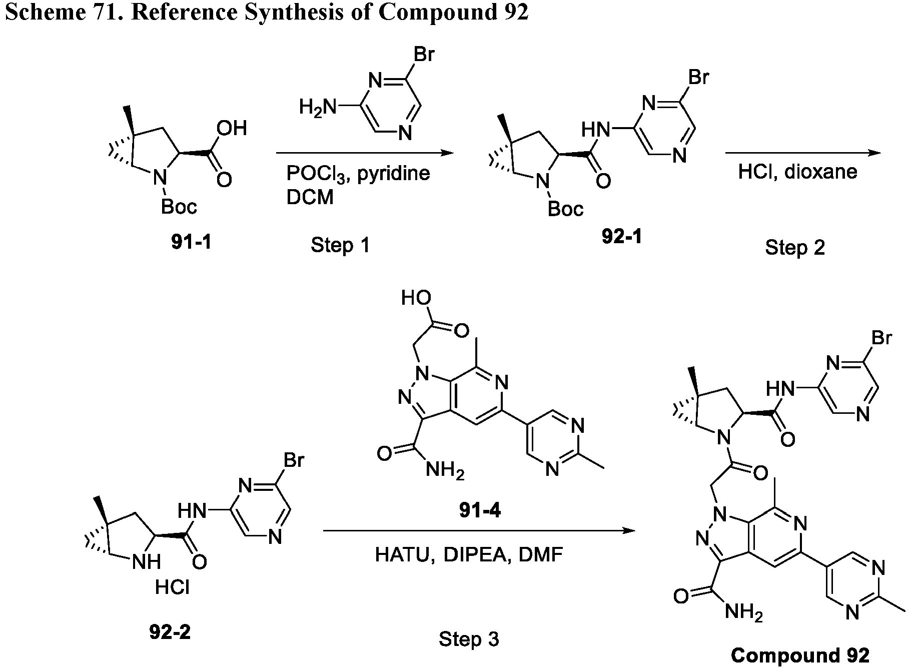

**Step** 1: **3-(6-Bromo-pyrazin-2-ylcarbamoyl)-5-methyl-2-aza-bicyclo[3.1.0]hexane-2-carboxylic acid tert-butyl ester (92-1):** To a mixture of compound 91-1 (70 mg, 0.29 mmol) and 6-Bromo-pyrazin-2-ylamine (50 mg, 0.29 mmol) in DCM (5 mL) was added pyridine (115 mg, 1.45 mmol) followed by phosphoryl chloride (49 mg, 0.32 mmol) at 0°C, and the mixture was stirred at room temperature under N₂ atmosphere for 1 hr. The mixture was poured into ice water and extracted with DCM twice. The combined organic layers were washed with brine, dried with anhydrous Na₂SO₄, filtered, and concentrated to dryness. The residue was purified by chromatography on silica gel (PE: EtOAc= 30: 1 to 12: 1) to give compound 92-1 (110 mg, yield 96.5%) as a light yellow solid. LC/MS (ESI) m/z: 397/399 (M+H)⁺.

**Step 2: 5-Methyl-2-aza-bicyclo[3.1.0]hexane-3-carboxylic acid (6-bromo-pyrazin-2-yl)-amide hydrochloride (92-2):** A solution of 4 M hydrogen chloride in 1,4-dioxane (5 mL) containing compound **92-1** (110 mg, 0.28 mmol) was stirred at room temperature for 1 hr. The reaction mixture was concentrated to dryness, washed with ether, and dried under vacuum to give compound **92-2** (80 mg, yield 85.6%) as a white solid. LC/MS (ESI) m/z: 297/299 (M+H)⁺. **Step 3: 1-{2-[3-(6-Bromo-pyrazin-2-ylcarbamoyl)-5-methyl-2-aza-bicyclo[3.1.0]hex-2-yl]-2-oxo-ethyl}-7-methyl-5-(2-methyl-pyrimidin-5-yl)-1H-pyrazolo[3,4-c]pyridine-3-carboxylic acid amide (Compound 92):** To a mixture of compound **92-2** (20 mg, 0.067 mmol) and **91-4** (22 mg, 0.067 mmol) in DMF (3 mL) was added DIPEA (44 mg, 0.34 mmol) followed by HATU (38 mg, 0.10 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by prep-TLC (DCM: MeOH= 40: 1) to give **Compound 92** (6 mg, yield 15%) as a white solid. ¹H-NMR (400 MHz, DMSO-*d*₆) δ 11.20 (s, 1H), 9.30 (s, 2H), 9.27 (s, 1H), 8.54 (s, 1H), 8.47 (s, 1H), 7.89 (s, 1H), 7.61 (s, 1H), 6.08 (d, *J =* 18.0 Hz, 1H), 5.69 (d, *J =* 18.0 Hz, 1H), 4.44 (dd, *J =* 9.2, 9.2 Hz, 1H), 3.71 - 3.64 (m, 3H), 3.15 - 3.14 (m, 1H), 2.91 (s, 3H), 2.68 (s, 3H), 2.04 (dd, *J =* 13.2, 6.2 Hz, 1H), 1.32 (s, 3H), 1.01 (t, *J =* 5.4 Hz, 1H), 0.92 - 0.89 (m, 1H). LC/MS (ESI) m/z: 605/607 (M+H)⁺.

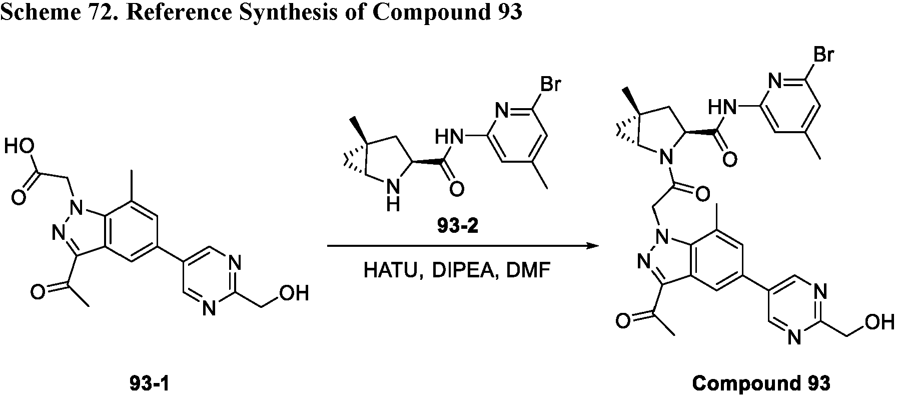

**(1R,3S,5R)-2-(2-(3-acetyl-5-(2-(hydroxymethyl)pyrimidin-5-yl)-7-methyl-1H-indazol-1-yl)acetyl)-N-(6-bromo-4-methylpyridin-2-yl)-5-methyl-2-azabicyclo[3.1.0]hexane-3-carboxamide (Compound 93):** To a solution of compound **93-1** (0.03 g, 0.088 mmol, 1 equiv.) in dimethylformamide (1 mL) at 0 °C under an atmosphere of argon were added compound **93-2** (0.027 g, 0.088 mmol, 1 equiv.), HATU (0.07 g, 0.185 mmol, 2.1 equiv.), and DIPEA (0.057 g, 0.441 mmol, 5 equiv.). The reaction mixture was stirred at room temperature for 1 h and then quenched with water (10 vol). The resulting mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound 93.** ¹H NMR (400 MHz, DMSO-d6) δ 0.85 - 0.93 (m, 1H), 1.00 (t, J = 5.5 Hz, 1H), 1.31 (s, 3H), 1.99 (dd, J = 6.0, 13.2 Hz, 1H), 2.29 (s, 3H), 2.45 - 2.49 (m, 1H), 2.65 (s, 3H), 2.72 (s, 3H), 3.60 - 3.70 (m, 1H), 4.43 (dd, J = 5.9, 9.0 Hz, 1H), 4.68 (d, J = 6.3 Hz, 2H), 5.33 (t, J = 6.3 Hz, 1H), 5.67 (d, J = 17.9 Hz, 1H), 6.07 (d, J = 17.9 Hz, 1H), 7.20 (s, 1H), 7.66 (s, 1H), 7.89 (s, 1H), 8.26 - 8.47 (m, 1H), 9.11 (s, 2H), 10.69 (s, 1H).

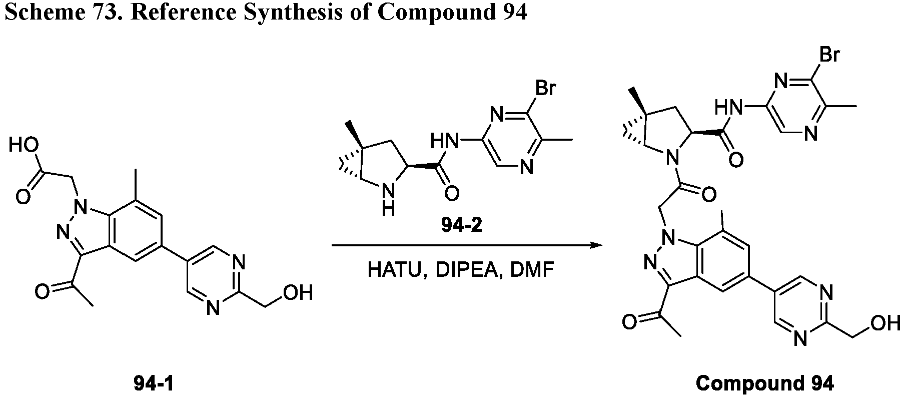

**(1R,3S,5R)-2-(2-(3-acetyl-5-(2-(hydroxymethyl)pyrimidin-5-yl)-7-methyl-1H-indazol-1-yl)acetyl)-N-(6-bromo-5-methylpyrazin-2-yl)-5-methyl-2-azabicyclo[3.1.0]hexane-3-carboxamide (Compound 94):** To a solution of compound **94-1** (0.033 g, 0.096 mmol, 1 equiv.) in dimethylformamide (1 mL) at 0 °C under an atmosphere of argon were added compound **94-2** (0.03 g, 0.096 mmol, 1 equiv.), HATU (0.07 g, 0.202 mmol, 2.1 equiv.), and DIPEA (0.062 g, 0.480 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 94.** ¹H NMR (400 MHz, DMSO-d6) δ 0.89 - 0.95 (m, 1H), 1.00 (t, J = 5.4 Hz, 1H), 1.32 (s, 3H), 2.03 (dd, J = 6.0, 13.2 Hz, 1H), 2.46 - 2.52 (m, 1H), 2.54 (s, 3H), 2.65 (s, 3H), 2.71 (s, 3H), 3.65 (dd, J = 2.4, 5.4 Hz, 1H), 4.44 (dd, J = 6.0, 9.1 Hz, 1H), 4.68 (s, 2H), 5.68 (d, J = 17.9 Hz, 1H), 6.08 (d, J = 17.9 Hz, 1H), 7.65 (s, 1H), 8.34 (d, J = 1.7 Hz, 1H), 9.00 - 9.19 (m, 3H), 11.03 (s, 1H).

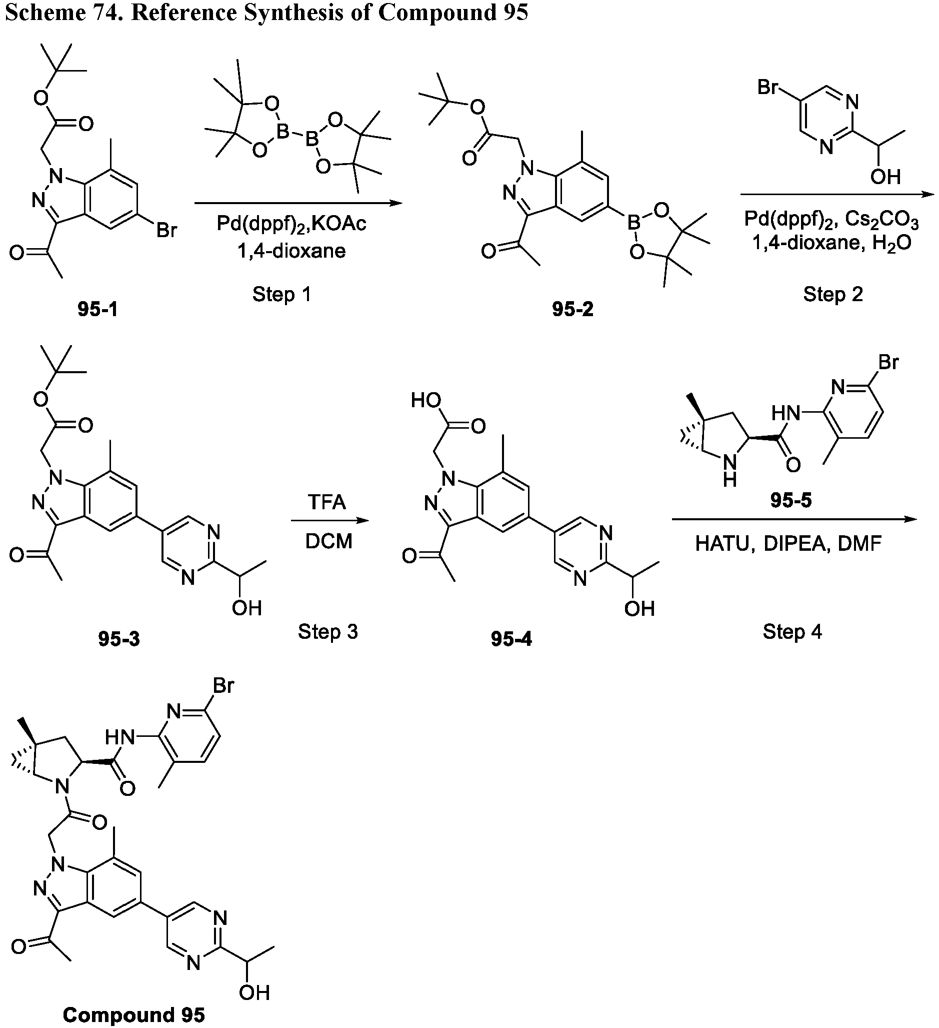

**Step 1: tert-butyl 2-(3-acetyl-7-methyl-5-(4,4,5,5-tetramethyl-1,3,2-dioxaborolan-2-yl)-1H-indazol-1-yl)acetate (95-2):** To a solution of compound **95-1** (0.15 g, 0.408 mmol, 1 equiv.) in 1,4-dioxane (3 mL) under an atmosphere of argon were added bis(pinacolato)diboron (0.104 g, 0.408 mmol, 1 equiv.), KOAc (0.08 g, 0.817 mmol, 2 equiv.) and [1,1'-bis(diphenylphosphino)ferrocene]dichloropalladium(II) (0.017 g, 0.02 mmol, 0.05 equiv.).The reaction mixture was stirred at 90 °C for 4 h and was used in the next synthetic step without further purification.

**Step 2: tert-butyl 2-(3-acetyl-5-(2-(1-hydroxyethyl)pyrimidin-5-yl)-7-methyl-1H-indazol-1-yl)acetate (95-3):** To a solution of compound **95-2** (0.169 g, 0.408 mmol, 1 equiv.) in 1,4-dioxane (3 mL) and water (0.3 mL) were added 1-(5-bromopyrimidin-2-yl)ethanol (0.083 g, 0.408 mmol, 1 equiv.), cesium carbonate (0.266 g, 0.816 mmol, 2 equiv.), and [1,1'-bis(diphenylphosphino)ferrocene]dichloropalladium(II) (0.017 g, 0.02 mmol, 0.05 equiv.). The reaction mixture was stirred at 90 °C for 5 h and then concentrated to dryness. The residue was purified by column chromatography on silica gel (eluted with DCM/ MeOH) to give compound **95-3.**

**Step 3: 2-(3-acetyl-5-(2-(1-hydroxyethyl)pyrimidin-5-yl)-7-methyl-1H-indazol-1-yl)acetic acid (95-4):** To a solution of compound **95-3** (0.095 g, 0.231 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The material was used directly in the next synthetic step without purification.

**Step 4: (1R,3S,5R)-2-(2-(3-acetyl-5-(2-(1-hydroxyethyl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 95):** To a solution of compound **95-5** (0.06 g, 0.169 mmol, 1 equiv.) in dimethylformamide (1 mL) at 0 °C under an atmosphere of argon were added compound **95-4** (0.053 g, 0.126 mmol, 1 equiv.), HATU (0.135 g, 0.356 mmol, 2.1 equiv.) and DIPEA, (0.109 g, 0.847 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The remaining residue was purified by column chromatography on silica gel (eluted with DCM/MeOH) to give **Compound 95.** ¹H NMR (400 MHz, DMSO-d6) δ 0.92 - 0.96 (m, 1H), 1.04 (d, J = 5.6 Hz, 1H), 1.33 (s, 3H), 1.47 (d, J = 6.6 Hz, 3H), 2.06 (s, 4H), 2.54 - 2.62 (m, 1H), 2.65 (s, 3H), 2.70 (s, 3H), 3.54 - 3.67 (m, 1H), 4.42 (dd, J = 5.4, 9.2 Hz, 1H), 4.86 (p, J = 6.4 Hz, 1H), 5.26 (d, J = 5.6 Hz, 1H), 5.69 (d, J = 17.8 Hz, 1H), 6.05 (d, J = 17.9 Hz, 1H), 7.44 (d, J = 7.9 Hz, 1H), 7.63 (d, J = 8.5 Hz, 2H), 8.35 (d, J = 1.6 Hz, 1H), 9.12 (s, 2H), 10.30 (s, 1H).

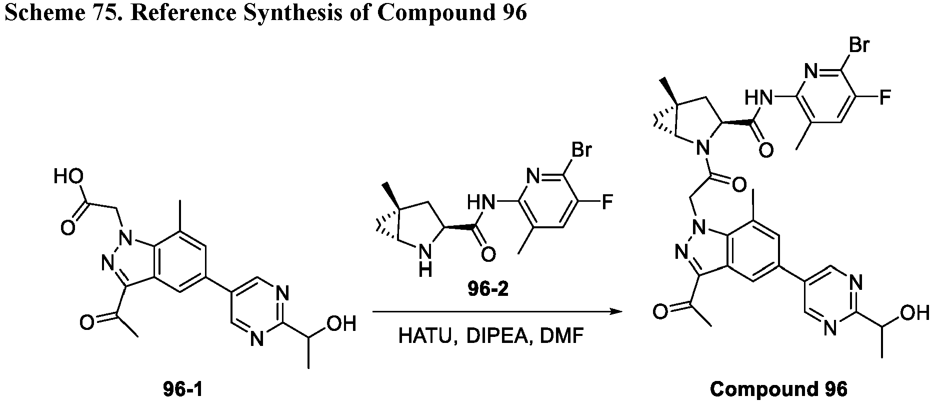

**(1R,3S,5R)-2-(2-(3-acetyl-5-(2-(1-hydroxyethyl)pyrimidin-5-yl)-7-methyl-1H-indazol-1-yl)acetyl)-N-(6-bromo-5-fluoro-3-methylpyridin-2-yl)-5-methyl-2-azabicyclo[3.1.0]hexane-3-carboxamide (Compound 96):** To a solution of compound **96-1** (0.034 g, 0.096 mmol, 1 equiv.) in dimethylformamide (1 mL) at 0 °C under an atmosphere of argon were added compound **96-2** (0.032 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 96.** ¹H NMR (400 MHz, DMSO-d6) δ 0.92 - 0.98 (m, 1H), 1.03 (t, J = 5.4 Hz, 1H), 1.33 (s, 3H), 1.47 (d, J = 6.6 Hz, 3H), 2.01 - 2.10 (m, 4H), 2.57 (dd, J = 9.2, 13.3 Hz, 1H), 2.65 (s, 3H), 2.69 (s, 3H), 3.57 - 3.67 (m, 1H), 4.40 (dd, J = 5.5, 9.2 Hz, 1H), 4.86 (p, J = 6.4 Hz, 1H), 5.26 (d, J = 5.6 Hz, 1H), 5.70 (d, J = 17.8 Hz, 1H), 6.04 (d, J = 17.9 Hz, 1H), 7.64 (s, 1H), 7.83 (d, J = 8.4 Hz, 1H), 8.35 (d, J = 1.6 Hz, 1H), 9.12 (s, 2H), 10.31 (s, 1H).

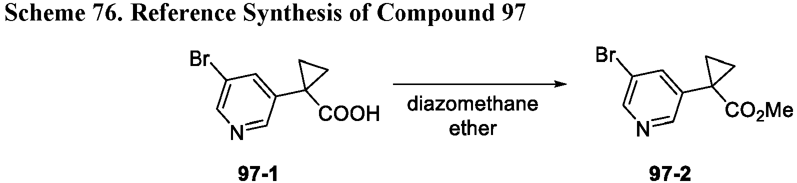

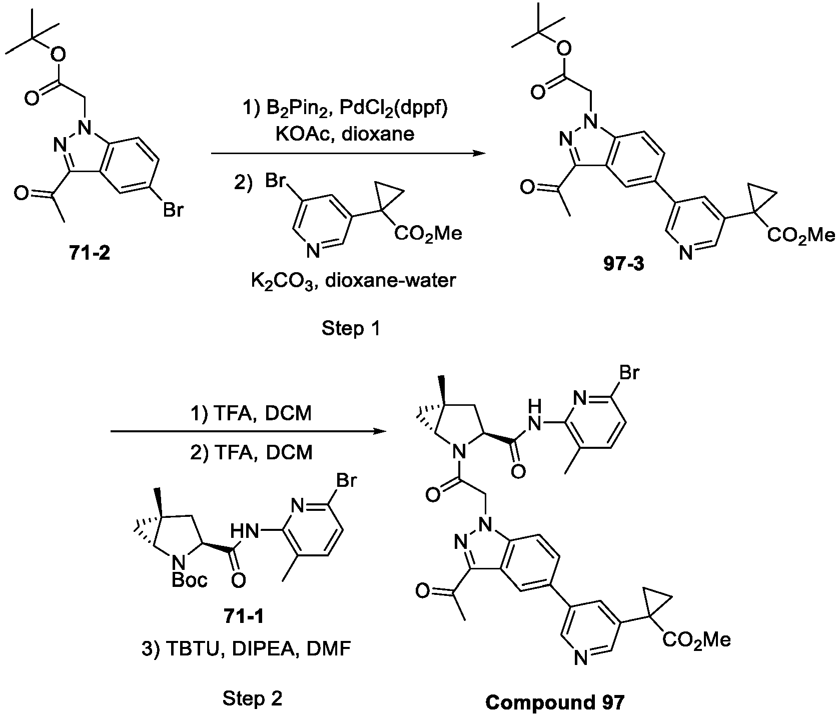

**Step 1: Methyl 1-(5-(3-acetyl-1-(2-(tert-butoxy)-2-oxoethyl)-1H-indazol-5-yl)pyridin-3-yl)cyclopropane-1-carboxylate (97-3):** Compound **97-1** (from Enamine) was esterified with freshly prepared diazomethane (from Diazald) in ether. A heterogeneous mixture of compound **71-2** (0.152 g, 0.43 mmol, 1.1 equiv.), bis(pinacolato)diboron (0.149 g, 0.586 mmol, 1.5 equiv.), and potassium acetate (0.077 g, 0.781 mmol, 2 equiv.) in dioxane (5 mL) was degassed and (0.016 g, 0.02 mmol, 0.05 equiv.) was added. The resulting mixture was heated at 90 ° C overnight. The reaction mixture was cooled to rt and compound **97-2** (0.1 g, 0.39 mmol, 1 equiv.), dissolved in 1 mL of 1,4-dioxane, was added to the reaction mixture followed by potassium carbonate (0.162 g, 1.171 mmol, 3 equiv.) and water (1 mL), and the mixture was degassed with argon. The reaction mixture was heated at 100 °C for 3 h, then cooled to room temperature. Solids were filtered off and washed thoroughly with dioxane. The filtrate was concentrated under reduced pressure and purified by silica gel flash column chromatography (eluent: 0-2 % MeOH in dichloromethane) to give **compound 97-3** as a light-yellow resin.

**Step 2: Methyl 1-(5-(3-acetyl-1-(2-((1R,3S,5R)-3-((6-bromo-3-methylpyridin-2-yl)carbamoyl)-5-methyl-2-azabicyclo[3.1.0]hexan-2-yl)-2-oxoethyl)-1*H*-indazol-5-yl)pyridin-3-yl)cyclopropane-1-carboxylate (Compound 97):** Compound **97-3** (0.06 g, 0.133 mmol, 1 equiv.) was stirred with 2 mL of TFA and 1 mL of dichloromethane for 3 h at rt in a vial. In another vial, compound **71-1** (0.055 g, 0.133 mmol, 1 equiv.) was stirred with 1 mL of TFA and 1 mL of dichloromethane for 30 min at rt. The contents of both the vials were combined and the volatiles were removed under reduced pressure. The residue was dissolved in DMF (2 mL) and cooled in an ice bath. DIPEA (0.116 mL, 0.667 mmol, 5 equiv.) and TBTU (0.047 g, 0.147 mmol, 1.1 equiv.) were added successively and the cooling bath was removed. The reaction mixture was stirred at rt for 30 min and the solvent was removed under reduced pressure. The residue was dissolved in dichloromethane and washed with saturated aqueous sodium bicarbonate solution. The organic layer was dried (Na₂SO₄) and concentrated, and the crude product was purified by silica gel flash column chromatography (eluent: 0-3 % MeOH in dichloromethane) to give **Compound 97** as a white solid. ¹H NMR (400 MHz, Chloroform-d) δ 0.91 (dd, *J =* 2.4, 5.4 Hz, 1H), 1.15 (t, *J =* 5.5, 5.5 Hz, 1H), 1.29 (q, *J =* 4.2 Hz, 2H), 1.41 (s, 3H), 1.72 (q, *J=* 4.1 Hz, 2H), 2.06 (s, 3H), 2.29 (dd, *J =* 8.7, 13.5 Hz, 1H), 2.72 (s, 4H), 3.18 (dd, *J =* 2.3, 5.5 Hz, 1H), 3.67 (s, 3H), 4.84 (dd, *J =* 3.1, 8.9 Hz, 1H), 5.50 (s, 2H), 7.23 (d, *J=* 7.9 Hz, 1H), 7.34 (d, *J =* 7.9 Hz, 1H), 7.57 (d, *J =* 8.7 Hz, 1H), 7.72 (dd, *J =* 1.7, 8.7 Hz, 1H), 7.90 (t, *J = 2.2* Hz, 1H), 8.58 (d, *J =* 1.8 Hz, 2H), 8.67 (s, 1H), 8.78 (d, *J=* 2.2 Hz, 1H).

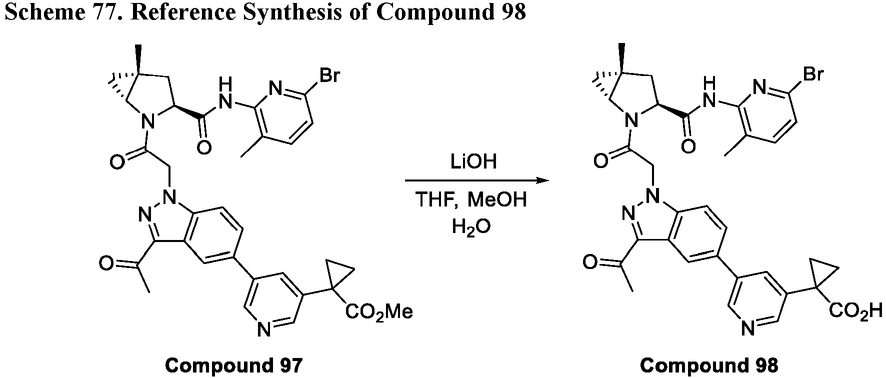

**1-(5-(3-acetyl-1-(2-((1R,3S,5R)-3-((6-bromo-3-methylpyridin-2-yl)carbamoyl)-5-methyl-2-azabicyclo[3.1.0]hexan-2-yl)-2-oxoethyl)-1H-indazol-5-yl)pyridin-3-yl)cyclopropane-1-carboxylic acid (Compound 98):** To a solution of **Compound** 97 (0.065 g, 0.095 mmol, 1 equiv.) in THF (1 mL) and methanol (0.5 mL) was added a solution of LiOH hydrate (0.006 g, 0.143 mmol, 1.5 equiv.) in water (0.5 mL). The reaction mixture was stirred overnight at rt. The organic solvents were removed under reduced pressure, and the aqueous phase was diluted with water and extracted with ether. The ether extract was discarded. The aqueous phase was cooled in an ice-bath and acidified with cold 2N aqueous HCl. Some white solid separated, which then dissolved upon adding more acid. This reaction mixture was then purified by reverse phase HPLC (Gilson) to give **Compound 98** as a white solid.

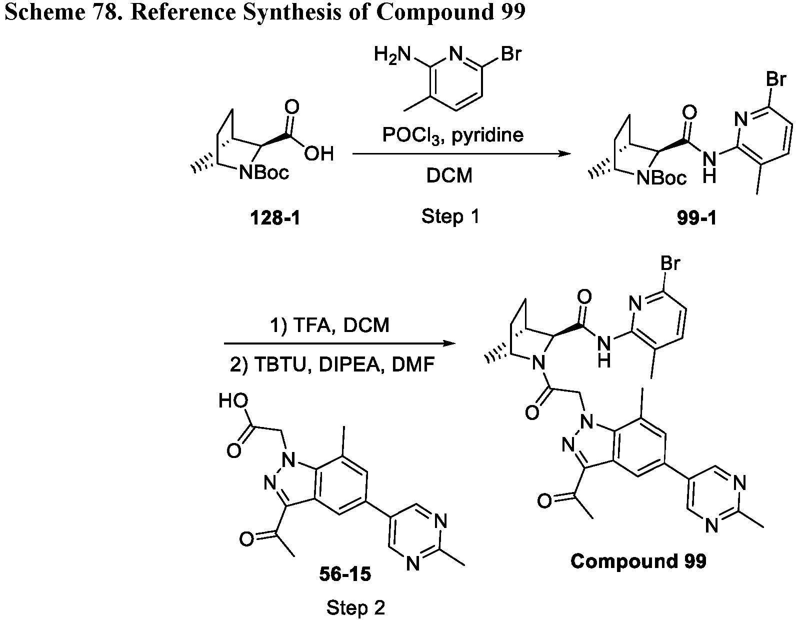

**Step 1. tert-Butyl (1S,3S,4R)-3-((6-bromo-3-methylpyridin-2-yl)carbamoyl)-2-azabicyclo[2.2.1]heptane-2-carboxylate (99-1):** To an ice-cooled solution of compound **128-1** (0.15 g, 0.622 mmol, 1 equiv.), 6-bromo-3-methylpyridin-2-amine (0.128 g, 0.684 mmol, 1.1 equiv.), and pyridine (0.246 g, 0.251 mL, 3.108 mmol, 5 equiv.) in dichloromethane (3 mL) was added phosphorous oxychloride (0.095 g, 0.056 mL, 0.622 mmol, 1 equiv.) dropwise. The solution was stirred at 0 °C for 30 min and quenched by the careful addition of saturated aqueous sodium bicarbonate. The reaction mixture was stirred at rt for 30 min, diluted with dichloromethane, and washed repeatedly with cold 1N aqueous HCl. Finally, the organic layer was washed with water, dried (Na₂SO₄), and concentrated to give 0.25 g of slightly reddish white solid, which was used in the next step without further purification.

**Step 2. (1S,3S,4R)-2-(2-(3-Acetyl-7-methyl-5-(2-methylpyrimidin-5-yl)-1H-indazol-1-yl)acetyl)-N-(6-bromo-3-methylpyridin-2-yl)-2-azabicyclo[2.2.1]heptane-3-carboxamide (Compound 99):** Compound **99-1** (0.06 g, 0.146 mmol, 1 equiv.) was stirred at rt with 1 mL of TFA and 1 mL of dichloromethane for 30 min. The volatiles were removed under reduced pressure, the residue was dissolved in DMF (1 mL), and compound **56-15** (0.047 g, 0.146 mmol, 1 equiv.) was added to this solution followed by the addition of DIPEA (0.094 g, 0.127 mL, 0.731 mmol, 5 equiv.). The reaction mixture was cooled in an ice-bath and TBTU (0.052 g, 0.161 mmol, 1.1 equiv.) was added. The cooling bath was removed and the reaction mixture was stirred at rt for 30 min. The solvent was removed under reduced pressure, and the residue was dissolved in dichloromethane then washed with saturated aqueous sodium bicarbonate solution. Finally, the organic layer was dried (Na₂SO₄) and concentrated. The residue was purified by silica gel flash column chromatography (eluent: 0-3 % EtOAc in dichloromethane) to give **Compound 99** as a cream colored solid. ¹H NMR (400 MHz, DMSO-*d*₆) δ1.58-1.82 (m, 6H), 2.05 (s, 3H), 2.63 (s, 3H), 2.67 (s, 3H), 2.72 (s, 3H), 2.88 (s, 1H), 4.40 (d, *J =* 3.6 Hz, 1H), 4.60 (s, 1H), 5.70 (d, *J =* 17.6 Hz, 1H), 5.83 (d, *J =* 17.6 Hz, 1H), 7.41 (d, *J =* 7.9 Hz, 1H), 7.48 - 7.64 (m, 2H), 8.29 (s, 1H), 9.01 (s, 2H), 10.32 (s, 1H).

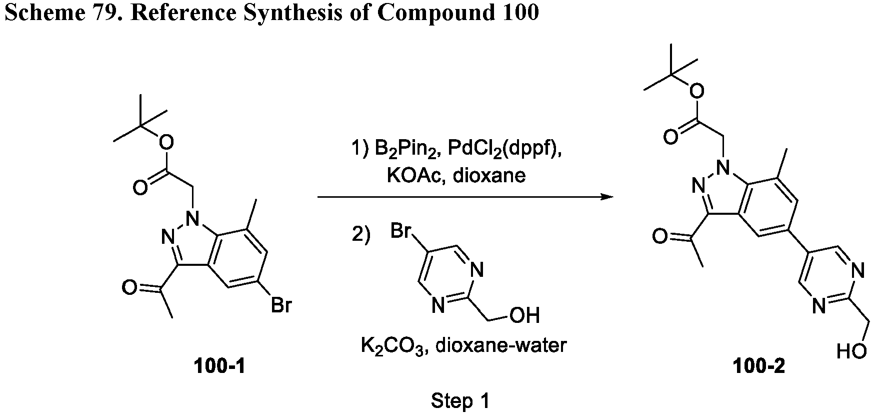

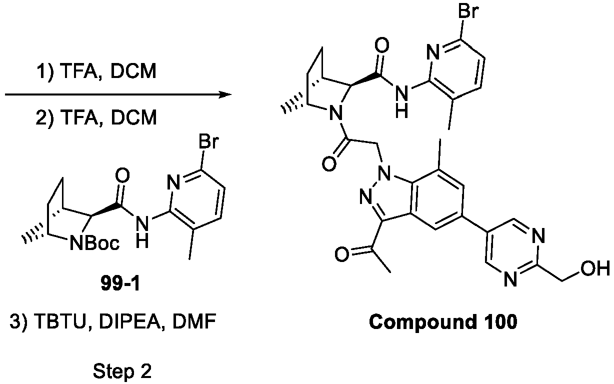

**Step 1. tert-Butyl 2-(3-acetyl-5-(2-(hydroxymethyl)pyrimidin-5-yl)-7-methyl-1H-indazol-1-yl)acetate (100-2):** A heterogeneous solution of compound **100-1** (0.1 g, 0.272 mmol, 1 equiv.), bis(pinacolato)diboron (0.104 g, 0.408 mmol, 1.5 equiv.), PdCl₂(dppf) (0.011 g, 0.014 mmol, 0.05 equiv.), and KOAc (0.053 g, 0.545 mmol, 2 equiv.) in 1,4-dioxane (4 mL) was degassed with argon and heated at 90° C overnight with vigorous stirring. The resulting black heterogenous mixture was cooled to rt and (5-bromopyrimidin-2-yl)methanol (0.051 g, 0.3 mmol, 1.1 equiv.), potassium carbonate (0.113 g, 0.817 mmol, 3 equiv.), and water (1 mL) were added to the reaction mixture. The reaction mixture was again degassed with argon and heated at 100 °C for 4 h. Then the reaction mixture was cooled to rt, and the solvent was removed under reduced pressure. The residue was purified by silica gel flash column chromatography (eluent:0-1 % MeOH in dichloromethane) to give compound **100-2** as a colorless solid.

**Step 2. (1S,3S,4R)-2-(2-(3-acetyl-5-(2-(hydroxymethyl)pyrimidin-5-yl)-7-methyl-1H-indazol-1-yl)acetyl)-N-(6-bromo-3-methylpyridin-2-yl)-2-azabicyclo[2.2.1]heptane-3-carboxamide (Compound 100):** Compound **100-2** (0.046 g, 0.117 mmol, 1 equiv.) was stirred with 1 mL of TFA and 0.5 mL of dichloromethane for 3 h at rt in a vial. In another vial, compound 99-1 (0.048 g, 0.117 mmol, 1 equiv.) was stirred with 1 mL of TFA and 1 mL of dichloromethane for 30 min at rt. The contents of both the vials were combined, and the volatiles were removed under reduced pressure. The residue was dissolved in DMF (2 mL) and cooled in an ice bath. DIPEA (0.076 g, 0.102 mL, 0.587 mmol, 5 equiv.), and TBTU (0.042 g, 0.131 mmol, 1.113 equiv.) were added successively and the cooling bath was removed, and the reaction mixture was stirred at rt for 30 min. The solvent was removed under reduced pressure, and the residue was dissolved in dichloromethane then washed with saturated aqueous sodium bicarbonate. The organic layer was dried (Na₂SO₄) and concentrated, and the crude product was purified by silica gel flash column chromatography (eluent: 0-2.5 % MeOH in dichloromethane) **to** give **Compound** 100 as a white solid. ¹H NMR (400 MHz, Chloroform-d) δ 1.58-1.88 (m, 6H), 2.12 (s, 3H), 2.70 (s, 3H), 2.81 (s, 3H), 3.12 (s, 1H), 3.71 (t, *J=* 5.1 Hz, 1H), 4.45 (s, 1H), 4.73 (s, 1H), 4.91 (d, *J=* 4.9 Hz, 2H), 5.46 - 5.61 (m, 2H), 7.19 (d, *J=* 7.9 Hz, 1H), 7.33-7.34 (m, 2H), 8.05 (s, 1H), 8.45 (s, 1H), 8.97 (s, 2H).

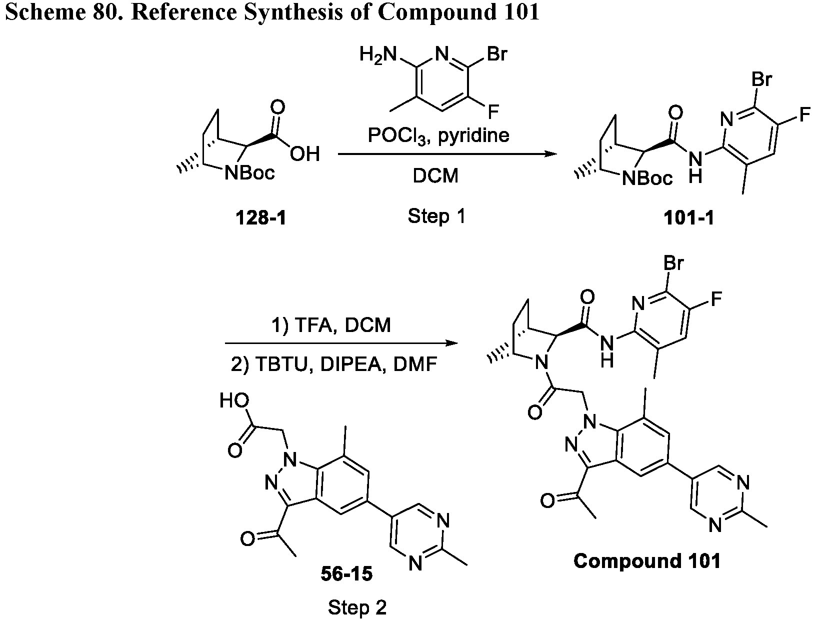

**Step 1. tert-Butyl (1S,3S,4R)-3-((6-bromo-5-fluoro-3-methylpyridin-2-yl)carbamoyl)-2-azabicyclo[2.2.1]heptane-2-carboxylate (101-1):** To an ice-cooled solution of compound 128-1 (0.1 g, 0.414 mmol, 1 equiv.), 6-bromo-5-fluoro-3-methylpyridin-2-amine (0.085 g, 0.414 mmol, 1 equiv.), and pyridine (0.168 mL, 2.072 mmol, 5 equiv.) in dichloromethane (2 mL) was added phosphorous oxychloride (0.037 mL, 1 equiv.) dropwise. The solution was stirred at 0 °C for 30 min and quenched by the careful addition of saturated aqueous sodium bicarbonate. The reaction mixture was stirred at room temperature for 30 min, diluted with dichloromethane, and washed repeatedly with cold 1N aqueous HCl. Finally, the organic layer was washed with water, dried (Na₂SO₄), and concentrated to give 0.14 g of a slightly brown solid, which was used in the next step without further purification.

**Step 2. (1S,3S,4R)-2-(2-(3-Acetyl-7-methyl-5-(2-methylpyrimidin-5-yl)-1H-indazol-1-yl)acetyl)-N-(6-bromo-5-fluoro-3-methylpyridin-2-yl)-2-azabicyclo[2.2.1]heptane-3-carboxamide (Compound 101):** Compound **101-1** (*tert*-Butyl (1S,3S,4R)-3-((6-bromo-5-fluoro-3-methylpyridin-2-yl)carbamoyl)-2-azabicyclo[2.2.1]heptane-2-carboxylate, 0.07 g, 0.163 mmol, 1 equiv.) was stirred at rt with 1 mL of TFA and 1 mL of dichloromethane for 30 min in a vial. The volatiles were removed under reduced pressure. The residue was dissolved in DMF and compound **56-15** (0.053 g, 0.163 mmol, 1 equiv.) was added to this solution followed by the addition of DIPEA (0.106 g, 0.142 mL, 0.817 mmol, 5 equiv.). The reaction mixture was cooled in an ice-bath and TBTU (0.058 g, 0.18 mmol, 1.1 equiv.) was added, then the cooling bath was removed and the reaction mixture was stirred at rt for 30 min. The solvent was removed under reduced pressure. The residue was dissolved in dichloromethane and washed with saturated aqueous sodium bicarbonate. Finally, the organic layer was dried (Na₂SO₄) and concentrated. The residue was purified by silica gel flash column chromatography (eluent: 0-3 % EtOAc in dichloromethane) to give **Compound 101** as a white solid. ¹H NMR (400 MHz, Chloroform-d) δ 1.69-1.88 (m, 6H), 2.15 (s, 3H), 2.31 (s, 0H), 2.62 (s, 1H), 2.69 (s, 3H), 2.79 (s, 3H), 2.8 (s, 3H), 3.06 (s, 1H), 4.45 (s, 1H), 4.58 (s, 1H), 5.53 (d, *J =* 2.4 Hz, 2H), 7.26 (d, *J =* 7.8 Hz, 1H), 7.32 (s, 1H), 8.01 (s, 1H), 8.43 (d, *J=* 1.7 Hz, 1H), 8.88 (s, 2H).

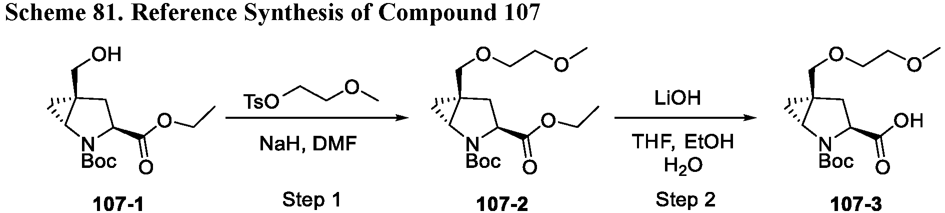

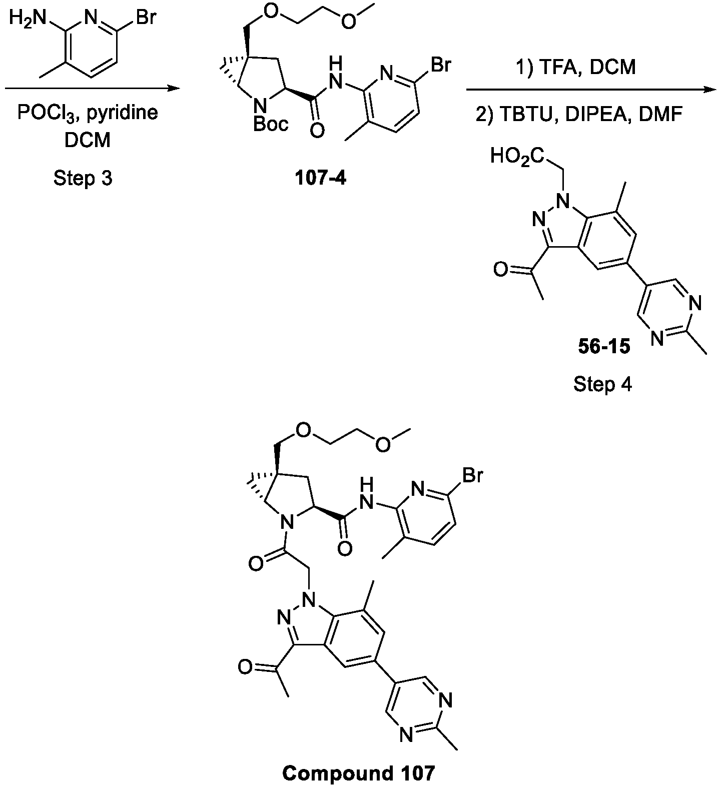

**Step 1: *2-(tert-Butyl)* 3-ethyl (1R,3S,5S)-5-((2-methoxyethoxy)methyl)-2-azabicyclo[3.1.0]hexane-2,3-dicarboxylate (107-2):** To an ice-cooled solution of compound **107-1** (1 g, 3.51 mmol, 1 equiv.) in DMF (10 mL) was added NaH (0.211 g, 60 % in paraffin oil) in portions. The reaction mixture was stirred for 30 min at 0 °C after all of the NaH was added, and 2-methoxyethyl 4-methylbenzenesulfonate (2.42 g, 10.5 mmol, 3 equiv.) was added dropwise. The reaction mixture was gradually allowed to come to rt and stirred overnight at rt. The reaction mixture was cooled in an ice bath and quenched by the careful addition of water (100 mL) and extracted with EtOAc. The organic layer was dried (Na₂SO₄) and concentrated, and the residue was purified by silica gel column chromatography (eluent:0-15 % EtOAc in hexanes) to give compound **107-2** as colorless liquid.

**Step 2: (1R,3S,5S)-2-(*tert*-Butoxycarbonyl)-5-((2-methoxyethoxy)methyl)-2-azabicyclo[3.1.0]hexane-3-carboxylic acid (107-3):** To a solution of compound **107-2** (0.5 g, 1.46 mmol, 1 equiv.) in THF (4 mL) and EtOH (2 mL) was added lithium hydroxide monohydrate (0.092 g, 2.184 mmol, 1.5 equiv.) as a solution in water (2 mL). The reaction mixture was stirred at rt for 4 h, and the organic solvents were removed under reduced pressure. The aqueous residue was diluted with water (10 mL) and extracted with ether, and the ether extract was discarded. The aqueous phase was then acidified with cold 1N aqueous HCl and extracted with dichloromethane. The organic layer was dried (Na₂SO₄) and concentrated to give compound **107-3** as a colorless resin, which was used without further purification in the next step.

**Step 3: tert-Butyl (1R,3S,5S)-3-((6-bromo-3-methylpyridin-2-yl)carbamoyl)-5-((2-methoxyethoxy)methyl)-2-azabicyclo[3.1.0]hexane-2-carboxylate (107-4):** To an ice-cooled solution of compound **107-3** (0.12 g, 0.381 mmol, 1 equiv.), 6-bromo-3-methylpyridin-2-amine (0.078 g, 0.419 mmol, 1.1 equiv.), and pyridine (0.155 mL, 1.903 mmol, 5 equiv.) in dichloromethane (5 mL) was added phosphorus oxychloride (0.034 mL, 0.381 mmol, 1 equiv.) dropwise. The solution was stirred at 0 °C for 30 min and quenched by the careful addition of saturated aqueous sodium bicarbonate. The reaction mixture was then stirred at rt for 30 min, diluted with dichloromethane, and washed repeatedly with cold 1N aqueous HCl to remove unreacted 6-bromo-3-methylpyridin-2-amine. Finally, the organic layer was washed with water, dried (Na₂SO₄), and concentrated to give compound **107-4** as a slightly orange solid, which was used without further purification.

**Step 4. (1R,3S,5S)-2-(2-(3-acetyl-7-methyl-5-(2-methylpyrimidin-5-yl)-1*H*-indazol-1-yl)acetyl)-*N-*(6-bromo-3-methylpyridin-2-yl)-5-((2-methoxyethoxy)methyl)-2-azabicyclo[3.1.0]hexane-3-carboxamide (Compound 107):** Compound **107-4** (0.09 g, 0.185 mmol, 1 equiv.) was stirred at rt with 1 mL of TFA and 1 mL of dichloromethane for 30 min. The volatiles were then removed under reduced pressure. The residue was dissolved in DMF and compound **56-15** (0.06 g, 0.185 mmol, 1 equiv.) was added to this solution followed by the addition of DIPEA (0.101 mL, 0.925 mmol, 5 equiv.). The reaction mixture was cooled in an ice-bath and TBTU (0.065 g, 0.203 mmol, 1.1 equiv.) was added, then the cooling bath was removed and the reaction mixture was stirred at rt for 30 min. The solvent was removed under reduced pressure, and the residue was dissolved in dichloromethane and washed with saturated aqueous sodium bicarbonate. Finally, the organic layer was dried (Na₂SO₄) and concentrated, and the residue was purified by silica gel flash column chromatography (eluent: 0-3 % EtOAc in dichloromethane) to give **Compound 107** as a white solid. ¹H NMR (400 MHz, Chloroform-d) δ 0.98 - 1.10 (m, 2H), 1.49 (dd, *J =* 2.7, 6.3 Hz, 1H), 1.99 (s, 3H), 2.53-2.61 (m, 2H), 2.70 (s, 3H), 2.78 (s, 3H), 2.80 (s, 3H), 3.41 (s, 3H), 3.45 - 3.57 (m, 2H), 3.57-3.58 (m, 2H), 3.64-3.66 (m, 2H), 3.75 (d, *J =* 10.3 Hz, 1H), 5.15(brs, 1H), 5.68 (s, 2H), 7.19 (d, *J =* 7.9 Hz, 1H), 7.27 - 7.37 (m, 2H), 8.45 (s, 1H), 8.66 (s, 1H), 8.89 (s, 2H).

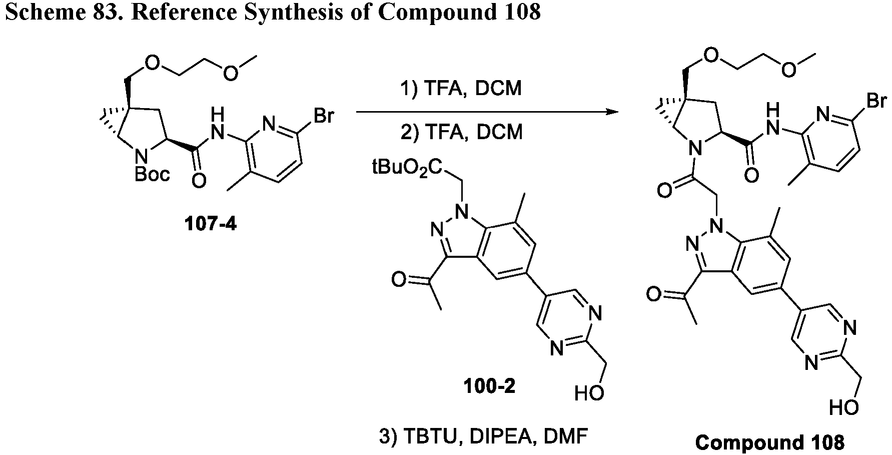

**Step 1. (1R,3S,5S)-2-(2-(3-acetyl-5-(2-(hydroxymethyl)pyrimidin-5-yl)-7-methyl-1H-indazol-1-yl)acetyl)-N-(6-bromo-3-methylpyridin-2-yl)-5-((2-methoxyethoxy)methyl)-2-azabicyclo[3.1.0]hexane-3-carboxamide (Compound 108):** Compound 100-2 (0.047 g, 0.117 mmol, 1 equiv.) was stirred with 1 mL of TFA and 0.5 mL of dichloromethane for 3 h at rt in a vial. In another vial, compound **107-4** (0.057 g, 0.117 mmol, 1 equiv.) was stirred with 1 mL of TFA and 1 mL of dichloromethane for 30 min at rt. The contents of both the vials were combined and the volatiles were removed under reduced pressure. The residue was dissolved in DMF (2 mL) and cooled in an ice bath, then DIPEA (0.102 mL, 0.117 mmol, 1 equiv.) and TBTU (0.042 g, 0.131 mmol, 1.113 equiv.) were added successively, and the cooling bath was removed. The reaction mixture was stirred at rt for 30 min and the solvent was removed under reduced pressure. The residue was dissolved in dichloromethane and washed with saturated aqueous sodium bicarbonate, and the organic layer was dried (Na₂SO₄) and concentrated. The crude product was purified by silica gel flash column chromatography (eluent: 0-3 %MeOH in dichloromethane) to give **Compound 108** as a white solid. ¹H NMR (400 MHz, Chloroform-d) δ 0.92 - 1.11 (m, 2H), 1.49 (dd, *J =* 2.7, 6.3 Hz, 1H), 2.01 (s, 3H), 2.53-2.61 (m, 2H), 2.71 (s, 3H), 2.78 (s, 3H), 3.41 (s, 3H), 3.48-3.52 (m, 2H), 3.56-3.59 (m, 2H), 3.62 - 3.73 (m, 3H), 3.76 (d, *J =* 10.4 Hz, 1H), 4.91 (d, *J =* 4.7 Hz, 2H), 5.17 (s, 1H), 5.69 (s, 2H), 7.20 (d, *J =* 7.9 Hz, 1H), 7.28 - 7.39 (m, 2H), 8.47 (s, 1H), 8.63 (s, 1H), 8.98 (s, 2H).

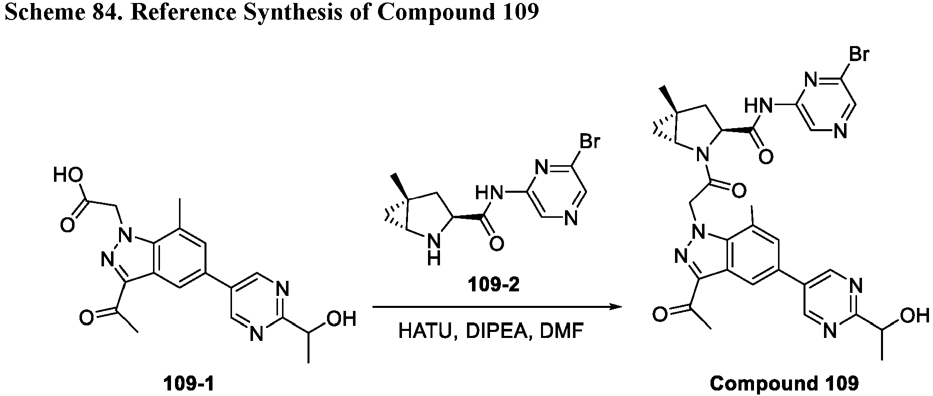

**(1R,3S,5R)-2-(2-(3-acetyl-5-(2-(1-hydroxyethyl)pyrimidin-5-yl)-7-methyl-1H-indazol-1-yl)acetyl)-N-(6-bromopyrazin-2-yl)-5-methyl-2-azabicyclo[3.1.0]hexane-3-carboxamide (Compound 109):** To a solution of compound **109-1** (0.03 g, 0.085 mmol, 1 equiv.) in dimethylformamide (1 mL) at 0 °C under an atmosphere of argon were added compound **109-2** (0.025 g, 0.085 mmol, 1 equiv.), HATU (0.068 g, 0.178 mmol, 2.1 equiv.), and DIPEA (0.055 g, 0.423 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 109.** ¹H NMR (400 MHz, DMSO-d6) δ 0.89 - 0.97 (m, 1H), 1.01 (t, J = 5.5 Hz, 1H), 1.32 (s, 3H), 1.47 (d, J = 6.6 Hz, 3H), 2.04 (dd, J = 6.1, 13.2 Hz, 1H), 2.53 - 2.56 (m, 1H), 2.65 (s, 3H), 2.71 (s, 3H), 3.53 - 3.72 (m, 1H), 4.45 (dd, J = 6.1, 9.1 Hz, 1H), 4.85 (p, J = 6.4 Hz, 1H), 5.25 (d, J = 5.6 Hz, 1H), 5.68 (d, J = 17.8 Hz, 1H), 6.09 (d, J = 17.9 Hz, 1H), 7.65 (s, 1H), 8.33 (s, 1H), 8.54 (s, 1H), 9.11 (s, 2H), 9.27 (s, 1H), 11.16 (s, 1H).

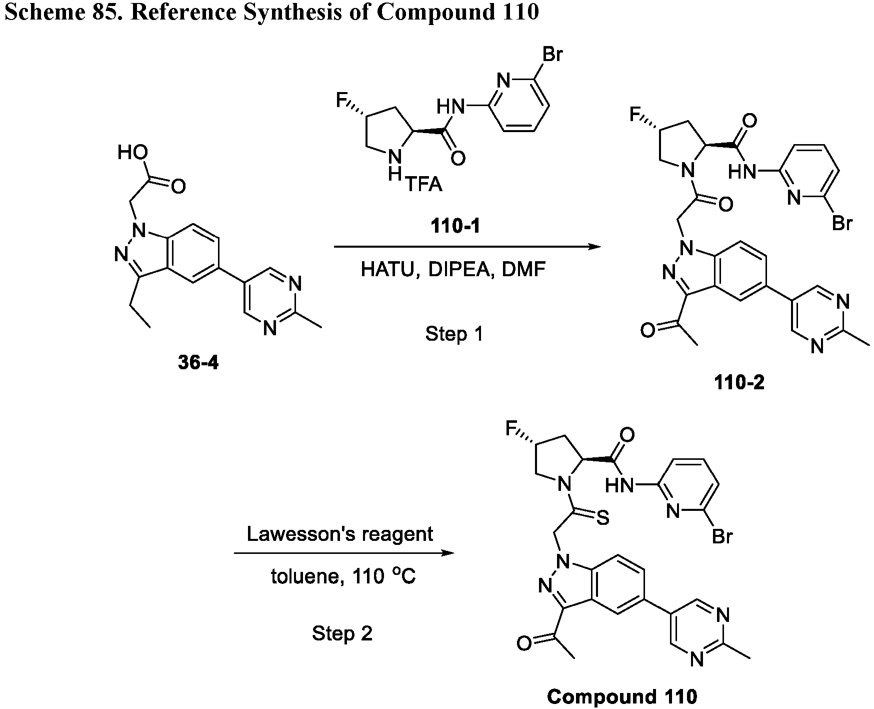

**Step 1: 1-{2-[3-Acetyl-5-(2-methyl-pyrimidin-5-yl)-indazol-1-yl]-acetyl}-4-fluoro-pyrrolidine-2-carboxylic acid (6-bromo-pyridin-2-yl)-amide (110-2):** To a mixture of compound **36-4** (95 mg, 0.31 mmol) and compound **110-1** (119 mg, 0.31 mmol) in DMF (5 mL) was added DIPEA (200 mg, 1.55 mmol) followed by HATU (177 mg, 0.47 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by chromatography on silica gel (DCM: MeOH= 100: 1 to 60: 1) to give compound **110-2** (170 mg, yield 95.0%) as a white solid. LC/MS (ESI) m/z: 580/582 (M+H)⁺.

**Step 2: 1-{2-[3-Acetyl-5-(2-methyl-pyrimidin-5-yl)-indazol-1-yl]-thioacetyl}-4-fluoro-pyrrolidine-2-carboxylic acid (6-bromo-pyridin-2-yl)-amide (Compound 110):** To a solution of compound **110-2** (106 mg, 0.18 mmol) in toluene (6 mL) was added Lawesson's reagent (94 mg, 0.22 mmol) at 0 °C under N₂ atmosphere and the mixture was stirred at 110 °C under N₂ atmosphere overnight. The mixture was diluted with water and extracted with EtOAC twice. The combined organic layers were washed with brine, dried with anhydrous Na₂SO₄, filtered, and concentrated to dryness. The residue was purified by prep-TLC (DCM: MeOH= 30: 1) to give **Compound 110** (2.4 mg, yield 2.2%) as a white solid. ¹H-NMR (400 MHz, DMSO-*d*₆) δ 11.10 (s, 1H), 9.05 (s, 2H), 8.43 *(d, J =* 1.2 Hz, 1H), 8.03 *(d, J =* 8.0 Hz, 1H), 7.86 *(t, J=* 1.6 Hz, 2H), 7.72 (t, *J =* 8.0 Hz, 1H), 7.34 (d, *J =* 7.6 Hz, 1H), 6.07 (d, *J =* 17.6 Hz, 1H), 5.85 (d, *J =* 17.6 Hz, 1H), 5.70 - 5.54 (m, 1H), 5.14 (t, *J =* 8.6 Hz, 1H), 4.67 (dd, *J =* 20.8, 20.8 Hz, 1H), 4.30 - 4.14 (m, 1H), 2.69 (s, 3H), 2.68 - 2.67 (m, 1H), 2.66 (s, 3H), 2.39 - 2.27 (m, 1H). LC/MS (ESI) m/z: 596/598 (M+H)⁺.

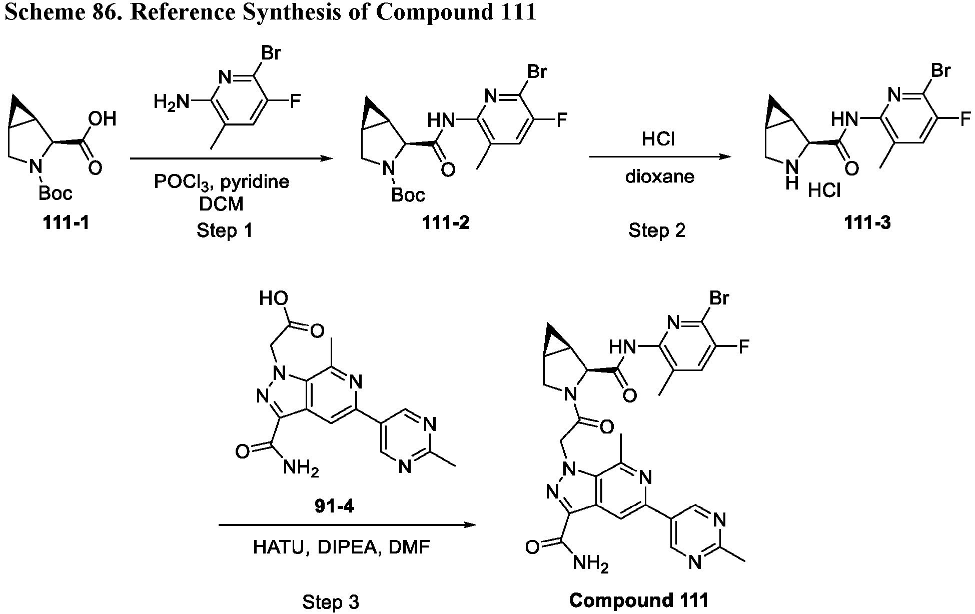

**Step 1: 2-(6-Bromo-5-fluoro-3-methyl-pyridin-2-ylcarbamoyl)-3-aza-bicyclo[3.1.0]hexane-3-carboxylic acid tert-butyl ester (111-2):** To a mixture of compound **111-1** (100 mg, 0.44 mmol) and 6-Bromo-5-fluoro-3-methyl-pyridin-2-ylamine (90 mg, 0.44 mmol) in DCM (8 mL) was added pyridine (174 mg, 2.2 mmol) followed by phosphoryl chloride (101 mg, 0.66 mmol) at 0 °C, and the mixture was stirred at room temperature under N₂ atmosphere for 2 hours. The mixture was poured into ice water and extracted with DCM twice. The combined organic layers were washed with brine, dried with anhydrous Na₂SO₄, filtered, and concentrated to dryness. The residue was purified by chromatography on silica gel (PE: EtOAc= 30: 1 to 15: 1) to give compound **111-2** (140 mg, yield 77.3%) as a white solid. LC/MS (ESI) m/z: 414/416 (M+H)⁺.

**Step 2: 3-Aza-bicyclo[3.1.0]hexane-2-carboxylic acid (6-bromo-5-fluoro-3-methyl-pyridin-2-yl)-amide hydrochloride (111-3):** A solution of 4 M hydrogen chloride in 1,4-dioxane (3 mL) containing compound **111-2** (45 mg, 0.11 mmol) at 0 °C was stirred at room temperature for 1 hr. The reaction mixture was concentrated to dryness, washed with ether and dried under vacuum to give compound **111-3** (33 mg, yield 97.1%) as a white solid. LC/MS (ESI) m/z: 314/316 (M+H)⁺.

**Step 3: 1-{2-[2-(6-Bromo-5-fluoro-3-methyl-pyridin-2-ylcarbamoyl)-3-aza-bicyclo[3.1.0]hex-3-yl]-2-oxo-ethyl}-7-methyl-5-(2-methyl-pyrimidin-5-yl)-1H-pyrazolo[3,4-c]pyridine-3-carboxylic acid amide (Compound 111):** To a mixture of compound **111-3** (10 mg, 0.032 mmol) and compound 91-4 ([3-Carbamoyl-7-methyl-5-(2-methyl-pyrimidin-5-yl)-pyrazolo[3,4-c]pyridin-1-yl]-acetic acid, 10 mg, 0.032 mmol) in DMF (3 mL) was added DIPEA (21 mg, 0.16 mmol) followed by HATU (18 mg, 0.048 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by prep-TLC (DCM: MeOH= 40: 1) to give **Compound 111** (9.1 mg, yield 45.8%) as a white solid. ¹H-NMR (400 MHz, DMSO-*d*₆) δ 10.42 (s, 1H), 9.30 (s, 2H), 8.46 (s, 1H), 7.79 (d, *J =* 8.8 Hz, 2H), 7.62 (s, 1H), 5.75 (d, *J =* 17.6 Hz, 1H), 5.63 (d, *J =* 17.6 Hz, 1H), 4.62 (d, *J =* 5.6 Hz, 1H), 4.01 (dd, *J =* 10.0, 10.0 Hz, 1H), 3.89 (d, *J =* 9.6 Hz, 1H), 2.88 (s, 3H), 2.68 (s, 3H), 2.05 (s, 3H), 2.04 - 1.96 (m, 1H), 1.93-1.88(m, 1H), 0.82 - 0.76 (m, 2H). LC/MS (ESI) m/z: 622/624 (M+H)⁺.

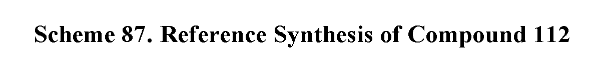

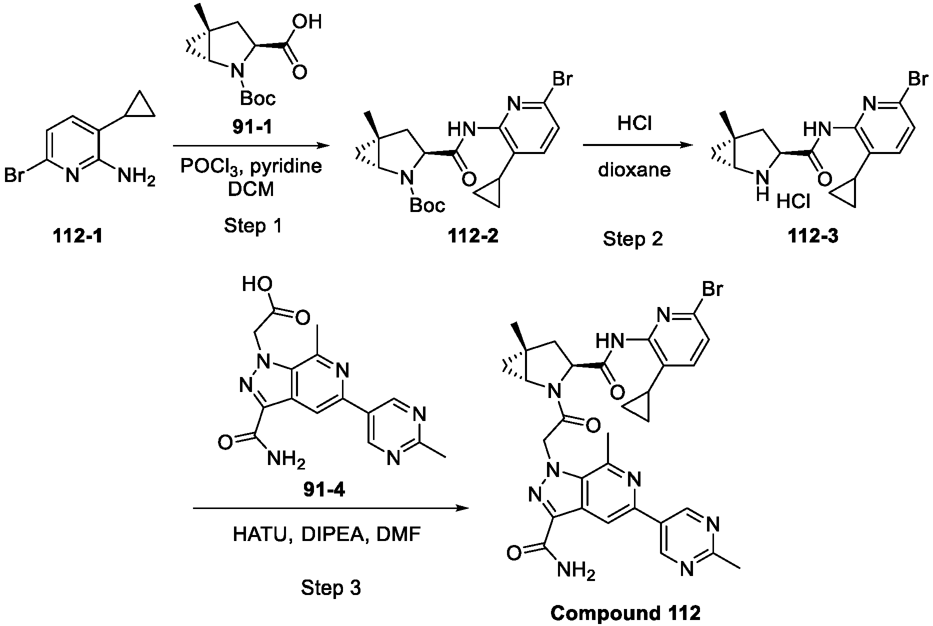

**Step** 1: **3-(6-Bromo-3-cyclopropyl-pyridin-2-ylcarbamoyl)-S-methyl-2-aza-bicyclo[3.1.0]hexane-2-carboxylic acid tert-butyl ester (112-2):** To a mixture of compound 91-1 ((1R,3S,5R)-2-(tert-butoxycarbonyl)-5-methyl-2-azabicyclo[3.1.0]hexane-3-carboxylic acid, 33 mg, 0.14 mmol) and compound **112-1** (6-Bromo-3-cyclopropyl-pyridin-2-ylamine, 30 mg, 0.14 mmol) in DCM (5 mL) was added pyridine (55 mg, 0.7 mmol) followed by phosphoryl chloride (32 mg, 0.21 mmol) at 0 °C, and the mixture was stirred at room temperature under N₂ atmosphere for 2 hrs. The mixture was poured into ice water and extracted with DCM twice. The combined organic layers were washed with brine, dried with anhydrous Na₂SO₄, filtered, and concentrated to dryness. The residue was purified by chromatography on silica gel (PE: EtOAc= 30: 1 to 10: 1) to give compound **112-2** (46 mg, yield 75.5%) as a white solid. LC/MS (ESI) m/z: 436/438 (M+H)⁺.

**Step 2: 5-Methyl-2-aza-bicyclo[3.1.0]hexane-3-carboxylic acid (6-bromo-3-cyclopropyl-pyridin-2-yl)-amide hydrochloride (112-3):** A solution of 4 M hydrogen chloride in 1,4-dioxane (3 mL) containing compound **112-2** (46 mg, 0.11 mmol) was stirred at room temperature for 1 hr. The reaction mixture was concentrated to dryness, washed with ether and dried under vacuum to give compound **112-3** (35 mg, yield 85.3%) as a white solid. LC/MS (ESI) m/z: 336/338 (M+H)⁺.

**Step 3: 1-{2-[3-(6-Bromo-3-cyclopropyl-pyridin-2-ylcarbamoyl)-5-methyl-2-aza-bicyclo[3.1.0]hex-2-yl]-2-oxo-ethyl}-7-methyl-5-(2-methyl-pyrimidin-5-yl)-1H-pyrazolo[3,4-c]pyridine-3-carboxylic acid amide (Compound 112):** To a mixture of compound **112-3** (17 mg, 0.046 mmol) and compound **91-4** ([3-Carbamoyl-7-methyl-5-(2-methyl-pyrimidin-5-yl)-pyrazolo[3,4-c]pyridin-1-yl]-acetic acid, 15 mg, 0.046 mmol) in DMF (3 mL) was added DIPEA (30 mg, 0.23 mmol) followed by HATU (26 mg, 0.069 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated. The residue was purified by prep-TLC (DCM: MeOH= 40: 1) to give **Compound 112** (9.6 mg, yield 32.4%) as a white solid. ¹H-NMR (400 MHz, DMSO-*d*₆) δ 10.29 (s, 1H), 9.31 (s, 2H), 8.48 (s, 1H), 7.89 (s, 1H), 7.61 (s, 1H), 7.41 (d, *J = 8.0* Hz, 1H), 7.31 (d, *J = 8.4* Hz, 1H), 6.05 (d, *J = 18.0* Hz, 1H), 5.67 (d, *J =* 18.0 Hz, 1H), 4.43 (dd, *J = 9.2,* 9.2 Hz, 1H), 3.63 (dd, *J=* 5.6, 5.6 Hz, 1H), 2.89 (s, 3H), 2.69 (s, 3H), 2.59 - 2.54 (m, 1H), 2.06 (dd, *J =* 13.2, 13.2 Hz, 1H), 1.85-1.77 (m, 1H), 1.33 (s, 3H), 1.03 (t, *J = 5.4* Hz, 1H), 0.93 - 0.90 (m, 1H), 0.80 - 0.73 (m, 2H), 0.55 (m, 2H). LC/MS (ESI) m/z: 644/646 (M+H)⁺.

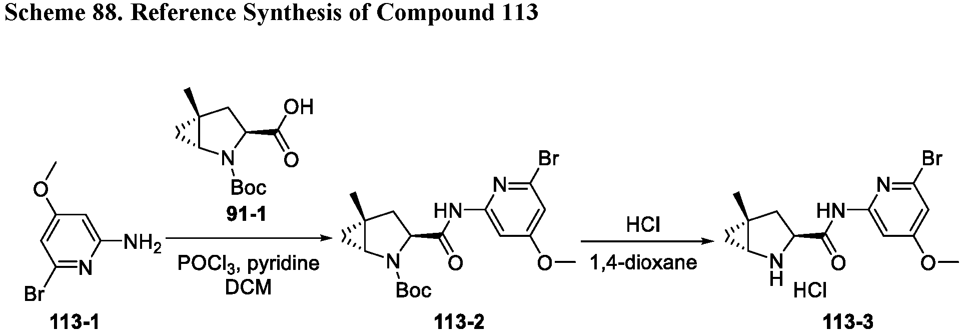

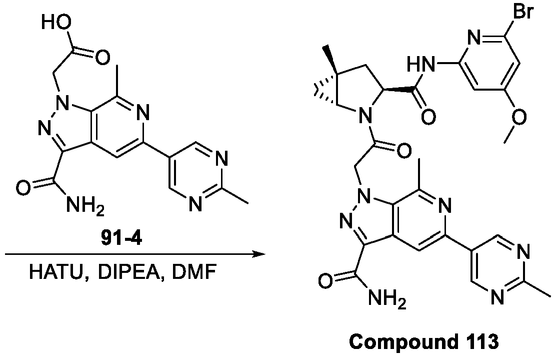

**Step** 1: 3-(6-Bromo-4-methoxy-pyridin-2-ylcarbamoyl)-5-methyl-2-aza-bicyclo[3.1.0]hexane-2-carboxylic acid tert-butyl ester (113-2): To a mixture of compound 91-1 (40 mg, 0.17 mmol) and compound 113-1 (6-Bromo-4-methoxy-pyridin-2-ylamine, 34 mg, 0.17 mmol) in DCM (5 mL) was added pyridine (67 mg, 0.85 mmol) followed by phosphoryl chloride (39 mg, 0.26 mmol) at 0 °C, and the mixture was stirred at room temperature under N₂ atmosphere for 2 hrs. The mixture was poured into ice water and extracted with DCM twice. The combined organic layers were washed with brine, dried with anhydrous Na₂SO₄, filtered, and concentrated to dryness. The residue was purified by chromatography on silica gel (PE: EtOAc= 30: 1 to 13: 1) to give compound 113-2 (63 mg, yield 87.2%) as a light oil. LC/MS (ESI) m/z: 426/428 (M+H)⁺.

Step 2: 5-Methyl-2-aza-bicyclo[3.1.0]hexane-3-carboxylic acid (6-bromo-4-methoxy-**pyridin-2-yl)-amide hydrochloride (113-3):** A solution of 4 M hydrogen chloride in 1,4-dioxane (3 mL) containing compound **113-2** (63 mg, 0.15 mmol) was stirred at room temperature for 1 hr. The reaction mixture was concentrated to dryness, washed with ether, and dried under vacuum to give compound **113-3** (45 mg, yield 82.8%) as a yellow solid. LC/MS (ESI) m/z: 326/328 (M+H)⁺.

**Step 3: 1-(2-((1R,3S,5R)-3-((6-bromo-4-methoxypyridin-2-yl)carbamoyl)-5-methyl-2-azabicyclo[3.1.0]hexan-2-yl)-2-oxoethyl)-7-methyl-5-(2-methylpyrimidin-5-yl)-1H-pyrazolo[3,4-c]pyridine-3-carboxamide (Compound 113):** To a mixture of compound **113-3** (11 mg, 0.03 mmol) and compound **91-4** (10 mg, 0.03 mmol) in DMF (3 mL) was added DIPEA (19 mg, 0.15 mmol) followed by HATU (17 mg, 0.045 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered and concentrated to dryness. The residue was purified by prep-TLC (DCM: MeOH= 40: 1) to give Compound 113 (8.0 mg, yield 42.1%) as a white solid. ¹H-NMR (400 MHz, DMSO-*d*₆) δ 10.77 (s, 1H), 9.30 (s, 2H), 8.47 (s, 1H), 7.90 (s, 1H), 7.65 - 7.58 (m, 2H), 6.96 (d, *J = 2.0* Hz, 1H), 6.06 (d, *J =* 18.0 Hz, 1H), 5.68 (d, *J =* 18.0 Hz, 1H), 4.41 (dd, *J =* 9.2, 9.2 Hz, 1H), 3.83 (s, 3H), 3.64 (dd, *J =* 5.6, 5.6 Hz, 1H), 3.24 - 3.08 (m, 1H), 2.90 (s, 3H), 2.69 (s, 3H), 1.98 (dd, *J =* 13.6, 13.6 Hz, 1H), 1.31 (s, 3H), 1.00 (t, *J =* 5.4 Hz, 1H), 0.87 (dd, *J =* 5.2, 5.2 Hz, 1H). LC/MS (ESI) m/z: 634/636 (M+H)⁺.

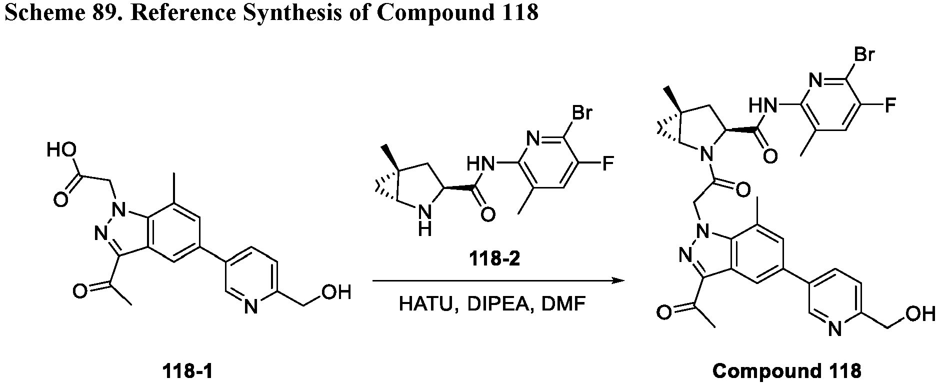

**(1R,3S,5R)-2-(2-(3-acetyl-5-(6-(hydroxymethyl)pyridin-3-yl)-7-methyl-1H-indazol-1-yl)acetyl)-N-(6-bromo-5-fluoro-3-methylpyridin-2-yl)-5-methyl-2-azabicyclo[3.1.0]hexane-3-carboxamide (Compound 118):** To a solution of compound **118-1** (0.06 g, 0.177 mmol, 1 equiv.) in dimethylformamide (1 mL) at 0 °C under an atmosphere of argon were added compound **118-2** (0.058 g, 0.177 mmol, 1 equiv.), HATU (0.141 g, 0.371 mmol, 2.1 equiv.), and DIPEA (0.114 g, 0.884 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 118.** ¹H NMR (400 MHz, DMSO-d6) δ 0.92 - 0.98 (m, 1H), 1.03 (t, J = 5.5 Hz, 1H), 1.33 (s, 3H), 1.98 - 2.13 (m, 4H), 2.53 - 2.61 (m, 1H), 2.65 (s, 3H), 2.69 (s, 3H), 3.57 - 3.66 (m, 1H), 4.40 (dd, J = 5.5, 9.2 Hz, 1H), 4.63 (s, 2H), 5.45 (s, 1H), 5.69 (d, J = 17.8 Hz, 1H), 6.03 (d, J = 17.8 Hz, 1H), 7.49 - 7.66 (m, 2H), 7.83 (d, J = 8.4 Hz, 1H), 8.11 (dd, J = 2.4, 8.1 Hz, 1H), 8.30 (d, J = 1.6 Hz, 1H), 8.80 (d, J = 2.3 Hz, 1H), 10.31 (s, 1H).

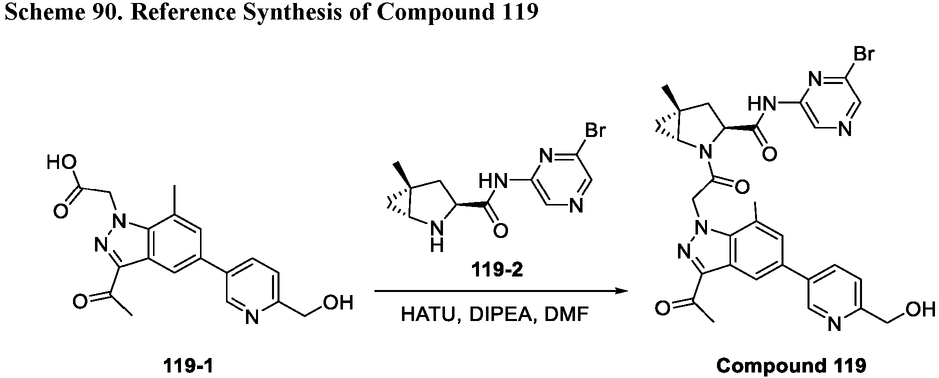

**Step 1: (1R,3S,5R)-2-(2-(3-acetyl-5-(6-(hydroxymethyl)pyridin-3-yl)-7-methyl-1H-indazol-1-yl)acetyl)-N-(6-bromopyrazin-2-yl)-5-methyl-2-azabicyclo[3.1.0]hexane-3-carboxamide (Compound 119):** To a solution of compound **119-1** (0.03 g, 0.088 mmol, 1 equiv.) in dimethylformamide (1 mL) at 0 °C under an atmosphere of argon were added compound **119-2** (0.026 g, 0.088 mmol, 1 equiv.), HATU (0.071 g, 0.186 mmol, 2.1 equiv.), and DIPEA (0.057 g, 0.442 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 119.** ¹H NMR (400 MHz, DMSO-d6) δ 0.87 - 0.95 (m, 1H), 1.01 (t, J = 5.5 Hz, 1H), 1.32 (s, 3H), 1.96 - 2.08 (m, 1H), 2.53 - 2.55 (m, 1H), 2.64 (s, 3H), 2.71 (s, 3H), 3.56 - 3.70 (m, 1H), 4.46 (dd, J = 6.2, 9.1 Hz, 1H), 4.62 (d, J = 5.8 Hz, 2H), 5.44 (t, J = 5.8 Hz, 1H), 5.68 (d, J = 17.9 Hz, 1H), 6.09 (d, 1H), 7.53 - 7.67 (m, 2H), 8.10 (dd, J = 2.4, 8.1 Hz, 1H), 8.28 (d, J = 1.6 Hz, 1H), 8.54 (s, 1H), 8.80 (s, 1H), 9.27 (s, 1H), 11.16 (s, 1H).

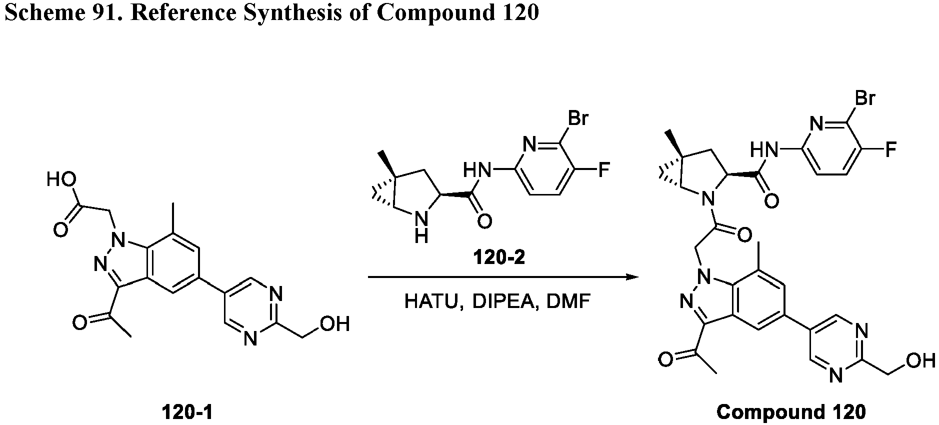

**(1R,3S,5R)-2-(2-(3-acetyl-5-(2-(hydroxymethyl)pyrimidin-5-yl)-7-methyl-1H-indazol-1-yl)acetyl)-N-(6-bromo-5-fluoropyridin-2-yl)-5-methyl-2-azabicyclo[3.1.0]hexane-3-carboxamide (Compound 120):** To a solution of compound **120-1** (0.043 g, 0.126 mmol, 1 equiv.) in dimethylformamide (1 mL) at 0 °C under an atmosphere of argon were added compound **120-2** (0.04 g, 0.126 mmol, 1 equiv.), HATU (0.101 g, 0.265 mmol, 2.1 equiv.), and DIPEA (0.081 g, 0.630 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound** 120. ¹H NMR (400 MHz, DMSO-d6) δ 0.87 - 0.92 (m, 1H), 1.00 (t, J = 5.5 Hz, 1H), 1.31 (s, 3H), 2.00 (dd, J = 6.1, 13.1 Hz, 1H), 2.46 - 2.55 (m, 1H), 2.65 (s, 3H), 2.71 (s, 3H), 3.64 (dd, J = 2.3, 5.7 Hz, 1H), 4.42 (dd, J = 6.1, 9.0 Hz, 1H), 4.68 (d, J = 6.3 Hz, 2H), 5.33 (t, J = 6.3 Hz, 1H), 5.67 (d, J = 17.8 Hz, 1H), 6.08 (d, J = 17.9 Hz, 1H), 7.65 (s, 1H), 7.80 - 7.90 (m, 1H), 8.06 (dd, J = 3.3, 8.9 Hz, 1H), 8.32 - 8.39 (m, 1H), 9.11 (s, 2H), 10.88 (s, 1H).

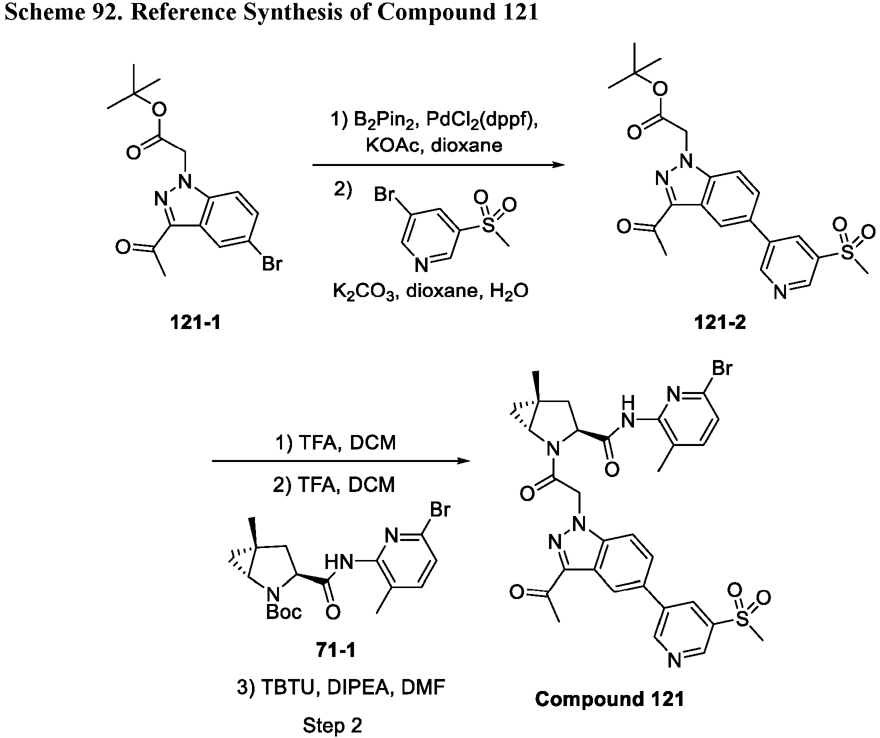

**Step 1. *tert-Butyl* 2-(3-acetyl-5-(5-(methylsulfonyl)pyridin-3-yl)-1H-indazol-1-yl)acetate (121-2):** A heterogeneous solution of compound **121-1** (0.1 g, 0.272 mmol, 1 equiv.), bis(pinacolato)diboron (0.079 g, 0.31 mmol, 1.2 equiv.), PdCl₂(dppf) (0.011 g, 0.014 mmol, 0.05 equiv.), and KOAc (0.053 g, 0.545 mmol, 2 equiv.) in dioxane (4 mL) was degassed with argon and heated at 90° C overnight with vigorous stirring. The resulting black heterogenous mixture was cooled to rt and 3-bromo-5-methanesulfonylpyridine (0.061 g, 0.258 mmol, 1 equiv.), potassium carbonate (0.113 g, 0.817 mmol, 3 equiv.), and water (1 mL) were added to the reaction mixture. The reaction mixture was again degassed with argon and heated at 100 °C for 4 h. Then the reaction mixture was cooled to rt and the solvent was removed under reduced pressure. The residue was purified by silica gel flash column chromatography (eluent:0-2 % MeOH in dichloromethane) to give compound **121-2** as a white solid.

**Step 2. (1R,3S,5R)-2-(2-(3-Acetyl-5-(5-(methylsulfonyl)pyridin-3-yl)-1*H*-indazol-1-yl)acetyl)-*N*-(6-bromo-3-methylpyridin-2-yl)-5-methyl-2-azabicyclo[3.1.0]hexane-3-carboxamide (Compound 121):** Compound **121-2** (0.07 g, 0.163 mmol, 1 equiv.) was stirred with 2 mL of TFA and 1 mL of dichloromethane for 3 h at rt in a vial. In another vial, compound **71-1** (0.067 g, 0.163 mmol, 1 equiv.) was stirred with 1 mL of TFA and 1 mL of dichloromethane for 30 min at rt. The contents of both the vials were combined and the volatiles were removed under reduced pressure. The residue was dissolved in DMF (2 mL) and cooled in an ice bath. DIPEA (0.142 mL, 0.815 mmol, 5 equiv.) and TBTU (0.058 g, 0.179 mmol, 1.1 equiv.) were added successively and the cooling bath was removed. The reaction mixture was stirred at rt for 30 min and the solvent was removed under reduced pressure. The residue was dissolved in dichloromethane and washed with saturated aqueous sodium bicarbonate, then the organic layer was dried (Na₂SO₄) and concentrated. The crude product was purified by silica gel flash column chromatography (eluent: 0-2.5 % MeOH in dichloromethane) to give **Compound 121** as a white solid. ¹H NMR (400 MHz, Chloroform-*d*) δ 0.93 (dd, *J* = 2.4*,* 5.6 Hz, 2H), 1.17 (t, *J =* 5.5 Hz, 2H), 1.42 (s, 3H), 2.07 (s, 3H), 2.30 (dd, *J =* 8.8, 13.5 Hz, 1H), 2.70 (s, 1H), 2.73 (s, 3H), 3.19 (s, 4H), 4.84 (dd, *J =* 3.0, 8.5 Hz, 1H), 5.52 (s, 2H), 7.24 (d, *J =* 7.8 Hz, 1H), 7.35 (d, *J =* 7.9 Hz, 1H), 7.63 (d, *J =* 8.8 Hz, 1H), 7.73 (dd, *J =* 1.7, 8.8 Hz, 1H), 8.44 (d, *J =* 2.2 Hz, 1H), 8.65 (s, 2H), 9.15 (dd, *J =* 2.2, 10.0 Hz, 2H).

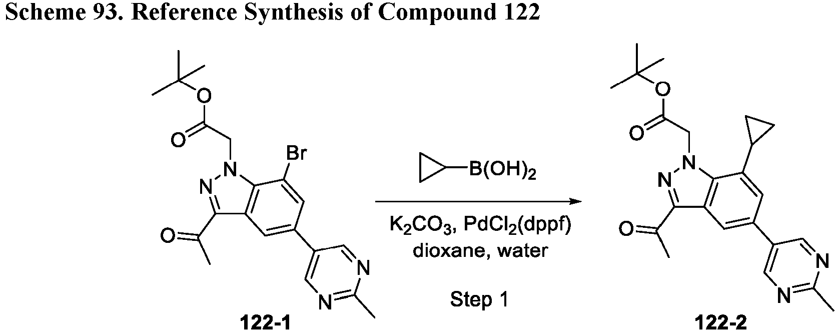

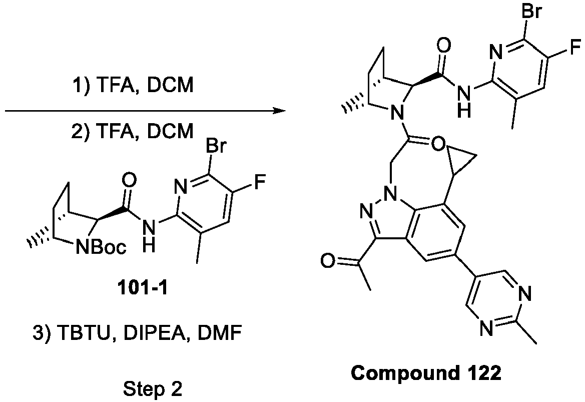

**Step 1. *tert*-Butyl 2-(3-acetyl-7-cyclopropyl-5-(2-methylpyrimidin-5-yl)-1*H*-indazol-1-yl)acetate (122-2):** A mixture of compound **122-1** (0.1 g, 0.225 mmol, 1 equiv.), cyclopropylboronic acid (0.058 g, 0.674 mmol, 3 equiv.), and potassium carbonate (0.093 g, 0.674 mmol, 3 equiv.) in 1,4-dioxane (4 mL) and water (1 mL) was degassed with argon and subjected to microwave irradiation for 45 min at 100 ° C. The reaction mixture was cooled to rt and the organic layer was concentrated under reduced pressure. The residue was purified by silica gel flash column chromatography (0-2 % MeOH in dichloromethane) to give compound **122-2** as cream colored solid.

**Step 2. (1S,3S,4R)-2-(2-(3-Acetyl-7-cyclopropyl-5-(2-methylpyrimidin-5-yl)-1*H-*indazol-1-yl)acetyl)-*N*-(6-bromo-5-fluoro-3-methylpyridin-2-yl)-2-azabicyclo[2.2.1]heptane-3-carboxamide (Compound 122):** Compound **122-2** (0.07 g, 0.172 mmol, 1 equiv.) was stirred with 2 mL of TFA and 1 mL of dichloromethane for 3 h at rt in a vial. In another vial, compound **101-1** (0.074 g, 0.172 mmol, 1 equiv.) was stirred with 1 mL of TFA and 1 mL of dichloromethane for 30 min at rt. The contents of both the vials were combined and the volatiles were removed under reduced pressure. The residue was dissolved in DMF (2 mL) and cooled in an ice bath. DIPEA (0.151 mL, 0.861 mmol, 5 equiv.) and TBTU (0.061 g, 0.189 mmol, 1.1 equiv.) were added successively, and the cooling bath was removed. The reaction mixture was stirred at rt for 30 min, and the solvent was removed under reduced pressure. The residue was dissolved in dichloromethane and washed with saturated aqueous sodium bicarbonate. The organic layer was dried and concentrated, and the crude product was purified by silica gel flash column chromatography (eluent: 0-2.5 % MeOH in dichloromethane) to give Compound **122** as a white solid. ¹H NMR (400 MHz, Chloroform-*d*) δ 0.84-0.87 (m, 1H), 1.02-1.26 (m, 2H), 1.67-1.85 (m, 6H), 2.14 (s, 3H), 2.43-2.49 (m, 1H) 2.69 (s, 3H), 2.80 (s, 3H), 3.07 (s, 1H), 4.45 (s, 1H), 4.59 (s, 1H), 5.70 (d, *J=* 16.4 Hz, 1H), 5.89 (d, *J =* 16.4 Hz, 1H), 7.26 (d, *J =* 7.9 Hz, 1H), 7.33 (s, 1H), 7.97 (s, 1H), 8.44 (d, *J =* 1.7 Hz, 1H), 8.87 (s, 2H).

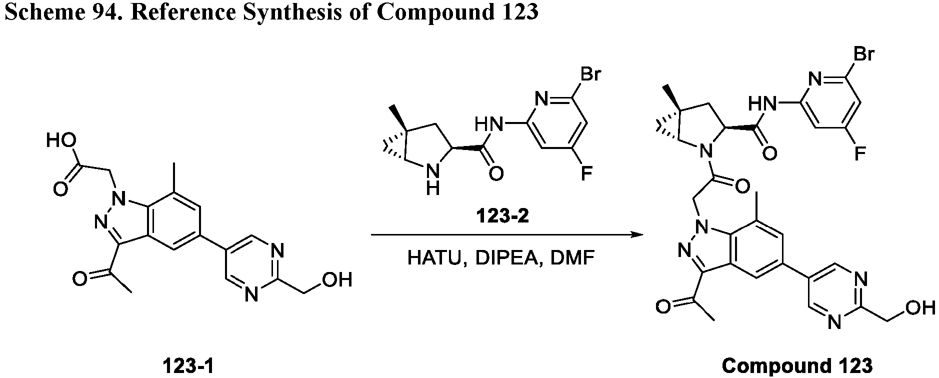

**(1R,3S,5R)-2-(2-(3-acetyl-5-(2-(hydroxymethyl)pyrimidin-5-yl)-7-methyl-1H-indazol-1-yl)acetyl)-N-(6-bromo-4-fluoropyridin-2-yl)-5-methyl-2-azabicyclo[3.1.0]hexane-3-carboxamide (Compound 123):** To a solution of compound **123-1** (0.03 g, 0.088 mmol, 1 equiv.) in dimethylformamide (1 mL)at 0 °C under an atmosphere of argon were added **123-2** (0.028 g, 0.088 mmol, 1 equiv.), HATU (0.07 g, 0.185 mmol, 2.1 equiv.), and DIPEA (0.057 g, 0.441 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 123.** ¹H NMR (400 MHz, DMSO-d6) δ 0.89 - 0.96 (m, 1H), 1.00 (t, J = 5.6 Hz, 1H), 1.31 (s, 3H), 2.00 (dd, J = 6.2, 13.0 Hz, 1H), 2.43 - 2.56 (m, 1H), 2.65 (s, 3H), 2.71 (s, 3H), 3.60 - 3.68 (m, 1H), 4.43 (dd, J = 6.1, 9.1 Hz, 1H), 4.68 (d, J = 6.3 Hz, 2H), 5.33 (t, J = 6.3 Hz, 1H), 5.67 (d, J = 17.8 Hz, 1H), 6.08 (d, J = 17.9 Hz, 1H), 7.43 (dd, J = 2.0, 7.6 Hz, 1H), 7.66 (s, 1H), 7.86 (dd, J = 2.0, 11.0 Hz, 1H), 8.34 (d, J = 1.6 Hz, 1H), 9.11 (s, 2H), 11.11 (s, 1H).

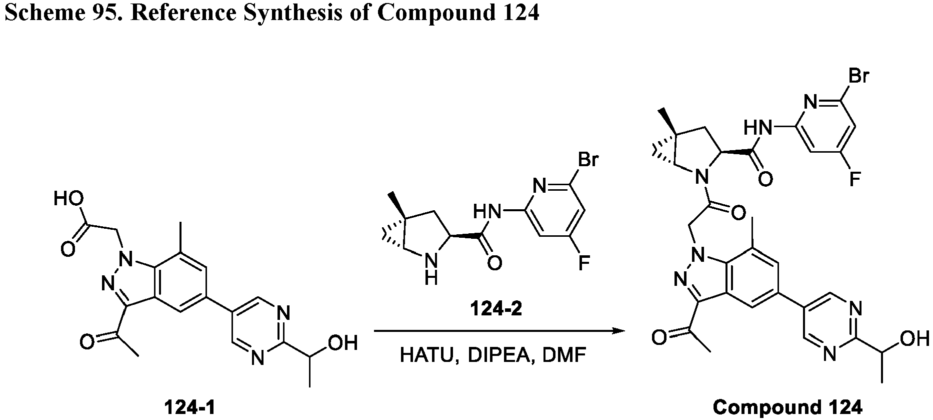

**Step 1: (1R,3S,5R)-2-(2-(3-acetyl-5-(2-(1-hydroxyethyl)pyrimidin-5-yl)-7-methyl-1H-indazol-1-yl)acetyl)-N-(6-bromo-4-fluoropyridin-2-yl)-5-methyl-2-azabicyclo[3.1.0]hexane-3-carboxamide (Compound 124):** To a solution of compound **124-1** (0.034 g, 0.096 mmol, 1 equiv.) in dimethylformamide (1 mL) at 0 °C under an atmosphere of argon were added compound **124-2** (0.03 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and concentrated to dryness. The residue was purified by column chromatography on silica gel (eluted with DCM/MeOH) to give **Compound 124.** ¹H NMR (400 MHz, DMSO-d6) δ 0.89 - 0.92 (m, 1H), 1.00 (t, J = 5.5 Hz, 1H), 1.31 (s, 3H), 1.47 (d, J = 6.6 Hz, 3H), 2.00 (dd, J = 6.0, 13.3 Hz, 1H), 2.46 - 2.54 (m, 1H), 2.65 (s, 3H), 2.70 (s, 3H), 3.64 (dd, J = 2.4, 5.8 Hz, 1H), 4.43 (dd, J = 6.1, 9.1 Hz, 1H), 4.85 (q, J = 6.5 Hz, 1H), 5.67 (d, J = 17.8 Hz, 1H), 6.08 (d, J = 17.9 Hz, 1H), 7.43 (dd, J = 2.0, 7.7 Hz, 1H), 7.65 (s, 1H), 7.86 (dd, J = 2.0, 11.0 Hz, 1H), 7.96 (s, 1H), 8.34 (d, J = 1.6 Hz, 1H), 9.11 (s, 2H), 11.11 (s, 1H).

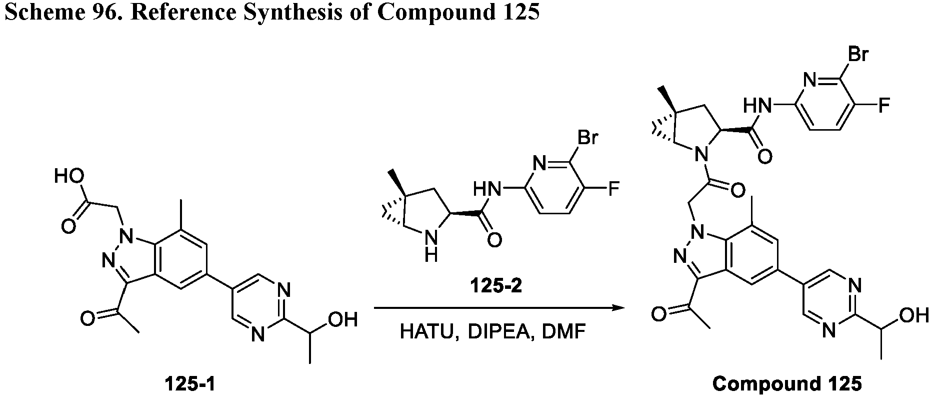

**(1R,3S,5R)-2-(2-(3-acetyl-5-(2-(1-hydroxyethyl)pyrimidin-5-yl)-7-methyl-1H-indazol-1-yl)acetyl)-N-(6-bromo-5-fluoropyridin-2-yl)-5-methyl-2-azabicyclo[3.1.0]hexane-3-carboxamide (Compound 125):** To a solution of compound **125-1** (0.03 g, 0.085 mmol, 1 equiv.) in dimethylformamide (1 mL) at 0 °C under an atmosphere of argon were added compound **125-2** (0.027 g, 0.085 mmol, 1 equiv.), HATU (0.068 g, 0.178 mmol, 2.1 equiv.), and DIPEA (0.055 g, 0.423 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 125.** ¹H NMR (400 MHz, DMSO-d6) δ 0.89 - 0.93 (m, 1H), 1.00 (t, J = 5.4 Hz, 1H), 1.31 (s, 3H), 1.47 (d, J = 6.6 Hz, 3H), 2.00 (dd, J = 6.1, 13.3 Hz, 1H), 2.45 - 2.56 (m, 1H), 2.65 (s, 3H), 2.71 (s, 3H), 3.64 (dd, J = 2.4, 5.5 Hz, 1H), 4.42 (dd, J = 6.1, 9.1 Hz, 1H), 4.85 (p, J = 6.3 Hz, 1H), 5.25 (d, J = 5.5 Hz, 1H), 5.64 - 5.79 (m, 1H), 6.08 (d, J = 17.9 Hz, 1H), 7.64 (s, 1H), 7.84 - 7.95 (m, 1H), 8.06 (dd, J = 3.3, 8.9 Hz, 1H), 8.33 (d, J = 1.6 Hz, 1H), 9.11 (s, 2H), 10.88 (s, 1H).

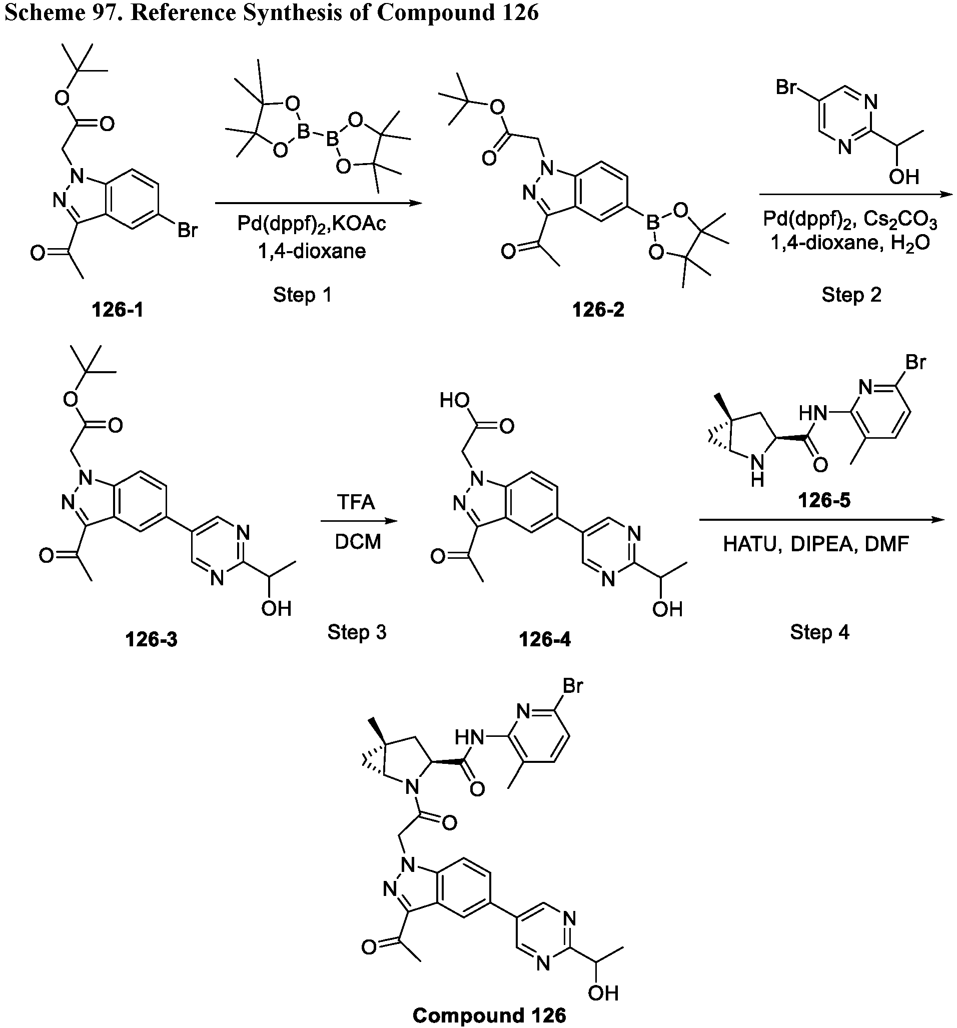

**Step 1: tert-butyl 2-(3-acetyl-5-(4,4,5,5-tetramethyl-1,3,2-dioxaborolan-2-yl)-1H-indazol-1-yl)acetate (126-2):** To a solution of compound **126-1** (0.1 g, 0.283 mmol, 1 equiv.) in 1,4-dioxane (1 mL) under an atmosphere of argon were added bis(pinacolato)diboron (0.072 g, 0.283 mmol, 1 equiv.), KOAc (0.056 g, 0.566 mmol, 2 equiv.) and [1,1'-bis(diphenylphosphino)ferrocene]dichloropalladium(II) (0.012 g, 0.014 mmol, 0.05 equiv.). The reaction mixture was stirred at 90 °C for 4 h and was used in the next synthetic step without purification.

**Step 2: tert-butyl 2-(3-acetyl-5-(2-(1-hydroxyethyl)pyrimidin-5-yl)-1H-indazol-1-yl)acetate (126-3):** To a solution of compound **126-2** (0.113 g, 0.283 mmol, 1 equiv.) in 1,4-dioxane (1 mL) and water (0.1 mL) were added 1-(5-bromopyrimidin-2-yl)ethanol (0.057 g, 0.283 mmol, 1 equiv.), cesium carbonate (0.184 g, 0.566 mmol, 2 equiv.), and [1,1'-bis(diphenylphosphino)ferrocene]dichloropalladium(II) (0.012 g, 0.014 mmol, 0.05 equiv.). The reaction mixture was stirred at 90 °C for 5 h and then concentrated to dryness. The residue was purified by column chromatography on silica gel (eluted with DCM/ MeOH) to give compound **126-3.**

**Step 3: 2-(3-acetyl-5-(2-(1-hydroxyethyl)pyrimidin-5-yl)-1H-indazol-1-yl)acetic acid (126-4):** To a solution of compound **126-3** (0.092 g, 0.231 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The material was used directly in the next synthetic step without purification.

**Step 4: (1R,3S,5R)-2-(2-(3-acetyl-5-(2-(1-hydroxyethyl)pyrimidin-5-yl)-1H-indazol-1-yl)acetyl)-N-(6-bromo-3-methylpyridin-2-yl)-5-methyl-2-azabicyclo[3.1.0]hexane-3-carboxamide (Compound 126):** To a solution of compound **126-5** (0.033 g, 0.096 mmol, 1 equiv.) in dimethylformamide (1 mL) at 0 °C under an atmosphere of argon were added compound **126-4** (0.03 g, 0.096 mmol, 1 equiv.), HATU (0.077 g, 0.202 mmol, 2.1 equiv.), and DIPEA (0.062 g, 0.11 mL, 0.480 mmol, 5 equiv.). The reaction mixture was stirred at room temperature for 1 h and then quenched with water (10 vol). The mixture was extracted with DCM and the organic layer was washed with brine, dried over anhydrous Na₂SO₄, filtered, and then concentrated to dryness. The residue was purified by column chromatography on silica gel (eluted with DCM/MeOH) to give **Compound 126.** ¹H NMR (400 MHz, DMSO-d6) δ 0.97 - 1.06 (m, 2H), 1.23 - 1.29 (m, 1H), 1.33 (s, 3H), 1.48 (d, J = 6.5 Hz, 3H), 2.05 (s, 4H), 2.55 - 2.61 (m, 1H), 2.67 (s, 3H), 3.60 (dd, J = 5.6, 2.4 Hz, 1H), 4.42 (dd, J = 9.2, 5.1 Hz, 1H), 4.87 (q, J = 6.6 Hz, 1H), 5.59 (d, J = 17.2 Hz, 1H), 5.93 (d, J = 17.3 Hz, 1H), 7.45 (d, J = 7.9 Hz, 1H), 7.63 (d, J = 8.0 Hz, 1H), 7.82 - 7.91 (m, 2H), 8.48 (s, 1H), 9.14 (s, 2H), 10.26 (s, 1H).

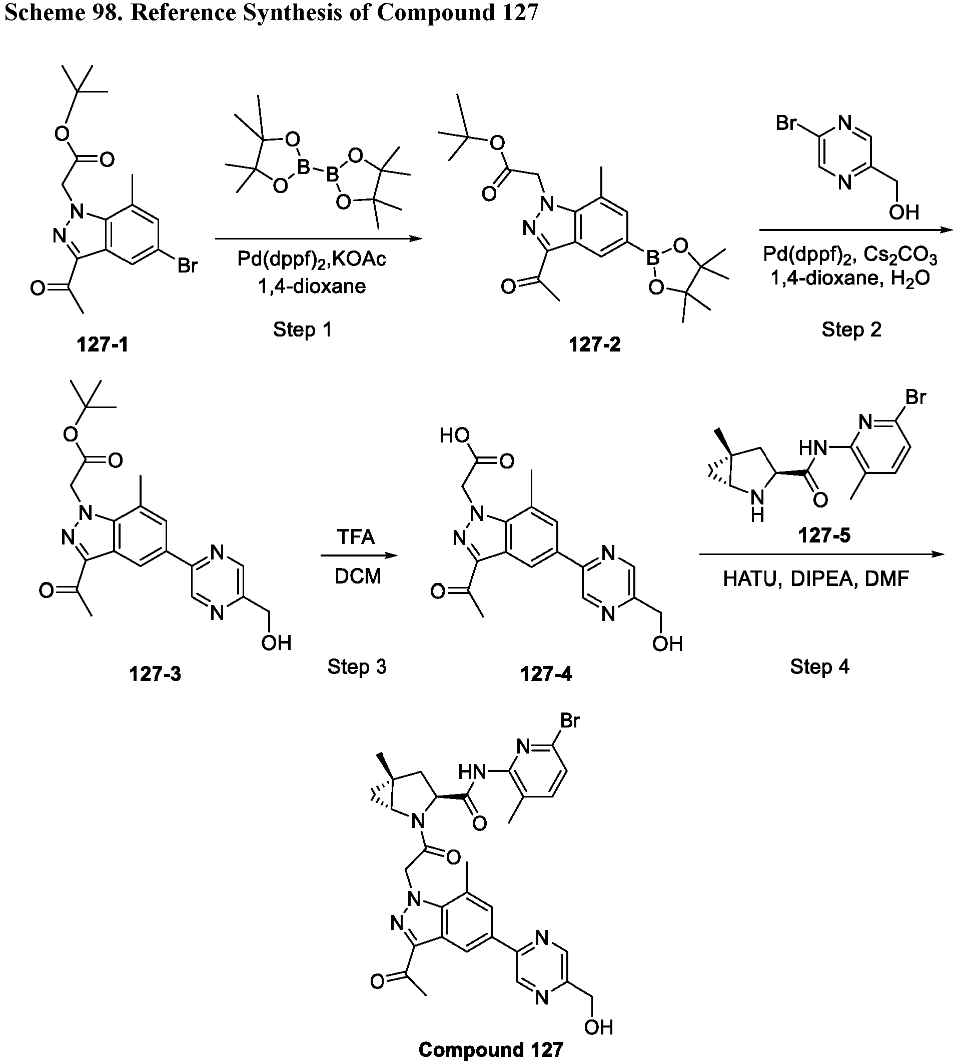

**Step 1: tert-butyl 2-(3-acetyl-7-methyl-5-(4,4,5,5-tetramethyl-1,3,2-dioxaborolan-2-yl)-1H-indazol-1-yl)acetate (127-2):** To a solution of compound **127-1** (0.136 g, 0.37 mmol, 1 equiv.) in 1,4-dioxane (1 mL) under an atmosphere of argon were added bis(pinacolato)diboron (0.094 g, 0.37 mmol, 1 equiv.), KOAc (0.073 g, 0.741 mmol, 2 equiv.), and [1,1'-bis(diphenylphosphino)ferrocene]dichloropalladium(II) (0.015 g, 0.019 mmol, 0.05 equiv.). The reaction mixture was stirred at 90 °C for 4 h and was used in the next synthetic step without purification.

**Step 2: tert-butyl 2-(3-acetyl-5-(5-(hydroxymethyl)pyrazin-2-yl)-7-methyl-1H-indazol-1-yl)acetate (127-3):** To a solution of compound **127-2** (0.153 g, 0.37 mmol, 1 equiv.) in 1,4-dioxane (1 mL) and water (0.1 mL) were added (5-bromopyrazin-2-yl)methanol (0.07 g, 0.37 mmol, 1 equiv.), cesium carbonate (0.355 g, 1.09 mmol, 2 equiv.) and [1,1'-bis(diphenylphosphino)ferrocene]dichloropalladium(II) (0.015 g, 0.019 mmol, 0.05 equiv.). The reaction mixture was stirred at 90 °C for 5 h and then concentrated to dryness. The residue was purified by column chromatography on silica gel (eluted with DCM/ MeOH) to give compound **127-3.**

**Step 3: 2-(3-acetyl-5-(5-(hydroxymethyl)pyrazin-2-yl)-7-methyl-1H-indazol-1-yl)acetic acid (127-4):** To a solution of compound **127-4** (0.092g, 0.231 mmol, 1 equiv.) in dichloromethane (2 mL) at 0 °C under an atmosphere of argon was added trifluoroacetic acid (1 mL). The reaction mixture was stirred at rt for 3 h and then concentrated to dryness. The remaining material was used directly in the next synthetic step.

**Step 4: (1R,3S,5R)-2-(2-(3-acetyl-5-(5-(hydroxymethyl)pyrazin-2-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 127):** To a solution of compound **127-5** (0.033 g, 0.096 mmol, 1 equiv.) in dimethylformamide (2 mL) at 0 °C under an atmosphere of argon were compound **127-4** (0.03 g, 0.096 mmol, 1 equiv.), HATU (0.077 g, 0.202 mmol, 2.1 equiv.), and DIPEA (0.062 g, 0.480 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 127.** ¹H NMR (400 MHz, DMSO-d6) δ 0.92 - 0.96 (m, 1H), 1.03 (t, J = 5.5 Hz, 1H), 1.34 (s, 3H), 2.00 - 2.08 (m, 4H), 2.54 - 2.61 (m, 1H), 2.66 (s, 3H), 2.71 (s, 3H), 3.60 (dd, J = 2.4, 5.5 Hz, 1H), 4.39 - 4.48 (m, 1H), 4.70 (d, J = 5.8 Hz, 2H), 5.61 (t, J = 5.8 Hz, 1H), 5.70 (d, J = 17.8 Hz, 1H), 6.03 (d, J = 17.9 Hz, 1H), 7.44 (d, J = 7.9 Hz, 1H), 7.63 (d, J = 7.9 Hz, 1H), 8.01 (s, 1H), 8.74 - 8.90 (m, 2H), 9.18 (d, J = 1.5 Hz, 1H), 10.31 (s, 1H).

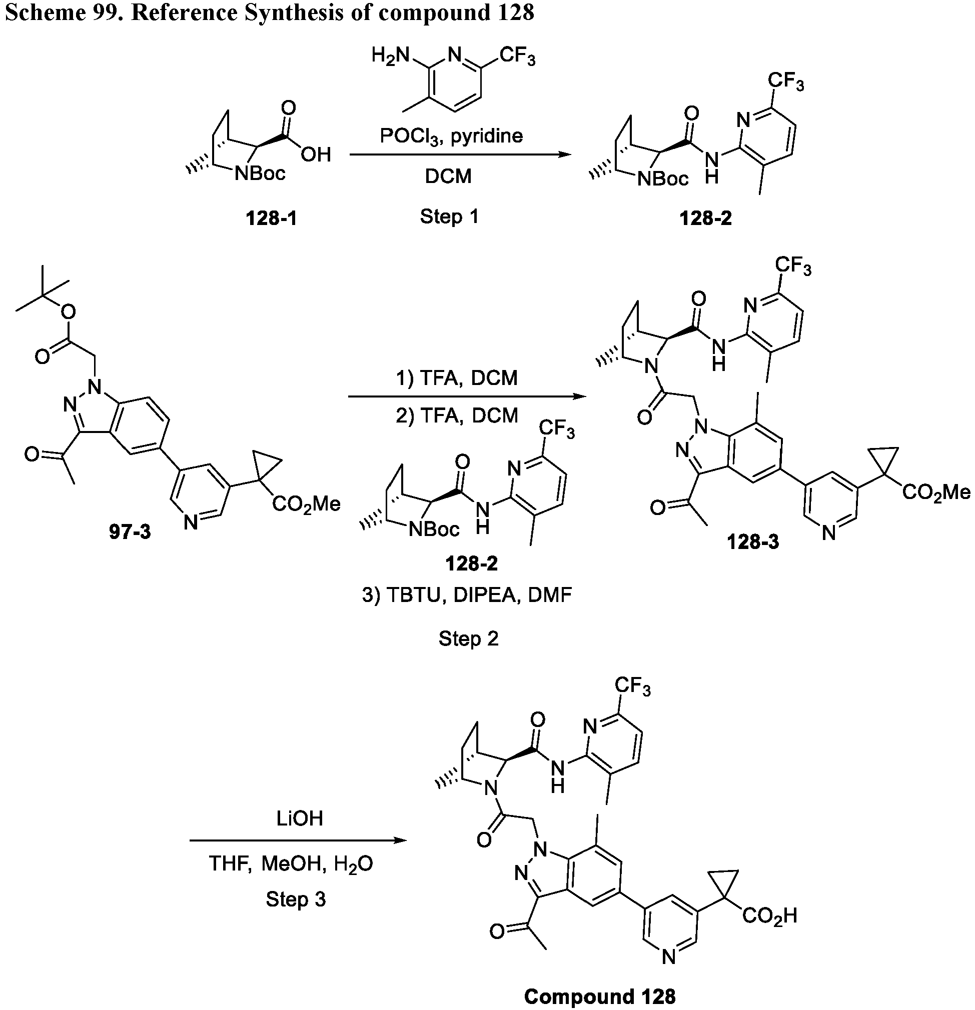

**Step 1. *tert-Butyl* (1S,3S,4R)-3-((3-methyl-6-(trifluoromethyl)pyridin-2-yl)carbamoyl)-2-azabicyclo[2.2.1]heptane-2-carboxylate (128-2):** To an ice-cooled solution of compound **128-1** ((3S)-2-(*tert*-butoxycarbonyl)-2-azabicyclo[2.2.1]heptane-3-carboxylic acid, 0.1 g, 0.414 mmol, 1 equiv.), 3-methyl-6-(trifluoromethyl)pyridin-2-amine (0.073 g, 0.414 mmol, 1 equiv.), and pyridine (0.168 mL, 2.072 mmol, 5 equiv.) in dichloromethane (2 mL) was added phosphorous oxychloride (0.037 mL, 1 equiv.) dropwise. The solution was stirred at 0 °C for 1 h and quenched by the careful addition of saturated aqueous sodium bicarbonate solution. The reaction mixture was then stirred at rt for 30 min, diluted with dichloromethane, and washed repeatedly with cold 1N aqueous HCl to remove unreacted 3-methyl-6-(trifluoromethyl)pyridin-2-amine. Finally, the organic layer was washed with water, dried, and concentrated to give 0.10 g of pale yellow solid, which was used in the next step without further purification.

**Step 2. Methyl 1-(5-(3-acetyl-7-methyl-1-(2-((1S,3S,4R)-3-((3-methyl-6-(trifluoromethyl)pyridin-2-yl)carbamoyl)-2-azabicyclo[2.2.1]heptan-2-yl)-2-oxoethyl)-1*H-*indazol-5-yl)pyridin-3-yl)cyclopropane-1-carboxylate (128-3):** Compound **97-3** (methyl 1-(5-{3-acetyl-1-[2-(*tert*-butoxy)-2-oxoethyl]-7-methylindazol-5-yl}pyridin-3-yl)cyclopropane-1-carboxylate, 0.05 g, 0.108 mmol, 1 equiv.) was stirred with 2 mL of TFA and 1 mL of dichloromethane for 3 h at rt in a vial. In another vial, compound **128-2** (*tert*-butyl (3S)-3-{[3-methyl-6-(trifluoromethyl)pyridin-2-yl]carbamoyl}-2-azabicyclo[2.2.1]heptane-2-carboxylate, 0.047 g, 0.119 mmol, 1.1 equiv.) was stirred with 1 mL of TFA and 1 mL of dichloromethane for 30 min at rt. The contents of both the vials were combined and the volatiles were removed under reduced pressure. The residue was dissolved in DMF (1 mL) and cooled in an ice bath. DIPEA (0.094 mL, 5 equiv.) and TBTU (0.038 g, 0.119 mmol, 1.1 equiv.) were added successively and the cooling bath was removed. The reaction mixture was stirred at rt for 30 min and the solvent was removed under reduced pressure. The residue was dissolved in dichloromethane and washed with saturated aqueous sodium bicarbonate. The organic layer was dried (Na₂SO₄) and concentrated, and the crude product was purified by silica gel flash column chromatography (eluent: 0-2.5 % MeOH in dichloromethane) to give compound **128-3** as a white solid

**Step 3. 1-(5-(3-Acetyl-7-methyl-1-(2-((1S,3S,4R)-3-((3-methyl-6-(trifluoromethyl)pyridin-2-yl)carbamoyl)-2-azabicyclo[2.2.1]heptan-2-yl)-2-oxoethyl)-1*H-*indazol-5-yl)pyridin-3-yl)cyclopropane-1-carboxylic acid (Compound 128):** To a solution of compound **128-3** (methyl 1-[5-(3-acetyl-7-methyl-1-{2-[(3S)-3-{[3-methyl-6-(trifluoromethyl)pyridin-2-yl]carbamoyl}-2-azabicyclo[2.2.1]heptan-2-yl]-2-oxoethyl}indazol-5-yl)pyridin-3-yl]cyclopropane-1-carboxylate, 0.04 g, 0.058 mmol, 1 equiv.) in THF (2 mL) and MeOH (1 mL) was added lithium hydroxide (0.004 g, 0.087 mmol, 1.5 equiv.) in water (1 mL). The rm was stirred for 24 h, then the organic solvents were removed under reduced pressure. The aqueous phase was extracted once with ether and the ether extracts were discarded. The aqueous layer was cooled in an ice bath and acidified with 2N aqueous HCl. Some white precipitate appeared, which dissolved during the addition of additional aqueous HCl. This aqueous phase was then subjected to prep-HPLC to give **Compound 128** as a white solid. ¹H NMR (400 MHz, DMSO-*d*₆) δ 1.37-1.39 (m, 2H), 1.49 - 1.70 (m, 5H), 1.78-1.87 (m, 3H), 2.19 (s, 3H), 2.64 (s, 3H), 2.73 (s, 3H), 2.92 (s, 1H), 4.47 (d, *J=* 3.6 Hz, 1H), 4.61 (s, 1H), 5.72 (d, J =17.7 Hz, 1H), 5.84 (d, *J=* 17.7 Hz, 1H), 7.61 (s, 1H), 7.67 (d, J = 8 Hz, 1H), 7.93 (d, *J* = 7.8 Hz, 1H), 8.30 - 8.39 (m, 2H), 8.70 (s, 1H), 8.91 (s, 1H), 10.49 (s, 1H), 12.63 (s, 1H).

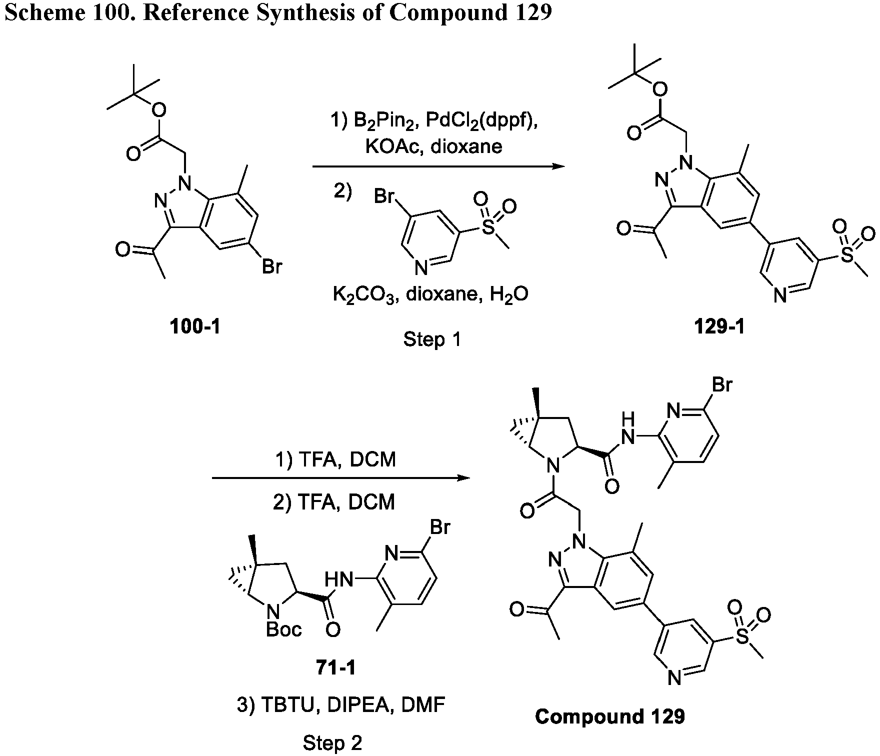

**Step 1. *tert-*Butyl 2-(3-acetyl-7-methyl-5-(5-(methylsulfonyl)pyridin-3-yl)-1H-indazol-1-yl)acetate (129-1):** A heterogeneous solution of compound **100-1** (0.104 g, 0.283 mmol, 1 equiv.), bis(pinacolato)diboron (0.108 g, 0.425 mmol, 1.5 equiv.), PdCl₂(dppf) (0.011 g, 0.014 mmol, 0.05 equiv.), and KOAc (0.056 g, 0.566 mmol, 2 equiv.) in 1,4-dioxane (4 mL) was degassed with argon and heated at 90 °C overnight with vigorous stirring. The resulting black heterogenous mixture was cooled to rt and 3-bromo-5-methanesulfonylpyridine (0.08 g, 0.34 mmol, 1.2 equiv.), potassium carbonate (0.113 g, 0.817 mmol, 3 equiv.), and water (1 mL) were added to the reaction mixture. The reaction mixture was again degassed with argon and heated at 100 °C for 4 h, then the reaction mixture was cooled to rt and the solvent was removed under reduced pressure. The residue was purified by silica gel flash column chromatography (eluent:0-2 % MeOH in dichloromethane) to give compound **129-1** as a colorless resin.

**Step 2. (1R,3S,5R)-2-(2-(3-Acetyl-7-methyl-5-(5-(methylsulfonyl)pyridin-3-yl)-1H-indazol-1-yl)acetyl)-N-(6-bromo-3-methylpyridin-2-yl)-5-methyl-2-azabicyclo[3.1.0]hexane-3-carboxamide (Compound 129):** Compound **129-1** (0.088 g, 0.08 mL, 0.197 mmol, 1 equiv.) was stirred with 2 mL of TFA and 1 mL of dichloromethane for 3 h at rt in a vial. In another vial, compound **71-1** (0.081 g, 0.197 mmol, 1 equiv.) was stirred with 1 mL of TFA and 1 mL of dichloromethane for 30 min at rt. The contents of both vials were combined and the volatiles were removed under reduced pressure. The residue was dissolved in DMF (2 mL) and cooled in an ice bath. DIPEA (0.157 mL, 5 equiv.) and TBTU (0.064 g, 1.1 equiv.) were added successively and the cooling bath was removed. The reaction mixture was stirred at rt for 30 min and the solvent was removed under reduced pressure. The residue was dissolved in dichloromethane, washed with saturated aqueous sodium bicarbonate, and the organic layer was dried (Na₂SO₄) and concentrated. The crude product was purified by silica gel flash column chromatography (eluent: 0-2.5 % MeOH in dichloromethane) to give **Compound 129** as a white solid. ¹H NMR (400 MHz, Chloroform-d) δ 0.95 (dd, *J =* 2.4, 5.5 Hz, 1H), 1.19 (t, *J =* 5.5 Hz, 1H), 1.43 (s, 3H), 2.08 (s, 3H), 2.32 (dd, *J =* 8.9, 13.5 Hz, 1H), 2.71 (s, 4H), 2.77 (s, 3H), 3.18 (s, 4H), 4.80 - 4.88 (m, 1H), 5.67 (d, *J =* 17.3 Hz, 1H), 5.74 (d, *J* = 17.3 Hz, 1H), 7.22 (d, *J* = 7.9 Hz, 1H), 7.34 (d, *J* = 7.9 Hz, 1H), 7.41 (s, 1H), 8.42 (t, *J* = 2.2 Hz, 1H), 8.51 (s, 1H), 8.67 (s, 1H), 9.13 (dd, *J* = 2.2*,* 14.9 Hz, 2H).

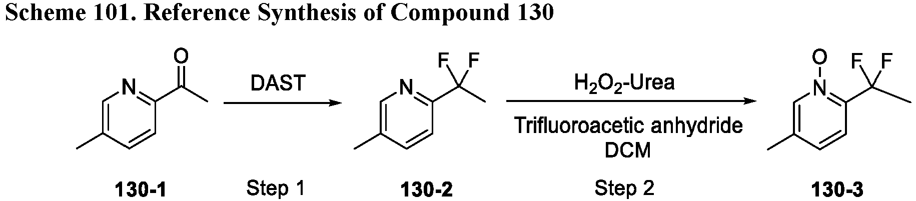

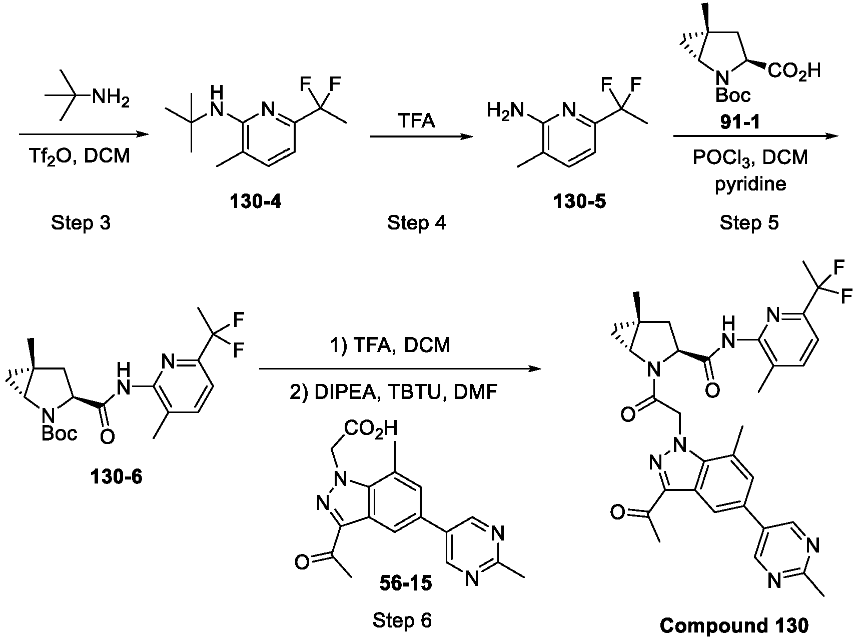

**Step 1: 2-(1,1-Difluoroethyl)-5-methylpyridine (130-2):** Prepared following the procedure from Los, M. R. et al U. S., 8288422, 2012**.**

**Step 2: 2-(1,1-Difluoroethyl)-5-methylpyridine-*N*-oxide (130-3):** To an ice-cold solution of compound **130-2** (2.2 g) in CH₂Cl₂ (50 mL), solid hydrogen peroxide urea complex (2.03 g) was added, then trifluoroacetic anhydride (3.2 mL) was added dropwise. After the addition was complete, the cooling bath was removed, and the reaction mixture was stirred overnight at rt. The reaction mixture was diluted with CH₂Cl₂, and the organic layer was washed with water, saturated aqueous sodium metabisulfite solution, and dried (Na₂SO₄). The organic layer was filtered and concentrated to give a light brown liquid, which was used in the next step without further purification.

**Step 3: *N*-(tert-Butyl)-6-(1,1-difluoroethyl)-3-methylpyridin-2-amine (130-4):** To a solution of compound **130-3** (2.2 g) at -20 °C in CH₂Cl₂ (50 mL), *tert*-butylamine (6.9 mL) was added followed by the dropwise addition of trifluoromethanesulfonic anhydride (6.6 mL). After being stirred for 1 h at -20 °C, the reaction mixture was quenched by the addition of water. The layers were separated, and the organic layer was washed with saturated K₂CO₃. Then organic layer was dried (Na₂SO₄) and concentrated. The residue was purified by silica gel flash column chromatography (eluent: 0-0.5 % EtOAc in hexanes) to give compound **130-4** as a colorless liquid.

**Step 4: 6-(1,1-Difluoroethyl)-3-methylpyridin-2-amine (130-5):** A solution of compound **130-4** (0.303 g) in TFA (3 mL) was stirred at rt overnight. The volatiles were removed under reduced pressure. The residue was dissolved in dichloromethane and washed with saturated aqueous sodium bicarbonate. The organic layer was separated, dried (Na₂SO₄), and concentrated to give a light-yellow oil, which was used without further purification.

**Step 5: *tert-*Butyl (1R,3S,5R)-3-((6-(1,1-difluoroethyl)-3-methylpyridin-2-yl)carbamoyl)-5-methyl-2-azabicyclo[3.1.0]hexane-2-carboxylate (130-6):** To an ice-cold solution of compound **91-1** (0.112 g, 0.464 mmol, 1 equiv.), compound **130-5** (0.08 g, 0.464 mmol, 1 equiv.), and pyridine (0.184 g, 0.188 mL, 2.321 mmol, 5 equiv.) in dichloromethane (2 mL) was added phosphorus oxychloride (0.071 g, 0.042 mL, 0.464 mmol, 1 equiv.) dropwise. After the addition was complete, the reaction mixture was stirred at 0 °C for 1h. The reaction was then quenched by the addition of saturated aqueous sodium bicarbonate and stirred at rt for 15 min. The layers were separated, and the aqueous layer was extracted with dichloromethane. The combined organic layers were washed with cold 1M aqueous HCl solution (2 X 25 mL) and with water. The organic layer was dried (Na₂SO₄), filtered, and concentrated to give compound **130-6** as a light yellow solid, which was used in the next step without further purification.

**Step 6: (1R,3S,5R)-2-(2-(3-Acetyl-7-methyl-5-(2-methylpyrimidin-5-yl)-1*H*-indazol-1-yl)acetyl)-N-(6-(1,1-difluoroethyl)-3-methylpyridin-2-yl)-5-methyl-2-azabicyclo[3.1.0]hexane-3-carboxamide (Compound 130):** Compound **130-6** (0.07 g, 0.177 mmol, 1 equiv.) was stirred at rt with 1 mL of TFA and 1 mL of dichloromethane for 30 min. The volatiles were removed under reduced pressure, and the residue was dissolved in DMF (1 mL). Compound **56-15** (0.057 g, 0.177 mmol, 1 equiv.) was added to this solution followed by the addition of DIPEA (0.154 mL, 0.885 mmol, 5 equiv.). The reaction mixture was cooled in an ice-bath and TBTU (0.063 g, 0.195 mmol, 1.1 equiv.) was added, then the cooling bath was removed and the reaction mixture was stirred at rt for 30 min. The solvent was removed under reduced pressure, and the residue was dissolved in dichloromethane, then washed with saturated aqueous sodium bicarbonate. Finally, the organic layer was dried (Na₂SO₄) and concentrated. The residue was purified by silica gel flash column chromatography (eluent: 0-2.5 % MeOH in dichloromethane) to give **Compound 130** as cream colored solid. ¹H NMR (400 MHz, Chloroform-d) δ 0.94 (dd, *J* = 2.4*,* 5.6 Hz, 1H), 1.19 (t, *J=* 5.5 Hz, 1H), 1.44 (s, 3H), 1.91 (t, *J* = 18.5 Hz, 3H), 2.16 (s, 3H), 2.33 (dd, *J* = 8.8, 13.4 Hz, 1H), 2.70 (s, 3H), 2.75 (s, 3H), 2.80 (s, 3H), 3.18 (dd, *J=* 2.3, 5.6 Hz, 1H), 4.89 (d, *J=* 8.1 Hz, 1H), 5.67 (d*, J* = 17.2 Hz, 1H), 5.74 (d, *J=* 17.3 Hz, 1H), 7.31 - 7.41 (m, 2H), 7.58 (d, *J=* 7.8 Hz, 1H), 8.46 (s, 1H), 8.62 (s, 1H), 8.89 (s, 2H). ¹⁹F NMR (376 MHz, Chloroform-*d*) δ -91.1.

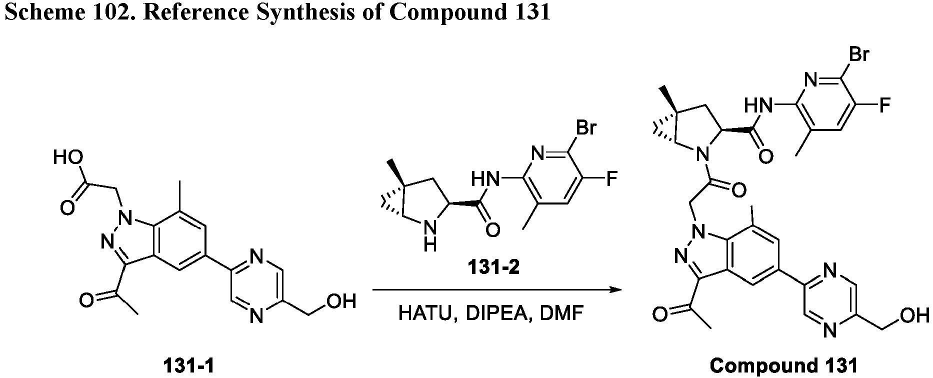

**(1R,3S,5R)-2-(2-(3-acetyl-5-(5-(hydroxymethyl)pyrazin-2-yl)-7-methyl-1H-indazol-1-yl)acetyl)-N-(6-bromo-5-fluoro-3-methylpyridin-2-yl)-5-methyl-2-azabicyclo[3.1.0]hexane-3-carboxamide (Compound 131):** To a solution of compound **131-1** (0.01 g, 0.029 mmol, 1 equiv.) in dimethylformamide (1 mL) at 0 °C under an atmosphere of argon were added compound **131-2** (0.01 g, 0.029 mmol, 1 equiv.), HATU (0.023 g, 0.062 mmol, 2.1 equiv.), and DIPEA (0.019 g, 0.147 mmol, 5 equiv.). The reaction mixture was stirred at room temperature for 1 h and then quenched with water (10 vol). The mixture was extracted with DCM, and the organic layer was washed with brine, dried over anhydrous Na₂SO₄, filtered, and then concentrated to dryness. The residue was purified by column chromatography on silica gel (eluted with DCM/MeOH) to give **Compound 131.** ¹H NMR (400 MHz, DMSO-d6) δ 0.91 - 0.98 (m, 1H), 1.03 (t, J = 5.4 Hz, 1H), 1.33 (s, 3H), 1.98 - 2.10 (m, 4H), 2.53 - 2.59 (m, 1H), 2.66 (s, 3H), 2.70 (s, 3H), 3.60 (dd, J = 2.4, 5.6 Hz, 1H), 4.41 (dd, J = 5.5, 9.2 Hz, 1H), 4.70 (d, J = 5.8 Hz, 2H), 5.61 (d, J = 5.9 Hz, 1H), 5.74 (d, J = 14.8 Hz, 1H), 6.03 (d, J = 17.8 Hz, 1H), 7.83 (d, J = 8.4 Hz, 1H), 8.01 (s, 1H), 8.76 - 8.85 (m, 2H), 9.18 (d, J = 1.4 Hz, 1H), 10.31 (s, 1H).

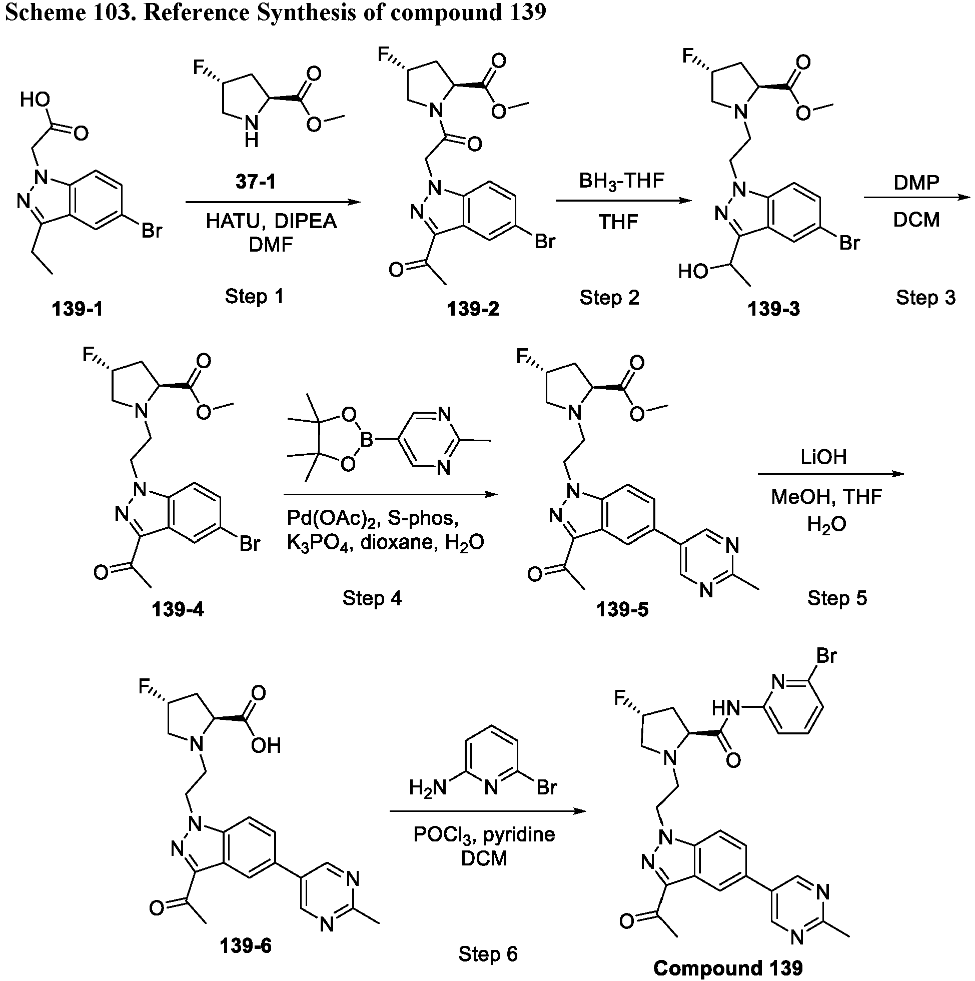

**Step 1: (2S,4R)-methyl 1-(2-(3-acetyl-5-bromo-1H-indazol-1-yl)acetyl)-4-fluoropyrrolidine-2-carboxylate (139-2):** To a solution of compound **139-1** (1.0 g, 3.38 mmol) and compound **37-1** (497 mg, 3.38 mmol) in DMF (10 mL) was added DIPEA (2.18 g, 16.9 mmol) followed by HATU (1.927 g, 5.07 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by chromatography on silica gel (DCM: MeOH= 100: 1 to 60: 1) to give compound **139-2** (1.25 g, yield 87.1%) as a white solid. LC/MS (ESI) m/z: 426/428 (M+H)⁺.

**Step 2: (2S,4R)-methyl 1-(2-(5-bromo-3-(1-hydroxyethyl)-1H-indazol-1-yl)ethyl)-4-fluoropyrrolidine-2-carboxylate (139-3):** To a solution of compound **139-2** (600 mg, 1.4 mmol) in THF (30 mL) was added BH₃-THF (7mL, 7 mmol, 1M) drop-wise at 0 °C, and the mixture was stirred at 65 °C under N₂ atmosphere for 2 hrs. The reaction mixture was cooled to 0 °C and quenched via the drop-wise addition of MeOH (8 mL) and the mixture was stirred at 70 °C for 1 hr. The mixture was concentrated to dryness and diluted with EtOAc, washed with brine, dried, and concentrated to dryness. The residue was purified by chromatography on silica gel (DCM: MeOH= 100: 1) to give compound **139-3** (120 mg, yield 20.8%) as a white solid. LC/MS (ESI) m/z: 414/416 (M+H)⁺.

**Step 3: (2S,4R)-methyl 1-(2-(3-acetyl-5-bromo-1H-indazol-1-yl)ethyl)-4-fluoropyrrolidine-2-carboxylate (139-4):** To a solution of compound **139-3** (120 mg, 0.29 mmol) in DCM was added Dess-Martin Periodinane (246 mg, 0.58 mmol) and the mixture was stirred at room temperature for 2 hrs. The mixture was diluted with DCM and washed with saturated aqueous NaHCO₃ solution and brine, dried over anhydrous Na₂SO₄, filtered, and concentrated to dryness. The residue was purified by chromatography on silica gel (PE: EtOAc= 10: 1 to 5: 1) to give compound **139-4** (77 mg, yield 64.6%) as a light-yellow oil. LC/MS (ESI) m/z: 412/414 (M+H)⁺.

**Step 4: (2S,4R)-methyl 1-(2-(3-acetyl-5-(2-methylpyrimidin-5-yl)-1H-indazol-1-yl)ethyl)-4-fluoropyrrolidine-2-carboxylate (139-5):** To a mixture of compound **139-4** (50 mg, 0.12 mmol), 2-methyl-5-(4,4,5,5-tetramethyl-1,3,2-dioxaborolan-2-yl)pyrimidine (42 mg, 0.19 mmol), and K₂PO₄ (64mg, 0.3 mmol) in 1,4-dioxane (4 mL) and water (0.5 mL) was added Pd(OAc)₂ (3 mg, 0.012 mmol) and S-phos (5 mg, 0.012 mmol), and the mixture was degassed under N₂ three times. The reaction mixture was stirred at 95 °C under N₂ atmosphere for 4 hrs. The mixture was filtered, and the filtrate was diluted with water then extracted with EtOAc twice. The combined organic layers were washed with brine, dried over anhydrous Na₂SO₄, filtered, and concentrated under reduced pressure. The residue was purified by chromatography on silica gel (DCM: MeOH= 100: 1 to 40: 1) to give compound **139-5** (43 mg, yield 84.3%) as a light yellow solid. LC/MS (ESI) m/z: 426 (M+H)⁺.

**Step 5: (2S,4R)-1-(2-(3-acetyl-5-(2-methylpyrimidin-5-yl)-1H-indazol-1-yl)ethyl)-4-fluoropyrrolidine-2-carboxylic acid (139-6):** To a solution of compound **139-5** (43 mg, 0.10 mmol) in methanol (2 mL) and THF (1 mL) was added a solution of LiOH (21 mg, 0.50 mmol) in water (1 mL) at 0 °C and the mixture was stirred at room temperature for 1 hr. The mixture was diluted with water and washed with ether. The aqueous layer was acidified with 0.5N aqueous HCl to pH~3 and extracted with DCM twice. The combined organic layers were washed with brine, dried with anhydrous Na₂SO₄, filtered, and concentrated to dryness to give compound **139-6** (40 mg, yield 97.6%) as a yellow solid. LC/MS (ESI) m/z: 412 (M+H)⁺.

**Step 6: (2S,4R)-1-(2-(3-acetyl-5-(2-methylpyrimidin-5-yl)-1H-indazol-1-yl)ethyl)-N-(6-bromopyridin-2-yl)-4-fluoropyrrolidine-2-carboxamide (Compound 139):** To a solution of compound **139-6** (20 mg, 0.049 mmol) and 6-bromopyridin-2-amine (8 mg, 0.049 mmol) in DCM (3 mL) was added pyridine (32 mg, 0.25 mmol) and POCl₃ (8 mg, 0.054 mmol) at 0 °C, and the mixture was stirred at room temperature for 1 hr. The mixture was poured into ice water and extracted with DCM twice. The combined organic layers were washed with brine, dried with anhydrous Na₂SO₄, filtered, and concentrated to dryness. The residue was purified by prep-HPLC to give **Compound 139** (2.7 mg, yield 9.8%) as a white solid. ¹H-NMR (400 MHz, CD₃OD) δ 8.94 (s, 2H), 8.48 (s, 1H), 7.82 (dd, J = 8.8, 8.8 Hz, 2H), 7.68 (dd, J = 8.8, 8.8 Hz, 1H), 7.50 (t, J = 8.0 Hz, 1H), 7.11 (d, J = 7.6 Hz, 1H), 5.26 (t, J = 4.2 Hz, 0.5H), 5.13 (t, J = 3.8 Hz, 0.5H), 4.78 - 4.72 (m, 1H), 4.63 - 4.57 (m, 1H), 3.71 (dd, J = 10.0, 10.0 Hz, 1H), 3.62 - 3.47 (m, 2H), 3.44 - 3.37 (m, 1H), 3.10 (dd, J = 26.4, 26.4 Hz, 1H), 2.75 (s, 3H), 2.57 (s, 3H), 2.53 - 2.42 (m, 1H), 2.01 - 1.86 (m, 1H). LC/MS (ESI) m/z: 566/568 (M+H)⁺.

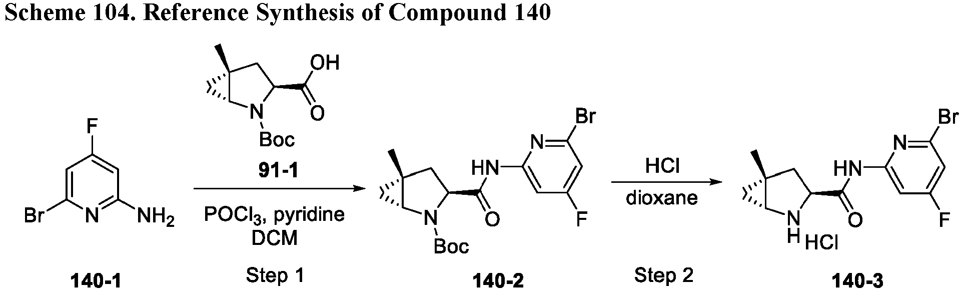

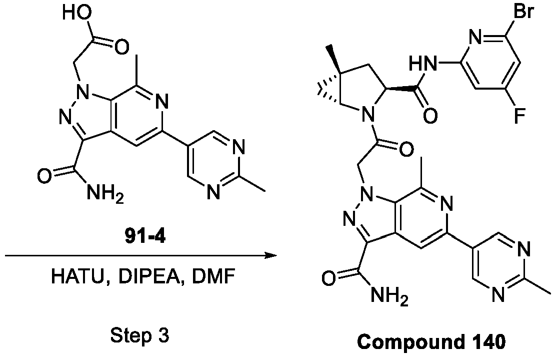

**Step 1: 3-(6-Bromo-4-fluoro-pyridin-2-ylcarbamoyl)-5-methyl-2-aza-bicyclo[3.1.0]hexane-2-carboxylic acid tert-butyl ester (140-2):** To a solution of compound **140-1** (15 mg, 0.079 mmol) and compound **91-1** (19 mg, 0.079 mmol) in DCM (3 mL) was added pyridine (31 mg, 0.4 mmol) and POCl₃ (18 mg, 0.12 mmol) at 0 °C, and the mixture was stirred at room temperature for 1 hr. The mixture was poured into ice water and extracted with DCM twice. The combined organic layers were washed with brine, dried with anhydrous Na₂SO₄, filtered, and concentrated to dryness. The residue was purified by chromatography on silica gel (PE: EtOAc= 30: 1 to 12: 1) to give compound **140-2** (20 mg, yield 61.3%) as a light oil. LC/MS (ESI) m/z: 414/416 (M+H)⁺.

**Step 2: 5-Methyl-2-aza-bicyclo[3.1.0]hexane-3-carboxylic acid (6-bromo-4-fluoro-pyridin-2-yl)-amide hydrochloride (140-3):** A solution of 4 M hydrogen chloride in 1,4-dioxane (3 mL) containing compound **140-2** (20 mg, 0.048 mmol) was stirred at room temperature for 1 hr. The reaction mixture was concentrated to dryness, washed with ether, and dried under vacuum to give compound **140-3** (15 mg, yield 86.2%) as a white solid. LC/MS (ESI) m/z: 314/316 (M+H)⁺.

**Step 3: 1-(2-((1R,3S,5R)-3-((6-bromo-4-fluoropyridin-2-yl)carbamoyl)-5-methyl-2-azabicyclo[3.1.0]hexan-2-yl)-2-oxoethyl)-7-methyl-5-(2-methylpyrimidin-5-yl)-1H-pyrazolo[3,4-c]pyridine-3-carboxamide (Compound 140):** To a mixture of compound **140-3** (15 mg, 0.048 mmol) and compound **91-4** (16 mg, 0.048 mmol) in DMF (3 mL) was added DIPEA (31 mg, 0.24 mmol) followed by HATU (27 mg, 0.072 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by prep-TLC (DCM: MeOH= 40: 1) to **Compound 140** (3.0 mg, yield 10.1%) as a white solid. ¹H-NMR (400 MHz, CD₃OD) δ 9.33 (s, 2H), 8.55 (s, 1H), 7.90 (d, *J* = 10.8 Hz, 1H), 7.14 (dd, *J* = 7.6, 7.6 Hz, 1H), 6.02 (d, *J* = 17.6 Hz, 1H), 5.85 (d, *J* = 17.6 Hz, 1H), 4.49 (dd, *J =* 9.2, 9.2 Hz, 1H), 3.59 - 3.54 (m, 1H), 2.97 (s, 3H), 2.75 (s, 3H), 2.58 (dd, J = 13.2, 13.2 Hz, 1H), 2.18 (dd, J = 13.2, 13.2 Hz, 1H), 1.38 (s, 3H), 1.11 (t, J = 5.6 Hz, 1H), 0.98 - 0.93 (m, 1H). LC/MS (ESI) m/z: 622/624 (M+H)⁺.

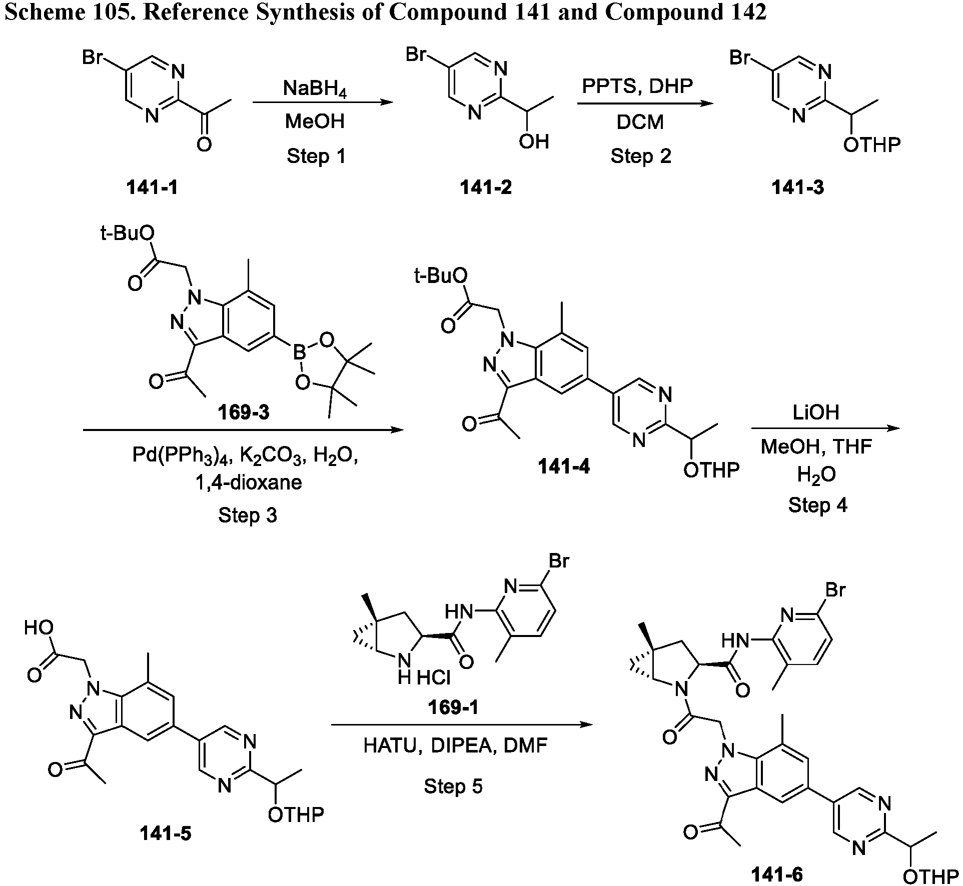

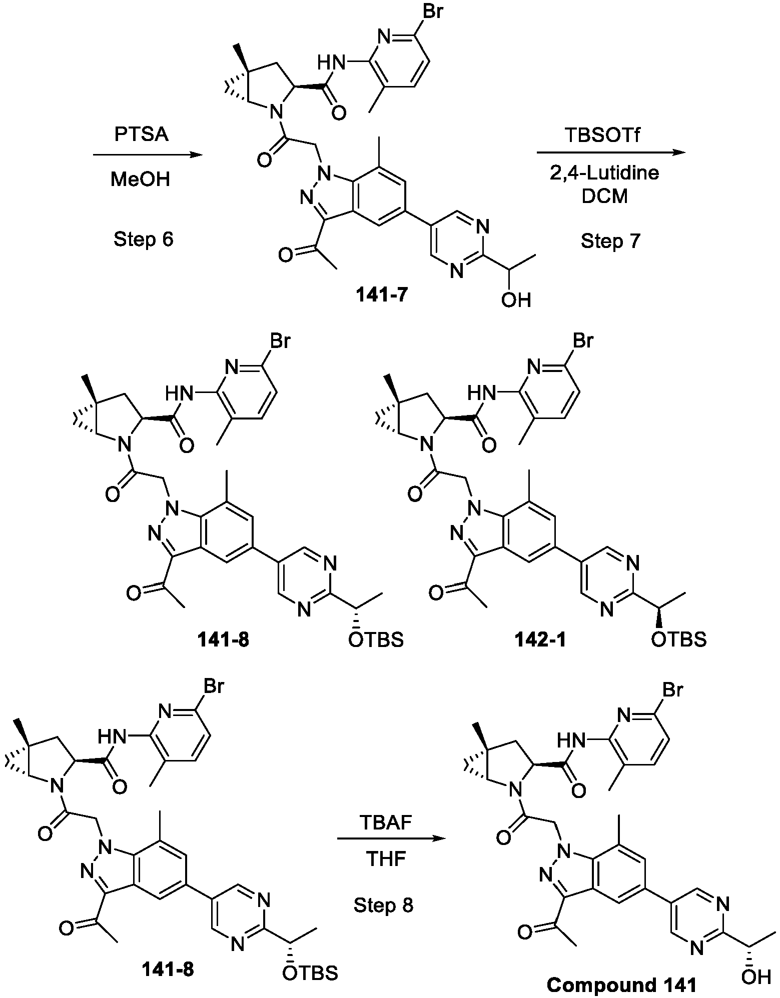

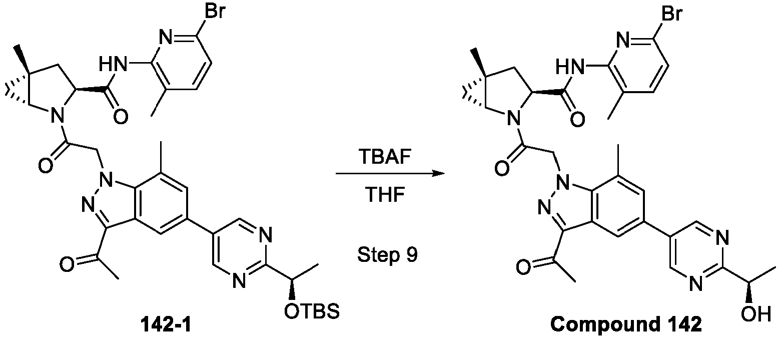

**Step 1: 1-(5-bromopyrimidin-2-yl)ethanol (141-2):** To a solution of compound **141-1** (1.0 g, 5.0 mmol) in MeOH (12 mL) was added NaBH₄ (190 mg, 5.0 mmol) at 0 °C and the mixture was stirred at room temperature for 1 hr. The mixture was quenched with ice water and extracted with EtOAc. The mixture was washed with water and brine, dried over anhydrous Na₂SO₄, filtered, and concentrated to dryness. The residue was purified by chromatography on silica gel (PE: EtOAc= 50: 1 to 5: 1) to give compound **141-2** (320 mg, yield 31.7%) as a white solid. LC/MS (ESI) m/z: 203/205 (M+H)⁺.

**Step 2: 5-bromo-2-(1-((tetrahydro-2H-pyran-2-yl)oxy)ethyl)pyrimidine (141-3):** To a solution of compound **141-2** (320 mg, 1.58 mmol) in DCM (8 mL) was added PPTS (138 mg, 0.55 mmol) followed by 3,4-Dihydro-2H-pyran (265 mg, 3.16 mmol) at 0 °C and the mixture was stirred at room temperature under N₂ atmosphere for 16 hrs. The mixture was poured into ice water and extracted with DCM twice. The combined organic layers were washed with brine, dried with anhydrous Na₂SO₄, filtered, and concentrated to dryness. The residue was purified by chromatography on silica gel (PE: EtOAc= 100: 1 to 8: 1) to give compound **141-3** (450 mg, yield 99.6%) as a light oil. LC/MS (ESI) m/z: 287/289 (M+H)⁺.

**Step 3: tert-butyl 2-(3-acetyl-7-methyl-5-(2-(1-((tetrahydro-2H-pyran-2-yl)oxy)ethyl)pyrimidin-5-yl)-1H-indazol-1-yl)acetate (141-4):** To a mixture of compound **169-3** (220 mg, 0.53 mmol) and compound **141-3** (152 mg, 0.53 mmol) in 1,4-dioxane (7 mL) and water (1 mL) was added K₂CO₃ (183 mg, 1.3 mmol) and Pd(PPh₃)₄ (61 mg, 0.053 mmol) at 0 °C, and the mixture was degassed under N2 atmosphere three times and stirred at 95 °C under N₂ atmosphere for 2 hrs. The mixture was poured into ice water and extracted with EtOAC twice. The combined organic layers were washed with brine, dried with anhydrous Na₂SO₄, filtered, and concentrated to dryness. The residue was purified by flash chromatography (PE: EtOAc= 50: 1 to 5: 1) to give compound **141-4** (245 mg, yield 93.9%) as yellow solid. LC/MS (ESI) m/z: 495 (M+H)⁺.

**Step 4: 2-(3-acetyl-7-methyl-5-(2-(1-((tetrahydro-2H-pyran-2-yl)oxy)ethyl)pyrimidin-5-yl)-1H-indazol-1-yl)acetic acid (141-5):** To a solution of compound **141-4** (245 mg, 0.50 mmol) in methanol (4 mL) and THF (2 mL) was added a solution of LiOH (105 mg, 2.50 mmol) in water (2 mL) at 0 °C, and the mixture was stirred at room temperature for 2 hrs. The mixture was diluted with water and washed with ether, and the aqueous layer was acidified with 0.5N aqueous HCl solution to pH~3 and extracted with DCM twice. The combined organic layers were washed with brine, dried with anhydrous Na₂SO₄, filtered, and concentrated to dryness to give compound **141-5** (218 mg, yield 99.5%) as a white solid. LC/MS (ESI) m/z: 439 (M+H)⁺.

**Step 5: (1R,3S,5R)-2-(2-(3-acetyl-7-methyl-5-(2-(1-((tetrahydro-2H-pyran-2-yl)oxy)ethyl)pyrimidin-5-yl)-1H-indazol-1-yl)acetyl)-N-(6-bromo-3-methylpyridin-2-yl)-5-methyl-2-azabicyclo[3.1.0]hexane-3-carboxamide (141-6):** To a mixture of compound **141-5** (218 mg, 0.5 mmol) and compound **169-1** ((1R,3S,5R)-N-(6-bromopyridin-2-yl)-5-methyl-2-azabicyclo[3.1.0]hexane-3-carboxamide hydrochloride, 171 mg, 0.5 mmol) in DMF (8 mL) was added DIPEA (323 mg, 2.5 mmol) followed by HATU (285 mg, 0.75 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by chromatography on silica gel (DCM: MeOH= 100: 1 to 60: 1) to give compound **141-6** (300 mg, yield 82.3%) as a white solid. LC/MS (ESI) m/z: 730/732 (M+H)⁺.

**Step 6: (1R,3S,5R)-2-(2-(3-acetyl-5-(2-(1-hydroxyethyl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (141-7):** To a solution of compound **141-6** (300 mg, 0.41 mmol) in MeOH (6 mL) was added p-toluenesulfonic acid (21 mg, 0.12 mmol) and the mixture was stirred at 60 °C for 1 hr. The reaction mixture was concentrated to dryness, and the residue was purified by chromatography on silica gel (DCM: MeOH= 100: 1 to 20: 1) to give compound **141-7** (240 mg, yield 90.9%) as white solid. LC/MS (ESI) m/z: 646/648 (M+H)⁺.

**Step 7: (1R,3S,5R)-2-(2-(3-acetyl-5-(2-(1-((tert-butyldimethylsilyl)oxy)ethyl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (141-8 and 142-1):** To a solution of compound **141-7** in DCM (8 mL) was added 2,4-lutidine (47 mg, 0.44 mmol) followed by drop-wise addition of trifluoromethanesulfonic acid tert-butyldimethylsilyl ester (92 mg, 0.35 mmol) at 0 °C, and the mixture was stirred at room temperature overnight. The mixture was diluted with DCM and washed with brine, dried over anhydrous Na₂SO₄, filtered, and concentrated to dryness. The residue was purified by chromatography on silica gel (DCM: MeOH= 100: 1 to 80: 1) to give **141-8** and **142-1** as a mixture of diastereomers. 210 mg, yield 95.5%, white solid. Chiral resolution by SFC gave compound **141-8** (60 mg, Peak 1, tᵣ=12.34 min) and **compound 142-1** (70 mg, Peak 2, tᵣ=12.99 min). LC/MS (ESI) m/z: 760/762 (M+H)⁺. SFC condition: Instrument: Waters Thar 80 preparative SFC; Column: ChiralCel OD, 250×21.2 mm I.D., 5µm; Mobile phase: A for CO₂ and B for Methanol (0.1% NH₄OH); Gradient: B 22%; Flow rate: 50 mL /min; Column temperature: 35°C.

**Step 8: (1R,3S,5R)-2-(2-(3-acetyl-5-(2-((S)-1-hydroxyethyl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 141):** A solution of compound **141-8** (60 mg, 0.079 mmol) in TBAF/THF solution (3 mL, 1M) was stirred at 60 °C for 1 hr. The reaction mixture was diluted with EtOAc and washed with saturated aqueous NH₄Cl solution and brine, dried, and concentrated to dryness. The residue was purified by prep-HPLC to give **Compound 141** (18.7 mg, yield 36.7%) as a white solid. ¹H-NMR (400 MHz, DMSO-*d₆*) δ 10.32 (s, 1H), 9.11 (s, 2H), 8.34 (d, *J =* 1.6 Hz, 1H), 7.64 - 7.61 (m, 2H), 7.44 (d, *J =* 8.0 Hz, 1H), 6.04 (d, *J* = 18.0 Hz, 1H), 5.69 (d, *J =* 17.6 Hz, 1H), 5.29 (d, *J =* 5.6 Hz, 1H), 4.88 - 4.82 (m, 1H), 4.41 (dd, *J* = 9.2, 9.2 Hz, 1H), 3.60 (dd, *J =* 5.6, 5.6 Hz, 1H), 2.69 (s, 3H), 2.65 (s, 3H), 2.56 (dd, *J* = 13.6, 13.6 Hz, 1H), 2.05 (s, 4H), 1.46 (d, *J=* 6.4 Hz, 3H), 1.33 (s, 3H), 1.02 (t, *J* = 5.4 Hz, 1H), 0.94 (dd, *J=* 5.2, 5.2 Hz, 1H). LC/MS (ESI) m/z: 646/648 (M+H)⁺.

**Step 9: (1R,3S,5R)-2-(2-(3-acetyl-5-(2-((R)-1-hydroxyethyl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 142):** A solution of compound **142-1** (70 mg, 0.11 mmol) in TBAF/THF solution (3 mL, 1M) was stirred at 60 °C for 1 hr. The reaction mixture was diluted with EtOAc and washed with saturated aqueous NH₄Cl solution and brine, dried, and concentrated to dryness. The residue was purified by prep-HPLC to give **Compound 142** (15.6 mg, yield 22.0%) as a white solid. ¹H-NMR (400 MHz, DMSO-*d₆*) δ 10.32 (s, 1H), 9.11 (s, 2H), 8.34 (d, *J* = 1.6 Hz, 1H), 7.64 - 7.60 (m, 2H), 7.44 (d, *J* = 8.0 Hz, 1H), 6.05 (d, *J* = 18.0 Hz, 1H), 5.68 (d, *J =* 18.0 Hz, 1H), 5.28 (d, *J* = 5.6 Hz, 1H), 4.85 (t, *J =* 6.0 Hz, 1H), 4.41 (dd, *J* = 9.2, 9.2 Hz, 1H), 3.60 (dd, *J* = 5.6*,* 5.6 Hz, 1H), 2.69 (s, 3H), 2.65 (s, 3H), 2.56 (dd, *J* = 13.2, 13.2 Hz, 1H), 2.04 (s, 4H), 1.46 (d, *J =* 6.4 Hz, 3H), 1.32 (s, 3H), 1.02 (t, *J =* 5.4 Hz, 1H), 0.94 (dd, *J =* 5.2, 5.2 Hz, 1H). LC/MS (ESI) m/z: 646/648 (M+H)⁺.

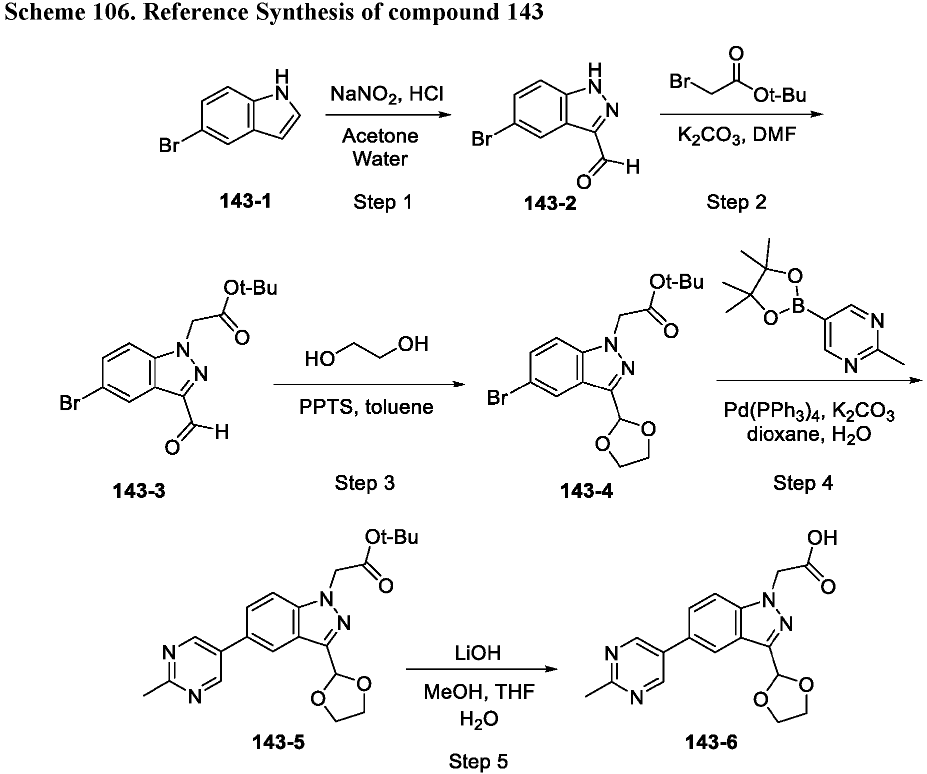

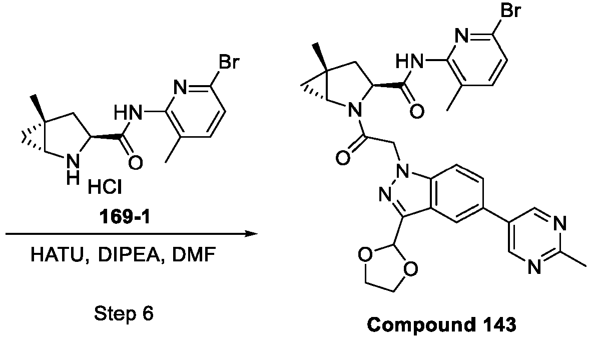

**Step 1: 5-bromo-1H-indazole-3-carbaldehyde (143-2):** To a solution of compound **143-1** (5-bromo-1H-indole, 4.0 g, 20.4 mmol) in acetone (100 mL) was added a solution of NaNO₂ (11.265 g, 163.2 mmol) in water (20 mL) via dropwise addition at -10 °C followed by 2M aqueous HCl (48.96 mL, 97.9 mmol) at 0-10°C, and the mixture was stirred at 20 °C for 3 hrs. The mixture was concentrated while keeping the temperature below 35°C. The mixture was filtered, and the filter cake was stirred with DCM at -5°C for 1 hr. The slurry was filtered and dried under vacuum at 40 °C to give compound **143-2** (3.27 g, yield 71.6%) as a yellow oil. LC/MS (ESI) m/z: 224/227 (M+H)⁺.

**Step 2: tert-butyl 2-(5-bromo-3-formyl-1H-indazol-1-yl)acetate (143-3):** To a solution of compound **143-3** (390 mg, 1.74 mmol) in DMF (8 mL) was added K₂CO₃ (720 mg, 5.22 mmol) followed by tert-butyl 2-bromoacetate (441 mg, 2.26 mmol), and the reaction mixture was stirred at room temperature overnight. The mixture was diluted with water and extracted with EtOAc twice. The combined organic layers were washed with brine, dried over anhydrous Na₂SO₄ and concentrated under reduced pressure. The residue was purified by chromatography on silica gel (PE: EtOAc= 100: 1 to 10: 1) to give compound **143-3** (390 mg, yield 66.3%) as a yellow solid. LC/MS (ESI) m/z: 339/341 (M+H)⁺.

**Step 3: tert-butyl 2-(5-bromo-3-(1,3-dioxolan-2-yl)-1H-indazol-1-yl)acetate (143-4):** To a mixture of compound **143-3** (130 mg, 0.38 mmol) and ethane-1,2-diol (119 mg, 1.90 mmol) in toluene (5 mL) was added pyridinium p-toluenesulfonate (5 mg, 0.019 mmol) and the mixture was stirred at 110 °C under N₂ atmosphere overnight. The mixture was concentrated, and the residue was purified by chromatography on silica gel (PE: EtOAc= 50: 1 to 15: 1) to give compound **143-4** (115 mg, yield 79.2%) as a yellow solid. LC/MS (ESI) m/z: 383/385 (M+H)⁺.

**Step 4: tert-butyl 2-(3-(1,3-dioxolan-2-yl)-5-(2-methylpyrimidin-5-yl)-1H-indazol-1-yl)acetate (143-5):** To a mixture of compound **143-4** (115 mg, 0.30 mmol) and 2-methyl-5-(4,4,5,5-tetramethyl-1,3,2-dioxaborolan-2-yl)pyrimidine (66 mg, 0.3 mmol) in 1,4-dioxane/water (4.5 mL, v/v=8:1) was added Pd(PPh₃)₄ (35 mg, 0.03 mmol) and K₂CO₃ (104 mg, 0.75 mmol), and the mixture was stirred at 95 °C under N₂ atmosphere for 3 hrs. The mixture was diluted with water and extracted with EtOAc twice. The combined organic layers were washed with brine, dried over anhydrous Na₂SO₄, and concentrated under reduced pressure. The residue was purified by chromatography on silica gel (PE: EtOAc= 10: 1 to 3: 1) to give compound **143-5** (94 mg, yield 80.0%) as a yellow oil. LC/MS (ESI) m/z: 397 (M+H)⁺.

**Step 5: 2-(3-(1,3-dioxolan-2-yl)-5-(2-methylpyrimidin-5-yl)-1H-indazol-1-yl)acetic acid (143-6):** To a solution of compound **143-5** (45 mg, 0.11 mmol) in MeOH/THF (3 mL, v/v=2/1) was added a solution of LiOH (23 mg, 0.55 mmol) in water (1 mL) at 0 °C, and the mixture was stirred at room temperature for 2 hrs. The mixture was concentrated to dryness and the residue was diluted with water and washed with EtOAc twice. The aqueous layer was acidified with 0.5 M aqueous HCl and extracted with EtOAc twice. The combined organic layers were washed with brine, dried with anhydrous Na₂SO₄, filtered, and concentrated to dryness to give compound **143-6** (35 mg, yield 93.6%) as a white solid. LC/MS (ESI) m/z: 341 (M+H)⁺.

**Step 6: (1R,3S,5R)-2-(2-(3-(1,3-dioxolan-2-yl)-5-(2-methylpyrimidin-5-yl)-1H-indazol-1-yl)acetyl)-N-(6-bromo-3-methylpyridin-2-yl)-5-methyl-2-azabicyclo[3.1.0]hexane-3-carboxamide (Compound 143):** To a mixture of compound **143-6** (35 mg, 0.10 mmol) and compound **169-1** (35 mg, 0.10 mmol) in DMF (3 mL) was added DIPEA (65 mg, 0.5 mmol) followed by HATU (57 mg, 0.15 mmol) at 0 °C, and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by prep-TLC to give Compound **143** (10.0 mg, yield 15.8%) as a white solid. ¹H-NMR (400 MHz, DMSO-*d₆*) δ 10.26 (s, 1H), 9.03 (s, 2H), 8.07 (dd, *J* = 1.6, 1.6 Hz, 1H), 7.78 (dd, *J* = 8.8, 8.8 Hz, 1H), 7.70 (dd, *J=* 8.8, 8.8 Hz, 1H), 7.64 - 7.61 (m, 1H), 7.45 (d, *J=* 7.6 Hz, 1H), 6.09 (s, 1H), 5.71 (d, *J =* 17.2 Hz, 1H), 5.41 (d, *J =* 17.2 Hz, 1H), 4.38 (dd, *J =* 9.2, 9.2 Hz, 1H), 4.29 - 4.25 (m, 2H), 4.05 - 4.01 (m, 2H), 3.54 (dd, *J =* 4.8, 4.8 Hz, 1H), 2.68 (s, 3H), 2.56 - 2.51 (m, 1H), 2.04 (s, 3H), 2.01 (d, *J* = 5.2 Hz, 1H), 1.31 (s, 3H), 0.99 - 0.94 (m, 2H). LC/MS (ESI) m/z: 632/634 (M+H)⁺.

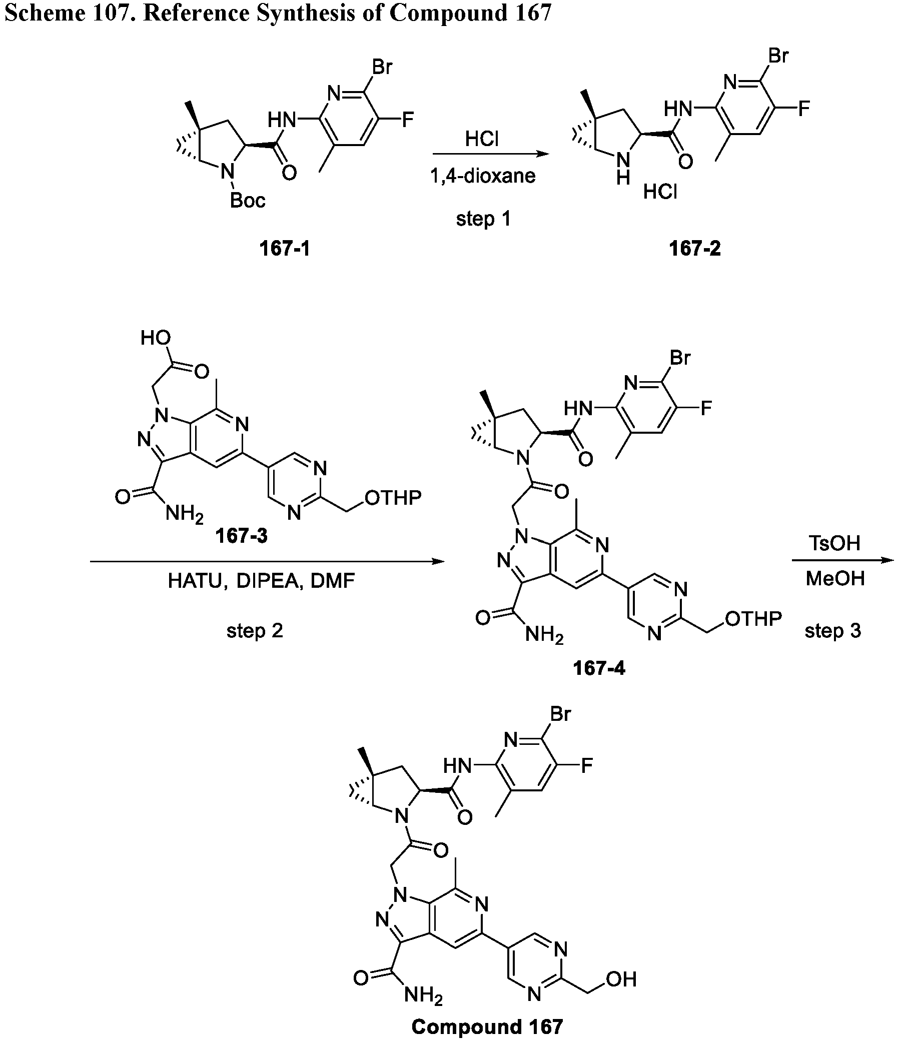

**Step 1: (1R,3S,5R)-N-(6-bromo-5-fluoro-3-methylpyridin-2-yl)-5-methyl 2-azabicyclo[3.1.0]hexane-3-carboxamide hydrochloride (167-2):** A solution of 4 M hydrogen chloride in 1,4-dioxane (6 mL) containing compound **167-1** (446 mg, 1.04 mmol) was stirred at 0 °C and warmed to room temperature over a 1 hour period. The reaction mixture was concentrated to dryness, washed with ether and dried under vacuum to give compound **167-2** (340 mg, yield 100%) as a yellow solid, which was used directly in the next step. LC/MS (ESI) m/z: 328/330 (M+H)⁺.

**Step 2: 1-(2-((1R,3S,5R)-3-((6-bromo-5-fluoro-3-methylpyridin-2-yl)carbamoyl)-5-methyl-2-azabicyclo[3.1.0]hexan-2-yl)-2-oxoethyl)-7-methyl-5-(2-(((tetrahydro-2H-pyran-2-yl)oxy)methyl)pyrimidin-5-yl)-1H-pyrazolo[3,4-c]pyridine-3-carboxamide (167-4):** To a mixture of compound **167-2** (34 mg, 0.094 mmol) and compound **167-3** (40 mg, 0.094 mmol) in DMF (5 mL) was added DIPEA (61 mg, 0.47 mmol) followed by HATU (53 mg, 0.14 mmol) at 0 °C, and the mixture was stirred at 30 °C for 2 hours. The mixture was diluted with EtOAc and washed with saturated aqueous NH₄Cl solution and brine, dried over anhydrous Na₂SO₄, filtered and concentrated to dryness. The residue was purified by chromatography on silica gel (DCM: MeOH = 100:1 to 50:1) to give compound **167-4** (43 mg, yield 62.2%) as a yellow solid. LC/MS (ESI) m/z: 736/738 (M+H)⁺.

**Step 3: (1R,3S,5R)-N-(6-bromo-5-fluoro-3-methylpyridin-2-yl)-2-(2-{3-carbamoyl-5-[2-(hydroxymethyl)pyrimidin-5-yl]-7-methylpyrazolo[3,4-c]pyridin-1-yl}acetyl)-5-methyl-2-azabicyclo[3.1.0]hexane-3-carboxamide (Compound 167)** To a solution of compound **167-4** (25 mg, 0.034 mmol) in MeOH (3 mL) was added *p*-toluenesulfonic acid (1.8 mg, 0.01 mmol) at 0 °C, and the mixture was stirred at 60 °C for 1 hour. The mixture was concentrated to dryness under reduced pressure and the residue was purified by prep-HPLC to give **Compound 167** (2.7 mg, yield 12.2%) as a white solid. ¹H-NMR (400 MHz, DMSO-*d*₆) δ 10.36 (s, 1H), 9.40 (s, 2H), 8.52 (s, 1H), 7.90 (s, 1H), 7.83 (d, *J =* 8.4 Hz, 1H), 7.63 (s, 1H), 6.05 (d, *J =* 18.0 Hz, 1H), 5.69 (d, *J* = 18.0 Hz, 1H), 5.39 (t, *J =* 6.4 Hz, 1H), 4.69 (d, *J =* 5.6 Hz, 2H), 4.39 (dd, *J=* 9.2, 9.2 Hz, 1H), 3.63 (dd, *J =* 5.6, 5.6 Hz, 1H), 2.90 (s, 3H), 2.57 (dd, *J =* 13.6, 13.6 Hz, 1H), 2.07 (s, 3H), 2.03 (dd, *J =* 13.6, 13.6 Hz, 1H), 1.33 (s, 3H), 1.03 (t, *J* = 5.4 Hz, 1H), 0.93 (dd, *J* = 5.2*,* 5.2 Hz, 1H); LC/MS (ESI) m/z: 652/654 (M+H)⁺.

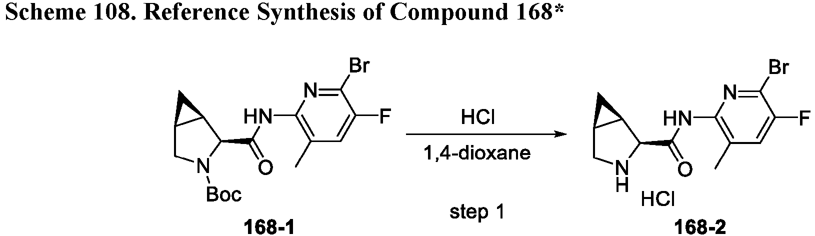

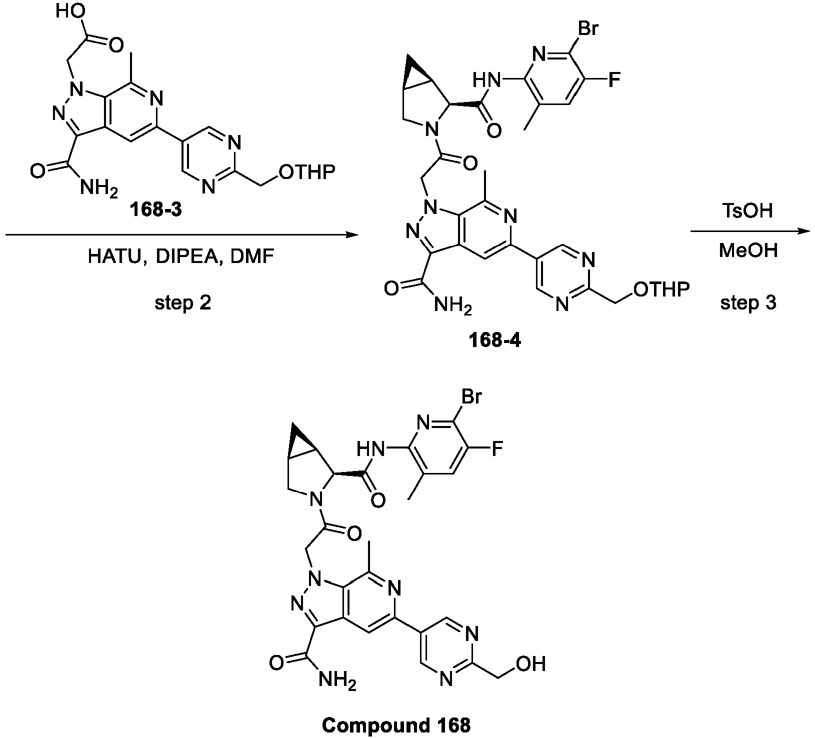

**Step 1: (1R,2S,5S)-N-(6-bromo-5-fluoro-3-methylpyridin-2-yl)-3-azabicyclo[3.1.0]hexane-2-carboxamide hydrochloride (168-2):** A 4 M solution of hydrogen chloride in 1,4-dioxane (3 mL) containing compound **168-1** (100 mg, 0.24 mmol) was stirred at 0 °C and warmed to room temperature over a 1 hour period. After completion of the reaction, the mixture was concentrated to dryness, washed with ether, and dried under vacuum to give compound **168-2** (80 mg, yield 94.5%) as a white solid, which was used directly in the next step. LC/MS (ESI) m/z: 314/316 (M+H)⁺.

**Step 2: 1-(2-((1R,2S,5S)-2-((6-bromo-5-fluoro-3-methylpyridin-2-yl)carbamoyl)-3-azabicyclo[3.1.0]hexan-3-yl)-2-oxoethyl)-7-methyl-5-(2-(((tetrahydro-2H-pyran-2-yl)oxy)methyl)pyrimidin-5-yl)-1H-pyrazolo[3,4-c]pyridine-3-carboxamide (168-4):** To a mixture of compound **168-2** (33 mg, 0.094 mmol) and compound **168-3** (40 mg, 0.094 mmol) in DMF (5 mL) was added DIPEA (61 mg, 0.47 mmol) followed by HATU (53 mg, 0.14 mmol) at 0 °C, and the mixture was stirred at 30 °C for 2 hours. The mixture was diluted with EtOAc and washed with saturated aqueous NH₄Cl solution and brine, dried over anhydrous Na₂SO₄, filtered, and concentrated to dryness under reduced pressure. The residue was purified by chromatography on silica gel (DCM: MeOH = 100:1 to 20:1) to give compound **168-4** (35 mg, yield 51.6%) as white solid. LC/MS (ESI) m/z: 722/724 (M+H)⁺.

**Step 3: 1-(2-((1R,2S,5S)-2-((6-bromo-5-fluoro-3-methylpyridin-2-yl)carbamoyl)-3-azabicyclo[3.1.0]hexan-3-yl)-2-oxoethyl)-5-(2-(hydroxymethyl)pyrimidin-5-yl)-7-methyl-1H-pyrazolo[3,4-c]pyridine-3-carboxamide (Compound 168):** To a solution of compound **168-4** (35 mg, 0.048mmol) in MeOH (3 mL) was added *p*-toluenesulfonic acid (2.5 mg, 0.015 mmol) at 0 °C, and the mixture was stirred at 60 °C for 1 hour. The mixture was concentrated to dryness and the residue was purified by prep-HPLC to give **Compound 168** (0.9 mg, yield 2.9%) as a white solid. ¹H-NMR (400 MHz, CD₃OD) δ 9.43 (s, 2H), 8.56 (s, 1H), 7.53 (d, *J =* 8.4 Hz, 1H), 5.72 (d, *J =* 4.8 Hz, 2H), 4.73 (d*, J =* 5.2 Hz, 1H), 4.07 (dd, *J =* 10.0, 10.0 Hz, 1H), 3.95 (d, *J* =10.0 Hz, 1H), 2.95 (s, 3H), 2.20 - 2.15 (m, 1H), 2.13 (s, 3H), 2.01 - 1.95 (m, 1H), 0.96 (t, *J* = 4.4 Hz, 1H), 0.93 - 0.88 (m, 1H); LC/MS (ESI) m/z: 638/640 (M+H)⁺.

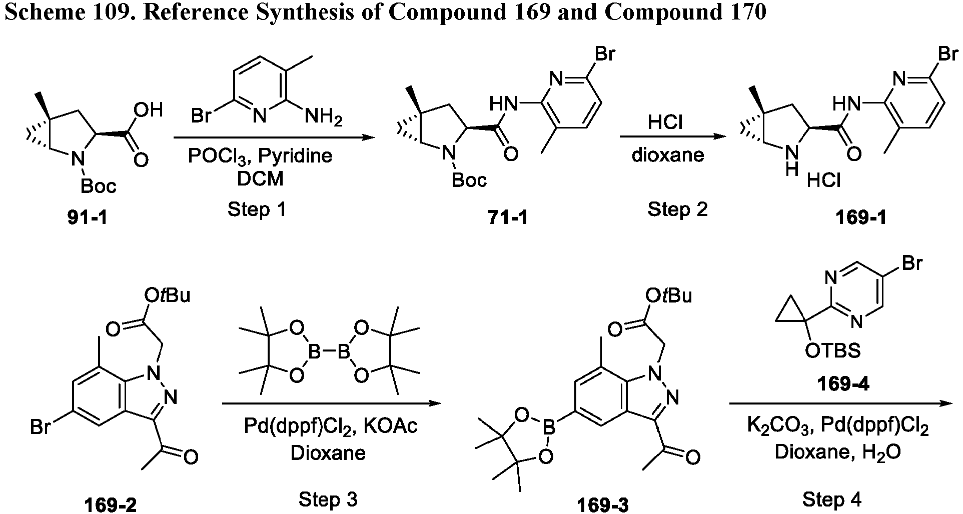

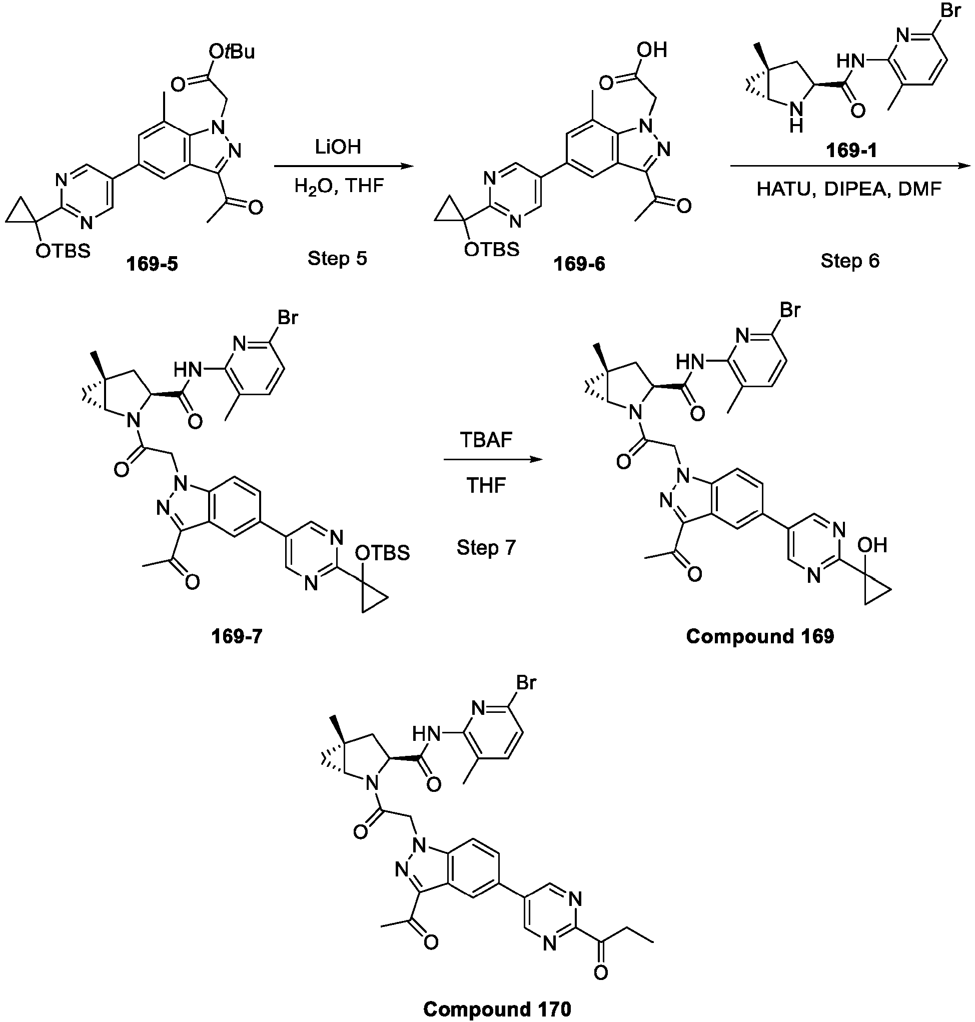

**Step 1: tert-butyl-(1R,3S,5R)-3-((6-bromo-3-methylpyridin-2-yl)carbamoyl)-5-methyl-2-azabicyclo[3.1.0]hexane-2-carboxylate (71-1):** To a mixture of compound **91-1** (50 mg, 0.12 mmol) and 6-bromo-3-methylpyridin-2-amine (231 mg, 1.24 mmol) in anhydrous DCM (6 mL) was added pyridine (41 mg, 0.3 mmol) followed by drop-wise addition of POCl₃ (285 mg, 1.86 mmol) at 0 °C. The reaction was stirred at 25 °C for 1 hour. After completion of the reaction, the reaction mixture was poured into ice water and extracted with DCM twice. The organic layers were washed with brine, dried over Na₂SO₄, concentrated to dryness, and purified by column chromatography on silica gel (PE: EtOAc = 5:1) to give compound **71-1** (370 mg, yield 72.7%) as a yellow solid. LC/MS (ESI) m/z: 410/412 (M+H)⁺.

**Step 2: (1R,3S,5R)-N-(6-bromo-3-methylpyridin-2-yl)-5-methyl-2-azabicyclo[3.1.0]hexane-3-carboxamide hydrochloride (169-1):** A solution of 4 M hydrogen chloride in 1,4-dioxane (5 mL) containing compound **71-1** (370 mg, 0.90 mmol) was stirred at room temperature for 1 hour. The mixture was concentrated to dryness to give compound **169-1** (310 mg, yield 99.3%) as a light yellow solid, which was used directly used in the next step without purification. LC/MS (ESI) m/z: 310/312 (M+H)⁺.

**Step 3: tert-butyl-2-(3-acetyl-7-methyl-5-(4,4,5,5-tetramethyl-1,3,2-dioxaborolan-2-yl)-1H-indazol-1-yl)acetate (169-3):** To a mixture of compound **169-2** (390 mg, 0.94 mmol) and 4,4,4',4',5,5,5',5'-octamethyl-2,2'-bi(1,3,2-dioxaborolane) (358 mg, 1.41 mmol) in 1,4-dioxane (8 mL) was added KOAc (276 mg, 2.82 mmol) and Pd(dppf)Cl₂ (66 mg, 0.09 mmol) at 0 °C. The reaction mixture was degassed three times and stirred at 100 °C under N₂ atmosphere overnight. After completion of the reaction, the reaction mixture was concentrated to dryness and diluted with EtOAc, washed with water and brine, dried over Na₂SO₄, and concentrated to dryness. The residue was purified by column chromatography on silica gel (PE: EtOAc = 6: 1) to give compound **169-3** (390 mg, yield 88.2%) as a white solid. LC/MS (ESI) m/z: 415 (M+H)⁺.

**Step 4: tert-butyl-2-(3-acetyl-5-(2-(1-((tert-butyldimethylsilyl)oxy)cyclopropyl)pyrimidin-5-yl)-7-methyl-1H-indazol-1-yl)acetate (169-5):** To a mixture of compound **169-3** (50 mg, 0.12 mmol) and 5-bromo-2-(1-((tert-butyldimethylsilyl)oxy)cyclopropyl)pyrimidine (compound **169-4,** 59 mg, 0.18 mmol) in 1,4-dioxane (5 mL) and water (0.5 mL) was added K₂CO₃ (41 mg, 0.3 mmol) and Pd(PPh₃)₄ (14 mg, 0.01 mmol) at 0 °C. The mixture was degassed under N₂ atmosphere three times and stirred at 100 °C under N₂ atmosphere overnight. The reaction mixture was diluted with EtOAc and washed with water and brine, dried over Na₂SO₄, filtered, and concentrated to dryness. The residue was purified by column chromatography on silica gel (PE: EtOAc =2: 1) to give compound **169-5** (35 mg, yield 55.6%) as a yellow solid. LC/MS (ESI) m/z: 537 (M+H)⁺.

**Step 5: tert-butyl-2-(3-acetyl-5-(2-(1-((tert-butyldimethylsilyl)oxy)cyclopropyl)pyrimidin-5-yl)-7-methyl-1H-indazol-1-yl)acetate (169-6):** To a solution of compound **169-5** (35 mg, 0.065 mmol) in THF (1 mL), MeOH (1 mL) and water (1 mL) was added LiOH (8.2 mg, 0.20 mmol). The mixture was stirred at room temperature for 2 hours. The mixture was concentrated to dryness, diluted with water and washed with EtOAc twice. The aqueous layer was acidified with 1N aqueous HCl to pH~3 at 0 °C and extracted with DCM twice. The combined organic layers were washed with brine, dried over anhydrous Na₂SO₄, filtered, and concentrated to dryness to give compound **169-6** (30 mg, yield 96.2%) as a yellow solid. LCMS: LC/MS (ESI) m/z: 481 (M+H)⁺.

**Step 6: (1R,3S,5R)-2-(2-(3-acetyl-5-(2-(1-((tert-butyldimethylsilyl)oxy)cyclopropyl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (169-7):** To a mixture of compound **169-6** (30 mg, 0.063 mmol) and compound **169-1** (22 mg, 0.063 mmol) in DMF (2mL) was added DIPEA (32 mg, 0.25 mmol) followed by HATU (43 mg, 0.11 mmol) at 0 °C and the mixture was stirred at room temperature for 1 hr. The mixture was diluted with EtOAc and washed with saturated aqueous NH₄Cl solution and brine, dried over anhydrous Na₂SO₄, filtered, and concentrated to dryness. The residue was purified by column chromatography on silica gel (eluted with DCM: MeOH = 80: 1) to give compound **169-7** (30 mg, yield 61.8%) as yellow solid. LC/MS (ESI) m/z: 772/774 (M+H)⁺.

**Step 7: (1R,3S,5R)-2-(2-(3-acetyl-5-(2-(1-hydroxycyclopropyl)pyrimidin-5-yl)-1H-indazol-1-yl)acetyl)-N-(6-bromo-3-methylpyridin-2-yl)-5-methyl-2-azabicyclo[3.1.0]hexane-3-carboxamide (Compound 169) and (1R,3S,5R)-2-(2-(3-acetyl-5-(2-propionylpyrimidin-5-yl)-1H-indazol-1-yl)acetyl)-N-(6-bromo-3-methylpyridin-2-yl)-5-methyl-2-azabicyclo[3.1.0]hexane-3-carboxamide (Compound 170):** To a mixture of compound **169-7** (30 mg, 0.04 mmol) in THF (2mL) was added TBAF/THF (1 mL, 1 M) at 0 °C and the mixture was stirred at room temperature for 2 hours. The mixture was diluted EtOAc and washed with saturated aqueous NH₄Cl solution and brine, dried over anhydrous Na₂SO₄, filtered, and concentrated to dryness. The residue was purified by prep. HPLC to give **Compound 169** (3 mg, yield 11.6%) as a white solid. ¹H-NMR (400 MHz, CD₃OD) δ 8.97 (s, 2H), 8.41 (s, 1H), 7.62 - 7.48 (m, 2H), 7.38 (d, J = 7.9 Hz, 1H), 5.96 (d, J = 17.8 Hz, 1H), 5.80 (d, J = 17.7 Hz, 1H), 4.53 (dd, J = 9.2, 5.7 Hz, 1H), 3.53 (dd, J = 5.5, 2.3 Hz, 1H), 2.75 (s, 3H), 2.68 (s, 3H), 2.63 (dd, J = 13.5, 9.4 Hz, 1H), 2.30 (dd, J = 13.1, 5.7 Hz, 1H), 2.12 (s, 3H), 1.48 (dd, J = 7.5, 4.4 Hz, 2H), 1.41 (s, 3H), 1.31 (dd, J = 7.6, 4.4 Hz, 2H), 1.12 (t, J = 5.3 Hz, 1H), 0.97 (dd, J = 5.5, 2.3 Hz, 1H); LC/MS (ESI)m/z: 644/646 (M+H)⁺.

**Compound 170** (1.9 mg, yield 7.4%) as a white solid. ¹H-NMR (400 MHz, CD₃OD) δ 9.25 (s, 2H), 8.52 (s, 1H), 7.61 (s, 1H), 7.54 (d, J = 8.0 Hz, 1H), 7.37 (d, J = 7.9 Hz, 1H), 5.97 (d, J = 17.8 Hz, 1H), 5.80 (d, J = 17.8 Hz, 1H), 4.53 (dd, J = 9.1, 5.5 Hz, 1H), 3.54 (dd, J = 5.5, 2.2 Hz, 1H), 3.34 (d, J = 7.2 Hz, 2H), 2.76 (s, 3H), 2.68 (s, 3H), 2.67 - 2.61 (m, 1H), 2.30 (dd, J = 12.8, 5.8 Hz, 1H), 2.12 (s, 3H), 1.41 (s, 3H), 1.24 (t, J = 7.2 Hz, 3H), 1.12 (t, J = 5.2 Hz, 1H), 0.97 (dd, J = 5.4, 2.3 Hz, 1H); LC/MS (ESI) m/z: 644/646 (M+H)⁺.

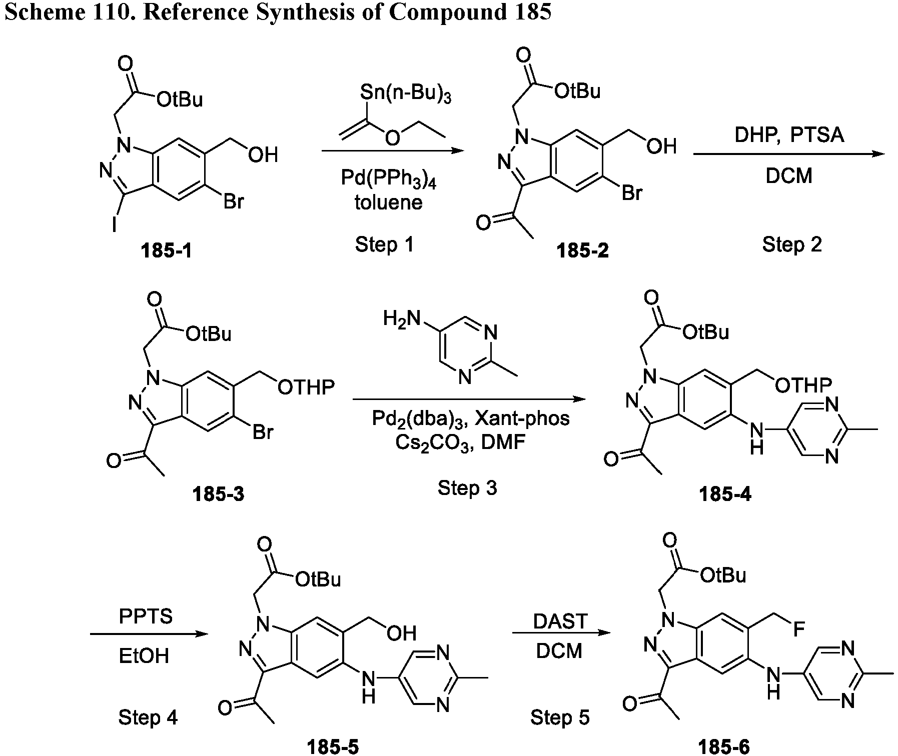

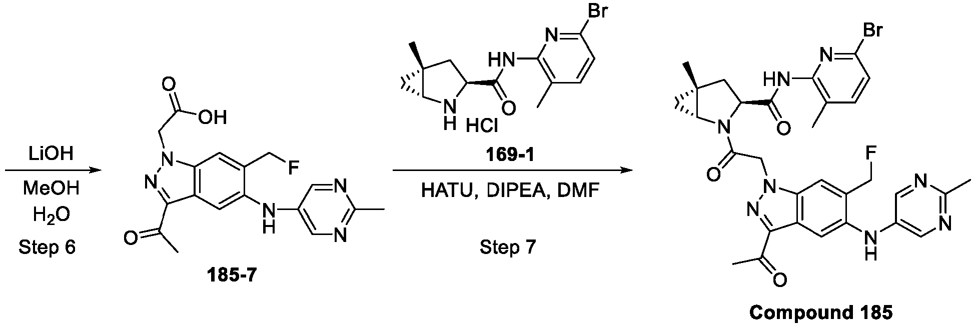

**Step 1: tert-butyl 2-(3-acetyl-5-bromo-6-(hydroxymethyl)-1H-indazol-1-yl)acetate (185-2):** To a solution of compound **185-1** (860 mg, 1.85 mmol) in toluene (15 mL) under N₂ atmosphere was added tributyl(1-ethoxyvinyl)stannane (866 mg, 2.40 mmol) and Pd(PPh₃)₄ (214 mg, 0.19 mmol) at 0 °C. The mixture was degassed under N₂ atmosphere three times and stirred at 100 °C under N₂ atmosphere overnight. The mixture was concentrated to dryness and purified by column chromatography on silica gel (eluted with PE: EtOAc = 3: 1) to give compound **185-2** (580 mg, yield 82.2%) as a brown solid. LC/MS (ESI) m/z: 383/385 (M+H)⁺.

**Step 2: tert-butyl 2-(3-acetyl-5-bromo-6-(((tetrahydro-2H-pyran-2-yl)oxy)methyl)-1H-indazol-1-yl)acetate (185-3):** To a solution of compound **185-2** (200 mg, 0.52 mmol) in DCM (5 mL) was added 3,4-dihydro-2H-pyran (66 mg, 0.78 mmol) and *p*-toluenesulfonic acid (19.8 mg, 0.11 mmol) at 0 °C. The mixture was stirred at 25 °C overnight. The mixture was concentrated to dryness, and the residue was purified by column chromatography on silica gel (eluted with PE: EtOAc= 6: 1) to give compound **185-3** (226 mg, yield 93.4%) as a brown solid. LC/MS (ESI) m/z: 467/469 (M+H)⁺.

**Step 3: tert-butyl 2-(3-acetyl-5-((2-methylpyrimidin-5-yl)amino)-6-(((tetrahydro-2H-pyran-2-yl)oxy)methyl)-1H-indazol-1-yl)acetate (185-4):** To a mixture of compound **185-3** (200 mg, 0.43 mmol) and 2-methylpyrimidin-5-amine (70 mg, 0.65 mmol) in DMF (5 mL) and water (0.5 mL) was added Cs₂CO₃ (420 mg, 1.29 mmol), Pd₂(dba)₃ (36.6 mg, 0.04 mmol) and Xant-phos (52 mg, 0.09 mmol) at 0 °C. The mixture was degassed under N₂ three times and stirred at 100°C in a microwave reactor for 1 hour. The reaction mixture was diluted with EtOAc, washed with saturated aqueous NH₄Cl solution and brine, dried over Na₂SO₄, concentrated to dryness, and purified by column chromatography on silica gel (PE: EtOAc = 1: 1) to give compound **185-4** (130 mg, yield 61.0%) as a yellow solid. LC/MS (ESI) m/z: 496 (M+H)⁺.

**Step** 4: **tert-butyl 2-(3-acetyl-6-(hydroxymethyl)-5-((2-methylpyrimidin-5-yl)amino)-1H-indazol-1-yl)acetate (185-5):** To a solution of the compound **185-4** (130 mg, 0.26 mmol) in EtOH (5 mL) was added pyridinium *p*-toluenesulfonate (19.6 mg, 0.078 mmol) at 0 °C. The mixture was stirred at 60 °C for 5 hours. The mixture was concentrated to dryness and the residue was purified by column chromatography on silica gel (eluted with DCM: MeOH = 80: 1) to give compound **185-5** (80 mg, 74.8 % yield) as a brown solid. LC/MS (ESI) m/z: 412 (M+H)⁺.

### Step 5: tert-butyl 2-(3-acetyl-6-(fluoromethyl)-5-((2-methylpyrimidin-5-yl)amino)-1H-indazol-1-yl)acetate (185-6)

To a solution of compound **185-5** (80 mg, 0.19 mmol) in DCM (5 mL) was added DAST (46 mg, 0.29 mmol) at -60 °C and the mixture was stirred at -60 °C for 1 hour. The mixture was concentrated to dryness and purified by column chromatography on silica gel (eluted with DCM: MeOH = 80: 1) to give compound **185-6** (45 mg, yield 57.3%) as a yellow oil. LC/MS (ESI) m/z: 414 (M+H)⁺.

**Step 6: 2-(3-acetyl-6-(fluoromethyl)-5-((2-methylpyrimidin-5-yl)amino)-1H-indazol-1-yl)acetic** acid **(185-7):** To a solution of compound **185-6** (35 mg, 0.085 mmol) in THF (1 mL), MeOH (1 mL) and water (1 mL) was added LiOH (10.7 mg, 0.25 mmol). The mixture was stirred at room temperature for 2 hours. The mixture was concentrated to dryness, diluted with water and washed with EtOAc twice. The aqueous layer was acidified by adding 1N aqueous HCl to pH~3 at 0 °C and extracted with DCM twice. The combined organic layers were washed with brine, dried over anhydrous Na₂SO₄, filtered, and concentrated to dryness to give compound **185-7** (30 mg, yield 98.6%) as a yellow solid. LCMS: LC/MS (ESI) m/z: 358 (M+H)⁺.

**Step 7: (1R,3S,5R)-2-(2-(3-acetyl-6-(fluoromethyl)-5-((2-methylpyrimidin-5-yl)amino)-1H-indazol-1-yl)acetyl)-N-(6-bromo-3-methylpyridin-2-yl)-5-methyl-2-azabicyclo[3.1.0]hexane-3-carboxamide (Compound 185):** To a mixture of compound **185-7** (30 mg, 0.084 mmol) and compound **169-1** ((1R,3S,5R)-N-(6-bromo-3-methylpyridin-2-yl)-5-methyl-2-azabicyclo[3.1.0]hexane-3-carboxamide hydrochloride, 29 mg, 0.084 mmol) in DMF (2mL) was added DIPEA (43 mg, 0.34 mmol) followed by HATU (57 mg, 0.15 mmol) at 0 °C and the mixture was stirred at room temperature for 1 hour. The mixture was diluted with EtOAc and washed with saturated aqueous NH₄Cl solution and brine, dried with anhydrous Na₂SO₄, filtered, and concentrated to dryness. The residue was purified by prep-HPLC to give **Compound 185** (3 mg, yield 5.5%) as a white solid. ¹H-NMR (400 MHz, CD₃OD) δ 8.29 (s, 2H), 8.12 (s, 1H), 7.79 (s, 1H), 7.55 (d, J = 8.0 Hz, 1H), 7.39 (d, J = 8.0 Hz, 1H), 5.78 (d, J = 17.2 Hz, 1H), 5.63 (d, J = 17.1 Hz, 1H), 5.60 (s, 1H), 5.48 (s, 1H), 4.53 (dd, J = 9.4, 5.3 Hz, 1H), 3.53 (dd, J = 5.6, 2.4 Hz, 1H), 2.64 (s, 3H), 2.63 - 2.59 (m, 1H), 2.58 (s, 3H), 2.31 (dd, J = 13.8, 5.6 Hz, 1H), 2.12 (s, 3H), 1.40 (s, 3H), 1.09 (t, J = 5.4 Hz, 1H), 1.00 (dd, J = 5.5, 2.4 Hz, 1H). LC/MS (ESI) m/z: 649/651 (M+H)⁺.

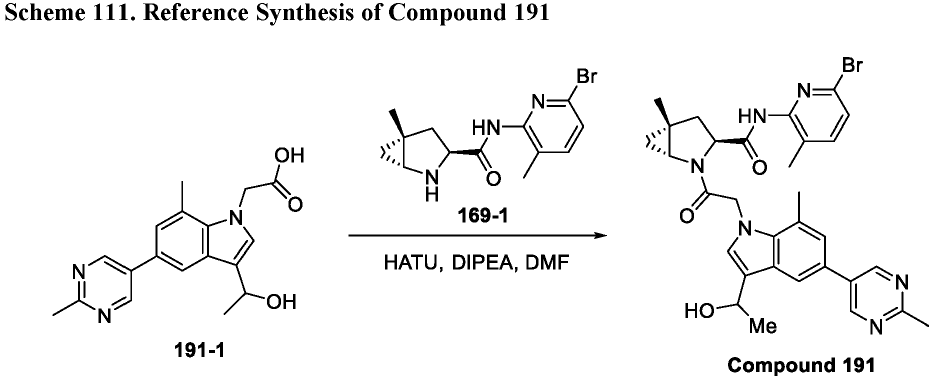

**(1R,3S,5R)-N-(6-bromo-3-methylpyridin-2-yl)-2-(2-(3-(1-hydroxyethyl)-7-methyl-5-(2-methylpyrimidin-5-yl)-1H-indol-1-yl)acetyl)-5-methyl-2-azabicyclo[3.1.0]hexane-3-carboxamide (Compound 191):** To a mixture of compound **191-1** (40 mg, 0.124 mmol) and compound **169-1** (38 mg, 0.12 mmol) in DMF (3 mL) was added DIPEA (79 mg, 0.62 mmol) followed by HATU (70 mg, 0.18 mmol) at 0 °C, and the mixture was stirred at room temperature for 1 hour. The mixture was diluted with EtOAc and washed with saturated aqueous NH₄Cl solution and brine, dried over anhydrous Na₂SO₄, filtered, and concentrated to dryness. The residue was purified by prep-TLC (DCM: MeOH = 20: 1) to give **Compound 191** (2.5 mg, yield 3.3%) as a white solid. ¹H-NMR (400 MHz, CD₃OD) δ 8.96 (s, 2H), 7.86 (t, (*t, J =* 2.0 Hz, 1H), 7.55 (d, *J* = 8.0 Hz, 1H), 7.38 (d, *J* = 8.0 Hz, 1H), 7.19 (d, *J* = 1.6 Hz, 1H), 7.13 (s, 1H), 5.47 (d, *J* = 4.4 Hz, 2H), 5.21 - 5.15 (m, 1H), 4.51 (dd, *J =* 9.2, 9.2 Hz, 1H), 3.45 - 3.42 (m, 1H), 2.72 (s, 3H), 2.68 (s, 3H), 2.63 - 2.58 (m, 1H), 2.28 (dd, *J* = 13.6, 13.6 Hz, 1H), 2.12 (s, 3H), 1.63 (d*, J =* 6.4 Hz, 3H), 1.38 (s, 3H), 1.07 (t, *J =* 5.6 Hz, 1H), 0.91 (dd, *J* = 5.6, 5.6 Hz, 1H); LC/MS (ESI) m/z: 617/619 (M+H)⁺.

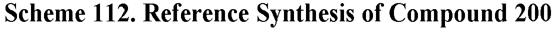

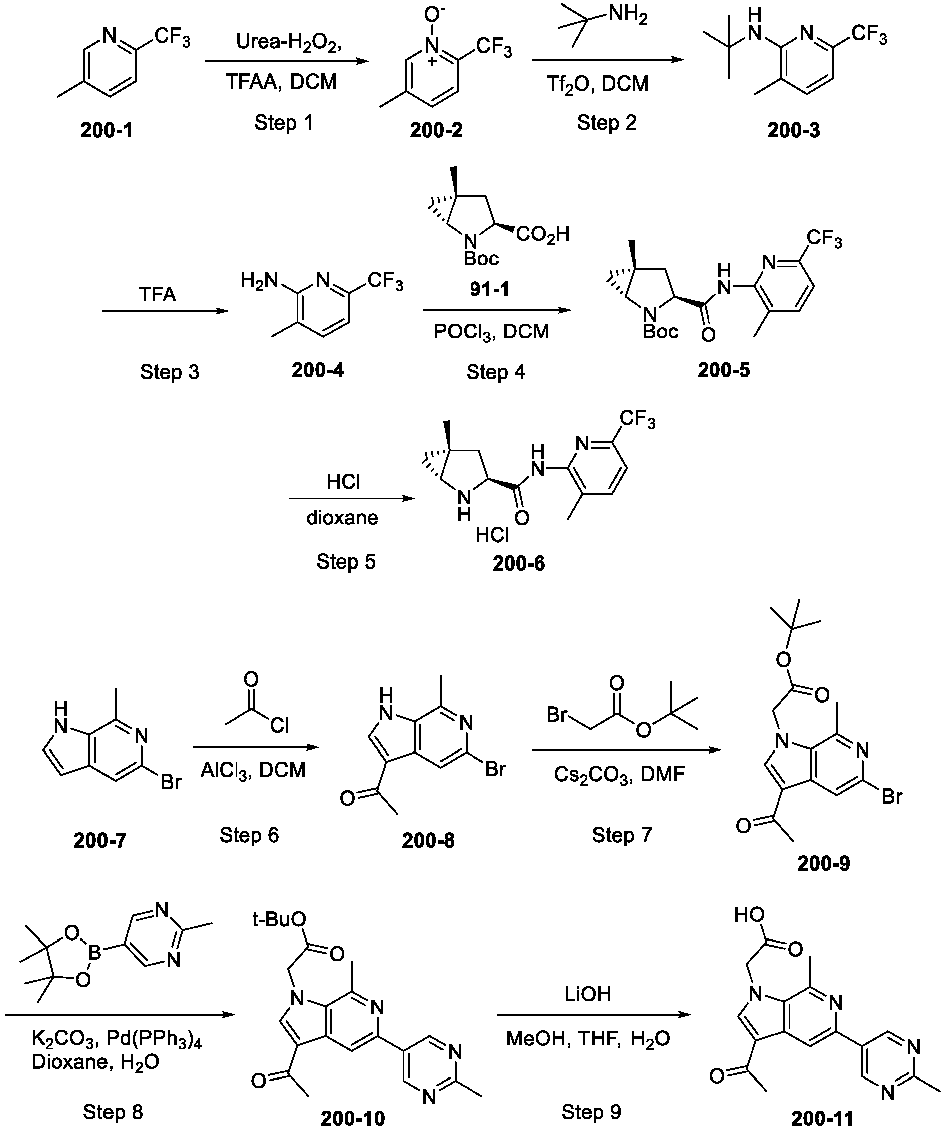

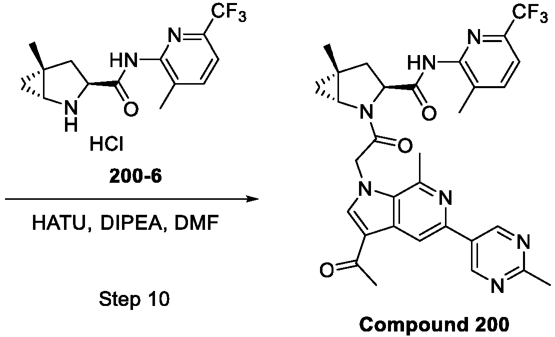

**Step 1: 5-Methyl-2-(trifluoromethyl)pyridine-N-oxide (200-2):** Compound **200-1** (1 g, 6.21 mmol, 1 equiv.) was dissolved in dichloromethane and the solution was cooled in an ice bath. Powdered urea -hydrogen peroxide complex (0.899 g, 9.56 mmol, 1.5 equiv.) was added followed by the dropwise addition of trifluoroacetic anhydride (3.26 g, 2.16 mL, 15.5 mmol, 2.5 equiv.). The cooling bath was removed, and the reaction mixture was stirred overnight at rt. The reaction mixture was quenched by the careful addition of saturated sodium metabisulfite solution and the reaction mixture was stirred at rt for 15 minutes. The solution was diluted with dichloromethane and made basic by the careful addition of saturated aqueous sodium bicarbonate. The organic layer was separated, and the aqueous layer was extracted with dichloromethane. The combined organic layer was dried and concentrated to give an orange red oil which was used in the next step without purification.

**Step 2: *N*-(tert-Butyl)-3-methyl-6-(trifluoromethyl)pyridin-2-amine (200-3):** To a stirred solution of compound **200-2** (1.05 g, 5.928 mmol, 1 equiv.) in dichloromethane (25 mL) at -20 ° C was added **tert**-butylamine (3.21 mL) followed by the dropwise addition of triflic anhydride (3 mL), and the reaction mixture was stirred at -20 ° C for 1h then quenched by the addition of water. After stirring for 15 minutes, the layers were separated, the aqueous layer was extracted once with dichloromethane, and the combined organic layers was dried and concentrated. The residue was purified by silica gel column chromatography (eluent: hexane) to give a colorless liquid.

**Step 3: 3-Methyl-6-(trifluoromethyl)pyridin-2-amine (200-4):** A solution of compound **200-3** (0.72 g, 3.1 mmol, 1 equiv.) in TFA was stirred at 65 ° C for 1h. The mixture was cooled to room temperature, the volatiles were removed under reduced pressure, and the resulting residue was dissolved in dichloromethane and made basic with saturated aqueous NaHCO₃ solution. The organic layer was separated, dried, and concentrated to give 0.49 g of white solid which was used in the next step without purification.

**Step 4: *tert-*Butyl (1R,3S,5R)-5-methyl-3-((3-methyl-6-(trifluoromethyl)pyridin-2-yl)carbamoyl)-2-azabicyclo[3.1.0]hexane-2-carboxylate (200-5):** To an ice cold solution of compound **200-4** (0.39 g, 2.21 mmol, 1 equiv.) and compound **91-1** (0.588 g, 2.44 mmol, 1.1 equiv.) in dichloromethane (8 mL), pyridine (0.9 mL) was added followed by the dropwise addition of POCl₃ (0.21 mL). The reaction mixture was stirred at 0 ° C for 1h and was quenched by the addition of saturated aqueous NaHCO₃. The cooling bath was removed, and the reaction mixture was stirred at rt for 15 minutes. The layers were separated, and the organic layer was washed with cold 1N aqueous HCl and water then dried and concentrated to give 0.85 g of white solid, which was used in subsequent steps without further purification.

**Step 5: (1R,3S,5R)-5-methyl-N-(3-methyl-6-(trifluoromethyl)pyridin-2-yl)-2-azabicyclo[3.1.0]hexane-3-carboxamide (200-6):** A solution of compound **200-5** in dioxane containing 4 M hydrogen chloride is stirred at room temperature. The volatiles are removed and the resultant residue is used without further purification.

**Step 6: 1-(5-bromo-7-methyl-1H-pyrrolo[2,3-c]pyridin-3-yl)ethenone (200-8):** To a solution of aluminum chloride (758 mg, 5.69 mmol) in DCM (4 mL) was added compound **200-7** (300 mg, 1.42 mmol) at 0 °C under N₂ atmosphere, and the mixture was stirred at 0 °C for 30 minutes. Acetyl chloride (446 mg, 5.69 mmol) was added to the stirred reaction mixture in a drop-wise manner, and the reaction was stirred at room temperature overnight under N₂ atmosphere. The mixture was quenched with MeOH at 0 °C and concentrated to dryness under reduced pressure to give compound **200-8** (360 mg, yield 99.9%) as a yellow solid. LC/MS (ESI) m/z: 253/255 (M+H)⁺.

**Step 7: tert-butyl 2-{3-acetyl-5-bromo-7-methylpyrrolo[2,3-c]pyridin-1-yl}acetate (200-9):** To a solution of compound **200-8** (360 mg, 1.43 mmol) in DMF (6 mL) was added cesium carbonate (700 mg, 2.15 mmol) and tert-butyl 2-bromoacetate (306 mg, 1.57 mmol) at 0 °C, and the mixture was stirred at room temperature for 2 hours. The mixture was diluted with ice-water and extracted with EtOAc twice. The combined organic layers were washed with brine, dried over anhydrous Na₂SO₄, filtered, and concentrated to dryness under reduced pressure. The residue was purified by chromatography on silica gel (PE: EtOAc = 15:1 to 2:1) to give compound **200-9** (250 mg, yield 47.8%) as a yellow solid. LC/MS (ESI) m/z: 367/369 (M+H)⁺.

**Step 8: tert-butyl 2-[3-acetyl-7-methyl-5-(2-methylpyrimidin-5-yl)pyrrolo[2,3-c]pyridin-1-yl]acetate (200-10):** To a mixture of compound **200-9** (50 mg, 0.14 mmol) and 2-methyl-5-(4,4,5,5-tetramethyl-1,3,2-dioxaborolan-2-yl)pyrimidine (33 mg, 0.15 mmol) in 1,4-dioxane (2.4 mL) and water (0.3 mL) was added potassium carbonate (47 mg, 0.34 mmol). The mixture was degassed under N₂ three times and Pd(PPh₃)₄ (16 mg, 0.014 mmol) was added. The mixture was stirred at 92 °C for 2 hours under N₂ atmosphere, then poured into ice-water and extracted with EtOAc twice. The combined organic layers were washed with brine, dried over anhydrous Na₂SO₄, filtered and concentrated to dryness under reduced pressure. The residue was purified by chromatography on silica gel (DCM: MeOH = 100:1 to 10:1) to give compound **200-10** (52 mg, yield 98.1%) as a white solid. LC/MS (ESI) m/z: 381 (M+H)⁺.

**Step 9: [3-acetyl-7-methyl-5-(2-methylpyrimidin-5-yl)pyrrolo[2,3-c]pyridin-1-yl]acetic acid (200-11):** To a solution of compound **200-10** (52 mg, 0.14 mmol) in MeOH (2 mL) and THF (1 mL) was added a solution of lithium hydroxide hydrate (29 mg, 0.68 mmol) in water (1 mL) at 0 °C, and the mixture was stirred at room temperature for 1 hour. The mixture was washed with Et₂O and water. The aqueous layer was acidified with 0.5 M aqueous HCl and extracted with DCM twice. The combined organic layers were washed with brine, dried over anhydrous Na₂SO₄, filtered, and concentrated to dryness to give compound **200-11** (44 mg, yield 97.0%) as a white solid. LC/MS (ESI) m/z: 325 (M+H)⁺.

**Step 10: (1R,3S,5R)-2-{2-[3-acetyl-7-methyl-5-(2-methylpyrimidin-5-yl)pyrrolo[2,3-c]pyridin-1-yl]acetyl}-5-methyl-N-[3-methyl-6-(trifluoromethyl)pyridin-2-yl]-2-azabicyclo[3.1.0]hexane-3-carboxamide (Compound 200):** To a mixture of compound **200-11** (44 mg, 0.14 mmol) and compound **200-6** (46 mg, 0.14 mmol) in DMF (3mL) was added DIPEA (88 mg, 0.68 mmol) followed by HATU (77 mg, 0.20 mmol) at 0 °C, and the mixture was stirred at room temperature for 1 hour. The mixture was diluted with EtOAc and washed with aqueous NH₄Cl solution and brine, dried over anhydrous Na₂SO₄, filtered, and concentrated to dryness under reduced pressure. The residue was purified by prep-HPLC to give **Compound 200** (13 mg, yield 15.8%) as a light yellow solid. ¹H-NMR (400 MHz, CD₃OD) δ 9.27 - 9.20 (m, 2H), 8.60 - 8.51 (m, 1H), 8.35 - 8.26 (m, 1H), 7.84 (t, *J* = 6.4 Hz, 1H), 7.61 - 7.55 (m, 1H), 5.76 - 5.54 (m, 2H), 4.64 - 4.54 (m, 2H), 3.54 - 3.48 (m, 1H), 2.91 - 2.85 (m, 3H), 2.77 - 2.72 (m, 3H), 2.71 - 2.61 (m, 1H), 2.56 - 2.48 (m, 4H), 2.29 (dd, *J=* 13.6, 13.6 Hz, 1H), 2.24 (d, *J=* 2.8 Hz, 3H), 1.41 (s, 3H), 1.12 (t, *J* = 4.4 Hz, 1H), 1.01 - 0.95 (m, 1H); LC/MS(ESI) m/z: 606 (M+H)⁺.

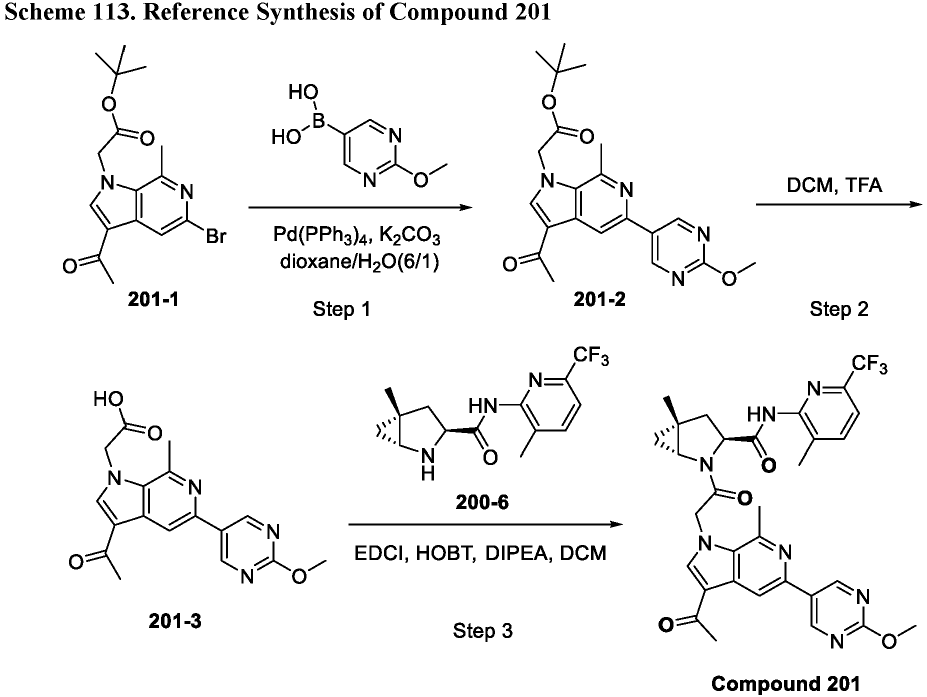

**Step 1: tert-butyl 2-[3-acetyl-5-(2-methoxypyrimidin-5-yl)-7-methylpyrrolo [2, 3-c] pyridin-1-yl] acetate (201-2):** To a mixture of compound **201-1** (0.05 g, 0.13 mmol), 2-methoxypyrimidin-5-ylboronic acid (0.025 g, 0.16 mmol) in 1,4-dioxane (2 mL) and water (0.2 mL) was added potassium carbonate (0.047 g, 0.34 mmol) and tetrakis(triphenylphosphine)palladium (0.031 g, 0.027 mmol) at room temperature under N₂ atmosphere. The mixture was stirred at 90 °C for 2 hours under N₂ atmosphere before extracted with DCM twice. The combined organic layers were washed with brine, dried over anhydrous Na₂SO₄, filtered, and concentrated. The residue was purified by chromatography on silica gel (PE: EtOAc = 7: 3) to give compound **201-2** (0.06 g, yield 55.8%) as a light yellow solid. LC/MS (ESI) (m/z): 397 (M+H)⁺.

**Step 2: [3-acetyl-5-(2-methoxypyrimidin-5-yl)-7-methylpyrrolo [2, 3-c] pyridin-1-yl] acetic acid (201-3):** To a solution of compound **201-2** (0.07 g, 0.17 mmol) in DCM (1 mL) was added TFA (1 mL) at 0 °C and the mixture was stirred at 25 °C for 2 hours before concentrated to dryness under reduced pressure to give 60 mg compound **201-3** (66 mg, yield 100%) as brown semi-solid, which was used directly in the next step. LC/MS (ESI) (m/z): 341 (M+H)⁺.

**Step 3: (1R,3S,5R)-2-(2-(3-acetyl-5-(2-methoxypyrimidin-5-yl)-7-methyl-1H-pyrrolo[2,3-c]pyridin-1-yl)acetyl)-5-methyl-N-(3-methyl-6-(trifluoromethyl)pyridin-2-yl)-2-azabicyclo[3.1.0]hexane-3-carboxamide (Compound 201):** To a mixture of compound **201-3** (60 mg, 0.17 mmol), **201-4** (1R,3S,5R)-5-methyl-N-[3-methyl-6-(trifluoromethyl)pyridin-2-yl]-2-azabicyclo[3.1.0]hexane-3-carboxamide, 79 mg, 0.26 mmol) in DMF (5 mL) was added DIPEA (91 mg, 0.12 mL, 0.70 mmol), HATU (0.12 g, 0.31 mmol) at 0 °C. The mixture was stirred at 25 °C for 1 hour then diluted with water and extracted with EtOAc twice. The combined organic layers were washed with brine, dried over anhydrous Na₂SO₄, filtered, and concentrated to dryness. The residue was purified by prep-HPLC to give **Compound 201** (11.0 mg, yield 10%) as a white solid. ¹H-NMR (400 MHz, CD₃OD) δ 9.13 (s, 2H), 8.49 (s, 1H), 8.30 (s, 1H), 7.85 (d, J = 7.8 Hz, 1H), 7.58 (d, J = 7.8 Hz, 1H), 5.70 (d, J = 18.0 Hz, 1H), 5.60 (d, J = 18.0 Hz, 1H), 4.58 (s, 2H), 4.07 (s, 3H), 3.52 (dd, J = 5.6, 2.4 Hz, 1H), 2.88 (s, 3H), 2.66 (dd, J = 13.5, 9.2 Hz, 1H), 2.53 (s, 3H), 2.30 (dd, J = 13.5, 5.6 Hz, 1H), 2.25 (s, 3H), 1.41 (s, 3H), 1.13 (t, J = 5.4 Hz, 1H), 0.98 (dd, J = 5.5, 2.4 Hz, 1H); LC/MS (ESI) (m/z): 622 (M+H)⁺.

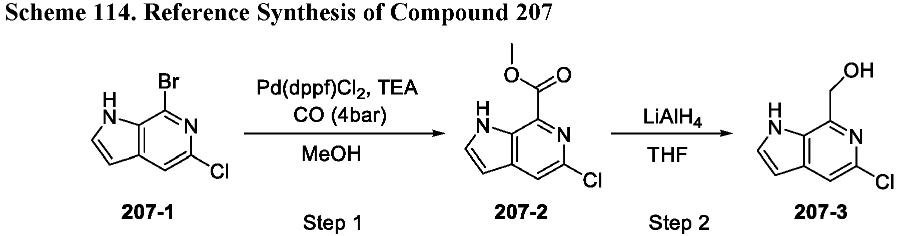

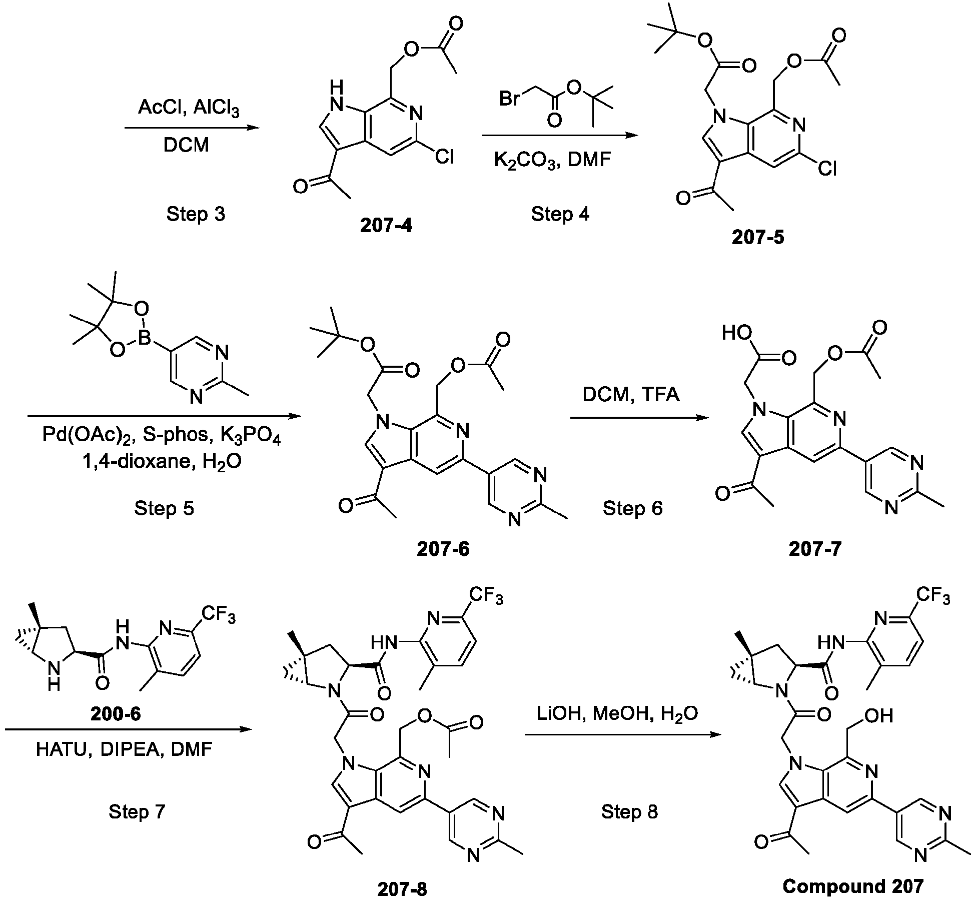

**Step 1: methyl 5-chloro-1H-pyrrolo[2,3-c]pyridine-7-carboxylate (207-2):** To a solution of compound **207-1** (0.17 g, 0.73 mmol) in methanol (10 mL) was added TEA and [1, 1'-bis(diphenylphosphino)ferrocene]dichloropalladium(II) (54 mg, 0.073 mmol). The mixture was degassed under N₂ atmosphere three times and stirred under CO (4 bar) at 70 °C for 16 hours. The reaction was extracted with DCM twice and the combined organic layers were washed with brine, dried over anhydrous Na₂SO₄, filtered, and concentrated to dryness. The residue was purified by chromatography on silica gel (PE: EtOAc = 3: 1) to give compound **207-2** (83 mg, yield 53.5%) as an off-white solid. LC/MS (ESI) (m/z): 211 (M+H)⁺.

**Step 2: {5-chloro-1H-pyrrolo[2,3-c]pyridin-7-yl}methanol (207-3):** To a solution of compound **207-2** (0.13 g, 0.61 mmol) in THF (5 mL) was added lithium aluminum hydride (70 mg, 1.85 mmol) in portions at 0 °C. The mixture was stirred at 0 °C for 1.5 hours before quenched with water (0.1 mL), 0.1 mL aq.NaOH solution (15% wt) and 0.21 mL water in sequence. The aqueous layer was extracted with DCM twice and the combined organic layers were washed with brine, dried over anhydrous Na₂SO₄, filtered and concentrated and the residue was purified by prep-TLC (PE: EtOAc = 7: 3) to give compound **207-3** (71 mg, yield 63%) as white solid. LC/MS (ESI) (m/z): 183 (M+H)⁺.

**Step 3: {3-acetyl-5-chloro-1H-pyrrolo[2,3-c]pyridin-7-yl}methyl acetate (207-4):** To a mixture of compound **207-3** (71 mg, 0.38 mmol) in DCM (2 mL) was added aluminum chloride (0.156 g, 1.16 mmol) at 0 °C. The mixture was stirred at 0 °C for half an hour before acetyl chloride (92 mg, 1.166 mmol) was added. The mixture was allowed to stir at room temperature for 12 hours and poured into ice-water. The mixture was extracted with DCM twice and the combined organic layers were washed with brine, dried over anhydrous Na₂SO₄, filtered, and concentrated to dryness. The residue was purified by chromatography on silica gel (PE: EtOAc = 1: 1) to give compound **207-4** (84 mg, yield 81.0%) as a white solid. LC/MS (ESI) (m/z): 267 (M+H)⁺

**Step 4: {3-acetyl-1-[2-(tert-butoxy)-2-oxoethyl]-5-chloropyrrolo[2,3-c]pyridin-7-yl}methyl acetate (207-5):** To a solution of compound **207-4** (84 mg, 0.31 mmol) in DMF (3 mL) was added potassium carbonate (87 mg, 0.63 mmol) and tert-butyl 2-bromoacetate (92 mg, 0.472 mmol) and the mixture was stirred at room temperature for 16 hours. The mixture was diluted with EtOAc and washed with saturated aqueous NH₄Cl solution and brine, dried over anhydrous Na₂SO₄, filtered, and concentrated to dryness. The residue was purified by chromatography on silica gel (PE: EtOAc = 7:3) to give compound **207-5** (0.108 g, yield 90.0%) as a colorless oil. LC/MS (ESI) (m/z): 381 (M+H)⁺.

**Step 5: {3-acetyl-1-[2-(tert-butoxy)-2-oxoethyl]-5-(2-methylpyrimidin-5-yl)pyrrolo[2,3-c]pyridin-7-yl}methyl acetate (207-6):** To a mixture of compound **207-5** (0.108 g, 0.284 mmol) and 2-methyl-5-(4,4,5,5-tetramethyl-1,3,2-dioxaborolan-2-yl)pyrimidine (0.1 g, 0.45 mmol) in 1,4-dioxane (2.4 mL) and water (0.3 mL) was added S-Phos (14 mg, 0.028 mmol), potassium phosphate (0.15 g, 0.70 mmol) and palladium (II) acetate (6 mg, 0.028 mmol) under N₂ atmosphere. The mixture was degassed under N₂ atmosphere three times and stirred at 95 °C under N₂ atmosphere for 12 hours. The mixture was diluted with EtOAc and washed with water and brine, dried, and concentrated to dryness to give crude product. The crude residue was purified by flash chromatography (PE: EtOAc = 50: 1 to 3: 2) to give compound **207-6** (0.1 g, yield 80.2%) as a white solid. LC/MS (ESI) (m/z): 439 (M+H)⁺.

**Step 6: {3-acetyl-7-[(acetyloxy)methyl]-5-(2-methylpyrimidin-5-yl)pyrrolo[2,3-c]pyridin-1-yl}acetic acid (207-7):** To a solution of compound **207-6** (70 mg, 0.16 mmol) in DCM (2 mL) was added TFA (2 mL, 26.13 mmol) at 0 °C. The mixture was stirred at 25 °C for 2 hours and concentrated to dryness under reduced pressure to give compound **207-7** (0.060 g, yield 99%) as a brown solid, which was used directly in the next step. LC/MS (ESI) (m/z): 383 (M+H)⁺.

**Step 7: (3-acetyl-1-{2-[(1R,5R)-5-methyl-3-{[3-methyl-6-(trifluoromethyl)pyridin-2-yl]carbamoyl}-2-azabicyclo[3.1.0]hexan-2-yl]-2-oxoethyl}-5-(2-methylpyrimidin-5-yl)pyrrolo[2,3-c]pyridin-7-yl)methyl acetate (207-8):** To a mixture of compound **207-7** (61 mg, 0.16 mmol) and compound **200-6** (72 mg, 0.23 mmol) in DMF (3 mL) was added DIPEA (82 mg, 0.63 mmol) and HATU (0.109 g, 0.28 mmol) at 0 °C, and the mixture was stirred at room temperature for 12 hours under N₂ atmosphere. The mixture was diluted with EtOAc and washed with saturated aqueous NH4Cl solution and brine, dried over anhydrous Na₂SO₄, filtered and concentrated to dryness. The residue was purified by chromatography on silica gel (DCM: MeOH = 97: 3) to give compound **207-8** (0.1 g, yield 94.5%) as a brown solid. LC/MS (ESI) (m/z): 664 (M+H)⁺.

**Step 8: (1R,3S,5R)-2-(2-(3-acetyl-7-(hydroxymethyl)-5-(2-methylpyrimidin-5-yl)-1H-pyrrolo[2,3-c]pyridin-1-yl)acetyl)-5-methyl-N-(3-methyl-6-(trifluoromethyl)pyridin-2-yl)-2-azabicyclo[3.1.0]hexane-3-carboxamide (Compound 207):** To a solution of compound **207-8** (0.1 g, 0.151 mmol) in MeOH (5 mL) and water (1 mL) was added lithium hydroxide monohydrate (32 mg, 0.753 mmol) and the mixture was stirred at room temperature for 2 hours. The mixture was concentrated to 1/5 volume and diluted with water. The mixture was washed with MTBE twice and the aqueous layer was acidified with 1N aqueous HCl to pH~3. The mixture was extracted with DCM twice and the combined organic layers were washed with brine, dried over anhydrous Na₂SO₄, and concentrated to dryness under reduced pressure. The residue was purified by prep-HPLC to give **Compound 207** (20 mg, yield 21.3%) as a white solid. ¹H-NMR (400 MHz, CD₃OD) δ 9.33 (s, 2H), 8.72 (s, 1H), 8.36 (s, 1H), 7.85 (d, *J* = 7.8 Hz, 1H), 7.58 (d, *J* = 7.8 Hz, 1H), 5.89 (d, *J =* 17.7 Hz, 1H), 5.77 (d, *J =* 17.7 Hz, 1H), 5.04 (d, *J* = 12.8 Hz, 1H), 4.93 (d, *J* = 12.8 Hz, 1H), 4.58 (s, 2H), 3.51 (dd, *J=* 5.6, 2.3 Hz, 1H), 3.34 (s, 1H), 2.74 (s, 3H), 2.65 (dd, *J* = 13.5, 9.3 Hz, 1H), 2.55 (s, 3H), 2.31 (dd, *J =* 13.6, 5.4 Hz, 1H), 2.23 (s, 3H), 1.41 (s, 3H), 1.14 (t, *J =* 5.6 Hz, 1H), 1.05 (dd, *J* = 5.7*,* 2.4 Hz, 1H); LC/MS (ESI) m/z: 622(M+H)⁺.

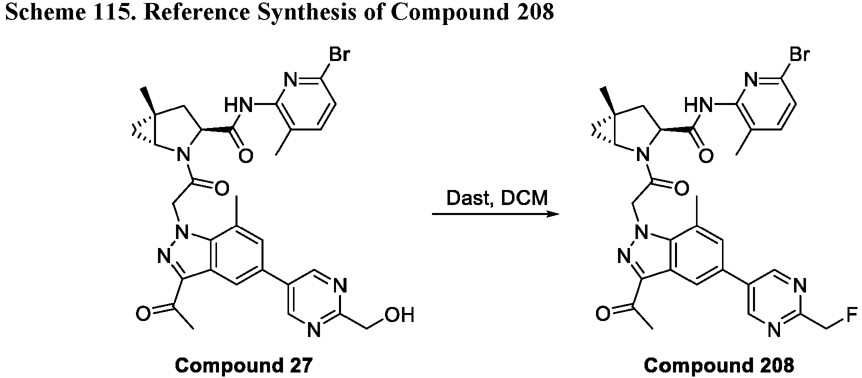

**(1R,3S,5R)-2-(2-(3-acetyl-5-(2-(fluoromethyl)pyrimidin-5-yl)-7-methyl-1H-indazol-1-yl)acetyl)-N-(6-bromo-3-methylpyridin-2-yl)-5-methyl-2-azabicyclo[3.1.0]hexane-3-carboxamide (Compound 208):** To a solution of **Compound 27** (0.033 g, 0.052 mmol) in DCM (3 mL) was added DAST (13 mg, 0.078mmol) at -20 °C. The mixture was degassed under N₂ atmosphere and stirred for 1 hour, then poured into phosphate buffered water (pH=7) and extracted with DCM twice. The combined organic layers were washed with brine, dried over anhydrous Na₂SO₄, filtered, and concentrated to dryness. The residue was purified by chromatography on silica gel (DCM: MeOH = 97: 3) and further purified by prep-HPLC to give **Compound 208** (2 mg, yield 6.3%) as a yellow solid. ¹H-NMR (400 MHz, CD₃OD) δ 9.14 (s, 2H), 8.47 (d, J = 1.6 Hz, 1H), 7.57 (d, J = 8.4 Hz, 2H), 7.40 (d, J = 7.9 Hz, 1H), 5.99 (d, J = 17.8 Hz, 1H), 5.82 (d, J = 17.7 Hz, 1H), 5.66 (s, 1H), 5.54 (s, 1H), 4.61 (s, 28H), 4.55 (dd, J = 9.1, 5.5 Hz, 1H), 3.56 (dd, J = 5.6, 2.4 Hz, 1H), 2.80 - 2.61 (m, 7H), 2.32 (dd, J = 13.4, 5.7 Hz, 1H), 2.14 (s, 3H), 1.43 (s, 3H), 1.33 (d, J = 15.8 Hz, 1H), 1.15 (t, J = 5.5 Hz, 1H), 0.99 (dd, J = 5.5, 2.4 Hz, 1H). LC/MS (ESI) m/z: 634/636 (M+H)⁺.

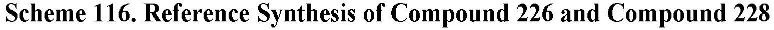

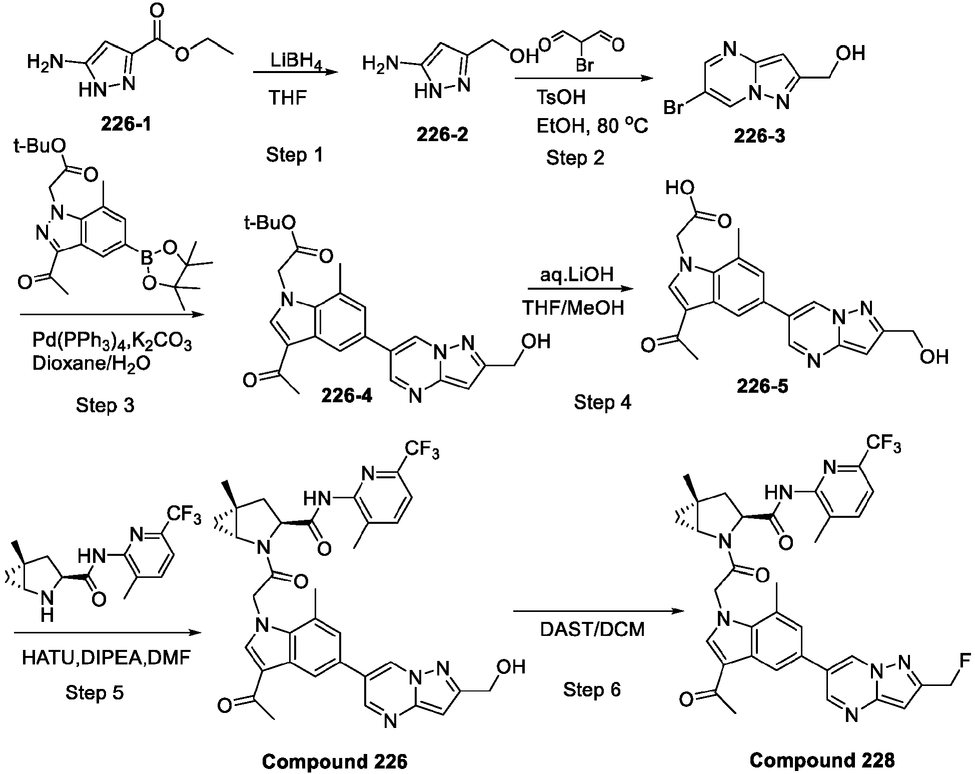

**Step 1: (5-amino-1H-pyrazol-3-yl)methanol (226-2):** To a mixture of compound **226-1** (1.0 g, 6.45 mmol) in THF (10 mL) was added LiBH₄ (8.0 mL, 16.01 mmol, 2M in THF) drop-wisely at 0 °C and the reaction was stirred at 60°C overnight. After completion of the reaction, the reaction mixture was quenched with MeOH and concentrated to dryness under reduced pressure. The residue was purified by column chromatography on silica gel (DCM: MeOH = 80: 1 to 20: 1) to give compound **2** (600 mg, yield 82.3%) as white solid. LC/MS (ESI) m/z: 114 (M+H)⁺.

**Step 2: (6-bromopyrazolo[1,5-a]pyrimidin-2-yl)methanol (226-3):** To a mixture of compound **226-2** (600 mg, 5.31mmol) and 2-bromomalonaldehyde (796 mg, 5.31 mmol) in EtOH (10 mL) was added TsOH (55 mg, 0.32 mmol). The mixture was stirred at 80 °C for 4 hours before concentrated to dryness under reduced pressure. The residue was washed with ethanol and filtered to give compound **226-3** (360 mg, yield 29.9%) as white solid. LC/MS (ESI) m/z: 228/230 (M+H)⁺.

**Step 3: tert-butyl 2-(3-acetyl-5-(2-(hydroxymethyl)pyrazolo[1,5-a]pyrimidin-6-yl)-7-methyl-1H-indol-1-yl)acetate (226-4):** To a mixture of compound **226-3** (120 mg, 0.53 mmol) and tert-butyl 2-(3-acetyl-7-methyl-5-(4,4,5,5-tetramethyl-1,3,2-dioxaborolan-2-yl)-1H-indol-1-yl)acetate (218 mg, 0.53 mmol) in 1,4-dioxane (8 mL) and water (1 mL) was added K₂CO₃ (183 mg, 1.33mmol) and Pd(PPh₃)₄ (61 mg, 0.05 mmol) at 0 °C. The reaction was stirred at 95 °C for 4 hours under N₂ atmosphere before diluted with EtOAc, washed with water and brine, dried over Na₂SO₄ and concentrated to dryness. The residue was purified by column chromatography on silica gel (DCM: MeOH= 100: 1 to 20: 1) to give compound **226-4** (180 mg, yield 78.6%) as yellow solid. LC/MS (ESI) m/z: 435 (M+H)⁺.

**Step 4: 2-(3-acetyl-5-(2-(hydroxymethyl)pyrazolo[1,5-a]pyrimidin-6-yl)-7-methyl-1H-indol-1-yl)acetic acid (226-5):** To a solution of compound **226-4** (180 mg, 0.41 mmol) in MeOH (4 mL) and THF (2 mL) was added a solution of sodium hydroxide (66 mg, 1.66 mmol) in water (2 mL) at 0 °C. The mixture was stirred at 40 °C for 2 hours before washed with Et₂O and water. The aqueous layer was acidified with 0.5M aq. HCl solution and extracted with DCM twice. The combined organic layers were washed with brine, dried over anhydrous Na₂SO₄, filtered and concentrated to dryness to give compound **226-5** (110 mg, yield 70.2%) as white solid. LC/MS (ESI) m/z: 379 (M+H)⁺.

**Step 5: (1R,3S,5R)-2-(2-(3-acetyl-5-(2-(hydroxymethyl)pyrazolo[1,5-a]pyrimidin-6-yl)-7-methyl-1H-indol-1-yl)acetyl)-5-methyl-N-(3-methyl-6-(trifluoromethyl)pyridin-2-yl)-2-azabicyclo[3.1.0]hexane-3-carboxamide (Compound 226):** To a mixture of compound **5** (100 mg, 0.26 mmol) and (1R,3S,5R)-5-methyl-N-(3-methyl-6-(trifluoromethyl)pyridin-2-yl)-2-azabicyclo[3.1.0]hexane-3-carboxamide hydrochloride (87 mg, 0.26 mmol) in DMF (5 mL) was added DIPEA (168 mg, 1.3 mmol) HATU (148 mg, 0.39 mmol) at 0 °C. The mixture was stirred at room temperature under N₂ atmosphere for 1 hour before diluted with EtOAc and washed with saturated aq.NH₄Cl solution and brine, dried over anhydrous Na₂SO₄, filtered and concentrated to dryness under reduced pressure. The residue was purified by column chromatography on silica gel (DCM: MeOH= 50: 1 to 10: 1) to give **Compound 226** (60 mg, yield 34.4%) as white solid. ¹H-NMR (400 MHz, DMSO-*d₆*) δ 10.49 (s, 1H), 9.24 (d, J = 2.0 Hz, 1H), 8.83 (d, J = 2.0 Hz, 1H), 8.39 (d, J = 1.6 Hz, 1H), 8.31 (s, 1H), 7.94 (d, J = 8.0 Hz, 1H), 7.69 (d, J = 7.6 Hz, 1H), 7.38 (d, J = 1.6 Hz, 1H), 6.68 (s, 1H), 5.75 (d, J = 18.0 Hz, 1H), 5.45-5.36 (m, 2H), 4.68 (d, J = 4.4 Hz, 2H), 4.47 (dd, J = 9.2, 9.2 Hz, 1H), 3.56 (dd, J = 5.6, 5.6 Hz, 1H), 2.65 (s, 3H), 2.61-2.55 (m, 1H), 2.46 (s, 3H), 2.19 (s, 3H), 2.08-2.03 (m, 1H), 1.33 (s, 3H), 1.02 (t, J = 5.4 Hz, 1H), 0.90 (dd, J = 5.6, 5.6 Hz, 1H); LC/MS (ESI) (m/z):660 (M+H)⁺.

**Step 6: (1R,3S,5R)-2-(2-(3-acetyl-5-(2-(fluoromethyl)pyrazolo[1,5-a]pyrimidin-6-yl)-7-methyl-1H-indol-1-yl)acetyl)-5-methyl-N-(3-methyl-6-(trifluoromethyl)pyridin-2-yl)-2-azabicyclo[3.1.0]hexane-3-carboxamide (Compound 228):** To a solution of **Compound 226** (40 mg, 0.06 mmol) in DCM (5 mL) was added DAST (29 mg, 0.18 mmol) at -30 °C. The mixture was degassed under N₂ atmosphere and stirred for 1 hour before poured into phosphate buffer (pH=7) and extracted with DCM twice. The combined organic layers were washed with brine, dried over anhydrous Na₂SO₄, filtered and concentrated to dryness. The residue was purified by prep-TLC (DCM: MeOH= 10: 1) to give **Compound 228** (5.8 mg, yield 14.5%) as white solid. ¹H-NMR (400 MHz, DMSO-*d₆*) δ 10.49 (s, 1H), 9.34 (s, 1H), 8.92 (d, J = 2.0 Hz, 1H), 8.42 (d, J = 2.0 Hz, 1H), 8.33 (s, 1H), 7.94 (d, J = 7.6 Hz, 1H), 7.70 (d, J = 8.0 Hz, 1H), 7.41 (s, 1H), 6.91 (s, 1H), 5.79-5.39 (m, 4H), 4.48 (dd, J = 9.6, 9.6 Hz, 1H), 3.56 (dd, J = 5.6, 5.6 Hz, 1H), 2.66 (s, 3H), 2.60-2.55 (m, 1H), 2.47 (s, 3H), 2.19 (s, 3H), 2.06 (dd, J = 13.6, 13.6 Hz, 1H), 1.34 (s, 3H), 1.03 (t, J = 5.4 Hz, 1H), 0.90 (dd, J = 5.6, 5.6 Hz, 1H); LC/MS (ESI) m/z: 662 (M+H)⁺.

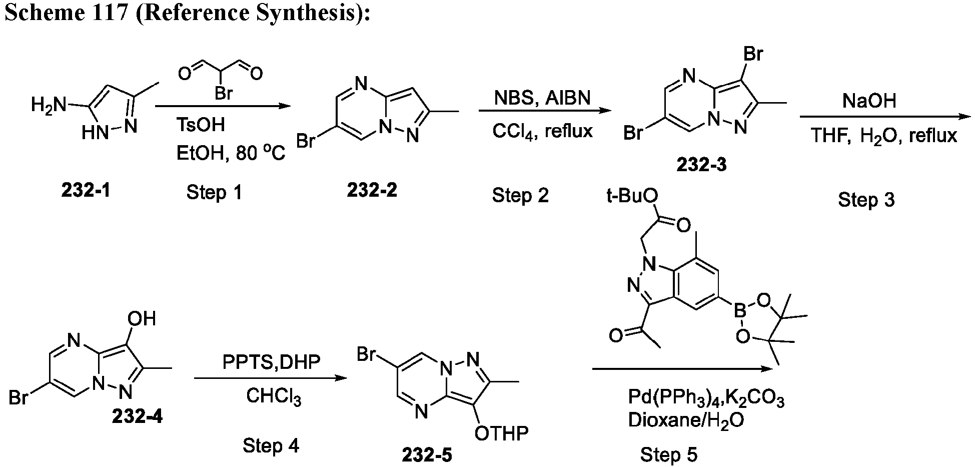

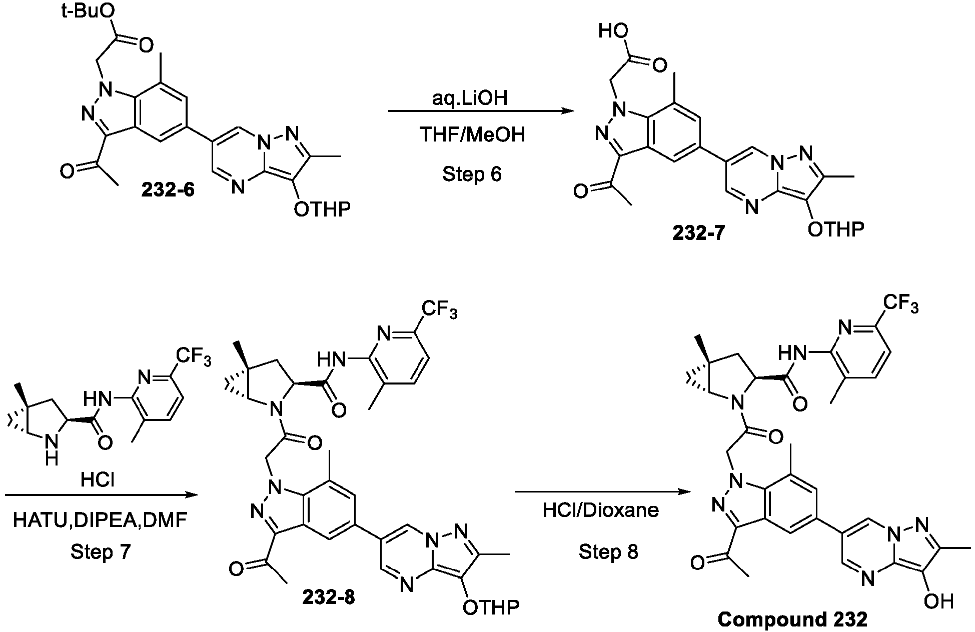

**Step 1: 6-bromo-2-methylpyrazolo[1,5-a]pyrimidine (232-2):** To a mixture of compound **232-1** (1.85 g, 19.07 mmol) and 2-bromomalonaldehyde (2.89 g, 19.27 mmol) in EtOH (20 mL) was added TsOH (185 mg, 1.08 mmol) and the mixture was stirred at 80 °C for 2 hours. The mixture was concentrated to dryness, the residue was washed with ethanol and filtered to give compound **232-2** (3.60 g, yield 89.6 %) as yellow solid. LC/MS (ESI) m/z: 212/214 (M+H)⁺.

**Step 2: 3,6-dibromo-2-methylpyrazolo[1,5-a]pyrimidine (232-3):** To a solution of compound **232-2** (4.0 g, 18.96 mmol) in CCl₄ (40 mL) under N₂ atmosphere was added NBS (3.54 g, 19.91 mmol) and AIBN (311 mg, 1.90 mmol) at 0 °C. The mixture was stirred at 80 °C for 45 minutes under N₂ atmosphere before diluted with DCM and washed with water and brine, dried over anhydrous Na₂SO₄, filtered and concentrated to dryness under reduced pressure to give compound **232-3** (5.4 g, yield 98.6%) as yellow solid. LC/MS (ESI) (m/z): 290/292 (M+H)⁺.

**Step 3: 6-bromo-2-methylpyrazolo[1,5-a]pyrimidin-3-ol (232-4):** To a solution of compound **232-3** (3.0 g, 10.38 mmol) in methanol (10 mL) and THF (20 mL) was added a solution of lithium hydroxide monohydrate (872 mg, 20.76 mmol) in water (5 mL) at 0 °C. The mixture was stirred at 60 °C for 3 hours before concentrated under reduced pressure. The residue was diluted with EtOAc and washed with brine, dried over anhydrous Na₂SO₄, filtered and concentrated under reduced pressure to give compound **232-4** (360 mg, yield 15.3%) as yellow oil. LC/MS (ESI) (m/z): 228/230 (M+H)⁺.

**Step 4: 6-bromo-2-methyl-3-((tetrahydro-2H-pyran-2-yl)oxy)pyrazolo[1,5-a]pyrimidine (232-5):** To a solution of compound **232-4** (360 mg, 1.59 mmol) in CHCl₃ (5 mL) was added 3,4-dihydro-2H-pyran (200 mg, 2.39 mmol) and 4-methylbenzenesulfonic acid (120 mg, 0.48 mmol) at 0 °C. The mixture was stirred at 60°C overnight before concentrated to dryness and the residue was purified by column chromatography on silica gel (eluted with PE: EtOAc= 10: 1 to 1: 1) to give compound **232-5** (270 mg, yield 54.8%) as yellow solid. LC/MS (ESI) m/z: 312/314 (M+H)⁺.

**Step 5: tert-butyl 2-(3-acetyl-7-methyl-5-(2-methyl-3-((tetrahydro-2H-pyran-2-yl)oxy)pyrazolo[1,5-a]pyrimidin-6-yl)-1H-indazol-1-yl)acetate (232-6):** To a mixture of compound **232-5** (75 mg, 0.24 mmol) and tert-butyl 2-(3-acetyl-7-methyl-5-(4,4,5,5-tetramethyl-1,3,2-dioxaborolan-2-yl)-1H-indazol-1-yl)acetate (90 mg, 0.22 mmol) in 1,4-dioxane (5.6 mL) and water (0.7 mL) was added K₂CO₃ (76 mg, 0.55 mmol) and Pd(PPh₃)₄ (25 mg, 0.02 mmol) at 0 °C. The reaction was stirred at 95 °C for 16 hours under N₂ atmosphere before diluted with EtOAc, washed with water and brine, dried over Na₂SO₄ and concentrated to dryness. The residue was purified by column chromatography on silica gel (PE: EtOAc = 10: 1 to 1: 1) to give compound **232-6 (55** mg, yield 48.2%) as yellow solid. LC/MS (ESI) m/z: 520 (M+H)⁺.

**Step 6: 2-(3-acetyl-7-methyl-5-(2-methyl-3-((tetrahydro-2H-pyran-2-yl)oxy)pyrazolo[1,5-a]pyrimidin-6-yl)-1H-indazol-1-yl)acetic acid (232-7): To** a solution of compound **232-6** (55 mg, 0.11 mmol) in MeOH (2 mL) and THF (1 mL) was added a solution of sodium hydroxide (17 mg, 0.42 mmol) in water (1 mL) at 0 °C. The mixture was stirred at 30 °C for 6 hours before washed with Et₂O and water. The aqueous layer was acidified with 0.5 M aq.HCl solution and extracted with DCM twice. The combined organic layers were washed with brine, dried over anhydrous Na₂SO₄, filtered and concentrated to dryness to give compound 232-7 (47 mg, yield 95.8%) as yellow solid. LC/MS (ESI) m/z: 464 (M+H)⁺.

**Step 7: (1R,3S,5R)-2-(2-(3-acetyl-7-methyl-5-(2-methyl-3-((tetrahydro-2H-pyran-2-yl)oxy)pyrazolo[1,5-a]pyrimidin-6-yl)-1H-indazol-1-yl)acetyl)-5-methyl-N-(3-methyl-6-(trifluoromethyl)pyridin-2-yl)-2-azabicyclo[3.1.0]hexane-3-carboxamide (232-8):** To a mixture of compound **232-7** (47 mg, 0.10 mmol) and (1R,3S,5R)-5-methyl-N-(3-methyl-6-(trifluoromethyl)pyridin-2-yl)-2-azabicyclo[3.1.0]hexane-3-carboxamide hydrochloride (34 mg, 0.10 mmol) in DMF (3 mL) was added DIPEA (65 mg, 0.50 mmol), HATU (57 mg, 0.15 mmol) at 0 °C. The mixture was stirred at room temperature under N₂ atmosphere for 1 hour before diluted with EtOAc and washed with saturated aq.NH₄Cl solution and brine, dried over anhydrous Na₂SO₄, filtered and concentrated to dryness. The residue was purified by column chromatography on silica gel (DCM: MeOH= 50: 1 to 10: 1) to give compound 232-8 (70 mg, yield 82.4%) as yellow solid. LC/MS (ESI) (m/z): 745 (M+H)⁺.

**Step 8: (1R,3S,5R)-2-(2-(3-acetyl-5-(3-hydroxy-2-methylpyrazolo[1,5-a]pyrimidin-6-yl)-7-methyl-1H-indazol-1-yl)acetyl)-5-methyl-N-(3-methyl-6-(trifluoromethyl)pyridin-2-yl)-2-azabicyclo[3.1.0]hexane-3-carboxamide (Compound 232):** A solution of compound **232-8** (70 mg, 0.094 mmol) in HCl/1,4-dioxane (3 mL) was stirred at 25 °C for 1 hour. The mixture was concentrated to dryness, the residue was purified by prep-HPLC to give Compound 232 (5.3 mg, yield 8.5%) as yellow solid. ¹H-NMR (400 MHz, DMSO-*d₆*) δ 10.49 (s, 1H), 8.47 (d, J = 3.2 Hz, 1H), 8.41 (d, J = 2.4 Hz, 1H), 8.32 (d, J = 1.6 Hz, 1H), 8.18 (s, 1H), 7.95 (d, J = 7.6 Hz, 1H), 7.70 (d, J = 7.6 Hz, 1H), 7.58 (s, 1H), 6.03 (d, J = 18.0 Hz, 1H), 5.67 (d, J = 18.0 Hz, 1H), 4.48 (dd, J = 5.2, 5.2 Hz, 1H), 3.61 (dd, J = 5.6, 5.6 Hz, 1H), 2.66 (s, 3H), 2.63 (s, 3H), 2.61-2.56 (m, 1H), 2.53 (s, 3H), 2.19 (s, 3H), 2.06 (dd, J = 13.6, 13.6 Hz, 1H), 1.34 (s, 3H), 1.03 (t, J = 5.4 Hz, 1H), 0.95 (dd, J = 5.2, 5.2 Hz, 1H); LC/MS (ESI) m/z: 661 (M+H)⁺.

### EXAMPLE 1. NON-LIMITING SYNTHETIC EXAMPLES OF COMPOUNDS OF THE PRESENT INVENTION

The below schemes are non-limiting examples of methods to make compounds of the present invention.

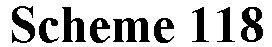

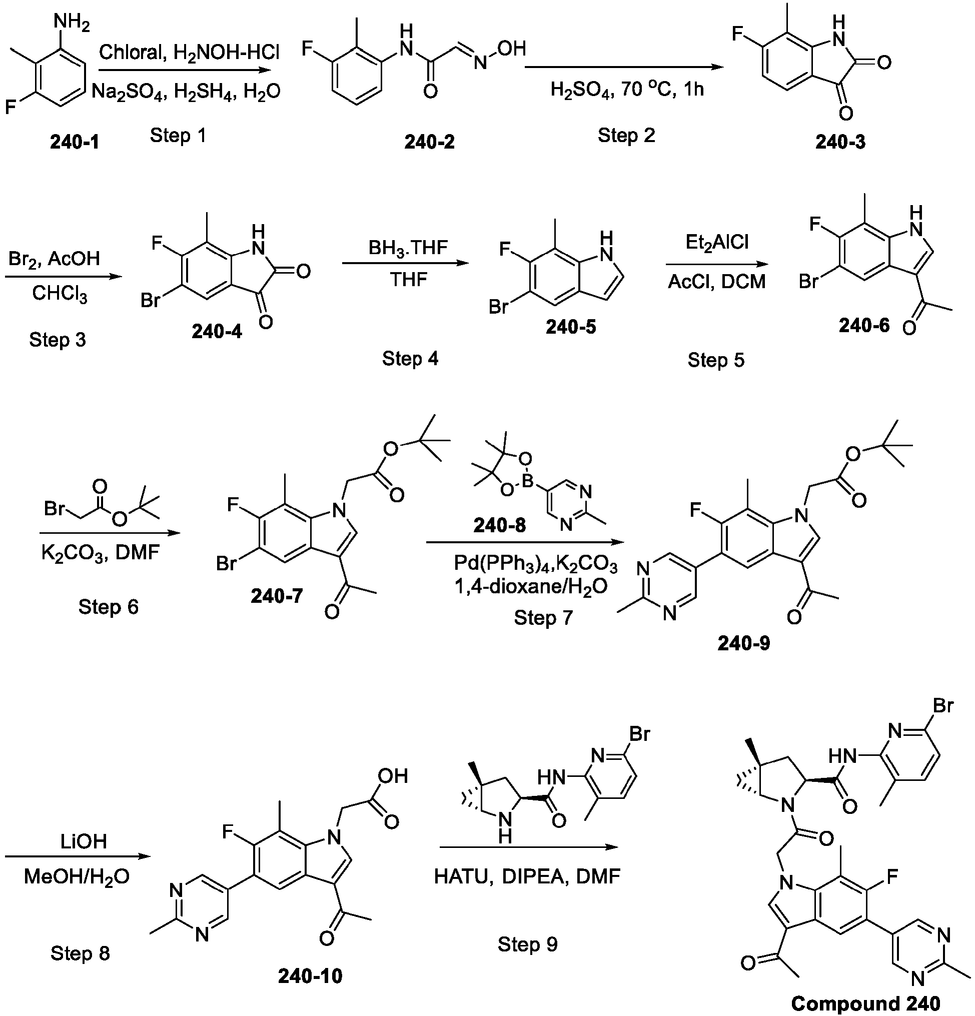

**Step 1: (E)-N-(3-fluoro-2-methylphenyl)-2-(hydroxyimino)acetamide \\\(240-2):** To a solution of chloral hydrate (4.53 g, 30.8 mmol) in deionized water (50 mL), Na₂SO₄ (51.64 g, 364 mmol), compound **240-1** (3.5 g, 28.0 mmol), H₂SO₄ (10 mL, 1 M) and hydroxylamine hydrochloride (5.84 g, 84.0 mmol) were added in sequence. The mixture was stirred at 130 °C for 1 hour before cooled to 80 °C and filtered to collect the product. The product was washed with deionized water and dried under vacuum to give compound **240-2** (3.2 g, yield 58.2%) as a yellow solid, which was directly used in the next step without further purification. LC/MS (ESI) (m/z): 197 (M+H)⁺.

**Step 2: 6-fluoro-7-methylindoline-2, 3-dione (240-3):** To a solution of compound **240-2** (3.2 g, 16.2 mmol) in 98% H₂SO₄ (30 mL) was stirred at 70 °C for 1.5 hours before poured into ice water. The mixture was extracted with EtOAc twice and the combined organic layers were washed with brine, dried over anhydrous Na₂SO₄, filtered and concentrated under reduced pressure. The residue was purified by chromatography on silica gel (PE: EtOAc = 5: 1) to give compound **240-3** (2.8 g, yield 96.5 %) as red solid. LC/MS (ESI) (m/z): 180 (M+H)⁺.

**Step 3: 5-bromo-6-fluoro-7-methylindoline-2,3-dione (240-4):** To a mixture of compound **240-3** (3.4 g, 18.89 mmol) in CHCl₃ (50 mL) was added AcOH (3mL), followed by Br₂ (1.5 mL, 23.61 mmol) in CHCl₃ (10 mL) at 0 °C. The mixture was stirred at room temperature for 1 hour before poured into ice water and the precipitation of product as a red solid happened. The precipitate was collected and further purified by chromatography on silica gel (PE: EtOAc = 4: 1) to give compound **240-4** (3.9 g, yield 78.0%) as red solid. LC/MS (ESI) (m/z): 258/260 (M+H)⁺.

**Step 4: 5-bromo-6-fluoro-7-methyl-1H-indole (240-5):** To a solution of compound 240-**4** (2.1 g, **8.13** mmol) **in** THF (20 mL) were added BH₃.THF (20.4 mL, 20.4 mmol) drop-wisely at 0 °C. The mixture was stirred at room temperature for 16 hours before quenched with ice-water and extracted with EtOAc. The organic layer was washed with brine, dried over anhydrous Na₂SO₄, filtered and concentrated to dryness. The residue was purified by chromatography on silica gel (PE: EtOAc = 94: 6) to give compound **240-5** (460 mg, yield 24.86%) as brown oil. LC/MS (ESI) (m/z): 228/230 (M+H)⁺.

**Step 5: 1-(5-bromo-6-fluoro-7-methyl-1H-indol-3-yl)ethanone (240-6):** To a solution of compound **240-5** (200 mg, 0.88 mmol) in DCM (5mL) was added diethylaluminum chloride (0.88 mL, 1M in hexane) drop-wisely at 0 °C under N₂ atmosphere. The mixture was stirred at 0 °C under N₂ atmosphere for 0.5 hour before acetyl chloride (69 mg, 0.88 mmol) was slowly added. The mixture was stirred at 25 °C under N₂ atmosphere for 12 hours before quenched with ice water, extracted with EtOAc twice. The combined organic layers were washed with water and brine, dried over anhydrous Na₂SO₄ and concentrated to dryness under reduced pressure. The resulting residue was purified by flash chromatography on silica gel (PE: EtOAc = 4: 1) to give compound **240-6** (160 mg, yield 66.7%) as yellow solid. LC/MS (ESI) (m/z): 270/272 (M+H)⁺.

**Step 6: tert-butyl 2-(3-acetyl-5-bromo-6-fluoro-7-methyl-1H-indol-1-yl)acetate (240-7)**:To a solution of compound **240-6** (160 mg, 0.6 mmol) in DMF (5 mL) were added potassium carbonate (0.25 g, 1.8 mmol) and tert-butyl 2-bromoacetate (170 mg, 0.14 mL, 0.9 mmol). The mixture was stirred at room temperature for 2 hours before diluted with EtOAc and washed with saturated aq.NH₄Cl solution and brine, dried over anhydrous Na₂SO₄, filtered and concentrated to dryness. The residue was purified by chromatography on silica gel (PE: EtOAc =4: 1) to give compound **240-7** (0.2 g, yield 87.0%) as white solid. LC/MS (ESI) (m/z): 384/386 (M+H)⁺.

**Step 7: tert-butyl 2-(3-acetyl-6-fluoro-7-methyl-5-(2-methylpyrimidin-5-yl)-1H-indol-1-yl)acetate (240-9):** To a mixture of compound **240-7** (0.1 g, 0.26 mmol) and 2-methyl-5-(4,4,5,5-tetramethyl-1,3,2-dioxaborolan-2-yl)pyrimidine (69 mg, 0.31 mmol) in 1,4-dioxane (4 mL) and water (0.5 mL) was added K₂CO₃ (90 mg, 0.65 mmol) and Pd(PPh₃)₄ (30 mg, 0.026 mmol) under N₂ atmosphere. The mixture was degassed under N₂ atmosphere for three times and stirred at 90 °C under N₂ atmosphere for 2 hours before diluted with EtOAc and washed with water and brine, dried over anhydrous Na₂SO₄ and concentrated to dryness under reduced pressure. The residue was purified by flash chromatography on silica gel (PE: EtOAc = 10: 1) to give compound **240-9** (0.15 g, yield 100%) as white solid. LC/MS (ESI) (m/z): 398 (M+H)⁺.

**Step 8: 2-(3-acetyl-6-fluoro-7-methyl-5-(2-methylpyrimidin-5-yl)-1H-indol-1-yl)acetic acid (240-10):** To a solution of compound **240-9** (0.15 g, 0.38 mmol) in MeOH (5 mL) and water (1 mL) was added a solution of lithium hydroxide monohydrate (80 mg, 1.88 mmol) at 0 °C. The mixture was stirred at room temperature for 1 hour before concentrated to 1/5 volume, diluted with water and washed with MTBE twice. The aqueous layer was acidified with 1N aq.HCl to pH~3, extracted with DCM twice. The combined organic layers were washed with brine, dried over anhydrous Na₂SO₄, and concentrated under reduced pressure to dryness to give compound **240-10** (0.12 g, yield 93.1%) as white solid. LC/MS (ESI) (m/z): 342 (M+H)⁺.

**Step 9: (1R,3S,5R)-2-(2-(3-acetyl-6-fluoro-7-methyl-5-(2-methylpyrimidin-5-yl)-1H-indol-1-yl)acetyl)-N-(6-bromo-3-methylpyridin-2-yl)-5-methyl-2-azabicyclo[3.1.0]hexane-3-carboxamide (Compound 240):** To a mixture of compound **240-10** (60 mg, 0.18 mmol) and (1R,3S,5R)-N-(6-bromo-3-methylpyridin-2-yl)-5-methyl-2-azabicyclo[3.1.0]hexane-3-carboxamide (81 mg, 0.26 mmol) in DMF (5 mL) was added DIPEA (0.12 mL, 0.72 mmol) and HATU (0.134 g, 0.36 mmol) at 0 °C. The mixture was stirred at room temperature for 2 hours under N₂ atmosphere before diluted with EtOAc and washed with saturated aq.NH₄Cl solution and brine, dried over anhydrous Na₂SO₄, filtered and concentrated to dryness. The residue was purified by chromatography on silica gel (PE: EtOAc= 4: 1) and further purified by prep-HPLC to give **Compound 240 (28** mg, yield 25.2%) as white solid. ¹H-NMR (400 MHz, CD₃OD) δ 8.85 (d, J = 1.6 Hz, 2H), 8.26 (dd, J = 7.8, 2.2 Hz, 1H), 8.13 (d, J = 2.3 Hz, 1H), 7.53 (d, J = 7.9 Hz, 1H), 7.36 (dd, J = 7.9, 1.5 Hz, 1H), 5.61 (dd, J = 18.0, 3.1 Hz, 1H), 5.51 (dd, J = 18.0, 3.1 Hz, 1H), 4.54-4.48 (m, 1H), 3.47 (dd, J = 5.5, 2.4 Hz, 1H), 2.74 (s, 3H), 2.62 (dd, J = 13.5, 9.2 Hz, 1H), 2.55 (d, J = 2.0 Hz, 3H), 2.47 (d, J = 2.4 Hz, 3H), 2.29 (dd, J = 13.5, 5.5 Hz, 1H), 2.12 (s, 3H), 1.40 (s, 3H), 1.10 (t, J = 5.6 Hz, 1H), 0.95 (dd, J = 5.6, 2.4 Hz, 1H); LC/MS (ESI) m/z: 633(M+H)⁺.

### Additional Compounds of the present invention

| **Compounds** | **Characterization** |
|---|---|
| 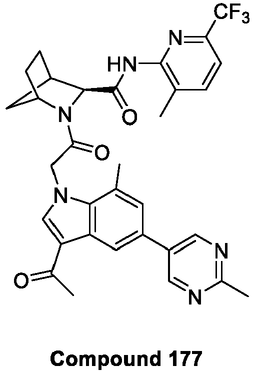 | **Compound 177** ¹H NMR (400 MHz, DMSO-d6) δ 1.66 (dd, J = 11.8, 21.7 Hz, 4H), 1.70 - 1.86 (m, 2H), 2.20 (s, 3H), 2.45 (s, 3H), 2.67 (d, J = 2.5 Hz, 6H), 2.93 (s, 1H), 4.47 (d, J = 3.6 Hz, 1H), 4.56 (s, 1H), 5.38 - 5.63 (m, 2H), 7.32 (d, J = 1.8 Hz, 1H), 7.69 (d, J = 7.8 Hz, 1H), 7.94 (d, J = 7.8 Hz, 1H), 8.24 (s, 1H), 8.29 - 8.42 (m, 1H), 8.96 (d, J = 7.2 Hz, 2H), 10.52 (s, 1H). |
| 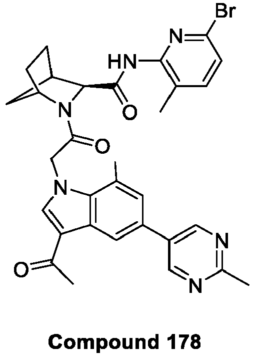 | **Compound 178** ¹H NMR (400 MHz, DMSO-d6) δ 1.65 (dd, J = 10.6, 24.4 Hz, 3H), 1.74 - 1.90 (m, 3H), 2.07 (s, 3H), 2.44 (d, J = 4.3 Hz, 3H), 2.67 (s, 6H), 2.90 (s, 1H), 4.42 (d, J = 3.6 Hz, 1H), 4.55 (s, 1H), 5.36 - 5.55 (m, 2H), 7.32 (d, J = 1.8 Hz, 1H), 7.43 (d, J = 7.9 Hz, 1H), 7.62 (d, J = 8.0 Hz, 1H), 8.24 (s, 1H), 8.31 - 8.37 (m, 1H), 8.96 (d, J = 5.8 Hz, 2H), 10.35 (s, 1H). |
| 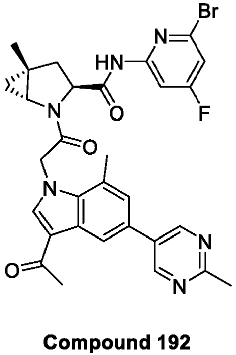 | **Compound 192** ¹H NMR (400 MHz, DMSO-d6) δ 0.85 (dd, J = 2.3, 5.4 Hz, 1H), 0.99 (t, J = 5.5 Hz, 1H), 1.31 (s, 3H), 1.99 (dd, J = 6.1, 13.3 Hz, 1H), 2.45 (s, 3H), 2.48 - 2.50 (m, 1H), 2.66 (d, J = 3.4 Hz, 6H), 3.57 (dd, J = 2.3, 5.5 Hz, 1H), 4.42 (dd, J = 6.1, 9.1 Hz, 1H), 5.41 (d, J = 17.9 Hz, 1H), 5.76 (d, J = 17.9 Hz, 1H), 7.33 - 7.38 (m, 1H), 7.44 (dd, J = 2.0, 7.6 Hz, 1H), 7.86 (dd, J = 2.0, 11.0 Hz, 1H), 8.30 - 8.38 (m, 2H), 8.96 (s, 2H), 11.14 (s, 1H). |
| 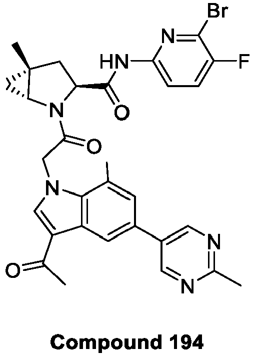 | **Compound 194** ¹H NMR (400 MHz, DMSO-d6) δ 0.85 (dd, J = 2.3, 5.5 Hz, 1H), 0.99 (t, J = 5.4 Hz, 1H), 1.31 (s, 3H), 1.99 (dd, J = 6.1, 13.2 Hz, 1H), 2.45 (s, 3H), 2.49 (d, J = 4.3 Hz, 1H), 2.67 (d, J = 3.2 Hz, 6H), 3.58 (dd, J = 2.4, 5.6 Hz, 1H), 4.41 (dd, J = 6.2, 9.0 Hz, 1H), 5.40 (d, J = 17.9 Hz, 1H), 5.76 (d, J = 17.9 Hz, 1H), 7.35 (d, J = 1.8 Hz, 1H), 7.86 (dd, J = 7.7, 8.9 Hz, 1H), 8.07 (dd, J = 3.3, 8.9 Hz, 1H), 8.32 (s, 1H), 8.35 (d, J = 1.8 Hz, 1H), 8.95 (s, 2H), 10.92 (s, 1H). |
| 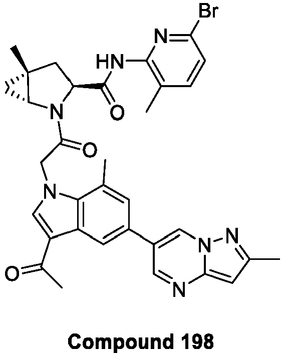 | **Compound 198** ¹H NMR (400 MHz, DMSO-d6) δ 0.90 (dd, J = 2.3, 5.4 Hz, 1H), 1.02 (t, J = 5.4 Hz, 1H), 1.33 (s, 3H), 2.06 (s, 4H), 2.46 (d, J = 1.7 Hz, 6H), 2.53 - 2.60 (m, 1H), 2.65 (s, 3H), 3.55 (dd, J = 2.3, 5.7 Hz, 1H), 4.42 (dd, J = 5.6, 9.1 Hz, 1H), 5.41 (d, J = 17.9 Hz, 1H), 5.74 (d, J = 17.9 Hz, 1H), 6.56 (s, 1H), 7.38 (d, J = 1.8 Hz, 1H), 7.44 (d, J = 7.9 Hz, 1H), 7.62 (d, J = 8.0 Hz, 1H), 8.31 (s, 1H), 8.38 (s, 1H), 8.80 (d, J = 2.3 Hz, 1H), 9.21 (d, J = 2.2 Hz, 1H), 10.33 (s, 1H). |
| 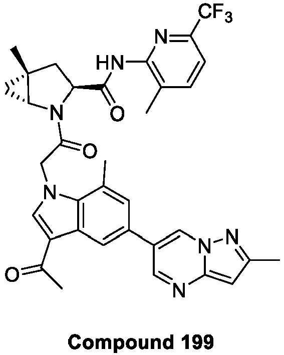 | **Compound 199** ¹H NMR (400 MHz, DMSO-d6) δ 0.88 - 0.93 (m, 1H), 1.03 (t, J = 5.4 Hz, 1H), 1.34 (s, 3H), 2.06 (dd, J = 5.7, 13.2 Hz, 1H), 2.19 (s, 3H), 2.46 (s, 6H), 2.53 - 2.61 (m, 1H), 2.65 (s, 3H), 3.55 - 3.60 (m, 1H), 4.48 (dd, J = 5.6, 9.1 Hz, 1H), 5.42 (d, J = 17.8 Hz, 1H), 5.75 (d, J = 17.9 Hz, 1H), 6.56 (s, 1H), 7.38 (s, 1H), 7.70 (d, J = 7.8 Hz, 1H), 7.95 (d, J = 7.8 Hz, 1H), 8.32 (s, 1H), 8.38 (d, J = 1.9 Hz, 1H), 8.80 (d, J = 2.3 Hz, 1H), 9.20 (d, J = 2.2 Hz, 1H), 10.49 (s, 1H). |
| 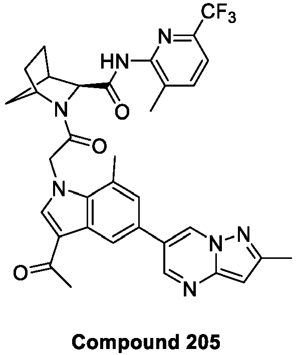 | **Compound 205** ¹H NMR (400 MHz, DMSO-d6) δ 1.55 - 1.71 (m, 3H), 1.73 - 1.88 (m, 3H), 2.21 (s, 3H), 2.46 (s, 6H), 2.68 (s, 3H), 2.94 (s, 1H), 4.46 - 4.50 (m, 1H), 4.53 - 4.59 (m, 1H), 5.38 - 5.54 (m, 2H), 6.56 (s, 1H), 7.36 (s, 1H), 7.69 (d, J = 7.8 Hz, 1H), 7.94 (d, J = 7.8 Hz, 1H), 8.24 (s, 1H), 8.36 (d, J = 1.8 Hz, 1H), 8.74 - 8.86 (m, 1H), 9.19 (d, J = 2.2 Hz, 1H), 10.52 (s, 1H). |
| 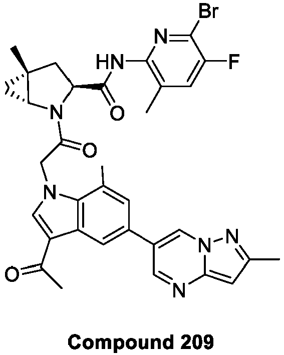 | **Compound 209** ¹H NMR (400 MHz, DMSO-d6) δ 0.88 - 0.95 (m, 1H), 1.02 (t, J = 5.5 Hz, 1H), 1.33 (s, 3H), 1.95 - 2.11 (m, 4H), 2.47 (d, J = 1.5 Hz, 6H), 2.54 - 2.59 (m, 1H), 2.65 (s, 3H), 3.54 - 3.57 (m, 1H), 4.37 - 4.42 (m, 1H), 5.42 (d, J = 17.8 Hz, 1H), 5.74 (d, J = 17.9 Hz, 1H), 6.56 (s, 1H), 7.38 (d, J = 1.7 Hz, 1H), 7.83 (d, J = 8.5 Hz, 1H), 8.31 (s, 1H), 8.39 (s, 1H), 8.80 (d, J = 2.3 Hz, 1H), 9.21 (d, J = 2.2 Hz, 1H), 10.33 (s, 1H). |
| 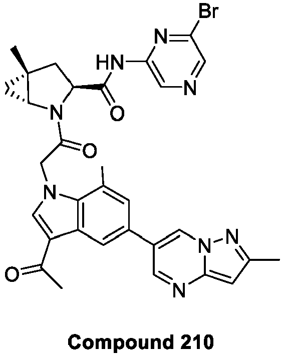 | **Compound 210** ¹H NMR (400 MHz, DMSO-d6) δ 0.84 - 0.89 (m, 1H), 1.01 (t, J = 5.4 Hz, 1H), 1.32 (s, 3H), 2.04 (dd, J = 6.1, 13.3 Hz, 1H), 2.44 - 2.48 (m, 7H), 2.67 (s, 3H), 3.54 - 3.64 (m, 1H), 4.42 - 4.48 (m, 1H), 5.43 (d, J = 17.9 Hz, 1H), 5.76 (d, J = 17.9 Hz, 1H), 6.56 (s, 1H), 7.40 (s, 1H), 8.32 (s, 1H), 8.37 (d, J = 1.8 Hz, 1H), 8.54 (s, 1H), 8.79 (d, J = 2.3 Hz, 1H), 9.20 (d, J = 2.2 Hz, 1H), 9.28 (s, 1H), 11.20 (s, 1H). |
| 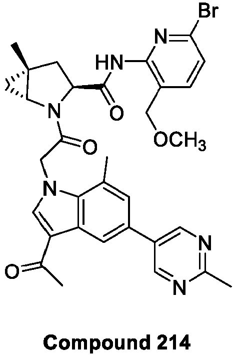 | **Compound 214** ¹H NMR (400 MHz, DMSO-d6) δ 0.85 - 0.93 (m, 1H), 1.01 (t, J = 5.4 Hz, 1H), 1.34 (s, 3H), 2.04 (dd, J = 5.9, 13.2 Hz, 1H), 2.45 (s, 3H), 2.54 - 2.60 (m, 1H), 2.65 (s, 3H), 2.67 (s, 3H), 3.06 (s, 3H), 3.52 - 3.61 (m, 1H), 4.07 - 4.25 (m, 2H), 4.32 - 4.43 (m, 1H), 5.41 (d, J = 17.9 Hz, 1H), 5.76 (d, J = 17.9 Hz, 1H), 7.34 (d, J = 1.8 Hz, 1H), 7.53 (d, J = 8.1 Hz, 1H), 7.76 (d, J = 8.1 Hz, 1H), 8.28 - 8.37 (m, 2H), 8.95 (s, 2H), 10.50 (s, 1H). |
| 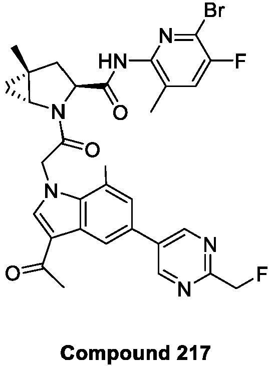 | **Compound 217** ¹H NMR (400 MHz, Chloroform-d) δ 0.84 - 0.99 (m, 2H), 1.28 (s, 3H), 1.45 (s, 3H), 2.35 (dd, J = 8.6, 13.5 Hz, 1H), 2.54 (s, 3H), 2.62 - 2.73 (m, 4H), 3.09 - 3.13 (m, 1H), 4.75 - 4.85 (m, 1H), 5.30 - 5.49 (m, 2H), 5.61 (s, 1H), 5.73 (s, 1H), 7.17 (s, 1H), 7.28 (s, 1H), 7.73 (s, 1H), 8.43 (s, 1H), 8.60 (d, J = 1.7 Hz, 1H), 9.09 (s, 1H), 9.40 (s, 1H). |
| 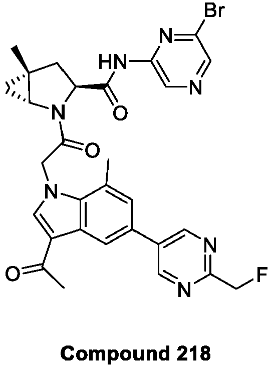 | **Compound 218** ¹H NMR (400 MHz, Chloroform-d) δ 0.84 - 0.99 (m, 2H), 1.28 (s, 3H), 2.35 (dd, J = 8.6, 13.5 Hz, 1H), 2.54 (s, 3H), 2.62 - 2.73 (m, 4H), 3.09 - 3.13 (m, 1H), 4.75 - 4.85 (m, 1H), 5.30 - 5.49 (m, 2H), 5.61 (s, 1H), 5.73 (s, 1H), 7.17 (s, 1H), 7.28 (s, 2H), 7.73 (s, 1H), 8.43 (s, 1H), 8.60 (d, J = 1.7 Hz, 1H), 9.09 (s, 1H), 9.40 (s, 1H). |
| 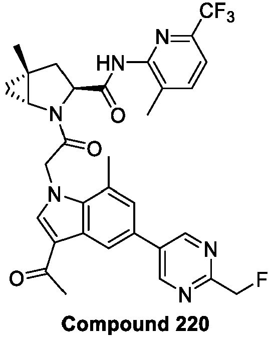 | **Compound 220** (2.0 mg, yield 5%) as white solid. ¹H-NMR (400 MHz, CD₃OD) δ 9.10 (s, 2H), 8.51 (d, J = 1.6 Hz, 1H), 8.17 (s, 1H), 7.86 (d, J = 8.0 Hz, 1H), 7.59 (d, J = 8.0 Hz, 1H), 7.36 (d, J = 2.0 Hz, 1H), 5.68-5.61 (m, 2H), 5.56 (d, J = 18.0 Hz, 1H), 5.50 (s, 1H), 4.57-4.55 (m, 1H), 3.50 (dd, J = 5.6, 5.6 Hz, 1H), 2.72 (s, 3H), 2.65 (dd, J = 13.6, 13.6 Hz, 1H), 2.53 (s, 3H), 2.32-2.27 (m, 1H), 2.25 (s, 3H), 1.41 (s, 3H), 1.11 (t, J = 5.4 Hz, 1H), 0.96 (dd, J = 5.6, 5.6 Hz, 1H). LC/MS (ESI) m/z: 623 (M+H)⁺. |
| 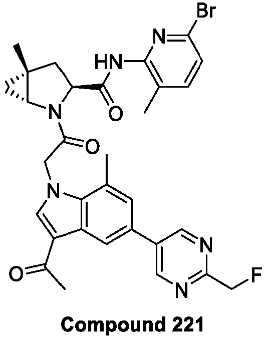 | **Compound 221** (2.0 mg, yield 3%) as white solid. ¹H-NMR (400 MHz, CD₃OD) δ 9.10 (s, 2H), 8.53-8.45 (m, 3H), 8.16 (s, 1H), 7.54 (d, J = 8.0 Hz, 1H), 7.38-7.34 (m, 2H), 5.67-5.60 (m, 2H), 5.55 (d, J = 18.0 Hz, 1H), 5.50 (s, 1H), 4.56-4.52 (m, 1H), 3.49-3.47 (m, 1H), 2.71 (s, 3H), 2.65-2.59 (m, 1H), 2.52 (s, 3H), 2.30 (dd, J = 13.2, 13.2 Hz, 1H), 2.12 (s, 3H), 1.40 (s, 3H), 1.10 (t, J = 5.4 Hz, 1H), 0.95 (dd, J = 5.6, 5.6 Hz, 1H). LC/MS (ESI) m/z: 633 (M+H)⁺. |
| 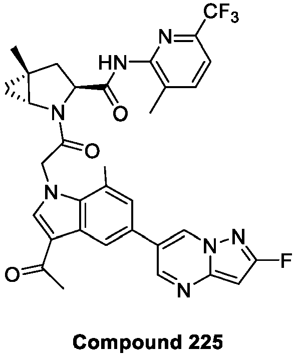 | **Compound 225** ¹H NMR (400 MHz, DMSO-d6) δ 0.91 (dd, J = 2.4, 5.4 Hz, 1H), 1.03 (t, J = 5.4 Hz, 1H), 1.34 (s, 3H), 2.06 (dd, J = 5.6, 13.3 Hz, 1H), 2.19 (s, 3H), 2.47 (s, 3H), 2.58 (dd, J = 3.6, 9.8 Hz, 1H), 2.65 (s, 3H), 3.55 - 3.58 (m, 1H), 4.48 (dd, J = 5.6, 9.1 Hz, 1H), 5.25 - 5.55 (m, 1H), 5.76 (d, J = 17.9 Hz, 1H), 6.55 (d, J = 4.9 Hz, 1H), 7.39 (d, J = 1.8 Hz, 1H), 7.70 (d, J = 7.8 Hz, 1H), 7.95 (d, J = 7.8 Hz, 1H), 8.32 (s, 1H), 8.39 (d, J = 1.9 Hz, 1H), 8.94 (dd, J = 2.2, 5.7 Hz, 1H), 9.30 (dd, J = 2.2, 6.5 Hz, 1H), 10.49 (s, 1H). |
| 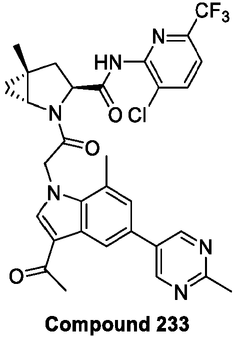 | **Compound 233** (3.0 mg, yield 9%) as white solid. ¹H-NMR (400 MHz, DMSO-d₆) δ 10.69 (s, 1H), 8.95 (s, 2H), 8.35 (d, J = 2.0 Hz, 1H), 8.31-8.26 (m, 2H), 7.82 (d, J = 8.4 Hz, 1H), 7.35-7.29 (m, 1H), 5.74 (d, J = 18.0 Hz, 1H), 5.40 (d, J = 18.0 Hz, 1H), 4.52 (dd, J = 9.2, 9.2 Hz, 1H), 3.54 (dd, J = 5.6, 5.6 Hz, 1H), 2.66 (s, 3H), 2.64 (s, 3H), 2.58-2.52 (m, 1H), 2.45 (s, 3H), 2.12 (dd, J = 13.2, 13.2 Hz, 1H), 1.33 (s, 3H), 1.03 (t, J = 5.4 Hz, 1H), 0.90 (dd, J = 5.2, 5.2 Hz, 1H). LC/MS (ESI) m/z: 625 (M+H)⁺. |

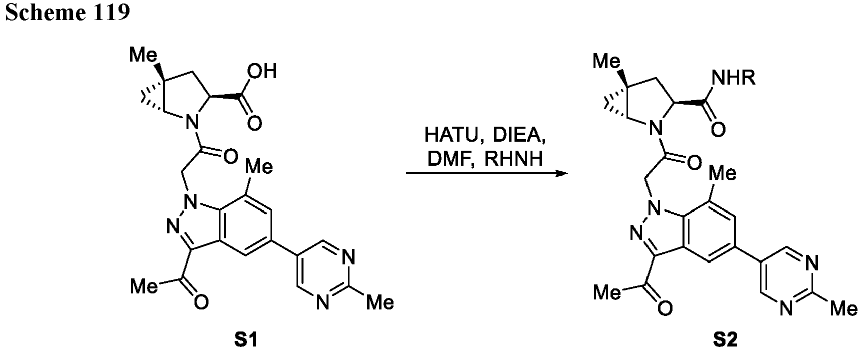

### General Procedure (GP) 1

To a stirring solution of **S1** (1R,3S,5R)-2-(2-(3-acetyl-7-methyl-5-(2-methylpyrimidin-5-yl)-1H-indazol-1-yl)acetyl)-5-methyl-2-azabicyclo[3.1.0]hexane-3-carboxylic acid (1 equiv) in DMF (10 vol) at rt under nitrogen atmosphere is added N,N-diisopropylethylamine (DIEA) (3 equiv) and 1-[Bis(dimethylamino)methylene]-1H-1,2,3-triazolo[4,5-b]pyridinium 3-oxid hexafluorophosphate (HATU) (1.05 equiv). The reaction mixture is stirred at rt for 10 minutes. Next, amine (RHNH) is added to the reaction mixture and the mixture is stirred at rt for an additional 30 min. After completion of the reaction, the reaction mixture is diluted with MeOH and purified directly by reverse phase HPLC to give compound **S2.**

### EXAMPLE 2. NON-LIMITING EXAMPLES OF COMPOUNDS OF THE PRESENT INVENTION

Table 1 shows illustrative Factor D inhibitors with characaterizing data. The assay of Example 3 was used to determine the IC₅₀'s of the compounds. Other standard factor D inhibition assays are also available. Three ***s are used to denote compounds with an IC₅₀ less than 1 micromolar; two **s indicate compound with an IC₅₀ between 1 micromolar and 10 micromolar, and one * denotes compounds with an IC₅₀ greater than 10 micromolar.

**Table 2. Additional Non-limiting Examples of Compounds of the Present Invention**

| Cm p No. | Structure | Name | IC₅₀ (Sta rs) | RT min (Method A,B, C or D) | MS (M +1) |
|---|---|---|---|---|---|
| 144 | 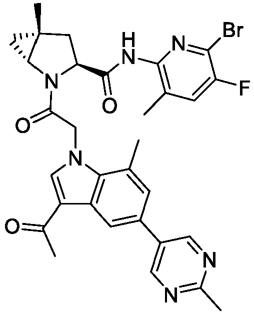 | (1R,3S,5R)-2-(2-(3-acetyl-7-methyl-5-(2-methylpyrimidin-5-yl)-1H-indol-1-yl)acetyl)-N-(6-bromo-5-fluoro-3-methylpyridin-2-yl)-5-methyl-2-azabicyclo[3.1.0]he xane-3-carboxamide | *** | 1.84 (A) | 634 |
| 155 | 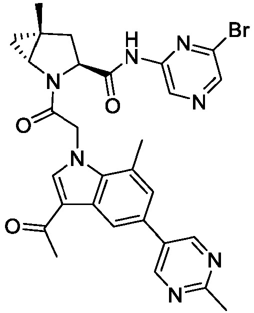 | (1R,3S,5R)-2-(2-(3-acetyl-7-methyl-5-(2-methylpyrimidin-5-yl)-1H-indol-1-yl)acetyl)-N-(6-bromopyrazin-2-yl)-5-methyl-2-azabicyclo[3.1.0]he xane-3-carboxamide | *** | 1.80 (A) | 604 |
| 156 | 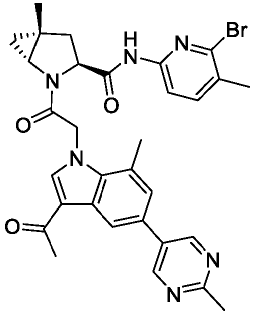 | (1R,3S,5R)-2-(2-(3-acetyl-7-methyl-5-(2-methylpyrimidin-5-yl)-1H-indol-1-yl)acetyl)-N-(6-bromo-5-methylpyridin-2-yl)-5-methyl-2-azabicyclo[3.1.0]he xane-3-carboxamide | *** | 2.10 (A) | 615 |
| 160 | 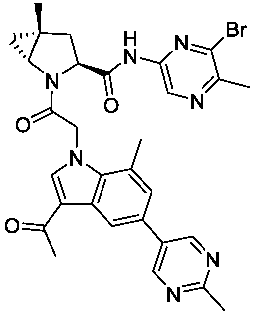 | (1R,3S,5R)-2-(2-(3-acetyl-7-methyl-5-(2-methylpyrimidin-5-yl)-1H-indol-1-yl)acetyl)-N-(6-bromo-5-methylpyrazin-2-yl)-5-methyl-2-azabicyclo[3.1.0]he xane-3-carboxamide | *** | 1.90 (A) | 616 |
| 177 | 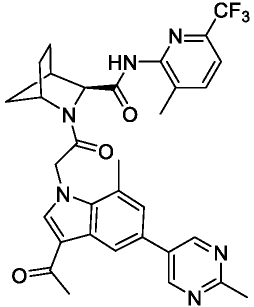 | (1S,3S,4R)-2-(2-(3-acetyl-7-methyl-5-(2-methylpyrimidin-5-yl)-1H-indol-1-yl)acetyl)-N-(3-methyl-6-(trifluoromethyl)py ridin-2-yl)-2-azabicyclo[2.2.1]he ptane-3-carboxamide | *** | 1.81 (A) | 605 |
| 178 | 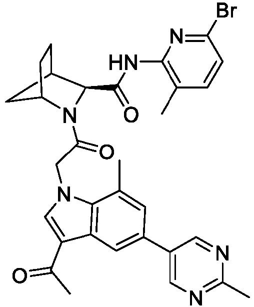 | (1S,3S,4R)-2-(2-(3-acetyl-7-methyl-5-(2-methylpyrimidin-5-yl)-1H-indol-1-yl)acetyl)-N-(6-bromo-3-methylpyridin-2-yl)-2-azabicyclo[2.2.1]he ptane-3-carboxamide | *** | 1.68 (A) | 616 |
| 192 | 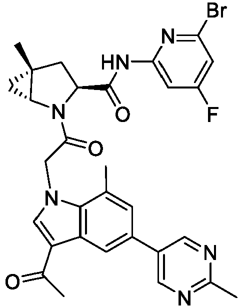 | (1R,3S,5R)-2-(2-(3-acetyl-7-methyl-5-(2-methylpyrimidin-5-yl)-1H-indol-1-yl)acetyl)-N-(6-bromo-4-fluoropyridin-2-yl)-5-methyl-2-azabicyclo[3.1.0]he xane-3-carboxamide | *** | 2.15(A) | 620 |
| 194 | 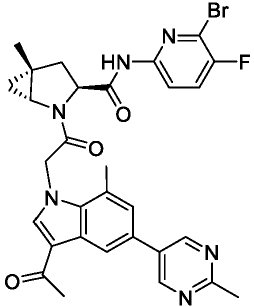 | (1R,3S,5R)-2-(2-(3-acetyl-7-methyl-5-(2-methylpyrimidin-5-yl)-1H-indol-1-yl)acetyl)-N-(6-bromo-5-fluoropyridin-2-yl)-5-methyl-2-azabicyclo[3.1.0]he xane-3-carboxamide | *** | 2.05(A) | 619 |
| 198 | 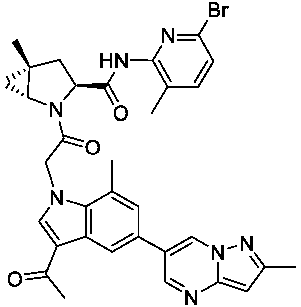 | (1R,3S,5R)-2-(2-(3-acetyl-7-methyl-5-(2-methylpyrazolo[1,5 -a]pyrimidin-6-yl)-1H-indol-1-yl)acetyl)-N-(6-bromo-3-methylpyridin-2-yl)-5-methyl-2-azabicyclo[3.1.0]he xane-3-carboxamide | *** | 2.03 (A) | 654 |
| 199 | 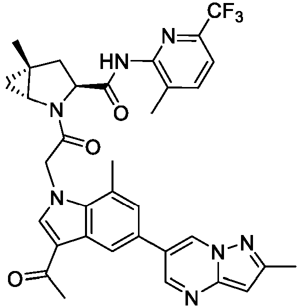 | (1R,3S,5R)-2-(2-(3-acetyl-7-methyl-5-(2-methylpyrazolo[1,5 -a]pyrimidin-6-yl)-1H-indol-1-yl)acetyl)-5-methyl-N-(3-methyl-6-(trifluoromethyl)py ridin-2-yl)-2-azabicyclo[3.1.0]he xane-3-carboxamide | *** | 2.15 (A) | 644 |
| 205 | 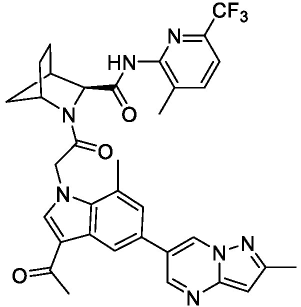 | (1S,3S,4R)-2-(2-(3-acetyl-7-methyl-5-(2-methylpyrazolo[1,5 -a]pyrimidin-6-yl)-1H-indol-1-yl)acetyl)-N-(3-methyl-6-(trifluoromethyl)py ridin-2-yl)-2-azabicyclo[2.2.1]he ptane-3-carboxamide | *** | 2.07 (A) | 644 |
| 209 | 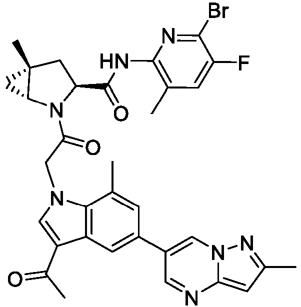 | (1R,3S,5R)-2-(2-(3-acetyl-7-methyl-5-(2-methylpyrazolo[1,5 -a]pyrimidin-6-yl)-1H-indol-1-yl)acetyl)-N-(6-bromo-5-fluoro-3-methylpyridin-2-yl)-5-methyl-2-azabicyclo[3.1.0]he xane-3-carboxamide | *** | 2.09 (A) | 674 |
| 210 | 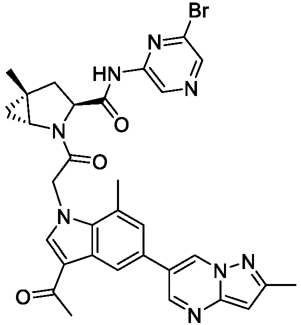 | (1R,3S,5R)-2-(2-(3-acetyl-7-methyl-5-(2-methylpyrazolo[1,5 -a]pyrimidin-6-yl)-1H-indol-1-yl)acetyl)-N-(6-bromopyrazin-2-yl)-5-methyl-2-azabicyclo[3.1.0]he xane-3-carboxamide | *** | 2.06 (A) | 642 |
| 214 | 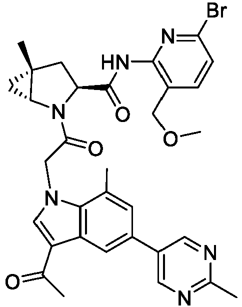 | (1R,3S,5R)-2-(2-(3-acetyl-7-methyl-5-(2-methylpyrimidin-5-yl)-1H-indol-1-yl)acetyl)-N-(6-bromo-3-(methoxymethyl)py ridin-2-yl)-5-methyl-2-azabicyclo[3.1.0]he xane-3-carboxamide | *** | 1.80 (A) | 646 |
| 216 | 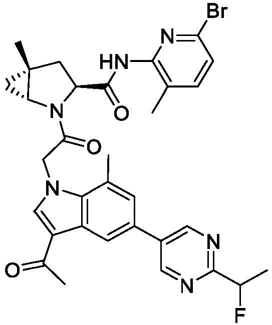 | (1R,3S,5R)-2-(2-(3-acetyl-5-(2-(1-fluoroethyl)pyrimid in-5-yl)-7-methyl-1H-indol-1-yl)acetyl)-N-(6-bromo-3-methylpyridin-2-yl)-5-methyl-2-azabicyclo[3.1.0]he xane-3-carboxamide | *** | 2.01 (A) | 647 |
| 217 | 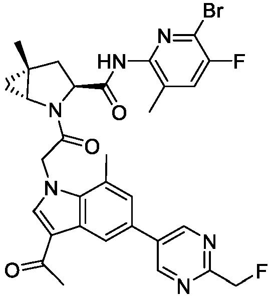 | (1R,3S,5R)-2-(2-(3-acetyl-5-(2-(fluoromethyl)pyri midin-5-yl)-7-methyl-1H-indol-1-yl)acetyl)-N-(6-bromo-5-fluoro-3-methylpyridin-2-yl)-5-methyl-2-azabicyclo[3.1.0]he xane-3-carboxamide | *** | 1.94 (A) | 651 |
| 218 | 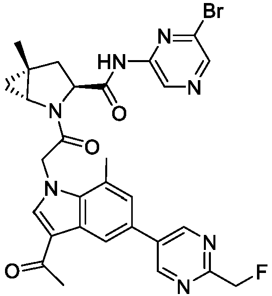 | (1R,3S,5R)-2-(2-(3-acetyl-5-(2-(fluoromethyl)pyri midin-5-yl)-7-methyl-1H-indol-1-yl)acetyl)-N-(6-bromopyrazin-2-yl)-5-methyl-2-azabicyclo[3.1.0]he xane-3-carboxamide | ** | 1.89 (A) | 622 |
| 220 | 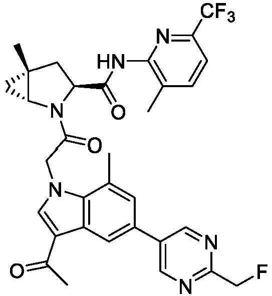 | (1R,3S,5R)-2-(2-(3-acetyl-5-(2-(fluoromethyl)pyri midin-5-yl)-7-methyl-1H-indol-1-yl)acetyl)-5-methyl-N-(3-methyl-6-(trifluoromethyl)py ridin-2-yl)-2-azabicyclo[3.1.0]he xane-3-carboxamide | *** | 3.55(B) | 623 |
| 221 | 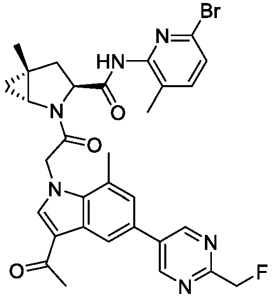 | (1R,3S,5R)-2-(2-(3-acetyl-5-(2-(fluoromethyl)pyri midin-5-yl)-7-methyl-1H-indol-1-yl)acetyl)-N-(6-bromo-3-methylpyridin-2-yl)-5-methyl-2-azabicyclo[3.1.0]he xane-3-carboxamide | *** | 3.52 (B) | 635 |
| 225 | 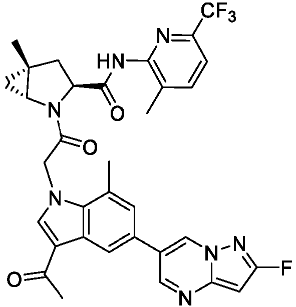 | (1R,3S,5R)-2-(2-(3-acetyl-5-(2-fluoropyrazolo[1,5-a]pyrimidin-6-yl)-7-methyl-1H-indol-1-yl)acetyl)-5-methyl-N-(3-methyl-6-(trifluoromethyl)py ridin-2-yl)-2-azabicyclo[3.1.0]he xane-3-carboxamide | *** | 2.30 (A) | 648 |
| 233 | 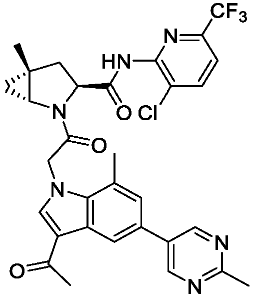 | (1R,3S,5R)-2-(2-(3-acetyl-7-methyl-5-(2-methylpyrimidin-5-yl)-1H-indol-1-yl)acetyl)-N-(3-chloro-6-(trifluoromethyl)py ridin-2-yl)-5-methyl-2-azabicyclo[3.1.0]he xane-3-carboxamide | *** | 3.62 (B) | 625 |
| 240 | 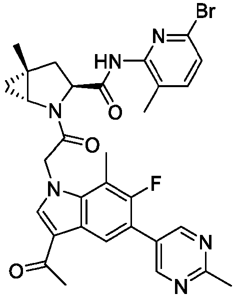 | (1R,3S,5R)-2-(2-(3-acetyl-6-fluoro-7-methyl-5-(2-methylpyrimidin-5-yl)-1H-indol-1-yl)acetyl)-N-(6-bromo-3-methylpyridin-2-yl)-5-methyl-2-azabicyclo[3.1.0]he xane-3-carboxamide | *** | 3.25 (B) | 633 |
| 243 | 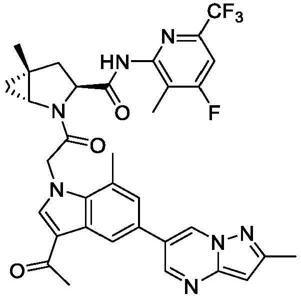 | (1R,3S,5R)-2-(2-(3-acetyl-7-methyl-5-(2-methylpyrazolo[1,5 -a]pyrimidin-6-yl)-1H-indol-1-yl)acetyl)-N-(4-fluoro-3-methyl-6-(trifluoromethyl)py ridin-2-yl)-5-methyl-2-azabicyclo[3.1.0]he xane-3-carboxamide | | | |
| 244 | 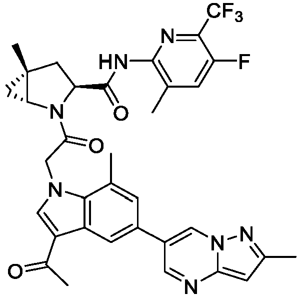 | (1R,3S,5R)-2-(2-(3-acetyl-7-methyl-5-(2-methylpyrazolo[1,5 -a]pyrimidin-6-yl)-1H-indol-1-yl)acetyl)-N-(5-fluoro-3-methyl-6-(trifluoromethyl)py ridin-2-yl)-5-methyl-2-azabicyclo[3.1.0]he xane-3-carboxamide | | | |
| 253 | 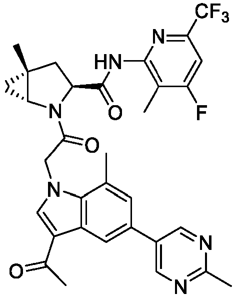 | (1R,3S,5R)-2-(2-(3-acetyl-7-methyl-5-(2-methylpyrimidin-5-yl)-1H-indol-1-yl)acetyl)-N-(4-fluoro-3-methyl-6-(trifluoromethyl)py ridin-2-yl)-5-methyl-2-azabicyclo[3.1.0]he xane-3-carboxamide | | | |
| 254 | 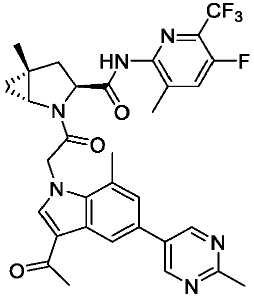 | (1R,3S,5R)-2-(2-(3-acetyl-7-methyl-5-(2-methylpyrimidin-5-yl)-1H-indol-1-yl)acetyl)-N-(5-fluoro-3-methyl-6-(trifluoromethyl)py ridin-2-yl)-5-methyl-2-azabicyclo[3.1.0]he xane-3-carboxamide | | | |
| 256 | 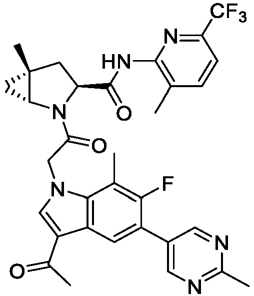 | (1R,3S,5R)-2-(2-(3-acetyl-6-fluoro-7-methyl-5-(2-methylpyrimidin-5-yl)-1H-indol-1-yl)acetyl)-5-methyl-N-(3-methyl-6-(trifluoromethyl)py ridin-2-yl)-2-azabicyclo[3.1.0]he xane-3-carboxamide | | | |
| 259 | 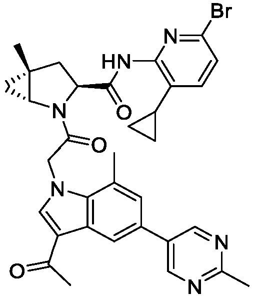 | (1R,3S,5R)-2-(2-(3-acetyl-7-methyl-5-(2-methylpyrimidin-5-yl)-1H-indol-1-yl)acetyl)-N-(6-bromo-3-cyclopropylpyridin-2-yl)-5-methyl-2-azabicyclo[3.1.0]he xane-3-carboxamide | | | |
| 269 | 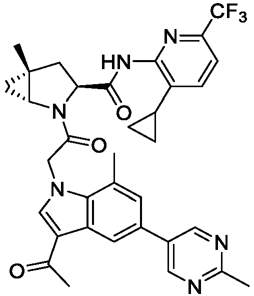 | (1R,3S,5R)-2-(2-(3-acetyl-7-methyl-5-(2-methylpyrimidin-5-yl)-1H-indol-1-yl)acetyl)-N-(3-cyclopropyl-6-(trifluoromethyl)py ridin-2-yl)-5-methyl-2-azabicyclo[3.1.0]he xane-3-carboxamide | | 3.2(B) | 631 |
| 271 | 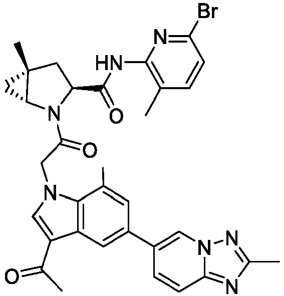 | (1R,3S,5R)-2-(2-(3-acetyl-7-methyl-5-(2-methyl-[1,2,4]triazolo[1,5-a]pyridin-6-yl)-1H-indol-1-yl)acetyl)-N-(6-bromo-3-methylpyridin-2-yl)-5-methyl-2-azabicyclo[3.1.0]he xane-3-carboxamide | | 1.82 (A) | 656 |
| 272 | 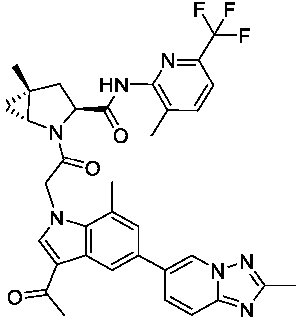 | (1R,3S,5R)-2-(2-(3-acetyl-7-methyl-5-(2-methyl-[1,2,4]triazolo[1,5-a]pyridin-6-yl)-1H-indol-1-yl)acetyl)-5-methyl-N-(3-methyl-6-(trifluoromethyl)py ridin-2-yl)-2-azabicyclo[3.1.0]he xane-3-carboxamide | | 1.95 (A) | 644 |

### EXAMPLE 3. HUMAN FACTOR D ASSAY

Human Factor D (purified from human serum, Complement Technology, Inc.) at 80 nM final concentration is incubated with test compound at various concentrations for 5 min at room temperature in 50 mM Tris, 1M NaCl, pH 7.5. A synthetic substrate Z-L-Lys-SBzl and DTNB (Ellman's reagent) are added to final concentrations of 100 µM each. Absorbance at 405 nm (A₄₀₅) is recorded at 30 second intervals for 30 min using a microplate spectrophotometer. IC₅₀ values are calculated by nonlinear regression of complement Factor D reaction rates as a function of test compound concentration.

### EXAMPLE 4. HEMOLYSIS ASSAY

The hemolysis assay was previously described by G. Ruiz-Gomez, et al., J. Med. Chem. (2009) 52: 6042-6052. Prior to the assay, the optimum concentration of Normal Human Serum (NHS) needed to achieve 100% lysis of rabbit erythrocytes (RE) is determined by titration. In the assay, NHS (Complement Technology) is diluted in GVB⁰ Buffer (0.1 % gelatin, 5 mM Veronal, 145 mM NaCl, 0.025 % NaN₃, pH 7.3, Complement Technology) plus 10 mM Mg-EGTA and incubated with test compound at various concentrations for 15 min at 37°C. RE (Complement Technology) freshly suspended in GVB⁰ plus 10 mM Mg-EGTA are added to a final concentration of 1 x 10⁸ cells/mL and reactions are incubated for 30 min at 37°C. Positive control reactions (100% lysis) consist of GVB⁰ plus 10 mM Mg-EGTA with NHS and RE but without test compound; negative control reactions (0% lysis) consist of GVB⁰ plus 10 mM Mg-EGTA with RE only. Samples are centrifuged at 2000g for 3 min and supernatants collected. Absorbance at 405 nm (A₄₀₅) is recorded using a microplate spectrophotometer. IC₅₀ values are calculated by nonlinear regression from the percentage of hemolysis as a function of test compound concentration.

This specification has been described with reference to embodiments of the invention. However, one of ordinary skill in the art appreciates that various modifications and changes can be made without departing from the scope of the invention as set forth in the claims below. Accordingly, the specification is to be regarded in an illustrative rather than a restrictive sense, and all such modifications are intended to be included within the scope of invention.

## Claims

1. A compound selected from 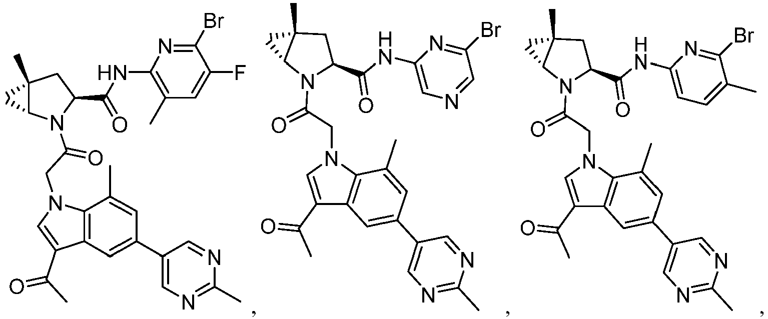 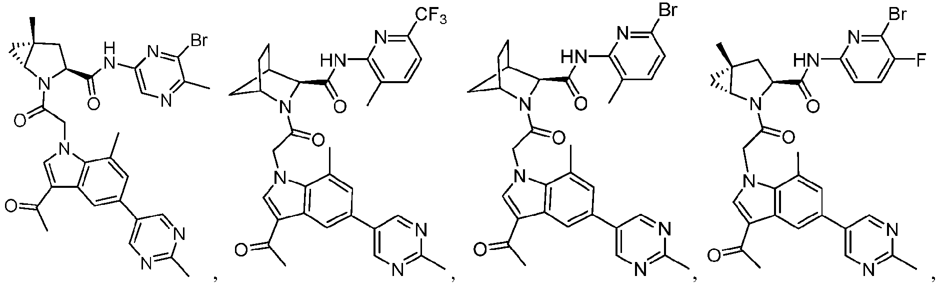 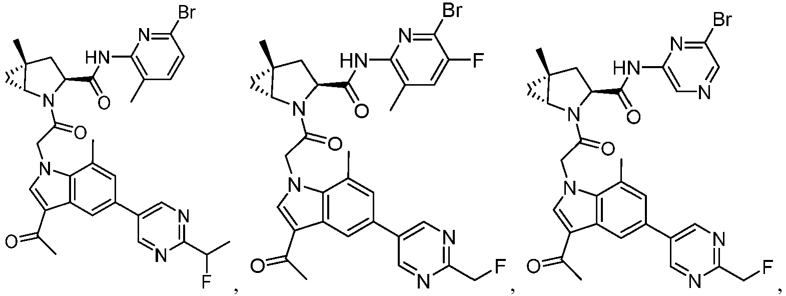 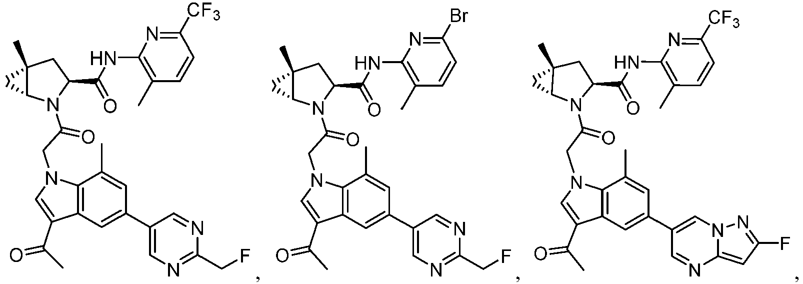 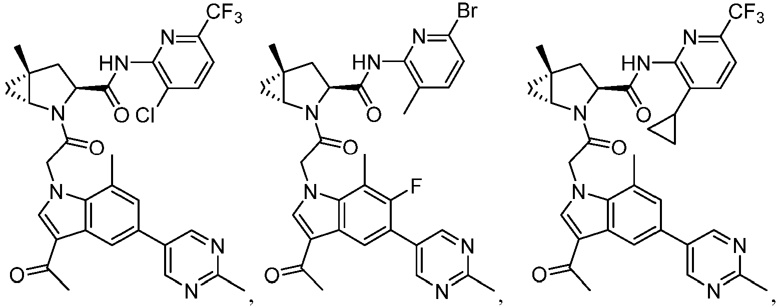 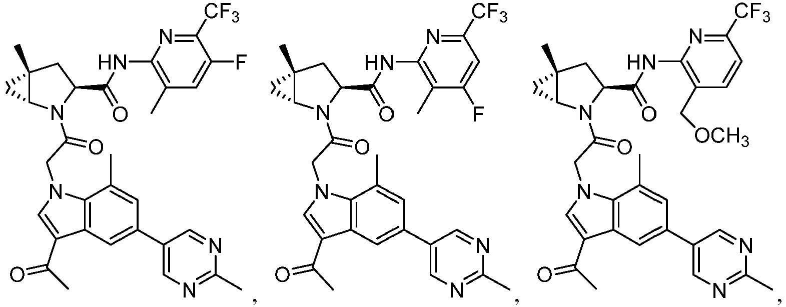 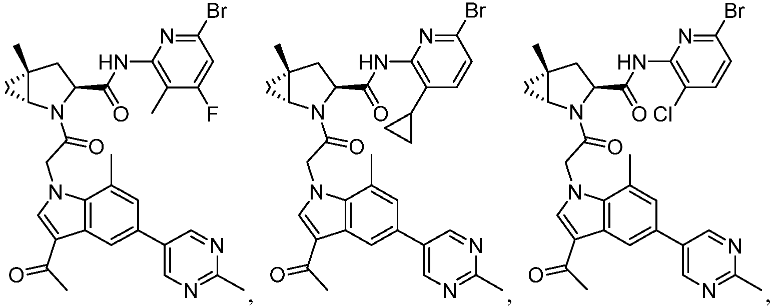 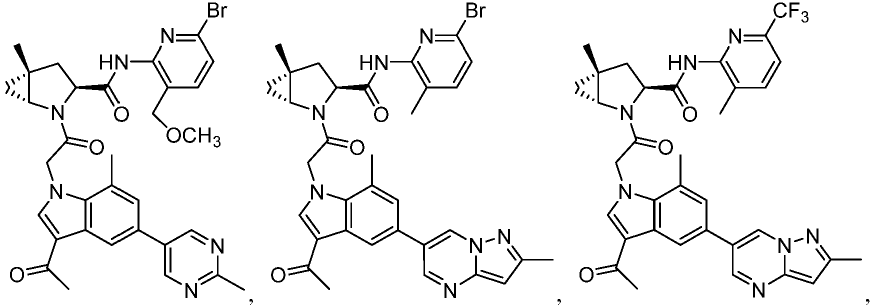 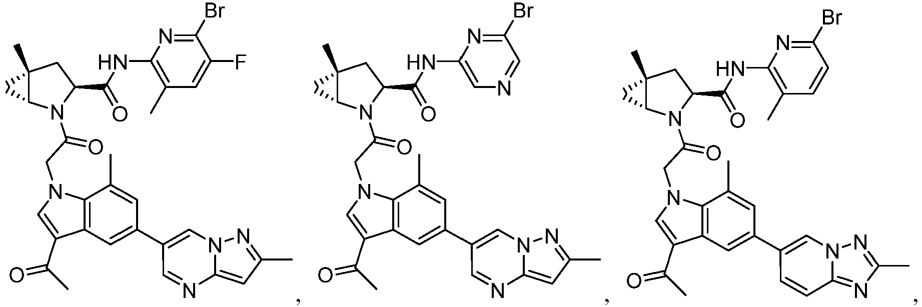 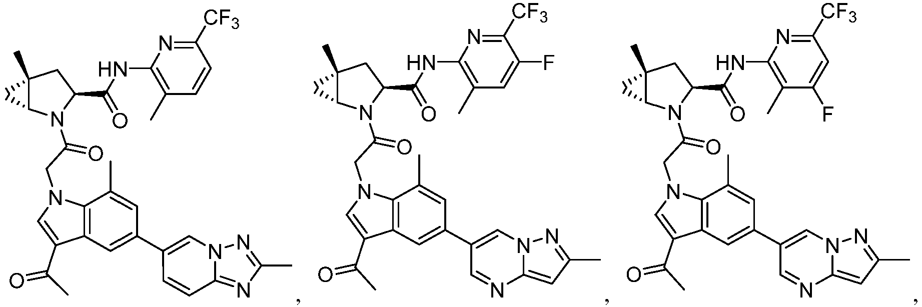 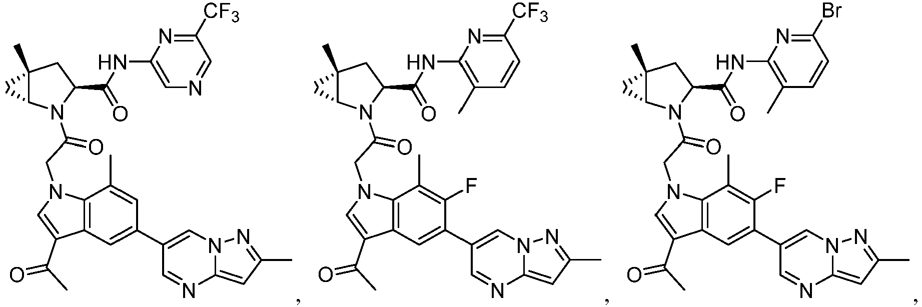 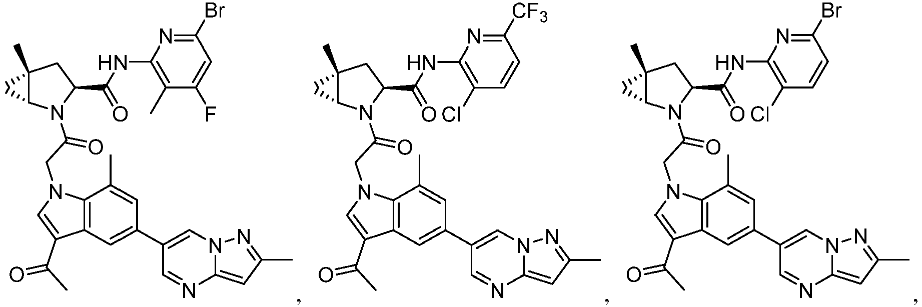 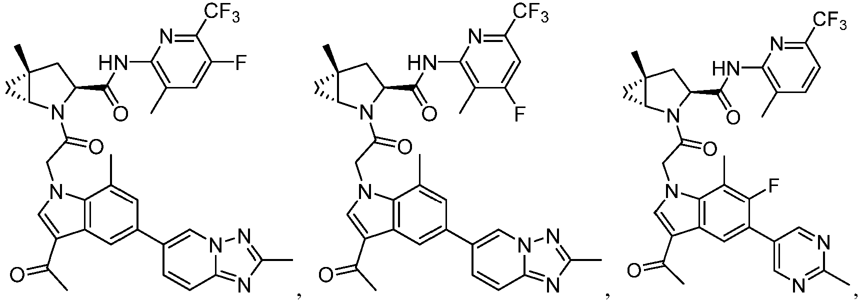 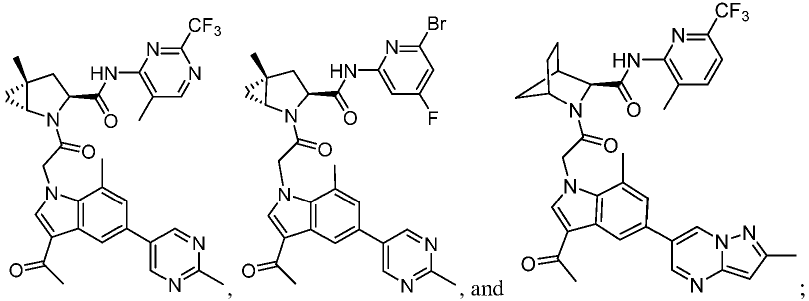 or a pharmaceutically acceptable salt thereof.

2. The compound of claim 1, selected from: 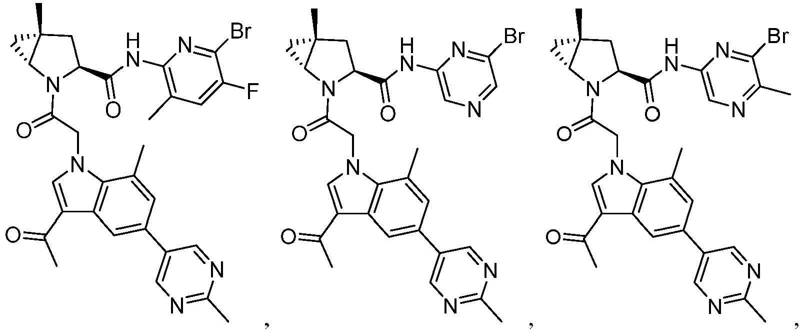 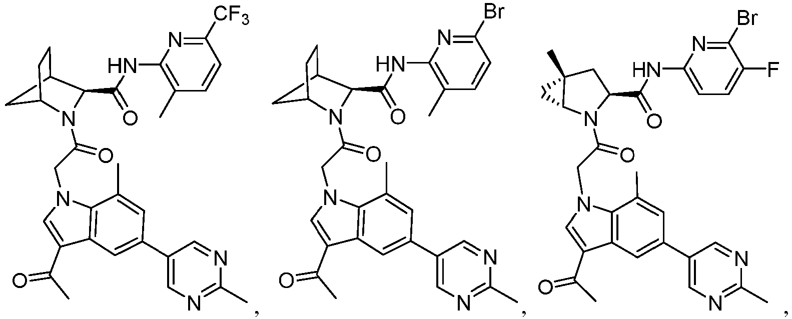 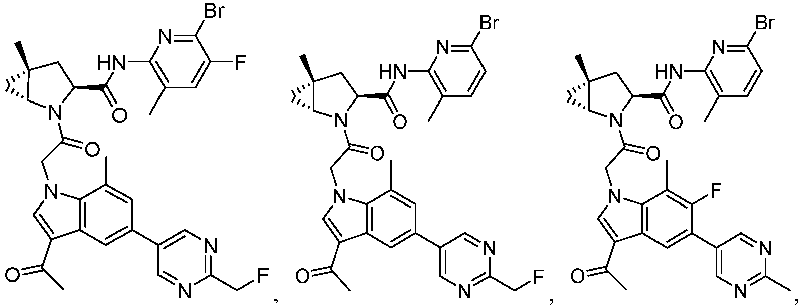 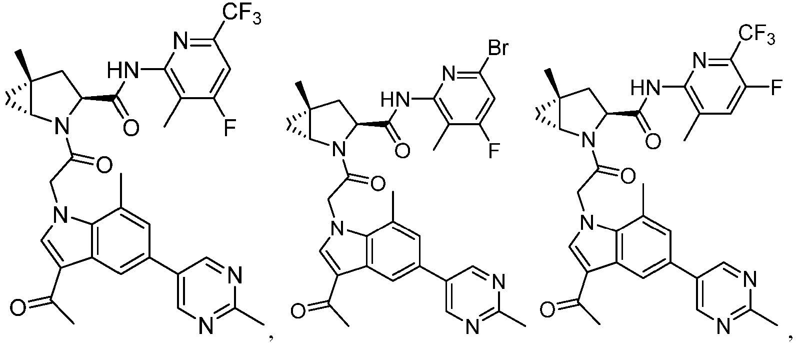 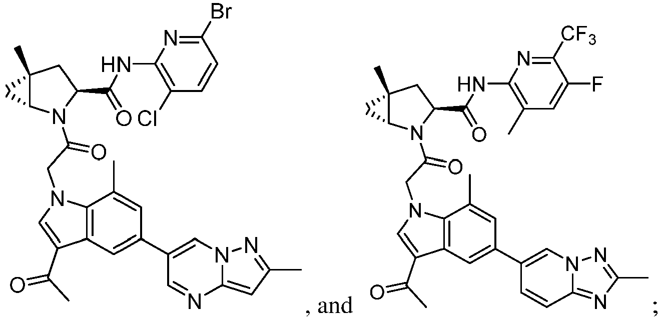 or a pharmaceutically acceptable salt thereof.

3. A pharmaceutical composition comprising a compound of claim 1 or claim 2 or a pharmaceutically acceptable salt thereof, and a pharmaceutically acceptable carrier.

4. A compound according to claim 1 or claim 2 or a pharmaceutically acceptable salt thereof, or a composition according to claim 3, for use in treating a complement factor D mediated disorder in a subject in need thereof, wherein said use comprises administering a therapeutically effective amount of said compound or pharmaceutical composition to said subject;
wherein the disorder is selected from age-related macular degeneration (AMD), Alzheimer's disease, amyotrophic lateral sclerosis (ALS), antibody-mediated transplant rejection, arthritis, atypical hemolytic uremic syndrome (aHUS), an autoimmune disease, a cardiovascular disease, C3 glomerulopathy, C3 glomerulonephritis, dense deposit disease, dermatomyositis, geographic atrophy, IgA nephropathy, lupus, lupus nephritis, multiple sclerosis, myasthenia gravis, neuromyelitis optica, an ophthalmic disorder, paroxysmal nocturnal hemoglobinuria (PNH), a respiratory disease, rheumatoid arthritis, and sickle cell disease.

5. A compound for use or composition for use according to claim 4, wherein the disorder is an ophthalmic disorder.

6. A compound for use or composition for use according to claim 4, wherein the disorder is C3 glomerulopathy, AMD, PNH, C3 glomerulonephritis, or dense deposit disease.

7. A compound for use or composition for use according to claim 4, wherein the disorder is lupus nephritis or IgA nephropathy.

8. A compound according to claim 1 or 2 or a pharmaceutically acceptable salt thereof, or a composition according to claim 3, for use in treating a complement factor D mediated disorder in a subject in need thereof, wherein said use comprises administering a therapeutically effective amount of said compound or pharmaceutical composition to said subject,
wherein the disorder is selected from hereditary angioedema, capillary leak syndrome, hemolytic uremic syndrome (HUS), a neurological disorder, Guillain Barre Syndrome, a disease of the central nervous system or other neurodegenerative condition, glomerulonephritis, SLE nephritis, proliferative nephritis, liver fibrosis, tissue regeneration and neural regeneration, Barraquer-Simons Syndrome, inflammatory effects of sepsis, systemic inflammatory response syndrome (SIRS), a disorder of inappropriate or undesirable complement activation, interleukin-2 induced toxicity during IL-2 therapy, an inflammatory disorder, inflammation of autoimmune diseases, system lupus erythematosus (SLE), lupus nephritis, arthritis, an immune complex disorder, an autoimmune disease, systemic lupus or lupus erythematosus, ischemia/ reperfusion injury (I/R injury), myocardial infarction, myocarditis, post-ischemic reperfusion conditions, balloon angioplasty, atherosclerosis, post-pump syndrome in cardiopulmonary bypass or renal bypass, renal ischemia, mesenteric artery reperfusion after aortic reconstruction, antiphospholipid syndrome, autoimmune heart disease, obesity, diabetes, Alzheimer's dementia, stroke, schizophrenia, traumatic brain injury, trauma, Parkinson's disease, epilepsy, transplant rejection, prevention of fetal loss, a biomaterial reaction, hyperacute allograft rejection, xenograft rejection, transplantation, psoriasis, burn injury, thermal injury, crush injury, asthma, allergy, acute respiratory distress syndrome (ARDS), cystic fibrosis, adult respiratory distress syndrome, dyspnea, hemoptysis, chronic obstructive pulmonary disease (COPD), emphysema, pulmonary embolisms and infarcts, pneumonia, a fibrogenic dust disease, pulmonary fibrosis, an organic dust disease, chemical injury, smoke injury, bronchoconstriction, hypersensitivity pneumonitis, a parasitic disease, Goodpasture's Syndrome, pulmonary vasculitis, Pauci-immune vasculitis, and immune complex-associated inflammation.

9. A compound according to claim 1 or 2 or a pharmaceutically acceptable salt thereof, or a composition according to claim 3, for use in treating a complement factor D mediated disorder in a subject in need thereof, wherein said use comprises administering a therapeutically effective amount of said compound or pharmaceutical composition to said subject, wherein the disorder is selected from fatty liver, nonalcoholic steatohepatitis (NASH), liver inflammation, cirrhosis, and liver failure.

10. A compound according to claim 1 or 2 or a pharmaceutically acceptable salt thereof, or a composition according to claim 3, for use in treating a complement factor D mediated disorder in a subject in need thereof, wherein said use comprises administering a therapeutically effective amount of said compound or pharmaceutical composition to said subject, wherein the disorder is selected from glaucoma, diabetic retinopathy, a blistering cutaneous disease, ocular cicatricial pemphigoid, uveitis, adult macular degeneration, diabetic retinopathy retinitis pigmentosa, macular edema, diabetic macular edema, Behcet's uveitis, multifocal choroiditis, Vogt-Koyanagi-Harada syndrome, intermediate uveitis, birdshot retinochoroiditis, sympathetic ophthalmia, ocular pemphigus, non-arteritic ischemic optic neuropathy, postoperative inflammation, retinal vein occlusion, and central retinal vein occlusion (CVRO).

11. A compound for use or composition for use according to any one of claims 4 to 10, wherein the subject is a human.

## Patentansprüche

1. Verbindung, ausgewählt aus: 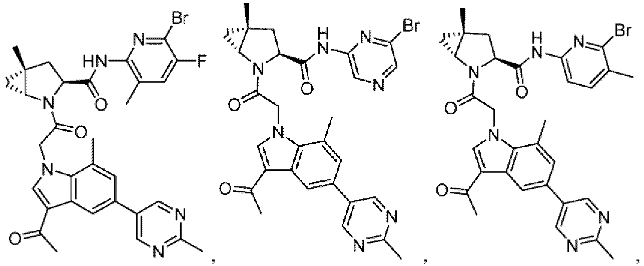 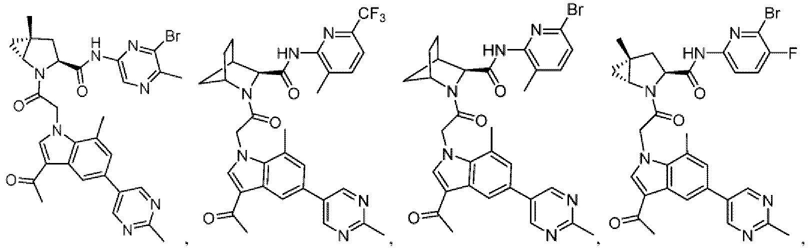 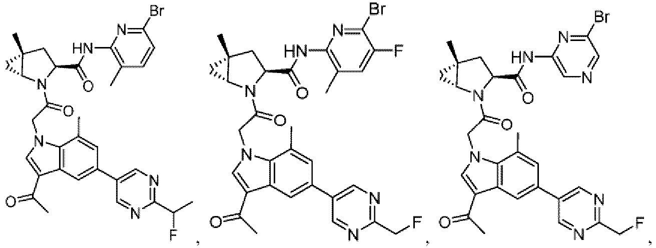 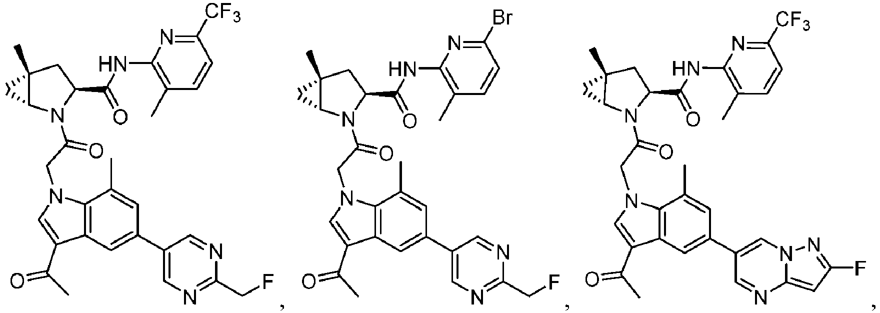 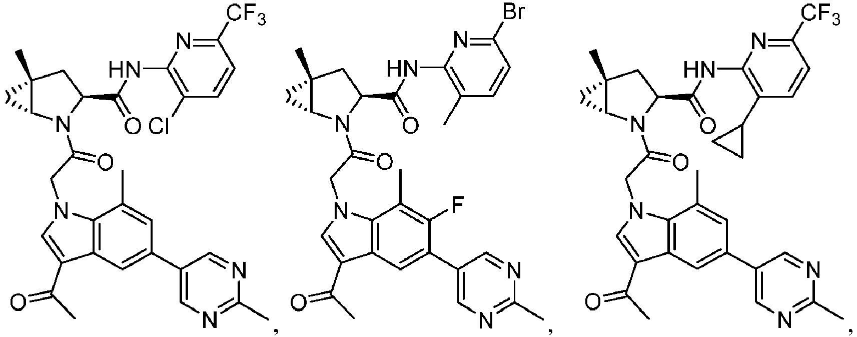 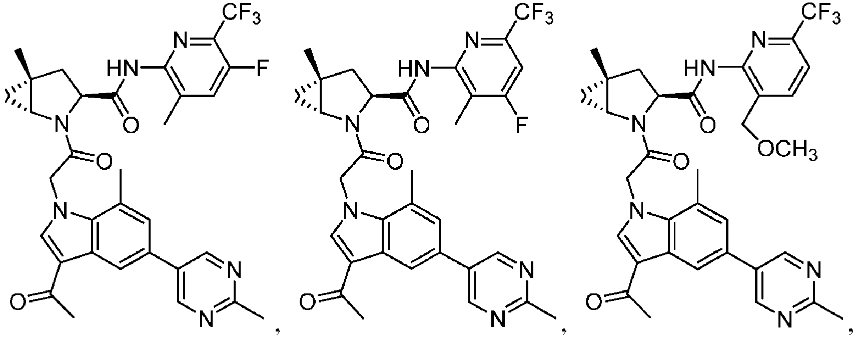 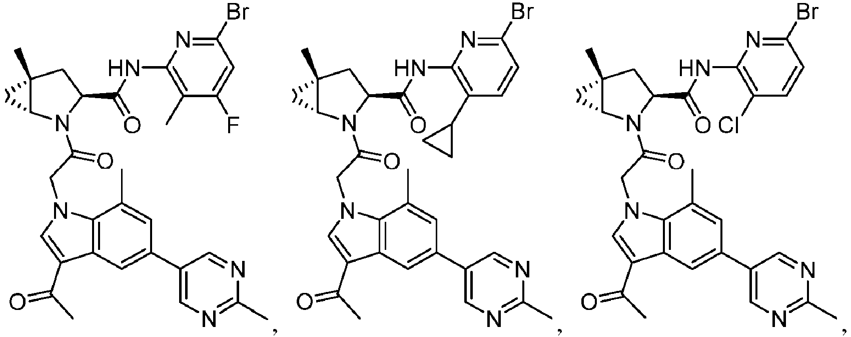 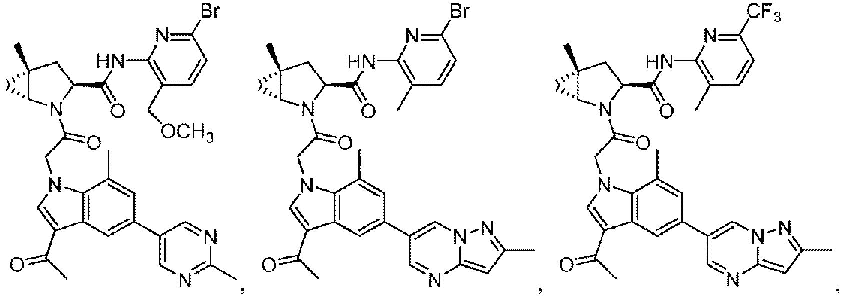 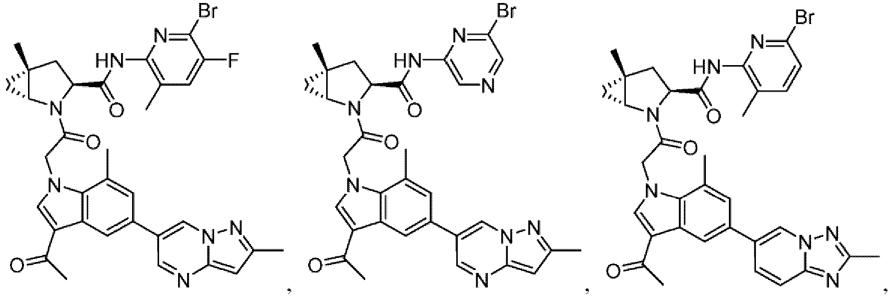 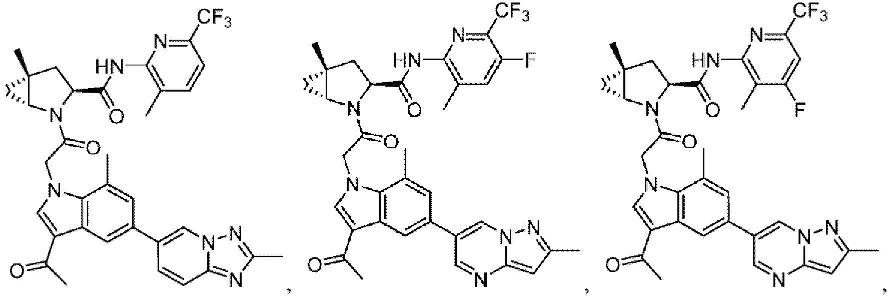 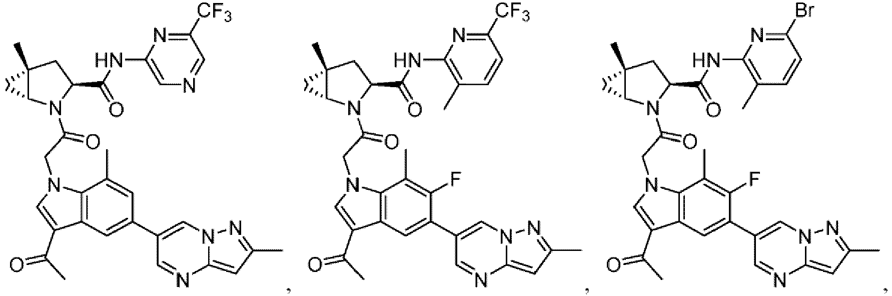 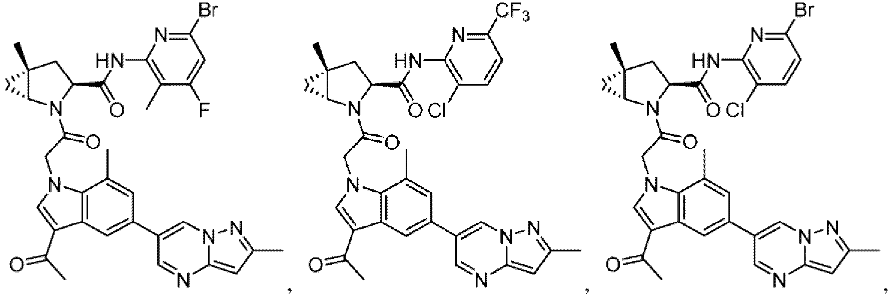 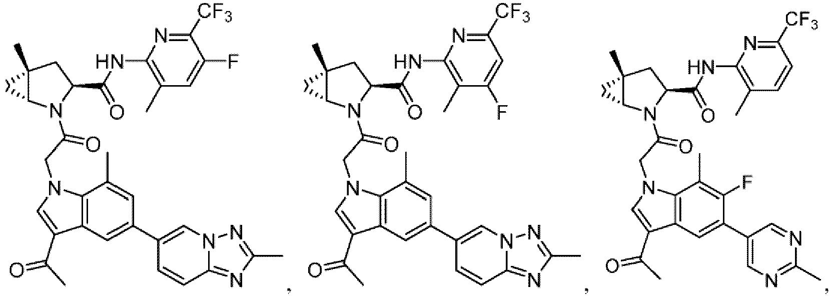 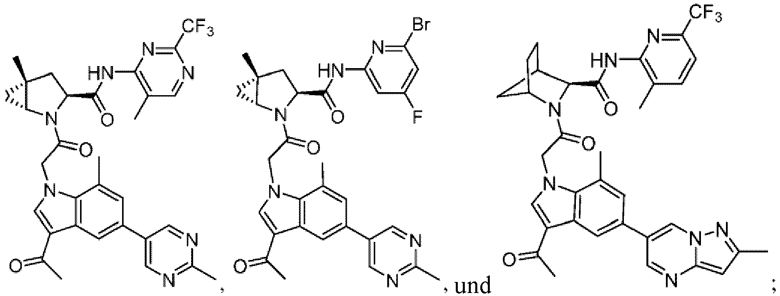 oder einem pharmazeutisch unbedenklichen Salz davon.

2. Verbindung nach Anspruch 1, ausgewählt aus: 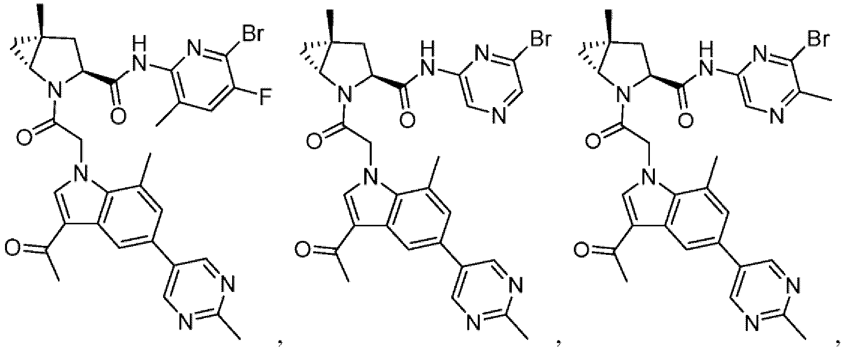 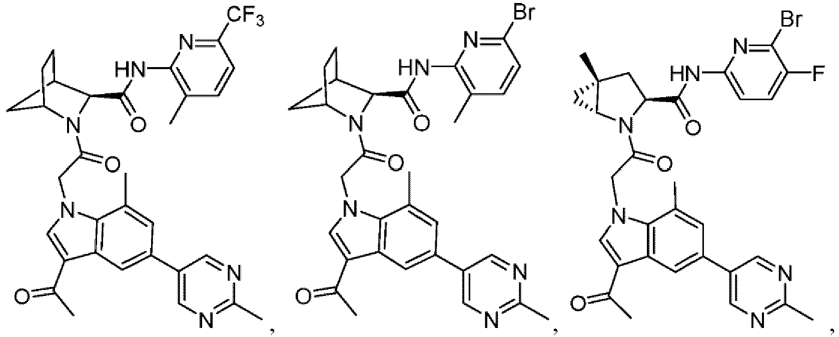 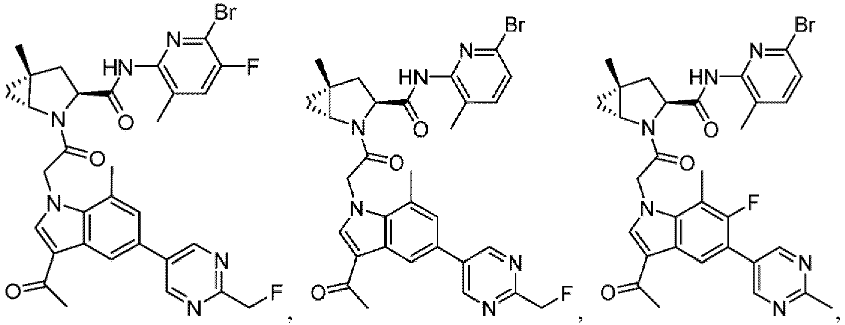 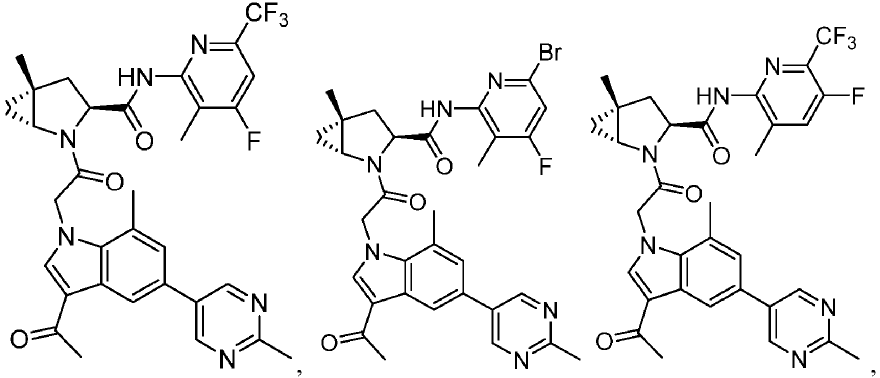 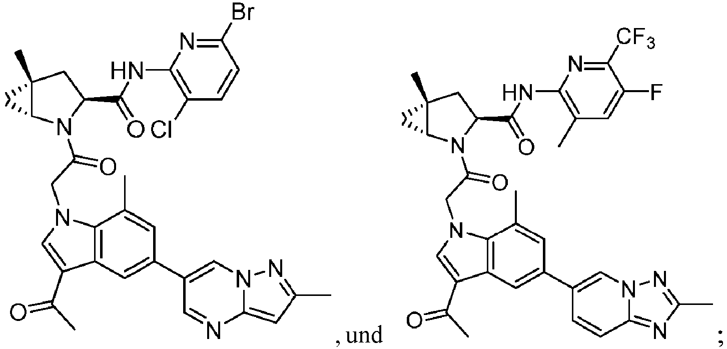 oder einem pharmazeutisch unbedenklichen Salz davon.

3. Pharmazeutische Zusammensetzung, umfassend eine Verbindung nach Anspruch 1 oder 2 oder ein pharmazeutisch unbedenkliches Salz davon und einen pharmazeutisch unbedenklichen Träger.

4. Verbindung nach Anspruch 1 oder 2 oder ein pharmazeutisch unbedenkliches Salz davon oder Zusammensetzung nach Anspruch 3 für die Verwendung bei der Behandlung einer durch den Komplementfaktor D vermittelten Erkrankung bei einem Subjekt, das dessen bedarf, wobei die Verwendung die Verabreichung einer therapeutisch wirksamen Menge der Verbindung oder der pharmazeutischen Zusammensetzung an das Subjekt umfasst;
wobei die Erkrankung aus altersbedingter Makuladegeneration (AMD), Alzheimer-Krankheit, amyotropher Lateralsklerose (ALS), Antikörper-vermittelter Transplantatabstoßung, Arthritis, atypischem hämolytisch-urämisches Syndrom (aHUS), einer Autoimmunerkrankung, einer kardiovaskulären Erkrankung, C3-Glomerulopathie, C3-Glomerulonephritis, membranoproliferativer Glomerulonephritis, Typ II, Dermatomyositis, geografischer Atrophie, IgA-Nephropathie, Lupus, Lupus nephritis, Multipler Sklerose, Myasthenia gravis, Neuromyelitis optica, einer Augenerkrankung, paroxysmaler nächtlicher Hämoglobinurie (PNH), einer Atemwegserkrankung, rheumatoider Arthritis und Sichelzellenanämie ausgewählt ist.

5. Verbindung für die Verwendung oder Zusammensetzung für die Verwendung nach Anspruch 4, wobei die Erkrankung eine Augenerkrankung ist.

6. Verbindung für die Verwendung oder Zusammensetzung für die Verwendung nach Anspruch 4, wobei die Erkrankung C3-Glomerulopathie, AMD, PNH, C3-Glomerulonephritis oder membranoproliferative Glomerulonephritis, Typ II, ist.

7. Verbindung für die Verwendung oder Zusammensetzung für die Verwendung nach Anspruch 4, wobei die Erkrankung Lupus nephritis oder IgA-Nephropathie ist.

8. Verbindung nach Anspruch 1 oder 2 oder ein pharmazeutisch unbedenkliches Salz davon, oder Zusammensetzung nach Anspruch 3 für die Verwendung bei der Behandlung einer durch den Komplementfaktor D vermittelten Erkrankung bei einem Subjekt, das dessen bedarf, wobei die Verwendung die Verabreichung einer therapeutisch wirksamen Menge der Verbindung oder der pharmazeutischen Zusammensetzung an das Subjekt umfasst,
wobei die Erkrankung aus dem hereditären Angioödem, dem Kapillarlecksyndrom, dem hämolytisch-urämischen Syndrom (HUS), einer neurologischen Erkrankung, dem Guillain-Barre-Syndrom, einer Erkrankung des zentralen Nervensystems oder einem anderen neurodegenerativen Zustand, Glomerulonephritis, SLE-Nephritis, proliferativer Nephritis, Leberfibrose, Geweberegeneration und neuronaler Regeneration, Barraquer-Simons-Syndrom, entzündlichen Auswirkungen der Sepsis, systemischem Entzündungsreaktionssyndrom (SIRS), einer Erkrankung der unangemessenen oder unerwünschten Komplementaktivierung, Interleukin-2-induzierter Toxizität während der IL-2-Therapie, einer Entzündungskrankheit, Entzündung bei Autoimmunerkrankungen, System-Lupus erythematosus (SLE), Lupus nephritis, Arthrose, einer Immunkomplexerkankung, einer Autoimmunerkrankung, systemischem Lupus oder Lupus erythematodes, Ischämie-/Reperfusionsverletzung (I/R-Verletzung), Myokardinfarkt, Myokarditis, post-ischämischen Reperfusionsbedingungen, Ballonangioplastie, Atherosklerose, Postperfusionssyndrom bei kardiopulmonalem Bypass oder Nierenbypass, Nierenischämie, Reperfusion der Mesenterialarterie nach Aortenrekonstruktion, Antiphospholipid-Syndrom, autoimmuner Herzerkrankung, Fettleibigkeit, Diabetes, Alzheimer-Demenz, Schlaganfall, Schizophrenie, traumatischer Hirnverletzung, Trauma, Parkinson-Krankheit, Epilepsie, Transplantatabstoßung, Verhinderung von Fötusverlusten, Reaktion auf Biomaterial, hyperakuter Allotransplantatabstoßung, Xenotransplantat-Abstoßung, Transplantation, Psoriasis, Verbrennungsverletzung, thermischer Verletzung, Quetschverletzung, Asthma, Allergie, akutem Atemnotsyndrom (ARDS), zystischer Fibrose, Atemnotsyndrom bei Erwachsenen, Dyspnoe, Hämoptyse, chronisch obstruktiver Lungenerkrankung (COPD), Emphysem, Lungenembolien und -infarkten, Lungenentzündung, einer fibrogenen Staubkrankheit, Lungenfibrose, einer organischen Staubkrankheit, chemischen Verletzungen, Rauchschäden, Bronchokonstriktion, Hypersensitivitätspneumonitis, einer parasitären Erkrankung, Goodpasture-Syndrom, pulmonaler Vaskulitis, Pauci-Immunvaskulitis und Immunkomplex-assoziierter Entzündung ausgewählt ist.

9. Verbindung nach Anspruch 1 oder 2 oder ein pharmazeutisch unbedenkliches Salz davon, oder Zusammensetzung nach Anspruch 3 für die Verwendung bei der Behandlung einer durch den Komplementfaktor D vermittelten Erkrankung bei einem Subjekt, das dessen bedarf, wobei die Verwendung die Verabreichung einer therapeutisch wirksamen Menge der Verbindung oder der pharmazeutischen Zusammensetzung an das Subjekt umfasst, wobei die Erkrankung aus Fettleber, nichtalkoholischer Steatohepatitis (NASH), Leberentzündung, Zirrhose und Leberversagen ausgewählt ist.

10. Verbindung nach Anspruch 1 oder 2 oder ein pharmazeutisch unbedenkliches Salz davon oder Zusammensetzung nach Anspruch 3 für die Verwendung bei der Behandlung einer durch den Komplementfaktor D vermittelten Erkrankung bei einem Subjekt, das dessen bedarf, wobei die Verwendung die Verabreichung einer therapeutisch wirksamen Menge der Verbindung oder der pharmazeutischen Zusammensetzung an das Subjekt umfasst, wobei die Erkrankung aus Glaukom, diabetischer Retinopathie, einer blasenbildenden Hauterkrankung, okularem cicatricialem Pemphigoid, Uveitis, Makuladegeneration bei Erwachsenen, diabetischer Retinopathie, Retinitis pigmentosa, Makulaödem, diabetischem Makulaödem, Behcet-Uveitis, multifokaler Choroiditis, Vogt-Koyanagi-Harada-Syndrom, intermediärer Uveitis, Birdshot-Retinochoroiditis, sympathischer Ophthalmie, okulärem Pemphigus, nichtarterieller ischämischer Optikusneuropathie, postoperativer Entzündung, Netzhautvenenverschluss und Zentralvenenverschluss (CVRO) ausgewählt ist.

11. Verbindung für die Verwendung oder Zusammensetzung für die Verwendung nach einem der Ansprüche 4 bis 10, wobei das Subjekt ein Mensch ist.

## Revendications

1. Composé sélectionné parmi 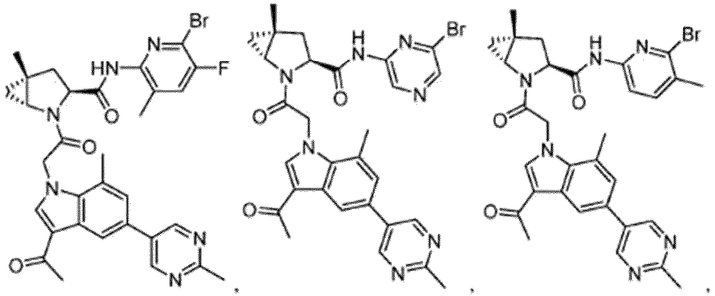 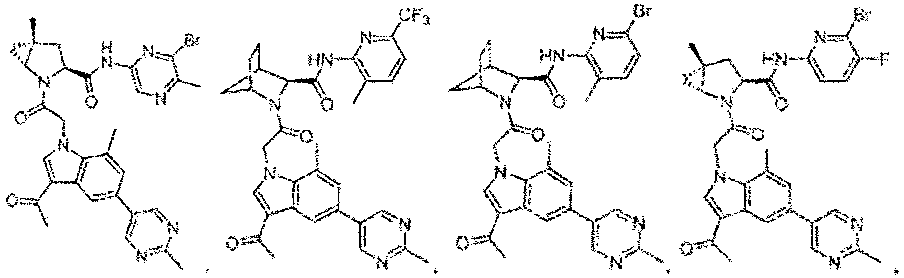 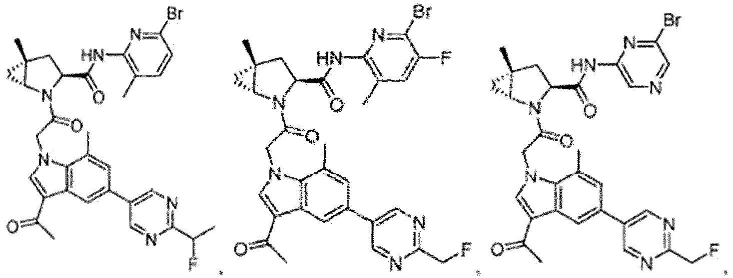 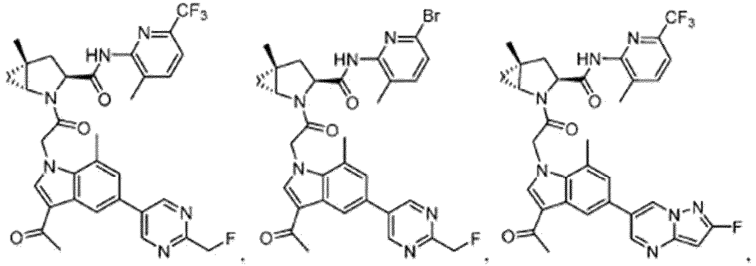 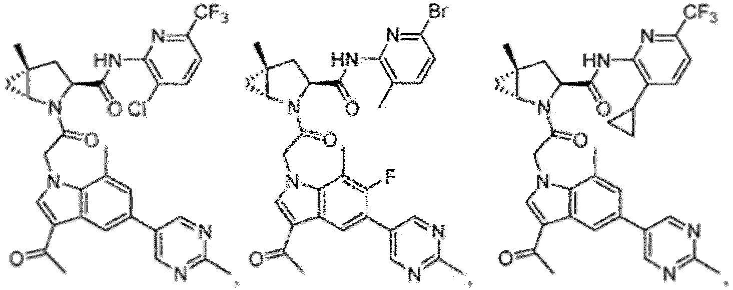 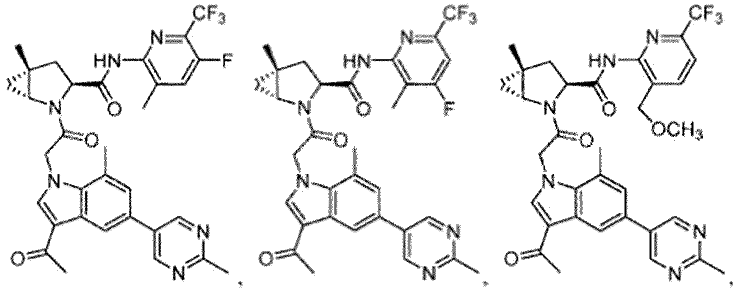 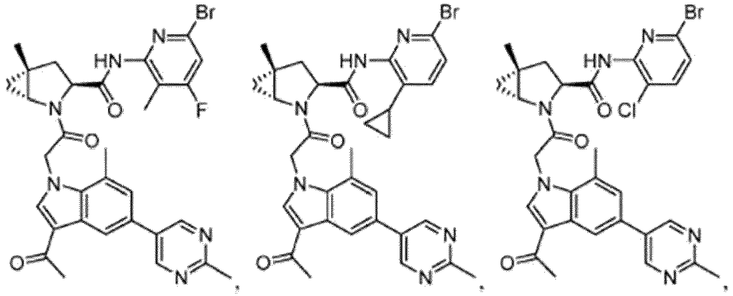 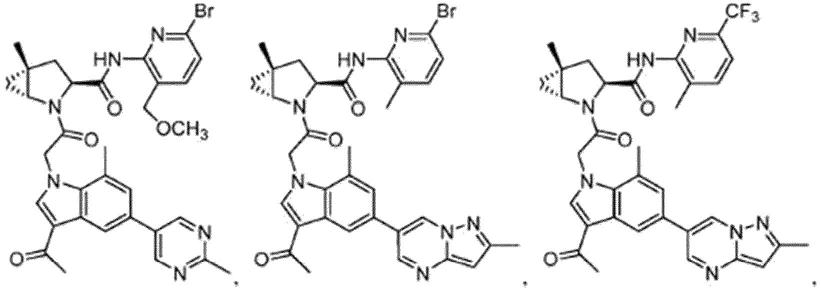 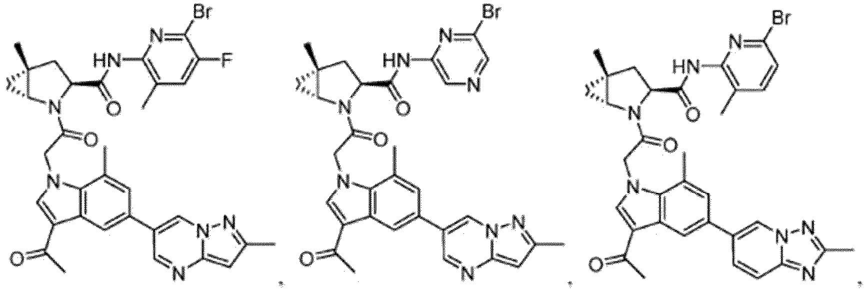 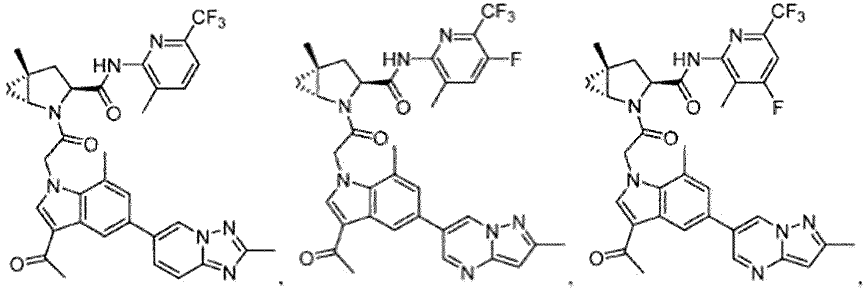 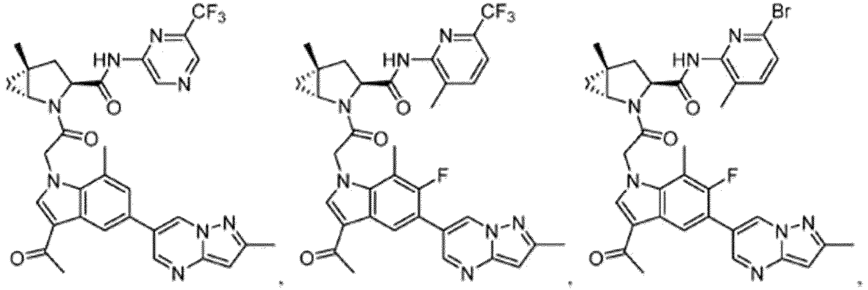 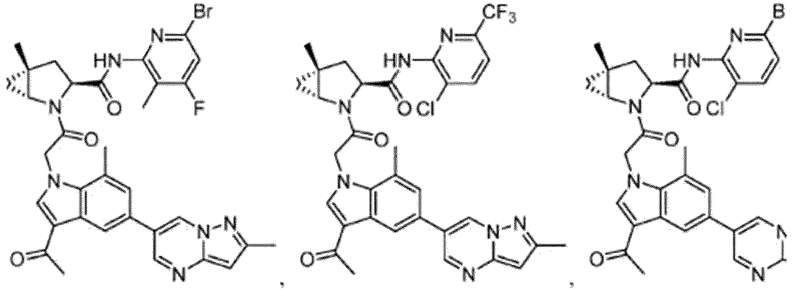 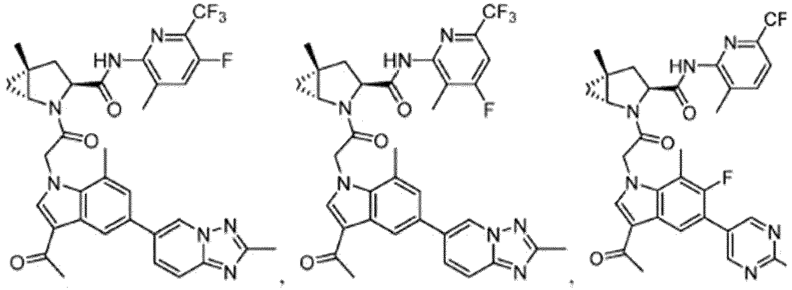 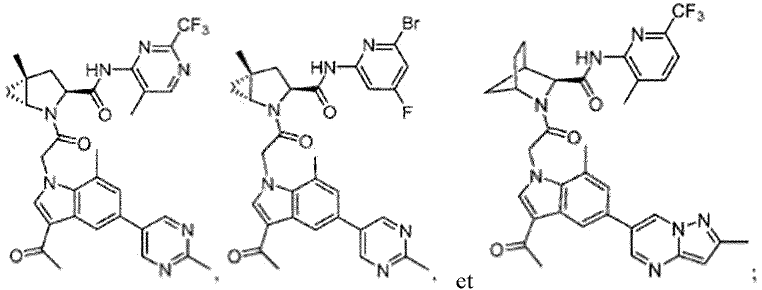 ou sel pharmaceutiquement acceptable de celui-ci.

2. Composé selon revendication 1, sélectionné parmi : 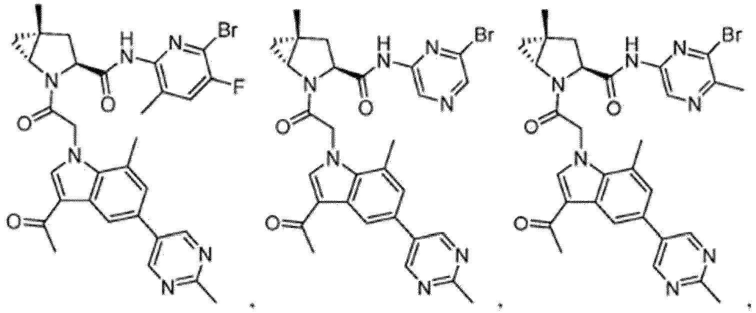 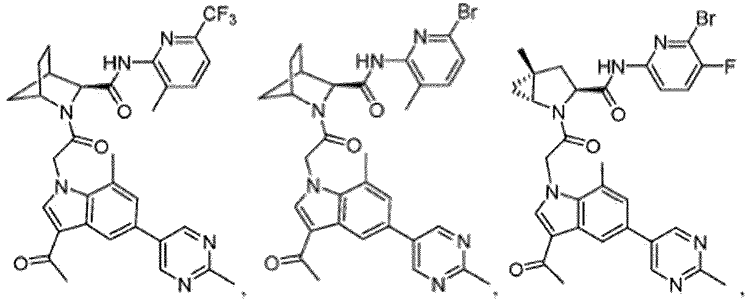 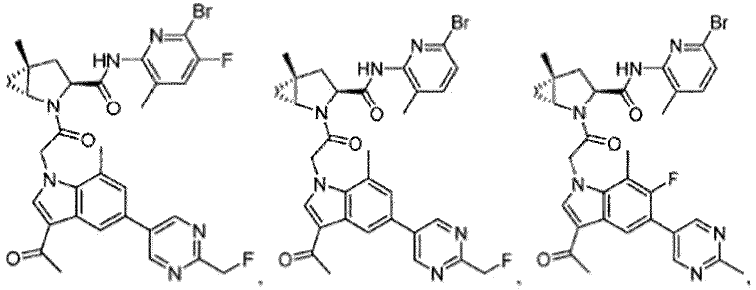 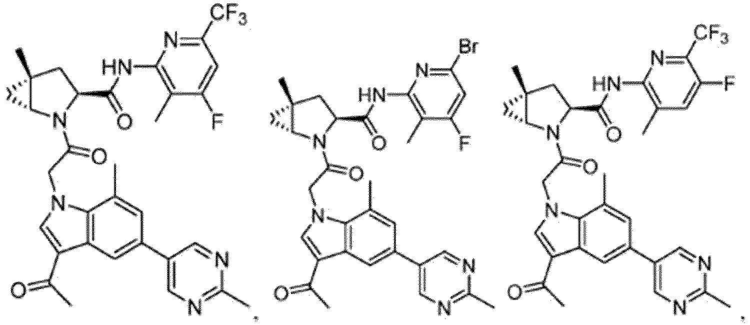 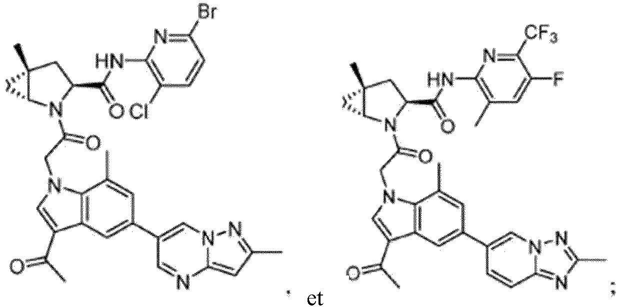 ou sel pharmaceutiquement acceptable de celui-ci.

3. Composition pharmaceutique comprenant un composé de la revendication 1 ou de la revendication 2 ou un sel pharmaceutiquement acceptable de celui-ci, et un support pharmaceutiquement acceptable.

4. Composé selon la revendication 1 ou la revendication 2 ou sel pharmaceutiquement acceptable de celui-ci, ou composition selon la revendication 3, pour une utilisation dans le traitement d'un trouble médié par le facteur D du complément chez un sujet qui en a besoin, dans lequel ladite utilisation comprend l'administration d'une quantité thérapeutiquement efficace dudit composé ou de ladite composition pharmaceutique audit sujet ;
dans lequel le trouble est sélectionné parmi la dégénérescence maculaire liée à l'âge (DMLA), la maladie d'Alzheimer, la sclérose latérale amyotrophique (SLA), le rejet de greffe à médiation par anticorps, l'arthrite, le syndrome hémolytique et urémique atypique (SHUa), une maladie auto-immune, une maladie cardiovasculaire, la glomérulopathie C3, la glomérulonéphrite C3, la maladie des dépôts denses, la dermatomyosite, l'atrophie géographique, la néphropathie à IgA, le lupus, la néphrite lupique, la sclérose en plaques, la myasthénie grave, la neuromyélite optique, un trouble ophtalmique, l'hémoglobinurie paroxystique nocturne (HPN), une maladie respiratoire, la polyarthrite rhumatoïde, et la drépanocytose.

5. Composé pour une utilisation ou composition pour une utilisation selon la revendication 4, dans lequel le trouble est un trouble ophtalmique.

6. Composé pour une utilisation ou composition pour une utilisation selon la revendication 4, dans lequel le trouble est la glomérulopathie C3, la DMLA, la HPN, la glomérulonéphrite C3, ou la maladie des dépôts denses.

7. Composé pour une utilisation ou composition pour une utilisation selon la revendication 4, dans lequel le trouble est la néphrite lupique ou la néphropathie IgA.

8. Composé selon la revendication 1 ou 2 ou sel pharmaceutiquement acceptable de celui-ci, ou composition selon la revendication 3, pour une utilisation dans le traitement d'un trouble médié par le facteur D du complément chez un sujet qui en a besoin, dans lequel ladite utilisation comprend l'administration d'une quantité thérapeutiquement efficace dudit composé ou de ladite composition pharmaceutique audit sujet,
dans lequel le trouble est sélectionné parmi l'angio-œdème héréditaire, le syndrome de fuite capillaire, le syndrome hémolytique et urémique (SHU), un trouble neurologique, le syndrome de Guillain-Barré, une maladie du système nerveux central ou une autre affection neurodégénérative, la glomérulonéphrite, la néphrite du LED, la néphrite proliférative, la fibrose hépatique, la régénération tissulaire et neuronale, le syndrome de Barraquer-Simons, les effets inflammatoires de la septicémie, le syndrome de réponse inflammatoire systémique (SIRS), un trouble d'activation inappropriée ou indésirable du complément, une toxicité induite par l'interleukine-2 au cours d'un traitement par IL-2, un trouble inflammatoire, l'inflammation de maladies auto-immunes, le lupus érythémateux disséminé (LED), la néphrite lupique, l'arthrite, un trouble du complexe immunitaire, une maladie auto-immune, le lupus systémique ou le lupus érythémateux, une lésion d'ischémie/reperfusion (I/R), l'infarctus du myocarde, la myocardite, des affections de reperfusion post-ischémique, l'angioplastie par ballonnet, l'athérosclérose, le syndrome post-pompe dans le cadre d'une dérivation cardio-pulmonaire ou d'une dérivation rénale, l'ischémie rénale, une reperfusion d'artère mésentérique après une reconstruction aortique, le syndrome des antiphospholipides, une maladie cardiaque auto-immune, l'obésité, le diabète, la démence d'Alzheimer, l'accident vasculaire cérébral, la schizophrénie, une lésion cérébrale traumatique, un traumatisme, la maladie de Parkinson, l'épilepsie, le rejet de greffe, la prévention de la perte fœtale, une réaction à un biomatériau, un rejet suraigu d'allogreffe, un rejet de xénogreffe, une transplantation, le psoriasis, une lésion par brûlure, une lésion thermique, une lésion par écrasement, l'asthme, l'allergie, le syndrome de détresse respiratoire aiguë (SDRA), la fibrose kystique, le syndrome de détresse respiratoire de l'adulte, la dyspnée, l'hémoptysie, la bronchopneumopathie chronique obstructive (BPCO), l'emphysème, les embolies et infarctus pulmonaires, la pneumonie, une maladie fibrogène due à la poussière, la fibrose pulmonaire, une maladie due à la poussière organique, une lésion chimique, une lésion due à la fumée, la bronchoconstriction, la pneumopathie d'hypersensibilité, une maladie parasitaire, le syndrome de Goodpasture, la vascularite pulmonaire, une vascularite pauci-immune et une inflammation associée au complexe immunitaire.

9. Composé selon la revendication 1 ou 2 ou sel pharmaceutiquement acceptable de celui-ci, ou composition selon la revendication 3, pour une utilisation dans le traitement d'un trouble médié par le facteur D du complément chez un sujet qui en a besoin, dans lequel ladite utilisation comprend l'administration d'une quantité thérapeutiquement efficace dudit composé ou de ladite composition pharmaceutique audit sujet, dans lequel le trouble est sélectionné parmi la stéatose hépatique, la stéatohépatite non alcoolique (SHNA), l'inflammation hépatique, la cirrhose, et l'insuffisance hépatique.

10. Composé selon la revendication 1 ou 2 ou sel pharmaceutiquement acceptable de celui-ci, ou composition selon la revendication 3, pour une utilisation dans le traitement d'un trouble médié par le facteur D du complément chez un sujet qui en a besoin, dans lequel ladite utilisation comprend l'administration d'une quantité thérapeutiquement efficace dudit composé ou de ladite composition pharmaceutique audit sujet, dans lequel le trouble est sélectionné parmi le glaucome, la rétinopathie diabétique, une maladie cutanée bulleuse, la pemphigoïde cicatricielle oculaire, l'uvéite, la dégénérescence maculaire de l'adulte, la rétinopathie diabétique et la rétinite pigmentaire, l'œdème maculaire, l'œdème maculaire diabétique, l'uvéite de Behçet, la choroïdite multifocale, le syndrome de Vogt-Koyanagi-Harada, l'uvéite intermédiaire, la rétinochoroïdite de type Birdshot, l'ophtalmie sympathique, le pemphigus oculaire, la neuropathie optique ischémique non artéritique, l'inflammation postopératoire, l'occlusion de veine rétinienne, et l'occlusion de la veine centrale de la rétine (OVCR).

11. Composé pour une utilisation ou composition pour une utilisation selon l'une quelconque des revendications 4 à 10, dans lequel le sujet est un humain.
